# EUROPEAN PATENT SPECIFICATION

(11) **EP 4 055 192 B1**
(45) Date of publication and mention of the grant of the patent: **31.12.2025**
(21) Application number: 20885701.1
(22) Date of filing: 05.11.2020
(51) Int. Cl.: C12Q 1/6883

(54) **DIAGNOSIS AND TREATMENT OF NAFLD AND LIVER FIBROSIS**
DIAGNOSE UND BEHANDLUNG VON NAFLD UND LEBERFIBROSE
DIAGNOSTIC ET TRAITEMENT DE LA NAFLD ET DE LA FIBROSE HÉPATIQUE

(30) Priority: 05.11.2019 US 201962931138 P
(43) Date of publication of application: 14.09.2022
(73) Proprietor: Beth Israel Deaconess Medical Center, Inc., Boston, MA 02215 (US)
(72) Inventor: MANTZOROS, Christos, Boston, MA 02215 (US); PERAKAKIS, Nikolaos, Boston, MA 02215 (US); YAZDANI, Alireza, New York, NY 10036 (US)
(74) Representative: Simmons & Simmons LLP (Munich)
(86) International application number: PCT/US2020/059229
(87) International publication number: WO 2021/092265

(56) References cited:
- WO-A1-2017/001600
- WO-A1-2017/167821
- WO-A1-2017/167821
- WO-A2-2013/149258
- US-B2- 7 244 619
- RINA SA ET AL: "Discovering a critical transition state from nonalcoholic hepatosteatosis to nonalcoholic steatohepatitis by lipidomics and dynamical network biomarkers", JOURNAL OF MOLECULAR CELL BIOLOGY, vol. 8, no. 3, 18 March 2016 (2016-03-18), pages 195 - 206, XP055320126, ISSN: 1674-2788, DOI: 10.1093/jmcb/mjw016
- IRUARRIZAGA-LEJARRETA M ET AL: "Role of Aramchol in Steatohepatitis and Fibrosis in Mice", HEPATOLOGY COMMUNICATIONS,, vol. 1, no. 9, 3 November 2017 (2017-11-03), pages 911 - 927, XP002778683
- KANG-YU PENG ET AL: "Mitochondrial dysfunction-related lipid changes occur in nonalcoholic fatty liver disease progression", JOURNAL OF LIPID RESEARCH, vol. 59, no. 10, 24 July 2018 (2018-07-24), US, pages 1977 - 1986, XP055690950, ISSN: 0022-2275, DOI: 10.1194/jlr.M085613
- GORDEN D.LEE ET AL: "Biomarkers of NAFLD progression: a lipidomics approach to an epidemic", JOURNAL OF LIPID RESEARCH, vol. 56, no. 3, 1 March 2015 (2015-03-01), US, pages 722 - 736, XP093084291, ISSN: 0022-2275, DOI: 10.1194/jlr.P056002
- "Adiponectin", WIKIPEDIA, 23 September 2019 (2019-09-23), XP055825247, Retrieved from the Internet <URL:https://en.wikipedia.org/w/index.php?title=Adiponectin&oldid=917314304>

## Description

The specification includes Lengthy Tables 6-16. Lengthy Tables 6-16 have been submitted via EFS-Web in electronic format as follows: File name: Lengthy Table 6.txt, Date created: November 4, 2019, File Size 731 bytes; File name: Lengthy Table 7.txt, Date created: November 4, 2019, File Size 10.6 KB; File name: Lengthy Table 8.txt, Date created: November 4, 2019, File Size 40.7 KB; File name: Lengthy Table 9.txt, Date created: November 4, 2019, File Size 2.38 KB; File name: Lengthy Table 10.txt, Date created: November 4, 2019, File Size 15.7 KB; File name: Lengthy Table 11.txt, Date created: November 4, 2019, File Size 258 KB; File name: Lengthy Table 12.txt, Date created: November 4, 2019, File Size 18.3 KB; File name: Lengthy Table 13.txt, Date created: November 4, 2019, File Size 57.3 KB; File name: Lengthy Table 14.txt, Date created: November 4, 2019, File Size 6.13 KB; File name: Lengthy Table 15.txt, Date created: November 4, 2019, File Size 16.6 KB; File name: Lengthy Table 16.txt, Date created: November 4, 2019, File Size 353 KB.

Please refer to the end of the specification for access instructions.

### Background of the Invention

Non-alcoholic fatty liver disease (NAFLD) affects 25% of the general population and is characterized by the presence of non-alcoholic fatty liver (NAFL) that can progress to non-alcoholic steatohepatitis (NASH), liver fibrosis, and cirrhosis leading to hepatocellular carcinoma. To date, liver biopsy is the gold standard for the diagnosis of NASH and for staging liver fibrosis, although liver biopsy is invasive, expensive, and may lead to complications such as bleeding, infection, and death. In addition, the liver biopsy specimen may not be representative of the status of the entire liver, which may lead to misdiagnosis. Moreover, it is not feasible to perform a liver biopsy on every patient at risk of NASH or liver fibrosis. Therefore, NAFLD remains undiagnosed in most patients.

Thus, there remains an unmet clinical need for non-invasive, reliable, highly-sensitive tests for NAFL, NASH, and liver fibrosis. SA et al., JOURNAL OF MOLECULAR CELL BIOLOGY, vol. 8, no. 3, 18 March 2016 (2016-03-18), pages 195-206 describes that they identified a critical transition stage (termed pre-NASH) during the progression from hepatosteatosis to nonalcoholic steatohepatitis (NASH) in a mouse model of high fat-induced nonalcoholic fatty liver disease (NAFLD) using lipidomics and a mathematical model termed dynamic network biomarkers (DNB).

IRUARRIZAGA-LEJARRETA et al., HEPATOLOGY COMMUNICATIONS,, vol. 1, no. 9, 3 November 2017 (2017-11-03), pages 911-927 investigated Aramchol's mechanism of action and its effect on fibrosis using the methionine- and choline-deficient (MCD) mice diet model of NASH. Comparison of the serum metabolomic pattern between 0.1MCD-fed mice and patients with NAFLD showed a substantial overlap. They conclude that Aramchol treatment improved steatohepatitis and fibrosis

PENG et al., JOURNAL OF LIPID RESEARCH, vol. 59, no. 10, 24 July 2018 (2018-07-24), pages 1977-1986, performed lipidomic analysis of plasma and liver biopsy samples from obese patients with nonalcoholic fatty liver (NAFL) or nonalcoholic steatohepatitis (NASH) and from those without nonalcoholic fatty liver disease (NAFLD).

GORDEN et al., JOURNAL OF LIPID RESEARCH, vol. 56, no. 3, 1 March 2015 (2015-03-01), pages 722-736, describe an "omics" approach to detecting a reproducible signature of lipid metabolites, aqueous intracellular metabolites, SNPs, and mRNA transcripts in a double-blinded study of patients with different stages of NAFLD that involves profiling liver biopsies, plasma, and urine samples

WO 2017/167821 relates to the field of diagnosis, prognosis and treatment of liver conditions, including nonalcoholic steatohepatitis (NASH), in a human individual. The inventors have identified herein a lipid signature associated with the occurrence of liver conditions, by combining, in a global approach, lipidomics with an unbiased learning machine statistical analysis. They suggested a plurality of lipids, for medical uses and for the preparation of a medicament.

### Summary of the Invention

The claimed invention is as defined in the claims. Particularly, the claims are directed to a method for diagnosing nonalcoholic steatohepatitis (NASH) or nonalcoholic fatty liver (NAFL) in a human subject, a method of monitoring treatment efficacy in a human subject being treated with a NASH or NAFL therapy, and a method of monitoring disease progression in a human subject with NAFL, wherein these methods comprise determining a level of 29 lipids in a biological sample obtained from the subject, wherein the 29 lipids comprise:
(i) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); or
(ii) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0);
wherein the biological sample is a whole blood, serum, or plasma sample.

The claims are also directed to a NASH or NAFL therapeutic for use in treating in a human subject diagnosed as having NASH or NAFL by the above method.

In some embodiments the NASH therapy or treatment is weight loss, a dietary change, increased physical activity and/or wherein the NASH therapy, treatment or therapeutic is an anti-inflammatory agent, an anti-apoptosis agent, a medication to treat insulin resistance or type 2 diabetes, a medication to improve cholesterol, Vitamin E, a peroxisome proliferator-activated receptor (PPAR) agonist, glutathione, usrodeoxycholic acid, pemafibrate, aramchol, GS0976, an anti-hypertensive drug, a farnesoid X receptor (FXR) agonist, MGL-3196, BI 1467335, IMM-124E, solithromycin, BMS-986036, Cenicriviroc (CVC), or JKB-121. In some embodiments the NAFL therapy or treatment is weight loss, a dietary change, increased physical activity. In some embodiments modifying the NASH therapy or NAFL therapy comprises modifying the dosage, frequency of administration, or therapeutic agent to be administered to the subject.

In a first aspect, the description provides a method for diagnosing nonalcoholic steatohepatitis (NASH) or nonalcoholic fatty liver (NAFL) in a subject by determining a level of at least 5 lipids in a biological sample obtained from the subject, in which a level of the at least 5 lipids in the biological sample that differs from a reference level is indicative of the presence of NASH or NAFL, and in which the at least 5 lipids are selected from the group consisting of AcCa(10:0), AcCa(10:1), Cer(d34:0), Cer(d34:2), Cer(d18:2_25:1), Cer(d43:0), Cer(d43:3), DG(34:1), DG(34:2), DG(36:2), DG(36:4), Hex1Cer(d34:2), LPC(20:0e), LPC(20:3), LPC(22:5), LPE(16:0), LPE(18:0), LPE(22:5), PA(44:4), PC(32:0), PC(32:1e), PC (32:1e)=> *PC(16:0e_16:1),* PC (32:1e) => *PC(16:1e_16:0),* PC(34:0), PC(34:1), PC(34:1e), PC(34:2e), PC(35:2), PC(35:3), PC(36:2), PC(36:3), PC(36:4), PC(36:5e), PC(37:2), PC(37:3), PC(40:5e), PC(40:6e), PC(40:7), PC(40:8), PC(42:6), PE(34:1), PE(34:3e), PE(38:1), PE(38:6), PE(40:4e), PI(36:1), SM(d32:0), SM(d32:2), SM(d35:1), SM(d36:0), SM(d36:4), SM(d37:1), SM(d38:0), SM(d38:1), SM(d40:1), CO(36:1), TG(38:0), TG(38:2), TG(40:0), TG(43:1), TG(52:4), and TG(53:5).

In another aspect, the description provides a method of treating NASH or NAFL in a subject, the method including the steps of (a) identifying the subject as having NASH or NAFL by determining that a level of at least 5 lipids in a biological sample obtained from the subject differs from a reference level, in which the at least 5 lipids are selected from the group consisting of AcCa(10:0), AcCa(10:1), Cer(d34:2), Cer(d18:2_25:1), Cer(d34:0), Cer(d43:0), Cer(d43:3), DG(34:1), DG(34:2), DG(36:2), DG(36:4), Hex1Cer(d34:2), LPC(20:0e), LPC(20:3), LPC(22:5), LPE(16:0), LPE(18:0), LPE(22:5), PA(44:4), PC(32:0), PC(32:1e), PC (32:1e)=> *PC(16:0e_16:1),* PC (32:1e) => *PC(16:1e_16:0),* PC(34:0), PC(34:1), PC(34:1e), PC(34:2e), PC(35:2), PC(35:3), PC(36:2), PC(36:3), PC(36:4), PC(36:5e), PC(37:2), PC(37:3), PC(40:5e), PC(40:6e), PC(40:7), PC(40:8), PC(42:6), PE(34:1), PE(34:3e), PE(38:1), PE(38:6), PE(40:4e), PI(36:1), SM(d32:0), SM(d32:2), SM(d35:1), SM(d36:0), SM(d36:4), SM(d37:1), SM(d38:0), SM(d38:1), SM(d40:1), CO(36:1), TG(38:0), TG(38:2), TG(40:0), TG(43:1), TG(52:4), and TG(53:5); and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of at least 5 lipids in a biological sample obtained from the subject differs from a reference level or is unchanged relative to a previous sample obtained from the subject, in which the at least 5 lipids are selected from the group consisting of AcCa(10:0), AcCa(10:1), Cer(d34:2), Cer(d18:2_25:1), Cer(d34:0), Cer(d43:0), Cer(d43:3), DG(34:1), DG(34:2), DG(36:2), DG(36:4), Hex1Cer(d34:2), LPC(20:0e), LPC(20:3), LPC(22:5), LPE(16:0), LPE(18:0), LPE(22:5), PA(44:4), PC(32:0), PC(32:1e), PC (32:1e)=> *PC(16:0e_16:1),* PC (32:1e) => *PC(16:1e_16:0),* PC(34:0), PC(34:1), PC(34:1e), PC(34:2e), PC(35:2), PC(35:3), PC(36:2), PC(36:3), PC(36:4), PC(36:5e), PC(37:2), PC(37:3), PC(40:5e), PC(40:6e), PC(40:7), PC(40:8), PC(42:6), PE(34:1), PE(34:3e), PE(38:1), PE(38:6), PE(40:4e), PI(36:1), SM(d32:0), SM(d32:2), SM(d35:1), SM(d36:0), SM(d36:4), SM(d37:1), SM(d38:0), SM(d38:1), SM(d40:1), CO(36:1), TG(38:0), TG(38:2), TG(40:0), TG(43:1), TG(52:4), and TG(53:5); and (b) modifying the therapy administered to the subject.

In another aspect, the description provides a method of monitoring disease progression in a subject with NAFL, the method including the steps of (a) identifying that the NAFL has progressed to NASH by determining that a level of at least 5 lipids in a biological sample obtained from the subject differs from a reference level, in which the at least 5 lipids are selected from the group consisting of AcCa(10:0), AcCa(10:1), Cer(d34:2), Cer(d18:2_25:1), Cer(d34:0), Cer(d43:0), Cer(d43:3), DG(34:1), DG(34:2), DG(36:2), DG(36:4), Hex1Cer(d34:2), LPC(20:0e), LPC(20:3), LPC(22:5), LPE(16:0), LPE(18:0), LPE(22:5), PA(44:4), PC(32:0), PC(32:1e), PC (32:1e)=> *PC(16:0e_16:1),* PC (32:1e) => *PC(16:1e_16:0)*, PC(34:0), PC(34:1), PC(34:1e), PC(34:2e), PC(35:2), PC(35:3), PC(36:2), PC(36:3), PC(36:4), PC(36:5e), PC(37:2), PC(37:3), PC(40:5e), PC(40:6e), PC(40:7), PC(40:8), PC(42:6), PE(34:1), PE(34:3e), PE(38:1), PE(38:6), PE(40:4e), PI(36:1), SM(d32:0), SM(d32:2), SM(d35:1), SM(d36:0), SM(d36:4), SM(d37:1), SM(d38:0), SM(d38:1), SM(d40:1), CO(36:1), TG(38:0), TG(38:2), TG(40:0), TG(43:1), TG(52:4), and TG(53:5); and (b) administering a NASH therapy to said subject.\

In some cases of any of the foregoing aspects, the at least 5 lipids include LPC(22:5), LPE(16:0), PC(35:3), PC(42:6), and PE(38:1). In some cases, the at least 5 lipids further include PC(34:1), PC(34:1e), and/or SM(d35:1). In some cases, the at least 5 lipids further include SM(d40:1).

In some cases of any of the foregoing aspects, the at least 5 lipids include or consist of LPC(22:5), LPE(16:0), PC(35:3), PC(42:6), PE(38:1), PC(34:1), PC(34:1e), SM(d35:1), and SM(d40:1). In some cases, the at least 5 lipids further include Cer(d18:2_25:1), LPC(20:0e), PC (32: 1e)=> *PC(16:0e_16:1),* PC (32:1e) => *PC(16:1e_16:0),* PC(34:0), PC(40:5e), PC(40:7), PE(38:6), SM(d38:1), TG(40:0), and/or TG(52:4).

In some cases of any of the foregoing aspects, the at least 5 lipids include or consist of LPC(22:5), LPE(16:0), PC(35:3), PC(42:6), PE(38:1), PC(34:1), PC(34:1e), SM(d35:1), Cer(d18:2_25:1), LPC(20:0e), PC (32:1e)=> *PC(16:0e_16:1),* PC (32:1e) => *PC(16:1e_16:0),* PC(34:0), PC(40:5e), PC(40:7), PE(38:6), SM(d38:1), TG(40:0), and TG(52:4).

In some cases of any of the foregoing aspects, the at least 5 lipids include DG(34:1), LPC(20:0e), PC(34:2e), PC(36:4), PC(36:5e), PC(40:8), and PE(38:1). In some cases, the at least 5 lipids further include LPE(18:0) and/or PC(37:3).

In some cases of any of the foregoing aspects, the at least 5 lipids include or consist of DG(34:1), LPC(20:0e), PC(34:2e), PC(36:4), PC(36:5e), PC(40:8), PE(38:1), LPE(18:0), and PC(37:3). In some cases, the at least 5 lipids further include AcCa(10:1), Cer(d43:0), DG(36:4), LPC(22:5), PC(36:3), PC(37:2), PE(34:1), SM(d40:1), TG(38:0), and/or TG(38:2).

In some cases of any of the foregoing aspects, the at least 5 lipids include or consist of DG(34:1), LPC(20:0e), PC(34:2e), PC(36:4), PC(36:5e), PC(40:8), PE(38:1), LPE(18:0), PC(37:3), AcCa(10:1), Cer(d43:0), DG(36:4), LPC(22:5), PC(36:3), PC(37:2), PE(34:1), SM(d40:1), TG(38:0), and TG(38:2).

In some cases of any of the foregoing aspects or cases, the at least 5 lipids further include DG(34:1), LPC(20:0e), PC(34:2e), PC(36:4), PC(36:5e), and/or PC(40:8).

In some cases of any of the foregoing aspects or cases, the at least 5 lipids further include LPC(22:5), LPE(16:0), PC(35:3), and/or PC(42:6).

In some cases of any of the foregoing aspects or cases, the at least 5 lipids further include AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and/or TG(53:5).

In some cases of any of the foregoing aspects, the at least 5 lipids are selected from the group consisting of LPC(20:0e), LPC(22:5), DG(34:1), DG(36:4), LPE(16:0), PC(32:1e), PC(34:0), PC(34:2e), PC(35:3), PC(36:4), PC(36:5e), PC(37:2), PC(40:7), PC(40:8), PE(38:1), PE(38:6), SM(d40:1), TG(38:0), and TG(38:2).

In some cases of any of the foregoing aspects, the at least 5 lipids include LPC(20:0e), LPC(22:5), DG(34:1), DG(36:4), LPE(16:0), PC(32:1e), PC(34:0), PC(34:2e), PC(35:3), PC(36:4), PC(36:5e), PC(37:2), PC(40:7), PC(40:8), PE(38:1), PE(38:6), SM(d40:1), TG(38:0), and TG(38:2).

In some cases of any of the foregoing aspects, the at least 5 lipids include or consist of DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5).

In some cases of any of the foregoing aspects, the at least 5 lipids comprise DG(34:1), LPC(20:0e), LPC(22:5), PC(40:7), PE(34:1), and PE(34:3e). In some cases, the at least 5 lipids further comprise Cer(d43:3), LPE(16:0), and/or PC(35:2). In some cases, the at least 5 lipids further include DG(36:2).

In some cases of any of the foregoing aspects, the at least 5 lipids include or consist of DG(34:1), LPC(20:0e), LPC(22:5), PC(40:7), PE(34:1), PE(34:3e), LPE(16:0), PC(35:2), Cer(d43:3), and DG(36:2). In some cases, the at least 5 lipids further include Cer(d34:0), DG(36:4), PC(34:0), PC(34:1), PC(36:2), PC(42:6), CO(36:1), TG(38:0), and/or TG(38:2).

In some cases of any of the foregoing aspects, the at least 5 lipids include or consist of DG(34:1), LPC(20:0e), LPC(22:5), PC(40:7), PE(34:1), PE(34:3e), LPE(16:0), PC(35:2), Cer(d43:3), DG(36:2), Cer(d34:0), DG(36:4), PC(34:0), PC(34:1), PC(36:2), PC(42:6), CO(36:1), TG(38:0), and TG(38:2).

In some cases of any of the foregoing aspects, the at least 5 lipids include LPC(20:0e), PC(34:1), PC(36:5e), PC(40:8), and PE(38:1). In some cases, the at least 5 lipids further include LPC(20:3), LPE(16:0), PC(34:2e), and/or SM(d32:0).

In some cases of any of the foregoing aspects, the at least 5 lipids include or consist of LPC(20:0e), PC(34:1), PC(36:5e), PC(40:8), PE(38:1), LPC(20:3), LPE(16:0), PC(34:2e), and SM(d32:0). In some cases, the at least 5 lipids further include Cer(d34:0), DG (36:4), LPE(18:0), LPE(22:5), PC(34:0), PC(37:2), PC(40:7), PE(40:4e), SM(d36:0), and/or SM(d40:1).

In some cases of any of the foregoing aspects, the at least 5 lipids include or consist of LPC(20:0e), PC(34:1), PC(36:5e), PC(40:8), PE(38:1), LPC(20:3), LPE(16:0), PC(34:2e), SM(d32:0), Cer(d34:0), DG (36:4), LPE(18:0), LPE(22:5), PC(34:0), PC(37:2), PC(40:7), PE(40:4e), SM(d36:0), and SM(d40:1).

In some cases of any of the foregoing aspects, the at least 5 lipids comprise LPC(22:5), PC(34:1), PC(40:8), PE(34:1), PI(36:1), and SM(d40:1). In some cases, the at least 5 lipids further include LPE(16:0) and/or SM(d38:0).

In some cases of any of the foregoing aspects, the at least 5 lipids include or consist of LPC(22:5), PC(34:1), PC(40:8), PE(34:1), PI(36:1), SM(d40:1), LPE(16:0), and SM(d38:0). In some cases, the at least 5 lipids further include DG(34:2), DG(36:2), LPC(20:0e), LPE(18:0), PA(44:4), PC(34:2e), PC(36:3), PC(36:4), PE(40:04e), and/or SM(d35:1).

In some cases of any of the foregoing aspects, the at least 5 lipids include or consist of LPC(22:5), PC(34:1), PC(40:8), PE(34:1), PI(36:1), SM(d40:1), LPE(16:0), SM(d38:0), DG(34:2), DG(36:2), LPC(20:0e), LPE(18:0), PA(44:4), PC(34:2e), PC(36:3), PC(36:4), PE(40:04e), and SM(d35:1).

In some cases of any of the foregoing aspects, the at least 5 lipids are selected from the group consisting of DG(34:1), DG(36:4), LPC(20:0e), LPC(22:5), LPE(18:0), LPE(22:5), PA(44:4), SM(d32:0), PI(36:1), SM(d36:0), SM(d40:1), LPE(16:0), PC(34:0), PC(34:2e), PC(40:7), PC(40:8), and PE(40:4e).

In some cases of any of the foregoing aspects, the at least 5 lipids include DG(34:1), DG(36:4), LPC(20:0e), LPC(22:5), LPE(18:0), LPE(22:5), PA(44:4), SM(d32:0), PI(36:1), SM(d36:0), SM(d40:1), LPE(16:0), PC(34:0), PC(34:2e), PC(40:7), PC(40:8), and PE(40:4e).

In some cases of any of the foregoing aspects or cases, at least 5 lipids further include PC(34:1), PC(36:5e), PC(40:8), PE(38:1), PI(36:1), and/or SM(d40:1).

In some cases of any of the foregoing aspects or cases, the at least 5 lipids further comprise DG(34:1), LPC(22:5), PC(40:7), PE(34:1), PE(34:3e), PI(36:1), and/or SM(d40:1).

In some cases of any of the foregoing aspects, the at least 5 lipids further include DG(34:1), PC(40:7), PE(34:3e), LPC(20:0e), PC(36:5e), and/or PE(38:1).

In some cases of any of the foregoing aspects or cases, the at least 5 lipids further include AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and/or TG(40:0).

In some cases of any of the foregoing aspects, the at least 5 lipids include or consist of DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0).

In some cases of any of the foregoing aspects, the method further includes determining a level of a glycan in the biological sample obtained from the subject, wherein a level of the glycan in the biological sample that differs from a reference level is indicative of the presence of NASH or NAFL. In some cases, a) the glycan is 1988 and/or 2592; b) the glycan is 2592; or c) the glycan is 1988 and 2592.

In some cases of any of the foregoing aspects, the method further includes determining a level of a hormone in the biological sample obtained from the subject, wherein a level of the hormone in the biological sample that differs from a reference level is indicative of the presence of NASH or NAFL. In some cases, the hormone is adiponectin.

In another aspect, the description provides a method for diagnosing nonalcoholic steatohepatitis (NASH) or nonalcoholic fatty liver (NAFL) in a subject by determining a level of nine lipids and one glycan in a biological sample obtained from the subject, in which a level of the nine lipids and one glycan in the biological sample that differs from a reference level is indicative of the presence of NASH or NAFL, and in which the nine lipids include LPC(22:5), LPE(16:0), PC(34:1), PC(34:1e), PC(35:3), PC(42:6), PE(38:1), SM(d35:1), and SM(d40:1), and the glycan includes 2592.

In another aspect, the description provides a method of treating NASH or NAFL in a subject, the method including the steps of (a) identifying the subject as having NASH or NAFL by determining that a level of nine lipids and one glycan in a biological sample obtained from the subject differs from a reference level, in which the nine lipids include LPC(22:5), LPE(16:0), PC(34:1), PC(34:1e), PC(35:3), PC(42:6), PE(38:1), SM(d35:1), and SM(d40:1), and the glycan includes 2592; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of nine lipids and one glycan in a biological sample obtained from the subject differs from a reference level or is unchanged relative to a previous sample obtained from the subject, in which the nine lipids include LPC(22:5), LPE(16:0), PC(34:1), PC(34:1e), PC(35:3), PC(42:6), PE(38:1), SM(d35:1), and SM(d40:1), and the glycan includes 2592; and (b) modifying the therapy administered to the subject.

In another aspect, the description provides a method of monitoring disease progression in a subject with NAFL, the method including the steps of (a) identifying that the NAFL has progressed to NASH by determining that a level of nine lipids and one glycan in a biological sample obtained from the subject differs from a reference level, in which the nine lipids comprise LPC(22:5), LPE(16:0), PC(34:1), PC(34:1e), PC(35:3), PC(42:6), PE(38:1), SM(d35:1), and SM(d40:1), and the glycan includes 2592; and (b) administering a NASH therapy to said subject.

In another aspect, the description provides a method for diagnosing nonalcoholic steatohepatitis (NASH) or nonalcoholic fatty liver (NAFL) in a subject by determining a level of 19 lipids and one glycan in a biological sample obtained from the subject, in which a level of the 19 lipids and one glycan in the biological sample that differs from a reference level is indicative of the presence of NASH or NAFL, and in which the 19 lipids include Cer(d18:2_25:1), LPC(20:0e), LPC(22:5), LPE(16:0), PC (32:1e)=> *PC(16:0e_16:1),* PC (32:1e) => *PC(16:1e_16:0),* PC(34:0), PC(34:1), PC(34:1e), PC(35:3), PC(40:5e), PC(40:7), PC(42:6), PE(38:1), PE(38:6), SM(d35:1), SM(d38:1), TG(40:0), and TG(52:4), and the glycan include 2592.

In another aspect, the description provides a method of treating NASH or NAFL in a subject, the method including the steps of (a) identifying the subject as having NASH or NAFL by determining that a level of 19 lipids and one glycan in a biological sample obtained from the subject differs from a reference level, in which the 19 lipids include Cer(d18:2_25:1), LPC(20:0e), LPC(22:5), LPE(16:0), PC (32:1e)=> *PC(16:0e_16:1),* PC (32:1e) => *PC(16:1e_16:0),* PC(34:0), PC(34:1), PC(34:1e), PC(35:3), PC(40:5e), PC(40:7), PC(42:6), PE(38:1), PE(38:6), SM(d35:1), SM(d38:1), TG(40:0), and TG(52:4), and the glycan includes 2592; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 19 lipids and one glycan in a biological sample obtained from the subject differs from a reference level or is unchanged relative to a previous sample obtained from the subject, in which the 19 lipids include Cer(d18:2_25:1), LPC(20:0e), LPC(22:5), LPE(16:0), PC (32:1e)=> *PC(16:0e_16:1),* PC (32:1e) => *PC(16:1e_16:0),* PC(34:0), PC(34:1), PC(34:1e), PC(35:3), PC(40:5e), PC(40:7), PC(42:6), PE(38:1), PE(38:6), SM(d35:1), SM(d38:1), TG(40:0), and TG(52:4), and the glycan includes 2592; and (b) modifying the therapy administered to the subject.

In another aspect, the description provides a method of monitoring disease progression in a subject with NAFL, the method including the steps of (a) identifying that the NAFL has progressed to NASH by determining that a level of 19 lipids and one glycan in a biological sample obtained from the subject differs from a reference level, in which the 19 lipids include Cer(d18:2_25:1), LPC(20:0e), LPC(22:5), LPE(16:0), PC (32:1e)=> *PC(16:0e_16:1),* PC (32:1e) => *PC(16:1e_16:0),* PC(34:0), PC(34:1), PC(34:1e), PC(35:3), PC(40:5e), PC(40:7), PC(42:6), PE(38:1), PE(38:6), SM(d35:1), SM(d38:1), TG(40:0), and TG(52:4), and the glycan includes 2592; and (b) administering a NASH therapy to said subject.

In another aspect, the description provides a method for diagnosing nonalcoholic steatohepatitis (NASH) or nonalcoholic fatty liver (NAFL) in a subject by determining a level of nine lipids and one hormone in a biological sample obtained from the subject, in which a level of the nine lipids and one hormone in the biological sample that differs from a reference level is indicative of the presence of NASH or NAFL, and wherein the nine lipids include DG(34:1), LPC(20:0e), LPE(18:0), PC(34:2e), PC(36:4), PC(36:5e), PC(37:3), PC(40:8), and PE(38:1), and the hormone includes adiponectin.

In another aspect, the description provides a method of treating NASH or NAFL in a subject, the method including the steps of (a) identifying the subject as having NASH or NAFL by determining that a level of nine lipids and one hormone in a biological sample obtained from the subject differs from a reference level, in which the nine lipids include DG(34:1), LPC(20:0e), LPE(18:0), PC(34:2e), PC(36:4), PC(36:5e), PC(37:3), PC(40:8), and PE(38:1), and the hormone includes adiponectin; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of nine lipids and one hormone in a biological sample obtained from the subject differs from a reference level or is unchanged relative to a previous sample obtained from the subject, in which the nine lipids include DG(34:1), LPC(20:0e), LPE(18:0), PC(34:2e), PC(36:4), PC(36:5e), PC(37:3), PC(40:8), and PE(38:1), and the hormone includes adiponectin; and (b) modifying the therapy administered to the subject.

In another aspect, the description provides a method of monitoring disease progression in a subject with NAFL, the method including the steps of (a) identifying that the NAFL has progressed to NASH by determining that a level of nine lipids and one hormone in a biological sample obtained from the subject differs from a reference level, in which the nine lipids include DG(34:1), LPC(20:0e), LPE(18:0), PC(34:2e), PC(36:4), PC(36:5e), PC(37:3), PC(40:8), and PE(38:1), and the hormone includes adiponectin; and (b) administering a NASH therapy to said subject.

In another aspect, the description provides a method for diagnosing nonalcoholic steatohepatitis (NASH) or nonalcoholic fatty liver (NAFL) in a subject by determining a level of 19 lipids and one hormone in a biological sample obtained from the subject, in which a level of the 19 lipids and one hormone in the biological sample that differs from a reference level is indicative of the presence of NASH or NAFL, and in which the 19 lipids include AcCa(10:1), Cer(d43:0), DG(34:1), DG(36:4), LPC(20:0e), LPC(22:5), LPE(18:0), PC(34:2e), PC(36:3), PC(36:4), PC(36:5e), PC(37:2), PC(37:3), PC(40:8), PE(34:1), PE(38:1), SM(d40:1), TG(38:0), and TG(38:2), and the hormone includes adiponectin.

In another aspect, the description provides a method of treating NASH or NAFL in a subject, the method including the steps of (a) identifying the subject as having NASH or NAFL by determining that a level of 19 lipids and one hormone in a biological sample obtained from the subject differs from a reference level, in which the 19 lipids include AcCa(10:1), Cer(d43:0), DG(34:1), DG(36:4), LPC(20:0e), LPC(22:5), LPE(18:0), PC(34:2e), PC(36:3), PC(36:4), PC(36:5e), PC(37:2), PC(37:3), PC(40:8), PE(34:1), PE(38:1), SM(d40:1), TG(38:0), and TG(38:2), and the hormone includes adiponectin; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 19 lipids and one hormone in a biological sample obtained from the subject differs from a reference level or is unchanged relative to a previous sample obtained from the subject, in which the 19 lipids include AcCa(10:1), Cer(d43:0), DG(34:1), DG(36:4), LPC(20:0e), LPC(22:5), LPE(18:0), PC(34:2e), PC(36:3), PC(36:4), PC(36:5e), PC(37:2), PC(37:3), PC(40:8), PE(34:1), PE(38:1), SM(d40:1), TG(38:0), and TG(38:2), and the hormone includes adiponectin; and (b) modifying the therapy administered to the subject.

In another aspect, the description provides a method of monitoring disease progression in a subject with NAFL, the method including the steps of (a) identifying that the NAFL has progressed to NASH by determining that a level of 19 lipids and one hormone in a biological sample obtained from the subject differs from a reference level, in which the 19 lipids include AcCa(10:1), Cer(d43:0), DG(34:1), DG(36:4), LPC(20:0e), LPC(22:5), LPE(18:0), PC(34:2e), PC(36:3), PC(36:4), PC(36:5e), PC(37:2), PC(37:3), PC(40:8), PE(34:1), PE(38:1), SM(d40:1), TG(38:0), and TG(38:2), and the hormone includes adiponectin; and (b) administering a NASH therapy to said subject.

In another aspect, the description provides a method for diagnosing nonalcoholic steatohepatitis (NASH) or nonalcoholic fatty liver (NAFL) in a subject by determining a level of 10 lipids in a biological sample obtained from the subject, in which a level of the 10 lipids in the biological sample that differs from a reference level is indicative of the presence of NASH or NAFL, and in which the 10 lipids include Cer(d43:3), DG(34:1), DG(36:2), LPC(20:0e), LPC(22:5), LPE(16:0), PC(35:2), PC(40:7), PE(34:1), and PE(34:3e).

In another aspect, the description provides a method of treating NASH or NAFL in a subject, the method including the steps of (a) identifying the subject as having NASH or NAFL by determining that a level of 10 lipids in a biological sample obtained from the subject differs from a reference level, in which the 10 lipids include Cer(d43:3), DG(34:1), DG(36:2), LPC(20:0e), LPC(22:5), LPE(16:0), PC(35:2), PC(40:7), PE(34:1), and PE(34:3e); and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 10 lipids in a biological sample obtained from the subject differs from a reference level or is unchanged relative to a previous sample obtained from the subject, in which the 10 lipids include Cer(d43:3), DG(34:1), DG(36:2), LPC(20:0e), LPC(22:5), LPE(16:0), PC(35:2), PC(40:7), PE(34:1), and PE(34:3e); and (b) modifying the therapy administered to the subject.

In another aspect, the description provides a method of monitoring disease progression in a subject with NAFL, the method including the steps of (a) identifying that the NAFL has progressed to NASH by determining that a level of 10 lipids in a biological sample obtained from the subject differs from a reference level, in which the 10 lipids include Cer(d43:3), DG(34:1), DG(36:2), LPC(20:0e), LPC(22:5), LPE(16:0), PC(35:2), PC(40:7), PE(34:1), and PE(34:3e); and (b) administering a NASH therapy to said subject.

In another aspect, the description provides a method for diagnosing nonalcoholic steatohepatitis (NASH) or nonalcoholic fatty liver (NAFL) in a subject by determining a level of 18 lipids and two glycans in a biological sample obtained from the subject, in which a level of the 18 lipids and two glycans in the biological sample that differs from a reference level is indicative of the presence of NASH or NAFL, and in which the 18 lipids include Cer(d34:0), Cer(d43:3), DG(34:1), DG(36:4), LPC(20:0e), LPC(22:5), LPE(16:0), PC(34:0), PC(34:1), PC(35:2), PC(35:3), PC(36:2), PC(40:7), PC(42:6), PE(34:1), PE(34:3e), CO(36:1), TG(38:0), and TG(38:2), and the two glycans include 1988 and 2592.

In another aspect, the description provides a method of treating NASH or NAFL in a subject, the method including the steps of (a) identifying the subject as having NASH or NAFL by determining that a level of 18 lipids and two glycans in a biological sample obtained from the subject differs from a reference level, in which the 18 lipids include Cer(d34:0), Cer(d43:3), DG(34:1), DG(36:4), LPC(20:0e), LPC(22:5), LPE(16:0), PC(34:0), PC(34:1), PC(35:2), PC(35:3), PC(36:2), PC(40:7), PC(42:6), PE(34:1), PE(34:3e), CO(36:1), TG(38:0), and TG(38:2), and the two glycans include1988 and 2592; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 18 lipids and two glycans in a biological sample obtained from the subject differs from a reference level or is unchanged relative to a previous sample obtained from the subject, in which the 18 lipids include Cer(d34:0), Cer(d43:3), DG(34:1), DG(36:4), LPC(20:0e), LPC(22:5), LPE(16:0), PC(34:0), PC(34:1), PC(35:2), PC(35:3), PC(36:2), PC(40:7), PC(42:6), PE(34:1), PE(34:3e), CO(36:1), TG(38:0), and TG(38:2), and the two glycans include1988 and 2592; and (b) modifying the therapy administered to the subject.

In another aspect, the description provides a method of monitoring disease progression in a subject with NAFL, the method including the steps of (a) identifying that the NAFL has progressed to NASH by determining that a level of 18 lipids and two glycans in a biological sample obtained from the subject differs from a reference level, in which the 18 lipids include Cer(d34:0), Cer(d43:3), DG(34:1), DG(36:4), LPC(20:0e), LPC(22:5), LPE(16:0), PC(34:0), PC(34:1), PC(35:2), PC(35:3), PC(36:2), PC(40:7), PC(42:6), PE(34:1), PE(34:3e), CO(36:1), TG(38:0), and TG(38:2), and the two glycans include 1988 and 2592; and (b) administering a NASH therapy to said subject.

In another aspect, the description provides a method for diagnosing nonalcoholic steatohepatitis (NASH) or nonalcoholic fatty liver (NAFL) in a subject by determining a level of 9 lipids and one hormone in a biological sample obtained from the subject, in which a level of the 9 lipids and one hormone in the biological sample that differs from a reference level is indicative of the presence of NASH or NAFL, and in which the 9 lipids include LPC(20:0e), LPC(20:3), LPE(16:0), PC(34:1), PC(34:2e), PC(36:5e), PC(40:8), PE(38:1), and SM(d32:0), and the hormone includes adiponectin.

In another aspect, the description provides a method of treating NASH or NAFL in a subject, the method including the steps of (a) identifying the subject as having NASH or NAFL by determining that a level of nine lipids and one hormone in a biological sample obtained from the subject differs from a reference level, in which the 9 lipids include LPC(20:0e), LPC(20:3), LPE(16:0), PC(34:1), PC(34:2e), PC(36:5e), PC(40:8), PE(38:1), and SM(d32:0), and the hormone includes adiponectin; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of nine lipids and one hormone in a biological sample obtained from the subject differs from a reference level or is unchanged relative to a previous sample obtained from the subject, in which the 9 lipids include LPC(20:0e), LPC(20:3), LPE(16:0), PC(34:1), PC(34:2e), PC(36:5e), PC(40:8), PE(38:1), and SM(d32:0), and the hormone includes adiponectin; and (b) modifying the therapy administered to the subject.

In another aspect, the description provides a method of monitoring disease progression in a subject with NAFL, the method including the steps of (a) identifying that the NAFL has progressed to NASH by determining that a level of nine lipids and one hormone in a biological sample obtained from the subject differs from a reference level, in which the 9 lipids include LPC(20:0e), LPC(20:3), LPE(16:0), PC(34:1), PC(34:2e), PC(36:5e), PC(40:8), PE(38:1), and SM(d32:0), and the hormone includes adiponectin; and (b) administering a NASH therapy to said subject.

In another aspect, the description provides a method for diagnosing nonalcoholic steatohepatitis (NASH) or nonalcoholic fatty liver (NAFL) in a subject by determining a level of 19 lipids and one hormone in a biological sample obtained from the subject, in which a level of the 19 lipids and one hormone in the biological sample that differs from a reference level is indicative of the presence of NASH or NAFL, and in which the 19 lipids include Cer(d34:0), DG(36:4), LPC(20:0e), LPC(20:3), LPE(16:0), LPE(18:0), LPE(22:5), PC(34:0), PC(34:1), PC(34:2e), PC(36:5e), PC(37:2), PC(40:7), PC(40:8), PE(38:1), PE(40:4e), SM(d32:0), SM(d36:0), and SM(d40:1), and the hormone includes adiponectin.

In another aspect, the description provides a method of treating NASH or NAFL in a subject, the method including the steps of (a) identifying the subject as having NASH or NAFL by determining that a level of 19 lipids and one hormone in a biological sample obtained from the subject differs from a reference level, in which the 19 lipids include Cer(d34:0), DG(36:4), LPC(20:0e), LPC(20:3), LPE(16:0), LPE(18:0), LPE(22:5), PC(34:0), PC(34:1), PC(34:2e), PC(36:5e), PC(37:2), PC(40:7), PC(40:8), PE(38:1), PE(40:4e), SM(d32:0), SM(d36:0), and SM(d40:1), and the hormone includes adiponectin; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 19 lipids and one hormone in a biological sample obtained from the subject differs from a reference level or is unchanged relative to a previous sample obtained from the subject, in which the 19 lipids include Cer(d34:0), DG(36:4), LPC(20:0e), LPC(20:3), LPE(16:0), LPE(18:0), LPE(22:5), PC(34:0), PC(34:1), PC(34:2e), PC(36:5e), PC(37:2), PC(40:7), PC(40:8), PE(38:1), PE(40:4e), SM(d32:0), SM(d36:0), and SM(d40:1), and the hormone includes adiponectin; and (b) modifying the therapy administered to the subject.

In another aspect, the description provides a method of monitoring disease progression in a subject with NAFL, the method including the steps of (a) identifying that the NAFL has progressed to NASH by determining that a level of 19 lipids and one hormone in a biological sample obtained from the subject differs from a reference level, in which the 19 lipids include Cer(d34:0), DG(36:4), LPC(20:0e), LPC(20:3), LPE(16:0), LPE(18:0), LPE(22:5), PC(34:0), PC(34:1), PC(34:2e), PC(36:5e), PC(37:2), PC(40:7), PC(40:8), PE(38:1), PE(40:4e), SM(d32:0), SM(d36:0), and SM(d40:1), and the hormone includes adiponectin; and (b) administering a NASH therapy to said subject.

In another aspect, the description provides a method for diagnosing nonalcoholic steatohepatitis (NASH) or nonalcoholic fatty liver (NAFL) in a subject by determining a level of 8 lipids, one glycan, and one hormone in a biological sample obtained from the subject, in which a level of the 8 lipids, one glycan, and one hormone in the biological sample that differs from a reference level is indicative of the presence of NASH or NAFL, and in which the 8 lipids include LPC(22:5), LPE(16:0), PC(34:1), PC(40:8), PE(34:1), PI(36:1), SM(d38:0), and SM(d40:1), the glycan includes 2592, and the hormone includes adiponectin.

In another aspect, the description provides a method of treating NASH or NAFL in a subject, the method including the steps of (a) identifying the subject as having NASH or NAFL by determining that a level of 8 lipids, one glycan, and one hormone in a biological sample obtained from the subject differs from a reference level, in which the 8 lipids include LPC(22:5), LPE(16:0), PC(34:1), PC(40:8), PE(34:1), PI(36:1), SM(d38:0), and SM(d40:1), the glycan includes 2592, and the hormone includes adiponectin; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 8 lipids, one glycan, and one hormone in a biological sample obtained from the subject differs from a reference level or is unchanged relative to a previous sample obtained from the subject, in which the 8 lipids include LPC(22:5), LPE(16:0), PC(34:1), PC(40:8), PE(34:1), PI(36:1), SM(d38:0), and SM(d40:1), the glycan includes 2592, and the hormone includes adiponectin; and (b) modifying the therapy administered to the subject.

In another aspect, the description provides a method of monitoring disease progression in a subject with NAFL, the method including the steps of (a) identifying that the NAFL has progressed to NASH by determining that a level of 8 lipids, one glycan, and one hormone in a biological sample obtained from the subject differs from a reference level, in which the 8 lipids include LPC(22:5), LPE(16:0), PC(34:1), PC(40:8), PE(34:1), PI(36:1), SM(d38:0), and SM(d40:1), the glycan includes 2592, and the hormone includes adiponectin; and (b) administering a NASH therapy to said subject.

In another aspect, the description provides a method for diagnosing nonalcoholic steatohepatitis (NASH) or nonalcoholic fatty liver (NAFL) in a subject by determining a level of 18 lipids, one glycan, and one hormone in a biological sample obtained from the subject, in which a level of the 18 lipids, one glycan, and one hormone in the biological sample that differs from a reference level is indicative of the presence of NASH or NAFL, and in which the 18 lipids include DG(34:2), DG(36:4), LPC(20:0e), LPC(20:3), LPC(22:5), LPE(16:0), LPE(18:0), PA(44:4), PC(34:1), PC(34:2e), PC(36:3), PC(36:4), PC(40:8), PE(34:1), PE(40:4e), PI(36:1), SM(d35:1), SM(d36:0), SM(d38:0), and SM(d40:1), the glycan includes 2592, and the hormone includes adiponectin.

In another aspect, the description provides a method of treating NASH or NAFL in a subject, the method including the steps of (a) identifying the subject as having NASH or NAFL by determining that a level of 18 lipids, one glycan, and one hormone in a biological sample obtained from the subject differs from a reference level, in which the 18 lipids include DG(34:2), DG(36:4), LPC(20:0e), LPC(20:3), LPC(22:5), LPE(16:0), LPE(18:0), PA(44:4), PC(34:1), PC(34:2e), PC(36:3), PC(36:4), PC(40:8), PE(34:1), PE(40:4e), PI(36:1), SM(d35:1), SM(d36:0), SM(d38:0), and SM(d40:1), the glycan includes 2592, and the hormone includes adiponectin; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 18 lipids, one glycan, and one hormone in a biological sample obtained from the subject differs from a reference level or is unchanged relative to a previous sample obtained from the subject, in which the 18 lipids include DG(34:2), DG(36:4), LPC(20:0e), LPC(20:3), LPC(22:5), LPE(16:0), LPE(18:0), PA(44:4), PC(34:1), PC(34:2e), PC(36:3), PC(36:4), PC(40:8), PE(34:1), PE(40:4e), PI(36:1), SM(d35:1), SM(d36:0), SM(d38:0), and SM(d40:1), the glycan includes 2592, and the hormone includes adiponectin; and (b) modifying the therapy administered to the subject.

In another aspect, the description provides a method of monitoring disease progression in a subject with NAFL, the method including the steps of (a) identifying that the NAFL has progressed to NASH by determining that a level of 18 lipids, one glycan, and one hormone in a biological sample obtained from the subject differs from a reference level, in which the 18 lipids include DG(34:2), DG(36:4), LPC(20:0e), LPC(20:3), LPC(22:5), LPE(16:0), LPE(18:0), PA(44:4), PC(34:1), PC(34:2e), PC(36:3), PC(36:4), PC(40:8), PE(34:1), PE(40:4e), PI(36:1), SM(d35:1), SM(d36:0), SM(d38:0), and SM(d40:1), the glycan includes 2592, and the hormone includes adiponectin; and (b) administering a NASH therapy to said subject.

In another aspect, the description provides a method for diagnosing nonalcoholic steatohepatitis (NASH) or nonalcoholic fatty liver (NAFL) in a subject by determining a level of 29 lipids in a biological sample obtained from the subject, in which a level of the 29 lipids in the biological sample that differs from a reference level is indicative of the presence of NASH or NAFL, and in which the 29 lipids include DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5).

In another aspect, the description provides a method of treating NASH or NAFL in a subject, the method including the steps of (a) identifying the subject as having NASH or NAFL by determining that a level of 29 lipids in a biological sample obtained from the subject differs from a reference level, in which the 29 lipids include DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 29 lipids in a biological sample obtained from the subject differs from a reference level or is unchanged relative to a previous sample obtained from the subject, in which the 29 lipids include DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); and (b) modifying the therapy administered to the subject.

In another aspect, the description provides a method of monitoring disease progression in a subject with NAFL, the method including the steps of (a) identifying that the NAFL has progressed to NASH by determining that a level of 29 lipids in a biological sample obtained from the subject differs from a reference level, in which the 29 lipids include DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); and (b) administering a NASH therapy to said subject.

In another aspect, the description provides a method for diagnosing nonalcoholic steatohepatitis (NASH) or nonalcoholic fatty liver (NAFL) in a subject by determining a level of 29 lipids in a biological sample obtained from the subject, in which a level of the 29 lipids in the biological sample that differs from a reference level is indicative of the presence of NASH or NAFL, and in which the 29 lipids include DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0).

In another aspect, the description provides a method of treating NASH or NAFL in a subject, the method including the steps of (a) identifying the subject as having NASH or NAFL by determining that a level of 29 lipids in a biological sample obtained from the subject differs from a reference level, in which the 29 lipids include DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0); and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of 29 lipids in a biological sample obtained from the subject differs from a reference level or is unchanged relative to a previous sample obtained from the subject, in which the 29 lipids include DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0); and (b) modifying the therapy administered to the subject.

In another aspect, the description provides a method of monitoring disease progression in a subject with NAFL, the method including the steps of (a) identifying that the NAFL has progressed to NASH by determining that a level of 29 lipids in a biological sample obtained from the subject differs from a reference level, in which the 29 lipids include DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0); and (b) administering a NASH therapy to said subject.

In another aspect, the description provides a method for diagnosing NASH or NAFL in a subject by determining a level of one or more fatty acid in a biological sample obtained from the subject, in which a level of the one or more fatty acid in the biological sample that differs from a reference level is indicative of the presence of NASH or NAFL, and in which the one or more (e.g., 1, 2, 3, 4, 5 or more) fatty acid is selected from the group consisting of C18:2n6, C18:3n6, C20:0, C20:4n6, C22:4n6, C16:0, and C18:1n9cis.

In another aspect, the description provides a method of treating NASH or NAFL in a subject, the method including the steps of (a) identifying the subject as having NASH or NAFL by determining that a level of one or more (e.g., 1, 2, 3, 4, 5 or more) fatty acid in a biological sample obtained from the subject differs from a reference level, in which the one or more fatty acid is selected from the group consisting of C18:2n6, C18:3n6, C20:0, C20:4n6, C22:4n6, C16:0, and C18:1n9cis; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of one or more (e.g., 1, 2, 3, 4, 5 or more) fatty acid in a biological sample obtained from the subject differs from a reference level or is unchanged relative to a previous sample obtained from the subject, in which the one or more fatty acid is selected from the group consisting of C18:2n6, C18:3n6, C20:0, C20:4n6, C22:4n6, C16:0, and C18:1n9cis; and (b) modifying the therapy administered to the subject.

In another aspect, the description provides a method of monitoring disease progression in a subject with NAFL, the method including the steps of (a) identifying that the NAFL has progressed to NASH by determining that a level of one or more (e.g., 1, 2, 3, 4, 5 or more) fatty acid in a biological sample obtained from the subject differs from a reference level, in which the one or more fatty acid is selected from the group consisting of C18:2n6, C18:3n6, C20:0, C20:4n6, C22:4n6, C16:0, and C18:1n9cis; and (b) administering a NASH therapy to said subject.

In some cases of any of the foregoing aspects, the method includes determining the level of two or more fatty acids selected from the group consisting of C18:2n6, C18:3n6, C20:0, C20:4n6, C22:4n6, C16:0, and C18:1n9cis.

In some cases of any of the foregoing aspects, the method includes determining the level of three or more fatty acids selected from the group consisting of C18:2n6, C18:3n6, C20:0, C20:4n6, C22:4n6, C16:0, and C18:1n9cis. In some cases, the three or more fatty acids include C18:2n6, C18:3n6, and C22:4n6.

In some cases of any of the foregoing aspects, the method includes determining the level of four or more fatty acids selected from the group consisting of C18:2n6, C18:3n6, C20:0, C20:4n6, C22:4n6, C16:0, and C18:1n9cis.

In some cases of any of the foregoing aspects, the method includes determining the level of five fatty acids selected from the group consisting of C18:2n6, C18:3n6, C20:0, C20:4n6, C22:4n6, C16:0, and C18:1n9cis. In some cases, the five fatty acids include or consist of C18:2n6, C18:3n6, C20:0, C20:4n6, C22:4n6. In some cases, the five fatty acids include or consist of C18:2n6, C18:3n6, C22:4n6, C16:0, and C18:1n9cis.

In another aspect, the description provides a method for diagnosing NASH or NAFL in a subject by determining a level of one or more (e.g., 1, 2, 3, 4, 5 or more) glycan in a biological sample obtained from the subject, in which a level of the one or more glycan in the biological sample that differs from a reference level is indicative of the presence of NASH or NAFL, and in which the one or more glycan is selected from the group consisting of 1661, 1988, 2592, 2764, 2968, 3953, and 4039.

In another aspect, the description provides a method of treating NASH or NAFL in a subject, the method including the steps of (a) identifying the subject as having NASH or NAFL by determining that a level of one or more (e.g., 1, 2, 3, 4, 5 or more) glycan in a biological sample obtained from the subject differs from a reference level, in which the one or more glycan is selected from the group consisting of 1661, 1988, 2592, 2764, 2968, 3953, and 4039; and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of one or more (e.g., 1, 2, 3, 4, 5 or more) glycan in a biological sample obtained from the subject differs from a reference level or is unchanged relative to a previous sample obtained from the subject, in which the one or more glycan is selected from the group consisting of 1661, 1988, 2592, 2764, 2968, 3953, and 4039; and (b) modifying the therapy administered to the subject.

In another aspect, the description provides a method of monitoring disease progression in a subject with NAFL, the method including the steps of (a) identifying that the NAFL has progressed to NASH by determining that a level of one or more (e.g., 1, 2, 3, 4, 5 or more) glycan in a biological sample obtained from the subject differs from a reference level, in which the one or more glycan is selected from the group consisting of 1661, 1988, 2592, 2764, 2968, 3953, and 4039; and (b) administering a NASH therapy to said subject.

In some cases of any of the foregoing aspects, the method includes determining the level of two or more glycans selected from the group consisting of 1661, 1988, 2592, 2764, 2968, 3953, and 4039.

In some cases of any of the foregoing aspects, the method comprises determining the level of three or more glycans selected from the group consisting of 1661, 1988, 2592, 2764, 2968, 3953, and 4039. In some cases, the three or more glycans comprise 2592, 2764, and 2968.

In some cases of any of the foregoing aspects, the method comprises determining the level of four or more glycans selected from the group consisting of 1661, 1988, 2592, 2764, 2968, 3953, and 4039.

In some cases of any of the foregoing aspects, the method comprises determining the level of five glycans selected from the group consisting of 1661, 1988, 2592, 2764, 2968, 3953, and 4039. In some cases, the five glycans include or consist of 1661, 2592, 2764, 2968, and 4039. In some cases, the five glycans include or consist of 1988, 2592, 2764, 2968, and 3953.

In another aspect, the description provides a method for diagnosing NASH or NAFL in a subject by determining a level of two or more hormones in a biological sample obtained from the subject, in which a level of the two or more hormones in the biological sample that differs from a reference level is indicative of the presence of NASH or NAFL, and in which the two or more (e.g., 2, 3, 4, or more) hormones are selected from the group consisting of leptin, adiponectin, activin A, follistatin, the ratio of activin A to follistatin (activin A/follistatin), and one or more triglyceride (e.g., total circulating triglycerides).

In another aspect, the description provides a method of treating NASH or NAFL in a subject, the method including the steps of (a) identifying the subject as having NASH or NAFL by determining that a level of two or more (e.g., 2, 3, 4, or more) hormones in a biological sample obtained from the subject differs from a reference level, in which the two or more hormones are selected from the group consisting of leptin, adiponectin, activin A, follistatin, activin A/follistatin, and one or more triglyceride (e.g., total circulating triglycerides); and (b) administering a treatment for NASH or NAFL to the subject.

In another aspect, the description provides a method of monitoring treatment efficacy in a subject being treated with a NASH or NAFL therapy, the method including the steps of (a) identifying the subject's condition as unchanged or worsened by determining that a level of two or more (e.g., 2, 3, 4, or more) hormones in a biological sample obtained from the subject differs from a reference level or is unchanged relative to a previous sample obtained from the subject, in which the two or more hormones are selected from the group consisting of leptin, adiponectin, activin A, follistatin, activin A/follistatin, and one or more triglycerides (e.g., total circulating triglycerides); and (b) modifying the therapy administered to the subject.

In another aspect, the description provides a method of monitoring disease progression in a subject with NAFL, the method including the steps of (a) identifying that the NAFL has progressed to NASH by determining that a level of two or more (e.g., 2, 3, 4, or more) hormones in a biological sample obtained from the subject differs from a reference level, in which the two or more hormones are selected from the group consisting of leptin, adiponectin, activin A, follistatin, activin A/follistatin, and one or more triglyceride (e.g., total circulating triglycerides); and (b) administering a NASH therapy to said subject.

In some cases of any of the foregoing aspects, determining the level of one or more triglyceride includes determining the level of total circulating triglycerides.

In some cases of any of the foregoing aspects, the method comprises determining the level of three or more hormones selected from the group consisting of leptin, adiponectin, activin A/follistatin, and total circulating triglycerides.

In some cases of any of the foregoing aspects, the method comprises determining the level of four hormones selected from the group consisting of leptin, adiponectin, activin A/follistatin, and total circulating triglycerides.

In another aspect, the description provides a method of treating NASH or NAFL in a subject by administering a treatment for NASH or NAFL to a subject diagnosed as having NASH or NAFL by the method of any of the foregoing aspects or cases.

In some cases of any of the foregoing aspects, the reference level is established by performing classifier training using lipid, glycan, fatty acid, and/or hormone levels obtained from a subject clinically diagnosed as having NASH, a subject clinically diagnosed as having NAFL, and a healthy subject.

In some cases of any of the foregoing aspects, the reference level is established by training a machine learning algorithm using a reference dataset.

In some cases of any of the foregoing aspects, the subject has a BMI less than 27.5 kg/m².

In some cases of any of the foregoing aspects, the subject has a BMI greater than or equal to 27.5 kg/m².

In some cases of any of the foregoing aspects, method prevents or delays the development of NASH in a subject having NAFL.

In another aspect, the description provides a method for diagnosing liver fibrosis in a subject by determining a level of one or more (e.g., 1, 2, 3, 4, 5, 6, 7, 8, 9, or 10) lipid in a biological sample obtained from the subject, in which a level of the one or more lipid in the biological sample that differs from a reference level is indicative of the presence of liver fibrosis, and in which the one or more lipid is selected from the group consisting of DG(36:3), LPC(18:0), PC(36:2), PC(37:2), PC(40:5), TG(38:0), TG(50:0), TG(51:1), TG(57:1), and TG(60:2).

In another aspect, the description provides a method of treating liver fibrosis in a subject, the method including the steps of (a) identifying the subject as having liver fibrosis by determining that a level of one or more (e.g., 1, 2, 3, 4, 5, 6, 7, 8, 9, or 10) lipid in a biological sample obtained from the subject differs from a reference level, in which the one or more lipid is selected from the group consisting of DG(36:3), LPC(18:0), PC(36:2), PC(37:2), PC(40:5), TG(38:0), TG(50:0), TG(51:1), TG(57:1), and TG(60:2); and (b) administering a treatment for liver fibrosis to the subject.

In another aspect, the description provides a method of monitoring treatment efficacy in a subject being treated with a liver fibrosis therapy, the method including the steps of (a) identifying the subject's condition as unchanged by determining that a level of one or more (e.g., 1, 2, 3, 4, 5, 6, 7, 8, 9, or 10) lipid in a biological sample obtained from the subject differs from a reference level or is unchanged relative to a previous sample obtained from the subject, in which the one or more lipid is selected from the group consisting of DG(36:3), LPC(18:0), PC(36:2), PC(37:2), PC(40:5), TG(38:0), TG(50:0), TG(51:1), TG(57:1), and TG(60:2); and (b) modifying the therapy administered to the subject.

In another aspect, the description provides a method of monitoring the development of liver fibrosis in a subject with NASH, the method including the steps of (a) identifying that the subject has developed liver fibrosis by determining that a level of one or more (e.g., 1, 2, 3, 4, 5, 6, 7, 8, 9, or 10) lipid in a biological sample obtained from the subject differs from a reference level, in which the one or more lipid is selected from the group consisting of DG(36:3), LPC(18:0), PC(36:2), PC(37:2), PC(40:5), TG(38:0), TG(50:0), TG(51:1), TG(57:1), and TG(60:2); and (b) administering a liver fibrosis therapy to said subject.

In some cases of any of the foregoing aspects, the method includes determining the level of two or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of three or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of four or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of five or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of six or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of seven or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of eight or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, the method includes determining the level of nine or more lipids selected from the group consisting of DG(36:3), LPC(18:0), PC(36:2), PC(37:2), PC(40:5), TG(38:0), TG(50:0), TG(51:1), TG(57:1), and TG(60:2).

In some cases of any of the foregoing aspects, method includes determining the level of all ten of the lipids selected from the group consisting of DG(36:3), LPC(18:0), PC(36:2), PC(37:2), PC(40:5), TG(38:0), TG(50:0), TG(51:1), TG(57:1), and TG(60:2).

In another aspect, the description provides a method for diagnosing liver fibrosis in a subject by determining a level of one or more (e.g., 1, 2, 3, 4, or 5) glycan in a biological sample obtained from the subject, in which a level of the one or more glycan in the biological sample that differs from a reference level is indicative of the presence of liver fibrosis, and in which the one or more glycan is selected from the group consisting of 2081, 2315, 2592, 2605, and 4587.

In another aspect, the description provides a method of treating liver fibrosis in a subject, the method including the steps of (a) identifying the subject as having liver fibrosis by determining that a level of one or more (e.g., 1, 2, 3, 4, or 5) glycan in a biological sample obtained from the subject differs from a reference level, in which the one or more glycan is selected from the group consisting of 2081, 2315, 2592, 2605, and 4587; and (b) administering a treatment for liver fibrosis to the subject.

In another aspect, the description provides a method of monitoring treatment efficacy in a subject being treated with a liver fibrosis therapy, the method including the steps of (a) identifying the subject's condition as unchanged by determining that a level of one or more (e.g., 1, 2, 3, 4, or 5) glycan in a biological sample obtained from the subject differs from a reference level or is unchanged relative to a previous sample obtained from the subject, in which the one or more glycan is selected from the group consisting of 2081, 2315, 2592, 2605, and 4587; and (b) modifying the therapy administered to the subject.

In another aspect, the description provides a method of monitoring the development of liver fibrosis in a subject with NASH, the method including the steps of (a) identifying that the subject has developed liver fibrosis by determining that a level of one or more (e.g., 1, 2, 3, 4, or 5) glycan in a biological sample obtained from the subject differs from a reference level, wherein the one or more glycan is selected from the group consisting of 2081, 2315, 2592, 2605, and 4587; and (b) administering a liver fibrosis therapy to said subject.

In some cases of any of the foregoing aspects, the method includes determining the level of two or more glycans selected from the group consisting of 2081, 2315, 2592, 2605, and 4587.

In some cases of any of the foregoing aspects, the method comprises determining the level of three or more glycans selected from the group consisting of 2081, 2315, 2592, 2605, and 4587.

In some cases of any of the foregoing aspects, the method includes determining the level of four or more glycans selected from the group consisting of 2081, 2315, 2592, 2605, and 4587.

In some cases of any of the foregoing aspects, the method includes determining the level of all five of the glycans selected from the group consisting of 2081, 2315, 2592, 2605, and 4587.

In another aspect, the description provides a method for diagnosing liver fibrosis in a subject by determining a level of one or more (e.g., 1, 2, 3, 4, or 5) fatty acid in a biological sample obtained from the subject, in which a level of the one or more fatty acid in the biological sample that differs from a reference level is indicative of the presence of liver fibrosis, and in which the one or more fatty acid is selected from the group consisting of C14:0, C18:1n9trans, C18:3n3, C20:3n6, and C22:6n3.

In another aspect, the description provides a method of treating liver fibrosis in a subject, the method including the steps of (a) identifying the subject as having liver fibrosis by determining that a level of one or more (e.g., 1, 2, 3, 4, or 5) fatty acid in a biological sample obtained from the subject differs from a reference level, in which the one or more fatty acid is selected from the group consisting of C14:0, C18:1n9trans, C18:3n3, C20:3n6, and C22:6n3; and (b) administering a treatment for liver fibrosis to the subject.

In another aspect, the description provides a method of monitoring treatment efficacy in a subject being treated with a liver fibrosis therapy, the method including the steps of (a) identifying the subject's condition as unchanged by determining that a level of one or more (e.g., 1, 2, 3, 4, or 5) fatty acid in a biological sample obtained from the subject differs from a reference level or is unchanged relative to a previous sample obtained from the subject, in which the one or more fatty acid is selected from the group consisting of C14:0, C18:1n9trans, C18:3n3, C20:3n6, and C22:6n3; and (b) modifying the therapy administered to the subject.

In another aspect, the description provides a method of monitoring the development of liver fibrosis in a subject with NASH, the method including the steps of (a) identifying that the subject has developed liver fibrosis by determining that a level of one or more (e.g., 1, 2, 3, 4, or 5) fatty acid in a biological sample obtained from the subject differs from a reference level, in which the one or more fatty acid is selected from the group consisting of C14:0, C18:1n9trans, C18:3n3, C20:3n6, and C22:6n3; and (b) administering a liver fibrosis therapy to said subject.

In some cases of any of the foregoing aspects, the method includes determining the level of two or more fatty acids selected from the group consisting of C14:0, C18:1n9trans, C18:3n3, C20:3n6, and C22:6n3.

In some cases of any of the foregoing aspects, the method includes determining the level of three or more fatty acids selected from the group consisting of C14:0, C18:1n9trans, C18:3n3, C20:3n6, and C22:6n3.

In some cases of any of the foregoing aspects, the method includes determining the level of four or more fatty acids selected from the group consisting of C14:0, C18:1n9trans, C18:3n3, C20:3n6, and C22:6n3.

In some cases of any of the foregoing aspects, the method includes determining the level of all five of the fatty acids selected from the group consisting of C14:0, C18:1n9trans, C18:3n3, C20:3n6, and C22:6n3.

In another aspect, the description provides a method of treating liver fibrosis in a subject by administering a treatment for liver fibrosis to a subject diagnosed as having liver fibrosis by any of the foregoing aspects or cases.

In some cases of any of the foregoing aspects, the reference level is established by performing classifier training using lipid, glycan, and/or fatty acid levels obtained from subjects clinically diagnosed as having liver fibrosis and healthy subjects.

In some cases of any of the foregoing aspects, the reference level is established by training a machine learning algorithm using a reference dataset.

In some cases of any of the foregoing aspects, the diagnosing further includes evaluating one or more additional parameters selected from the group consisting of liver transaminases, platelets, age, BMI, and PNPLA3 genotype.

In some cases of any of the foregoing aspects, the NASH therapy or treatment is weight loss, a dietary change, increased physical activity, an anti-inflammatory agent, an anti-apoptosis agent, a medication to treat insulin resistance or type 2 diabetes, a medication to improve cholesterol, Vitamin E, a peroxisome proliferator-activated receptor (PPAR) agonist, glutathione, usrodeoxycholic acid, pemafibrate, aramchol, GS0976, an anti-hypertensive drug, a farnesoid X receptor (FXR) agonist, MGL-3196, BI 1467335, IMM-124E, solithromycin, BMS-986036, Cenicriviroc (CVC), or JKB-121.

In some cases of any of the foregoing aspects, the NAFL therapy or treatment is weight loss, a dietary change, increased physical activity.

In some cases of any of the foregoing aspects, the liver fibrosis therapy or treatment is weight loss, a dietary change, increased physical activity, an anti-inflammatory agent, an anti-apoptosis agent, a medication to treat insulin resistance or type 2 diabetes, a medication to improve cholesterol, Vitamin E, a peroxisome proliferator-activated receptor (PPAR) agonist, glutathione, usrodeoxycholic acid, pemafibrate, aramchol, GS0976, an anti-hypertensive drug, a farnesoid X receptor (FXR) agonist, MGL-3196, BI 1467335, IMM-124E, solithromycin, BMS-986036, JKB-121, an anti-fibrotic agent, or liver transplant.

In some cases of any of the foregoing aspects, modifying the NASH therapy, NAFL therapy, or liver fibrosis therapy comprises modifying the dosage, frequency of administration, or therapeutic agent administered to the subject.

In some cases of any of the foregoing aspects, the level is determined by one or more of mass spectrometry, LC/MS, gas chromatography, or ELISA.

In some cases of any of the foregoing aspects, the biological sample is a whole blood, serum, or plasma sample. In some cases, the biological sample is a serum sample.

### Definitions

To facilitate the understanding of this invention, a number of terms are defined below. Terms defined herein have meanings as commonly understood by a person of ordinary skill in the areas relevant to the invention. Terms such as "a", "an," and "the" are not intended to refer to only a singular entity, but include the general class of which a specific example may be used for illustration. The terminology herein is used to describe specific embodiments of the invention, but their usage does not limit the invention, except as outlined in the claims.

As used herein, the term "about" refers to a value that is within 10% above or below the value being described.

As used herein, any values provided in a range of values include both the upper and lower bounds, and any values contained within the upper and lower bounds.

As used herein, "administration" refers to providing or giving a subject a therapeutic agent (e.g., a therapy for NASH or liver fibrosis), by any effective route. Exemplary routes of administration are described herein below.

By a "control" is meant any useful reference used to diagnose NASH or liver fibrosis. The control can be any sample, standard, standard curve, or level that is used for comparison purposes. The control may be a negative control (e.g., a sample or level from a subject diagnosed as not having NAFL, NASH, or liver fibrosis, e.g., a healthy subject) or a positive control (e.g., a sample or level from a subject clinically diagnosed as having NAFL, NASH, or liver fibrosis). A "reference sample" can be, for example, a sample from a normal healthy subject (e.g., a negative control), such as a blood sample (e.g., a serum or plasma sample) from a subject not having NASH or liver fibrosis, a related disease, or a condition to be differentiated from NASH or liver fibrosis, such as NAFL, or a sample from a subject having a liver disease, such as NAFL, NASH, or liver fibrosis (e.g., a positive control). By "reference standard or level" is meant a value or number derived from a reference sample, such as a level of one or more biomarkers (e.g., one or more lipids, glycans, fatty acids, and/or hormones) in a biological sample (e.g., a blood, serum, or plasma sample) obtained from a healthy subject (e.g., a negative control subject, such as a subject that does not have NAFL, NASH, or liver fibrosis), and/or a subject clinically diagnosed (e.g., diagnosed using liver biopsy or imaging) as having NAFL, NASH, or liver fibrosis (e.g., a positive control subject). The reference level can be based on measurements from a single subject or from a population of subjects and may represent raw data or data transformed using machine learning algorithms. The reference level is used for diagnosis of a subject as healthy or as having NAFL, NASH, or liver fibrosis. In preferred embodiments, the reference sample, standard, or level is matched to the sample from the subject by at least one of the following criteria: age, weight, sex, disease stage, and overall health. For example, the negative control sample for diagnosis of an obese subject (e.g., a subject having a BMI greater than or equal to than 27.5 kg/m²) may be a serum sample from an obese subject (e.g., a subject having a BMI greater than or equal to than 27.5 kg/m²) not having NAFL, NASH, or liver fibrosis.

By "diagnosing" is meant identifying a molecular or pathological state, disease, or condition, such as the identification of NASH, NAFL, or liver fibrosis or to refer to identification of a subject having NASH, NAFL, or liver fibrosis who may benefit from a particular treatment regimen.

As used herein, the terms "effective amount," "therapeutically effective amount," and a "sufficient amount" of a composition or therapy described herein refer to a quantity sufficient to, when administered to the subject in need thereof, including a mammal, for example a human, effect beneficial or desired results, including clinical results, and, as such, an "effective amount" or synonym thereto depends upon the context in which it is being applied. For example, in the context of treating NASH or liver fibrosis, it is an amount of the composition or therapy sufficient to achieve a treatment response as compared to the response obtained without administration of the composition of therapy. The amount of a given composition described herein that will correspond to such an amount will vary depending upon various factors, such as the given agent, the pharmaceutical formulation, the route of administration, the type of disease or disorder, the identity of the subject (e.g. age, sex, weight) being treated, and the like, but can nevertheless be routinely determined by one skilled in the art. Also, as used herein, a "therapeutically effective amount" of a composition or therapy of the present disclosure is an amount which results in a beneficial or desired result in a subject as compared to a control. Note that when a combination of active ingredients is administered, the effective amount of the combination may or may not include amounts of each ingredient that would have been effective if administered individually. As defined herein, a therapeutically effective amount of a composition or therapy of the present disclosure may be readily determined by one of ordinary skill by routine methods known in the art. Dosage regime may be adjusted to provide the optimum therapeutic response.

As used herein, the term "lean subject" refers to a subject that is lean or slightly overweight having a body mass index (BMI) less than 27.5 kg/m².

As used herein, the term "obese subject" refers to a subject that is overweight or obese having a BMI greater or equal to 27.5 kg/m².

As used herein, the terms "nonalcoholic fatty liver disease" and "NAFLD" refer to an accumulation of excess fat in the liver that is not caused by abuse of alcohol. NAFLD encompasses NAFL and NASH.

As used herein, the terms "nonalcoholic fatty liver" and "NAFL" refer to a condition in which the liver contains excess fat but does not exhibit liver inflammation or liver cell damage. The liver is considered fatty if there is fat deposition in more than 5% of hepatocytes.

As used herein, the terms "nonalcoholic steatohepatitis" and "NASH" refer to an advanced form of NAFLD in which a buildup of fat in the liver leads to liver inflammation and liver cell damage.

As used herein, the term "liver fibrosis" refers to the buildup of scar tissue in the liver, which may be caused by repetitive or long-lasting injury or inflammation. NASH can lead to liver fibrosis.

As used herein, the term "sample" refers to a specimen (e.g., blood, blood component (e.g., serum or plasma), urine, saliva, amniotic fluid, cerebrospinal fluid, tissue (e.g., placental or dermal), pancreatic fluid, chorionic villus sample, and cells) isolated from a subject. As used herein, the terms "subject" and "patient" refer to an animal (e.g., a mammal, such as a human), veterinary animals (e.g., cats, dogs, cows, horses, sheep, pigs, etc.) and experimental animal models of diseases (e.g., mice, rats). A subject to be diagnosed and treated according to the methods described herein may be one who has been diagnosed with NASH, NAFLD, or liver fibrosis, or one at risk of developing these conditions. Diagnosis may be performed using the methods described herein. One skilled in the art will understand that a subject to be treated according to the present disclosure may have been subjected to standard tests or may have been identified, without examination, as one at risk due to the presence of one or more risk factors associated with the disease or condition (e.g., obesity).

As used herein, "treatment" and "treating" in reference to a disease or condition, refer to an approach for obtaining beneficial or desired results, e.g., clinical results. Beneficial or desired results can include alleviation or amelioration of one or more symptoms or conditions; diminishment of extent of disease or condition; stabilized (i.e., not worsening) state of disease, disorder, or condition; preventing spread of disease or condition; delay or slowing the progress of the disease or condition; amelioration or palliation of the disease or condition; and remission (whether partial or total), whether detectable or undetectable. "Ameliorating" or "palliating" a disease or condition means that the extent and/or undesirable clinical manifestations of the disease, disorder, or condition are lessened and/or time course of the progression is slowed or lengthened, as compared to the extent or time course in the absence of treatment. "Treatment" can also mean prolonging survival as compared to expected survival if not receiving treatment. Those in need of treatment include those already with the condition or disorder, as well as those prone to have the condition or disorder or those in which the condition or disorder is to be prevented.

### Brief Description of the Drawings

The patent or application file contains at least one drawing in color.

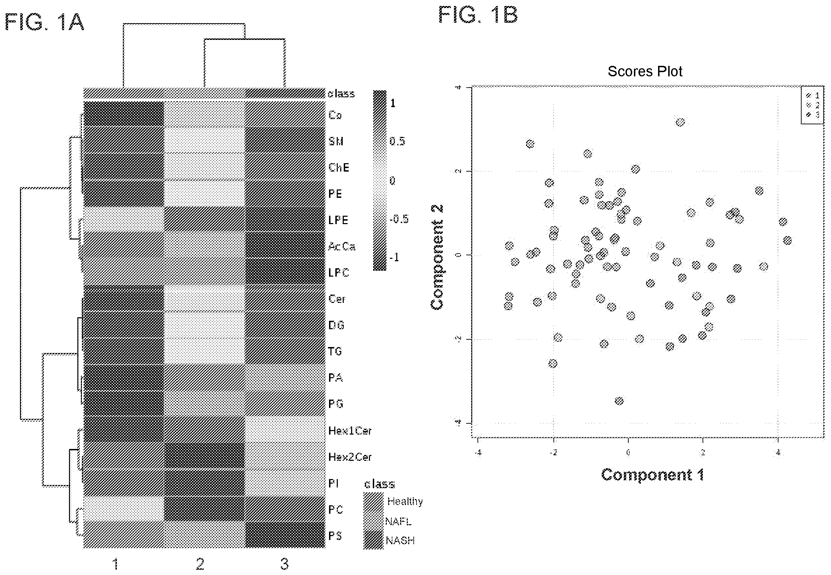

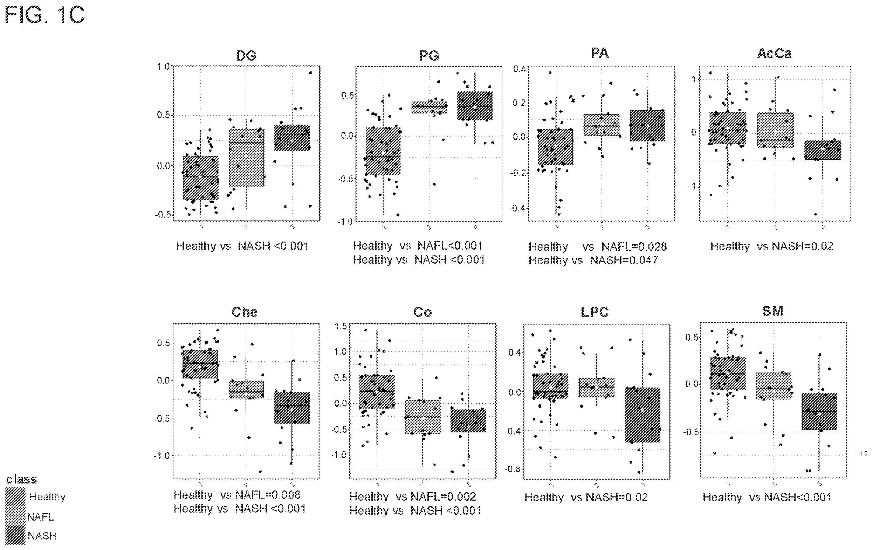

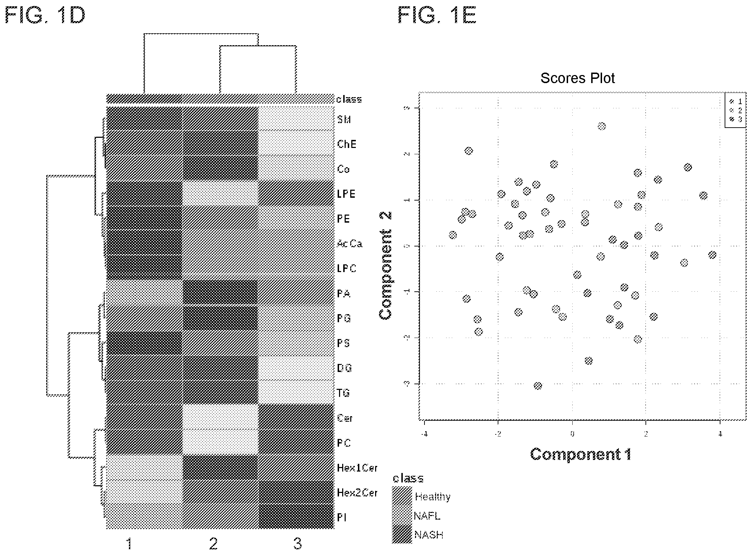

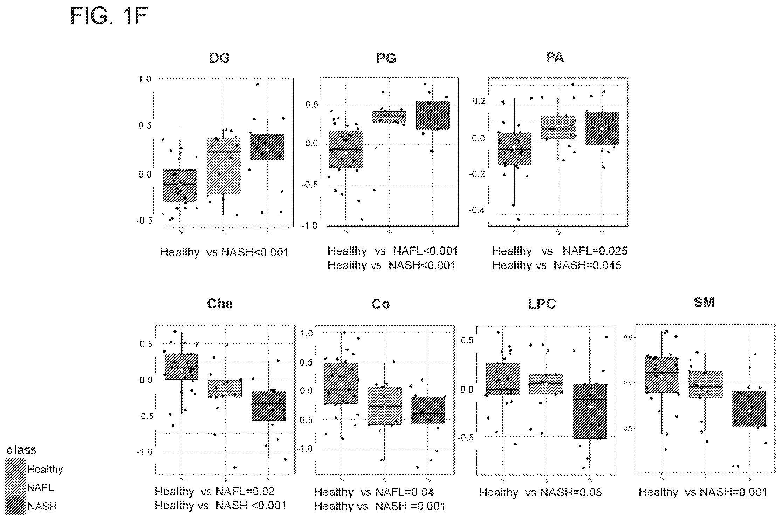

**FIGS. 1A-1F** are a series of graphs showing the profile of circulating lipid classes in healthy patients, patients with NAFL, and patients with NASH. FIG. 1A is a heatmap of hierarchical clustering displaying the relative concentrations of 17 lipid classes assessed in the study. FIG. 1B is a score plot of component 1 and component 3 of the sPLS-DA analysis. Component 1 was made up of 10 lipid classes: phosphatidylglycerols (PG), cholesterol esters (Che), coenzyme Q10 (Co), sphingomyelines (SM), diglycerides (DG), phosphatidic acids (PA), triglycerides (TG), acylcarnitines (AcCa), phosphatidylethanolamines (PE), and lysophosphatidylcholines (LPC); and component 2 was made up of a different combination of 10 lipid classes: phosphatidylcholines (PC), TG, ceramides (Cer), AcCa, phosphatidylinositols (PI), lysophosphatidylethanolamines (LPE), PE, PG, Hex2 ceramides (Hex2Cer), and SM (red dots: healthy, green dots: NAFL, blue dots: NASH). FIG. 1C shows boxplots of the significantly different lipid classes between the three groups. FIGS. 1D-1F display the results of a similar analysis when lean subjects (e.g., subjects having a BMI less than 27.5 kg/m²) were excluded from the control group (obese control vs. NAFL vs. NASH). FIG. 1D is a heatmap of hierarchical clustering displaying the relative concentrations of 17 lipid classes. FIG. 1E is a score plot of component 1 and component 3 of the sPLS-DA analysis. Component 1 was made up of 10 lipid classes: PG, Che, Co, DG, SM, PA, LPC, AcCa, TG, and phosphatidylserine (PS); and component 2 was made up of a different combination of 10 lipid classes: Cer, PC, AcCa, TG, PE, PI, PA, PG, SM, and Hex2Cer. FIG. 1F shows boxplots of lipid classes demonstrating significant differences between the three groups. The black dots in boxplots represent the concentrations of the selected feature from all samples. The notch indicates the 95% confidence interval. The yellow diamond indicates the mean concentration of each group. One-way ANOVA followed by Tukey's test was performed for the normally distributed variables and Kruskal-Wallis test followed by Dunn's test was performed for the non-normally distributed ones (FIG. 1C: DG, PG, Che, FIG. 1F: PG).

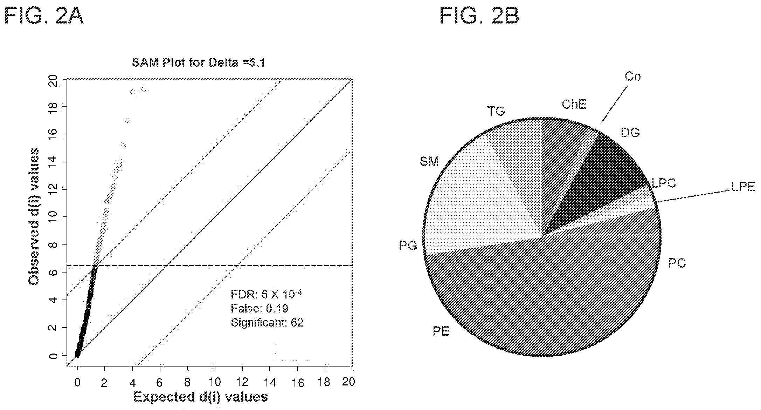

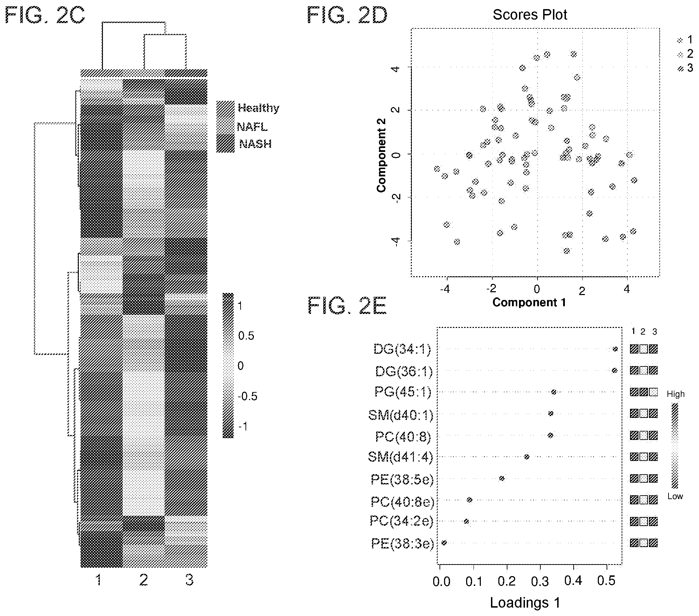

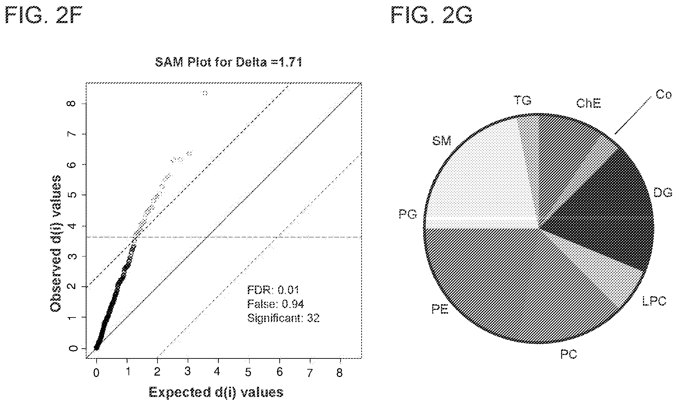

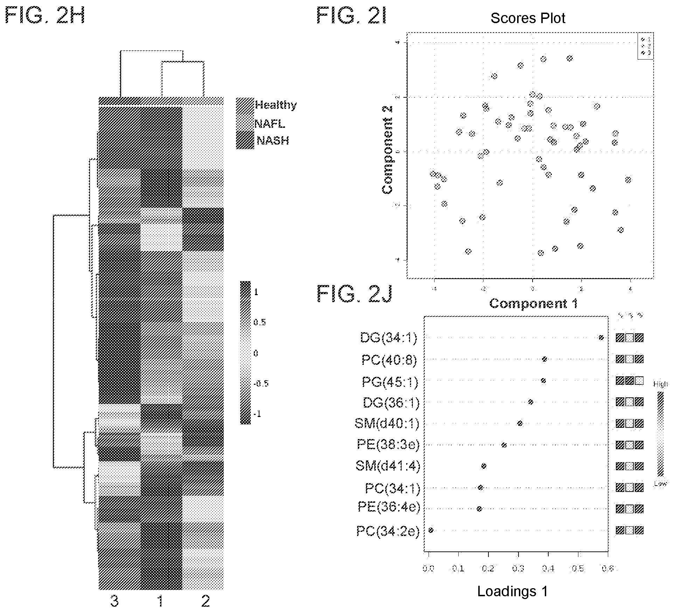

**FIGS. 2A-2J** are a series of graphs showing the prolife of circulating lipid species in healthy patients, patients with NAFL, and patients with NASH. Significance analysis of microarrays (SAM) identifies 62 lipid species that are significantly different between the three groups with a False Discovery Rate (FDR) of 6 x 10⁻⁴ (FIG. 2A). The x-axis represents the expected and the y-axis represents the observed score of the function that depends on a lipid concentration. If the absolute value of (observed-expected) score for a lipid is larger than delta, then this lipid is significantly different between the three groups (healthy, NAFL, NASH), which is marked by a green dot, while the non-significant lipids are marked by black dots. FIG. 2B is a pie chart showing class breakdown of the 62 significant lipid species. FIG. 2C is a heatmap of hierarchical clustering displaying all the lipid species in the three groups. FIG. 2D is a score plot of component 1 and component 2 of the sPLS-DA analysis. Component 1 was made up of 10 lipid species: DG(34:1), DG(36:1), PG(45:1), SM(d40:1), PC(40:8), SM(d41:4), PE(38:5e), PC(40:8e), PC(34:2e), and PE(38:3e); and component 2 was made up of a different combination of 10 lipid species: LPC(17:0), LPC(15:0), LPC(17:0).1 (second ion corresponding to LPC17:0), LPC(14:0), LPC(20:0e), LPC(22:5), LPC(16:1), SM(d32:0), Cer(d43:1), and LPC(20:2) (red dots: healthy, green dots: NAFL, blue: NASH). FIG. 2E displays loadings of component 1 shown for top 10 ranked lipids. FIGS. 2F-2J demonstrate a similar analysis when lean subjects (e.g., subjects having a BMI less than 27.5 kg/m²) were excluded from the control group (obese control vs. NAFL vs. NASH). SAM identifies 32 lipid species that are significantly different between the three groups with an FDR of 0.01 (FIG. 2F). FIG. 2G is a pie chart showing class breakdown of the 32 significant lipid species. FIG. 2H is a heatmap of hierarchical clustering displaying all the lipid species. FIG. 2I is a score plot of component 1 and component 2 of the sPLS-DA analysis. Component 1 was made up of 10 lipid species: DG(34:1), PC(40:8), PG(45:1), DG(36:1), SM(d40:1), PE(38:3e), SM(d41:4), PC(34:1), PE(36:4e), and PC(34:2e); and component 2 was made up of a different combination of lipid species: LPC(22:5), LPC(20:0e), LPC(16:1), SM(d32:0), LPC(17:0), LPC(17:0).1 (second ion corresponding to LPC17:0), PC(37:1), LPC(14:0), and PI(36:1). FIG. 2J displays loadings of component 1 shown for top 10 ranked lipids.

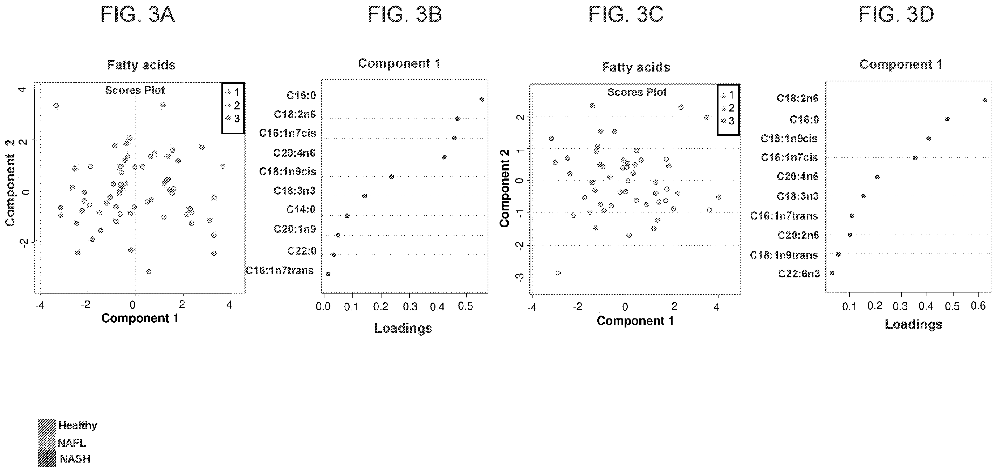

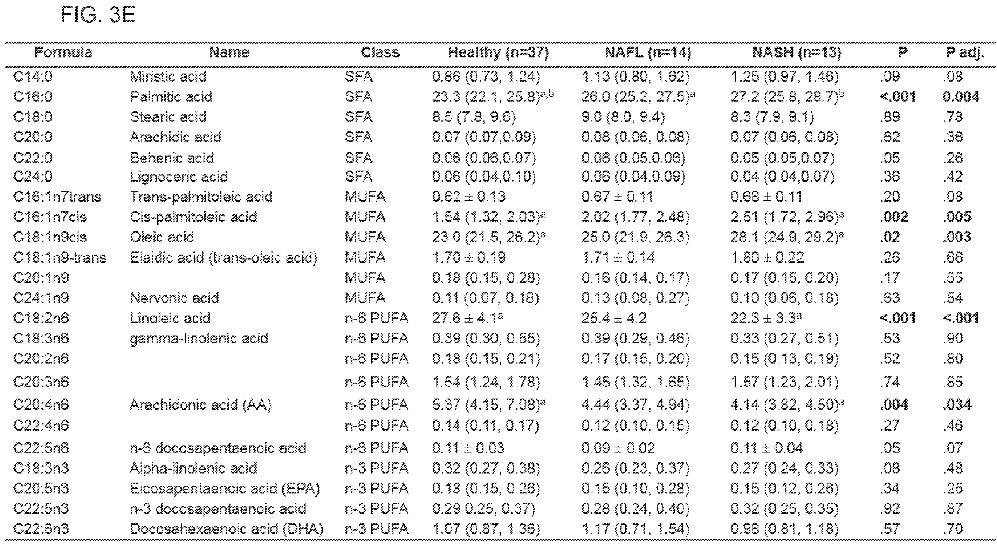

**FIGS. 3A-3E** are a series of graphs and a table showing fatty acid concentrations in healthy patients, patients with NAFL, and patients with NASH. FIG. 3A is a scores Plot of component 1 and component 2 of the sPLS-DA analysis. Component 1 was made up of 10 fatty acids: C16:0, C18:2n6, C16:1n7cis, C20:4n6, C18:1n9cis, C17:3n3, C14:0, C20:1n9, C22:0, C16:1n7 trans; and component 2 was also made up of 10 fatty acids C22:5n6, C22:4n6, C22:0, C20:1n9, C24:1n9, C20:5n3, C18:2n6, C18:1n7trans, C16:1n7trans, and C18:3n3 (red= healthy, green= NAFL, blue= NASH). FIG. 3B displays loadings of component 1 showing that mainly the first five fatty acids (C16:0, C18:2n6, C16:1n7cis, C20:4n6, and C18:1n9cis) are contributing to the component. FIGS. 3C-3D demonstrate a similar analysis when lean subjects (e.g., subjects having a BMI less than 27.5 kg/m²) were excluded from the control group (obese control vs. NAFL vs. NASH). FIG. 3C is a scores Plot of component 1 and component 2. Component 1 was made up of 10 fatty acids: C18:2n6, C16:0, C18:1n9cis, C16:1n7cis, C20:4n6, C18:3n3, C16:1n7trans, C20:2n6, C18:1n9trans, and C22:6n3; and component 2 was also made up of 10 fatty acids C20:5n3, C22:5n6, C18:3n6, C22:4n6, C22:0, C14:0, C20:1n9, C20:0, C22:5n3, and C16:1n7trans (red= healthy, green= NAFL, blue= NASH). FIG. 3D displays loadings of component 1 showing that five fatty acids (C18:2n6, C16:0, C18:1n9cis, C16:1n7cis, and C20:4n6) are mainly contributing to the component. FIG. 3E is a table of the 23 fatty acids measured in the study. p-values are derived from one-way ANOVA or Kruskal-Wallis test; p adj.-values are derived from one-way ANCOVA after adjusting for BMI (non-normally distributed variables were logarithmically transformed). Same superscript letters indicate significant differences between the two groups based on post-hoc Tukey's or Dunn's test.

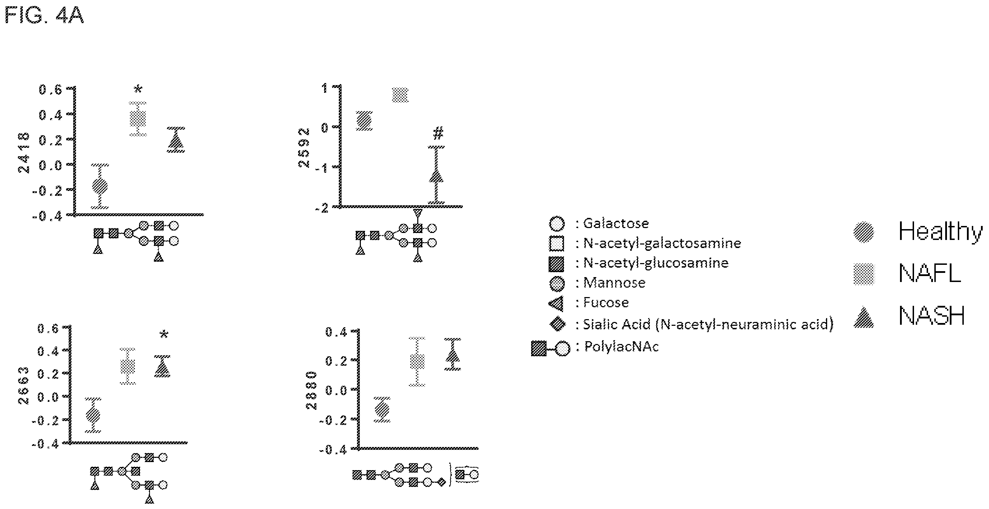

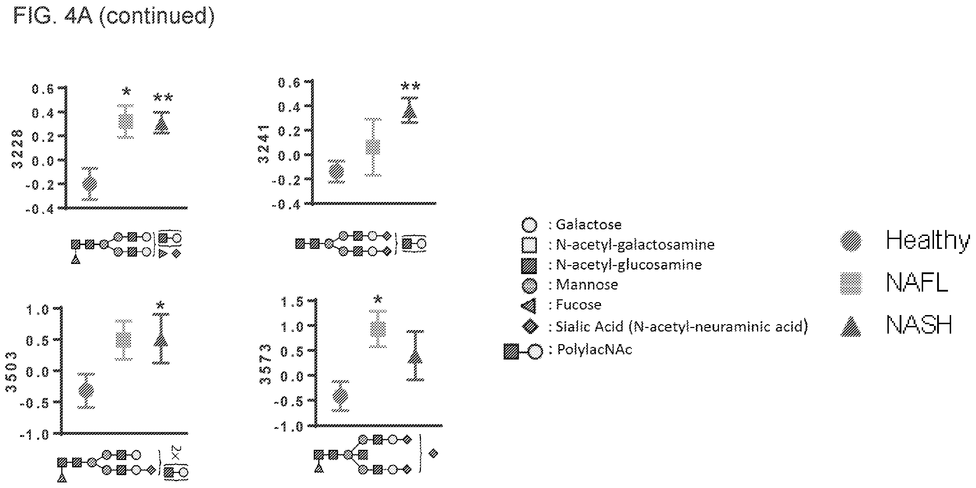

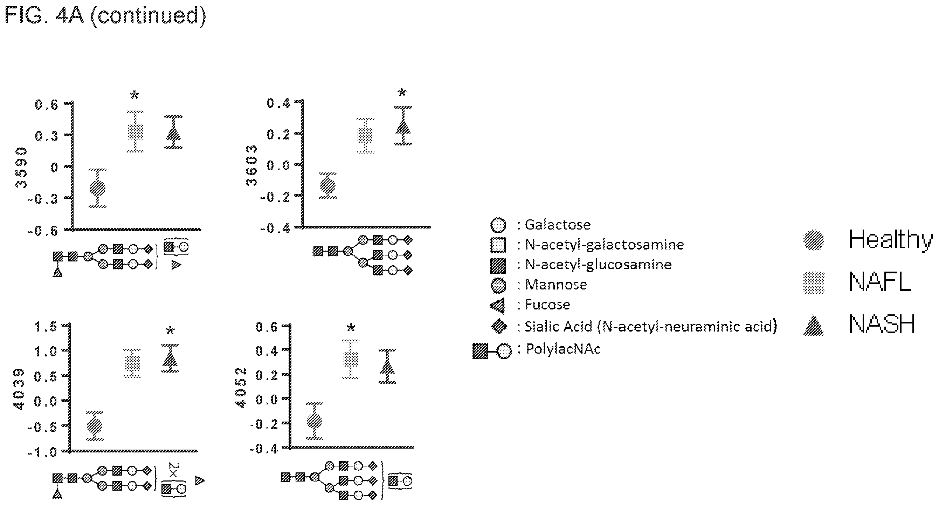

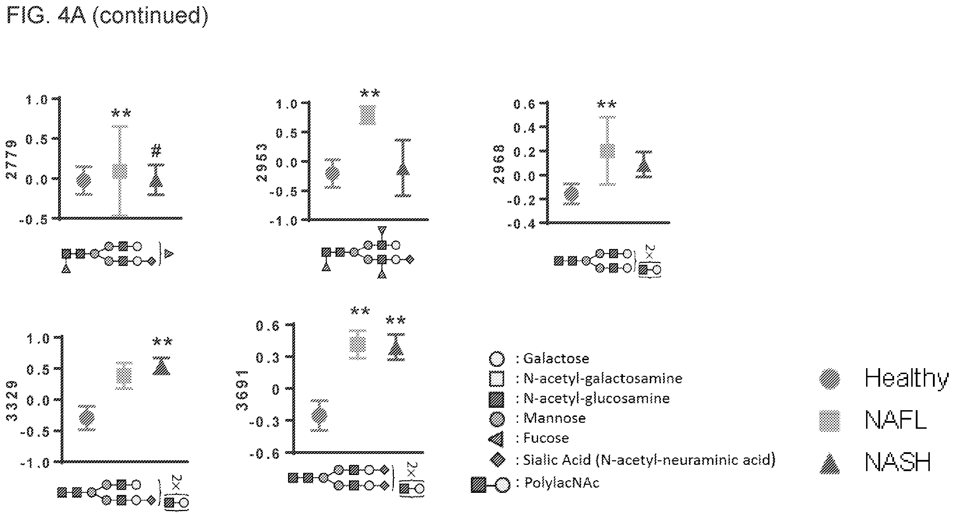

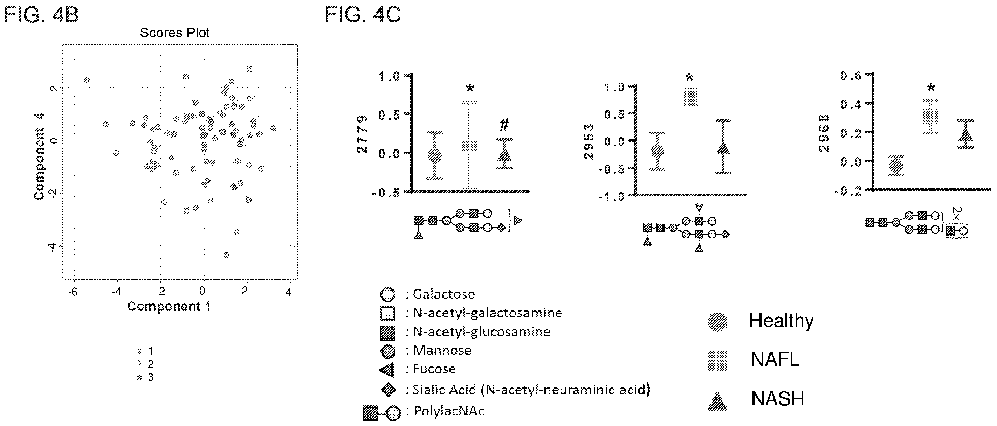

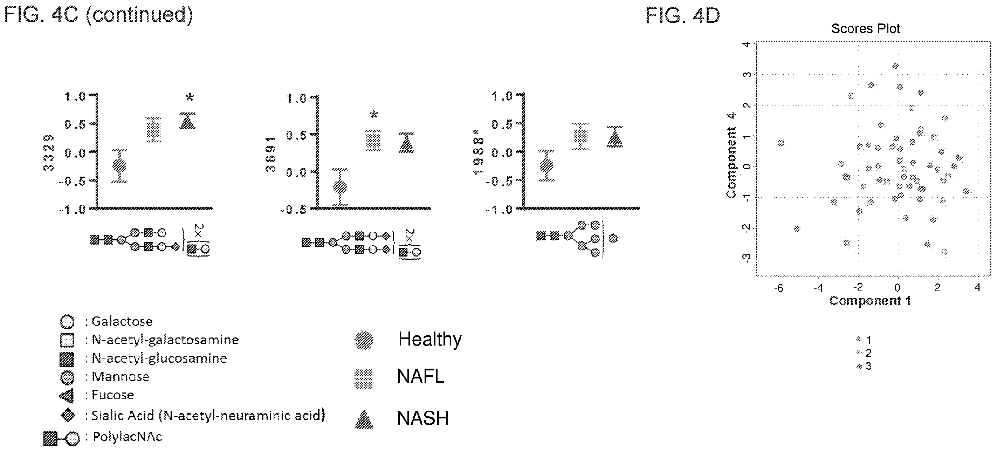

**FIGS. 4A-4D** are a series of graphs showing the N-glycan profile in healthy patients, patients with NAFL, and patients with NASH. FIG. 4A is a series of graphs for glycans with significantly different concentrations between the three groups. FIG. 4B. is a score plot of component 1 and component 4 from the sPLS-DA analysis. Component 1 was made up of 10 glycans (mass m/z is reported): 4039, 3691, 2968, 3603, 3329, 3228, 2880, 4400, 2693, and 3953; and component 4 was made up of a different combination of glycans: 2070, 2285, 2244, 2081, 2315, 1866, 3865, 2448, 2192, and 2738(red= healthy, green= NAFL, blue=NASH). FIGS. 4C-4D demonstrate a similar analysis when lean subjects (e.g., subjects having a BMI less than 27.5 kg/m²) were excluded from the control group (obese control vs. NAFL vs. NASH). FIG. 4C is a series of graphs for glycans with significantly different concentrations between the three groups. FIG. 4D is a score plot of component 1 and component 4 from the sPLS-DA analysis. Component 1 was made up of 10 glycans: 3953, 2968, 4039, 3691, 4400, 3329, 2880, 2663, 3228, and 2693; and component 4 was made up of a different combination of glycans: 2431, 2396, 1836, 2448, 3054, 2244, 2605, 3503, 2489, and 3415 (red= healthy, green= NAFL, blue=NASH). The x-axis shows the most likely structure of the glycan and y-axis shows its mass (m/z) according to mass spectrometry. *,**,***, p<0.05, <0.01 and <0.001 in the post hoc t-test between the group with the star (NAFL or NASH) vs. healthy. #, p<0.05 in the post-hoc t-test between NASH vs. NAFL. The 2953 and the 1988 glycans were participating regularly in the predictive models.

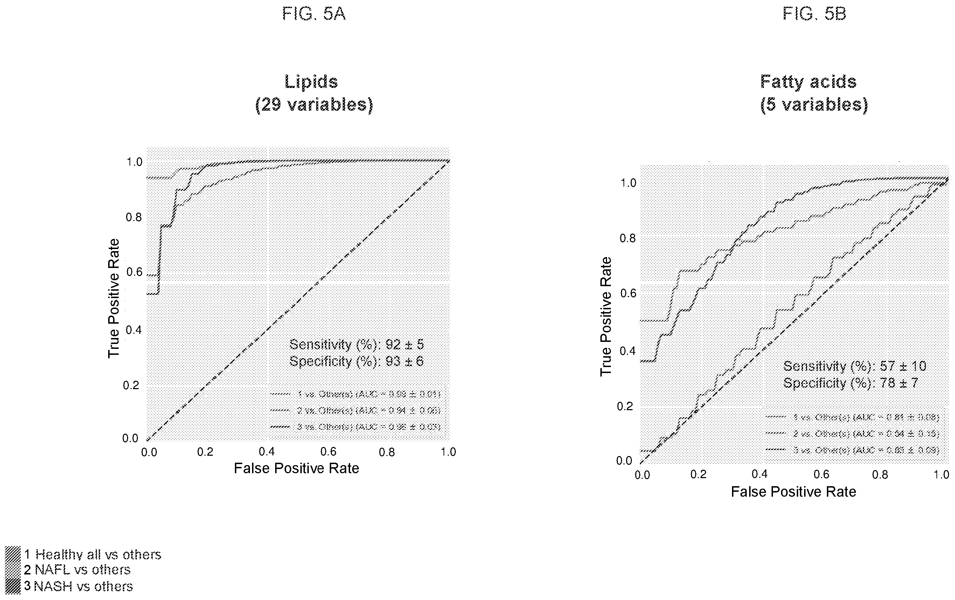

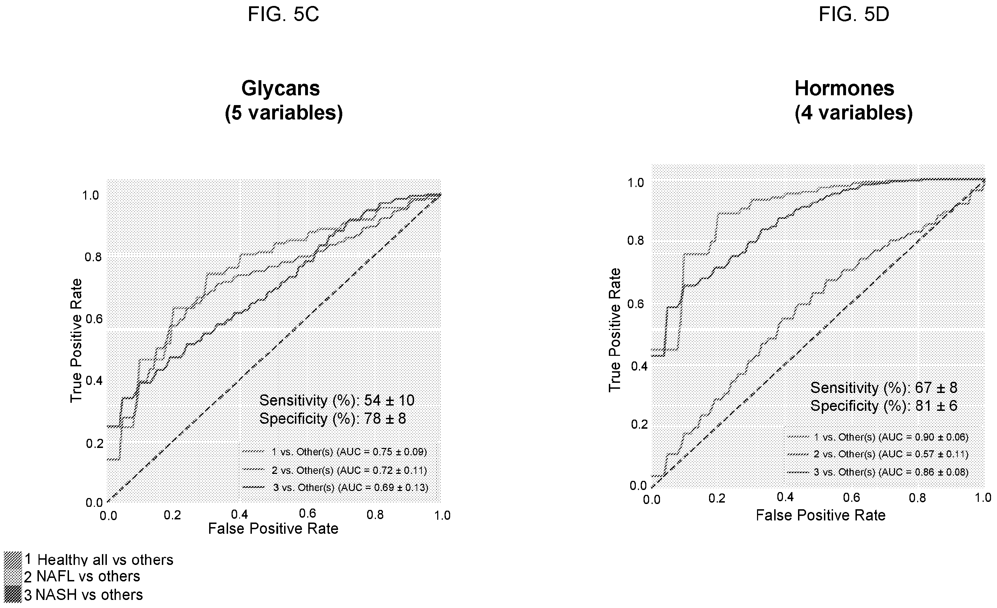

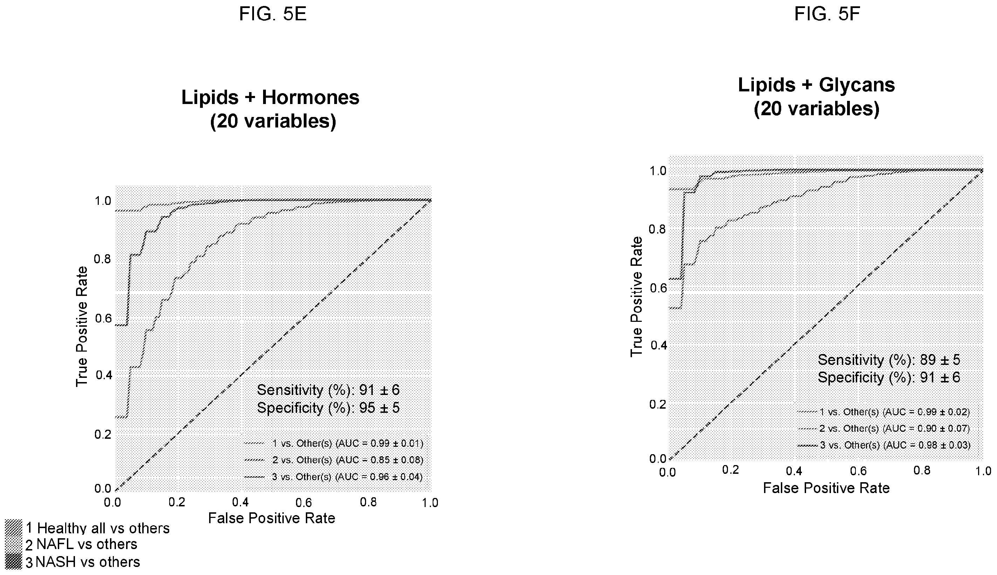

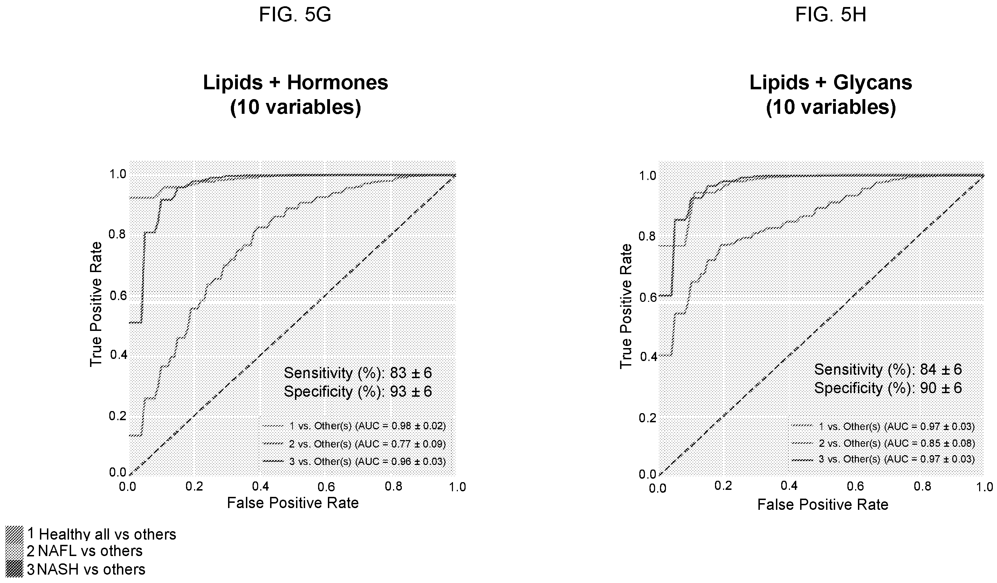

**FIGS. 5A-5H** are a series of graphs showing ROC Curves for the classification of healthy vs. NAFL vs. NASH. FIGS. 5A-5D show ROC curves for single lipids (FIG. 5A), fatty acids (FIG. 5B), glycans (FIG. 5C), and hormones (FIG. 5D) data sets computed using the nonlinear SVM classifier. FIGS. 5E-5H show ROC curves for the integrated lipids with hormones (FIGS. 5E and 5G) and lipids with glycans (FIGS. 5F and 5H) data sets computed using the nonlinear SVM classifier. The number of variables used to produce each curve is indicated in parenthesis.

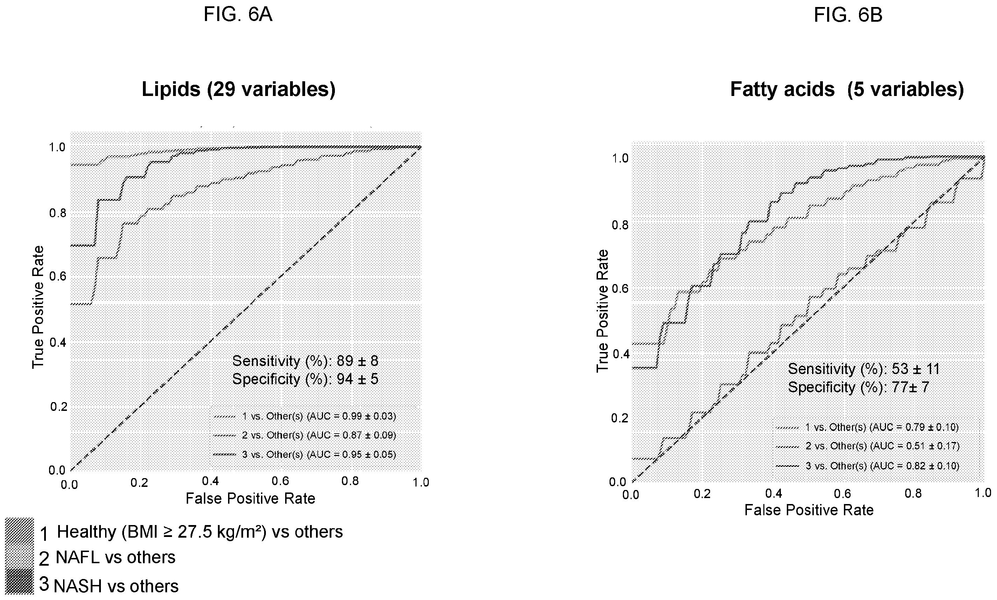

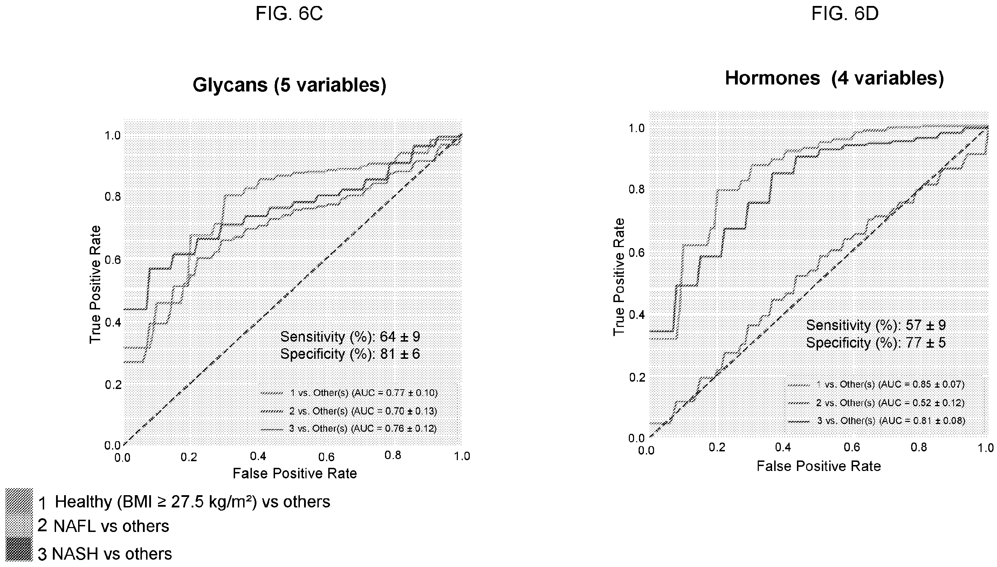

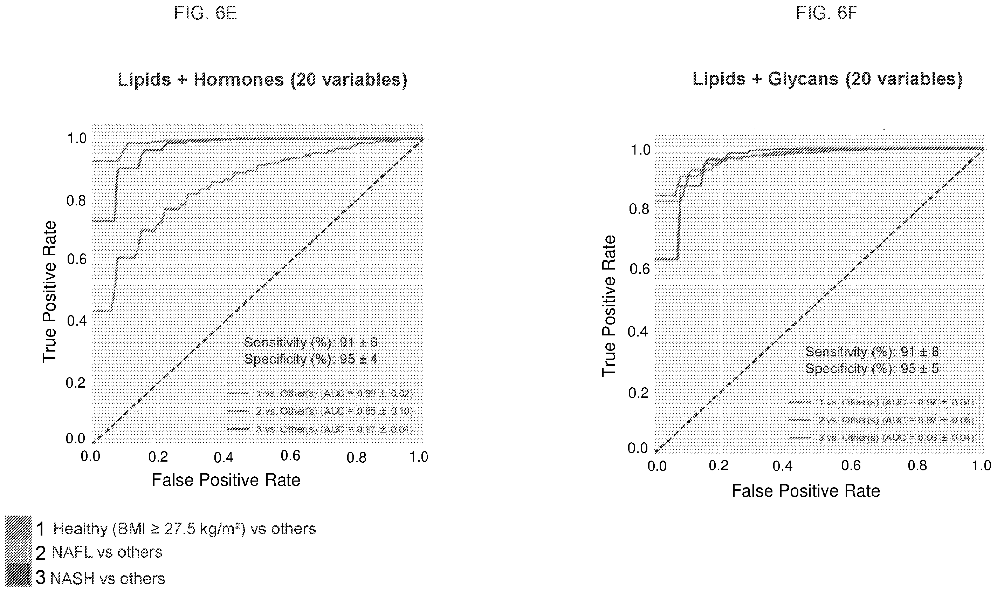

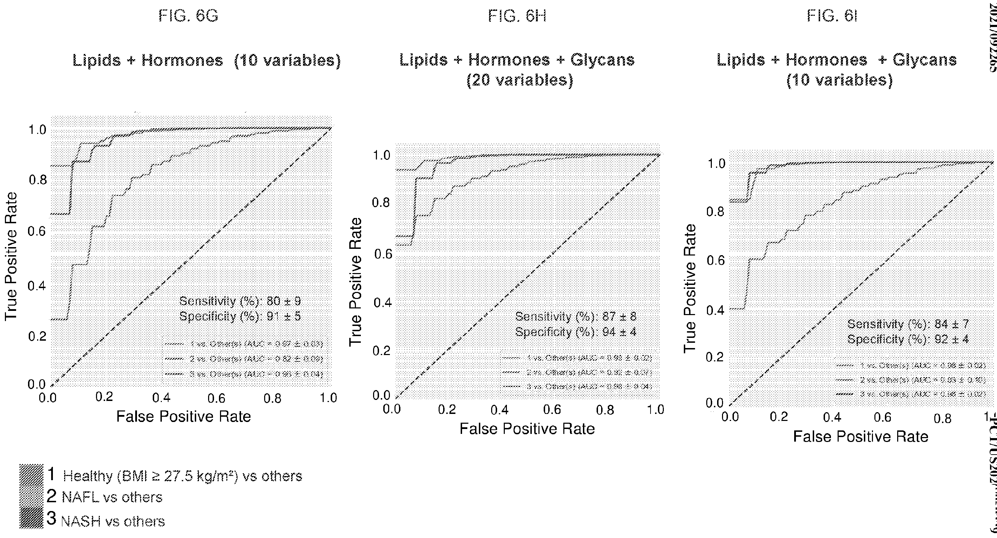

**FIGS. 6A-6I** are a series of graphs showing ROC Curves for the classification of healthy (obese) vs. NAFL vs. NASH. FIGS. 6A-6D show ROC curves for single lipids (FIG. 6A), fatty acids (FIG. 6B), glycans (FIG. 6C), and hormones (FIG. 6D) data sets computed using the nonlinear SVM classifier. FIGS. 6E-6I show ROC curves for the integrated lipids with hormones (FIG. 6G), lipids with glycans (FIG. 6F), and lipids with hormones and glycans (FIGS. 6H and 6I) data sets computed using the nonlinear SVM classifier. The number of variables used to produce each curve is indicated in parenthesis.

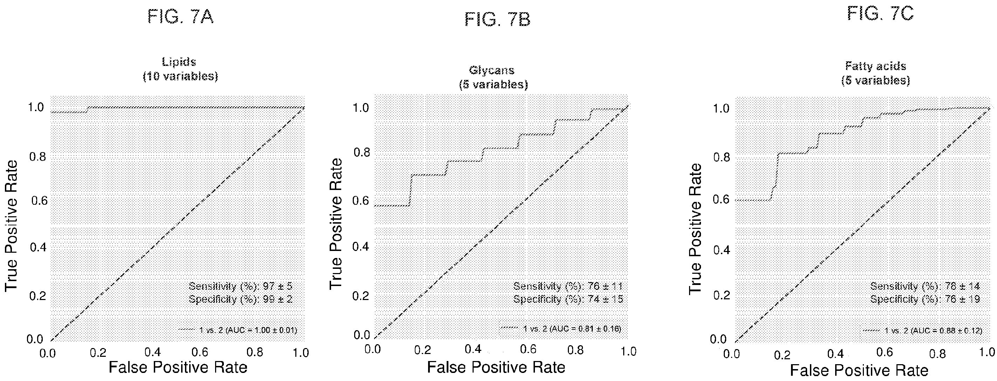

**FIGS. 7A-7C** are a series of graphs showing ROC Curves for the classification of fibrosis status. FIGS. 7A-7C show ROC curves for single lipids (FIG. 7A), glycans (FIG. 7B), and fatty acids (FIG. 7C) data sets in subjects with liver fibrosis (n=21) vs. subjects without liver fibrosis (n=10).

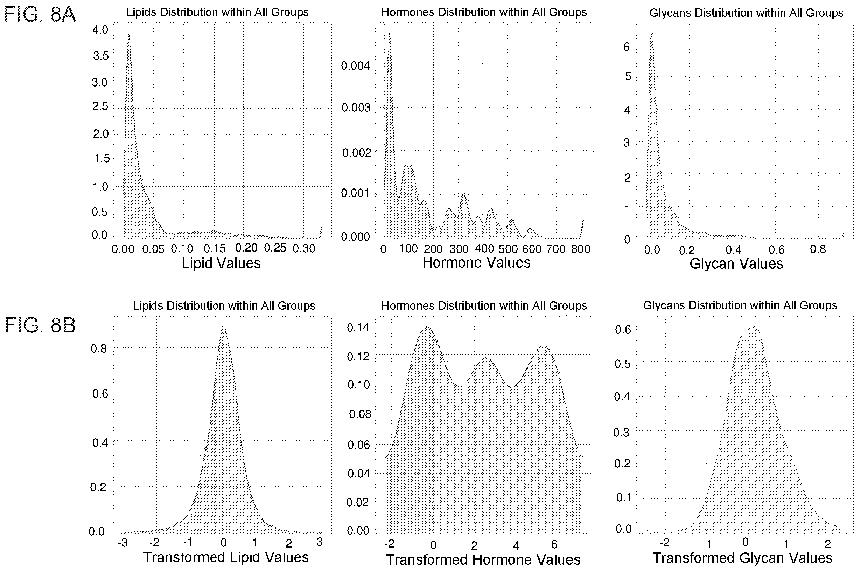

**FIGS. 8A-8B** are a series of graphs showing distributions of raw vs. transformed data for each data set. FIG. 8A shows the raw data distributions of lipids, hormones, and glycans for all variables within three groups of healthy, NAFL and NASH. FIG. 8B shows the transformed data distributions of lipids, hormones, and glycans for all variables within three groups of healthy, NAFL and NASH.

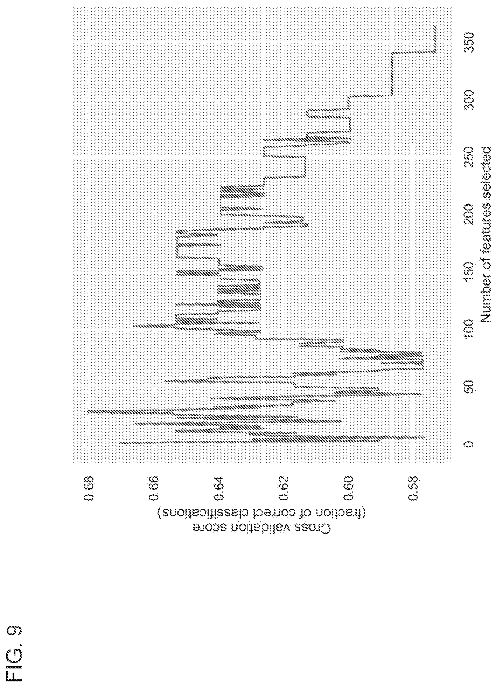

**FIG. 9** is a graph showing a representative cross-validation score diagram from the recursive feature elimination (RFE) algorithm. A linear SVM classifier was used in the RFE algorithm that identified the top-scored variables among the lipid species data set. The algorithm suggested 29 variables to be selected for further analysis.

### Detailed Description of the Invention

The present invention relates to the identification of biomarkers that can be used to differentiate between healthy subjects and subjects with NAFL, NASH, or liver fibrosis. These biomarkers include lipids, glycans, fatty acids, and hormones and can be detected in a blood sample (e.g., a serum sample or plasma sample). Accordingly, the methods described herein can be used to diagnose NAFL, NASH, or liver fibrosis in a subject, to guide treatment of such subjects, and to evaluate treatment efficacy. These methods provide a non-invasive, high-sensitivity alternative to liver biopsy, which is currently the only tool available for diagnosis of these conditions.

### Diagnosis of NAFLD and liver fibrosis

Non-alcoholic fatty liver disease (NAFLD), the hepatic component of metabolic syndrome, is now recognized as the most prevalent chronic liver disease worldwide, affecting 25-30% of the worldwide population. In the first stage of NAFLD, an accumulation of fat is observed in the liver (non-alcoholic fatty liver, NAFL), which can progress to an inflammatory state (non-alcoholic steatohepatitis, NASH) and, in later stages, to liver fibrosis and cirrhosis that can lead to hepatocellular carcinoma (HCC). The median time to develop advanced fibrosis when inflammation is absent on the initial biopsy (patients with NAFL) is 13.4 years, while the median time when it is present (patients with NASH) is only 4.2 years.

The gold standard for the diagnosis of NAFLD (NAFL or NASH) and for assessing liver fibrosis is liver biopsy. However, liver biopsy is a costly method that carries certain risks for the patient, such as pain after biopsy, bleeding, and infection, and the liver biopsy specimen is not always representative of the actual status of the entire liver, often resulting in misclassification due to sampling error and/or interobserver viability. Imaging techniques have emerged as attractive alternatives to assess NAFL, NASH, fibrosis, cirrhosis, and hepatocellular carcinoma. However, such tools are often not available in primary care or in smaller community-based gastroenterological or endocrinological departments. Additionally, there are no reliable biomarkers or scores for the non-invasive diagnosis of NASH or for staging liver fibrosis with a similar score as in liver histology. Due to the cost and invasiveness of the procedure, most patients with NAFLD go undiagnosed.

The methods described herein can be used to diagnose subjects with NAFL, NASH, and liver fibrosis, and, accordingly, to guide patient treatment and monitor treatment efficacy. These methods include detecting lipids, glycans, fatty acids, and/or hormones in a blood sample collected from the subject (e.g., a serum sample or plasma sample). The samples can be analyzed using techniques such as mass spectrometry, liquid chromatography, and gas chromatography that allow for high molecular specificity and detection sensitivity. These methods provide a non-invasive diagnostic alternative to liver biopsy with high sensitivity for distinguishing between benign cases (e.g., NAFL) and cases that require follow-up visits and treatment (e.g., NAFL and liver fibrosis).

### Diagnosis using lipids

Lipids are biomolecules that are soluble in non-polar solvents and not soluble in water. The biological functions of lipids include energy storage, signaling, and forming membranes, such as cell and organelle membranes. Lipids can be named based on the stereospecific numbering (sn) method, written as "Headgroup(sn1/sn2)" in which the structures of the side chains are indicated within parentheses. Common headgroup abbreviations include PC for phosphatidylcholines (LPC for lyso species), PE for phosphatidylethanolamines (LPE for lyso species), PG for phosphatidylglycerols (LPG for lyso species), PA for phosphatidic acids (LPA for lyso species), PI for glycerophosphoinositols (LPI for lyso species), AcCa for acylcarnitines, Co for coenzyme Q10, PPA for glyceropyrophosphates, DG for diglycerides, TG for triradylglycerolipids, Cer for ceramides, and SM for sphingomyelins

NAFL, NASH, and liver fibrosis can be diagnosed with high sensitivity and specificity based on lipid concentrations in a biological sample (e.g., a blood, serum, or plasma sample) obtained from the subject. Diagnosis can be performed by comparing lipid concentrations in the biological sample (e.g., a blood, serum, or plasma sample) obtained from the subject to lipid concentrations in a negative control sample of the same type (e.g., a blood, serum, or plasma sample) obtained from one or more subjects not having NASH, NAFL, or liver fibrosis or to one or more biological samples obtained from the subject (e.g., a blood, serum, or plasma sample) prior to the development of NAFLD. A subject may also be diagnosed by comparing a biological sample obtained from the subject (e.g., a blood, serum, or plasma sample) to a positive control, such as a biological sample (e.g., a blood, serum, or plasma sample) obtained from a subject clinically diagnosed with NASH, NAFL, or liver fibrosis (e.g., diagnosed using a liver biopsy or imaging). The biological sample from the subject may be compared directly to a reference level, which may be based on a negative control sample (e.g., a negative control sample from a subject not having NASH, NAFL, or liver fibrosis, e.g., a negative control sample obtained at the same or at a similar time to the biological sample obtained from the subject, or negative a control sample collected from the subject at an earlier time point when the subject was deemed healthy), a positive control sample (a biological sample of the same type (e.g., a blood, serum, or plasma sample) obtained from a subject clinically diagnosed with NASH, NAFL, or liver fibrosis), or a reference sample (e.g., lipid concentrations in a database obtained using samples collected from for one or more subjects not having NASH, NAFL, or liver fibrosis and/or samples collected from one or more subjects having clinically diagnosed NASH, NAFL, or liver fibrosis (e.g., diagnosed using a liver biopsy or imaging)). Lipids can be identified using LC/MS.

Lipids that can be measured to diagnose a subject with NAFL or NASH include five or more (e.g., 5, 6, 7, 8, 9, 10, 11, 12, 13, 14, 15, 16, 17, 18, 19, 20, 21, 22, 23, 24, 25, 26, 27, 28, 29 or more) of AcCa(10:0), AcCa(10:1), Cer(d34:0), Cer(d34:2), Cer(d18:2_25:1), Cer(d43:0), Cer(d43:3), DG(34:1), DG(34:2), DG(36:2), DG(36:4), Hex1Cer(d34:2), LPC(20:0e), LPC(20:3), LPC(22:5), LPE(16:0), LPE(18:0), LPE(22:5), PA(44:4), PC(32:0), PC(32:1e), PC(32:1e)=> *PC(16:0e_16:1),* PC(32:1e)=> *PC(16:1e_16:0),* PC(34:0), PC(34:1), PC(34:1e), PC(34:2e), PC(35:2), PC(35:3), PC(36:2), PC(36:3), PC(36:4), PC(36:5e), PC(37:2), PC(37:3), PC(40:5e), PC(40:6e), PC(40:7), PC(40:8), PC(42:6), PE(34:1), PE(34:3e), PE(38:1), PE(38:6), PE(40:4e), PI(36:1), SM(d32:0), SM(d32:2), SM(d35:1), SM(d36:0), SM(d36:4), SM(d37:1), SM(d38:0), SM(d38:1), SM(d40:1), CO(36:1), TG(38:0), TG(38:2), TG(40:0), TG(43:1), TG(52:4), and TG(53:5).

The lipids selected for diagnosis may vary depending on whether the subject to be diagnosed is lean (e.g., has a BMI less than 27.5 kg/m²) or obese (e.g., has a BMI greater than or equal to than 27.5 kg/m²)and/or whether the reference level is based on measurements from a healthy subject (e.g., a subject that does not have NAFL, NASH, or liver fibrosis) and/or a subject clinically diagnosed with NASH or NAFL (e.g., diagnosed using liver biopsy or imaging) that is lean (e.g., has a BMI less than 27.5 kg/m²) or obese (e.g., has a BMI greater than or equal to than 27.5 kg/m²). If the reference level is based on measurements obtained from a healthy lean subject (e.g., a subject having a BMI less than 27.5 kg/m²) or a lean subject (e.g., a subject having a BMI less than 27.5 kg/m²) with NASH or NAFL, a population of healthy lean subjects (e.g., a population of healthy subjects having a BMI less than 27.5 kg/m²) or a population of lean subjects (e.g., subjects having a BMI less than 27.5 kg/m²) with NASH or NAFL, a mixed population of healthy lean (e.g., subjects having a BMI less than 27.5 kg/m²) and obese subjects (e.g., subjects having a BMI greater than or equal to than 27.5 kg/m²)or a mixed population of lean (e.g., subjects having a BMI less than 27.5 kg/m²) and obese subjects (e.g., subjects having a BMI greater than or equal to than 27.5 kg/m²)with NASH or NAFL , one or more healthy subjects or one or more subjects with NASH or NAFL whose weight was not recorded, or a mixed population of healthy subjects and subjects with NASH and NAFL of various weights, the lipids used for diagnosis may include all five of LPC(22:5), LPE(16:0), PC(35:3), PC(42:6), and PE(38:1); or five or more (e.g., 5, 6, or all 7) of DG(34:1), LPC(20:0e), PC(34:2e), PC(36:4), PC(36:5e), PC(40:8), and PE(38:1). In some embodiments, the lipids used for diagnosis may include five or more (e.g., 5, 6, 7, 8, or all 9) of LPC(22:5), LPE(16:0), PC(35:3), PC(42:6), PE(38:1), PC(34:1), PC(34:1e), SM(d35:1), and SM(d40:1); or five or more (e.g., 5, 6, 7, 8, 9, 10, 11, 12, 13, 14, 15, 16, 17, 18, or all 19) of LPC(22:5), LPE(16:0), PC(35:3), PC(42:6), PE(38:1), PC(34:1), PC(34:1e), SM(d35:1), Cer(d18:2_25:1), LPC(20:0e), PC (32:1e)=> *PC(16:0e_16:1),* PC (32:1e) => *PC(16:1e_16:0),* PC(34:0), PC(40:5e), PC(40:7), PE(38:6), SM(d38:1), TG(40:0), and TG(52:4). PC (32:1e)=> *PC(16:0e_16:1)* and PC (32:1e) => *PC(16:1e_16:0)* represent two different isomers of PC(32:1e) that contribute to the classification of subjects. In some embodiments, the lipids used for diagnosis may include five or more (e.g., 5, 6, 7, 8, or all 9) of DG(34:1), LPC(20:0e), PC(34:2e), PC(36:4), PC(36:5e), PC(40:8), PE(38:1), LPE(18:0), and PC(37:3); or five or more (e.g., 5, 6, 7, 8, 9, 10, 11, 12, 13, 14, 15, 16, 17, 18, or all 19) of DG(34:1), LPC(20:0e), PC(34:2e), PC(36:4), PC(36:5e), PC(40:8), PE(38:1), LPE(18:0), PC(37:3), AcCa(10:1), Cer(d43:0), DG(36:4), LPC(22:5), PC(36:3), PC(37:2), PE(34:1), SM(d40:1), TG(38:0), and TG(38:2). In some embodiments, the lipids used for diagnosis may include five or more (e.g., 5, 6, 7, 8, 9, 10, 11, 12, 13, 14, 15, 16, 17, 18, or all 19) of LPC(20:0e), LPC(22:5), DG(34:1), DG(36:4), LPE(16:0), PC(32:1e), PC(34:0), PC(34:2e), PC(35:3), PC(36:4), PC(36:5e), PC(37:2), PC(40:7), PC(40:8), PE(38:1), PE(38:6), SM(d40:1), TG(38:0), and TG(38:2). A high-sensitivity and high-specificity diagnosis can be performed by measuring the concentration of the following 29 lipids: DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5). These lipids may be used for diagnosis of a lean (e.g., a subject having a BMI less than 27.5 kg/m²) or obese subject (e.g., a subject having a BMI greater than or equal to than 27.5 kg/m²).

If the reference level is based measurements obtained from a healthy obese subject (e.g., a subject having a BMI greater than or equal to than 27.5 kg/m²) or a population of healthy obese subjects (e.g., obese subjects not having NASH or NAFL) and/or an obese subject (e.g., a subject having a BMI greater than or equal to than 27.5 kg/m²) clinically diagnosed with NASH or NAFL (e.g., diagnosed using liver biopsy or imaging) or a population of such subjects, the lipids used for diagnosis may include five or more (e.g., 5 or all 6) of DG(34:1), LPC(20:0e), LPC(22:5), PC(40:7), PE(34:1), and PE(34:3e); all five of LPC(20:0e), PC(34:1), PC(36:5e), PC(40:8), and PE(38:1); or five or more (e.g., 5 or all 6) of LPC(22:5), PC(34:1), PC(40:8), PE(34:1), PI(36:1), and SM(d40:1). In some embodiments, the lipids used for diagnosis may include five or more (e.g., 5, 6, 7, 8, 9, or all 10) of DG(34:1), LPC(20:0e), LPC(22:5), PC(40:7), PE(34:1), PE(34:3e), LPE(16:0), PC(35:2), Cer(d43:3), and DG(36:2); or five or more (e.g., 5, 6, 7, 8, 9, 10, 11, 12, 13, 14, 15, 16, 17, 18, or all 19) of DG(34:1), LPC(20:0e), LPC(22:5), PC(40:7), PE(34:1), PE(34:3e), LPE(16:0), PC(35:2), Cer(d43:3), DG(36:2), Cer(d34:0), DG(36:4), PC(34:0), PC(34:1), PC(36:2), PC(42:6), CO(36:1), TG(38:0), and TG(38:2). In some embodiments, the lipids used for diagnosis may include five or more (e.g., 5, 6, 7, 8, or all 9) of LPC(20:0e), PC(34:1), PC(36:5e), PC(40:8), PE(38:1), LPC(20:3), LPE(16:0), PC(34:2e), and SM(d32:0); or five or more (e.g., 5, 6, 7, 8, 9, 10, 11, 12, 13, 14, 15, 16, 17, 18, or all 19) of LPC(20:0e), PC(34:1), PC(36:5e), PC(40:8), PE(38:1), LPC(20:3), LPE(16:0), PC(34:2e), Cer(d34:0), DG (36:4), LPE(18:0), LPE(22:5), PC(34:0), PC(37:2), PC(40:7), PE(40:4e), SM(d32:0), SM(d36:0), and SM(d40:1). In some embodiments, the lipids used for diagnosis may include five or more (e.g., 5, 6, 7, or all 8) of LPC(22:5), PC(34:1), PC(40:8), PE(34:1), PI(36:1), SM(d40:1), LPE(16:0), and SM(d38:0); or five or more (e.g., 5, 6, 7, 8, 9, 10, 11, 12, 13, 14, 15, 16, 17, or all 18) of LPC(22:5), PC(34:1), PC(40:8), PE(34:1), PI(36:1), SM(d40:1), LPE(16:0), SM(d38:0), DG(34:2), DG(36:2), LPC(20:0e), LPE(18:0), PA(44:4), PC(34:2e), PC(36:3), PC(36:4), PE(40:04e), and SM(d35:1). In some embodiments, the lipids used for diagnosis may include five or more (e.g., 5, 6, 7, 8, 9, 10, 11, 12, 13, 14, 15, 16, or all 17) of DG(34:1), DG(36:4), LPC(20:0e), LPC(22:5), LPE(18:0), LPE(22:5), PA(44:4), SM(d32:0), PI(36:1), SM(d36:0), SM(d40:1), LPE(16:0), PC(34:0), PC(34:2e), PC(40:7), PC(40:8), and PE(40:4e). A high-sensitivity and high-specificity diagnosis can be performed by measuring the concentration of the following 29 lipids: DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0). These lipids may also be used for diagnosis of an obese subject (e.g., a subject having a BMI greater than or equal to than 27.5 kg/m²).

Lipids that can be measured to diagnose a subject with liver fibrosis include one or more (e.g., 1, 2, 3, 4, 5, 6, 7, 8, 9, or all 10) of DG(36:3), LPC(18:0), PC(36:2), PC(37:2), PC(40:5), TG(38:0), TG(50:0), TG(51:1), TG(57:1), and TG(60:2). Diagnosis may be performed by comparing the level or concentration of the one or more lipids in a sample (e.g., a blood, serum, or plasma sample) obtained from a subject to a reference level, such as the level of the one or more lipids in a negative control sample (e.g., a sample of the same type from a subject that does not have liver fibrosis or a previous sample of the same type collected when the subject was diagnosed as not having liver fibrosis) or the level of the one or more lipids in a positive control sample (e.g., a sample of the same type collected from a subject clinically diagnosed as having liver fibrosis (e.g., diagnosed using a liver biopsy or imaging)). The reference level may be based on measurements obtained from an individual subject (e.g., a healthy lean (e.g., a subject having a BMI less than 27.5 kg/m²) or obese subject (e.g., a subject having a BMI greater than or equal to than 27.5 kg/m²) without liver fibrosis, a subject with NAFL or NASH that does not have liver fibrosis, or a subject clinically diagnosed as having liver fibrosis) or a population of such subjects (e.g., a population of subjects without fibrosis, a population of subjects clinically diagnosed as having liver fibrosis, or a mixed population of subjects with and without liver fibrosis), or the reference level may be determined using a database containing measurements collected from such subjects.

### Diagnosis using glycans

Glycans are polysaccharides that may be homo- or heteropolymers of monosaccharide residues and can be linear or branched. Glycans can be found attached to proteins, such as glycoproteins and proteoglycans, typically on the exterior surface of cells. Measurement of glycans can reflect the ability of the liver to synthesize proteoglycans, a process that may be affected by diseases or conditions affecting the liver.

NAFL, NASH, and liver fibrosis can be diagnosed with high sensitivity and specificity based on glycan concentrations in a biological sample (e.g., a blood, serum, or plasma sample) obtained from the subject. Diagnosis can be performed by comparing glycan concentrations in the biological sample (e.g., a blood, serum, or plasma sample) obtained from the subject to glycan concentrations in a negative control sample of the same type (e.g., a blood, serum, or plasma sample) obtained from one or more subjects not having NASH, NAFL, or liver fibrosis or to one or more biological samples obtained from the subject (e.g., a blood, serum, or plasma sample) prior to the development of NAFLD. A subject may also be diagnosed by comparing a biological sample obtained from the subject (e.g., a blood, serum, or plasma sample) to a positive control, such as a biological sample (e.g., a blood, serum, or plasma sample) obtained from a subject clinically diagnosed with NASH, NAFL, or liver fibrosis (e.g., diagnosed using a liver biopsy or imaging). The biological sample from the subject may be compared directly to a reference level, which may be based on a negative control sample (e.g., a negative control sample from a subject not having NASH, NAFL, or liver fibrosis, e.g., a negative control sample obtained at the same or at a similar time to the biological sample obtained from the subject, or negative a control sample collected from the subject at an earlier time point when the subject was deemed healthy), a positive control sample (a biological sample of the same type (e.g., a blood, serum, or plasma sample) obtained from a subject clinically diagnosed with NASH, NAFL, or liver fibrosis), or a reference sample (e.g., glycan concentrations in a database obtained using samples collected from for one or more subjects not having NASH, NAFL, or liver fibrosis and/or samples collected from one or more subjects having clinically diagnosed NASH, NAFL, or liver fibrosis (e.g., diagnosed using a liver biopsy or imaging)). Glycans can be identified using GC-MS or glycoblotting. Exemplary glycans identified in the serum of healthy, NAFL, and NASH patients are shown in FIGS. 4A and 4C. Glycans found to have diagnostic value for use in the methods described herein are shown in Table 1, below.

**Table 1. Glycans that can be used for diagnosis of NASH, NAFL, or liver fibrosis**

| **Mass (m/z)** | **N-glycan composition** | **Most likely structure** |
|---|---|---|
| **Glycans for diagnosis of NAFL or NASH** | | |
| 1661 | Hex3HexNAc4 | 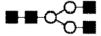 |
| 1988 | Hex7HexNAc2 | 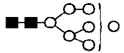 |
| 2592 | Hex5HexNAc4Fuc3 | 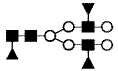 |
| 2764 | Hex6HexNAc6 | 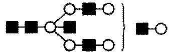 |
| 2968 | Hex7HexNAc6 | 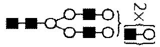 |
| 3953 | Hex8HexNAc7Fuc1 NeuAc1 | 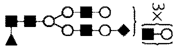 |
| 4039 | Hex7HexNAc6Fuc2NeuAc2 | 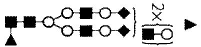 |

| **Glycans for diagnosis of liver fibrosis** | | |
|---|---|---|
| 2081 | Hex3HexNAc5Fuc1 | 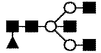 |
| 2315 | Hex5HexNAc5 | 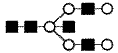 |
| 2592 | Hex5HexNAc4Fuc3 | 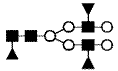 |
| 2605 | Hex5HexNAc4Fuc1 NeuAc1 | 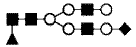 |
| 4587 | Hex7HexNAc6Fuc1NeuAc4 | 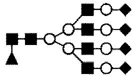 |

| | | |
|---|---|---|
| ○ : Galactose □ : N-acetyl-galactosamine ■ : N-acetyl-glucosamine ● : Mannose ◄ : Fucose ◆ : Sialic Acid (N-acetyl-neuraminic acid) ■-○ : PolylacNAc | | |

Glycans that can be measured to diagnose a subject with NAFL or NASH include one or more (e.g., 1, 2, 3, 4, 5, or more) of 1661, 1988, 2592, 2764, 2968, 3953, and 4039. In some embodiments, the glycans used for diagnosis include 2592, 2764, and 2968.

The glycans selected for diagnosis may vary depending on whether the reference level is based on measurements obtained from a healthy subject (e.g., a subject that does not have NAFL,NASH, or liver fibrosis) and/or a subject clinically diagnosed with NASH or NAFL (e.g., diagnosed using liver biopsy or imaging) that is lean (e.g., has a BMI less than 27.5 kg/m²) or obese (e.g., has a BMI greater than or equal to than 27.5 kg/m²). If the reference level is based on measurements obtained from a healthy lean subject (e.g., a subject having a BMI less than 27.5 kg/m²) or a lean subject (e.g., a subject having a BMI less than 27.5 kg/m²) with NASH or NAFL, a population of healthy lean subjects (e.g., healthy subjects having a BMI less than 27.5 kg/m²) or a population of lean subjects (e.g., subjects having a BMI less than 27.5 kg/m²) with NASH or NAFL, a mixed population of healthy lean (e.g., subjects having a BMI less than 27.5 kg/m²) and obese subjects (e.g., subjects having a BMI greater than or equal to than 27.5 kg/m²)or a mixed population of lean (e.g., subjects having a BMI less than 27.5 kg/m²) and obese subjects (e.g., subjects having a BMI greater than or equal to than 27.5 kg/m²) with NASH or NAFL , one or more healthy subjects or one or more subjects with NASH or NAFL whose weight was not recorded, or a mixed population of healthy subjects and subjects with NASH and NAFL of various weights, the one or more glycans may include 1661, 2592, 2764, 2968, and 4039. In some embodiments, all five of 1661, 2592, 2764, 2968, and 4039 are used for diagnosis. These glycans may be used for diagnosis of a lean (e.g., a subject having a BMI less than 27.5 kg/m²) or obese subject (e.g., a subject having a BMI greater than or equal to than 27.5 kg/m²).

If the reference level is based on measurements is obtained from a healthy obese subject (e.g., a subject having a BMI greater than or equal to than 27.5 kg/m²) or a population of healthy obese subjects (e.g., obese subjects not having NASH or NAFL) and/or an obese subject (e.g., a subject having a BMI greater than or equal to than 27.5 kg/m²) clinically diagnosed with NASH or NAFL (e.g., diagnosed using liver biopsy or imaging) or a population of such subjects, the one or more glycans may include 1988, 2592, 2764, 2968, and 3953. In some embodiments, all five of 1988, 2592, 2764, 2968, and 3953 are used for diagnosis. These glycans may also be used for diagnosis of an obese subject (e.g., a subject having a BMI greater than or equal to than 27.5 kg/m²).

Glycans that can be measured to diagnose a subject with liver fibrosis include one or more (e.g., 1, 2, 3, 4, or all 5) of 2081, 2315, 2592, 2605, and 4587. In some embodiments, all five of 2081, 2315, 2592, 2605, and 4587 are used for diagnosis. Diagnosis may be performed by comparing the level or concentration of the one or more glycans in a sample (e.g., a blood, serum, or plasma sample) obtained from a subject to a reference level, such as the level of the one or more glycans in a negative control sample (e.g., a sample of the same type from a subject that does not have liver fibrosis or a previous sample of the same type collected when the subject was diagnosed as not having liver fibrosis) or the level of the one or more lipids in a positive control sample (e.g., a sample of the same type collected from a subject clinically diagnosed as having liver fibrosis (e.g., diagnosed using a liver biopsy or imaging)). The reference level may be based on measurements obtained from an individual subject (e.g., a healthy lean (e.g., a subject having a BMI less than 27.5 kg/m²) or obese subject (e.g., a subject having a BMI greater than or equal to than 27.5 kg/m²) without liver fibrosis, a subject with NAFL or NASH that does not have liver fibrosis, or a subject clinically diagnosed as having liver fibrosis) or a population of such subjects (e.g., a population of subjects without fibrosis, a population of subjects clinically diagnosed as having liver fibrosis, or a mixed population of subjects with and without liver fibrosis), or the reference level may be determined using a database containing measurements collected from such subjects.

### Diagnosis using fatty acids

Fatty acids are carboxylic acids with a long aliphatic chain that can be either saturated or unsaturated. Fatty acids serve as dietary sources of fuel for animals and important structural components of cells. Fatty acids are often characterized by length and are typically defined as short-chain (fatty acids with aliphatic tails of five or fewer carbons), medium-chain (fatty acids with aliphatic tails of 6-12 carbons), long-chain (fatty acids with aliphatic tails of 13-21 carbons), or very long chain (fatty acids with aliphatic tails of 22 or more carbons) fatty acids. They can also be classified as saturated or unsaturated based on the absence (saturated) or presence (unsaturated) of C=C double bonds. Fatty acids can be named using a variety of naming systems, such as trivial names (e.g., common names) or lipid numbers, which take the form of C:D in which C is the number of carbon atoms in the fatty acid and D is the number of double bonds in the fatty acid. For example, the lipid numbers for palmitic acid, cis-palmitoleic acid, oleic acid, polyunsaturated linoleic fatty acid, and arachidonic fatty acid are C16:0, C16:1n7cis, C18:1n9cis, C18:2n6, and C20:4n6, respectively.

NAFL, NASH, and liver fibrosis can be diagnosed with high sensitivity and specificity based on free fatty acid concentrations in a biological sample (e.g., a blood, serum, or plasma sample) obtained from the subject. Diagnosis can be performed by comparing fatty acid concentrations in the biological sample (e.g., a blood, serum, or plasma sample) obtained from the subject to fatty acid concentrations in a negative control sample of the same type (e.g., a blood, serum, or plasma sample) obtained from one or more subjects not having NASH, NAFL, or liver fibrosis or to one or more biological samples obtained from the subject (e.g., a blood, serum, or plasma sample) prior to the development of NAFLD. A subject may also be diagnosed by comparing a biological sample obtained from the subject (e.g., a blood, serum, or plasma sample) to a positive control, such as a biological sample (e.g., a blood, serum, or plasma sample) obtained from a subject clinically diagnosed with NASH, NAFL, or liver fibrosis (e.g., diagnosed using a liver biopsy or imaging). The biological sample from the subject may be compared directly to a reference level, which may be based on a negative control sample (e.g., a negative control sample from a subject not having NASH, NAFL, or liver fibrosis, e.g., a negative control sample obtained at the same or at a similar time to the biological sample obtained from the subject, or negative a control sample collected from the subject at an earlier time point when the subject was deemed healthy), a positive control sample (a biological sample of the same type (e.g., a blood, serum, or plasma sample) obtained from a subject clinically diagnosed with NASH, NAFL, or liver fibrosis), or a reference sample (e.g., fatty acid concentrations in a database obtained using samples collected from for one or more subjects not having NASH, NAFL, or liver fibrosis and/or samples collected from one or more subjects having clinically diagnosed NASH, NAFL, or liver fibrosis (e.g., diagnosed using a liver biopsy or imaging)). Fatty acids can be identified using gas chromatography or mass spectrometry (MS).

Fatty acids that can be measured to diagnose a subject with NAFL or NASH include one or more (e.g., 1, 2, 3, 4, 5 or more) of C18:2n6, C18:3n6, C20:0, C20:4n6, C22:4n6, C16:0, and C18:1n9cis. In some embodiments, the fatty acids used for diagnosis include C18:2n6, C18:3n6, and C22:4n6.

The fatty acids selected for diagnosis may vary depending on whether the reference level is based on measurements obtained from a healthy subject (e.g., a subject that does not have NAFL, NASH, or liver fibrosis) and/or a subject clinically diagnosed with NASH or NAFL (e.g., diagnosed using liver biopsy or imaging) that is lean (e.g., has a BMI less than 27.5 kg/m²) or obese (e.g., has a BMI greater than or equal to than 27.5 kg/m²). If the reference level is based on measurements obtained from a healthy lean subject (e.g., a subject having a BMI less than 27.5 kg/m²) or a lean subject (e.g., a subject having a BMI less than 27.5 kg/m²) with NASH or NAFL, a population of healthy lean subjects (e.g., healthy subjects having a BMI less than 27.5 kg/m²) or a population of lean subjects (e.g., subjects having a BMI less than 27.5 kg/m²) with NASH or NAFL, a mixed population of healthy lean (e.g., subjects having a BMI less than 27.5 kg/m²) and obese subjects (e.g., subjects having a BMI greater than or equal to than 27.5 kg/m²)or a mixed population of lean (e.g., subjects having a BMI less than 27.5 kg/m²) and obese subjects (e.g., subjects having a BMI greater than or equal to than 27.5 kg/m²) with NASH or NAFL , one or more healthy subjects or one or more subjects with NASH or NAFL whose weight was not recorded, or a mixed population of healthy subjects and subjects with NASH and NAFL of various weights, the one or more fatty acids may include C18:2n6, C18:3n6, C20:0, C20:4n6, C22:4n6. In some embodiments, all five of C18:2n6, C18:3n6, C20:0, C20:4n6, C22:4n6 are used for diagnosis. These fatty acids may be used for diagnosis of a lean (e.g., a subject having a BMI less than 27.5 kg/m²) or obese subject (e.g., a subject having a BMI greater than or equal to than 27.5 kg/m²).

If the reference level is based on measurements obtained from a healthy obese subject (e.g., a subject having a BMI greater than or equal to than 27.5 kg/m²) or a population of healthy obese subjects (e.g., obese subjects not having NASH or NAFL) and/or an obese subject (e.g., a subject having a BMI greater than or equal to than 27.5 kg/m²) clinically diagnosed with NASH or NAFL (e.g., diagnosed using liver biopsy or imaging) or a population of such subjects, the one or more fatty acids may include C18:2n6, C18:3n6, C22:4n6, C16:0, and C18:1n9cis. In some embodiments, all five of C18:2n6, C18:3n6, C22:4n6, C16:0, and C18:1n9cis are used for diagnosis. These fatty acids may also be used for diagnosis of an obese subject.

Fatty acids that can be measured to diagnose a subject with liver fibrosis include one or more (e.g., 1, 2, 3, 4, or all 5) of C14:0, C18:1n9trans, C18:3n3, C20:3n6, and C22:6n3. In some embodiments, all five of C14:0, C18:1n9trans, C18:3n3, C20:3n6, and C22:6n3 are used for diagnosis. Diagnosis may be performed by comparing the level or concentration of the one or more fatty acids in a sample (e.g., a blood, serum, or plasma sample) obtained from a subject to a reference level, such as the level of the one or more fatty acids in a negative control sample (e.g., a sample of the same type from a subject that does not have liver fibrosis or a previous sample of the same type collected when the subject was diagnosed as not having liver fibrosis) or the level of the one or more lipids in a positive control sample (e.g., a sample of the same type collected from a subject clinically diagnosed as having liver fibrosis (e.g., diagnosed using a liver biopsy or imaging)). The reference level may be based on measurements obtained from an individual subject (e.g., a healthy lean (e.g., a subject having a BMI less than 27.5 kg/m²) or obese subject (e.g., a subject having a BMI greater than or equal to than 27.5 kg/m²) without liver fibrosis, a subject with NAFL or NASH that does not have liver fibrosis, or a subject clinically diagnosed as having liver fibrosis) or a population of such subjects (e.g., a population of subjects without fibrosis, a population of subjects clinically diagnosed as having liver fibrosis, or a mixed population of subjects with and without liver fibrosis), or the reference level may be determined using a database containing measurements collected from such subjects.

### Diagnosis using hormones

Hormones are signaling molecules produced by glands or organs that bind to receptors in target cells in the body to regulate a diverse array of behavioral and physiological activities, such as metabolism, digestion, respiration, tissue function, sleep, excretion, lactation, sensory perception, stress induction, growth and development, movement, reproduction, and mood. Hormone signaling is typically characterized as endocrine, paracrine, autocrine, or intracrine.

NAFL and NASH can be diagnosed with high sensitivity and specificity based on hormone concentrations in a biological sample (e.g., a blood, serum, or plasma sample) obtained from the subject. Diagnosis can be performed by comparing hormone concentrations in the biological sample (e.g., a blood, serum, or plasma sample) obtained from the subject to hormone concentrations in a negative control sample of the same type (e.g., a blood, serum, or plasma sample) obtained from one or more subjects not having NASH or NAFL or to one or more biological samples obtained from the subject (e.g., a blood, serum, or plasma sample) prior to the development of NAFLD. A subject may also be diagnosed by comparing a biological sample obtained from the subject (e.g., a blood, serum, or plasma sample) to a positive control, such as a biological sample (e.g., a blood, serum, or plasma sample) obtained from a subject clinically diagnosed with NASH, NAFL, or liver fibrosis (e.g., diagnosed using a liver biopsy or imaging). The biological sample from the subject may be compared directly to a reference level, which may be based on a negative control sample (e.g., a negative control sample from a subject not having NASH, NAFL, or liver fibrosis, e.g., a negative control sample obtained at the same or at a similar time to the biological sample obtained from the subject, or negative a control sample collected from the subject at an earlier time point when the subject was deemed healthy), a positive control sample (a biological sample of the same type (e.g., a blood, serum, or plasma sample) obtained from a subject clinically diagnosed with NASH, NAFL, or liver fibrosis), or a reference sample (e.g., hormone concentrations in a database obtained using samples collected from for one or more subjects not having NASH, NAFL, or liver fibrosis and/or samples collected from one or more subjects having clinically diagnosed NASH, NAFL, or liver fibrosis (e.g., diagnosed using a liver biopsy or imaging)). Hormones can be identified using a radioimmunoassay or enzyme-linked immunosorbent assay (ELISA).

Hormones that can be measured to diagnose a subject with NAFL or NASH include two or more (e.g., 2, 3, or all 4) of leptin, adiponectin, activin A, follistatin, the ratio of activin A to follistatin (activin A/follistatin), and one or more triglycerides (e.g., the total circulating triglycerides in blood). In some embodiments, all of leptin, adiponectin, activin A, follistatin, the ratio of activin A to follistatin (activin A/follistatin), and one or more triglycerides (e.g., the total circulating triglycerides in blood) are used for diagnosis. In some embodiments, the combination of leptin, adiponectin, the ratio of activin A to follistatin, and total circulating triglycerides are used for diagnosis. The same set of hormones can be used regardless of the weight of the subject to be diagnosed and the weight of the subject(s) from which the reference level was measured.

### Diagnosis using a combination of lipids, glycans, and hormones

In addition to using measurements of one or more lipids, glycans, or hormones individually to diagnose a subject with NAFL or NASH, it is also possible to achieve high diagnostic sensitivity and specificity by measuring combinations of lipids and glycans, lipids and hormones, or lipids, glycans, and hormones in a biological sample (e.g., a blood, serum, or plasma sample) obtained from the subject. Diagnosis can be performed similarly to diagnosis performed as described above for lipids, glycans, and hormones.

One combination of measurements that can be used to diagnose a subject with NASH or NAFL is a combination of lipids and glycans. Similar to diagnostic methods based on lipids or glycans alone, the lipids and glycans selected for diagnosis may vary depending on whether the reference level is based on measurements obtained from a healthy subject (e.g., a subject that does not have NAFL, NASH, or liver fibrosis) and/or a subject clinically diagnosed with NASH or NAFL (e.g., diagnosed using liver biopsy or imaging) that is lean (e.g., has a BMI less than 27.5 kg/m²) or obese (e.g., has a BMI greater than or equal to than 27.5 kg/m²). If the reference level is based on measurements obtained from a healthy lean subject (e.g., a subject having a BMI less than 27.5 kg/m²) or a lean subject (e.g., a subject having a BMI less than 27.5 kg/m²) with NASH or NAFL, a population of healthy lean subjects (e.g., healthy subjects having a BMI less than 27.5 kg/m²) or a population of lean subjects (e.g., subjects having a BMI less than 27.5 kg/m²) with NASH or NAFL, a mixed population of healthy lean (e.g., subjects having a BMI less than 27.5 kg/m²) and obese subjects (e.g., subjects having a BMI greater than or equal to than 27.5 kg/m²) or a mixed population of lean (e.g., subjects having a BMI less than 27.5 kg/m²) and obese subjects (e.g., subjects having a BMI greater than or equal to than 27.5 kg/m²) with NASH or NAFL, one or more healthy subjects or one or more subjects with NASH or NAFL whose weight was not recorded, or a mixed population of healthy subjects and subjects with NASH and NAFL of various weights, the lipids used for diagnosis may include at least five (e.g., 5, 6, 7, or all 8) of LPC(22:5), LPE(16:0), PC(35:3), PC(42:6), PE(38:1), PC(34:1), PC(34:1e), and SM(d35:1). In some embodiments, the lipids used for diagnosis further includes SM(d40:1) or further includes one or more (e.g., 1, 2, 3, 4, 5, 6, 7, 8, 9, 10, or all 11) of Cer(d18:2_25:1), LPC(20:0e), PC (32: 1e)=> *PC(16:0e_16:1),* PC (32:1e) => *PC(16:1e_16:0),* PC(34:0), PC(40:5e), PC(40:7), PE(38:6), SM(d38:1), TG(40:0), and TG(52:4). In some embodiments, the lipids used for diagnosis includes five or more (e.g., 5, 6, 7, 8, 9, or all 10) of LPC(20:0e), LPC(22:5), LPE(16:0), PC(32:1e), PC(34:0), PC(35:3), PC(40:7), PE(38:1), PE(38:6), and SM(d40:1). In addition, one or more (e.g., 1, 2, 3, 4, or all 5) glycans can be evaluated from the group consisting of 1661, 2592, 2764, 2968, and 4039. The glycan used for diagnosis in combination with at least 5 of the aforementioned lipids can be 2592. For example, the combination of lipids and glycans used for diagnosis of NAFL or NASH in a subject can include LPC(22:5), LPE(16:0), PC(35:3), PC(42:6), PE(38:1), PC(34:1), PC(34:1e), SM(d35:1), SM(d40:1), and 2592. In another example, the combination of lipids and glycans used for diagnosis of NAFL or NASH in a subject can include LPC(22:5), LPE(16:0), PC(35:3), PC(42:6), PE(38:1), PC(34:1), PC(34:1e), SM(d35:1), Cer(d18:2_25:1), LPC(20:0e), PC (32: 1e)=> *PC(16:0e_16:1),* PC (32:1e) => *PC(16:1e_16:0),* PC(34:0), PC(40:5e), PC(40:7), PE(38:6), SM(d38:1), TG(40:0), TG(52:4), and 2592. These combinations of lipids and glycans may be used for diagnosis of a lean (e.g., a subject having a BMI less than 27.5 kg/m²) or obese subject (e.g., a subject having a BMI greater than or equal to than 27.5 kg/m²).

If the reference level is based on measurements obtained from a healthy obese subject (e.g., a subject having a BMI greater than or equal to than 27.5 kg/m²) or a population of healthy obese subjects (e.g., obese subjects not having NASH or NAFL) and/or an obese subject (e.g., a subject having a BMI greater than or equal to than 27.5 kg/m²) clinically diagnosed with NASH or NAFL (e.g., diagnosed using liver biopsy or imaging) or a population of such subjects, the combination of lipids and glycans used for diagnosis of NAFL and NASH may differ. For example, if the control sample is obtained from a healthy obese subject (e.g., a subject having a BMI greater than or equal to than 27.5 kg/m²) and/or an obese subject (e.g., a subject having a BMI greater than or equal to than 27.5 kg/m²) clinically diagnosed with NASH or NAFL or populations thereof, the lipids used for diagnosis may include at least five (e.g., 5, 6, 7, 8, or all 9) of DG(34:1), LPC(20:0e), LPC(22:5), PC(40:7), PE(34:1), PE(34:3e), Cer(d43:3), LPE(16:0), and PC(35:2). In some embodiments, the lipids used for diagnosis further includes DG(36:2) or further includes one or more (e.g., 1, 2, 3, 4, 5, 6, 7, 8, or all 9) of Cer(d34:0), DG(36:4), PC(34:0), PC(34:1), PC(36:2), PC(42:6), CO(36:1), TG(38:0), and TG(38:2). In some embodiments, the lipids used for diagnosis includes five or more (e.g., 5, 6, or all 7) of DG(34:1), DG(36:4), LPC(20:0e), LPC(22:5), LPE(16:0), PC(34:0), and PC(40:7). In addition, one or more (e.g., 1, 2, 3, 4, or all 5) glycans can be evaluated from the group consisting of 1988, 2592, 2764, 2968, and 3953. In one example, the glycan used for diagnosis in combination with at least 5 of the aforementioned lipids can be 2592. In another example, both 1988 and 2592 can be used for diagnosis in combination with at least 5 of the aforementioned lipids. An exemplary combination of lipids and glycans for diagnosis of NAFL or NASH in a subject in comparison to a reference level obtained from measurements taken from a healthy obese subject (e.g., a subject having a BMI greater than or equal to than 27.5 kg/m²) and/or an obese subject (e.g., a subject having a BMI greater than or equal to than 27.5 kg/m²) clinically diagnosed with NASH or NAFL can include DG(34:1), LPC(20:0e), LPC(22:5), PC(40:7), PE(34:1), PE(34:3e), LPE(16:0), PC(35:2), Cer(d43:3), and DG(36:2). Another possible combination of lipids and glycans that can be used for diagnosis of NAFL or NASH in a subject in comparison to a reference level from a healthy obese subject (e.g., a subject having a BMI greater than or equal to than 27.5 kg/m²) and/or an obese subject (e.g., a subject having a BMI greater than or equal to than 27.5 kg/m²) clinically diagnosed with NASH or NAFL can include LPC(22:5), LPE(16:0), PC(35:3), PC(42:6), PE(38:1), PC(34:1), PC(34:1e), SM(d35:1), Cer(d18:2_ DG(34:1), LPC(20:0e), LPC(22:5), PC(40:7), PE(34:1), PE(34:3e), LPE(16:0), PC(35:2), Cer(d43:3), DG(36:2), Cer(d34:0), DG(36:4), PC(34:0), PC(34:1), PC(36:2), PC(42:6), CO(36:1), TG(38:0), and TG(38:2), 1988, and 2592. These combinations of lipids and glycans may also be used for diagnosis of an obese subject (e.g., a subject having a BMI greater than or equal to than 27.5 kg/m²).

A second combination of measurements that can be used to diagnose a subject with NASH or NAFL is a combination of lipids and hormones. Similar to diagnostic methods based on lipids or hormones alone, the lipids and hormones selected for diagnosis may vary depending on whether the reference level is based on measurements obtained from a healthy subject (e.g., a subject that does not have NAFL, NASH, or liver fibrosis) and/or a subject clinically diagnosed with NASH or NAFL (e.g., diagnosed using liver biopsy or imaging) that is lean (e.g., has a BMI less than 27.5 kg/m²) or obese (e.g., has a BMI greater than or equal to than 27.5 kg/m²). If the reference level is based on measurements obtained from a healthy lean subject (e.g., a subject having a BMI less than 27.5 kg/m²) or a lean subject (e.g., a subject having a BMI less than 27.5 kg/m²) with NASH or NAFL, a population of healthy lean subjects (e.g., healthy subjects having a BMI less than 27.5 kg/m²) or a population of lean subjects (e.g., subjects having a BMI less than 27.5 kg/m²) with NASH or NAFL, a mixed population of healthy lean (e.g., subjects having a BMI less than 27.5 kg/m²) and obese subjects (e.g., subjects having a BMI greater than or equal to than 27.5 kg/m²) or a mixed population of lean (e.g., subjects having a BMI less than 27.5 kg/m²) and obese subjects (e.g., subjects having a BMI greater than or equal to than 27.5 kg/m²) with NASH or NAFL , one or more healthy subjects or one or more subjects with NASH or NAFL whose weight was not recorded, or a mixed population of healthy subjects and subjects with NASH and NAFL of various weights, the lipids used for diagnosis may include at least five (e.g., 5, 6, 7, 8, or all 9) of DG(34:1), LPC(20:0e), PC(34:2e), PC(36:4), PC(36:5e), PC(40:8), PE(38:1), LPE(18:0), and PC(37:3). In some embodiments, the lipids used for diagnosis further includes one or more (e.g., 1, 2, 3, 4, 5, 6, 7, 8, 9, or all 10) of AcCa(10:1), Cer(d43:0), DG(36:4), LPC(22:5), PC(36:3), PC(37:2), PE(34:1), SM(d40:1), TG(38:0), and TG(38:2). In some embodiments, the lipids used for diagnosis include five or more (e.g., 5, 6, 7, 8, 9, 10, 11, 12, or all 13) of LPC(20:0e), LPC(22:5), DG(34:1), DG(36:4), PC(34:2e), PC(36:4), PC(36:5e), PC(37:2), PC(40:8), PE(38:1), SM(d40:1), TG(38:0), and TG(38:2). In addition, one or more (e.g., 1, 2, 3, or all 4) hormones can be evaluated from the group consisting of leptin, adiponectin, activin A/follistatin, and triglycerides. The hormone used for diagnosis in combination with at least 5 of the aforementioned lipids can be adiponectin. For example, the combination of lipids and hormones used for diagnosis of NAFL or NASH in a subject can include DG(34:1), LPC(20:0e), PC(34:2e), PC(36:4), PC(36:5e), PC(40:8), PE(38:1), LPE(18:0), PC(37:3), and adiponectin. In another example, the combination of lipids and hormones used for diagnosis of NAFL or NASH in a subject can include DG(34:1), LPC(20:0e), PC(34:2e), PC(36:4), PC(36:5e), PC(40:8), PE(38:1), LPE(18:0), PC(37:3), AcCa(10:1), Cer(d43:0), DG(36:4), LPC(22:5), PC(36:3), PC(37:2), PE(34:1), SM(d40:1), TG(38:0), and TG(38:2), and adiponectin. These combinations of lipids and hormones may be used for diagnosis of a lean (e.g., a subject having a BMI less than 27.5 kg/m²) or obese subject (e.g., a subject having a BMI greater than or equal to than 27.5 kg/m²).

If the reference level is based on measurements obtained from a healthy obese subject (e.g., a subject having a BMI greater than or equal to than 27.5 kg/m²) or a population of healthy obese subjects (e.g., obese subjects not having NAFL or NASH) and/or an obese subject (e.g., a subject having a BMI greater than or equal to than 27.5 kg/m²) clinically diagnosed with NASH or NAFL (e.g., diagnosed using liver biopsy or imaging) or a population of such subjects, the combination of lipids and hormones used for diagnosis of NAFL and NASH may differ. For example, if the reference level is based on measurements obtained from an obese subject (e.g., a subject having a BMI greater than or equal to than 27.5 kg/m²) and/or an obese subject (e.g., a subject having a BMI greater than or equal to than 27.5 kg/m²) clinically diagnosed with NASH or NAFL or populations thereof, the lipids used for diagnosis may include at least five (e.g., 5, 6, 7, 8, or all 9) of LPC(20:0e), PC(34:1), PC(36:5e), PC(40:8), PE(38:1), LPC(20:3), LPE(16:0), PC(34:2e), and SM(d32:0). In some embodiments, the lipids used for diagnosis further includes one or more (e.g., 1, 2, 3, 4, 5, 6, 7, 8, 9, or all 10) of Cer(d34:0), DG (36:4), LPE(18:0), LPE(22:5), PC(34:0), PC(37:2), PC(40:7), PE(40:4e), SM(d36:0), and SM(d40:1). In some embodiments, the lipids used for diagnosis include five or more (e.g., 5, 6, 7, 8, 9, 10, 11, or all 12) of DG(36:4), LPC(20:0e), LPE(18:0), LPE(22:5), SM(d32:0), SM(d36:0), SM(d40:1), LPE(16:0), PC(34:0), PC(34:2e), PC(40:7), and PE(40:4e). In addition, one or more (e.g., 1, 2, 3, or all 4) hormones can be evaluated from the group consisting of leptin, adiponectin, activin A/follistatin, and triglycerides. The hormone used for diagnosis in combination with at least 5 of the aforementioned lipids can be adiponectin. An exemplary combination of lipids and hormones for diagnosis of NAFL or NASH in a subject in comparison to a reference level based on measurements obtained from a healthy obese subject (e.g., a subject having a BMI greater than or equal to than 27.5 kg/m²) and/or an obese subject (e.g., a subject having a BMI greater than or equal to than 27.5 kg/m²) clinically diagnosed with NASH or NAFL can include LPC(20:0e), PC(34:1), PC(36:5e), PC(40:8), PE(38:1), LPC(20:3), LPE(16:0), PC(34:2e), and SM(d32:0), and adiponectin. Another possible combination of lipids and hormones that can be used for diagnosis of NAFL or NASH in a subject in comparison to a reference level based on measurements obtained from a healthy obese subject (e.g., a subject having a BMI greater than or equal to than 27.5 kg/m²) and/or an obese subject (e.g., a subject having a BMI greater than or equal to than 27.5 kg/m²) clinically diagnosed with NASH or NAFL can include LPC(20:0e), PC(34:1), PC(36:5e), PC(40:8), PE(38:1), LPC(20:3), LPE(16:0), PC(34:2e), SM(d32:0), Cer(d34:0), DG (36:4), LPE(18:0), LPE(22:5), PC(34:0), PC(37:2), PC(40:7), PE(40:4e), SM(d36:0), SM(d40:1), and adiponectin. These combinations of lipids and hormones may also be used for diagnosis of an obese subject (e.g., a subject having a BMI greater than or equal to than 27.5 kg/m²).

A third combination of measurements that can be used to diagnose a subject with NASH or NAFL is a combination of lipids, glycans, and hormones. This approach can be used if the reference level based on measurements obtained from a healthy obese subject (e.g., a subject having a BMI greater than or equal to than 27.5 kg/m²) or a population of healthy obese subjects (e.g., obese subjects not having NASH or NAFL) and/or an obese subject (e.g., a subject having a BMI greater than or equal to than 27.5 kg/m²) clinically diagnosed with NASH or NAFL (e.g., diagnosed using liver biopsy or imaging) or a population of such subjects. For this diagnostic method, the lipids used for diagnosis may include at least five (e.g., 5, 6, 7, or all 8) of LPC(22:5), PC(34:1), PC(40:8), PE(34:1), PI(36:1), SM(d40:1), LPE(16:0), and SM(d38:0). In some embodiments, the lipids used for diagnosis further includes one or more (e.g., 1, 2, 3, 4, 5, 6, 7, 8, 9, or all 10) of DG(34:2), DG(36:2), LPC(20:0e), LPE(18:0), PA(44:4), PC(34:2e), PC(36:3), PC(36:4), PE(40:04e), and SM(d35:1). In some embodiments, the lipids used for diagnosis includes five or more (e.g., 5, 6, 7, 8, 9, or all 10) of LPC(20:0e), LPC(22:5), LPE(18:0), PA(44:4), PI(36:1), SM(d40:1), LPE(16:0), PC(34:2e), PC(40:8), and PE(40:4e). In addition, one or more (e.g., 1, 2, 3, 4, or all 5) glycans can be evaluated from the group consisting of 1988, 2592, 2764, 2968, and 3953. In one example, the glycan used for diagnosis in combination with at least 5 of the aforementioned lipids and a hormone can be 2592. The method further includes evaluating one or more (e.g., 1, 2, 3, or all 4) hormone from the group consisting of leptin, adiponectin, activin A/follistatin, and triglycerides. The hormone used for diagnosis in combination with at least 5 of the aforementioned lipids and a glycan can be adiponectin. For example, the combination of lipids, glycans, and hormones used for diagnosis of NAFL or NASH in a subject can include LPC(22:5), PC(34:1), PC(40:8), PE(34:1), PI(36:1), SM(d40:1), LPE(16:0), SM(d38:0), 2592, and adiponectin. In another example, the combination of lipids, glycans, and hormones used for diagnosis of NAFL or NASH in a subject can include LPC(22:5), PC(34:1), PC(40:8), PE(34:1), PI(36:1), SM(d40:1), LPE(16:0), SM(d38:0), DG(34:2), DG(36:2), LPC(20:0e), LPE(18:0), PA(44:4), PC(34:2e), PC(36:3), PC(36:4), PE(40:04e), SM(d35:1), 2592, and adiponectin. These combinations of lipids, glycans, and hormones can also be used for diagnosis of an obese subject (e.g., a subject having a BMI greater than or equal to than 27.5 kg/m²).

### Subject selection and sample collection

Subjects that can be diagnosed according to the methods described herein are subjects at risk of developing NAFL, NASH, or liver fibrosis (e.g., subjects presenting with one or more features of metabolic syndrome, such as obesity, diabetes (e.g., Type 2 diabetes), insulin resistance, increased waist circumference, low HDL cholesterol, high LDL cholesterol, or higher than normal blood glucose levels, or subjects with a family history of NAFL, NASH, or liver fibrosis), subjects undergoing treatment for NAFL, NASH, or liver fibrosis, subjects previously treated for NAFL, NASH, or liver fibrosis, subjects suspected of having NAFL, NASH, or fibrosis (e.g., subjects found to have elevated liver enzyme levels or subjects in which steatosis has been observed in imaging (e.g., ultrasonography)), and subjects diagnosed with NAFL, NASH, or liver fibrosis using a different diagnostic test (e.g., imaging or liver biopsy).

A sample containing a biomarker described herein (e.g., a lipid, glycan, hormone, and/or fatty acid) can be obtained using methods well known in the art. For instance, samples from a subject may be obtained by venipuncture or from blood, such as serum or plasma. Biomarkers (e.g., lipids, glycans, hormones, and/or fatty acids) can be detected from these samples. The ease of collection of such samples makes this diagnostic approach available to many more patients than previous diagnostic methods, such as liver biopsy, as diagnosis can be performed non-invasively using blood samples (e.g., serum or plasma samples), such as samples collected during an annual physical exam.

The methods described herein can be used to diagnose NAFL, NASH, or liver fibrosis, to monitor a subject at risk of developing NAFL, NASH, or liver fibrosis and, optionally, to provide prophylactic treatment, or to evaluate a subject's response to treatment for NAFL, NASH, or liver fibrosis. The diagnostic methods described herein can also be used to select patients for enrollment in a clinical trial for a new therapy for NAFL, NASH, or liver fibrosis. These diagnostic methods can be used individually or in combination with another diagnostic method for NAFL, NASH, or liver fibrosis, such as imaging (e.g., abdominal ultrasound, computerized tomography scanning, magnetic resonance imaging, transient elastography, or magnetic resonance elastography) or liver biopsy.

### Reference level calculation

The level of one or more biomarker (e.g., one or more lipid, glycan, hormone, and/or fatty acid) in a biological sample obtained from a subject can be compared to a reference level to diagnose the subject as healthy or as having NAFL, NASH, or liver fibrosis. The reference level may be generated using a positive or negative control sample, e.g., the level of one or more biomarker in a subject can be compared directly to the level of one or more biomarker in a negative control sample (e.g., a biological sample from a healthy subject not having NAFL, NASH, or liver fibrosis or a biological sample obtained from the subject at an earlier timepoint when the subject was diagnosed as not having NAFL, NASH, or liver fibrosis) or a positive control sample (e.g., a biological sample from a subject clinically diagnosed (e.g., diagnosed using liver biopsy or imaging) with NAFL, NASH, or liver fibrosis). Raw data (e.g., the level of one or more biomarker in a sample from a subject) collected from the subject can be compared to a reference level representing raw data (e.g., the level of one or more biomarker in a sample from a positive or negative control). The reference level may be based on data from a single subject (e.g., a single positive or negative control) or from a population of such subjects.

The subject may also be diagnosed by comparing raw data from the subject (e.g., a level of one or more biomarker (e.g., one or more lipid, glycan, hormone, and/or fatty acid) in a biological sample obtained from the subject) to transformed data, such as data that have been transformed using a machine learning algorithm(s) to produce a reference level representative of a population of subjects (e.g., a population of healthy subjects, a population of subjects clinically diagnosed with NAFL, NASH, and/or liver fibrosis, or a mixed population of healthy subjects and subjects clinically diagnosed with NAFL, NASH, and/or liver fibrosis). Various machine learning techniques may be used to produce a reference level using programs such as Scikit-learn Library in Python or MetaboanalystR. In one example, the subject may be diagnosed using a reference level determined by the Python code github.com/alirezayazdani1/NAFLD (provided in Appendix A) once the Python code has been trained to produce the reference level using the datasets available in doi.org/10.6084/m9.figshare.9774809 (provided in Lengthy Tables 6-16) (e.g., after the code has been trained using the datasets, it can be used to diagnose a subject by entering measurements obtained from the subject into the Python program).

A subject may be diagnosed as having NASH, NAFL, or liver fibrosis if the level of one or more biomarkers (e.g., one or more lipid, glycan, hormone, and/or fatty acid) in a biological sample (e.g., a blood, serum, or plasma sample) obtained from the subject is substantially similar to a reference level of one or more of the same biomarkers derived from one or more subjects clinically diagnosed as having NASH, NAFL, or liver fibrosis (e.g., if the level of one or more biomarkers in the subject is within 20%, e.g., +/- 20%, 15%, 10%, 9%, 8%, 7%, 6%, 5%, 4%, 3%, 2%, 1%, or less, of a reference level of one or more of the same biomarkers derived from a positive control sample or population, e.g., one or more subjects clinically diagnosed as having NASH, NAFL, or liver fibrosis). A subject may also be diagnosed as having NASH, NAFL, or liver fibrosis if the level of one or more biomarkers (e.g., one or more lipid, glycan, hormone, and/or fatty acid) in a biological sample (e.g., a blood, serum, or plasma sample) obtained from the subject is substantially dissimilar to a reference level of one or more of the same biomarkers derived from one or more healthy subjects (e.g., if the level of one or more biomarkers in the subject deviates by more than 20%, e.g., +/- 25%, 30%, 40%, 50%, 60%, 70%, 80%, 90%, 95%, 99%, or more, from a reference level of one or more of the same biomarkers derived from a negative control sample or population, e.g., one or more healthy subjects not having NAFL, NASH, or liver fibrosis).

A subject may be diagnosed as not having NASH, NAFL, or liver fibrosis (e.g., as healthy) if the level of one or more biomarkers (e.g., one or more lipid, glycan, hormone, and/or fatty acid) in a biological sample (e.g., a blood, serum, or plasma sample) obtained from the subject is substantially similar to a reference level of one or more of the same biomarkers derived from one or more healthy subjects (e.g., if the level of one or more biomarkers in the subject is within 20%, e.g., +/- 20%, 15%, 10%, 9%, 8%, 7%, 6%, 5%, 4%, 3%, 2%, 1%, or less, of a reference level of one or more of the same biomarkers derived from a negative control sample or population, e.g., one or more healthy subjects not having NASH, NAFL, or liver fibrosis). A subject may also be diagnosed as not having NASH, NAFL, or liver fibrosis (e.g., as healthy) if the level of one or more biomarkers (e.g., one or more lipid, glycan, hormone, and/or fatty acid) in a biological sample (e.g., a blood, serum, or plasma sample) obtained from the subject is substantially dissimilar to a reference level of one or more of the same biomarkers derived from one or more subjects clinically diagnosed as having NASH, NAFL, or liver fibrosis (e.g., if the level of one or more biomarkers in the subject deviates by more than 20%, e.g., +/- 25%, 30%, 40%, 50%, 60%, 70%, 80%, 90%, 95%, 99%, or more, from a reference level of one or more of the same biomarkers derived from a positive control sample or population, e.g., one or more subjects clinically diagnosed as having NASH, NAFL, or liver fibrosis).

### Treatment of NAFLD and liver fibrosis

The diagnostic methods described herein can also be used to guide patient treatment. A subject can be treated by measuring a level of one or more biomarkers described herein (e.g., lipids, glycans, hormones, and/or fatty acids) in a biological sample (e.g., a blood, serum, or plasma sample) obtained from the subject, determining that the level of the one or more biomarkers identifies the subject as one in need of treatment for NAFL, NASH, or liver fibrosis, and treating the subject with an appropriate therapy. A subject may also be treated for NAFL, NASH, or liver fibrosis based on prior diagnosis using the methods described herein.

Therapies for NAFL include weight loss, dietary changes (e.g., eating a diet rich in fruits, vegetables, whole grains, and healthy fats (e.g., low glycemic index foods); reducing sugar intake (e.g., intake of simple sugars); limiting intake of fats (e.g., saturated and/or trans fats); or reducing daily calorie intake), and increased physical activity. Therapies for NASH include weight loss, dietary changes (e.g., eating a diet rich in fruits, vegetables, whole grains, and healthy fats (e.g., low glycemic index foods); reducing sugar intake (e.g., intake of simple sugars); limiting intake of fats (e.g., saturated and/or trans fats); or reducing daily calorie intake), increased physical activity, anti-inflammatory and anti-apoptosis agents (e.g., pentoxifylline, selonsertib, (GS-4997) tipelukast (MN-001), and emricasan), medications to treat insulin resistance or type 2 diabetes (e.g., metformin DPP4 inhibitors, sulfonyl urea, glucagon-like peptide 1 receptor agonists (e.g., liraglutide, dulaglutide, and semaglutide), and SGLT2 inhibitors (e.g., canagliflozin, luseogliflozin, ipragliflozin, and empagliflozin)), medications to improve cholesterol (e.g., ezetimibe, statins), Vitamin E, peroxisome proliferator-activated receptor (PPAR) agonists, including PPARα agonists bezafibrate, fenofibrate, and pemafibrate (K-877), PPARγ agonists such as pioglitazone, INT131, and MK0533, PPARδ agonists, such as seladepar (MBX-8025) and endurobol (GW501516), dual PPARα/δ agonists such as elafibranor (GFT505), dual PPARα/γ agonists, such as saroglitazar, and pan-PPAR agonists such as IVA337, glutathione, usrodeoxycholic acid, pemafibrate, aramchol, GS0976, anti-hypertensive drugs (e.g., losartan), farnesoid X receptor (FXR) agonists (e.g., Obeticholic acid (OCA), INT-767, GS-9674, LMB763, LJN452), MGL-3196, BI 1467335, IMM-124E, solithromycin, BMS-986036, Cenicriviroc (CVC), and JKB-121. Therapies for liver fibrosis include weight loss, dietary changes, increased physical activity, anti-inflammatory and anti-apoptosis agents (e.g., pentoxifylline, selonsertib, (GS-4997) tipelukast (MN-001), and emricasan), medications to treat insulin resistance or type 2 diabetes (e.g., metformin DPP4 inhibitors, sulfonyl urea, glucagon-like peptide 1 receptor agonists (e.g., liraglutide, dulaglutide, and semaglutide), and SGLT2 inhibitors (e.g., canagliflozin, luseogliflozin, ipragliflozin, and empagliflozin)), medications to improve cholesterol (e.g., ezetimibe, statins), Vitamin E, peroxisome proliferator-activated receptor (PPAR) agonists, including PPARα agonists bezafibrate, fenofibrate, and pemafibrate (K-877), PPARγ agonists such as pioglitazone, INT131, and MK0533, PPARδ agonists, such as seladepar (MBX-8025) and endurobol (GW501516), dual PPARα/δ agonists such as elafibranor (GFT505), dual PPARα/γ agonists, such as saroglitazar, and pan-PPAR agonists such as IVA337, glutathione, usrodeoxycholic acid, pemafibrate, aramchol, GS0976, anti-hypertensive drugs (e.g., losartan), farnesoid X receptor (FXR) agonists (e.g., Obeticholic acid (OCA), INT-767, GS-9674, LMB763, LJN452), MGL-3196, BI 1467335, IMM-124E, solithromycin, BMS-986036, JKB-121, anti-fibrotic agents (e.g., Cenicriviroc (CVC), Simtuzumab, GR-MD-02, and ND-LO2-s0201), and liver transplant if the fibrosis is severe or has progressed to cirrhosis. Therapies for NASH and liver fibrosis are discussed in Sumida and Yoneda, J. Gastroenterol. 53:362-376, 2018. Alternatively, the subject may be treated with an experimental therapy. For example, based on the level of one or more biomarkers described herein (e.g., lipids, glycans, hormones, and/or fatty acids), the subject may be identified as in need of treatment for NAFL, NASH, or liver fibrosis, and enrolled in a clinical trial for treatment with a new therapeutic for NAFL, NASH, or liver fibrosis.

The methods described herein can also be used to evaluate treatment efficacy. One or more of the biomarkers described herein (e.g., lipids, glycans, hormones, and/or fatty acids) can be measured in a biological sample (e.g., a blood sample, such as a serum or plasma sample) obtained from a subject undergoing treatment and compared to a reference level, or to a negative control sample (e.g., a sample from a subject not having NAFL, NASH, or liver fibrosis), a positive control sample (e.g., a sample from a subject clinically diagnosed as having NAFL, NASH, or liver fibrosis), or a biological sample obtained from the subject at an earlier timepoint (e.g., 1 week earlier, two weeks earlier, one month earlier, 2 months earlier, 3 months earlier, 4 months earlier, 6 months earlier, 9 months earlier, 1 year earlier, or more) or prior to treatment initiation. If the results indicate that the subject has not improved or that the subject's condition has progressed or worsened (e.g., from NAFL to NASH, or from NASH to liver fibrosis), treatment can be altered, for example, the therapeutic can be administered to the subject more frequently and/or at a higher dosage, or the subject can be treated using a different therapeutic. If the results indicate that the subject has improved, the subject can continue treatment with the same therapeutic at the same dose and/or frequency of administration, the frequency of administration and/or dosage of the therapeutic can be decreased, or the therapy may be discontinued. The subject may be evaluated once yearly, twice a year, three times a year, four times a year, six times a year, monthly, bi-weekly, weekly, or more frequently to evaluate treatment efficacy. These methods can be used to evaluate the efficacy of standard treatments or to evaluate the efficacy of experimental treatments, such as therapeutics tested in a clinical trial.

In addition, the methods described herein can be used to monitor a subject at risk of developing NAFL, NASH, or liver fibrosis, or a subject who has previously received treatment for NAFL, NASH, or liver fibrosis, to evaluate disease progression. One or more of the biomarkers described herein (e.g., lipids, glycans, hormones, and/or fatty acids) can be measured in a biological sample (e.g., a blood sample, such as a serum sample) obtained from a subject at risk of developing NAFL, NASH, or liver fibrosis (e.g., a subject presenting with one or more features of metabolic syndrome, such as obesity, diabetes (e.g., Type 2 diabetes), insulin resistance, increased waist circumference, low HDL cholesterol, high LDL cholesterol, or higher than normal blood glucose levels, a subject with a family history of NAFL, NASH, or liver fibrosis) or a subject previously treated for NAFL, NASH, or liver fibrosis (e.g., a subject that exhibited improvement and/or discontinued treatment) and compared to a reference level, or to a negative control sample (e.g., a sample from a subject not having NAFL, NASH, or liver fibrosis), a positive control sample (e.g., a sample from a subject clinically diagnosed as having NAFL, NASH, or liver fibrosis), or a biological sample obtained from the subject at an earlier timepoint (e.g., 1 week earlier, two weeks earlier, one month earlier, 2 months earlier, 3 months earlier, 4 months earlier, 6 months earlier, 9 months earlier, 1 year earlier, or more). If the results indicate that the subject's condition has worsened (e.g., that the disease or condition has progressed, for example, from NAFL to NASH, or from NASH to liver fibrosis) the subject may be treated with a therapy for NAFL, NASH, or liver fibrosis (e.g., a therapy described herein). If the subject was previously treated for the disease or condition, the subject be treated with the same therapeutic and/or treatment regimen or with a different therapeutic or treatment regimen. If the results indicate that the subject's condition has not changed, the subject may continue to be monitored at regular intervals. Monitoring may be performed once yearly, twice a year, three times a year, four times a year, six times a year, monthly, bi-weekly, weekly, or more frequently.

### Examples

The following examples are put forth so as to provide those of ordinary skill in the art with a description of how the compositions and methods described herein may be used, made, and evaluated, and are intended to be purely exemplary of the invention and are not intended to limit the scope of what the inventors regard as their invention.

### Example 1. Diagnosis of NAFL, NASH, and liver fibrosis using serum samples

### Methods

### Study design

For this study, 83 subjects were recruited initially from the Second Medical Clinic of Aristotle University of Thessaloniki and the Department of Endocrinology of 424 General Military Hospital, Thessaloniki, Greece. In our analysis, 80 subjects were finally included (three were excluded due to limited amount of serum) and were divided in three groups: a) Healthy controls without NAFLD (n=49, among them 22 were lean or slightly overweight (BMI<27.5 kg/m²) and 27 were overweight/obese (BMI ≥27.5 kg/m2) and had similar BMI with the other two groups), b) Non-alcoholic fatty liver (NAFL) group (n=15),and c) NASH group (n=16). For the recruitment of the subjects in the control groups, the following inclusion criteria were used: 1) age >18 years, 2) currently normal ultrasound of the liver and normal liver function tests, 3) no history of abnormal liver ultrasound imaging or abnormal liver function tests. For the simple steatosis (NAFL) and NASH groups the diagnosis was established histologically. Exclusion criteria included all the conditions related to secondary fatty liver disease, such as viral, alcoholic, autoimmune and drug-induced hepatitis, and have been described in our previous publications (Polyzos et al., Metabolism 65:1550-8 (2016); Polyzos et al., Metabolism 63:207-17 (2014)). Liver biopsy was performed under computed tomography-guidance and histology was interpreted by two independent pathologists. Serum was collected after an overnight fasting and 1-2 h prior to liver biopsy.

### Glycomics

Total N-glycans from the serum samples were released by the enzyme PNGaseF (New England Biolabs). The total released N-glycans were permethylated (a standard procedure to modify glycans for better mass spectrometry data acquisition). The data were acquired with Bruker UltraFlexll MALDI-TOF instrument. For each sample, the mass range from which the data were collected was from 1500 m/z to 6000 m/z. The typical range for the vast majority of N-glycans extracted from serum samples is 1500 m/z to 4-5000 m/z. For each sample reported in this study and used for subsequent data analysis, an accumulation of 20,000 laser shots or more spread over the sample spot (1 µl of prepared N-glycans sample and 1 µl of matrix-the matrix is added for the sample to "fly" in the instrument-were spotted in 396-wells MALDI target steel plate) were considered. Mass signals with a signal-to-noise ratio of at least 3 were considered and matched against a list of known N-glycan masses. 62 different N-glycans composition were identified across the serum samples analyzed in this study.

### Lipidomics

Chloroform was purchased from Sigma-Aldrich and was HPLC grade (without amylenes). Acetonitrile was purchased from Merck Millipore and was LC/MS Hypergrade. All other solvents were purchased from FISHER SCIENTIFIC^{™} and were Optima LC/MS grade. Cells were washed once with ice cold 0.9% NaCl. The cell pellet was extracted by sequential addition of ice-cold 600 µL methanol, 300 µL water, and 400 µL chloroform. Samples were vortexed for 10 min at 4 °C and centrifuged at 17,000 x g for 10 min at 4 °C. The lower lipid-containing layer was then carefully collected and dried under nitrogen. Dried lipid extracts were stored at -80 °C. Lipids were separated on an ASCENTIS^{®} Express C18 2.1 x 150mm 2.7 µm column (Sigma-Aldrich) connected to a DIONEX^{™} UlitMate 3000 UPLC system and a Q EXACTIVE^{™} benchtop ORBITRAP^{™} mass spectrometer (Thermo Fisher Scientific) equipped with a heated electrospray ionization (HESI) probe. Dried lipid extracts were reconstituted in 50 µL 65:30:5 acetonitrile: isopropanol: water (v/v/v). 5 µL of sample were injected into the column, with separate injections for positive and negative ionization modes. Mobile phase A in the chromatographic method consisted of 60:40 water: acetonitrile with 10 mM ammonium formate and 0.1% formic acid, and mobile phase B consisted of 90:10 isopropanol: acetonitrile, with 10 mM ammonium formate and 0.1% formic acid. The chromatographic gradient was described previously (Hu et al., Biomedical chromatography 22:1108-14 (2008)). The column oven and autosampler were held at 55 °C and 4 °C, respectively. The mass spectrometer parameters were described previously (Bird et al., Analytical chemistry 83:6648-57 (2011)) and were modified as described in (Ruzicka et al., Journal of chromatography A 1349:116-21 (2014)). The spray voltage was set to 4.2 kV, and the heated capillary and the HESI were held at 320 °C and 300 °C, respectively. The S-lens RF level was set to 50, and the sheath and auxiliary gas were set to 35 and 3 units, respectively. These conditions were held constant for both positive and negative ionization mode acquisitions. External mass calibration was performed every 7 days using the standard calibration mixture. Mass spectra were acquired in both full-scan and data-dependent MS/MS mode. For the full-scan acquisition, the resolution was set to 70,000, the AGC target was 1x10⁶, the maximum injection time was 50 msec, and the scan range was *m*/*z* = 133.4-2000. For data-dependent MS/MS, the top 10 ions in each full scan were isolated with a 1.0-Da window, fragmented with a step-wise collision energy of 15, 25, and 35 units, and analyzed at a resolution of 17,500 with an AGC target of 2x10⁵ and a maximum injection time of 100 msec. The underfill ratio was set to 0. The selection of the top 10 ions was set to isotopic exclusion, a dynamic exclusion window of 5.0 sec, and an exclusion list of background ions based on a solvent bank. High-throughput identification and relative quantification of lipids was performed separately for positive and negative ionization mode data, using LIPIDSEARCH^{™} software (THERMO FISHER SCIENTIFIC^{™}/ Mitsui Knowledge Industries) using the default parameters for Q EXACTIVE^{™} product search and alignment. After alignment, raw peak areas for all identified lipids were exported to Microsoft Excel and filtered according to the following predetermined quality control criteria: Rej ("Reject" parameter calculated by LIPIDSEARCH^{™}) equal to 0; PQ ("Peak Quality" parameter calculated by LIPIDSEARCH^{™} software) greater than 0.85; CV (standard deviation/ mean peak area across triplicate injections of a represented (pooled) biological sample) below 0.4; R (linear correlation across a three-point dilution series of the representative (pooled) biological sample) greater than 0.9. Typically, ~70% of identified lipids passed all four quality control criteria. Raw peak areas of the filtered lipids were added together to create a total lipid signal for each sample, and individual lipid peak areas were normalized to this total signal as a control for lipid extraction and efficiency, as well as sample loading.

### Quantification of serum fatty acids

Fatty acids of whole serum (both free and bound) were converted to their corresponding fatty acid methyl esters (Bondia-Pons et al., Journal of chromatography B 809:339-44 (2004)) and separated by gas-chromatography using an Agilent HP 7890 Gas Chromatograph equipped with a 30 m × 0.25 µm × 0.25 mm SupraWAX-280 capillary column (Teknokroma, Barcelona, Spain), an autosampler, and a flame ionization detector. The amount of each fatty acid is expressed as a percentage of the total identified fatty acids in the sample.

### Targeted biochemical measurements

Six parameters were included: adiponectin, leptin, activin A, follistatin, activin A/follistatin, triglycerides. Adiponectin was measured with radioimmunoassay (RIA) (Millipore, MA), activin A and follistatin with ELISA (R&D Systems, MN) and triglycerides with an automated analyzer (Olympus AU2700; Olympus, Germany).

### Statistical analysis

### Data processing, normalization, and scaling

The values from all glycans were normalized initially to the value (ion signal) of the most abandoned glycan (2792) (e.g., the value of the glycan that had the highest concentration in plasma in all subjects, which was 2792). Two glycans (1999 and 2519) were removed from the analysis, as their missing values were more than 1/3 of the sample size (which was the exclusion threshold in our study). The values from all the lipidomic measurements were normalized to total positive or total negative ion signal. Fatty acid measurements were normalized and expressed as percent of total fatty acid concentration. Targeted biochemical measurements were not normalized. A missing value in mass spectrometric analysis indicated very low signal. Thus, a fraction (1/5^{th}) of the minimum value was added to all variables including the missing values in lipidomics and glycomics. No missing values were present in the fatty acids data set. The missing values in targeted biochemical measurements were very few (2 in Activin A, 1 in cholesterol, triglycerides (TG), high- and low- density lipoproteins (HDL and LDL), and were related to the lack of available serum (and not to low signal) for these subjects and, thus, were replaced with the median value of the group. Since lipidomics, glycomics and fatty acids data are normalized, they carry relative information. For such compositional data, a centered log ratio transformation is recommended ( Kalivodová et al., Journal of Chemometrics 1:21-8 (2015), where the ratio of measurements by their geometric mean for each variable is log-transformed. Furthermore, a log transformation is performed on targeted biochemical measurements to achieve a balanced distribution. Each dataset is then mean-centered and scaled to have a unit variance (van den Berg et al., BMC genomics 1:142 (2006)).

### Data analysis and visualization

In Table 2 and in FIG. 3E, mean ± SD for normally distributed and median with 25^{th} and 75^{th} percentile are reported. Shapiro-Wilk test was used to assess the distributions. One-way ANOVA for normally distributed and Kruskal-Wallis test for non-normally distributed features was performed followed (when p<0.05) by post-hoc Tukey's or Dunn's test. ANCOVA was performed subsequently in order to adjust for BMI between groups (non-normally distributed features were logarithmically transformed before the ANCOVA). A multi-class significance analysis of microarrays (SAM) was performed with a false discovery rate adjusted each time so that less than one false significant parameter is expected among all significant features. Additionally, to visualize the differences between the groups, heatmaps were created with hierarchical clustering following the Euclidean distance measure and the ward clustering algorithm. For further visualization and identification of significant features a sparse Partial Least Squares - Discriminant Analysis was performed (sPLS-DA) with maximum 5 components each containing a maximum of 10 variables (Lê Cao et al., BMC bioinformatics 1:253 (2011)). Additionally, for two of the predictive models that derived from machine learning further visualization and identification of significant features was performed with a principal component analysis (PCA) and with a t-distributed stochastic neighbor embedding (t-SNE).

### Classification - Machine Learning

For the classification of the data and development of receiver operating characteristic (ROC) curves different machine learning techniques with different software (Scikit-learn Library in Python and MetaboanalystR) were tested. Specifically, with Scikit-learn (Pedregosa et al., Journal of Machine Learning Research 12:2825-30 (2011)), a recursive feature elimination (RFE) algorithm along with linear support vector machine (SVM) was performed on all data to rank and identify the important variables (Guyon et al., Machine learning 1-3:389-422 (2002)). We then used the One-vs-Rest (OvR) multiclass classification strategy (Bishop, Springer (2006)) using different classification techniques including the SVM with both linear and nonlinear (radial basis function or RBF) kernels, k-nearest neighbors and random forest. 2/3 of the data were used to train the model and 1/3 to test it with a 3-fold cross validation scheme, which was repeated 100 times with random slices of data into train and test sets to reduce the variance in the classification accuracy and ROC curves. The main parameters for each classifier (e.g., C and gamma in SVM and number of neighbors in kNN) were tuned using an exhaustive search over specified parameter values also known as "grid search" along with cross validation. Balanced accuracy was used as the metric to identify the best parameter set for each classifier. Optimal parameters were then kept fixed for the rest of the analysis. With Metaboanalyst-R (Chong and Xia, Bioinformatics 34:4313-4 (2018)), groups were reduced to two (healthy vs NAFL-NASH, NAFL vs healthy-NASH and healthy-NAFL vs NASH) and a linear PLS-DA, a linear SVM and random forest were performed, each time using the respective feature ranking method. ROC curves were generated by Monte-Carlo cross validation (MCCV) using balance sub-sampling. Two-thirds of the samples were used to evaluate the feature importance and the top 2, 3, 5...max important features were used to build the model which was tested on the one-third of the remaining sample. This procedure was repeated multiple times. The classification analysis and development of models were performed for glycans, lipid classes, individual lipids, hormones and combination of glycans, hormones and individual lipids before and after excluding the lean subjects. The same analysis was also performed between subjects with (n=10) vs without fibrosis (n=21). Data sets are available in figshare database doi.org/10.6084/m9.figshare.9774809, R command history related to Metaboanalyst-R is available in figshare doi.org/10.6084/m9.figshare.9773843, and code related to OvR-Python is available in github.com/alirezayazdanil/NAFLD. The data sets are also provided in lengthy tables submitted herewith. Lengthy Table 6 contains data related to patient ID groups. Lengthy Table 7 contains raw fatty acid data that have been normalized. Lengthy Table 8 contains raw glycan data that have been normalized. Lengthy Table 9 contains raw hormone data that have been normalized. Lengthy Table 10 contains raw data for lipid classes that have been normalized. Lengthy Table 11 contains raw data for lipid species that have been normalized. Lengthy Table 12 contains transformed fatty acid data. Lengthy Table 13 contains transformed glycan data. Lengthy Table 14 contains transformed hormone data. Lengthy Table 15 contains transformed data for lipid classes. Lengthy Table 16 contains transformed data for lipid species. The code is provided in Appendix A and is designed to perform the steps of the classifier training outlined below.

### Classifier Training

The training of a classifier based on the data was implemented and performed using scikit-learn in Python. The following steps were taken for the single-omics analysis using lipids/glycans/hormones/fatty acids data:
1. Preprocess and clean each dataset
   a. Remove the variables that have more than 1/3 missing values among the samples
   b. Replace missing values for normalized data i.e., lipids, glycans and fatty acids with 1/5^{th} of the smallest sample value. For unnormalized data e.g., hormones, fill the missing values with the median of the sample values for each variable.
   c. Take centered log-ratio transformation of the normalized data and take logarithm transformation of the unnormalized data.
   d. Mean center and scale the data to have a unit variance
2. Perform dimensionality reduction and variable selection
   a. Select the k most relevant variables for each dataset by their highest scores obtained based on recursive feature elimination algorithm that uses a support vector machine (SVM) classifier with linear kernel.
3. Perform a grid search to fine tune the hyperparameters
   a. The grid search is performed on two adjustable parameters of an SVM classifier with a radial basis function kernel. The lipids data are used for the grid search.
4. Fit SVM classifiers with RBF kernel and test them for each reduced dataset
   a. For each dataset, 3 different permutations of 2/3 of the data are randomly selected as training set and 1/3 as validation set, hence we have a 3-fold cross validation.
   b. Fit the SVM classifier to the training set and test it on the validation set 3 times for the cross validation.
   c. Repeat this procedure 100 times and compute the statistics (mean and variance) of each classifier's accuracy, sensitivity and specificity.
   d. Plot the ROC curves that resulted from the previous step for each classifier to measure the performance of the classification. If the classifier has a sensitivity of at least 70% (e.g., 70%, 75%, 80%, 85%, 90%, 91%, 92%, 93% 94%, 95%, 96%, 97%, 98%, 99%, or more) and/or a specificity of at least 70% (e.g., 70%, 75%, 80%, 85%, 90%, 91%, 92%, 93% 94%, 95%, 96%, 97%, 98%, 99%, or more), it will be deemed acceptable for diagnosis of subjects. Preferably, the classifier has a sensitivity of at least 90% and a specificity of at least 90%.

For multi-omics analysis, e.g., lipids+glycans/hormones or lipids+glycans+hormones, the process remains very much the same with minor differences:
1. Same as above.
2. Perform dimensionality reduction similar to above on concatenated datasets (e.g., lipids and glycans stacked together).
3. Same as above using the concatenated data for the grid search.
4. Same as above using the concatenated data.

### Results

### Anthropometric and clinical characteristics of study population

The anthropometric and clinical characteristics of study population are described in Table 2, below. The healthy group was composed primarily of two subgroups, i.e., lean healthy group and healthy group with similar BMI (overweight/obese) to the NAFLD (NAFL and NASH) group. Thus, we have performed two types of analysis to assess whether BMI is relevant in our measurements. In the first, we have compared the healthy group consisting of both subgroups vs. NAFL vs. NASH and in the second, we have compared only the overweight/obese healthy vs. NAFL vs. NASH.

**Table 2: Anthropometric and biochemical characteristics of study population**

| **Anthropometric Parameters** | **Healthy (n=49)** | **Healthy (BMI ≥ 27.5 kg/m²) (n=27)** | **NAFL (n=15)** | **NASH (n=16)** | **Pa** | **Pb** |
|---|---|---|---|---|---|---|
| Age (years) | 54.0 (49.5, 59) | 54.0 (48.0, 59.0) | 55.0 (44.0, 60.0) | 53.5 (50.3, 61.0) | 0.93 | 0.89 |
| BMI (kg/m²) | 28.3 (26.1, 30.4)^{a,b} | 29.9 (28.9, 32.0) | 30.3 (29.4, 36.3)^{a} | 34.8 (29.7, 39.8)^{b} | <0.001 | 0.15 |
| Waist Circumference (cm) | 96.0 (86.5, 100.5)^{a,b} | 100.0 (96.0, 102.0)^{c} | 103.0 (95.0, 109.0)a | 109.5 (101.5, 113.3)^{b,c} | <0.001 | 0.02 |

| **Liver parameters** | | | | | | |
|---|---|---|---|---|---|---|
| SGOT (U/I) | 19.0 (17.0, 22.8)^{a,b} | 19.0 (17.0, 21.0)^{c,d} | 24.0 (23.0, 31.0)^{a,c} | 41.5 (31.0, 55.3)^{b,d} | <0.001 | <0.001 |
| SGPT (U/I) | 18.0 (14.3, 26.0)^{a,b} | 18.0 (14.0, 25.0)^{c,d} | 36.0 (26.0, 52.0)^{a,c} | 52.5 (35.8, 76.3)^{b,d} | <0.001 | <0.001 |
| GGT (U/I) | 15.0 (12.3, 21.8)^{a,b} | 15.0 (13.0, 21.0)^{c,d} | 30.0 (22.0, 53.0)^{a,c} | 47.0 (25.8, 103.3)^{b,d} | <0.001 | <0.001 |

| **Biochemical parameters** | | | | | | |
|---|---|---|---|---|---|---|
| Glucose (mg/dl) | 85.1 (79.4, 93.1) | 85.1 (77.3, 97.2) | 78.4 (76.9, 94.1) | 90.4 (83.1, 108.2) | 0.11 | 0.15 |
| Insulin (ulU/ml) | 7.2 (4.6, 10.7)^{a,b,c} | 9.0 (5.9, 12.4) | 11.4 (8.6, 18.3)^{a} | 20.3 (8.6, 29.1)^{b,c} | <0.001 | 0.003 |
| HOMA-IR | 1.46 (1.00, 2.24)^{a,b} | 1.78 (1.31, 3.12)^{c} | 2.45 (1.91, 4.26)^{a} | 4.57 (1.93, 8.54)^{b,c} | <0.001 | 0.004 |

Values were not normally distributed thus medians with (25^{th}, 75^{th} percentiles) are reported. Kruskal-Wallis test followed by post-hoc Dunn's test between groups was performed for healthy vs. NAFL vs. NASH (Pa = p-value for the comparison of healthy vs. NAFL vs. NASH) or for healthy obese vs NAFL vs NASH (Pb = p-value for the comparison of healthy obese vs. NAFL vs. NASH). The same superscript letters indicate significance in post-hoc t-test for the two groups. For example, for BMI, the healthy group has the superscript letters a and b, the NAFL group has the superscript a, and the NASH group has the superscript b. That means that BMI is significantly different between healthy vs. NAFL (since both groups have the superscript a), as well as between healthy vs. NASH (since both groups have the superscript b) but not between NAFL vs NASH (since they do not share a common superscript letter).

### Lipidomics - Differences in blood concentrations of lipids can be used to discriminate between healthy subjects and patients with NAFL and NASH.

A lipidomic analysis was performed which identified 366 lipid species belonging in 17 different lipid classes. Hierarchical clustering based on concentrations of lipid classes (total amount for each class) showed that two main clusters are formed: one consisting of healthy subjects and the other consisting of patients with NAFL or NASH (FIG. 1A). Similarly, sPLS-DA analysis (FIG. 1B) showed that the two main components could mostly differentiate the healthy group (red dots clustering at the left side) from the other two groups (green and blue dots for NAFL and NASH, respectively, clustering together at the right). Specifically, serum concentrations of diglycerides (DG), phosphatidylglycerols (PG) and phosphatidic acids (PA) were significantly increased in NAFL and NASH, whereas the concentrations of acylcarnitines (AcCa), cholesterol esters (Che), coenzyme Q10 (Co), lysophosphatidylcholines (LPC) and sphingomyelines (SM) were reduced (FIG. 1C). Except from AcCa, the observed differences in the lipid classes remained significant after excluding the lean healthy controls (FIGS.1D-1F).

Given the limited discriminatory ability of lipid classes, we investigated whether the concentrations of individual lipid species can help differentiate between these groups. Significance Analysis of Microarrays (SAM) with corrected FDR for the number of investigated parameters identified 62 lipid species that significantly differ between healthy, NAFL and NASH (FIG. 2A). Most of these lipid species belong to Phosphatidylethanolamines (PE), Phosphatidylcholines (PC) and SM lipid classes (FIG. 2B). Hierarchical clustering showed that lipid species can specifically best discriminate healthy subjects from subjects with NAFL and NASH (FIG. 2C). sPLS-DA showed that with the use of two main components (FIG. 2D and 2E), each consisting of 10 lipid species, healthy, NAFL and NAFLD subjects converge into separate clusters (FIG. 2D: red dots healthy subjects located left, green dots NAFL subjects located upper right and blue dots subjects with NASH located low right).

The same analysis was repeated after excluding the lean subjects (FIGS. 2F-2J) from the healthy group (obese healthy vs. NAFL vs. NASH). The number of significant parameters according to SAM was reduced to 32, with similar representation (mainly PE, PC and SM) as that found with both the lean and obese subjects included in the healthy group. Hierarchical clustering demonstrated that lipid species could best differentiate NASH subjects from healthy and NAFL. Similar to the first analysis, sPLS-DA showed robust discrimination between healthy, NAFL, and NASH, indicating BMI-independent differences in lipidome between healthy subjects and patients with NAFL and NASH.

### Fatty acids - Differences in fatty acid composition are observed between healthy, NAFL and NASH subjects

The concentrations of 23 fatty acids as a relative percent of the total lipid concentration was assessed chromatographically in the NASH, NAFL, and healthy status. Serum from 64 subjects was available for these measurements. sPLS-DA analysis showed only a partial discrimination of healthy subjects (red dots) from NAFL and NASH (green and blue dots, respectively) before (FIG. 3A) and after excluding the lean controls (FIG. 3C). This discrimination was achieved by component 1, where five fatty acids were mainly contributing to its composition (FIGS. 3B and 3D). These fatty acids were significantly different between groups also in the univariate analysis (FIGS. 3E). Specifically, C16:0 (palmitic acid) was higher in NAFL and NASH compared to healthy population. Additionally, the monounsaturated fatty acids C16:1n7cis (cis-palmitoleic acid) and C18:1 n9cis (oleic acid) were higher in NASH compared to healthy group, whereas the polyunsaturated linoleic fatty acid (C18:2n6) and arachidonic fatty acid (C20:4n6) were lower (FIG. 3E). These differences between groups remained significant after adjusting for BMI.

### Glycomics - Differences in N-glycans are observed between NAFLD vs healthy subjects

GC-MS identified 62 N-glycans in the serum of healthy subjects and patients with NAFL and NASH. Concentrations of seventeen glycans were significantly different between groups (FIG. 4A), while five glycans concentrations were significantly different after excluding the lean controls (FIG. 4C). The main structural characteristics of the glycans were the presence of fucose (10 out of 17) and of sialic acids (13 out of 17). sPLS-DA analysis showed that only two from the five components could partially discriminate the healthy group from the other two (both before and after excluding the lean control) but could not discriminate patients with NAFL from those with NASH (FIGS. 4B, 4D).

### Evaluation of predictive accuracy of NASH, NAFL, or healthy status with the use of five machine learning methods

We have used two different platforms (Scikit-learn Library in Python and MetaboanalystR) to evaluate the performance of five different machine learning techniques (linear and non-linear SVM, k-nearest neighbor (kNN), linear PLS-DA and random forest) in predicting whether a subject belongs to a NASH, NAFL, or healthy group (Table 3, below). A binary classification method was used to reduce the groups from three to two following the OvR (One-vs-Rest) strategy in Scikit-learn. In MetaboanalystR, three individual classifications were performed, and subjects were grouped as healthy vs. NAFL-NASH, NAFL vs. healthy-NASH and NASH vs. healthy-NAFL. Each omics data set (lipidomics, glycomics, biochemical parameters including four hormones and triglycerides) was analyzed individually as well as in combination aiming to achieve the highest accuracy with the fewest number of variables. Fatty acids were analyzed only individually since the available fatty acids data were acquired for 64 subjects (due to limited serum availability), whereas the data from other measurements (i.e., glycomics, lipidomics, hormones) were collected for 80 subjects. The highest accuracy was observed for the SVM (with nonlinear radial basis function kernel) method for lipid species both alone and in different combinations with the other omics data (Table 3). The lipid, glycan, and hormone species assayed to produce the data shown in Table 3 are presented in Table 4.

**Table 3: Predictive accuracies (%) of five machine learning techniques for multiclass (OvR) and binary classification using two different software**

| **All subjects** | | | | | | | | | | |
|---|---|---|---|---|---|---|---|---|---|---|
| | **OvR (Scikit-learn)** | | | | | | **Binary (MetaboanalystR)** | | | |
| **Models (A)** | Variables (n) | SVM (linear) | kNN | SVM (RBF) | Random Forest | | Variables (n) | PLS-DA (linear) | Random Forest | SVM linear |
| 1. Lipid classes | 4 | 58 ± 8 | 50 ± 8 | 63 ± 10 | 56 ± 10 | | 3 | 84/58/75 | 80/60/73 | 76/51/72 |
| 2. Lipid species | 29 | 86 ± 9 | 65 ±10 | 88 ± 7 | 65 ± 9 | | 50 | 77/55/70 | 83/60/80 | 81/58/76 |
| 3. Hormones | 4 | 54 ± 8 | 49 ± 8 | 55 ± 9 | 54 ± 9 | | 4 | 86/60/80 | 80/57/76 | 85/54/77 |
| 4. Glycans | 5 | 57 ± 9 | 45 ± 7 | 54 ± 10 | 49 ± 9 | | 5 | 54/44/58 | 60/60/51 | 59/49/53 |
| 5. Fatty acids | 5 | 50 ± 9 | 43 ± 8 | 54 ± 11 | 45 ± 10 | | 5 | 61/48/57 | 71/49/63 | 66/48/62 |
| 6. Lipid species + Glycans | 10 | 80 ± 8 | 67 ± 10 | 78 ± 9 | 66 ± 9 | | | | | |
| 7. Lipid species + Glycans | 20 | 86 ± 8 | 67 ± 10 | 82 ± 9 | 66 ± 10 | | | | | |
| 8. Lipid species + Hormones | 10 | 74 ±10 | 59 ± 9 | 76 ± 9 | 67 ± 10 | | | | | |
| 9. Lipid species + Hormones | 20 | 80 ±10 | 64 ± 10 | 87 ± 8 | 65 ± 9 | | | | | |
| 10. Lipid species + Hormones + Glycans | 10 | 77 ± 9 | 71 ± 11 | 77 ± 9 | 68 ± 10 | | | | | |
| 11. Lipid species + Hormones + Glycans | 20 | 83 ± 8 | 63 ± 9 | 86 ± 7 | 65 ± 9 | | | | | |

| **All subjects (excluded controls with BMI<27.5 kg/m²)** | | | | | | | | | | |
|---|---|---|---|---|---|---|---|---|---|---|
| | **OvR (Scikit-learn)** | | | | | | **Binary (MetaboanalystR)** | | | |
| **Models (B)** | Variables (n) | SVM (linear) | kNN | SVM (RBF) | Random Forest | | | PLS-DA (linear) | Random Forest | SVM (linear) |
| 1. Lipid classes | 4 | 59 ± 9 | 49 ± 9 | 55 ± 9 | 56 ± 10 | | 5 | 77/50/67 | 77/53/68 | 77/52/69 |
| 2. Lipid species | 29 | 84 ± 9 | 70 ± 11 | 88 ± 9 | 64 ± 9 | | 50 | 70/52/68 | 79/49/77 | 75/53/72 |

| **All subjects** | | | | | | | | | | |
|---|---|---|---|---|---|---|---|---|---|---|
| | **OvR (Scikit-learn)** | | | | | | **Binary (MetaboanalystR)** | | | |
| **Models (A)** | Variables (n) | SVM (linear) | kNN | SVM (RBF) | Random Forest | | Variables (n) | PLS-DA (linear) | Random Forest | SVM linear |
| 3. Hormones | 4 | 52 ± 8 | 48 ± 9 | 51 ± 9 | 49 ± 9 | | 4 | 79/51/72 | 74/47/68 | 78/50/69 |
| 4. Glycans | 5 | 62 ± 10 | 54 ± 9 | 62 ± 9 | 49 ± 10 | | 5 | 58/41/63 | 52/53/48 | 55/49/53 |
| 5. Fatty acids | 5 | 51 ± 10 | 42 ±10 | 51 ± 12 | 50 ± 9 | | 5 | 57/43/57 | 74/43/62 | 61/41/59 |
| 6. Lipid species + Glycans | 10 | 71 ± 10 | 67 ± 10 | 71 ± 10 | 65 ± 11 | | | | | |
| 7. Lipid species + Glycans | 20 | 88 ± 7 | 67 ± 10 | 90 ± 9 | 64 ± 11 | | | | | |
| 8. Lipid species + Hormones | 10 | 78 ± 9 | 73 ± 10 | 79 ± 10 | 67 ± 11 | | | | | |
| 9. Lipid species + Hormones | 20 | 86 ± 7 | 72 ±10 | 89 ± 7 | 68 ± 10 | | | | | |
| 10. Lipid species + Hormones + Glycans | 10 | 82 ± 9 | 75 ± 9 | 83 ± 9 | 68 ± 10 | | | | | |
| 11. Lipid species + Hormones + Glycans | 20 | 83 ± 8 | 74 ± 10 | 85 ± 9 | 69 ± 9 | | | | | |

For the (Scikit-% of OvR learn) predictive accuracy ± SD of the cross validations is reported. For the Binary (MetaboanalystR) predictive accuracy of healthy vs. NAFL-NASH/NAFL vs. healthy-NASH/NASH vs. healthy-NAFL is reported. Number of variables included each time in the model is reported. For the Binary (MetaboanalystR) analysis the number of variables selected was the one that achieved the highest accuracy. The three numbers separated by slashes in the three columns for the binary classification refer to the respective accuracy if we try to distinguish healthy from NAFLD (NAFL+NASH) / or NAFL vs. healthy+NASH/ or NASH vs. healthy+NAFL. The highest accuracies were observed with SVM-RBF, and the parameters used for SVM-RBF are provided in Table 4.

### Development of predictive models for the differentiation between NASH, NAFL and healthy status

Based on the above analysis, nonlinear SVM with RFE was selected as a method to develop ROC curves by using a limited number of lipids, hormonal or glycan variables (Table 4, below). With the use of 29 different lipid species, a very high predictive accuracy was achieved (FIG. 5A) for all the three groups. Fatty acids and hormonal levels demonstrated high accuracy for discriminating healthy and NASH status (FIGS. 5B and 5D). Glycans could also be used to predict NAFL (FIG. 5C). Combining 20 variables of 19 lipids and one glycan (FIG. 5F) or 10 variables of 9 lipids and one glycan (FIG. 5H), as described in Table 4, led also to very high accuracies, as did the combination of lipids and hormones (FIGS. 5E and 5G).

The same analysis was repeated after excluding the lean subjects from the healthy group. This had a small impact on the observed predictive accuracy (FIG. 6A) in the model consisting of 29 lipids. Combinations of lipids and glycans (FIG. 6F) or lipids, hormones and glycans (FIG. 6H) could discriminate very efficiently between groups.

**Table 4: Top-ranked variables considered in different predictive models of NASH vs. NAFL vs. healthy**

| **All subjects** | | | | | | | | |
|---|---|---|---|---|---|---|---|---|
| **Lipids (29 variables)** | | | | **Fatty acids (5 variables)** | | **Glycans (5 variables)** | | **Hormones (4 variables)** |
| AcCa(10:0) | PC(34:2e) | PE(38:6) | | C18:2n6 | | 1661 | | Leptin |
| Cer(d34:2) | PC(35:3) | PI(36:1) | | C18:3n6 | | 2592 | | Adiponectin |
| DG(34:1) | PC(36:4) | SM(d32:0) | | C20:0 | | 2764 | | Activin |
| DG(36:4) | PC(36:5e) | SM(d32:2) | | C20:4n6 | | 2968 | | A/Follistatin |
| LPC(20:0e) | PC(37:2) | SM(d40:1) | | C22:4n6 | | 4039 | | Triglycerides |
| LPC(22:5) | PC(40:6e) | TG(38:0) | | | | | | |
| LPE(16:0) | PC(40:7) | TG(38:2) | | | | | | |
| PC(32:0) | PC(40:8) | TG(43:1) | | | | | | |
| PC(32:1e) | PC(42:6) | TG(53:5) | | | | | | |
| PC(34:0) | PE(38:1) | | | | | | | |
| | | | | | | | | |

| **Lipids + Hormones (20 variables)** | | | **Lipids + Glycans (20 variables)** | | | **Lipids + Hormones (10 variables)** | | **Lipids + Glycans (10 variables)** |
|---|---|---|---|---|---|---|---|---|
| AcCa(10:1) | PC(36:5e) | | Cer(d18:2_25:1) | PC(35:3) | | DG(34:1) | | LPC(22:5) |
| Cer(d43:0) | PC(37:2) | | LPC(20:0e) | PC(40:5e) | | LPC(20:0e) | | LPE(16:0) |
| DG(34:1) | PC(37:3) | | LPC(22:5) | PC(40:7) | | LPE(18:0) | | PC(34:1) |
| DG(36:4) | PC(40:8) | | LPE(16:0) | PC(42:6) | | PC(34:2e) | | PC(34:1e) |
| LPC(20:0e) | PE(34:1) | | PC (32: 1e)=> | PE(38:1) | | PC(36:4) | | PC(35:3) |
| LPC(22:5) | PE(38:1) | | *PC(16:0e_16:1)* | PE(38:6) | | PC(36:5e) | | PC(42:6) |
| LPE(18:0) | SM(d40:1) | | PC (32:1e) => | SM(d35:1) | | PC(37:3) | | PE(38:1) |
| PC(34:2e) | TG(38:0) | | *PC(16:1e_16:0)* | SM(d38:1) | | PC(40:8) | | SM(d35:1) |
| PC(36:3) | TG(38:2) | | PC(34:0) | TG(40:0) | | PE(38:1) | | SM(d40:1) |
| PC(36:4) | Adiponectin | | PC(34:1) | TG(52:4) | | Adiponectin | | 2592 |
| | | | PC(34:1e) | 2592 | | | | |

| **All subjects (excluded controls with BMI < 27.5 kg/m²)** | | | | | | | | |
|---|---|---|---|---|---|---|---|---|
| **Lipids (29 variables)** | | | | **Fatty acids (5 variables)** | | **Glycans (5 variables)** | | **Hormones (4 variables)** |
| AcCa(10:0) | PC(34:1) | PE(40:4e) | | C16:0 | | 1988 | | Leptin |
| Cer(d34:2) | PC(35:3) | PI(36:1) | | C18:1n9cis | | 2592 | | Adiponectin |
| DG(34:1) | PC(36:5e) | SM(d32:0) | | C18:2n6 | | 2764 | | Activin |
| | | | | | | 2968 | | A/Follistatin |
| DG(36:4) | PC(37:2) | SM(d32:2) | | C18:3n6 | | 3953 | | Triglycerides |
| Hex1Cer(d3 4:2) | PC(40:7) | SM(d36:0) | | C22:4n6 | | | | |
| | PC(40:8) | SM(d36:4) | | | | | | |
| LPC(20:0e) | PC(42:6) | SM(d37:1) | | | | | | |
| | | SM(d40:1) | | | | | | |
| LPC(22:5) | PE(34:1) | TG(40:0) | | | | | | |
| LPE(18:0) | PE(34:3e) | | | | | | | |
| LPE(22:5) | PE(38:1) | | | | | | | |
| PA(44:4) | | | | | | | | |
| | | | | | | | | |

| **Lipids + Hormones (20 variables)** | | | **Lipids + Glycans (20 variables)** | | | **Lipids + Hormones (10 variables)** | | **Lipids + Glycans (10 variables)** |
|---|---|---|---|---|---|---|---|---|
| Cer(d34:0) | PC(36:5e) | | Cer(d34:0) | PC(36:2) | | LPC(20:0e) | | Cer(d43:3) |
| DG(36:4) | PC(37:2) | | Cer(d43:3) | PC(40:7) | | LPC(20:3) | | DG(34:1) |
| LPC(20:0e) | PC(40:7) | | DG(34:1) | PC(42:6) | | LPE(16:0) | | DG(36:2) |
| LPC(20:3) | PC(40:8) | | DG(36:4) | PE(34:1) | | PC(34:1) | | LPC(20:0e) |
| LPE(16:0) | PE(38:1) | | LPC(20:0e) | PE(34:3e) | | PC(34:2e) | | LPC(22:5) |
| LPE(18:0) | PE(40:4e) | | LPC(22:5) | CO(36:1) | | PC(36:5e) | | LPE(16:0) |
| LPE(22:5) | SM(d32:0) | | LPE(16:0) | TG(38:0) | | PC(40:8) | | PC(35:2) |
| PC(34:0) | SM(d36:0) | | PC(34:0) | TG(38:2) | | PE(38:1) | | PC(40:7) |
| PC(34:1) | SM(d40:1) | | PC(34:1) | 1988 | | SM(d32:0) | | PE(34:1) |
| | | | | 2592 | | | | |
| PC(34:2e) | Adiponectin | | PC(35:2) | | | Adiponectin | | PE(34:3e) |
| | | | | | | | | |

| **Lipids + Hormones + Glycans(20 variables)** | | | | | | | | **Lipids + Hormones + Glycans(10 variables)** |
|---|---|---|---|---|---|---|---|---|
| DG(34:2) | | | | | | PC(36:4) | | LPC(22:5) |
| DG(36:4) | | | | | | PC(40:8) | | LPE(16:0) |
| LPC(20:0e) | | | | | | PE(34:1) | | PC(34:1) |
| LPC(22:5) | | | | | | PE(40:4e) | | PC(40:8) |
| | | | | | | | | PE(34:1) |
| LPE(16:0) | | | | | | PI(36:1) | | PI(36:1) |
| LPE(18:0) | | | | | | SM(d35:1) | | SM(d38:0) |
| PA(44:4) | | | | | | SM(d38:0) | | SM(d40:1) |
| PC(34:1) | | | | | | SM(d40:1) | | Adiponectin 2592 |
| PC(34:2e) | | | | | | Adiponectin 2592 | | |
| PC(36:3) | | | | | | | | |

### Exploratory analysis of the discriminatory and predictive potentials of lipidomics, hormones, glycomics, and fatty acids in liver fibrosis

Among the 31 subjects with NAFLD, 21 had fibrosis in the histology according to Kleiner fibrosis score and 10 did not. We performed an exploratory analysis of these two groups (fibrosis "yes" or "no"), which showed that similar to what we observed in the whole study population, lipidomics primarily were able to discriminate robustly between the two groups (FIGS. 7A-7C and Table 5).

**Table 5: Top-ranked variables considered in the predictive models of fibrosis**

| **Lipids** | **Glycans** | **Fatty acids** |
|---|---|---|
| DG(36:3) | 2081 | C14:0 |
| LPC(18:0) | 2315 | C18:1n9trans |
| PC(36:2) | 2592 | C18:3n3 |
| PC(37:2) | 2605 | C20:3n6 |
| PC(40:5) | 4587 | |
| TG(38:0) | | C22:6n3 |
| TG(50:0) | | |
| TG(51:1) | | |
| TG(57:1) | | |
| TG(60:2) | | |

### Discussion

We report that combinations of glycans, lipids, and hormonal variables can diagnose (e.g., simultaneously and with high accuracy) the presence of NASH, NAFL, or healthy status.

Additionally, we report a combination of lipids that can diagnose the presence of liver fibrosis. We obtained excellent classification performance. Both diagnostic models can be further trained and validated for large independent cohorts both cross-sectionally and, more importantly, prospectively.

Several studies have aimed to develop non-invasive diagnostic scores for advanced fibrosis, whereas fewer efforts have focused on the diagnosis of NASH (Castera et al., Gastroenterology 156:1264-81 (2019); Caussy et al., Gut (2018)). Among them, circulating cytokeratin-18 fragment (Ck-18f), has been the most investigated biomarker so far, with two meta-analyses suggesting an AUROC of 0.82 with sensitivity ranging from 66%-78% and specificity of 82%-97% for NASH diagnosis (Kwok et al., Alimentary pharmacology & therapeutics 39:254-69 (2014); Musso et al., Annals of medicine 43:617-49 (2011)). The low sensitivity, observed especially in studies including multiethnic populations (Cusi et al., Journal of hepatology 60:167-74 (2014)), the variations in proposed diagnostic cut-off levels and the lack of a widely available assay for clinical purposes have resulted in the limited use of CK-18f in the clinical setting. Several combinations of serum biomarkers and/or clinical variables have also been proposed (reviewed in Vilar-Gomez and Chalasani, Journal of Hepatology 68:305-15 (2018)), but none of them has reported AUROCs as high as in our study. Furthermore, many of these studies were performed in morbidly obese individuals, and, thus, may have limited applicability to the general population.

Our study, using a stepwise approach and multiple machine learning methods, has focused on variables that are key players in pathophysiological mechanisms related to the development of steatosis and NASH such as changes in lipidome representing the abnormal lipid metabolism (Buzzetti et al., Metabolism 65:1038-48)), in glycans reflecting the ability of the liver to synthesize proteoglycans (Blomme et al., Journal of hepatology 50:592-603 (2009), and in hormones associated with the increased IR and abnormal glucose homeostasis commonly observed in these patients (Reccia et al., Metabolism 72:94-108 (2017)). Glycomics is a subspecialty in omics systems sciences that offers substantial promise for next-generation biomarkers on disease susceptibility, drug target discovery, and precision medicine. Glycosylation disorders have been reported in NAFLD and loss of glycosylation of crucial uptake and efflux transporters in patients with NASH could influence transporter function and contribute to changed drug disposition. (Clarke et al., Liver international 37:1074-81 (2017))

Most importantly, in our study, we have followed an approach with several major advantages: First, we have comparatively tested several supervised learning methods to identify the most accurate to predict among the groups. Previous studies primarily used one classification method (mostly based on logistic regression analysis) and features were selected in many cases through univariate tests (t-tests, Welch's tests, individual AUC scores) (Poynard et al., BMC gastroenterology 6:34 (2006); Younossi et al., Obesity surgery 18:1430-7 (2008)). Such methods, however, tend to be overly optimistic and are highly susceptible to overfitting, and, thus, are difficult to reproduce. Among the methods we have tested, SVM with RFE has demonstrated the highest accuracy. RFE method removes the redundant features and builds a predictive model only with the highly scored variables, which helps to avoid overfitting. Since our study included a small number of subjects, we have additionally used the biggest part of the population to train the model but also a small part (through balanced subsampling) to validate it. Second, our predictive models are able to diagnose between three (and not two) conditions, i.e., healthy status, NAFL, and NASH. Thus, they allow the evaluation of a subject with a single blood draw without the need of an imaging modality for diagnosing NAFL. This concept is especially attractive for routine screening of the population at high risk for the development of the disease such as obese patients or patients with type 2 diabetes in a primary care setting and without the need of specially trained personnel. Additionally, our approach can be used to build a similar model for differentiating between "none vs. non-advanced vs. advanced fibrosis." Importantly, we have additionally developed models that can differentiate healthy status, NAFL, and NASH among overweight and obese people, taking into consideration that this population is not only more probable to have NASH, but is also more likely to be tested by doctors. We are reporting different models combining a variety of detection methods (mass-spectrometry, chromatography, ELISA) and a variable number of parameters. These models seem to be more cost-effective than liver biopsy. Specifically, the costs of liver biopsy have been reported to be at £956 (i.e., $1153) in 2012-2013 (Crossan et al., Health Technol Assess 19:1-409 (2015)), whereas in the US currently the costs of liver biopsy vary between $2,000-8,000. The costs of the model combining lipids, glycans, and adiponectin in our study was $605, thus, significantly lower compared to liver biopsy. We are also expecting the costs to be further reduced if targeted measurements of lipids and glycans are performed and as the mass-spectrometric methods are becoming widely available. Finally, the diagnostic methods can also include an evaluation of targeted biochemical, genetic and clinical parameters. The best candidates are liver transaminases, platelets, age, BMI, and PNPLA3 genotype (Vilar-Gomez and Chalasani, Journal of Hepatology 68:305-15 (2018)). These variables were not included in the current study since some of them were used as inclusion criteria to organize the study groups (e.g., liver transaminases and BMI), whereas no material was available for others (i.e., DNA for PNPLA3 genotype).

Regarding the variables that were selected by our models, the most robust differences and consequently the most profound contribution to the predictive algorithms were observed in lipid species. We observe in our study higher total DG, a trend to higher total TG, and lower levels in many of the PC species in NAFL and NASH. Several of these variables were also selected by our predictive models. Additionally, we observed a progressive increase of palmitoleic (16:1n7) and oleic acid (18:1n9), as well as a progressive decrease of gamma-linoleic acid (18:3n6) from healthy to NAFL and NASH. Quantification of the total amount of these fatty acids was conducted in a subset of the study population. Furthermore, we identified changes in TG species that can particularly contribute to the diagnosis of liver fibrosis (fibrotic model). We subsequently tested whether including adiponectin, leptin, follistatin, and activin A (measured all through validated immunoassays) could improve the accuracy of the models or maintain high accuracy with lower number of variables. Among them, only adiponectin is contributing to models combining lipids with hormones or lipids with hormones and glycans. Of note, adiponectin improves the accuracy of our predictive models even after excluding the lean subjects, suggesting that its relation to NAFL and NASH is not explained just by the higher weight and fat mass of these patients. Adiponectin, beyond its weight- and glucose-regulatory effects exerts anti-steatotic, anti-inflammatory and anti-fibrotic actions in the liver, suggesting a protective role against development and progress of NAFLD (Boutari et al., Endocrinol Metab 33:33-43 (2018); Polyzos et al., Metabolism 65:1550-8 (2016); Polyzos et al., Metabolism 96:66-82 (2019); Polyzos et al., Metabolism 60:313-26 (2011)). In line with the experimental results, in a meta-analysis of 27 studies, adiponectin levels progressively decreased from controls to subjects with NAFL and to subjects with NASH independently of BMI, age, sex and the presence of T2D (Polyzos et al., Metabolism 60:313-26 (2011)).

In our study, with the use of standardized GC-MS methods, 62 glycans were detected, among which 17 were significantly different between the groups. However, the differences were observed mainly between healthy and NAFLD patients and in many cases diminished after excluding the lean subjects from the control group, suggesting that they are primarily weight-related. Most of the significant glycans were core- or multi-fucosylated and many of them contained sialic acids. The levels of sialic acids have been associated with metabolic syndrome (Sriharan et al., Diabetes care 25:1331-5 (2002)) as well as NAFLD (He et al., Annals of nutrition & metabolism 67:69-75 (2015); Lu et al., BioMed research international 2016:5921589 (2016)). Increased core-fucosylation of proteins has been associated with liver fibrosis as well as HCC with a core-fucosylated form of alpha-fetoprotein being approved by the FDA as a biomarker of HCC (Ma et al., Journal of proteomics 189:67-74 (2018); Shimizu et al., Clinica chimica acta 254:23-40 (1996)). Using a recent algorithm for diagnosing advanced liver fibrosis in NASH, fucose levels were one of the ten metabolites included in the diagnostic panel (Caussy et al., Gut (2018)). In our diagnostic models of healthy, NAFL, and NASH, which combine lipids, glycans and/or hormones, a multi- and core-fucosylated glycan (2592) was often selected, along with a second agalactosylated non-fucosylated glycan (1988) in some cases. In the exploratory analysis for the presence of liver fibrosis, 4 out of 5 selected glycans were core-fucosylated. Nevertheless, N-glycome profile is strongly related to body weight, which can help improve the diagnostic accuracy of NAFL subjects in our models, but may be especially useful for the non-invasive discrimination between different fibrosis stages. Of note, serum fucosylated haptoglobin appears to be a useful glyco-biomarker of liver fibrosis and a predictor of HCC in patients with chronic hepatitis C, a prominent feature of which is NAFLD (Tawara et al., PloS one 11:e0151828 (2016); Adinolfi et al., International journal of molecular sciences 17 (2016)).

### Example 2. Treatment of NASH

A biological sample (e.g., a blood, serum, or plasma sample) obtained from a subject at risk of developing NASH (e.g., an obese subject, or a subject with type 2 diabetes and/or metabolic disease) can be evaluated for one or more of the biomarkers described herein (e.g., one or more lipids, glycans, hormones, and/or fatty acids). The measurements from the biological sample obtained from the subject could then be compared to a reference level, such as a reference level from one or more healthy controls and/or one or more subjects clinically diagnosed (e.g., diagnosed using liver biopsy or imaging) as having NASH or NAFL (e.g., which may be determined using Python code github.com/alirezayazdani1/NAFLD (provided in Appendix A), which has been trained using datasets available in doi.org/10.6084/m9.figshare.9774809 (provided in Lengthy Tables 6-16)). Based on the measurements obtained from the subject and the comparison to the reference level, a subject identified as having NASH would then be treated using a NASH therapy (e.g., weight loss, dietary changes, increased physical activity, anti-inflammatory or anti-apoptosis agents, medications to treat insulin resistance or type 2 diabetes, medications to improve cholesterol, Vitamin E, PPAR agonists, glutathione, usrodeoxycholic acid, pemafibrate, aramchol, GS0976, anti-hypertensive drugs, FXR agonists MGL-3196, BI 1467335, IMM-124E, solithromycin, BMS-986036, Cenicriviroc (CVC), or JKB-121) according to standard clinical guidelines.

The efficacy of the NASH therapy could be evaluated by collecting a biological sample from the subject (e.g., a blood, serum, or plasma sample) one month, two months, three months, six months, one year, or more after treatment initiation and measuring the same biomarkers evaluated previously to determine whether the subject's condition has improved. Treatment efficacy could be evaluated on an ongoing basis (e.g., once a month, once every two months, once every three months, once every six months), if needed. If the evaluation indicates that the subject's condition has not improved, the therapy can be adjusted (e.g., administered more frequently and/or at a higher dose) or changed (e.g., the subject may be treated with a different therapeutic). If the evaluation indicates improvement in the subject's condition, therapy may be deemed effective and continued, adjusted (e.g., administered less frequently or at a lower dose), or terminated if the subject is no longer diagnosed as having NASH. Routine monitoring could be performed by intermittent (e.g., once a month, once every two months, once every three months, once every six months, or once yearly) measurement of the biomarkers described herein (e.g., one or more lipids, glycans, hormones, and/or fatty acids) if treatment is altered or terminated to evaluate the subject's condition and assess whether additional changes are needed to the therapeutic regimen.

### Example 3. Diagnosis of NAFL

A biological sample (e.g., a blood, serum, or plasma sample) obtained from a subject at risk of developing NAFLD (e.g., NAFL or NAFLD, e.g., an obese subject, or a subject with type 2 diabetes and/or metabolic disease) can be evaluated for one or more of the biomarkers described herein (e.g., one or more lipids, glycans, hormones, and/or fatty acids). The measurements from the biological sample obtained from the subject could then be compared to a reference level, such as a reference level from one or more healthy controls and/or one or more subjects clinically diagnosed (e.g., diagnosed using liver biopsy or imaging) as having NASH or NAFL (e.g., which may be determined using Python code github.com/alirezayazdani1/NAFLD (provided in Appendix A) that has been trained using datasets available in doi.org/10.6084/m9.figshare.9774809 (provided in Lengthy Tables 6-16)). Based on the measurements obtained from the subject and the comparison to the reference level, a subject identified as having NAFL would then be advised to make lifestyle changes to reduce the fat in their liver and reduce their chance of progression to NASH (e.g., weight loss, dietary changes (e.g., eating a diet rich in fruits, vegetables, whole grains, and healthy fats (e.g., low glycemic index foods); reducing sugar intake (e.g., intake of simple sugars); limiting intake of fats (e.g., saturated and/or trans fats); or reducing daily calorie intake), and/or increased physical activity).

The subject's condition could be regularly monitored (e.g., once a month, once every two months, once every three months, once every six months, once a year, or once every two years) by collecting a biological sample from the subject (e.g., a blood, serum, or plasma sample) and evaluating the one or more of the biomarkers described herein (e.g., one or more lipids, glycans, hormones, and/or fatty acids, e.g., the biomarkers previously measured in the subject) to determine whether the subject's condition has changed (e.g., whether the subject has reduced their liver fat content and can now be classified as healthy, or whether the subject's condition has worsened to the point that they are diagnosed as having NASH). If the evaluation indicates that the subject's condition has not improved, the therapy could be adjusted (e.g., the subject may work with a nutritionist to make more effective dietary changes). If the evaluation indicates that the subject's condition has worsened (e.g., if the subject is diagnosed as having NASH), they could be treated with a NASH therapy (e.g., a NASH therapy described herein).

### Example 4. Diagnosis of liver fibrosis

A biological sample (e.g., a blood, serum, or plasma sample) obtained from a subject at risk of developing liver fibrosis (e.g., a subject having NASH) can be evaluated for one or more of the biomarkers described herein (e.g., one or more lipids, glycans, or fatty acids). The measurements from the biological sample obtained from the subject could then be compared to a reference level, such as a reference level from one or more healthy controls and/or one or more subjects clinically diagnosed (e.g., diagnosed using liver biopsy or imaging) as having NASH, NAFL, or liver fibrosis (e.g., which may be determined using Python code github.com/alirezayazdani1/NAFLD (provided in Appendix A) that has been trained using datasets available in doi.org/10.6084/m9.figshare.9774809 (provided in Lengthy Tables 6-16)). Based on the measurements obtained from the subject and analysis performed using the Python code, a subject identified as not having liber fibrosis would then be treated as before (e.g., treated with a NASH therapy, such as weight loss, dietary changes (e.g., eating a diet rich in fruits, vegetables, whole grains, and healthy fats (e.g., low glycemic index foods); reducing sugar intake (e.g., intake of simple sugars); limiting intake of fats (e.g., saturated and/or trans fats); or reducing daily calorie intake), increased physical activity, anti-inflammatory or anti-apoptosis agents, medications to treat insulin resistance or type 2 diabetes, medications to improve cholesterol, Vitamin E, PPAR agonists, glutathione, usrodeoxycholic acid, pemafibrate, aramchol, GS0976, anti-hypertensive drugs, FXR agonists MGL-3196, BI 1467335, IMM-124E, solithromycin, BMS-986036, Cenicriviroc (CVC), or JKB-121) according to standard clinical guidelines).

The subject could be routinely monitored for the development of liver fibrosis (e.g., assessed once a month, once every two months, once every three months, once every six months, once yearly, once every two years, or once every five years) based on measurement of the biomarkers described herein (e.g., one or more lipids, glycans, or fatty acids) in a biological sample (e.g., a blood, serum, or plasma sample) obtained from the subject. If the subject is identified as having liver fibrosis during routine monitoring, the result could be confirmed using other approaches (e.g., liver biopsy or imaging), and/or the subject could then be treated with a liver fibrosis therapy (e.g., an anti-fibrotic agent, such as Cenicriviroc (CVC), Simtuzumab, GR-MD-02, or ND-LO2-s0201).

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the invention that come within known or customary practice within the art to which the invention pertains and may be applied to the essential features hereinbefore set forth, and follows in the scope of the claims. Other embodiments are within the claims.

### Lengthy Tables

The patent application contains a lengthy table section. A copy of the tables is available in electronic form from the USPTO web site. An electronic copy of the tables will also be available from the USPTO upon request and payment of the fee set forth in 37 CFR § 1.19(b)(3).

### Appendix A

"""NAFLD Classification"""
from __future__ import division
from __future__ import print_function
import graphviz, csv
from itertools import cycle
import matplotlib.pyplot as plt
import seaborn as sns
sns.set(font_scale=1)
import numpy as np
from scipy import interp
import pandas as pd
import sompy
from mlxtend.feature_selection import SequentialFeatureSelector as SFS
from sklearn import neighbors, preprocessing, tree
from sklearn.decomposition import PCA
from sklearn.manifold import TSNE
from sklearn.feature_selection import f_classif, mutual_info_classif, SelectKBest, RFE, RFECV, SelectFromModel
from sklearn.ensemble import RandomForestClassifier, ExtraTreesClassifier
from sklearn.model_selection import train_test_split, cross_val_score, RepeatedStratifiedKFold, StratifiedKFold, GridSearchCV
from sklearn.svm import LinearSVC, SVC
from sklearn.cross_decomposition import PLSRegression
from sklearn.multiclass import OneVsRestClassifier
from sklearn.metrics import roc_curve, auc, roc_auc_score
def load_data(datasets, normalized, classify):

```
 [use_lipid, use_glyc, use_fatty, use_horm, use_bmi] = [datasets[0], datasets[1],
                                    datasets[2], datasets[3],
                                    datasets[4]]
 use_glyc = True
 if use_glyc:
    data = np.loadtxt('data/glycomics_final.csv')
    X, y = data[:, :-1], data[:, -1]
    y = y.astype(int)
    nglyc_var = X.shape[1]
 if use_lipid == 'All':
    data = np.loadtxt('data/lipidomics_all_raw.csv')
    QC_CV = np.array([])
 elif use_lipid == 'AllNorm':
    data = np.loadtxt('data/lipidomics_all_normalized.csv')
    QC_CV = np.array([])
 elif use_lipid == 'AllClass':
    data = np.loadtxt('data/lipidomics_class_normalized.csv')
    QC_CV = np.array([])
 if use_lipid == 'PA':
    data = np.loadtxt('data/lipidomics_pos_all_normalized.csv')
    QC_CV = np.loadtxt('data/QC_pos.csv')
 elif use_lipid == 'PC':
    data = np.loadtxt('data/lipidomics_pos_class.csv')
    QC_CV = np.array([])
 elif use_lipid == 'NA':
    data = np.loadtxt('data/lipidomics_neg_all_normalized.csv')
    QC_CV = np.loadtxt('data/QC_neg.csv')
 elif use_lipid == 'NC':
    data = np.loadtxt('data/lipidomics_neg_class.csv')
    QC_CV = np.array([])
 if use_lipid:
    if normalized and QC_CV.any():
      fail = np.where(QC_CV > 0.25)[0]
      data = np.delete(data, fail, axis=1)
    t = {}
    liploc = []
    for d in data:
      t[int(d[-1])] = d[:-1]
      liploc += [np.where(y == int(d[-1]))[0][0]]
    X = X[liploc]
    y = y[liploc]
    tmp = np.zeros((X.shape[0], X.shape[1] + data.shape[1] - 1))
    for i in range(len(y)):
      tmp[i, :X.shape[1]] = X[i]
      tmp[i, X.shape[1]:] = t[y[i]]
    X = tmp
 nlipid_var = X.shape[1] - nglyc_var
 if use_horm:
    data = np.loadtxt('data/hormones_final.csv')
    t = {}
    liploc = []
    for d in data:
      t[int(d[-1])] = d[:-1]
      if np.where(y == int(d[-1]))[0].any():
         liploc += [np.where(y == int(d[-1]))[0][0]]
      else:
         continue
    X = X[liploc]
    y = y[liploc]
    tmp = np.zeros((X.shape[0], X.shape[1] + data.shape[1] - 1))
    for i in range(len(y)):
      tmp[i, :X.shape[1]] = X[i]
      tmp[i, X.shape[1]:] = t[y[i]]
    X = tmp
 nhorm_var = X.shape[1] - nglyc_var - nlipid_var
 if use_fatty:
    data = np.loadtxt('data/fatty_acids_final.csv')
    data[:,:-1] /= 100.0
    t = {}
    liploc = []
    for d in data:
      t[int(d[-1])] = d[:-1]
      if np.where(y == int(d[-1]))[0].any():
         liploc += [np.where(y == int(d[-1]))[0][0]]
      else:
         continue
    X = X[liploc]
    y = y[liploc]
    tmp = np.zeros((X.shape[0], X.shape[1] + data.shape[1] - 1))
    for i in range(len(y)):
      tmp[i, :X.shape[1]] = X[i]
      tmp[i, X.shape[1]:] = t[y[i]]
    X = tmp
 nfatty_var = X.shape[1] - nglyc_var - nlipid_var - nhorm_var
 if use_bmi:
    data = np.loadtxt('data/BMI_age.csv')
    t = {}
    for d in data:
      t[int(d[0])] = d[1:]
    tmp = np.zeros((X.shape[0], X.shape[1] + 2))
    for i in range(len(y)):
      tmp[i, :-2] = X[i]
      tmp[i, -2:] = t[y[i]]
    X = tmp
 data = np.loadtxt('data/groups.csv', dtype=int)
 groups = dict(data)
 patientld = y
 y = np.array(list(map(lambda i: groups[i], y)))
 if classify == '-0':
    index = np.nonzero(y > 0)[0]
    X = X[index]
    y = y[index]
 else:
    y[np.where(y == 0)] = 1
 if classify == '1v2+3':
    y[np.where(y == 3)] = 2
 elif classify == '1+2v3':
    y[np.where(y == 2)] = 1
 elif classify == '2v3':
    index = np.concatenate((np.nonzero(y == 2)[0], np.nonzero(y == 3)[0]), axis=None)
    X = X[index]
    y = y[index]
 elif classify == 'fibrosis':
    A = [2, 3, 4, 6, 8, 12, 14, 18, 19, 21, 23, 24, 26, 30, 32, 42, 45, 58, 60, 64, 68]
    B = [16, 22, 25, 27, 29, 31, 41, 48, 49, 65]
    if use_lipid:
      B = [16, 22, 27, 29, 31, 41, 48, 49, 65]
    index = []
    for i in range(len(A)):
      index.append(np.where(patientld == A[i])[0][0])
    for i in range(len(B)):
      index.append(np.where(patientld == B[i])[0][0])
    X = X[index]
    y = y[index]
    y[:len(A)] = 1
    y[len(A):] = 2
 return X, y, [nlipid_var, nglyc_var, nfatty_var, nhorm_var], patientld
 def writedata(X, y, pid, fields, fname):
 Xnew = np.concatenate((X, y[:,None], pid[:,None]), axis=1)
 with open(fname, mode='w') as csv_file:
    writer = csv.DictWriter(csv_file, fieldnames=fields)
    writer.writeheader()
    for i in range(len(y)):
      writer.writerow(dict(zip(fields, Xnew[i,:])))
      def standardization(X):
 return preprocessing.scale(X)
 def clr(X):
 x = np.log(X)
 return x - np.mean(x, axis=0)
 def feature_selection(X, y, k, feature_names, op):
 if op == 'FTest':
    sel = SelectKBest(f_classif, k=k)
    X_new = sel.fit_transform(X, y)
    feature_ids = np.argsort(-sel.scores_)
    kk = k
 if op == 'RFE':
    estimator = SVC(C=0.125, kernel='linear', decision_function_shape='ovr')
    sel = RFE(estimator, n_features_to_select=k, step=1)
    X_new = sel.fit_transform(X, y)
    kk = sel.n_features_
    feature_ids = [np.argwhere(sel.support_)[i][0] for i in range(kk)]
 if op == 'RFECV':
    estimator = SVC(C=0.125, kernel='linear', decision_function_shape='ovr')
    # estimator = RandomForestClassifier(n_estimators=100, max_depth=None, min_samples_split=2,
    # max_features=None, bootstrap=True)
    sel = RFECV(estimator, step=1, min_features_to_select=k,
           cv=StratifiedKFold(n_splits=3, random_state=None),
           n_jobs=-1)
    X_new = sel.fit_transform(X, y)
    kk = sel.n_features_
    feature_ids = [np.argwhere(sel.support_)[i][0] for i in range(kk)]
    plt.figure()
    plt.xlabel("Number of features selected")
    plt.ylabel("Cross validation score (fraction of correct classifications)")
    plt.plot(range(1, len(sel.grid_scores_) + 1), sel.grid_scores_)
    plt.show()
 if op == 'Ensem':
 # estimator = ExtraTreesClassifier(n_estimators=50, max_depth=None)
    estimator = RandomForestClassifier(n_estimators=100, max_depth=None, min_samples_split=2,
                          max_features=None, bootstrap=True)
    estimator = estimator.fit(X, y)
    model = SelectFromModel(estimator, threshold='3.0*mean', prefit=True)
    X_new = model.transform(X)
    feature_ids = model.get_support(indices=True)
    kk = len(feature_ids)
 if op == 'SFS':
    estimator = SVC(C=8.0, kernel='rbf', gamma=0.0078125, decision_function_shape='ovr')
    # Sequential Forward Floating Selection
    sffs = SFS(estimator,
           k_features='best',
           forward=True,
           floating=False,
           scoring="accuracy',
           cv=4,
           n_jobs=-1)
    sffs = sffs.fit(X, y)
    X_new = sffs.transform(X)
    feature_ids = sffs.k_feature_idx_
    kk = len(feature_ids)
 selected = [feature_names[feature_ids[i]] for i in range(kk)]
 print('\n%d variables selected using %s method:' % (kk, op))
 print(selected)
 print()
 return X_new, kk, selected
 def linearSVM(X=None, Y=None):
 "'C: [10^-5, 10^5]
 '"
 clf = LinearSVC(dual=False, C=0.125)
 if X is None:
    return clf
 clf.fit(X, Y)
 return clf
 def kNN(X=None, Y=None):
 "'n_neighbors
 weights: uniform, distance
 '"
 clf = neighbors.KNeighborsClassifier(n_neighbors=5, weights='uniform')
 return clf
 def SVM(X=None, Y=None):
 "'C: [10^-5, 10^5]
 '"
 clf = SVC(C=8.0, gamma=0.0078125)
 if X is None:
    return clf
 clf.fit(X, Y)
 return clf
 def decision_tree(X=None, Y=None, feature_names=None, class_names=None):
 clf = tree.DecisionTreeClassifier(min_samples_leaf=2)
 if X is None:
    return clf
 clf = clf.fit(X, Y)
 dot_data = tree.export_graphviz(
    clf, out_file=None, # max_depth=3,
    feature_names=feature_names,
    class_names=class_names, filled=True, leaves_parallel=True,
    proportion=True, rounded=True)
 graph = graphviz.Source(dot_data)
 graph.render('decision_tree')
 return clf
 def random_forest(X=None, Y=None):
 clf = RandomForestClassifier(n_estimators=20, max_depth=None, min_samples_split=2,
                    max_features='auto', bootstrap=True)
 if X is None:
    return clf
 clf.fit(X, Y)
 return clf
 def plsr(X=None, Y=None):
 clf = PLSRegression(n_components=4, max_iter=5000, scale=False)
 if X is None:
    return clf
 clf.fit(X, Y)
 return clf
 def visualization(X, y):
 fig, axes = plt.subplots(1, 1, figsize=(6, 6))
 model = PCA(n_components=2)
 X_pca = model.fit_transform(X)
 for i in range(1, 4):
    axes.scatter(X_pca[y == i, 0], X_pca[y == i, 1], label='Group {}'.format(i))
 axes.set_title('PCA Visualization of Latent Components', fontsize=16)
 axes.set_xlabel('PCA $1^{st}$ Component', fontsize=14)
 axes.set_ylabel('PCA $2^{nd}$ Component', fontsize=14)
 axes.legend(loc='best', fontsize=12)
 fig, axes = plt.subplots(1, 1, figsize=(6, 6))
 model = TSNE(n_components=2, init='random', early_exaggeration = 10,
         random_state=0, perplexity=40)
 X_tsne = model.fit_transform(X)
 for i in range(1, 4):
    axes.scatter(X_tsne[y == i, 0], X_tsne[y == i, 1], label='Group {}'.format(i))
 axes.set_title('t-SNE Visualization of Latent Variales', fontsize=16)
 axes.set_xlabel('t-SNE $1^{st}$ Component', fontsize=14)
 axes.set_ylabel('t-SNE $2^{nd}$ Component', fontsize=14)
 axes.legend(loc='best', fontsize=12)
 def grid_search(X, y, param, method):
 clf = GridSearchCV(SVC(kernel=method, decision_function_shape='ovo'),
             param, iid=False, cv=3, n_jobs=-1)
 clf.fit(X, y)
 print("Best estimator for SVM found by grid search:")
 print(clf.cv_results_['params'][clf.best_index_])
 def SOM(dimy, dimx, names, X):
 mapsize = [dimy, dimx]
 som = sompy.SOMFactory.build(X, mapsize, mask=None, mapshape='planar',
                    lattice='rect', normalization='var',
                    initialization='pca', neighborhood='gaussian',
                    training='batch', name='sompy', component_names=names)
 return som
 def SOM_plot(som, n_c):
 v = sompy.mapview.View2D(200, 200, 'NAFLD/NASH', text_size=8)
 v.show(som, what='codebook', which_dim=1, cmap=None, col_sz=6, denormalize=True)
 som.cluster(n_clusters=n_c)
 h = sompy.hitmap.HitMapView(50, 50, 'Clustering', text_size=8, show_text=True)
 h.show(som, anotate=True, onlyzeros=False, labelsize=7, cmap="Pastel1")
 u = sompy.umatrix.UMatrixView(50, 50, 'umatrix', show_axis=True, text_size=8, show_text=True)
 u.build_u_matrix(som, distance=1, row_normalized=False)
 u.show(som, distance2=1, row_normalized=False, show _data=True,
     contooor=False, blob=False)
     def model_selection(X, y):
 clfs = {
    'Linear SVM': linearSVM(),
    'kNN': kNN(),
    'SVM': SVM(),
    'Decision tree': decision_tree(),
    'Random forest": random_forest(),
    # 'PLS-DA': plsr(),
    }
 print('Accuracy:')
 for name, clf in clfs.items():
    scores = cross_val_score(clf, X, y,
                    cv=RepeatedStratifiedKFold(n_splits=3, n_repeats=100, random_state=None),
                    n_jobs=-1)
    print(' %s: %0.2f (+/- %0.2f)' % (name, np.array(scores).mean(),
                          np.array(scores).std()))
                          def ROC(X, y, classlds, title):
 # Classification and ROC analysis
 # Run classifier with cross-validation
 cv = RepeatedStratifiedKFold(n_splits=3, n_repeats=100, random_state=None)
 # Learn to predict each class against the other
 classifier = OneVsRestClassifier(SVM())
 tprs = dict()
 roc_auc = dict()
 mean_fpr = np.linspace(0, 1, 100)
 mean_auc = []
 n_classes = len(classlds)
 if n_classes == 2:
    n_classes = 1
 for i in range(n_classes):
    tprs[i] = []
    roc_auc[i] = []
 for train, test in cv.split(X, y):
    y_score = classifier.fit(X[train], y[train]).decision_function(X[test])
    # Binarize the output
    y_test label = preprocessing.label_binarize(y[test], classes=classlds)
    mean_auc.append(roc_auc_score(y_test_label, y_score, average='weighted'))
    # Compute ROC curve and area under the curve
    for i in range(n_classes):
      if n_classes == 1:
         fpr, tpr, _ = roc_curve(y_test_label, y_score)
      else:
         fpr, tpr, _ = roc_curve(y_test_label[:, i], y_score[:, i])
      tprs[i].append(interp(mean_fpr, fpr, tpr))
      roc_auc[i].append(auc(fpr, tpr))
 print('Average AUC for all classes: %0.2f' % np.mean(mean_auc))
 # Plot all ROC curves
 plt.figure(figsize=(6,6))
 lw = 2
 colors = cycle(['red', 'green', 'blue'])
 for i, color in zip(range(n_classes), colors):
    plt.plot(mean_fpr, np.mean(tprs[i], axis=0), color=color, lw=lw,
         label=r'%d vs. Other(s) (AUC = %0.2f $\pm$ %0.2f)'
         # label=r'%d vs. 2 (AUC = %0.2f $\pm$ %0.2f)'
         % (classlds[i], np.mean(roc_auc[i]), np.std(roc_auc[i])))
 plt.plot([0, 1], [0, 1], 'k--', Iw=lw)
 plt.xlim([0.0, 1.0])
 plt.ylim([0.0, 1.05])
 plt.xlabel('False Positive Rate', fontsize=14)
 plt.ylabel('True Positive Rate', fontsize=14)
 plt.title(title, fontsize=16)
 # plt.title(', Group "0" Excluded\n(6 variables selected)'
 # 'Normilized Lipids + Hormonal Data\n(33 variables selected)')
 plt.legend(loc="lower right", fontsize=12)
 if _name_ == '_main_':
 min_features = 20
 lipid = 'AllNorm'
 glyc = True
 fatty = False
 horm = True
 bmi = False
 normalized = True
 classify = "
 classlds = [1, 2, 3]
 write_data = False
 X, y, nvar, patientld = load_data([lipid, glyc, fatty, horm, bmi], normalized, classify)
 [nlipid, nglyc, nfatty, nhorm] = [nvar[0], nvar[1], nvar[2], nvar[3]]
 if glyc:
    glyc_names = [
      '1579', '1661', '1784', '1825', '1836', '1866', '1907', '1988', '1999',
      '2040', '2070', '2081', '2111', '2192', '2244', '2274', '2285', '2315',
      '2396', '2418', '2431', '2448', '2489', '2519', '2592', '2605', '2635',
      '2646', '2663', '2676', '2693', '2738', '2764', '2779', '2809',
      '2850', '2867', '2880', '2953', '2968', '3054', '3213', '3228', '3241',
      '3329', '3415', '3503', '3572', '3590', '3603', '3691', '3777', '3865',
      '3953', '4039', '4052', '4226', '4400', '4413', '4587', '4761'
      ]
 if lipid == 'All':
    lipid_names = [str(i) for i in range(1,nlipid+1)]
    all_vars = pd.read_csv('data/lipids_all_names_raw.csv')
 elif lipid == 'AllNorm':
    lipid_names = [str(i) for i in range(1,nlipid+1)]
    all_vars = pd.read_csv('data/lipids_all_names_normalized.csv')
 elif lipid == 'AllClass':
    lipid_names = [
         'AcCa', 'Cer', 'ChE', 'Co', 'DG', 'Hex1Cer', 'Hex2Cer', 'LPC', 'LPE',
         'PA', 'PC', 'PE', 'PG', 'PI', 'PS', 'SM', 'TG'
    ]
 if lipid == 'PA':
    lipid_names = [str(i) for i in range(1,nlipid+1)]
 elif lipid == 'PC':
    lipid_names = [
      'AcCa', 'Cer', 'CerG1', 'CerG2', 'CerP', 'ChE', 'Co', 'DG', 'LPC',
      'LPE', 'PC', 'PE', 'PG', 'PI', 'PIP', 'PS', 'SM', 'So', 'TG', 'ZyE',
      'phSM'
    ]
 elif lipid == 'NA':
    lipid_names = [str(i) for i in range(1,nlipid+1)]
 elif lipid == 'NC':
    lipid_names = ['CL', 'Cer', 'LPC', 'LPE', 'PC', 'PE', 'PG', 'PI', 'PS', 'SM']
 if bmi:
    bmi_names = ['BMI', 'AGE']
 if fatty:
    fatty_names = [
      'C14_0', 'C16_0', 'C16_1n7trans', 'C16_1n7cis', 'C18_0', 'C18_1n9cis',
      'C18_1n9trans', 'C18_2n6', 'C18_3n6', 'C18_3n3', 'C20_0' , 'C20_1n9', 'C20_2n6',
      'C20_3n6', 'C20_4n6', 'C20_5n3', 'C22_0', 'C22_4n6', 'C22_5n6', 'C22_5n3',
      'C24_0', 'C22_6n3', 'C24_1n9'
    ]
 if horm:
    horm_names = [
      'Irisin_Mantz', 'merin_Mantz', 'Leptin_Mantz', 'Adiponectin_Mantz',
      '4_Mantz', 'Glucose_Mantz', 'Insulin', 'homa_IR', 'homa_beta','Activin A',
      'Folistatin', 'Activin_To_Follistatin', 'cholesterol', 'Tgs', 'HDL', 'LDL',
      'ActB', 'FSTL3' , 'IGF1', 'IGFBP3', 'Intact IGFBP3', 'Total IGFBP4',
      'Intact IGFBP4', 'PAPPA', 'Total IGF1 vs Total IGFBP3', 'Total IGF1 vs intact IGFBP3'
      ]
 if lipid == 'All' or lipid == 'AllNorm':
    lip_del = [
      'SM(d42:5)+H'
         ]
    ind_lip_del = [all_vars.index[all_vars['LipidIon'].str.find(s)==0].tolist()[0]
             for s in lip_del]
    X = np.delete(X, ind_lip_del, axis=1)
    lipid_names = list(np.delete(lipid_names, ind_lip_del))
    all_vars = all_vars.drop(ind_lip_del)
    nlipid -= len(ind_lip_del)
 feature_names = []
 if glyc:
    delete = []
    glycans = X[:,:nglyc]
    for i in range(glycans.shape[1]):
      col = glycans[:,i]
      if len(col[np.where(col == 0)]) >= np.floor(len(col)/3):
         delete += [i]
         print('Deleted glycan %s' % glyc_names[i])
    X = np.delete(X, delete, axis=1)
    glyc_names = list(np.delete(glyc_names, delete))
    feature_names += glyc_names
    nglyc -= len(delete)
    glycans = X[:,:nglyc]
    trans = clr(glycans + glycans[np.nonzero(glycans)].min()/10.)
    X[:,:nglyc] = standardization(trans)
 if lipid:
    delete = []
    lipids = X[:,nglyc:(nglyc+nlipid)]
    for i in range(lipids.shape[1]):
      col = lipids[:,i]
      if len(col[np.where(col == 0)]) >= np.floor(len(col)/3):
         delete += [i]
         print('Deleted lipid ion #%d' % (i+1))
    X = np.delete(X, delete, axis=1)
    lipid_names = list(np.delete(lipid_names, delete))
    feature_names += lipid_names
    if lipid == 'All' or lipid == 'AllNorm':
      all_vars = all_vars.drop(delete)
    nlipid -= len(delete)
    if normalized:
      lipids = X[:,nglyc:(nglyc+nlipid)]
      trans = clr(lipids + lipids[np.nonzero(lipids)].min()/10.)
      X[:,nglyc:(nglyc+nlipid)] = standardization(trans)
    else:
      lipids = X[:,nglyc:(nglyc+nlipid)]
      for i in range(lipids.shape[1]):
         col = lipids[:,i]
         colmed = np.median(col[np.nonzero(col)])
         col[np.where(col==0)] = colmed
         lipids[:,i] = np.log(col)
      X[:,nglyc:(nglyc+nlipid)] = standardization(lipids)
 if horm:
    delete = []
    hormones = X[:, (nglyc+nlipid):(nglyc+nlipid+nhorm)]
    for i in range(hormones.shape[1]):
      col = hormones[:,i]
      if len(col[np.where(col == 0)]) >= np.floor(len(col)/3):
         delete += [i]
         print('Deleted hormone %s' % horm_names[i])
    X = np.delete(X, delete, axis=1)
    horm_names = list(np.delete(horm_names, delete))
    feature_names += horm_names
    nhorm -= len(delete)
    hormones = X[:,(nglyc+nlipid):(nglyc+nlipid+nhorm)]
    for i in range(hormones.shape[1]):
      col = hormones[:,i]
      colmed = np.median(col[np.nonzero(col)])
      col[np.where(col==0)] = colmed
      hormones[:,i] = np.log(col)
    X[:,(nglyc+nlipid):(nglyc+nlipid+nhorm)] = standardization(hormones)
 if fatty:
    delete = []
    fatties = X[:, (nglyc+nlipid+nhorm):]
    for i in range(fatties.shape[1]):
      col = fatties[:,i]
      if len(col[np.where(col == 0)]) >= np.floor(len(col)/3):
         delete += [i]
         print('Deleted fatty acid %s' % fatty_names[i])
    X = np.delete(X, delete, axis=1)
    fatty_names = list(np.delete(fatty_names, delete))
    feature_names += fatty_names
    nfatty -= len(delete)
    fatties = X[:,(nglyc+nlipid+nhorm):]
    for i in range(fatties.shape[1]):
      col = fatties[:,i]
      colmed = np.median(col[np.nonzero(col)])
      col[np.where(col==0)] = colmed
      fatties[:,i] = np.log(col)
    X[:,(nglyc+nlipid+nhorm):] = standardization(fatties)
 if not glyc:
    X = X[:, nglyc:]
 print('# Glycans = %d, # Lipids
(classes or molecules) = %d, # Hormones = %d, # Fatty Acids = %d'
     % (nglyc, nlipid, nhorm, nfatty))
 if write_data:
    if lipid == 'All' or lipid == 'AllNorm':
      names = [np.int(feature_names[i])-1 for i in range(len(feature_names))]
      _names = list(all_vars['LipidIon'].loc[names][:])
    else:
       _names = feature_names
    writedata(X, y, patientld, _names + ['Group', 'Patientld'], 'DataGlycomics.csv')
    # param_grid = {'C':[2**i for i in range(-5, 16)],
    # 'gamma':[2 ** i for i in range(-15, 3)]}
    # grid_search(X, y, param_grid, 'linear')
 operation = 'RFE'
 X, n_features, feature_names = feature_selection(X, y, min_features,
                                 feature_names, operation)
 visualization(X, y)
 if lipid == 'All' or lipid == 'AllNorm':
    names = [np.int(s)-1 for s in feature_names if s.isdigit()]
    sel_vars = all_vars['LipidIon'].loc[names]
    print(list(sel_vars))
    print()
 model_selection(X, y)
 ROC(X, y, classlds, 'Normilized Lipids + Hormonal Data\n(33 variables selected)')
 "Self Organizing Map (SOM)"
 # som = SOM(20, 40, feature_names, X)
 # print('SOM Training:')
 # som.train(n_job=2, verbose='info')
 # SOM_plot(som, len(classlds)*5)
```

**Lengthy Table 6**

| Patient ID | Group | |
|---|---|---|
| 34 | 0 | 0 corresponds to Controls with BMI less than 27.5 kg/m2 |
| 50 | 0 | 1 corresponds to Controls with BMI greater than or equal to 27.5 kg/m² |
| 56 | 0 | 2 corresponds to NAFL |
| 63 | 0 | 3 corresponds to NASH |
| 67 | 0 | |
| 70 | 0 | |
| 72 | 0 | |
| 73 | 0 | |
| 74 | 0 | |
| 75 | 0 | |
| 76 | 0 | |
| 77 | 0 | |
| 78 | 0 | |
| 79 | 0 | |
| 80 | 0 | |
| 81 | 0 | |
| 82 | 0 | |
| 90 | 0 | |
| 91 | 0 | |
| 92 | 0 | |
| 93 | 0 | |
| 94 | 0 | |
| 13 | 1 | |
| 15 | 1 | |
| 33 | 1 | |
| 36 | 1 | |
| 37 | 1 | |
| 38 | 1 | |
| 39 | 1 | |
| 40 | 1 | |
| 43 | 1 | |
| 44 | 1 | |
| 46 | 1 | |
| 51 | 1 | |
| 52 | 1 | |
| 53 | 1 | |
| 54 | 1 | |
| 55 | 1 | |
| 57 | 1 | |
| 59 | 1 | |
| 61 | 1 | |
| 62 | 1 | |
| 66 | 1 | |
| 84 | 1 | |
| 85 | 1 | |
| 86 | 1 | |
| 87 | 1 | |
| 88 | 1 | |
| 89 | 1 | |
| 12 | 2 | |
| 18 | 2 | |
| 19 | 2 | |
| 24 | 2 | |
| 25 | 2 | |
| 27 | 2 | |
| 29 | 2 | |
| 30 | 2 | |
| 31 | 2 | |
| 32 | 2 | |
| 41 | 2 | |
| 42 | 2 | |
| 48 | 2 | |
| 49 | 2 | |
| 65 | 2 | |
| 2 | 3 | |
| 3 | 3 | |
| 4 | 3 | |
| 6 | 3 | |
| 8 | 3 | |
| 14 | 3 | |
| 16 | 3 | |
| 21 | 3 | |
| 22 | 3 | |
| 23 | 3 | |
| 26 | 3 | |
| 45 | 3 | |
| 58 | 3 | |
| 60 | 3 | |
| 64 | 3 | |
| 68 | 3 | |

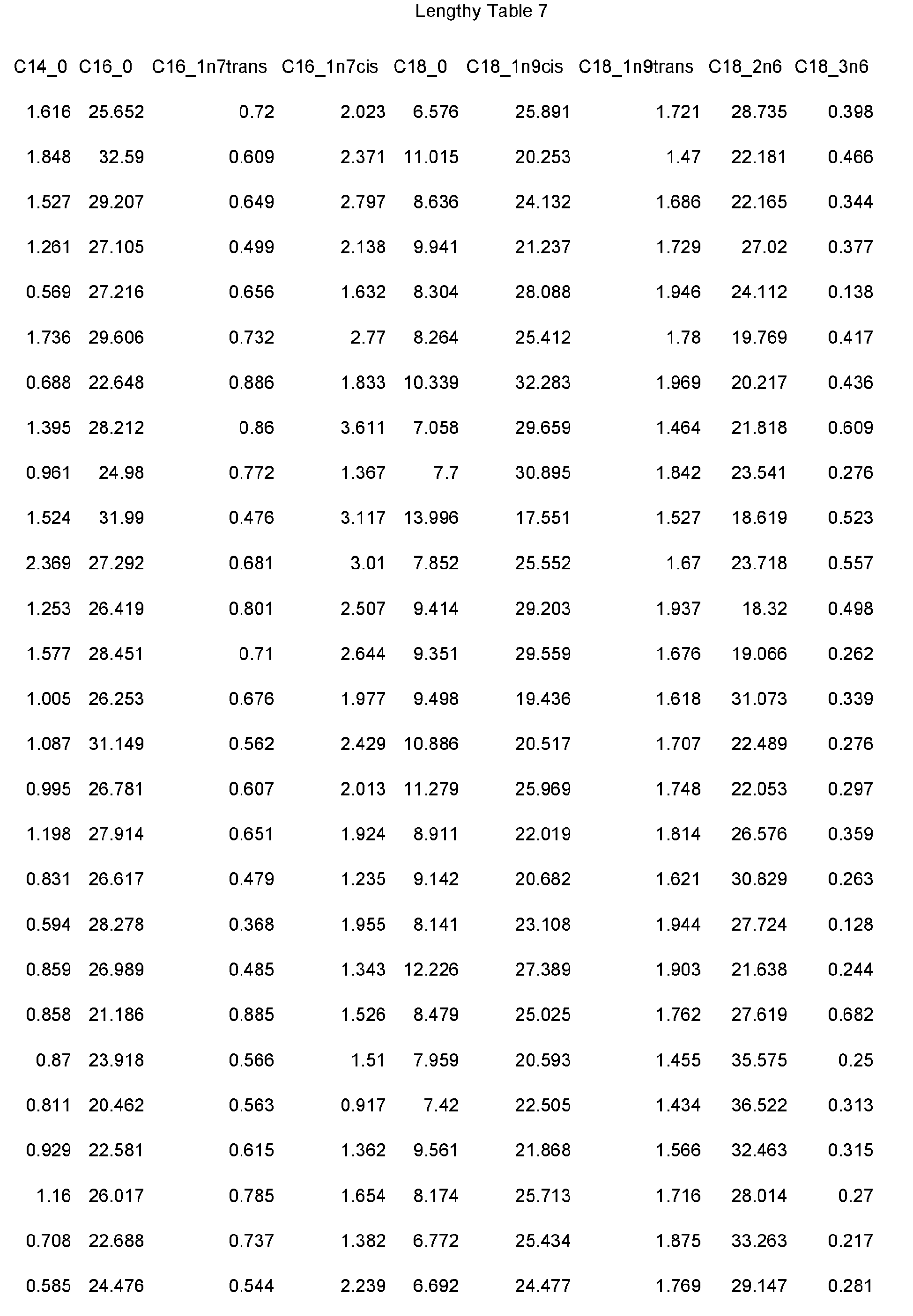

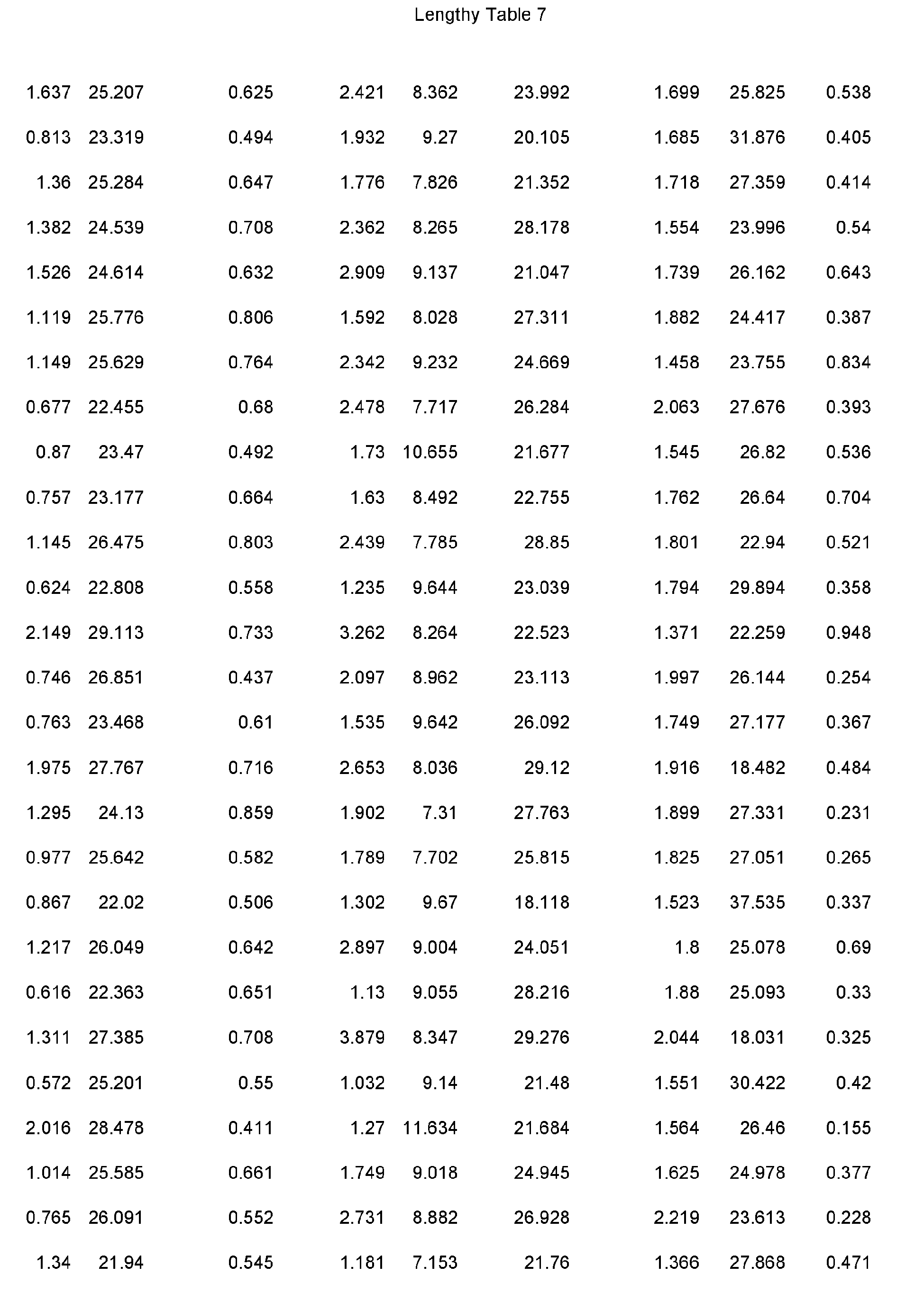

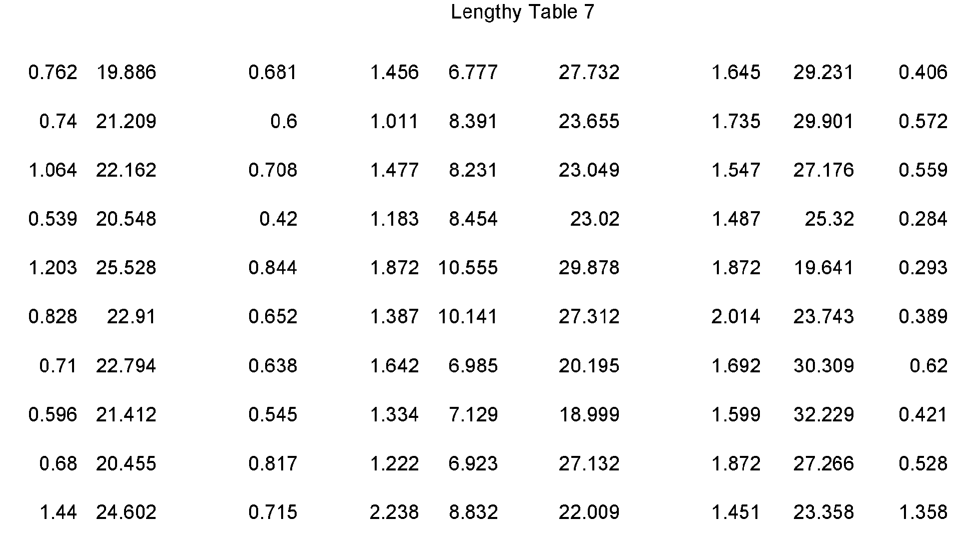

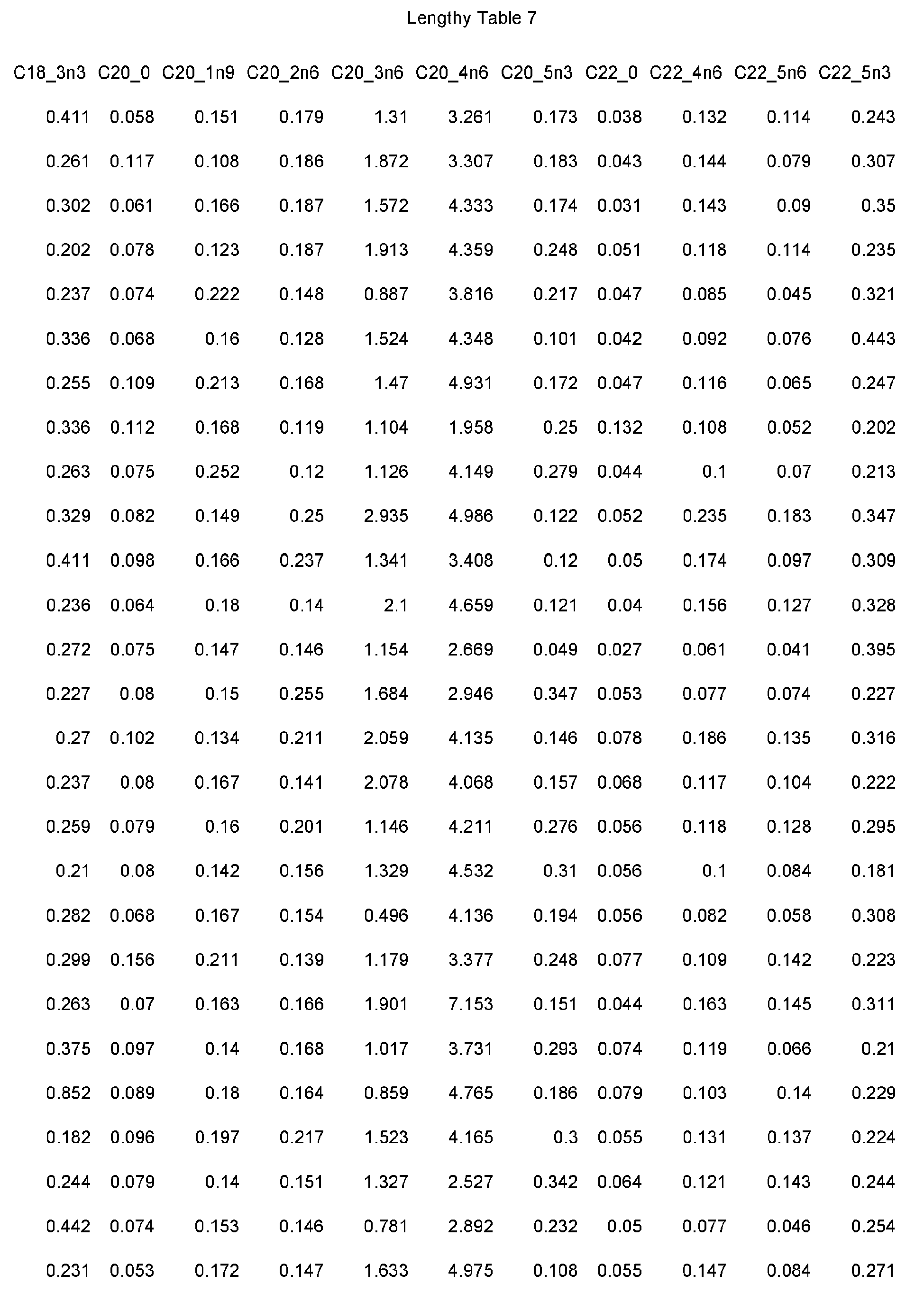

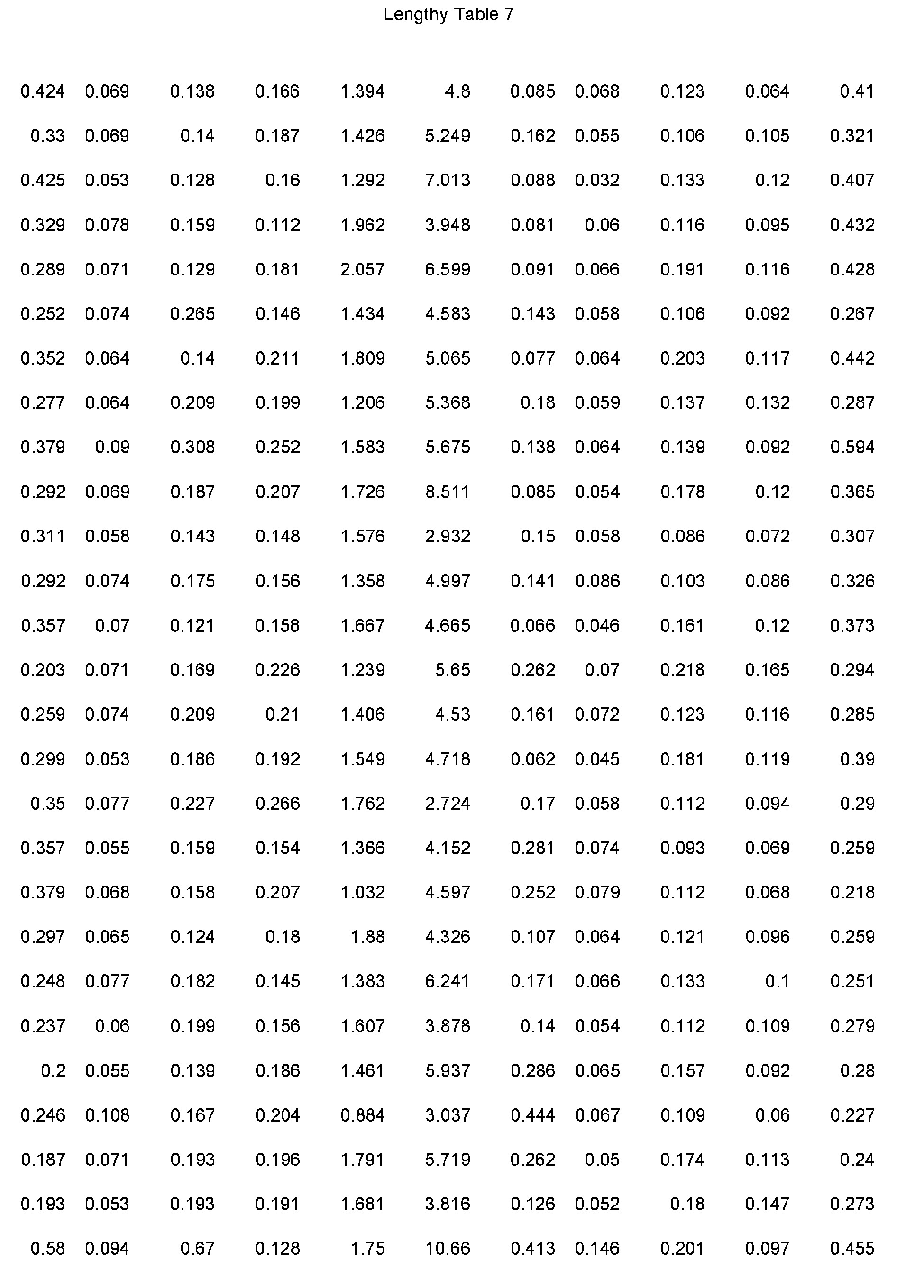

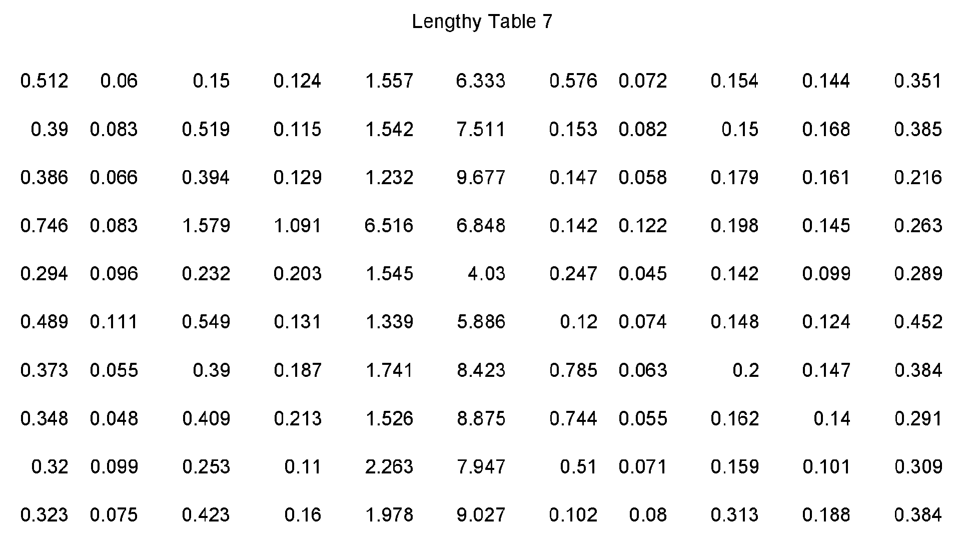

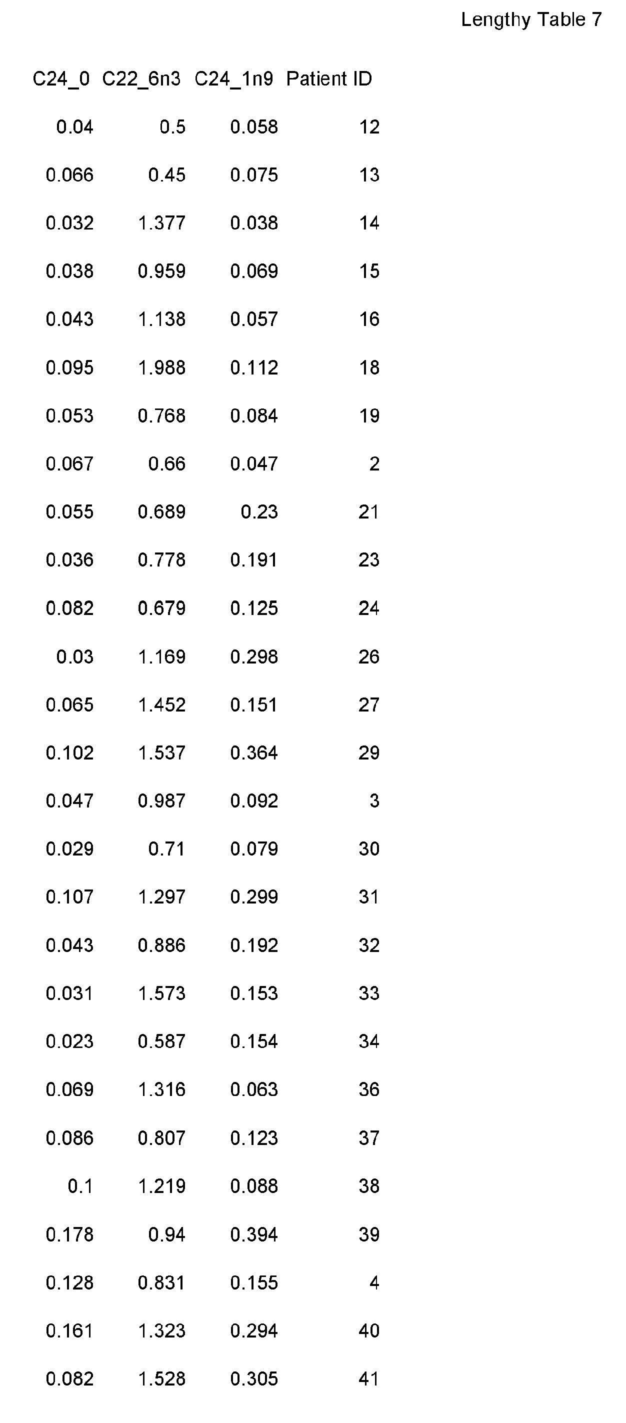

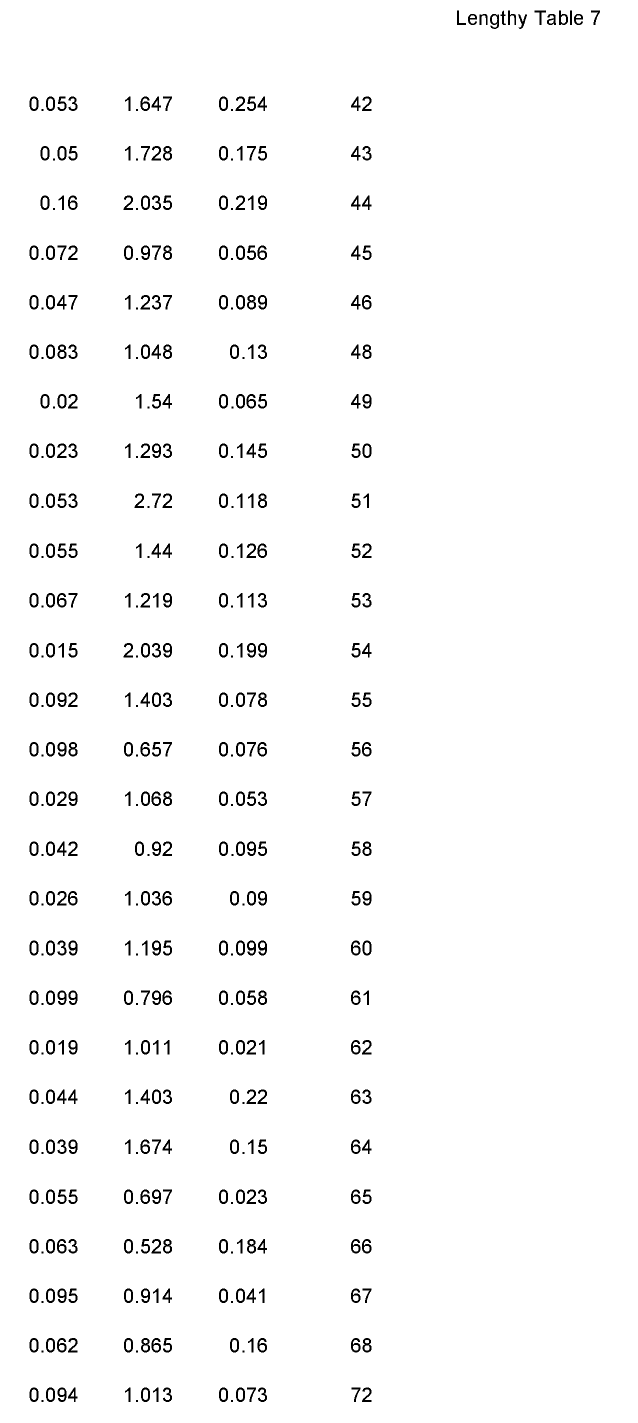

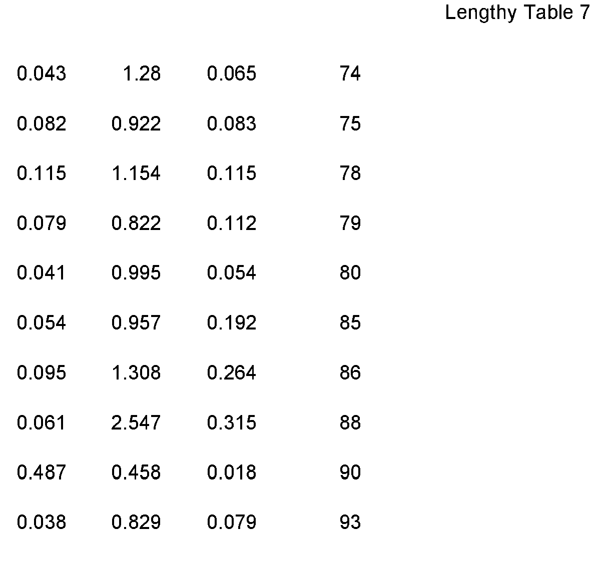

**Lengthy Table 8**

| | | | | | | | | | |
|---|---|---|---|---|---|---|---|---|---|
| 1579 | 1661 | 1784 | 1825 | 1836 | 1866 | 1907 | 1988 | 1999 | 2040 |
| 0.015794 | 0 | 0.02468 | 0 | 0.133089 | 0.012687 | 0.012242 | 0.00745 | 0 | 0.208356 |
| 0.040057 | 0.014486 | 0.040815 | 0.003657 | 0.366352 | 0.038013 | 0.008109 | 0.002543 | 0.002625 | 0.387011 |
| 0.019555 | 0.002831 | 0.026956 | 0.002633 | 0.226381 | 0.01677 | 0.017664 | 0.008776 | 0.002655 | 0.283231 |
| 0.044191 | 0.010413 | 0.056102 | 0.003798 | 0.21211 | 0.022908 | 0.017185 | 0.015739 | 0.003052 | 0.281073 |
| 0.085315 | 0.015028 | 0.094653 | 0 | 0.404104 | 0.046251 | 0.05353 | 0.027609 | 0 | 0.326074 |
| 0.062356 | 0.006643 | 0.077346 | 0.007318 | 0.097503 | 0.024841 | 0.00453 | 0.018918 | 0.002464 | 0.249709 |
| 0.024222 | 0.0027 | 0.030891 | 0 | 0.027826 | 0.011717 | 0.00453 | 0.006906 | 0 | 0.122337 |
| 0.031208 | 0.003466 | 0.032881 | 0.003676 | 0.082677 | 0.010132 | 0.006571 | 0.010403 | 0.002458 | 0.110247 |
| 0.029209 | 0.005341 | 0.037168 | 0.00361 | 0.217706 | 0.015813 | 0.022122 | 0.011109 | 0.002821 | 0.198694 |
| 0.044448 | 0.020283 | 0.061675 | 0.009373 | 0.249947 | 0.073119 | 0.015873 | 0.018468 | 0.004127 | 0.526125 |
| 0.063556 | 0.02075 | 0.109803 | 0.011589 | 0.393127 | 0.07835 | 0.062052 | 0.035523 | 0.005743 | 0.600695 |
| 0.113824 | 0.010347 | 0.131418 | 0.010475 | 0.346144 | 0.028141 | 0.03577 | 0.033641 | 0.005125 | 0.378381 |
| 0.102377 | 0.006093 | 0.076932 | 0.012245 | 0.153589 | 0.015286 | 0.010534 | 0.024268 | 0.006732 | 0.22355 |
| 0.069065 | 0.005756 | 0.073168 | 0.007856 | 0.131352 | 0.016217 | 0.010919 | 0.023202 | 0.00368 | 0.141241 |
| 0.033537 | 0 | 0.040327 | 0.005005 | 0.074552 | 0.010116 | 0.008277 | 0.010777 | 0 | 0.135967 |
| 0.083366 | 0.004944 | 0.105369 | 0.019153 | 0.241353 | 0.016948 | 0.012197 | 0.021487 | 0.007421 | 0.23785 |
| 0.023672 | 0.009381 | 0.023672 | 0.002465 | 0.148075 | 0.024035 | 0.015369 | 0.006903 | 0 | 0.18849 |
| 0.022356 | 0.004811 | 0.023864 | 0.002086 | 0.055263 | 0.008993 | 0.006626 | 0.006077 | 0 | 0.07033 |
| 0.039649 | 0.004866 | 0.048401 | 0.003793 | 0.144676 | 0.01392 | 0.010987 | 0.014598 | 0 | 0.200856 |
| 0.011648 | 0 | 0.011429 | 0 | 0.091201 | 0.005928 | 0.009325 | 0.002882 | 0 | 0.128987 |
| 0.01583 | 0.004642 | 0.016657 | 0 | 0.095015 | 0.012333 | 0.003907 | 0.004926 | 0 | 0.147548 |
| 0.036665 | 0.004539 | 0.03558 | 0.001937 | 0.102369 | 0.012774 | 0.005999 | 0.006123 | 0.001188 | 0.106539 |
| 0.019383 | 0.002027 | 0.024031 | 0.001658 | 0.148408 | 0.009045 | 0.007923 | 0.005586 | 0.001601 | 0.195059 |
| 0.018263 | 0.003723 | 0.019211 | 0.001855 | 0.103689 | 0.010678 | 0.004028 | 0.004095 | 0.001104 | 0.130279 |
| 0.026598 | 0.003531 | 0.025083 | 0.004341 | 0.108791 | 0.009941 | 0.007051 | 0.007148 | 0.002296 | 0.110328 |
| 0.06619 | 0.005206 | 0.058226 | 0.005386 | 0.127199 | 0.01883 | 0.008612 | 0.013954 | 0.002779 | 0.160887 |
| 0.033939 | 0.006533 | 0.035198 | 0 | 0.370247 | 0.016425 | 0.014159 | 0.004685 | 0.007644 | 0.287127 |
| 0.04356 | 0.002725 | 0.042934 | 0.003742 | 0.066018 | 0.01084 | 0.007777 | 0.007531 | 0.001635 | 0.130287 |
| 0.01652 | 0.003158 | 0.020473 | 0 | 0.11191 | 0.012731 | 0.006704 | 0.00271 | 0.004401 | 0.209168 |
| 0.015743 | 0.002759 | 0.017074 | 0 | 0.040499 | 0.009688 | 0.007438 | 0.003796 | 0.080809 | 0.014734 |
| 0.004797 | 0 | 0.007119 | 0 | 0.048708 | 0.005981 | 0.005689 | 0.002322 | 0.001973 | 0.087101 |
| 0.059709 | 0.00888 | 0.078416 | 0.011225 | 0.220525 | 0.033663 | 0.032249 | 0.025083 | 0.00512 | 0.403019 |
| 0.019573 | 0.003753 | 0.017806 | 0.001618 | 0.10957 | 0.010809 | 0.009041 | 0.004837 | 0.001414 | 0.139867 |
| 0.111621 | 0.01856 | 0.142545 | 0.019456 | 0.191297 | 0.046854 | 0.008446 | 0.030315 | 0.006499 | 0.266602 |
| 0.011687 | 0 | 0.0114 | 0.004097 | 0.025839 | 0.007869 | 0.003825 | 0.003901 | 0 | 0.054621 |
| 0.023849 | 0.002447 | 0.024404 | 0.001913 | 0.110902 | 0.006225 | 0.007828 | 0.005422 | 0.001245 | 0.128224 |
| 0.018553 | 0.003098 | 0.022684 | 0.002041 | 0.096624 | 0.011473 | 0.005471 | 0.00758 | 0.00158 | 0.19758 |
| 0.021134 | 0.003427 | 0.029736 | 0.002013 | 0.174206 | 0.011333 | 0.014455 | 0.008256 | 0 | 0.266573 |
| 0.040035 | 0.012177 | 0.038198 | 0.002803 | 0.127643 | 0.018438 | 0.008543 | 0.008877 | 0.001489 | 0.120911 |
| 0.032123 | 0.005414 | 0.035811 | 0.003324 | 0.101188 | 0.014797 | 0.004272 | 0.006929 | 0.001674 | 0.123759 |
| 0.014704 | 0.001375 | 0.017428 | 0.001602 | 0.052476 | 0.007928 | 0.001984 | 0.004154 | 0 | 0.129554 |
| 0.027542 | 0.00285 | 0.037066 | 0.002719 | 0.039683 | 0.012524 | 0.002695 | 0.007993 | 0.001722 | 0.09358 |
| 0.04623 | 0.015677 | 0.047835 | 0.004746 | 0.197233 | 0.037787 | 0.012941 | 0.012495 | 0.002159 | 0.263843 |
| 0.023087 | 0 | 0.022242 | 0 | 0.174085 | 0.013383 | 0.018608 | 0 | 0.006141 | 0.221863 |
| 0.116675 | 0.020099 | 0.145668 | 0.016914 | 0.282832 | 0.062003 | 0.014103 | 0.038105 | 0.006362 | 0.594978 |
| 0.011012 | 0 | 0.012157 | 0.01421 | 0.04381 | 0 | 0 | 0 | 0 | 0.072326 |
| 0.025339 | 0 | 0.026589 | 0.006485 | 0.1109 | 0.00891 | 0.008467 | 0.006731 | 0 | 0.182862 |
| 0.097067 | 0.03146 | 0.108967 | 0.014496 | 0.330235 | 0.110771 | 0.050429 | 0.035106 | 0.005047 | 0.673076 |
| 0.020719 | 0.003442 | 0.023045 | 0.001471 | 0.124387 | 0.016076 | 0.0088 | 0.006087 | 0.001547 | 0.22128 |
| 0.050815 | 0 | 0.052873 | 0 | 0.090259 | 0.010808 | 0.016281 | 0.014015 | 0 | 0.133984 |
| 0.03746 | 0 | 0.033474 | 0.01401 | 0.146078 | 0.019835 | 0.013304 | 0.008007 | 0 | 0.231442 |
| 0.023702 | 0 | 0.024195 | 0 | 0.072708 | 0.005987 | 0.004861 | 0.00613 | 0 | 0.087924 |
| 0.025563 | 0 | 0.036839 | 0.020273 | 0.009785 | 0.11463 | 0.011112 | 0.013518 | 0 | 0.174519 |
| 0.051319 | 0.002532 | 0.051404 | 0.002453 | 0.135149 | 0.011244 | 0.011719 | 0.012277 | 0.001768 | 0.161526 |
| 0.125005 | 0.011974 | 0.171933 | 0.022546 | 0.395375 | 0.052802 | 0.040749 | 0.055437 | 0.009109 | 0.875362 |
| 0.013936 | 0.004561 | 0.016432 | 0.002489 | 0.076038 | 0.015728 | 0.003074 | 0.004389 | 0 | 0.190576 |
| 0.03814 | 0 | 0.026487 | 0 | 0.260877 | 0.011264 | 0.064215 | 0.00913 | 0 | 0.428024 |
| 0.025815 | 0.005287 | 0.030669 | 0.003088 | 0.116211 | 0.015551 | 0.009188 | 0.007773 | 0.22239 | 0 |
| 0.073963 | 0 | 0.075089 | 0 | 0.310773 | 0.053505 | 0.035445 | 0.013336 | 0 | 0.430556 |
| 0.024602 | 0.007317 | 0.029536 | 0 | 0.087777 | 0.018035 | 0.003996 | 0.006711 | 0 | 0.124339 |
| 0.052995 | 0.005741 | 0.060481 | 0.007407 | 0.152269 | 0.025241 | 0.010546 | 0.015797 | 0 | 0.210539 |
| 0.050504 | 0.008385 | 0.060819 | 0 | 0.109957 | 0.0333 | 0.007464 | 0.011136 | 0 | 0.287675 |
| 0.083274 | 0.00675 | 0.091102 | 0.0054 | 0.130772 | 0.023776 | 0.013867 | 0.017617 | 0.002333 | 0.244992 |
| 0.028312 | 0.003021 | 0.0317 | 0.002067 | 0.160309 | 0.010295 | 0.007615 | 0.005639 | 0.001349 | 0.223666 |
| 0.035562 | 0 | 0.03639 | 0.003106 | 0.109552 | 0.009218 | 0.005368 | 0.008742 | 0 | 0.240045 |
| 0.05361 | 0.005054 | 0.05157 | 0.004386 | 0.168792 | 0.013028 | 0.010092 | 0.010842 | 0 | 0.229087 |
| 0.032814 | 0.005333 | 0.023363 | 0 | 0.115951 | 0.017377 | 0.009734 | 0.005414 | 0 | 0.153339 |
| 0.161071 | 0.008951 | 0.14161 | 0.008216 | 0.1397 | 0.028664 | 0.007431 | 0.022092 | 0 | 0.269486 |
| 0.084388 | 0.005525 | 0.096676 | 0.008525 | 0.080274 | 0.020356 | 0.008171 | 0.018534 | 0 | 0.200483 |
| 0.120148 | 0.019949 | 0.096135 | 0 | 0.250291 | 0.05228 | 0.019139 | 0.022047 | 0 | 0.561842 |
| 0.031134 | 0 | 0.036969 | 0 | 0.103091 | 0.014978 | 0.006438 | 0.006529 | 0 | 0.251283 |
| 0.012949 | 0 | 0.015774 | 0 | 0.058588 | 0.005011 | 0.003664 | 0.003298 | 0 | 0.09238 |
| 0.04804 | 0 | 0.044222 | 0 | 0.271771 | 0.021151 | 0.025299 | 0.012361 | 0 | 0.273958 |
| 0.020128 | 0 | 0.02826 | 0 | 0.107839 | 0.012392 | 0 | 0.005699 | 0 | 0.185771 |
| 0.068561 | 0.009416 | 0.083848 | 0.008122 | 0.188706 | 0.023322 | 0.012625 | 0.020101 | 0 | 0.420238 |
| 0.023051 | 0.005461 | 0.035136 | 0.003664 | 0.171065 | 0.018971 | 0.011574 | 0.009689 | 0.003162 | 0.333515 |
| 0.053296 | 0.011331 | 0.050354 | 0 | 0.226185 | 0.025539 | 0.020291 | 0.015262 | 0 | 0.301748 |
| 0.049864 | 0 | 0.046099 | 0 | 0.067812 | 0.011445 | 0 | 0.013434 | 0 | 0.167198 |
| 0.027913 | 0.00968 | 0.031396 | 0 | 0.155904 | 0.027586 | 0.011764 | 0 | 0 | 0.305235 |
| 0.035179 | 0.008026 | 0.03362 | 0.003338 | 0.147862 | 0.022065 | 0.010005 | 0.007763 | 0 | 0.287154 |
| 2070 | 2081 | 2111 | 2192 | 2244 | 2274 | 2285 | 2315 | 2396 | 2418 |
| 0.020305 | 0.035922 | 0.018222 | 0.014009 | 0.087946 | 0 | 0.061908 | 0.008913 | 0.026291 | 0.027316 |
| 0.040266 | 0.050003 | 0.011257 | 0.011937 | 0.136562 | 0.002657 | 0.0643 | 0.005754 | 0.015449 | 0.042955 |
| 0.029322 | 0.056625 | 0.02972 | 0.013608 | 0.112492 | 0.003994 | 0.090275 | 0.014841 | 0.020654 | 0.032563 |
| 0.031945 | 0.03109 | 0.015513 | 0.026348 | 0.120183 | 0.00244 | 0.059126 | 0.007388 | 0.033933 | 0.034338 |
| 0.059098 | 0.129524 | 0.064896 | 0.041609 | 0.118904 | 0.006662 | 0.139276 | 0.033424 | 0.045995 | 0.037842 |
| 0.034753 | 0.01413 | 0.007645 | 0.024694 | 0.15594 | 0.00508 | 0.03413 | 0.005431 | 0.030532 | 0.033679 |
| 0.023354 | 0.005909 | 0.009495 | 0.01057 | 0.09995 | 0.00329 | 0.023592 | 0.00583 | 0.026209 | 0.018344 |
| 0.012999 | 0.017549 | 0.009421 | 0.014042 | 0.040149 | 0.00241 | 0.027596 | 0.005457 | 0.012891 | 0.014468 |
| 0.022189 | 0.037455 | 0.030247 | 0.016679 | 0.063708 | 0.002645 | 0.052457 | 0.012014 | 0.026102 | 0.047978 |
| 0.080073 | 0.051281 | 0.028965 | 0.030177 | 0.350563 | 0.006282 | 0.127388 | 0.014947 | 0.032533 | 0.053796 |
| 0 | 0.152497 | 0.100212 | 0.058476 | 0.277214 | 0.007231 | 0.318029 | 0.038414 | 0.087323 | 0.06912 |
| 0.041788 | 0.083578 | 0.04276 | 0.048142 | 0.153611 | 0.003894 | 0.131133 | 0.016536 | 0.054418 | 0.049049 |
| 0.019837 | 0.033096 | 0.015846 | 0.039986 | 0.097941 | 0.005639 | 0.077095 | 0.010254 | 0.015529 | 0.038292 |
| 0.020746 | 0.03171 | 0.015866 | 0.046071 | 0.062269 | 0 | 0.057289 | 0.00821 | 0.028041 | 0.04097 |
| 0.014698 | 0.013205 | 0.010373 | 0.0146 | 0.07141 | 0 | 0.035602 | 0.004556 | 0.014294 | 0.050526 |
| 0.021712 | 0.052734 | 0.018183 | 0.029461 | 0.079162 | 0.003623 | 0.085479 | 0.011847 | 0.014351 | 0.076736 |
| 0.024049 | 0.029902 | 0.02032 | 0.010558 | 0.077716 | 0.001991 | 0.045505 | 0.007391 | 0.016419 | 0.0708 |
| 0.009866 | 0.011342 | 0.010585 | 0.007309 | 0.028758 | 0 | 0.026848 | 0.005252 | 0.011639 | 0.018281 |
| 0.018543 | 0.013265 | 0.014991 | 0.017055 | 0.083295 | 0.002485 | 0.032612 | 0.006643 | 0.029066 | 0.027191 |
| 0.009062 | 0.021974 | 0.012561 | 0.005169 | 0.042825 | 0 | 0.044566 | 0.005151 | 0.006936 | 0.040504 |
| 0.012834 | 0.015749 | 0.006541 | 0.005579 | 0.053734 | 0.001816 | 0.025895 | 0.003748 | 0.008535 | 0.044024 |
| 0.011621 | 0.004517 | 0.007271 | 0.008876 | 0.029783 | 0.001213 | 0.018454 | 0.002837 | 0.010414 | 0.013002 |
| 0.015703 | 0.031036 | 0.018933 | 0.009439 | 0.067913 | 0.002088 | 0.044649 | 0.006897 | 0.018469 | 0.018278 |
| 0.012355 | 0.026922 | 0.007484 | 0.006767 | 0.057456 | 0.001431 | 0.039398 | 0.003832 | 0.010628 | 0.002857 |
| 0.012177 | 0.029723 | 0.008042 | 0.013326 | 0.049982 | 0.001386 | 0.039254 | 0.004145 | 0.006917 | 0.022277 |
| 0.024153 | 0.026473 | 0.012133 | 0.019654 | 0.073007 | 0.001725 | 0.04525 | 0.005311 | 0.018067 | 0.041224 |
| 0.016324 | 0.04053 | 0.011449 | 0.013691 | 0.068931 | 0.001857 | 0.041285 | 0.003874 | 0.019762 | 0.034425 |
| 0.021772 | 0.017452 | 0.009354 | 0.011453 | 0.08231 | 0.001819 | 0.041322 | 0.005256 | 0.012421 | 0.018394 |
| 0.020917 | 0.020884 | 0.010153 | 0.008833 | 0.121063 | 0 | 0.046115 | 0.005083 | 0.015149 | 0.04086 |
| 0.004902 | 0.004863 | 0.012776 | 0.007183 | 0.042738 | 0 | 0.02401 | 0.005512 | 0.013142 | 0.024781 |
| 0.008534 | 0.009109 | 0.008847 | 0.003858 | 0.041808 | 0 | 0.020888 | 0.003991 | 0.007153 | 0.030371 |
| 0.045991 | 0.064923 | 0.041862 | 0.039043 | 0.222239 | 0.008801 | 0.148454 | 0.018713 | 0.040924 | 0.027576 |
| 0.015545 | 0.028858 | 0.012119 | 0.006961 | 0.051397 | 0.002144 | 0.048009 | 0.005143 | 0.014079 | 0.026412 |
| 0.043445 | 0.045709 | 0.018813 | 0.042838 | 0.12245 | 0.004378 | 0.105606 | 0.012181 | 0.026415 | 0.021954 |
| 0.006907 | 0.008547 | 0.00396 | 0.004724 | 0.028772 | 0 | 0.019785 | 0.00249 | 0.00665 | 0.030625 |
| 0.009579 | 0.024023 | 0.01039 | 0.010116 | 0.045197 | 0.002084 | 0.042971 | 0.004161 | 0.011941 | 0.019237 |
| 0.017793 | 0.016865 | 0.0107 | 0.010788 | 0.095599 | 0.001471 | 0.041927 | 0.004686 | 0.016829 | 0.03624 |
| 0.016606 | 0.02401 | 0.018083 | 0.012964 | 0.08969 | 0 | 0.05706 | 0.00682 | 0.019534 | 0.036451 |
| 0.014638 | 0.025354 | 0.007851 | 0.014714 | 0.042249 | 0 | 0.035705 | 0.00336 | 0.011932 | 0.021583 |
| 0.016064 | 0.019362 | 0.006895 | 0.012133 | 0.055271 | 0.001459 | 0.027764 | 0.0038 | 0.014767 | 0.013774 |
| 0.01691 | 0.008183 | 0.004282 | 0.006775 | 0.08042 | 0.002568 | 0.025806 | 0.00326 | 0.01162 | 0.019059 |
| 0.022152 | 0.010613 | 0.005777 | 0.012316 | 0.057603 | 0.002631 | 0.025031 | 0.004364 | 0.016904 | 0.022809 |
| 0.032217 | 0.028936 | 0.016449 | 0.018236 | 0.114174 | 0.002661 | 0.050477 | 0.007979 | 0.022041 | 0.017676 |
| 0.021441 | 0.034667 | 0.022973 | 0.010322 | 0.066573 | 0 | 0.065093 | 0.008266 | 0.020291 | 0.024717 |
| 0.069656 | 0.070608 | 0.029088 | 0.057587 | 0.31628 | 0.009012 | 0.172492 | 0.018393 | 0.054536 | 0.067584 |
| 0.008189 | 0.007602 | 0 | 0.00538 | 0.045737 | 0 | 0.019181 | 0 | 0.005656 | 0.011121 |
| 0.016604 | 0.026205 | 0.017417 | 0.010294 | 0.064458 | 0 | 0.060559 | 0.008209 | 0.016289 | 0.020134 |
| 0.121493 | 0.058034 | 0.074907 | 0.056571 | 0.39501 | 0.008831 | 0.165598 | 0.031426 | 0.077578 | 0.048551 |
| 0.028518 | 0.024019 | 0.012148 | 0.00844 | 0.112704 | 0.004054 | 0.052492 | 0.006751 | 0.018657 | 0.026781 |
| 0.016566 | 0.018742 | 0.011302 | 0.019456 | 0.052996 | 0.005871 | 0.02566 | 0.007133 | 0.017162 | 0.02624 |
| 0.026442 | 0.024073 | 0.022089 | 0.014858 | 0.084946 | 0 | 0.049904 | 0.008267 | 0.018339 | 0.023419 |
| 0.009777 | 0.017762 | 0.004869 | 0.008701 | 0.033063 | 0 | 0.034244 | 0.003581 | 0.011137 | 0.011817 |
| 0.020795 | 0.021444 | 0.009606 | 0.01332 | 0.072889 | 0 | 0.03852 | 0.005412 | 0.021986 | 0.02285 |
| 0.026093 | 0.025522 | 0.010142 | 0.020447 | 0.073959 | 0.002105 | 0.038802 | 0.004711 | 0.029083 | 0.029791 |
| 0.08557 | 0.085183 | 0.074262 | 0.09926 | 0.502426 | 0.016302 | 0.262561 | 0.036914 | 0.069996 | 0.078185 |
| 0.015907 | 0.014872 | 0.006883 | 0.007134 | 0.098178 | 0.002007 | 0.037392 | 0.00426 | 0.008763 | 0.014831 |
| 0.020055 | 0.064913 | 0.077186 | 0.012238 | 0.208652 | 0.005656 | 0.164895 | 0.020978 | 0.012032 | 0.028813 |
| 0.018423 | 0.025114 | 0.016319 | 0.015023 | 0.093913 | 0.001826 | 0.061961 | 0.007847 | 0.015751 | 0.024428 |
| 0.049657 | 0.102408 | 0.04602 | 0.025953 | 0.217111 | 0 | 0.157694 | 0.016782 | 0.035709 | 0.024107 |
| 0.012985 | 0.013177 | 0.00627 | 0.009875 | 0.04957 | 0 | 0.027387 | 0.003347 | 0.008181 | 0.010634 |
| 0.025469 | 0.040642 | 0.019612 | 0.024507 | 0.092253 | 0.002407 | 0.07868 | 0.010113 | 0.014968 | 0.018862 |
| 0.037953 | 0.022719 | 0.016455 | 0.025619 | 0.184366 | 0 | 0.059605 | 0.007586 | 0.040786 | 0.070627 |
| 0.032458 | 0.030773 | 0.022158 | 0.034456 | 0.129961 | 0.002399 | 0.074523 | 0.009302 | 0.038958 | 0.035062 |
| 0.013717 | 0.01985 | 0.010904 | 0.009879 | 0.071167 | 0.001798 | 0.036587 | 0.004457 | 0.012495 | 0 |
| 0.016149 | 0.012487 | 0.006787 | 0.013033 | 0.105429 | 0.002962 | 0.029673 | 0.003637 | 0.014764 | 0.018019 |
| 0.018053 | 0.019933 | 0.01264 | 0.015366 | 0.080745 | 0.003174 | 0.036679 | 0.005964 | 0.017527 | 0.016388 |
| 0.018952 | 0.015539 | 0.00981 | 0.007258 | 0.059343 | 0.002963 | 0.027587 | 0.004508 | 0.008124 | 0.0145 |
| 0.03779 | 0.023022 | 0.015796 | 0.030301 | 0.139923 | 0.004844 | 0.043786 | 0.00949 | 0.027778 | 0.029584 |
| 0.030516 | 0.015612 | 0.015189 | 0.030007 | 0.126381 | 0.004062 | 0.048023 | 0.010087 | 0.02434 | 0.020429 |
| 0.063448 | 0.044846 | 0.032959 | 0.031935 | 0.300721 | 0.007253 | 0.115687 | 0.019883 | 0.035016 | 0.065761 |
| 0.023187 | 0.01893 | 0.011088 | 0.016163 | 0.131934 | 0.003525 | 0.054317 | 0.006133 | 0.028481 | 0.023957 |
| 0.008744 | 0.009216 | 0.005471 | 0.004729 | 0.041724 | 0.002129 | 0.016458 | 0.003066 | 0.007252 | 0.016544 |
| 0.02629 | 0.04156 | 0.026517 | 0.017829 | 0.069934 | 0 | 0.064796 | 0.011638 | 0.024618 | 0.05097 |
| 0.014801 | 0.022384 | 0.008086 | 0.01112 | 0.076648 | 0 | 0.042522 | 0.004899 | 0.019156 | 0.02547 |
| 0.036507 | 0.028497 | 0.019193 | 0.027066 | 0.200299 | 0.007413 | 0.065228 | 0.010921 | 0.03239 | 0.029119 |
| 0.030945 | 0.037192 | 0.020465 | 0.017743 | 0.162299 | 0.004382 | 0.084812 | 0.010816 | 0.026089 | 0.036971 |
| 0.032369 | 0.024858 | 0.026044 | 0.021147 | 0.095217 | 0.00863 | 0.052352 | 0.012429 | 0.029865 | 0.035992 |
| 0.020194 | 0.014461 | 0.011894 | 0.018525 | 0.07609 | 0 | 0.030505 | 0 | 0.021028 | 0.022568 |
| 0.033322 | 0.033567 | 0.022838 | 0.013205 | 0.119333 | 0 | 0.072419 | 0.010622 | 0.018855 | 0.039629 |
| 0.029998 | 0.027165 | 0.013729 | 0.011513 | 0.118361 | 0.00462 | 0.053469 | 0.008102 | 0.016613 | 0.021369 |
| 2431 | 2448 | 2489 | 2519 | 2592 | 2605 | 2635 | 2646 | 2663 | 2676 |
| 0.404817 | 0.024661 | 0.025234 | 0 | 0 | 0.148378 | 0.009529 | 0.01177 | 0.012048 | 0.038153 |
| 0.499826 | 0.042567 | 0.016884 | 0.001653 | 0 | 0.128355 | 0.006629 | 0.003953 | 0.009919 | 0.008082 |
| 0.442413 | 0.025663 | 0.036444 | 0.002978 | 0 | 0.198754 | 0.014782 | 0.020299 | 0.012253 | 0.057945 |
| 0.481742 | 0.027963 | 0.025545 | 0 | 0 | 0.202636 | 0.011543 | 0.013781 | 0.011937 | 0.026837 |
| 0.616983 | 0.021695 | 0.05898 | 0.013635 | 0.007831 | 0.223051 | 0.019078 | 0.024647 | 0.012411 | 0.062824 |
| 0.417934 | 0.009652 | 0.017529 | 0.003869 | 0.005042 | 0.154797 | 0.008624 | 0.003541 | 0.007066 | 0.011719 |
| 0.429634 | 0.011908 | 0.016161 | 0.001594 | 0.003578 | 0.146857 | 0.009164 | 0.004233 | 0.005084 | 0.037476 |
| 0.207677 | 0.00935 | 0.014404 | 0.001984 | 0.002745 | 0.075758 | 0.005785 | 0.005758 | 0.005938 | 0.01938 |
| 0.515585 | 0.021085 | 0.03578 | 0.003842 | 0.009726 | 0.176098 | 0.012147 | 0.012266 | 0.015564 | 0.046329 |
| 0.527523 | 0.041336 | 0.057525 | 0.004559 | 0.012284 | 0.377519 | 0.016597 | 0.013022 | 0.016379 | 0.067951 |
| 0.913724 | 0.060924 | 0.115532 | 0.007536 | 0.016242 | 0.404801 | 0.02715 | 0.030191 | 0.023557 | 0.102381 |
| 0.58539 | 0.053942 | 0.063104 | 0.004161 | 0.009769 | 0.259365 | 0.017544 | 0.025393 | 0.020224 | 0.057424 |
| 0.413287 | 0.011136 | 0 | 0.046444 | 0.003638 | 0.262249 | 0.010888 | 0.019352 | 0.01338 | 0.002973 |
| 0.376898 | 0.024152 | 0.038345 | 0.002993 | 0.008977 | 0.207031 | 0.011805 | 0.014043 | 0.013762 | 0.037165 |
| 0.310836 | 0.013183 | 0.014969 | 0 | 0.007062 | 0.11454 | 0.006371 | 0.007109 | 0.015954 | 0.032779 |
| 0.32607 | 0.013198 | 0.046357 | 0.003169 | 0.013925 | 0.244576 | 0.014038 | 0.026233 | 0.015945 | 0.040457 |
| 0.454663 | 0.020684 | 0.020881 | 0.002078 | 0.008094 | 0.135039 | 0.010458 | 0.010859 | 0.015187 | 0.048389 |
| 0.276691 | 0.003253 | 0.007971 | 0.002094 | 0.003016 | 0.057467 | 0.005423 | 0.006161 | 0.007871 | 0.027119 |
| 0.473604 | 0.004305 | 0.019 | 0 | 0.006365 | 0.17898 | 0.01156 | 0.007867 | 0.010323 | 0.02875 |
| 0.23431 | 0.007696 | 0.020626 | 0.002013 | 0.006967 | 0.105449 | 0.002995 | 0.014678 | 0.009778 | 0.029158 |
| 0.243017 | 0.005717 | 0.010586 | 0.00141 | 0.00418 | 0.082264 | 0.005951 | 0.004191 | 0.00992 | 0.01422 |
| 0.200551 | 0.00675 | 0.007885 | 0.001007 | 0.002074 | 0.062527 | 0.005026 | 0.005 | 0.003516 | 0.011632 |
| 0.353059 | 0.01139 | 0.011347 | 0 | 0.003623 | 0.119299 | 0.008203 | 0.005856 | 0.006158 | 0.03647 |
| 0.30252 | 0.013055 | 0.010841 | 0.000864 | 0.00242 | 0.087165 | 0.005755 | 0.004379 | 0.003888 | 0.019314 |
| 0.257639 | 0.006573 | 0.01966 | 0.001402 | 0.004362 | 0.123013 | 0.006158 | 0.012319 | 0.020317 | 0.024854 |
| 0.442419 | 0.042892 | 0.02081 | 0.00178 | 0.005764 | 0.130661 | 0.005379 | 0.008577 | 0.011347 | 0.031471 |
| 0.339491 | 0.049365 | 0.014835 | 0 | 0.005574 | 0.120092 | 0.00457 | 0.011036 | 0.009778 | 0.012772 |
| 0.45725 | 0.027874 | 0.020985 | 0.001678 | 0.002786 | 0.134132 | 0.006689 | 0.00555 | 0.005369 | 0.020653 |
| 0.514813 | 0.032895 | 0.019346 | 0.001493 | 0.006621 | 0.160369 | 0.00693 | 0.006156 | 0.009914 | 0.031906 |
| 0.423357 | 0.012297 | 0.015426 | 0 | 0.003533 | 0.094434 | 0.005371 | 0.006669 | 0.014739 | 0.051617 |
| 0.274413 | 0.01269 | 0.012638 | 0 | 0.005968 | 0.105187 | 0.005979 | 0.006411 | 0.022745 | 0.033512 |
| 0.439455 | 0.02307 | 0.04716 | 0.003357 | 0.009904 | 0.271232 | 0.020567 | 0.021376 | 0.018051 | 0.076406 |
| 0.43196 | 0.015598 | 0.02004 | 0.001404 | 0.003714 | 0.102345 | 0.005047 | 0.008792 | 0.011535 | 0.026548 |
| 0.308717 | 0.019713 | 0.051174 | 0.0028 | 0.006592 | 0.233953 | 0.010491 | 0.030001 | 0.017199 | 0.053407 |
| 0.245777 | 0.00641 | 0.0074 | 0 | 0.003457 | 0.052023 | 0.003821 | 0.003523 | 0.013891 | 0.018299 |
| 0.266537 | 0.004064 | 0.018969 | 0.001355 | 0.002497 | 0.082212 | 0.003043 | 0.008481 | 0.005618 | 0.023293 |
| 0.39256 | 0.014326 | 0.019551 | 0 | 0.008168 | 0.180902 | 0.006316 | 0.006501 | 0.011354 | 0.041415 |
| 0.43481 | 0.015672 | 0.027285 | 0.001668 | 0.007 | 0.165928 | 0.00621 | 0.012038 | 0.012558 | 0.03882 |
| 0.267225 | 0.001982 | 0.014449 | 0.001053 | 0.003747 | 0.097286 | 0.003526 | 0.00941 | 0.008739 | 0.011922 |
| 0.335619 | 0.005916 | 0.012954 | 0.001166 | 0.002651 | 0.110714 | 0.003806 | 0.005363 | 0.006334 | 0.014813 |
| 0.335115 | 0.013531 | 0.009756 | 0.001097 | 0.003095 | 0.108364 | 0.008132 | 0.002163 | 0.006532 | 0.015293 |
| 0.353446 | 0.020816 | 0.013736 | 0.001961 | 0.002727 | 0.09429 | 0.008859 | 0.003045 | 0.004631 | 0.013932 |
| 0.380187 | 0.01153 | 0.023957 | 0.001858 | 0.003197 | 0.165066 | 0.006723 | 0.008704 | 0.006095 | 0.033006 |
| 0.347505 | 0.008565 | 0.026527 | 0 | 0.006368 | 0.121058 | 0.008361 | 0.01299 | 0.010297 | 0.02815 |
| 0.400065 | 0.020474 | 0.092399 | 0.00668 | 0 | 0.215174 | 0.013144 | 0.011683 | 0.012127 | 0.029845 |
| 0.181266 | 0.005347 | 0.008978 | 0 | 0 | 0.060454 | 0.004051 | 0.003637 | 0.006418 | 0 |
| 0.359927 | 0.007272 | 0.026213 | 0.003342 | 0 | 0.095698 | 0.005934 | 0.007022 | 0.00812 | 0.037341 |
| 0.605158 | 0.029629 | 0.08601 | 0.005156 | 0.01227 | 0.427278 | 0.023323 | 0.018695 | 0.015534 | 0.080336 |
| 0.426849 | 0.021322 | 0.02249 | 0.002138 | 0.004715 | 0.151822 | 0.010916 | 0.007455 | 0.007617 | 0.029438 |
| 0.262038 | 0.039197 | 0.013365 | 0 | 0.006102 | 0.115011 | 0.005925 | 0.007101 | 0.006854 | 0.011581 |
| 0.375295 | 0.013041 | 0.022189 | 0 | 0.005502 | 0.13543 | 0.006487 | 0.008003 | 0.009198 | 0.035752 |
| 0.224682 | 0.005289 | 0.013072 | 0.00186 | 0.002114 | 0.055067 | 0.003841 | 0.004562 | 0.005482 | 0.009506 |
| 0.279023 | 0.042803 | 0.016843 | 0 | 0.003636 | 0.077035 | 0.007154 | 0.003199 | 0.007064 | 0.013481 |
| 0.490678 | 0.01482 | 0.01975 | 0.002246 | 0.003491 | 0.146861 | 0.008959 | 0.00733 | 0.004612 | 0.018607 |
| 0.489462 | 0.021462 | 0.138293 | 0.005668 | 0.014505 | 0.353065 | 0.020939 | 0.024381 | 0.020868 | 0.072127 |
| 0.189113 | 0.001452 | 0.013641 | 0.001259 | 0.003012 | 0.09682 | 0.00472 | 0.003499 | 0.004422 | 0.018589 |
| 0.374382 | 0.00996 | 0.064798 | 0 | 0.009518 | 0.316681 | 0.00736 | 0.035506 | 0.018348 | 0.17592 |
| 0.262912 | 0.013437 | 0.029745 | 0.002226 | 0.004843 | 0.14268 | 0.007675 | 0.010289 | 0.0138 | 0.03011 |
| 0.285777 | 0.011134 | 0.0537 | 0 | 0.006584 | 0.206224 | 0.008855 | 0.010445 | 0.01087 | 0.042098 |
| 0.189961 | 0.006554 | 0.01132 | 0 | 0.002148 | 0.077002 | 0.003607 | 0.004057 | 0.006451 | 0.012351 |
| 0 | 0.236455 | 0.008799 | 0.002141 | 0.004104 | 0.143013 | 0.006862 | 0.012649 | 0.013432 | 0.038241 |
| 0.41219 | 0.016178 | 0.027835 | 0 | 0.007452 | 0.191565 | 0.008756 | 0.005241 | 0 | 0.033394 |
| 0.454293 | 0.015317 | 0.038031 | 0.002327 | 0.00601 | 0.259773 | 0.010512 | 0.010285 | 0.007523 | 0.041819 |
| 0.010164 | 0.003165 | 0.012395 | 0.001274 | 0.002685 | 0.110302 | 0.005644 | 0.005763 | 0.003907 | 0.017126 |
| 0.417045 | 0.009778 | 0.013504 | 0 | 0.003577 | 0.145659 | 0.004926 | 0.004852 | 0.005026 | 0.014516 |
| 0.453939 | 0.003981 | 0.017302 | 0.002325 | 0.004201 | 0.167705 | 0.007241 | 0.007651 | 0.005913 | 0.024202 |
| 0.317987 | 0.005652 | 0.010773 | 0.002487 | 0.002553 | 0.082605 | 0.005495 | 0.004376 | 0.004751 | 0.010378 |
| 0.584204 | 0.012414 | 0.025696 | 0 | 0.005767 | 0.181203 | 0.009079 | 0.007923 | 0.009608 | 0.030972 |
| 0.489052 | 0.005529 | 0.030736 | 0.002629 | 0.004964 | 0.211619 | 0.008974 | 0.007683 | 0.008158 | 0.046439 |
| 0.568161 | 0.052495 | 0 | 0 | 0.011408 | 0.261368 | 0.014233 | 0.011581 | 0.011275 | 0.054246 |
| 0.413963 | 0.021017 | 0.022119 | 0 | 0.005525 | 0.209748 | 0.007638 | 0.007299 | 0.007072 | 0.032757 |
| 0.331299 | 0.008501 | 0.008587 | 0.002299 | 0.003561 | 0.091431 | 0.005314 | 0.002779 | 0.004257 | 0.023614 |
| 0.448927 | 0.018428 | 0.031923 | 0 | 0.010194 | 0.121956 | 0.010648 | 0.014651 | 0.009492 | 0.024948 |
| 0.291157 | 0.0142 | 0.014894 | 0 | 0.004368 | 0.116709 | 0.005586 | 0.005787 | 0.006257 | 0.018728 |
| 0.628385 | 0.012352 | 0.029828 | 0 | 0.00806 | 0.248473 | 0.010846 | 0.008496 | 0.00811 | 0.030101 |
| 0.522997 | 0.043138 | 0.040184 | 0.00289 | 0.008967 | 0.253479 | 0.014074 | 0.012266 | 0.01311 | 0.05375 |
| 0.544534 | 0.012824 | 0.034016 | 0 | 0.011046 | 0.199526 | 0.011792 | 0.012341 | 0.009926 | 0.023387 |
| 0.470212 | 0.012407 | 0.017434 | 0 | 0 | 0.118189 | 0.009776 | 0.007872 | 0.007872 | 0.021798 |
| 0.375777 | 0.020063 | 0.029216 | 0 | 0.006687 | 0.163511 | 0.009153 | 0.009876 | 0.009856 | 0.050377 |
| 0.52938 | 0.010965 | 0.020638 | 0.002913 | 0.004408 | 0.130837 | 0.007767 | 0.00575 | 0.005983 | 0.021929 |
| 2693 | 2738 | 2764 | 2779 | 2809 | 2850 | 2867 | 2880 | 2953 | 2968 |
| 0.004496 | 0.041338 | 0.004455 | 0.01069 | 0.08357 | 0.104937 | 0.010428 | 0.065709 | 0 | 0.169979 |
| 0.002018 | 0.010614 | 0.006807 | 0.01317 | 0.128949 | 0.028346 | 0.004327 | 0.024305 | 0.003712 | 0.092528 |
| 0.005186 | 0 | 0.003829 | 0.009788 | 0.090761 | 0.14012 | 0.010223 | 0.054068 | 0 | 0.199947 |
| 0.002818 | 0.007786 | 0.002969 | 0.010043 | 0.091299 | 0.122683 | 0.009524 | 0.063824 | 0.002994 | 0.114548 |
| 0.005896 | 0.023671 | 0 | 0.033572 | 0.039492 | 0.205116 | 0.01061 | 0.086 | 0.003068 | 0.112561 |
| 0.00223 | 0 | 0 | 0 | 0.025078 | 0.049937 | 0.003957 | 0.02211 | 0.002333 | 0.055984 |
| 0.002482 | 0.021221 | 0.002259 | 0.032062 | 0.034665 | 0.080606 | 0.004113 | 0.031035 | 0.002547 | 0.100032 |
| 0.00239 | 0.013317 | 0.002126 | 0.033629 | 0.056063 | 0.087268 | 0.006506 | 0.021791 | 0.00215 | 0.066672 |
| 0.004266 | 0.029261 | 0.004626 | 0.072346 | 0.063342 | 0.136887 | 0.012416 | 0.070533 | 0.008268 | 0.173234 |
| 0.008032 | 0.034019 | 0 | 0.065149 | 0.098864 | 0.181103 | 0.016804 | 0.064052 | 0.005992 | 0.137402 |
| 0.010391 | 0.031364 | 0 | 0.061976 | 0.080525 | 0.35168 | 0.029204 | 0.140491 | 0.009095 | 0.184354 |
| 0.007239 | 0.046514 | 0 | 0.064168 | 0.12101 | 0.266858 | 0.02628 | 0.097288 | 0.00622 | 0.150752 |
| 0.002709 | 0.0083 | 0.004319 | 0.042341 | 0.02667 | 0.283021 | 0.008654 | 0.038186 | 0.004631 | 0.115815 |
| 0.003931 | 0.034788 | 0.007148 | 0.080673 | 0.11395 | 0.193808 | 0.015776 | 0.045619 | 0.006744 | 0.153646 |
| 0.002995 | 0.05627 | 0.007744 | 0.082926 | 0.064167 | 0.081148 | 0.006718 | 0.026463 | 0.005203 | 0.116954 |
| 0.004234 | 0.023237 | 0.011442 | 0.085556 | 0.058201 | 0.28718 | 0.014137 | 0.039854 | 0.006997 | 0.173777 |
| 0.004492 | 0.062587 | 0.013651 | 0.10745 | 0.095238 | 0.004309 | 0.00935 | 0.041936 | 0.007913 | 0.172593 |
| 0.002036 | 0.009114 | 0 | 0.044995 | 0.031977 | 0.062787 | 0.004275 | 0.033682 | 0.002693 | 0.07218 |
| 0.002866 | 0.013282 | 0.0034 | 0.048565 | 0.032095 | 0.081144 | 0.005364 | 0.062142 | 0.00508 | 0.152108 |
| 0.002819 | 0.036055 | 0.008635 | 0.075631 | 0.051987 | 0.12071 | 0.007061 | 0.034322 | 0.007815 | 0.227186 |
| 0.001919 | 0.018082 | 0.008334 | 0.065774 | 0.0371 | 0.042618 | 0.0037 | 0.026989 | 0.003865 | 0.135068 |
| 0.001159 | 0.008954 | 0.004185 | 0.04737 | 0.045518 | 0.047017 | 0.002758 | 0.016084 | 0.001799 | 0.071203 |
| 0.002948 | 0.02774 | 0.003316 | 0.044583 | 0.065932 | 0.083457 | 0.006026 | 0.039179 | 0.003397 | 0.130148 |
| 0.001724 | 0.015045 | 0.003794 | 0.050376 | 0.081783 | 0.064098 | 0.004823 | 0.02508 | 0.002121 | 0.082523 |
| 0.001678 | 0.018846 | 0.00455 | 0.063591 | 0.04171 | 0.136012 | 0.005309 | 0.025302 | 0.003156 | 0.104308 |
| 0.00376 | 0.073304 | 0.005056 | 0.067783 | 0.119596 | 0.107828 | 0.011025 | 0.031124 | 0.002878 | 0.078337 |
| 0.002689 | 0.042768 | 0.004934 | 0.068817 | 0.179278 | 0.068314 | 0.010137 | 0.016185 | 0.005339 | 0.118875 |
| 0.002266 | 0.019192 | 0.002186 | 0.035241 | 0.079372 | 0.115484 | 0.008608 | 0.041671 | 0.001672 | 0.078773 |
| 0.002819 | 0.049105 | 0.00492 | 0.078972 | 0.092613 | 0.07468 | 0.007188 | 0.043425 | 0.006865 | 0.133799 |
| 0.002434 | 0.027836 | 0.005476 | 0.070543 | 0.051363 | 0.083001 | 0.004495 | 0.028144 | 0.004321 | 0.121899 |
| 0.003227 | 0.04723 | 0.005955 | 0.079064 | 0.082327 | 0.065219 | 0.005307 | 0.019977 | 0.008256 | 0.214428 |
| 0.008366 | 0.03664 | 0.005324 | 0.068537 | 0.119176 | 0.232359 | 0.021111 | 0.066474 | 0.005862 | 0.166091 |
| 0.002511 | 0.019702 | 0.002676 | 0.047005 | 0.056828 | 0.091708 | 0.006521 | 0.040017 | 0.002798 | 0.09809 |
| 0.005226 | 0.021436 | 0.002215 | 0.04467 | 0.080807 | 0.434994 | 0.030173 | 0.041559 | 0.002486 | 0.091685 |
| 0.001906 | 0.032597 | 0.006612 | 0.061217 | 0.036107 | 0.042522 | 0.004192 | 0.03115 | 0.004647 | 0.104743 |
| 0.001113 | 0.011034 | 0.003319 | 0.056879 | 0.025506 | 0.114162 | 0.003875 | 0.026746 | 0.002329 | 0.07417 |
| 0.00267 | 0.046055 | 0.005986 | 0.100299 | 0.064084 | 0.097652 | 0.002474 | 0.018069 | 0.006967 | 0.176416 |
| 0.002921 | 0.040172 | 0.004461 | 0.086285 | 0.058495 | 0.117782 | 0.005349 | 0.029544 | 0.006141 | 0.138358 |
| 0.001156 | 0.020585 | 0.003295 | 0.070015 | 0.023435 | 0.088283 | 0.002534 | 0.014036 | 0.002792 | 0.100904 |
| 0.001304 | 0.011376 | 0.001643 | 0.031807 | 0.026193 | 0.085419 | 0.003095 | 0.023155 | 0.001617 | 0.067721 |
| 0.002019 | 0.030074 | 0.00342 | 0.052021 | 0.077066 | 0.058089 | 0.005602 | 0.027702 | 0.002324 | 0.097871 |
| 0.001929 | 0.014747 | 0.003197 | 0.053142 | 0.08978 | 0.07085 | 0.008387 | 0.048375 | 0.002111 | 0.094097 |
| 0.002562 | 0.019318 | 0.001466 | 0.029116 | 0.040795 | 0.107452 | 0.004329 | 0.033342 | 0.001498 | 0.087805 |
| 0.003585 | 0.0311 | 0.003973 | 0.050135 | 0.032021 | 0.110328 | 0.006468 | 0.035614 | 0.006275 | 0.174328 |
| 0.004174 | 0.020103 | 0 | 0.043407 | 0.057648 | 0.206985 | 0.013344 | 0.052608 | 0.0025 | 0.081199 |
| 0.012264 | 0.023012 | 0 | 0.030828 | 0.036515 | 0.040615 | 0 | 0.015405 | 0 | 0.050941 |
| 0.002413 | 0.025086 | 0.002573 | 0.037306 | 0.026316 | 0.109246 | 0.005007 | 0.04682 | 0.002229 | 0.078998 |
| 0.007468 | 0.028738 | 0 | 0.049594 | 0.060147 | 0.25551 | 0.013962 | 0.002558 | 0.005279 | 0.193573 |
| 0.002756 | 0.016521 | 0.00331 | 0.058968 | 0.077182 | 0.085456 | 0.007516 | 0.040671 | 0.00374 | 0.146393 |
| 0 | 0.029743 | 0 | 0.025225 | 0.170672 | 0.065936 | 0.012059 | 0.012279 | 0 | 0.055285 |
| 0.003379 | 0.036729 | 0.003463 | 0.046017 | 0.044594 | 0.108961 | 0.006156 | 0.026146 | 0.004206 | 0.121256 |
| 0 | 0.024113 | 0.001868 | 0.034536 | 0.030008 | 0.063197 | 0.003306 | 0.019101 | 0.001604 | 0.046644 |
| 0.003421 | 0.047569 | 0 | 0.049687 | 0.181526 | 0.046244 | 0.007361 | 0.025063 | 0.003694 | 0.120024 |
| 0.002408 | 0.016733 | 0.001979 | 0.037212 | 0.039006 | 0.09556 | 0.005393 | 0.047966 | 0.001576 | 0.066136 |
| 0.00801 | 0.029508 | 0 | 0.052339 | 0.04822 | 0.339031 | 0.015436 | 0.042957 | 0.005125 | 0.147017 |
| 0.001642 | 0.017647 | 0 | 0.038113 | 0.033945 | 0.048094 | 0.001846 | 0.009245 | 0.002829 | 0.089401 |
| 0.007132 | 0.025946 | 0 | 0.044649 | 0.029149 | 0.269941 | 0.007747 | 0.035355 | 0.007458 | 0.280681 |
| 0.003249 | 0.05415 | 0.003582 | 0.062203 | 0.077246 | 0.136573 | 0.008873 | 0.040465 | 0 | 0.140969 |
| 0.005348 | 0.043298 | 0 | 0.038544 | 0.036299 | 0.130844 | 0.007169 | 0.040286 | 0.003489 | 0.082054 |
| 0.001812 | 0.03233 | 0 | 0.027069 | 0.042555 | 0.07796 | 0.003815 | 0.017722 | 0.001867 | 0.069096 |
| 0.00322 | 0.045541 | 0.003402 | 0.046244 | 0.058765 | 0.175001 | 0.007743 | 0.02944 | 0.003075 | 0.120499 |
| 0.002582 | 0.03311 | 0 | 0.04996 | 0.05028 | 0 | 0.004279 | 0.027547 | 0.003016 | 0.099781 |
| 0.003619 | 0.026365 | 0.002098 | 0.042029 | 0.048037 | 0.183773 | 0.007205 | 0.045538 | 0.003234 | 0.003306 |
| 0.001685 | 0.005916 | 0 | 0.028431 | 0.019525 | 0.073315 | 0.002396 | 0.020057 | 0.002188 | 0.094461 |
| 0 | 0.005596 | 0.002902 | 0.030541 | 0.027247 | 0.063091 | 0.004579 | 0.027916 | 0.002317 | 0.076466 |
| 0.002044 | 0.010251 | 0.002329 | 0.024949 | 0.016164 | 0.103783 | 0.003605 | 0.024315 | 0.002622 | 0.086951 |
| 0 | 0.010555 | 0.002831 | 0.034126 | 0.019499 | 0.043085 | 0.003373 | 0.019357 | 0.001757 | 0.043273 |
| 0.003679 | 0.007121 | 0.002671 | 0.004312 | 0.023739 | 0.127303 | 0.00572 | 0.036389 | 0.002486 | 0.075382 |
| 0.002309 | 0.006108 | 0.002352 | 0.027711 | 0.019587 | 0.144336 | 0.004562 | 0.03557 | 0.002253 | 0.071616 |
| 0.005328 | 0.019156 | 0 | 0.03509 | 0.0349 | 0.165002 | 0.009607 | 0.044739 | 0.003948 | 0.07707 |
| 0.003721 | 0.018103 | 0.002683 | 0.035622 | 0.062627 | 0.111122 | 0.007487 | 0.021756 | 0.00283 | 0.076591 |
| 0.002321 | 0.011043 | 0.00196 | 0.034433 | 0.030069 | 0.04723 | 0.005788 | 0.027809 | 0.00317 | 0.128389 |
| 0 | 0.015869 | 0 | 0 | 0.038093 | 0.122658 | 0.010524 | 0.043128 | 0 | 0.096946 |
| 0.002094 | 0.052487 | 0.002803 | 0.048221 | 0.055428 | 0.061222 | 0.004168 | 0.017825 | 0.003398 | 0.089114 |
| 0 | 0.006331 | 0 | 0.029517 | 0.008433 | 0.109248 | 0.005038 | 0.027651 | 0.004453 | 0.074843 |
| 0.006547 | 0.032696 | 0.004157 | 0.054642 | 0.101858 | 0.171896 | 0.016996 | 0.04194 | 0.004175 | 0.125965 |
| 0 | 0 | 0 | 0.026417 | 0.018863 | 0.107602 | 0.008542 | 0.044337 | 0.007356 | 0.123787 |
| 0 | 0.008493 | 0 | 0.024002 | 0.020322 | 0.069823 | 0.006738 | 0.030719 | 0 | 0.049736 |
| 0.004556 | 0.056201 | 0.011158 | 0.051904 | 0.069125 | 0.131356 | 0.009804 | 0.040109 | 0.004528 | 0.132134 |
| 0.002259 | 0.009988 | 0.002824 | 0.030329 | 0.022859 | 0.072846 | 0.00355 | 0.032083 | 0.002807 | 0.077997 |
| 3054 | 3213 | 3228 | 3241 | 3329 | 3415 | 3503 | 3572 | 3590 | 3603 |
| 0.013991 | 0.047379 | 0.01006 | 0.080063 | 0.011436 | 0.012171 | 0.002385 | 0 | 0.001366 | 0.206921 |
| 0.005111 | 0.008571 | 0.004594 | 0.03062 | 0.001792 | 0.009121 | 0.000587 | 0.000905 | 0.006404 | 0.100823 |
| 0.019488 | 0.068671 | 0.008781 | 0.046549 | 0.004141 | 0.031208 | 0.002671 | 0.001377 | 0.009478 | 0.124067 |
| 0.010224 | 0.029712 | 0.00751 | 0.062675 | 0.006922 | 0.008446 | 0.000973 | 0.000812 | 0.00727 | 0.155274 |
| 0.01722 | 0.035636 | 0.004867 | 0.050105 | 0.007969 | 0.013034 | 0.001907 | 0 | 0.003523 | 0.082067 |
| 0.005026 | 0.007803 | 0.003655 | 0.015721 | 0.00183 | 0.003909 | 0.000367 | 0.000345 | 0.000693 | 0.032787 |
| 0.00791 | 0.040591 | 0.003933 | 0.044434 | 0.001892 | 0.013274 | 0.000939 | 0.001023 | 0.005323 | 0.130911 |
| 0.004452 | 0.017515 | 0.003666 | 0.025819 | 0.001919 | 0.00502 | 0 | 0.000988 | 0.003835 | 0.091066 |
| 0.01922 | 0.049486 | 0.010533 | 0.069931 | 0.011659 | 0.032364 | 0.003511 | 0.00256 | 0.011688 | 0.148171 |
| 0.019798 | 0.037141 | 0.007228 | 0.037558 | 0.006094 | 0.006701 | 0.001361 | 0.001269 | 0.005277 | 0.107777 |
| 0.031844 | 0.044311 | 0.007588 | 0.050903 | 0.015587 | 0.009789 | 0.002591 | 0.001828 | 0.005269 | 0.102107 |
| 0.023108 | 0.045732 | 0.008195 | 0.048515 | 0.007882 | 0.011323 | 0.002184 | 0.002125 | 0.006238 | 0.118623 |
| 0.012081 | 0.058551 | 0.0048 | 0.02583 | 0.002751 | 0.01339 | 0.001399 | 0 | 0.003569 | 0.039775 |
| 0.010178 | 0.045574 | 0.009527 | 0.052341 | 0.003812 | 0.013945 | 0.001504 | 0.00359 | 0.011839 | 0.177322 |
| 0.007295 | 0.059066 | 0.011192 | 0.050064 | 0.002895 | 0.00811 | 0.000922 | 0.004604 | 0.012993 | 0.180714 |
| 0.010188 | 0.065243 | 0.010805 | 0.043461 | 0.001798 | 0.00653 | 0 | 0.00161 | 0.008015 | 0.13221 |
| 0.012395 | 0.086331 | 0.014993 | 0.065696 | 0.003429 | 0.018155 | 0.001244 | 0.004241 | 0.013768 | 0.171041 |
| 0.005972 | 0.029442 | 0.004674 | 0.04918 | 0.004771 | 0.010197 | 0.001043 | 0.003255 | 0.008171 | 0.143148 |
| 0.012644 | 0.027628 | 0.004924 | 0.048665 | 0.011004 | 0.015982 | 0.002208 | 0.001654 | 0.006085 | 0.110727 |
| 0.009861 | 0.088765 | 0.010059 | 0.001776 | 0.003049 | 0.014257 | 0.000915 | 0.005168 | 0.010744 | 0.155479 |
| 0.005456 | 0.021546 | 0.006973 | 0.051164 | 0.003442 | 0.008501 | 0.000713 | 0.003804 | 0.008439 | 0.175329 |
| 0.003619 | 0.019359 | 0.002354 | 0.021798 | 0.00088 | 0.003555 | 0.00045 | 0.001462 | 0.004908 | 0.118701 |
| 0.008416 | 0.039313 | 0.004748 | 0.037137 | 0.003149 | 0.008262 | 0.000878 | 0.001103 | 0.003876 | 0.091328 |
| 0.004233 | 0.02532 | 0.004496 | 0.041582 | 0.001326 | 0.007168 | 0.000624 | 0.002077 | 0.00704 | 0.166435 |
| 0.006082 | 0.052092 | 0.00634 | 0.051529 | 0.00145 | 0.006854 | 0 | 0.001487 | 0.00944 | 0.172025 |
| 0.006094 | 0.028988 | 0.006697 | 0.033174 | 0.002279 | 0.004998 | 0.00074 | 0.000749 | 0.005378 | 0.084854 |
| 0.005229 | 0.02915 | 0.00755 | 0.022305 | 0.001197 | 0.008356 | 0.000703 | 0.000912 | 0.00667 | 0.079394 |
| 0.00614 | 0.023292 | 0.003334 | 0.033774 | 0.00248 | 0.0075 | 0.000845 | 0.000423 | 0.00256 | 0.073258 |
| 0.010215 | 0.034991 | 0.008217 | 0.04858 | 0.003242 | 0.02459 | 0.001979 | 0.000984 | 0.011052 | 0.108491 |
| 0.006247 | 0.064848 | 0.007714 | 0.058937 | 0.001815 | 0.010951 | 0.00123 | 0.002296 | 0.012177 | 0.193362 |
| 0.006713 | 0.06604 | 0.008566 | 0.041702 | 0.001558 | 0.016395 | 0.000928 | 0.00156 | 0.009647 | 0.137156 |
| 0.021043 | 0.083833 | 0.011206 | 0.055942 | 0.008199 | 0.010761 | 0.001436 | 0.002879 | 0.009227 | 0.167947 |
| 0.007157 | 0.036495 | 0.005589 | 0.056198 | 0.003565 | 0.011863 | 0.00084 | 0.000811 | 0.005467 | 0.115974 |
| 0.011392 | 0.05391 | 0.007052 | 0.036802 | 0.00215 | 0.005163 | 0.00072 | 0.001048 | 0.006021 | 0.139898 |
| 0.006064 | 0.026181 | 0.007714 | 0.065276 | 0.003636 | 0.019239 | 0.001291 | 0.002271 | 0.010598 | 0.15377 |
| 0.004893 | 0.035318 | 0.005257 | 0.043188 | 0.002497 | 0.004795 | 0.000553 | 0.001141 | 0.00902 | 0.162368 |
| 0.009094 | 0.059172 | 0.007131 | 0.036068 | 0.001361 | 0.016392 | 0.000671 | 0.000782 | 0.011932 | 0.121169 |
| 0.009504 | 0.051254 | 0.007429 | 0.043824 | 0.002525 | 0.014459 | 0.001063 | 0.00105 | 0.010135 | 0.134782 |
| 0.004791 | 0.023398 | 0.003489 | 0.025867 | 0.000875 | 0.0113 | 0.000592 | 0.000958 | 0.007108 | 0.099077 |
| 0.004556 | 0.01976 | 0.002466 | 0.032148 | 0.001439 | 0.004735 | 0.000532 | 0.00059 | 0.0035 | 0.10275 |
| 0.005257 | 0.026343 | 0.004525 | 0.044688 | 0.001463 | 0.008509 | 0.00078 | 0.001679 | 0.005748 | 0.135242 |
| 0.005939 | 0.014361 | 0.004947 | 0.05825 | 0.004618 | 0.007771 | 0.000775 | 0.001218 | 0.006162 | 0.142991 |
| 0.009929 | 0.034809 | 0.003244 | 0.037458 | 0.00285 | 0.007986 | 0.001103 | 0.000514 | 0.00246 | 0.094346 |
| 0.01193 | 0.056383 | 0.005577 | 0.043976 | 0.003707 | 0.013331 | 0.0016 | 0.001802 | 0.007291 | 0.127081 |
| 0.011356 | 0.02219 | 0.004456 | 0.023969 | 0.003667 | 0.00422 | 0.000895 | 0.00092 | 0.002668 | 0.063489 |
| 0.003648 | 0.023673 | 0.003798 | 0.030483 | 0.002302 | 0.004081 | 0 | 0.00282 | 0.005651 | 0.133065 |
| 0.008401 | 0.04776 | 0.006097 | 0.069426 | 0.004616 | 0.006778 | 0.001367 | 0.001794 | 0.008544 | 0.23991 |
| 0.033068 | 0.069821 | 0.007879 | 0.036332 | 0.008314 | 0.013395 | 0.002304 | 0.001695 | 0.004078 | 0.098245 |
| 0.007977 | 0.042819 | 0.007716 | 0.066072 | 0.003562 | 0.007843 | 0.001117 | 0.001166 | 0.010527 | 0.192166 |
| 0.004872 | 0.01424 | 0.00585 | 0.01265 | 0 | 0.005119 | 0 | 0 | 0.003169 | 0.042119 |
| 0.007666 | 0.041803 | 0.00522 | 0.031775 | 0.002171 | 0.005989 | 0.001208 | 0.001824 | 0.005975 | 0.120161 |
| 0.003962 | 0.01374 | 0.00262 | 0.025892 | 0.001365 | 0.004991 | 0.000807 | 0.001483 | 0.003864 | 0.100549 |
| 0.005209 | 0.013842 | 0.004387 | 0.027427 | 0.002262 | 0.005704 | 0 | 0.002194 | 0.004784 | 0.104267 |
| 0.009788 | 0.022376 | 0.003275 | 0.037995 | 0.003816 | 0.005039 | 0.000916 | 0 | 0.002893 | 0.086701 |
| 0.024692 | 0.079721 | 0.00731 | 0.023978 | 0.001663 | 0.010177 | 0.001434 | 0.001664 | 0.004141 | 0.080809 |
| 0.00375 | 0.025909 | 0.002535 | 0.01809 | 0.000824 | 0.009254 | 0 | 0.00182 | 0.003003 | 0.065915 |
| 0.029114 | 0.187618 | 0.008689 | 0.024025 | 0.003324 | 0.021909 | 0.002497 | 0.002266 | 0.00551 | 0.069346 |
| 0.010474 | 0.052662 | 0.008546 | 0.049084 | 0.003715 | 0.009057 | 0.001141 | 0 | 0.012964 | 0.200804 |
| 0.013219 | 0.035445 | 0.004846 | 0.02018 | 0.003515 | 0.005621 | 0.002033 | 0 | 0.005015 | 0.155293 |
| 0.004261 | 0.020514 | 0.003009 | 0.022837 | 0.001513 | 0.005164 | 0.001009 | 0.001879 | 0.004349 | 0.132627 |
| 0.010198 | 0.049669 | 0.004321 | 0.027784 | 0.001836 | 0.010991 | 0.000878 | 0.001779 | 0.004802 | 0.10246 |
| 0.008816 | 0.029055 | 0.003417 | 0.022033 | 0.001506 | 0.009545 | 0.001113 | 0.000784 | 0.003631 | 0.061673 |
| 0.012479 | 0.043074 | 0.003509 | 0.02704 | 0.002303 | 0.007675 | 0.001024 | 0.000752 | 0.003099 | 0.08566 |
| 0.005648 | 0.021201 | 0.001847 | 0.019051 | 0.001059 | 0.007522 | 0.000644 | 0.001025 | 0.002372 | 0.067195 |
| 0.005249 | 0.014452 | 0.002719 | 0.018833 | 0.002074 | 0.00575 | 0 | 0.001111 | 0.002724 | 0.050574 |
| 0.006956 | 0.017544 | 0.002048 | 0.012131 | 0.001488 | 0.007034 | 0.00113 | 0 | 0.001919 | 0.03363 |
| 0.003267 | 0.013199 | 0.002208 | 0.021089 | 0.001408 | 0.001621 | 0 | 0 | 0.002963 | 0.081223 |
| 0.008829 | 0.015877 | 0.002816 | 0.01521 | 0.002213 | 0.007809 | 0.001246 | 0 | 0.001839 | 0.02662 |
| 0.006923 | 0.021439 | 0.002486 | 0.019626 | 0.002128 | 0.00448 | 0 | 0 | 0.002011 | 0.041976 |
| 0.010796 | 0.02659 | 0.004601 | 0.021048 | 0.004023 | 0.007054 | 0 | 0 | 0.003403 | 0.050149 |
| 0.007374 | 0.0257 | 0.003483 | 0.014948 | 0.001506 | 0.007159 | 0.001193 | 0 | 0.002774 | 0.045936 |
| 0.006467 | 0.041523 | 0.004274 | 0.001421 | 0.00212 | 0.01018 | 0.000961 | 0.001166 | 0.004837 | 0.134407 |
| 0.012175 | 0.038011 | 0.007346 | 0.026228 | 0.007202 | 0.012485 | 0 | 0 | 0.005489 | 0.047648 |
| 0.004569 | 0.02063 | 0.003388 | 0.022593 | 0.001398 | 0.010189 | 0.001006 | 0.001223 | 0.004333 | 0.068818 |
| 0.009205 | 0.021432 | 0.004378 | 0.015523 | 0 | 0.005523 | 0 | 0 | 0 | 0.028372 |
| 0.012115 | 0.041926 | 0.006395 | 0.029856 | 0.002583 | 0.00696 | 0.001013 | 0 | 0.00314 | 0.063496 |
| 0.016733 | 0.026286 | 0.006149 | 0.022553 | 0.007335 | 0.016404 | 0 | 0 | 0.005556 | 0.024639 |
| 0.009412 | 0.018354 | 0 | 0.023018 | 0 | 0.008386 | 0 | 0 | 0 | 0.050164 |
| 0.010518 | 0.066112 | 0.007636 | 0.040088 | 0.00312 | 0.00999 | 0.001463 | 0.001127 | 0.006382 | 0.109008 |
| 0.007062 | 0.018859 | 0.002782 | 0.02345 | 0.002391 | 0.007907 | 0 | 0 | 0.002765 | 0.047095 |
| 3691 | 3777 | 3865 | 3953 | 4039 | 4052 | 4226 | 4400 | 4413 | 4587 |
| 0.021373 | 0.045538 | 0.00242 | 0.001624 | 0 | 0.015013 | 0.001884 | 0.000726 | 0.00985 | 0.001522 |
| 0.005899 | 0.061839 | 0.001644 | 0.001633 | 0.000661 | 0.007333 | 0.002794 | 0.000734 | 0.005104 | 0.002921 |
| 0.007337 | 0.134001 | 0.005902 | 0.008265 | 0.001307 | 0.005223 | 0.005305 | 0.001919 | 0.003313 | 0.00453 |
| 0.012485 | 0.034628 | 0.001155 | 0.000633 | 0.000415 | 0.007131 | 0.000881 | 0.000231 | 0.003506 | 0.000673 |
| 0.009506 | 0.034793 | 0.002163 | 0.001547 | 0.00069 | 0.004618 | 0.001419 | 0.000572 | 0.002846 | 0.00116 |
| 0.002883 | 0.010978 | 0.000681 | 0.000342 | 0 | 0.001509 | 0.000539 | 0.000259 | 0.000798 | 0.000345 |
| 0.004871 | 0.059325 | 0.001241 | 0.001369 | 0.000528 | 0.005154 | 0.001607 | 0.000569 | 0.005286 | 0 |
| 0.005085 | 0.026005 | 0.001093 | 0.000772 | 0.00063 | 0.004133 | 0.001073 | 0.000545 | 0.002725 | 0.001012 |
| 0.02579 | 0.116701 | 0.010674 | 0.005241 | 0.001703 | 0.01234 | 0.007387 | 0.001421 | 0.003818 | 0.003753 |
| 0.01007 | 0.026122 | 0.001086 | 0.000632 | 0.000434 | 0.005646 | 0.000768 | 0.000288 | 0.002815 | 0.000666 |
| 0.010955 | 0.025861 | 0.001389 | 0.000907 | 0.000403 | 0.003348 | 0.000564 | 0.000247 | 0.001372 | 0.000391 |
| 0.01214 | 0.051125 | 0.002388 | 0.001776 | 0.000596 | 0.007014 | 0.001788 | 0.000505 | 0.004392 | 0.00168 |
| 0.004261 | 0.043165 | 0.002603 | 0.002392 | 0.000861 | 0.002233 | 0.001922 | 0.00085 | 0.001109 | 0.001336 |
| 0.015304 | 0.081194 | 0.003354 | 0.002802 | 0.001146 | 0.012462 | 0.00395 | 0.001078 | 0.008575 | 0.004479 |
| 0.012131 | 0.032992 | 0.001271 | 0.000681 | 0.001296 | 0.013529 | 0.001566 | 0.000921 | 0.013704 | 0.002103 |
| 0.008225 | 0.030923 | 0.000837 | 0.000632 | 0.000629 | 0.006939 | 0.000805 | 0.000337 | 0.004123 | 0.000724 |
| 0.015196 | 0.080274 | 0.002885 | 0.002091 | 0.001446 | 0.015738 | 0.004301 | 0.001262 | 0.01233 | 0.004969 |
| 0.013956 | 0.054269 | 0.002464 | 0.001587 | 0.00082 | 0.009831 | 0.002459 | 0.000754 | 0.006069 | 0.002354 |
| 0.01724 | 0.064239 | 0.004557 | 0.002635 | 0.00097 | 0.008292 | 0.003354 | 0.000921 | 0.004624 | 0.003131 |
| 0.014271 | 0.070964 | 0.003153 | 0.003559 | 0.001093 | 0.013085 | 0.003571 | 0.000967 | 0.00949 | 0.003369 |
| 0.012628 | 0.050332 | 0.001384 | 0.001161 | 0.000633 | 0.010252 | 0.001572 | 0.00041 | 0.005765 | 0.001391 |
| 0.004419 | 0.030158 | 0.000703 | 0.000535 | 0.000469 | 0.006593 | 0.001097 | 0.000435 | 0.007972 | 0.001753 |
| 0.008457 | 0.033041 | 0.001442 | 0.001004 | 0.000578 | 0.006278 | 0.001432 | 0.000536 | 0.005271 | 0.001763 |
| 0.006994 | 0.055335 | 0.001087 | 0.000953 | 0.000572 | 0.010322 | 0.001896 | 0.000606 | 0.012428 | 0.003724 |
| 0.008776 | 0.031964 | 0.000754 | 0.000512 | 0.000822 | 0.012998 | 0.001191 | 0.000715 | 0.014153 | 0.001876 |
| 0.005599 | 0.019996 | 0.000785 | 0 | 0.000497 | 0.003582 | 0.000637 | 0.000348 | 0.002274 | 0.000584 |
| 0.004194 | 0.054632 | 0.001345 | 0.00183 | 0.000709 | 0.004659 | 0.001955 | 0.000734 | 0.004836 | 0.002651 |
| 0.007393 | 0.030404 | 0.001434 | 0.000685 | 0.00045 | 0.006312 | 0.001676 | 0.000451 | 0.005024 | 0.002009 |
| 0.009749 | 0.097341 | 0.004605 | 0.004201 | 0.001095 | 0.007003 | 0.004919 | 0.001386 | 0.005168 | 0.005251 |
| 0.009215 | 0.051664 | 0.001126 | 0.00094 | 0.000866 | 0.012836 | 0.001892 | 0.000856 | 0.015827 | 0.003482 |
| 0.008585 | 0.080254 | 0.002454 | 0.002298 | 0.000771 | 0.008899 | 0.003497 | 0.000869 | 0.006706 | 0.003738 |
| 0.01773 | 0.038303 | 0.001816 | 0.00077 | 0.000856 | 0.013877 | 0.001757 | 0.000672 | 0.010583 | 0.002021 |
| 0.011124 | 0.040813 | 0.001633 | 0.000977 | 0.000499 | 0.007301 | 0.001602 | 0.000429 | 0.004954 | 0.00173 |
| 0 | 0.024162 | 0.000886 | 0.00058 | 0.000726 | 0.010122 | 0.001012 | 0.00059 | 0.011337 | 0.001503 |
| 0.01092 | 0.081772 | 0.00251 | 0.003369 | 0.000789 | 0.007025 | 0.002431 | 0.000629 | 0.003323 | 0.001761 |
| 0.011808 | 0.024007 | 0.000798 | 0.000368 | 0.000783 | 0.012238 | 0.000901 | 0.000517 | 0.01033 | 0.001061 |
| 0.006466 | 0.08433 | 0.001701 | 0.002297 | 0.000752 | 0.006988 | 0.002615 | 0.000804 | 0.006113 | 0.003227 |
| 0.010308 | 0.068454 | 0.002604 | 0.001474 | 0.000897 | 0.009514 | 0.003289 | 0.00086 | 0.007232 | 0.003386 |
| 0.003652 | 0.082066 | 0.00153 | 0.00288 | 0.000637 | 0.005142 | 0.002674 | 0.00095 | 0.006188 | 0.004392 |
| 0.004685 | 0.021722 | 0.000652 | 0.000515 | 0.000382 | 0.004335 | 0.000682 | 0.000347 | 0.00455 | 0.000948 |
| 0.00597 | 0.045276 | 0.000893 | 0.00082 | 0 | 0.005762 | 0.001124 | 0.000355 | 0.005568 | 0.001584 |
| 0.012786 | 0.033542 | 0.001186 | 0.000653 | 0.000467 | 0.008148 | 0.000979 | 0.000312 | 0.004549 | 0.000774 |
| 0.007452 | 0.029115 | 0.001237 | 0.000609 | 0.000363 | 0.005314 | 0.001088 | 0.000309 | 0.003636 | 0.001149 |
| 0.008399 | 0.055403 | 0.002321 | 0.002795 | 0.000875 | 0.007206 | 0.00229 | 0.000837 | 0.005534 | 0.002262 |
| 0.005415 | 0.019488 | 0.000945 | 0.000598 | 0.000454 | 0.004098 | 0.000876 | 0.000385 | 0.003627 | 0.001173 |
| 0.006942 | 0.018079 | 0.001365 | 0 | 0.001277 | 0.00809 | 0.001406 | 0.001115 | 0.008581 | 0.001655 |
| 0.010334 | 0.028463 | 0.00082 | 0.000596 | 0.000592 | 0.00876 | 0.000702 | 0.000458 | 0.007592 | 0.000751 |
| 0.006243 | 0.05304 | 0.001628 | 0.001456 | 0.000457 | 0.004348 | 0.001541 | 0.000447 | 0.003139 | 0.001786 |
| 0.012767 | 0.029541 | 0.000746 | 0.000391 | 0.000627 | 0.012083 | 0.000764 | 0.000429 | 0.01116 | 0.000923 |
| 0.002401 | 0.021315 | 0 | 0 | 0 | 0.00209 | 0.001386 | 0 | 0.001773 | 0.001338 |
| 0.004304 | 0.025057 | 0.001039 | 0.001047 | 0.000822 | 0.004767 | 0.001001 | 0.00071 | 0.006261 | 0.001268 |
| 0.004275 | 0.03274 | 0.000994 | 0.00077 | 0.000611 | 0.005433 | 0.001285 | 0.000637 | 0.007002 | 0.002233 |
| 0.005172 | 0.036234 | 0.000986 | 0.00143 | 0.000593 | 0.00544 | 0.001167 | 0.000551 | 0.005611 | 0.001653 |
| 0.006617 | 0.016568 | 0.000759 | 0.000413 | 0.000391 | 0.004873 | 0.000648 | 0.000324 | 0.00438 | 0.000753 |
| 0.002446 | 0.047787 | 0.001083 | 0.001536 | 0 | 0.002926 | 0.00126 | 0.000532 | 0.004506 | 0.00229 |
| 0.002651 | 0.059822 | 0.001353 | 0.001336 | 0.000493 | 0.002885 | 0.002016 | 0.000617 | 0.001863 | 0.002113 |
| 0.00512 | 0.092027 | 0.004439 | 0.006795 | 0.001424 | 0.003245 | 0.003865 | 0.001728 | 0.002188 | 0.003703 |
| 0.013015 | 0.061546 | 0.001702 | 0.00148 | 0.000987 | 0.015401 | 0.002403 | 0.00109 | 0.017903 | 0.004501 |
| 0.005665 | 0.042696 | 0.001799 | 0.001439 | 0 | 0.007676 | 0.001929 | 0.001175 | 0.010987 | 0.003125 |
| 0.006247 | 0.056458 | 0.001419 | 0.001343 | 0.000771 | 0.00809 | 0.002172 | 0.000851 | 0.009376 | 0.003725 |
| 0.005626 | 0.081042 | 0.002112 | 0.00257 | 0.000569 | 0.005573 | 0.002974 | 0.000747 | 0.004886 | 0.004046 |
| 0.003309 | 0.050159 | 0.001532 | 0.001466 | 0.000567 | 0.003654 | 0.002004 | 0.000693 | 0.004967 | 0.003702 |
| 0.004584 | 0.039623 | 0.001181 | 0.001398 | 0.000414 | 0.004425 | 0.001159 | 0.000415 | 0.005107 | 0.001682 |
| 0.00278 | 0.060145 | 0.001251 | 0.001821 | 0 | 0.00235 | 0.001375 | 0.000428 | 0.001792 | 0.001601 |
| 0.003756 | 0.028656 | 0.001389 | 0.001191 | 0 | 0.002302 | 0.001156 | 0 | 0.00129 | 0.000933 |
| 0.00235 | 0.038003 | 0.001872 | 0.001699 | 0 | 0.00185 | 0.001544 | 0.000772 | 0.00144 | 0.001522 |
| 0.003525 | 0.002634 | 0 | 0 | 0 | 0.003576 | 0 | 0 | 0.003328 | 0 |
| 0.002617 | 0.029998 | 0.001566 | 0.001263 | 0 | 0.001449 | 0.001186 | 0.000633 | 0.000805 | 0.000808 |
| 0.004221 | 0.014222 | 0.001265 | 0 | 0 | 0.002646 | 0.001027 | 0 | 0.001765 | 0.00098 |
| 0.004287 | 0.021419 | 0.001842 | 0 | 0 | 0.002891 | 0.001578 | 0 | 0.001892 | 0.001413 |
| 0.002272 | 0.036006 | 0.001423 | 0.001204 | 0 | 0.002159 | 0.00143 | 0.000785 | 0.002185 | 0.001962 |
| 0.008958 | 0.062043 | 0.002127 | 0.001719 | 0.000839 | 0.008729 | 0.002476 | 0.000752 | 0.006681 | 0.002946 |
| 0.008461 | 0.039847 | 0.005056 | 0 | 0 | 0.005799 | 0.004499 | 0 | 0.004024 | 0 |
| 0.003656 | 0.055814 | 0.001531 | 0.001676 | 0 | 0 | 0 | 0 | 0 | 0 |
| 0.003819 | 0.00903 | 0 | 0 | 0 | 0.002848 | 0 | 0 | 0 | 0 |
| 0.004285 | 0.016639 | 0.000999 | 0.000635 | 0 | 0.002046 | 0.000605 | 0 | 0.001109 | 0.00049 |
| 0.007642 | 0.037419 | 0.006456 | 0.004941 | 0 | 0.00448 | 0.004458 | 0 0.000661 | 0 | 0 |
| 0.00646 | 0.022461 | 0 | 0 | 0 | 0.004941 | 0 | 0 | 0.004236 | 0 |
| 0.005669 | 0.040007 | 0.001393 | 0.001303 | 0.00078 | 0.004792 | 0.001322 | | 0.004375 | 0.001462 |
| 0.003503 | 0.028066 | 0.00169 | 0.001482 | 0 | 0.002229 | 0.001389 | 0 | 0.00169 | 0.001299 |
| 4761 | Patient ID | | | | | | | | |
| 0.000274 | 2 | | | | | | | | |
| 0.00061 | 3 | | | | | | | | |
| 0.002232 | 4 | | | | | | | | |
| 0 | 6 | | | | | | | | |
| 0.000443 | 8 | | | | | | | | |
| 0.000084 | 12 | | | | | | | | |
| 0.00056 | 13 | | | | | | | | |
| 0 | 14 | | | | | | | | |
| 0.000999 | 15 | | | | | | | | |
| 0.000138 | 16 | | | | | | | | |
| 0 | 18 | | | | | | | | |
| 0.000356 | 19 | | | | | | | | |
| 0.000781 | 21 | | | | | | | | |
| 0.000849 | 22 | | | | | | | | |
| 0.000288 | 23 | | | | | | | | |
| 0.000138 | 24 | | | | | | | | |
| 0.000789 | 25 | | | | | | | | |
| 0.000512 | 26 | | | | | | | | |
| 0.00086 | 27 | | | | | | | | |
| 0.000487 | 29 | | | | | | | | |
| 0.000215 | 30 | | | | | | | | |
| 0.000285 | 31 | | | | | | | | |
| 0.000395 | 32 | | | | | | | | |
| 0.000438 | 33 | | | | | | | | |
| 0.000188 | 34 | | | | | | | | |
| 0 | 36 | | | | | | | | |
| 0.000619 | 37 | | | | | | | | |
| 0.000405 | 38 | | | | | | | | |
| 0.00158 | 39 | | | | | | | | |
| 0.000329 | 40 | | | | | | | | |
| 0.000676 | 41 | | | | | | | | |
| 0.000292 | 42 | | | | | | | | |
| 0.000293 | 43 | | | | | | | | |
| 0.000269 | 44 | | | | | | | | |
| 0.00041 | 45 | | | | | | | | |
| 0 | 46 | | | | | | | | |
| 0.000598 | 48 | | | | | | | | |
| 0.000605 | 49 | | | | | | | | |
| 0.001164 | 50 | | | | | | | | |
| 0.000234 | 51 | | | | | | | | |
| 0.000235 | 52 | | | | | | | | |
| 0.000153 | 53 | | | | | | | | |
| 0.000249 | 54 | | | | | | | | |
| 0.000641 | 55 | | | | | | | | |
| 0.000289 | 56 | | | | | | | | |
| 0 | 57 | | | | | | | | |
| 0 | 58 | | | | | | | | |
| 0.000446 | 59 | | | | | | | | |
| 0.000108 | 60 | | | | | | | | |
| 0 | 61 | | | | | | | | |
| 0.00045 | 62 | | | | | | | | |
| 0.000499 | 63 | | | | | | | | |
| 0.000359 | 64 | | | | | | | | |
| 0.000211 | 65 | | | | | | | | |
| 0.000605 | 66 | | | | | | | | |
| 0.000727 | 67 | | | | | | | | |
| 0.002201 | 68 | | | | | | | | |
| 0.00051 | 70 | | | | | | | | |
| 0.000997 | 72 | | | | | | | | |
| 0.000831 | 73 | | | | | | | | |
| 0.001022 | 74 | | | | | | | | |
| 0.001024 | 75 | | | | | | | | |
| 0.000366 | 76 | | | | | | | | |
| 0.000525 | 77 | | | | | | | | |
| 0 | 78 | | | | | | | | |
| 0.000828 | 79 | | | | | | | | |
| 0 | 80 | | | | | | | | |
| 0.000556 | 81 | | | | | | | | |
| 0 | 82 | | | | | | | | |
| 0 | 84 | | | | | | | | |
| 0.000864 | 85 | | | | | | | | |
| 0.000661 | 86 | | | | | | | | |
| 0 | 87 | | | | | | | | |
| 0 | 88 | | | | | | | | |
| 0 | 89 | | | | | | | | |
| 0 | 90 | | | | | | | | |
| 0 | 91 | | | | | | | | |
| 0 | 92 | | | | | | | | |
| 0.000473 | 93 | | | | | | | | |
| 0 | 94 | | | | | | | | |

**Lengthy Table 9**

| Patient ID | Leptin | Activin A | Follistatin | Activin_To_Follistatin | Triglycerides |
|---|---|---|---|---|---|
| 34 | 4.15 | 371.13 | 1.42 | 0.26 | 69 |
| 50 | 3.42 | 388.93 | 1.25 | 0.31 | 92 |
| 56 | 15.16 | 340.94 | 1.8 | 0.19 | 79 |
| 63 | 2.56 | 171.51 | 0.69 | 0.25 | 82 |
| 67 | 2.99 | 114.43 | 1.12 | 0.1 | 76 |
| 70 | 16.11 | 353.37 | 0.82 | 0.43 | 140 |
| 72 | 2.53 | 518.04 | 0.78 | 0.67 | 93 |
| 73 | 7.86 | 596.98 | 1.44 | 0.42 | 153 |
| 74 | 7.95 | 423.59 | 0.71 | 0.6 | 83 |
| 75 | 1.33 | 328.24 | 1.47 | 0.22 | 64 |
| 76 | 4 | 495.17 | 0.91 | 0.55 | 48 |
| 77 | 20.82 | 265.45 | 0.95 | 0.28 | 65 |
| 78 | 28.3 | 261.5 | 1.3 | 0.2 | 125 |
| 79 | 1.77 | 464.86 | 0.35 | 1.34 | 70 |
| 80 | 16.33 | 432.59 | 0.86 | 0.51 | 104 |
| 81 | 16.88 | 467.99 | 1.5 | 0.31 | 111 |
| 82 | 21.99 | 348.9 | 1.68 | 0.21 | |
| 90 | 2.66 | 178.39 | 0.9 | 0.2 | 99 |
| 91 | 9.51 | 333.9 | 1.65 | 0.2 | 107 |
| 92 | 15.08 | 304.23 | 0.94 | 0.32 | 106 |
| 93 | 11.9 | 311.93 | 0.76 | 0.41 | 161 |
| 94 | 11.8 | 252.74 | 0.64 | 0.39 | 101 |
| 13 | 14.53 | 345.1 | 1.06 | 0.33 | 266 |
| 15 | 24.55 | 457.78 | 0.7 | 0.66 | 101 |
| 33 | 8.9 | 319 | 0.99 | 0.32 | 135 |
| 36 | 25.13 | 513.8 | 0.9 | 0.57 | 85 |
| 37 | 29.32 | 606.41 | 0.8 | 0.76 | 76 |
| 38 | 6.47 | 249.84 | 1.02 | 0.25 | 164 |
| 39 | 12.53 | 519.99 | 1.07 | 0.49 | 79 |
| 40 | 12.99 | 365.72 | 0.88 | 0.42 | 83 |
| 43 | 18.37 | 324.98 | 1.68 | 0.19 | 74 |
| 44 | 17.1 | 332.44 | 0.71 | 0.47 | 114 |
| 46 | 43.92 | 537.99 | 1.23 | 0.44 | 125 |
| 51 | 10.47 | 395.66 | 1 | 0.4 | 116 |
| 52 | 1.92 | 390.23 | 1.1 | 0.35 | 87 |
| 53 | 10.35 | 213.69 | 1.28 | 0.17 | 228 |
| 54 | 13.36 | 468.68 | 0.84 | 0.56 | 82 |
| 55 | 17.73 | 316.4 | 1.52 | 0.21 | 277 |
| 57 | 23.67 | 426.05 | 0.75 | 0.57 | 119 |
| 59 | 3.92 | 383.33 | 0.85 | 0.45 | 116 |
| 61 | 33.61 | 250.47 | 0.41 | 0.62 | 91 |
| 62 | 56.64 | 292.23 | 1.16 | 0.25 | 152 |
| 66 | 6.4 | 245.52 | 0.64 | 0.39 | 101 |
| 84 | 32.12 | 420.62 | 0.67 | 0.63 | 116 |
| 85 | 3.36 | 310.65 | 0.46 | 0.67 | 73 |
| 86 | 2.4 | 225.09 | 0.98 | 0.23 | 119 |
| 87 | 17.34 | 254.19 | 1.74 | 0.15 | 115 |
| 88 | 2.87 | 217.79 | 0.77 | 0.28 | 92 |
| 89 | 9.4 | 327.68 | 1.08 | 0.3 | 75 |
| 12 | 8.96 | 315.71 | 1.1 | 0.29 | 293 |
| 18 | 29.09 | | 1.03 | | 157 |
| 19 | 53.66 | 431.22 | 0.88 | 0.49 | 163 |
| 24 | 42.37 | 542.29 | 0.61 | 0.88 | 320 |
| 25 | 12.7 | 343.32 | 0.63 | 0.55 | 91 |
| 25 | 12.7 | 343.32 | 0.63 | 0.55 | 91 |
| 27 | 19.39 | 520.83 | 0.81 | 0.64 | 322 |
| 29 | 25.46 | 441.91 | 1.06 | 0.42 | 106 |
| 30 | 24.65 | 317.21 | 0.62 | 0.51 | 130 |
| 31 | 34.49 | 504.52 | 0.86 | 0.59 | 148 |
| 32 | 10.61 | 586.5 | 0.3 | 1.97 | 102 |
| 41 | 3.74 | 278.64 | 0.65 | 0.43 | 41 |
| 42 | 27.43 | 386.78 | 0.85 | 0.46 | 158 |
| 48 | 3.38 | 426.15 | 1.13 | 0.38 | 150 |
| 49 | 3.88 | 265.44 | 0.83 | 0.32 | 165 |
| 65 | 1.62 | 330.56 | 1.5 | 0.22 | 88 |
| | | | | | |
| 2 | 21.25 | 491.66 | 1.97 | 0.25 | 173 |
| 3 | 50.27 | | 1.09 | | 137 |
| 4 | 58.82 | 806.22 | 1.6 | 0.5 | 139 |
| 6 | 24.21 | 517.54 | 0.56 | 0.93 | 121 |
| 8 | 14.08 | 438.93 | 0.35 | 1.25 | 182 |
| 14 | 21.73 | 374.68 | 1.03 | 0.36 | 318 |
| 16 | 16.57 | 375.98 | 0.83 | 0.45 | 116 |
| 21 | 1.82 | 441.56 | 0.83 | 0.53 | 94 |
| 22 | 45.63 | 436.5 | 1.2 | 0.36 | 261 |
| 23 | 68.27 | 585.36 | 1.12 | 0.52 | 412 |
| 26 | 43.46 | 628.09 | 1.45 | 0.43 | 178 |
| 45 | 17.17 | 277.41 | 1.75 | 0.16 | 168 |
| 58 | 13.71 | 291.59 | 0.84 | 0.35 | 449 |
| 60 | 12.86 | 282.04 | 1.5 | 0.19 | 129 |
| 64 | 24.44 | 418.74 | 1.31 | 0.32 | 200 |
| 68 | 81.7 | 1350.01 | 0.74 | 1.82 | 105 |

**Lengthy Table 10**

| AcCa | Cer | ChE | Co | DG | Hex1Cer | Hex2Cer |
|---|---|---|---|---|---|---|
| 0.000891531 | 0.016293682 | 0.020649253 | 5.55E-05 | 0.001103937 | 0.00226872 | 0.001111855 |
| 0.000483677 | 0.022587743 | 0.015325044 | 8.22E-05 | 0.001415038 | 0.002771405 | 0.001338114 |
| 0.000920705 | 0.012410565 | 0.016129145 | 5.97E-05 | 0.000783197 | 0.002743358 | 0.001338027 |
| 0.001370459 | 0.018052846 | 0.024180499 | 0.000101555 | 0.00147277 | 0.002164862 | 0.001255387 |
| 0.001604843 | 0.015285383 | 0.018169922 | 4.26E-05 | 0.001232657 | 0.003126606 | 0.001304874 |
| 0.001925026 | 0.012343749 | 0.019363167 | 6.80E-05 | 0.000949677 | 0.002284742 | 0.000978214 |
| 0.001576957 | 0.011142286 | 0.02995847 | 7.77E-05 | 0.001178989 | 0.002846321 | 0.0008556 |
| 0.00136407 | 0.013837424 | 0.023553558 | 8.88E-05 | 0.001532456 | 0.002435523 | 0.00161861 |
| 0.001636594 | 0.013623775 | 0.020391833 | 9.58E-05 | 0.001489599 | 0.002634411 | 0.001401914 |
| 0.000835562 | 0.023836916 | 0.027026911 | 3.60E-05 | 0.001091048 | 0.005216412 | 0.003399725 |
| 0.001106375 | 0.021783428 | 0.014913555 | 6.12E-05 | 0.00121785 | 0.003469824 | 0.001471057 |
| 0.001520172 | 0.013909846 | 0.02280941 | 7.84E-05 | 0.001309178 | 0.002630152 | 0.001382352 |
| 0.001039591 | 0.018417734 | 0.02827488 | 0.000134147 | 0.001283214 | 0.00353206 | 0.001444454 |
| 0.000857695 | 0.009354141 | 0.02208665 | 5.98E-05 | 0.000822706 | 0.002559189 | 0.001362935 |
| 0.003347099 | 0.014944294 | 0.027422852 | 0.000160912 | 0.001447359 | 0.005050374 | 0.001909533 |
| 0.00096702 | 0.013473707 | 0.020967106 | 5.62E-05 | 0.001315889 | 0.001880426 | 0.000894425 |
| 0.001146544 | 0.014533798 | 0.026274148 | 0.000118525 | 0.000808348 | 0.003444509 | 0.001141166 |
| 0.001292603 | 0.014559951 | 0.028021732 | 7.30E-05 | 0.000767862 | 0.002251076 | 0.001097512 |
| 0.001681853 | 0.014510089 | 0.031737778 | 5.97E-05 | 0.001052757 | 0.003676736 | 0.001854132 |
| 0.002363021 | 0.014936096 | 0.026327572 | 0.00019853 | 0.000834616 | 0.002455538 | 0.001383041 |
| 0.000663962 | 0.021099524 | 0.017001769 | 7.14E-05 | 0.001172754 | 0.004212353 | 0.001223823 |
| 0.001186231 | 0.013571171 | 0.022686016 | 6.22E-05 | 0.001104652 | 0.003105596 | 0.00110898 |
| 0.000612871 | 0.014721087 | 0.012989219 | 8.10E-05 | 0.001551846 | 0.00230457 | 0.000985886 |
| 0.00245652 | 0.012881041 | 0.018259005 | 3.00E-05 | 0.000776848 | 0.004641539 | 0.002643241 |
| 0.002147252 | 0.022741885 | 0.030350916 | 5.11E-05 | 0.00091967 | 0.004876763 | 0.002295754 |
| 0.000797979 | 0.019180669 | 0.021456443 | 5.97E-05 | 0.000719799 | 0.003950443 | 0.001950841 |
| 0.001981468 | 0.012052362 | 0.029322396 | 5.20E-05 | 0.001048753 | 0.004061051 | 0.001820596 |
| 0.00084196 | 0.020037184 | 0.012935985 | 4.05E-05 | 0.001445026 | 0.002360175 | 0.001191262 |
| 0.001067005 | 0.020717539 | 0.031431909 | 4.56E-05 | 0.00075097 | 0.003698769 | 0.002009934 |
| 0.000957565 | 0.026951248 | 0.015254553 | 4.48E-05 | 0.000995919 | 0.003450503 | 0.00130342 |
| 0.000894903 | 0.020119426 | 0.030455092 | 4.72E-05 | 0.000860363 | 0.004623869 | 0.001986757 |
| 0.001612561 | 0.020415015 | 0.023597676 | 3.52E-05 | 0.001623288 | 0.004061235 | 0.001847901 |
| 0.000868874 | 0.029383084 | 0.021056515 | 3.65E-05 | 0.000902276 | 0.003664527 | 0.002393823 |
| 0.000470799 | 0.033805978 | 0.007863133 | 4.18E-05 | 0.002075191 | 0.003012872 | 0.00095779 |
| 0.000961925 | 0.021166844 | 0.017711413 | 5.90E-05 | 0.00097786 | 0.002378828 | 0.001326703 |
| 0.001307441 | 0.02221376 | 0.029905851 | 3.98E-05 | 0.000855963 | 0.006003081 | 0.00242914 |
| 0.000348354 | 0.023238045 | 0.011911302 | 5.60E-05 | 0.001674409 | 0.00273222 | 0.001046696 |
| 0.002055494 | 0.01768667 | 0.025996785 | 8.15E-05 | 0.001391 | 0.002927463 | 0.001349484 |
| 0.000957682 | 0.037130326 | 0.009580068 | 3.87E-05 | 0.001457968 | 0.002886789 | 0.001325096 |
| 0.001274849 | 0.017107934 | 0.019289863 | 0.000130913 | 0.000815944 | 0.003766212 | 0.001737237 |
| 0.000900449 | 0.02138934 | 0.015569447 | 0.00011769 | 0.001049253 | 0.002499019 | 0.001089589 |
| 0.001921799 | 0.016083335 | 0.021832159 | 3.90E-05 | 0.001018258 | 0.005657015 | 0.001847522 |
| 0.000680328 | 0.013325208 | 0.012166849 | 3.48E-05 | 0.001166552 | 0.002442879 | 0.001251218 |
| 0.001364121 | 0.025473302 | 0.018064798 | 2.50E-05 | 0.001570862 | 0.004085409 | 0.001167168 |
| 0.001145442 | 0.017824514 | 0.023063401 | 4.25E-05 | 0.000812656 | 0.002555743 | 0.001204939 |
| 0.000673228 | 0.024556911 | 0.013818223 | 4.26E-05 | 0.000970946 | 0.002320639 | 0.001313045 |
| 0.001298129 | 0.015257071 | 0.029133204 | 4.66E-05 | 0.001065349 | 0.002474946 | 0.001284999 |
| 0.001294417 | 0.019852512 | 0.035461752 | 7.28E-05 | 0.000814425 | 0.003903561 | 0.001776698 |
| 0.001012446 | 0.024778463 | 0.017028662 | 2.68E-05 | 0.001660925 | 0.003091891 | 0.001078792 |
| 0.003080922 | 0.019938627 | 0.024664856 | 5.22E-05 | 0.000893261 | 0.005871864 | 0.003333354 |
| 0.0009056 | 0.025300599 | 0.02106714 | 3.78E-05 | 0.000888221 | 0.003578593 | 0.00193053 |
| 0.001113309 | 0.022228535 | 0.021793757 | 4.30E-05 | 0.000731571 | 0.003854768 | 0.001616033 |
| 0.001164986 | 0.019233762 | 0.018616839 | 2.49E-05 | 0.001100765 | 0.004133687 | 0.001631117 |
| 0.002058412 | 0.02292212 | 0.030279359 | 8.16E-05 | 0.000761336 | 0.004549759 | 0.001333999 |
| 0.002273195 | 0.017366305 | 0.025895286 | 2.87E-05 | 0.001359113 | 0.003287172 | 0.001299139 |
| 0.002764033 | 0.019804885 | 0.024539189 | 4.01E-05 | 0.000828892 | 0.004479688 | 0.002056855 |
| 0.001856636 | 0.020988587 | 0.022906309 | 3.01E-05 | 0.001149856 | 0.004127329 | 0.001966943 |
| 0.000870098 | 0.018312885 | 0.019540017 | 4.94E-05 | 0.000869878 | 0.002060374 | 0.001276845 |
| 0.000752343 | 0.027400286 | 0.014513891 | 1.26E-05 | 0.000925334 | 0.003644815 | 0.001663117 |
| 0.000844701 | 0.019539626 | 0.014767408 | 7.75E-05 | 0.000759017 | 0.001498042 | 0.000636346 |
| 0.001660375 | 0.015990123 | 0.017351443 | 4.22E-05 | 0.00137735 | 0.003188858 | 0.001848672 |
| 0.000945861 | 0.021274059 | 0.005344047 | 3.58E-05 | 0.001860395 | 0.002565958 | 0.001118417 |
| 0.000711354 | 0.028500678 | 0.012070599 | 3.13E-05 | 0.001534505 | 0.002775461 | 0.001635606 |
| 0.000680614 | 0.018764289 | 0.008463032 | 2.52E-05 | 0.001569044 | 0.003123837 | 0.001513974 |
| 0.000243124 | 0.019988735 | 0.005954585 | 2.35E-05 | 0.001748001 | 0.002708209 | 0.001080605 |
| 0.000826045 | 0.018923223 | 0.007221529 | 2.71E-05 | 0.002980682 | 0.001771038 | 0.00103843 |
| 0.000971071 | 0.017879071 | 0.01528538 | 5.03E-05 | 0.000780515 | 0.003547786 | 0.001664465 |
| 0.00169989 | 0.020599558 | 0.016165039 | 2.24E-05 | 0.001694949 | 0.00245152 | 0.00119262 |
| 0.000971709 | 0.020942396 | 0.014249947 | 2.46E-05 | 0.001838051 | 0.002162381 | 0.000849225 |
| 0.001502521 | 0.024167449 | 0.015639054 | 2.81E-05 | 0.001803405 | 0.003367815 | 0.00178509 |
| 0.001152482 | 0.01793257 | 0.022704045 | 2.09E-05 | 0.00094905 | 0.002647243 | 0.000906773 |
| 0.00046641 | 0.022203615 | 0.01045915 | 3.13E-05 | 0.001677061 | 0.002986221 | 0.001152305 |
| 0.000410067 | 0.028165031 | 0.011219225 | 1.92E-05 | 0.001217872 | 0.003310367 | 0.001600838 |
| 0.000844659 | 0.02151937 | 0.014390445 | 4.86E-05 | 0.001726238 | 0.002901616 | 0.001080068 |
| 0.000806548 | 0.024243815 | 0.00966479 | 1.52E-05 | 0.001597143 | 0.002887717 | 0.00136242 |
| 0.00093453 | 0.024239019 | 0.013732938 | 1.25E-05 | 0.001697251 | 0.002884169 | 0.001097434 |
| 0.000888744 | 0.015252286 | 0.015743035 | 1.08E-05 | 0.001223011 | 0.004085653 | 0.001944753 |
| 0.00088746 | 0.015501402 | 0.012006724 | 5.65E-05 | 0.001408428 | 0.005061976 | 0.002146045 |
| 0.000672401 | 0.012651219 | 0.012889639 | 2.67E-05 | 0.002088533 | 0.001861294 | 0.000994013 |

| LPC | LPE | PA | PC | PE | PG | PI |
|---|---|---|---|---|---|---|
| 0.09290021 | 0.005724437 | 0.004317474 | 1.066739103 | 0.044815419 | 0.000707369 | 0.055141198 |
| 0.106929807 | 0.006797852 | 0.002921341 | 1.059298782 | 0.041461413 | 0.000640734 | 0.04702294 |
| 0.097046785 | 0.005498222 | 0.004531906 | 1.087650124 | 0.038007176 | 0.000564928 | 0.059756706 |
| 0.100350929 | 0.005537938 | 0.003676377 | 1.092903706 | 0.047554329 | 0.000777577 | 0.03924906 |
| 0.119703994 | 0.009604878 | 0.004804649 | 1.010771804 | 0.047869114 | 0.000670711 | 0.061036958 |
| 0.156713899 | 0.008597588 | 0.004069455 | 1.103524004 | 0.024287522 | 0.000373875 | 0.041369232 |
| 0.131206487 | 0.008217985 | 0.003769163 | 1.02155268 | 0.045752638 | 0.000630893 | 0.064302661 |
| 0.06444715 | 0.004668725 | 0.003567636 | 1.047500475 | 0.035234413 | 0.00069279 | 0.053651192 |
| 0.094656033 | 0.005839987 | 0.004013184 | 1.074597873 | 0.029873577 | 0.0004718 | 0.050001346 |
| 0.150317198 | 0.005190561 | 0.005446226 | 1.083228658 | 0.021807596 | 0.000720161 | 0.051217415 |
| 0.08836449 | 0.007261096 | 0.004253112 | 1.04145115 | 0.043556761 | 0.000791783 | 0.054916893 |
| 0.121192923 | 0.007030542 | 0.003622723 | 1.094307513 | 0.034588734 | 0.000463333 | 0.044035396 |
| 0.076874302 | 0.005367782 | 0.003039862 | 0.996515495 | 0.053754294 | 0.000478054 | 0.048534017 |
| 0.099160518 | 0.005932674 | 0.003764097 | 1.053876332 | 0.043816378 | 0.000564827 | 0.059120512 |
| 0.189330493 | 0.008674567 | 0.003923408 | 1.117935611 | 0.040515933 | 0.00059065 | 0.04477849 |
| 0.079891135 | 0.005601046 | 0.003272174 | 1.016723465 | 0.045669335 | 0.000740442 | 0.052394311 |
| 0.091716817 | 0.005609887 | 0.003931095 | 1.120181067 | 0.043573853 | 0.000549946 | 0.044896264 |
| 0.11223872 | 0.005318281 | 0.003601155 | 1.099934818 | 0.047707471 | 0.000553069 | 0.058721036 |
| 0.121797392 | 0.004889832 | 0.004253579 | 1.118496992 | 0.033555036 | 0.000639344 | 0.049080211 |
| 0.072760925 | 0.004667267 | 0.004498457 | 1.150112739 | 0.042147536 | 0.000518995 | 0.049395347 |
| 0.051754345 | 0.005364425 | 0.003579131 | 1.045012109 | 0.03921068 | 0.001035242 | 0.047192799 |
| 0.108818872 | 0.011653351 | 0.004037055 | 0.971460085 | 0.051047419 | 0.00071869 | 0.049166998 |
| 0.136443688 | 0.007817512 | 0.003601282 | 1.024609304 | 0.048016569 | 0.000721323 | 0.067575843 |
| 0.104678537 | 0.003654907 | 0.004643619 | 1.165943261 | 0.020852061 | 0.000868313 | 0.044942626 |
| 0.120461309 | 0.004858412 | 0.005000372 | 1.058877854 | 0.018970529 | 0.000599793 | 0.034263554 |
| 0.096349835 | 0.010547516 | 0.004305226 | 1.049607885 | 0.025274955 | 0.000512116 | 0.043588146 |
| 0.116668885 | 0.005938338 | 0.004455035 | 1.141058717 | 0.02496657 | 0.000537256 | 0.044108446 |
| 0.055814576 | 0.005368255 | 0.004642325 | 1.07298999 | 0.04178739 | 0.001147777 | 0.052932743 |
| 0.115783692 | 0.005822372 | 0.006226804 | 1.107092739 | 0.021362542 | 0.000793392 | 0.044649835 |
| 0.07469706 | 0.00652396 | 0.004771606 | 1.098391844 | 0.046423521 | 0.001303178 | 0.048634805 |
| 0.099322359 | 0.005477532 | 0.003770179 | 1.015355086 | 0.031849258 | 0.000532618 | 0.042330955 |
| 0.149743108 | 0.007185133 | 0.004435908 | 1.062121115 | 0.037731703 | 0.001689528 | 0.04494577 |
| 0.109830015 | 0.007466769 | 0.00303713 | 1.077451077 | 0.023534585 | 0.00121375 | 0.04563715 |
| 0.069100608 | 0.009179346 | 0.004848921 | 1.009921202 | 0.052754075 | 0.001291123 | 0.043742792 |
| 0.101156884 | 0.008750466 | 0.004052539 | 1.098377676 | 0.040901791 | 0.001191006 | 0.051468357 |
| 0.096558434 | 0.007730614 | 0.005235417 | 1.05517809 | 0.026463062 | 0.001178286 | 0.049112369 |
| 0.057096037 | 0.005390194 | 0.004520964 | 1.073040049 | 0.042509022 | 0.001284163 | 0.04646485 |
| 0.181123778 | 0.004497036 | 0.004126772 | 1.163274107 | 0.034748209 | 0.000895006 | 0.043384268 |
| 0.100080079 | 0.005864871 | 0.005353497 | 1.005732843 | 0.037918754 | 0.001425985 | 0.05501816 |
| 0.137957098 | 0.009181878 | 0.002809003 | 1.084180657 | 0.031259617 | 0.000991162 | 0.05762792 |
| 0.099552226 | 0.006414644 | 0.004042272 | 1.110049077 | 0.055460361 | 0.000862942 | 0.047051106 |
| 0.1545594 | 0.010939604 | 0.005291239 | 1.054039698 | 0.040277154 | 0.000907297 | 0.052006339 |
| 0.087428256 | 0.007119972 | 0.004610188 | 1.075118251 | 0.029086396 | 0.001254116 | 0.042311077 |
| 0.158792862 | 0.008226961 | 0.004953963 | 1.036375606 | 0.030195922 | 0.00135384 | 0.038570886 |
| 0.096300876 | 0.005226353 | 0.004321141 | 1.061511526 | 0.034296797 | 0.000898643 | 0.038362332 |
| 0.060162467 | 0.003775825 | 0.005515416 | 1.090290146 | 0.036788462 | 0.001328922 | 0.044645335 |
| 0.097460054 | 0.004764144 | 0.003719535 | 0.960059852 | 0.030266529 | 0.001062563 | 0.0542195 |
| 0.111906295 | 0.005189585 | 0.004496234 | 1.090657725 | 0.030876417 | 0.000771544 | 0.050013754 |
| 0.150035322 | 0.005926967 | 0.004693452 | 1.054517143 | 0.041719195 | 0.000905035 | 0.055987824 |
| 0.109149088 | 0.005498871 | 0.004823343 | 1.079849239 | 0.028104688 | 0.001353083 | 0.049308578 |
| 0.096799513 | 0.006141978 | 0.005305562 | 1.037571908 | 0.034182189 | 0.001036449 | 0.041695915 |
| 0.120290138 | 0.006926434 | 0.004355226 | 1.0473765 | 0.035418551 | 0.001143866 | 0.035195381 |
| 0.093373764 | 0.008530861 | 0.003586276 | 0.971053817 | 0.043765025 | 0.000682013 | 0.042857794 |
| 0.130730882 | 0.007554961 | 0.004516324 | 1.085091548 | 0.023898988 | 0.000726548 | 0.042787225 |
| 0.145060299 | 0.005726068 | 0.004108476 | 1.08461129 | 0.030375801 | 0.001047516 | 0.04877267 |
| 0.173533369 | 0.007083993 | 0.005414097 | 1.00972408 | 0.021607984 | 0.001534686 | 0.039195434 |
| 0.110622298 | 0.007091267 | 0.004701954 | 1.013057264 | 0.028738013 | 0.00122655 | 0.068622418 |
| 0.107762173 | 0.008549835 | 0.004358147 | 1.032679717 | 0.03586696 | 0.001232037 | 0.046744935 |
| 0.095584183 | 0.006908296 | 0.005879112 | 1.011844093 | 0.02905308 | 0.001199944 | 0.050508851 |
| 0.063808174 | 0.006173671 | 0.004447601 | 1.142743263 | 0.028171783 | 0.00135907 | 0.019254258 |
| 0.159239364 | 0.00786025 | 0.004200004 | 1.101205773 | 0.040760333 | 0.001327513 | 0.044372173 |
| 0.105347543 | 0.006463618 | 0.004600197 | 1.023727939 | 0.033811682 | 0.001467322 | 0.05378332 |
| 0.085565086 | 0.005106651 | 0.005024354 | 1.069876974 | 0.029232789 | 0.001562405 | 0.05357419 |
| 0.066502183 | 0.008045762 | 0.004056279 | 0.987558959 | 0.031088014 | 0.001267811 | 0.045779269 |
| 0.044367755 | 0.00406603 | 0.00416131 | 0.996445061 | 0.033550475 | 0.001132773 | 0.05135261 |
| 0.102053516 | 0.004997744 | 0.005097251 | 0.954868805 | 0.030416181 | 0.001789543 | 0.050012595 |
| 0.172377558 | 0.009530046 | 0.005647438 | 1.110613163 | 0.016529848 | 0.001978579 | 0.030267929 |
| 0.098013945 | 0.009189908 | 0.00547109 | 1.099167519 | 0.029896401 | 0.00179268 | 0.039954756 |
| 0.115678011 | 0.005148921 | 0.005483431 | 1.082359253 | 0.026190156 | 0.001447851 | 0.055367671 |
| 0.121252551 | 0.006063963 | 0.005044127 | 1.122549678 | 0.027470126 | 0.001696908 | 0.048758259 |
| 0.147722692 | 0.005690312 | 0.003822177 | 1.145405638 | 0.021208871 | 0.000875887 | 0.039165294 |
| 0.049183878 | 0.004542267 | 0.00373073 | 1.014199221 | 0.041882402 | 0.00144244 | 0.065893136 |
| 0.087187774 | 0.006378854 | 0.004456426 | 1.000568694 | 0.016780772 | 0.000994009 | 0.043080982 |
| 0.106142677 | 0.006622354 | 0.003890216 | 0.900592404 | 0.039041558 | 0.001431854 | 0.056270532 |
| 0.106325229 | 0.007829705 | 0.004062597 | 1.048263968 | 0.027454492 | 0.001444708 | 0.047096538 |
| 0.105426033 | 0.006421911 | 0.004361806 | 1.108465815 | 0.022884066 | 0.001146941 | 0.052054529 |
| 0.069584824 | 0.005058826 | 0.00408054 | 1.101908479 | 0.021935197 | 0.001077665 | 0.042125646 |
| 0.060691941 | 0.004393928 | 0.004269639 | 1.083446557 | 0.020090922 | 0.000874911 | 0.04569815 |
| 0.094970935 | 0.007061915 | 0.00461233 | 1.058024576 | 0.02919354 | 0.001373026 | 0.047492663 |

| PS | SM | TG | Patient ID | | | |
|---|---|---|---|---|---|---|
| 0.000308532 | 0.062085896 | 0.624885927 | 94 | | | |
| 0.000365347 | 0.05118075 | 0.639377788 | 93 | | | |
| 0.000326894 | 0.052890956 | 0.619341566 | 92 | | | |
| 0.000331582 | 0.074588117 | 0.586432008 | 91 | | | |
| 0.000341335 | 0.051057488 | 0.653372136 | 90 | | | |
| 0.000350085 | 0.053349402 | 0.569452343 | 89 | | | |
| 0.0003119 | 0.068225042 | 0.60839422 | 88 | | | |
| 0.000432777 | 0.062912029 | 0.682462379 | 87 | | | |
| 0.000273747 | 0.049502796 | 0.649495711 | 86 | | | |
| 0.000226565 | 0.060102704 | 0.560300335 | 85 | | | |
| 0.00030347 | 0.073404678 | 0.641673311 | 84 | | | |
| 0.000238645 | 0.068127909 | 0.582752787 | 82 | | | |
| 0.000582657 | 0.06260418 | 0.698123277 | 81 | | | |
| 0.000422365 | 0.074114573 | 0.62212459 | 80 | | | |
| 0.000385448 | 0.095440595 | 0.444142381 | 79 | | | |
| 0.000465632 | 0.054596341 | 0.701091378 | 78 | | | |
| 0.000250895 | 0.087688159 | 0.55413498 | 77 | | | |
| 0.000295497 | 0.100955337 | 0.522610879 | 76 | | | |
| 0.000172671 | 0.098916169 | 0.513625682 | 75 | | | |
| 0.000235016 | 0.072708287 | 0.554457015 | 74 | | | |
| 0.000357983 | 0.056916125 | 0.70413162 | 73 | | | |
| 0.000542213 | 0.058636972 | 0.701093457 | 72 | | | |
| 0.000368917 | 0.048925389 | 0.628673647 | 70 | | | |
| 0.00025767 | 0.05312498 | 0.559345797 | 68 | | | |
| 0.000161573 | 0.056272129 | 0.6371511 | 67 | | | |
| 0.00068869 | 0.041052517 | 0.679957262 | 66 | | | |
| 0.000179608 | 0.067187109 | 0.544561412 | 65 | | | |
| 0.000440168 | 0.034343024 | 0.691681681 | 64 | | | |
| 0.000125693 | 0.08471308 | 0.553708142 | 63 | | | |
| 0.000519606 | 0.050228722 | 0.619547654 | 62 | | | |
| 0.000273565 | 0.075585531 | 0.666515318 | 61 | | | |
| 0.000534653 | 0.066106421 | 0.572313755 | 60 | | | |
| 0.000349632 | 0.060915704 | 0.612258625 | 59 | | | |
| 0.000646878 | 0.022455651 | 0.7378318 | 58 | | | |
| 0.000270122 | 0.050773443 | 0.598475166 | 57 | | | |
| 0.0003623 | 0.061919971 | 0.633506444 | 56 | | | |
| 0.000430294 | 0.032002228 | 0.696255179 | 55 | | | |
| 0.000146258 | 0.099171236 | 0.417144976 | 54 | | | |
| 0.000409077 | 0.047364818 | 0.687455305 | 53 | | | |
| 0.000299771 | 0.06513328 | 0.566436663 | 52 | | | |
| 0.000266491 | 0.060328786 | 0.573357308 | 51 | | | |
| 0.000384011 | 0.070112072 | 0.563084084 | 50 | | | |
| 0.000338229 | 0.036783037 | 0.684882685 | 49 | | | |
| 0.000398876 | 0.054865368 | 0.614515098 | 48 | | | |
| 0.000158702 | 0.06046726 | 0.651807172 | 46 | | | |
| 0.000358313 | 0.04304224 | 0.670397238 | 45 | | | |
| 0.000189934 | 0.068504677 | 0.729192904 | 44 | | | |
| 0.00025878 | 0.093348666 | 0.5493048 | 43 | | | |
| 0.000312889 | 0.049062722 | 0.588161444 | 42 | | | |
| 0.000384564 | 0.078433498 | 0.585259926 | 41 | | | |
| 0.000492945 | 0.072430079 | 0.650634958 | 40 | | | |
| 0.000378782 | 0.07444035 | 0.623093844 | 39 | | | |
| 0.000442 | 0.038978622 | 0.750823735 | 38 | | | |
| 0.000346222 | 0.097013652 | 0.545347085 | 37 | | | |
| 0.000293593 | 0.074532991 | 0.553962396 | 36 | | | |
| 0.000292351 | 0.079586995 | 0.607513368 | 34 | | | |
| 0.000321041 | 0.064931168 | 0.64766228 | 33 | | | |
| 0.000493287 | 0.063800532 | 0.655532835 | 32 | | | |
| 0.000290143 | 0.062474531 | 0.687345398 | 31 | | | |
| 0.0002265 | 0.047777896 | 0.647915186 | 30 | | | |
| 0.000395816 | 0.071778241 | 0.527401535 | 29 | | | |
| 0.000353892 | 0.029642077 | 0.707657826 | 27 | | | |
| 0.000350553 | 0.048680925 | 0.653766558 | 26 | | | |
| 0.000466411 | 0.032807525 | 0.788287841 | 24 | | | |
| 0.000414098 | 0.022427998 | 0.810335171 | 23 | | | |
| 0.000461664 | 0.036847069 | 0.780667599 | 22 | | | |
| 0.000212873 | 0.050304594 | 0.562359344 | 21 | | | |
| 0.000311946 | 0.054415423 | 0.617960313 | 19 | | | |
| 0.000139917 | 0.05299826 | 0.614148206 | 18 | | | |
| 0.000390336 | 0.07691918 | 0.541561414 | 16 | | | |
| 0.000125847 | 0.07668775 | 0.512982491 | 15 | | | |
| 0.000398292 | 0.029139328 | 0.750612252 | 14 | | | |
| 0.000168821 | 0.027033856 | 0.767407219 | 13 | | | |
| 0.000290287 | 0.051207186 | 0.791999974 | 12 | | | |
| 0.000357338 | 0.039755594 | 0.676832187 | 8 | | | |
| 0.000157435 | 0.053104477 | 0.601379151 | 6 | | | |
| 0.000356271 | 0.041218705 | 0.673505533 | 4 | | | |
| 0.000215389 | 0.043283375 | 0.699966658 | 3 | | | |
| 0.000501795 | 0.035029171 | 0.690556248 | 2 | | | |

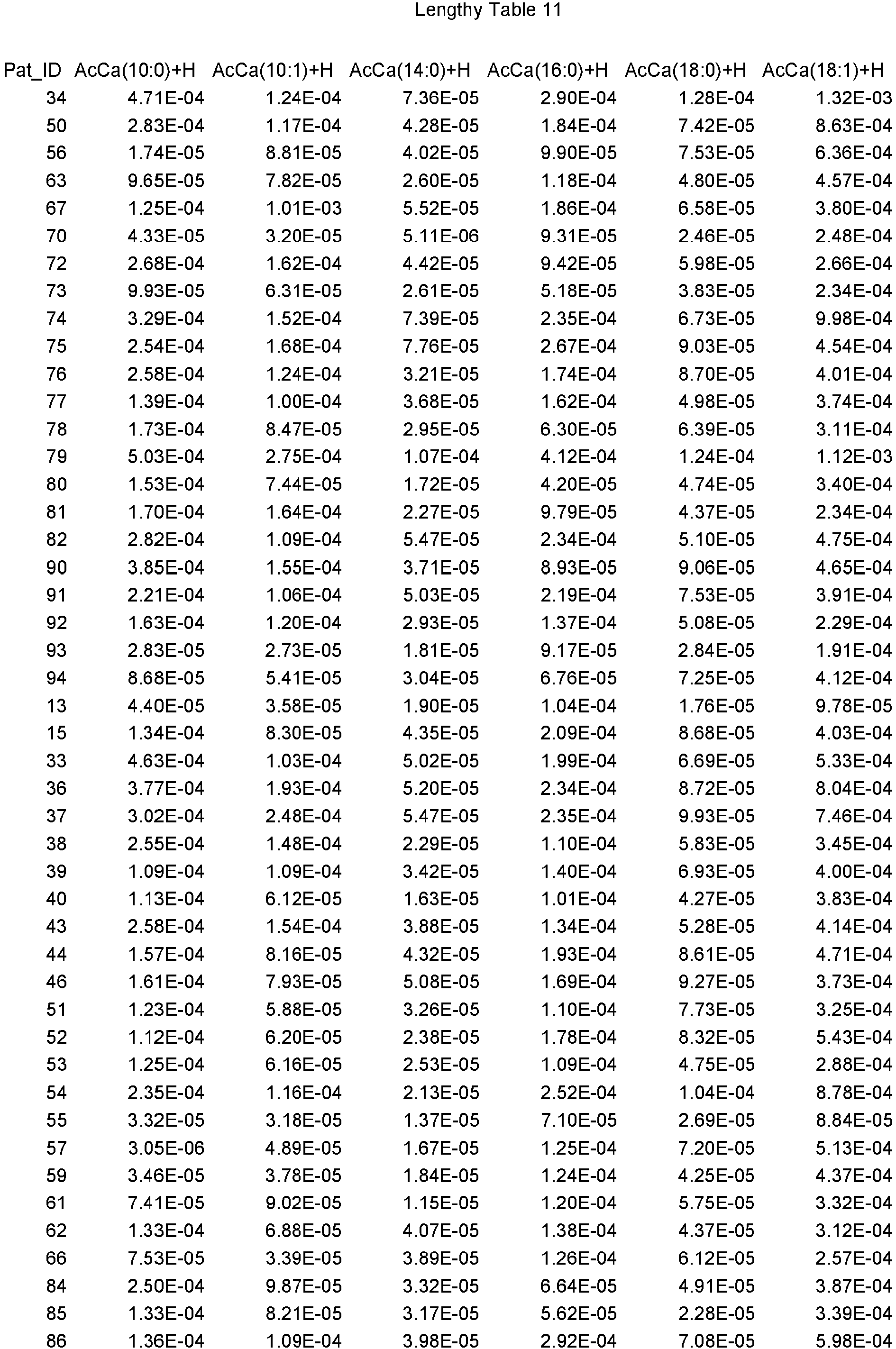

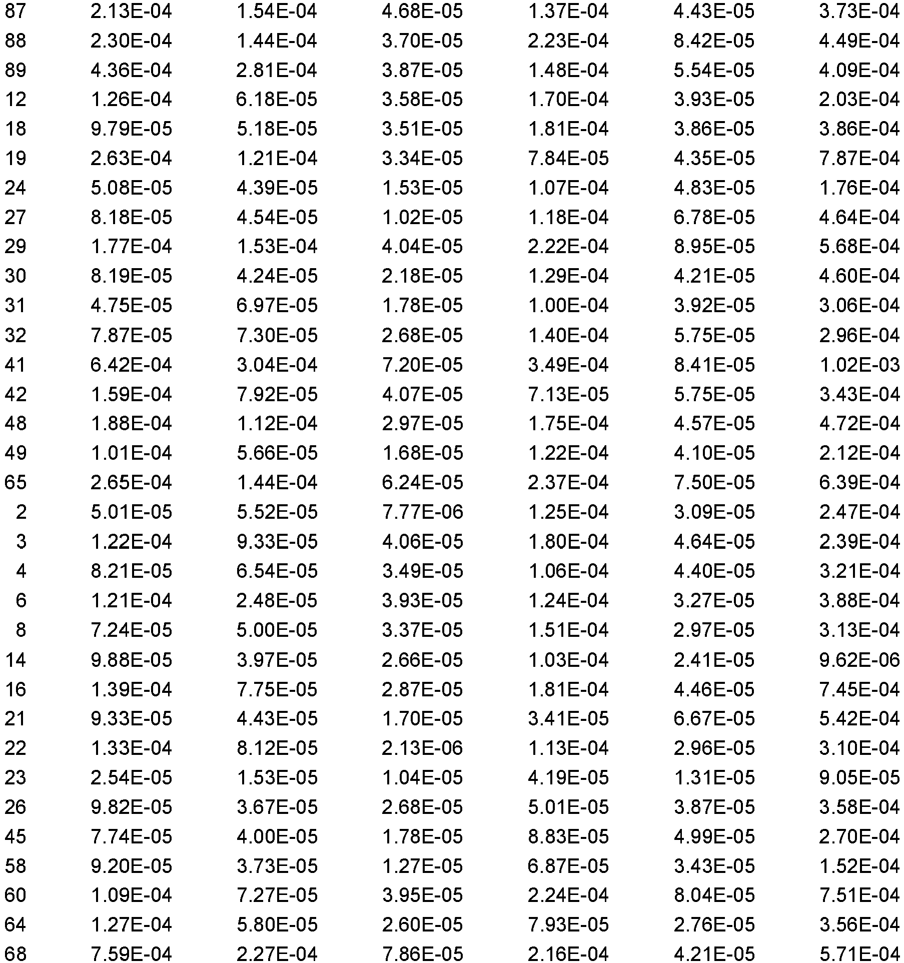

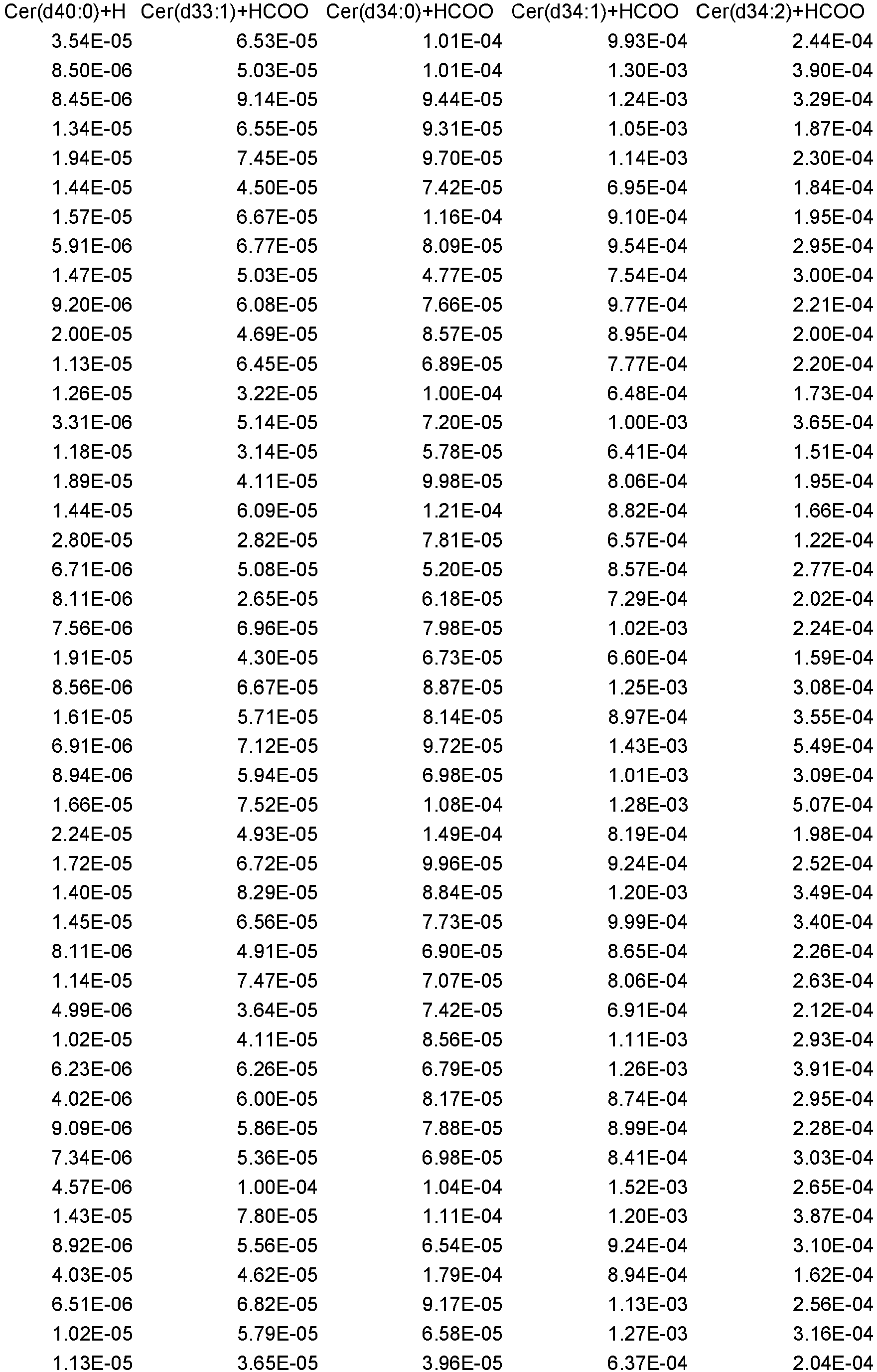

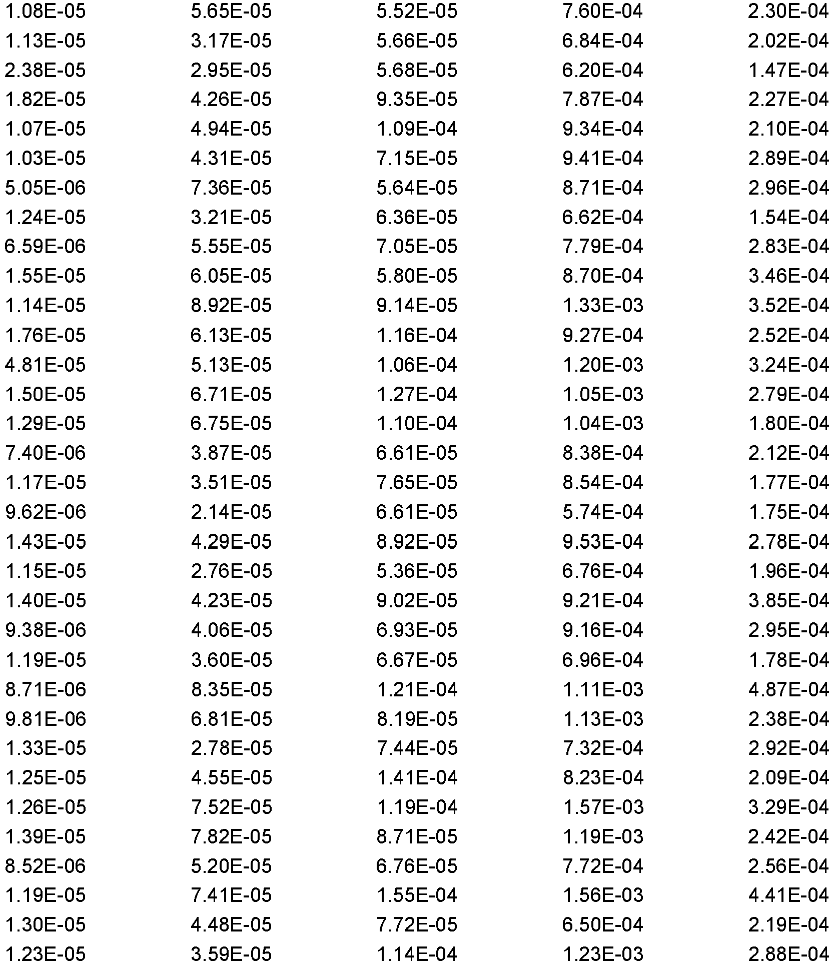

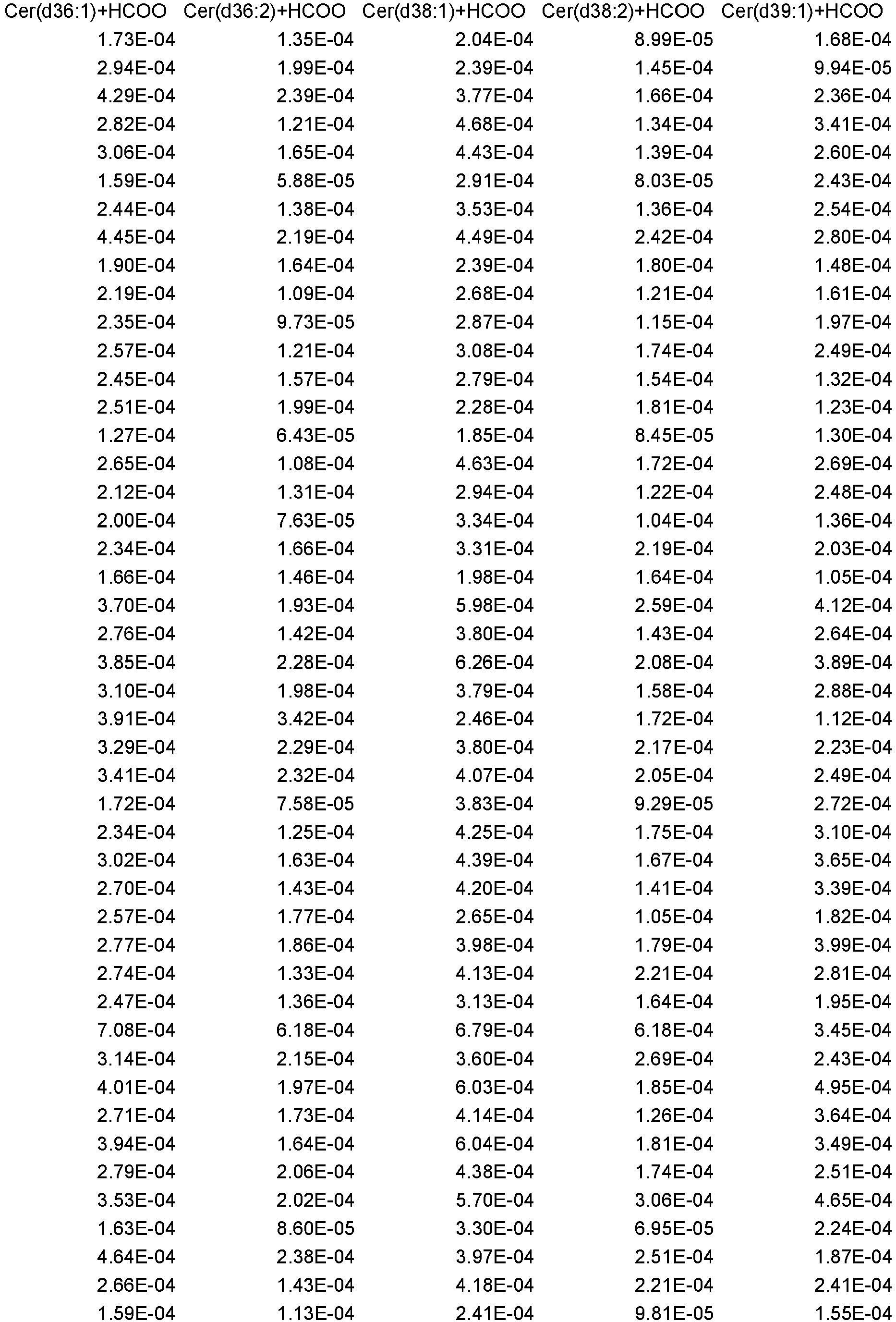

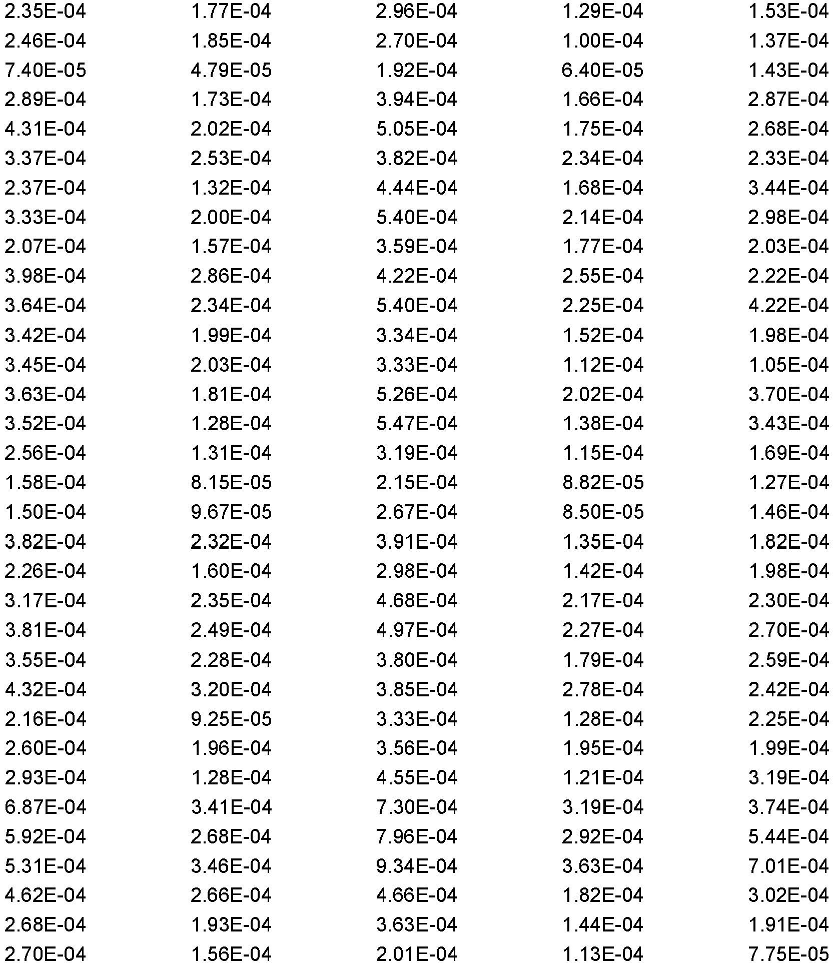

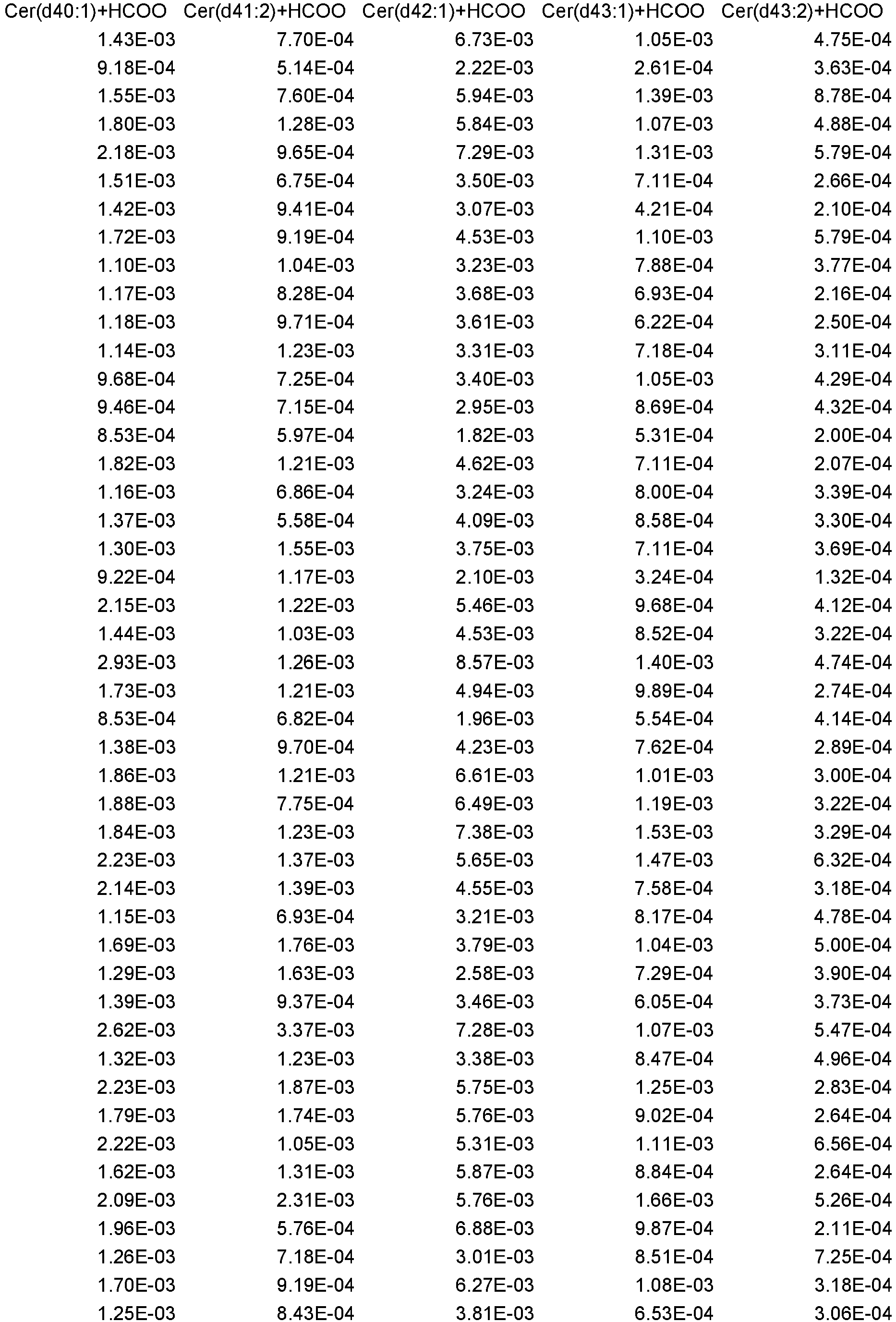

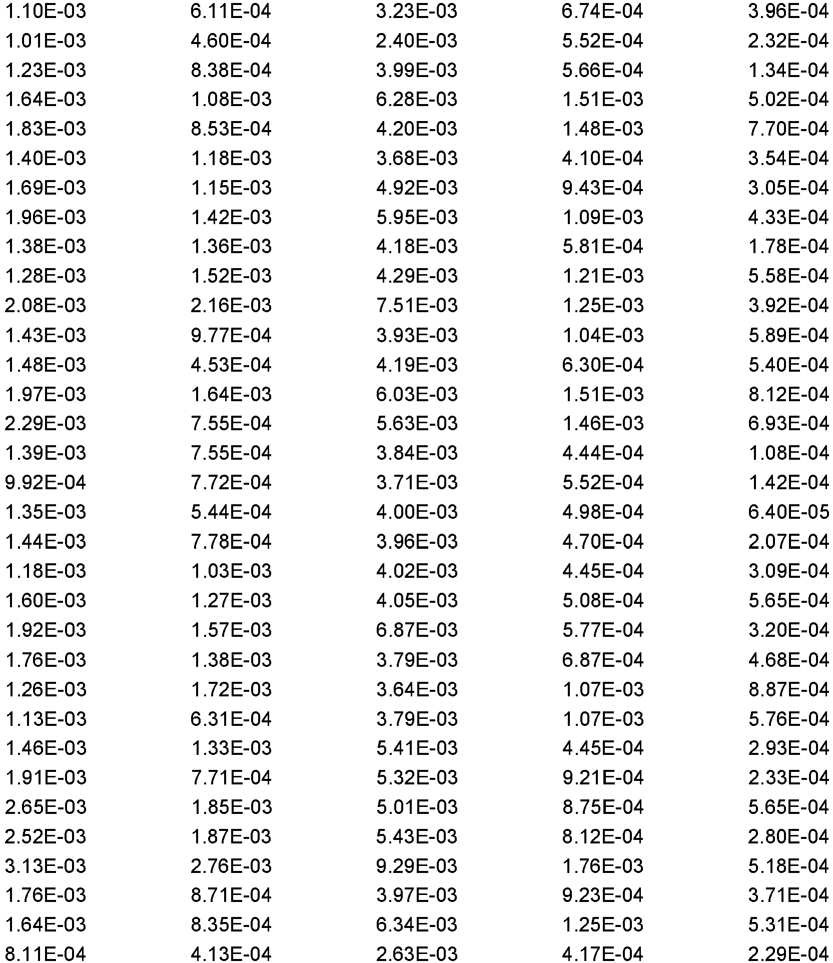

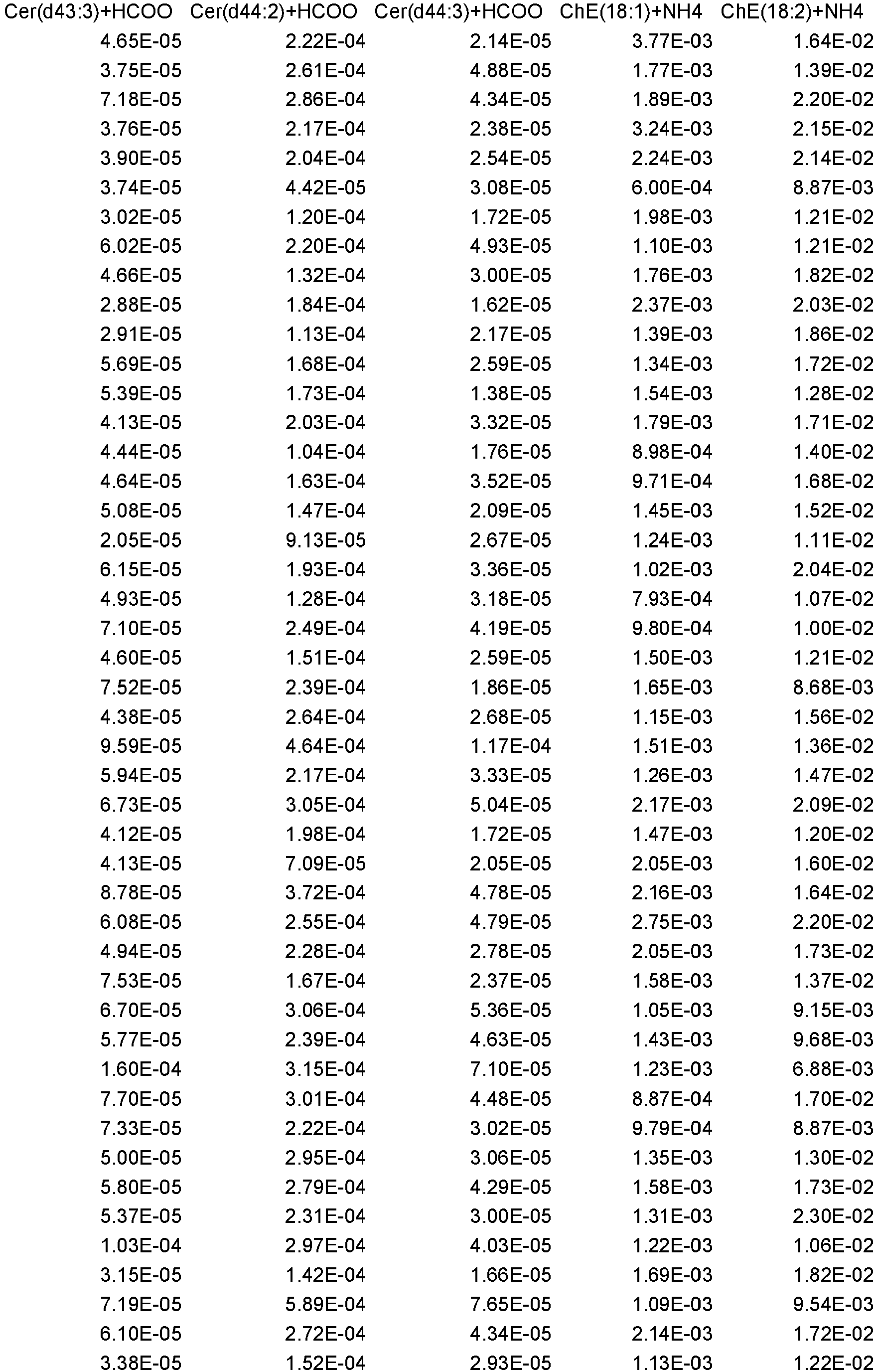

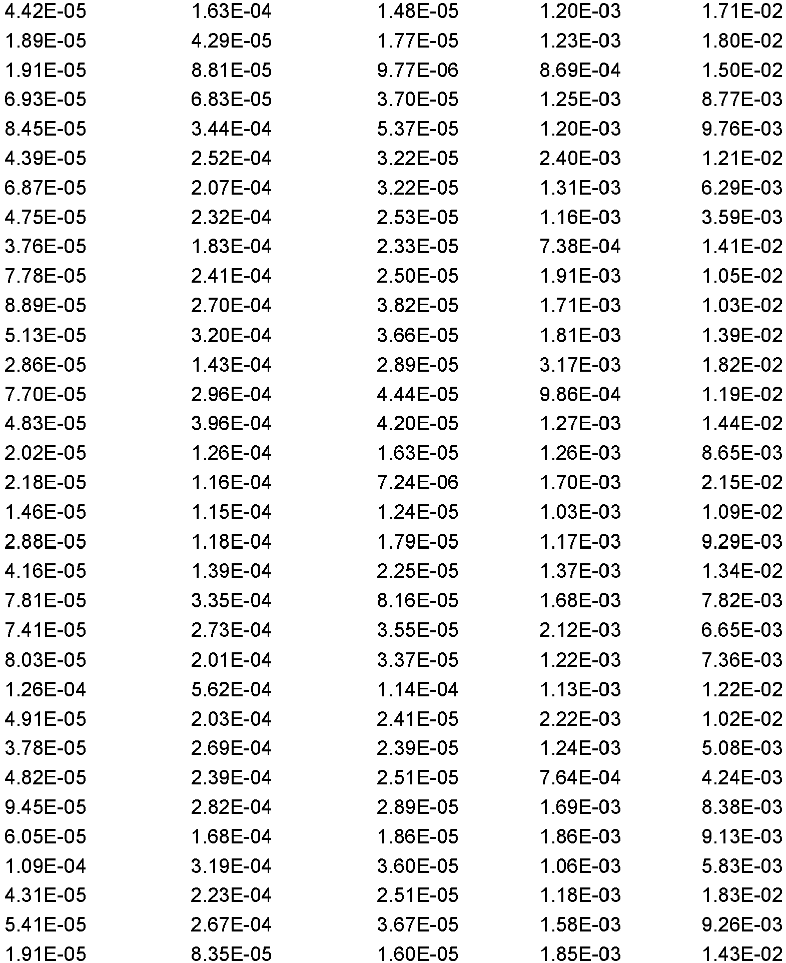

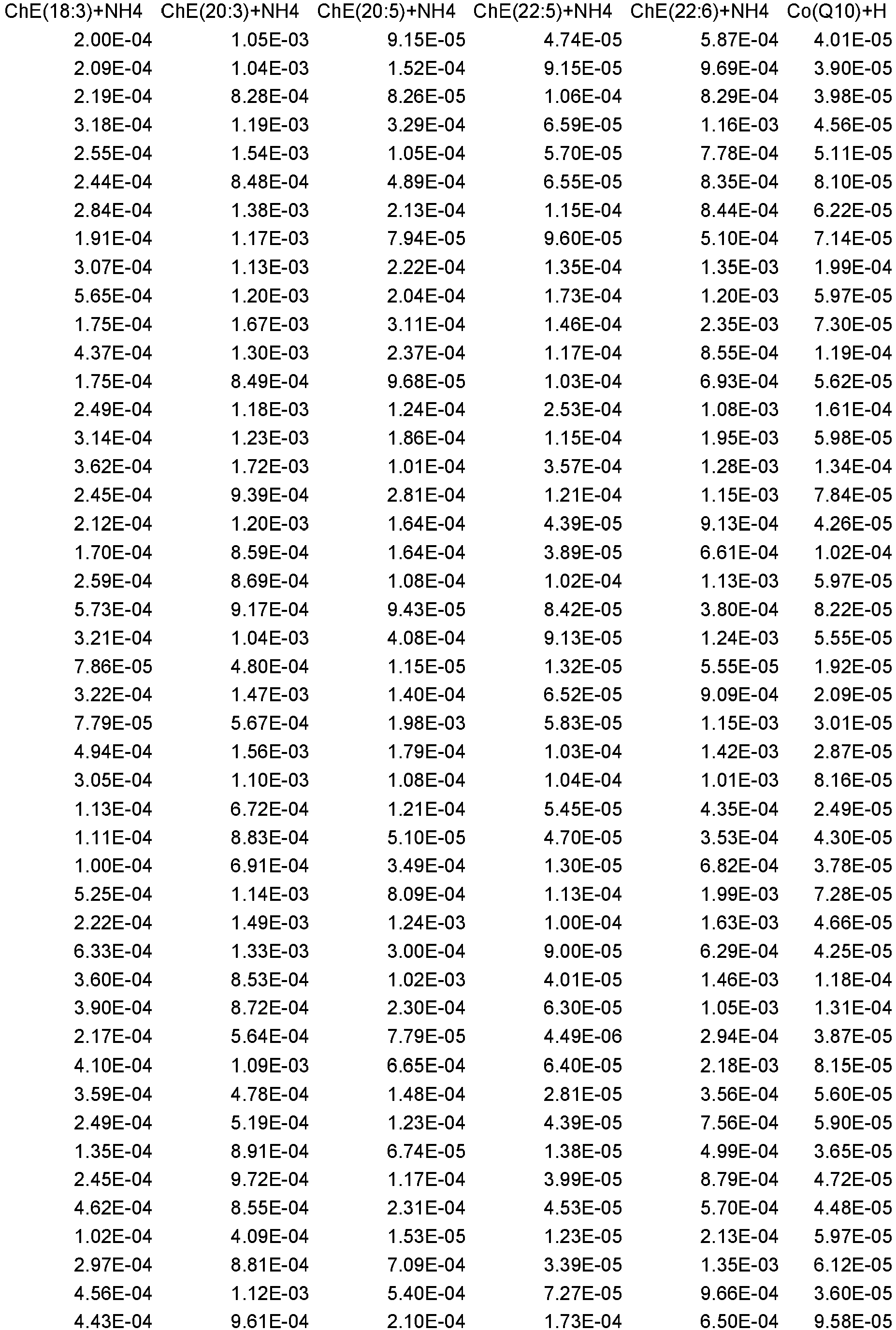

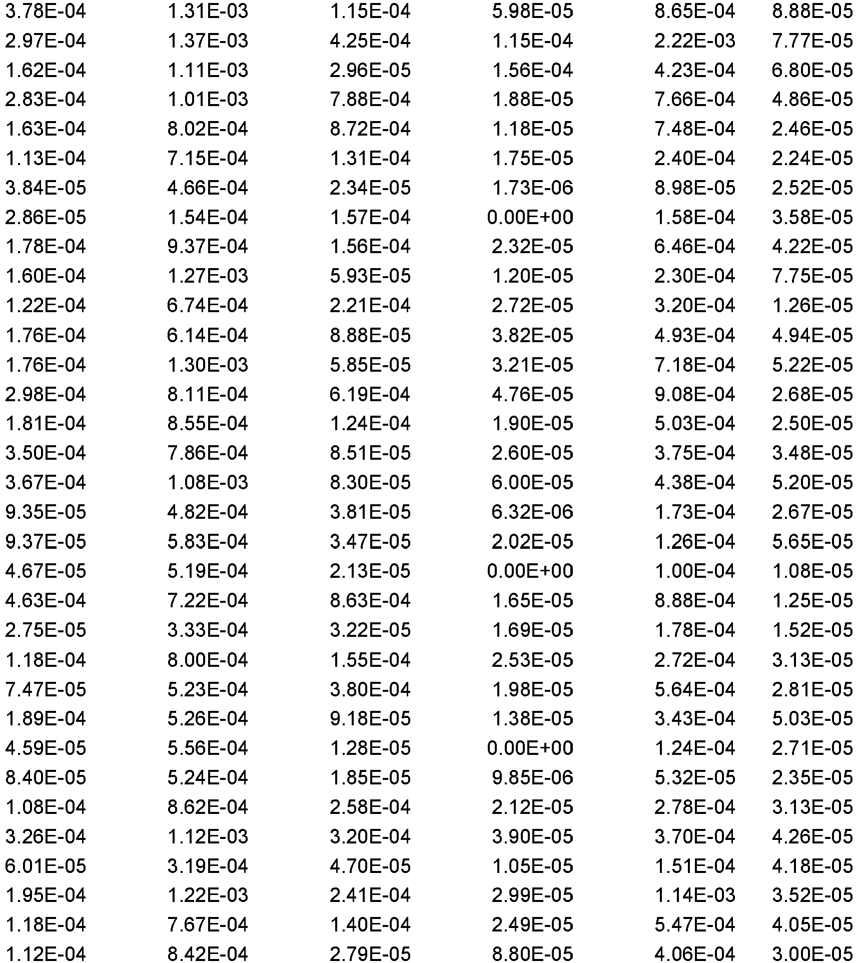

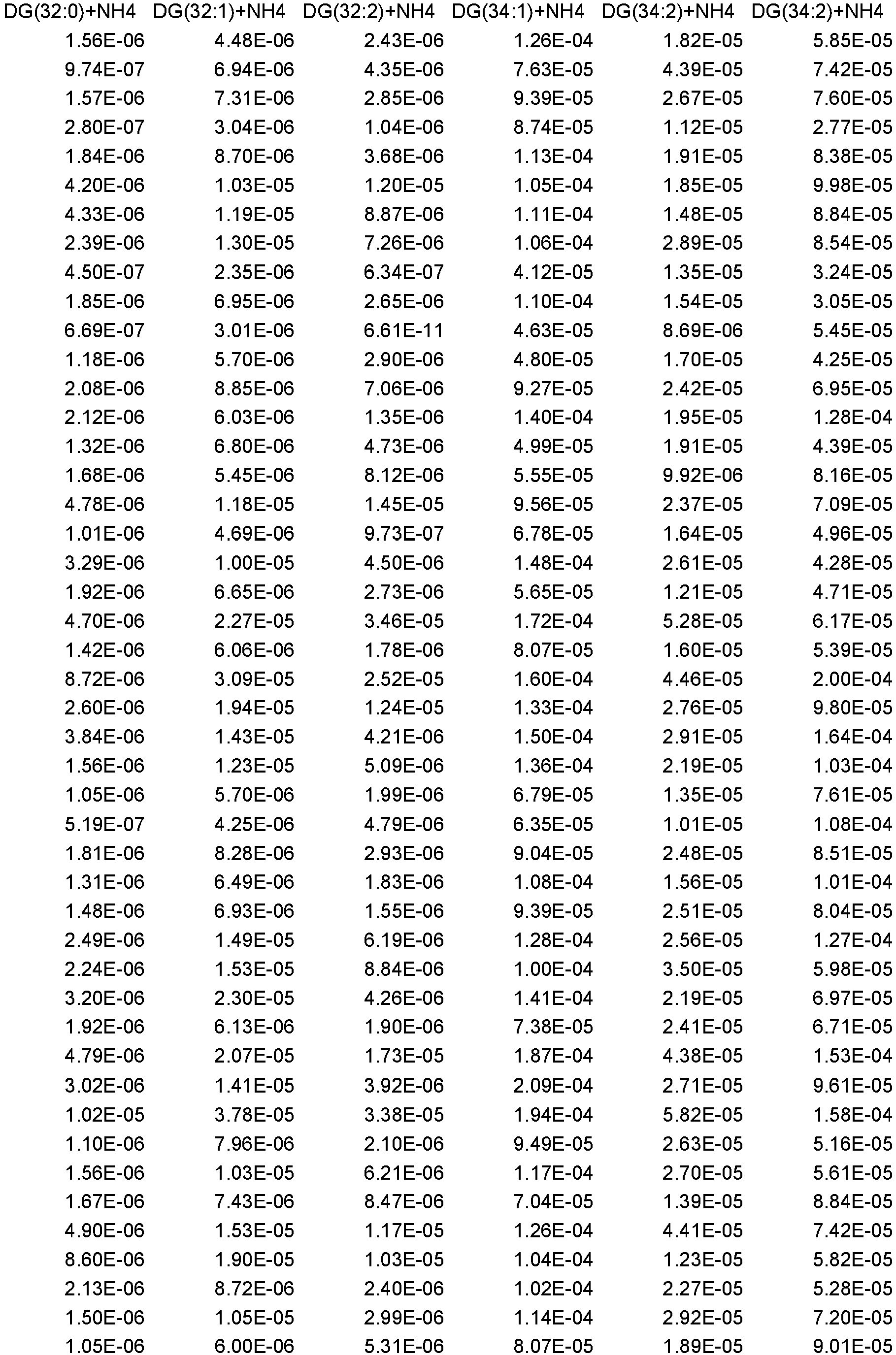

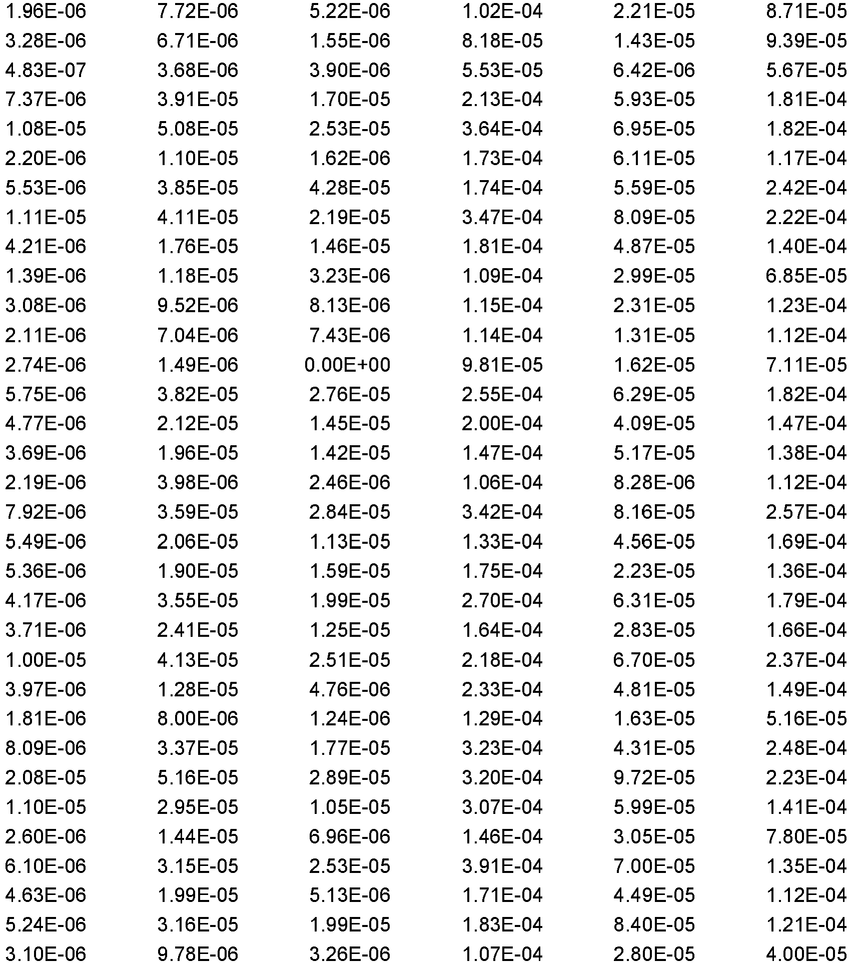

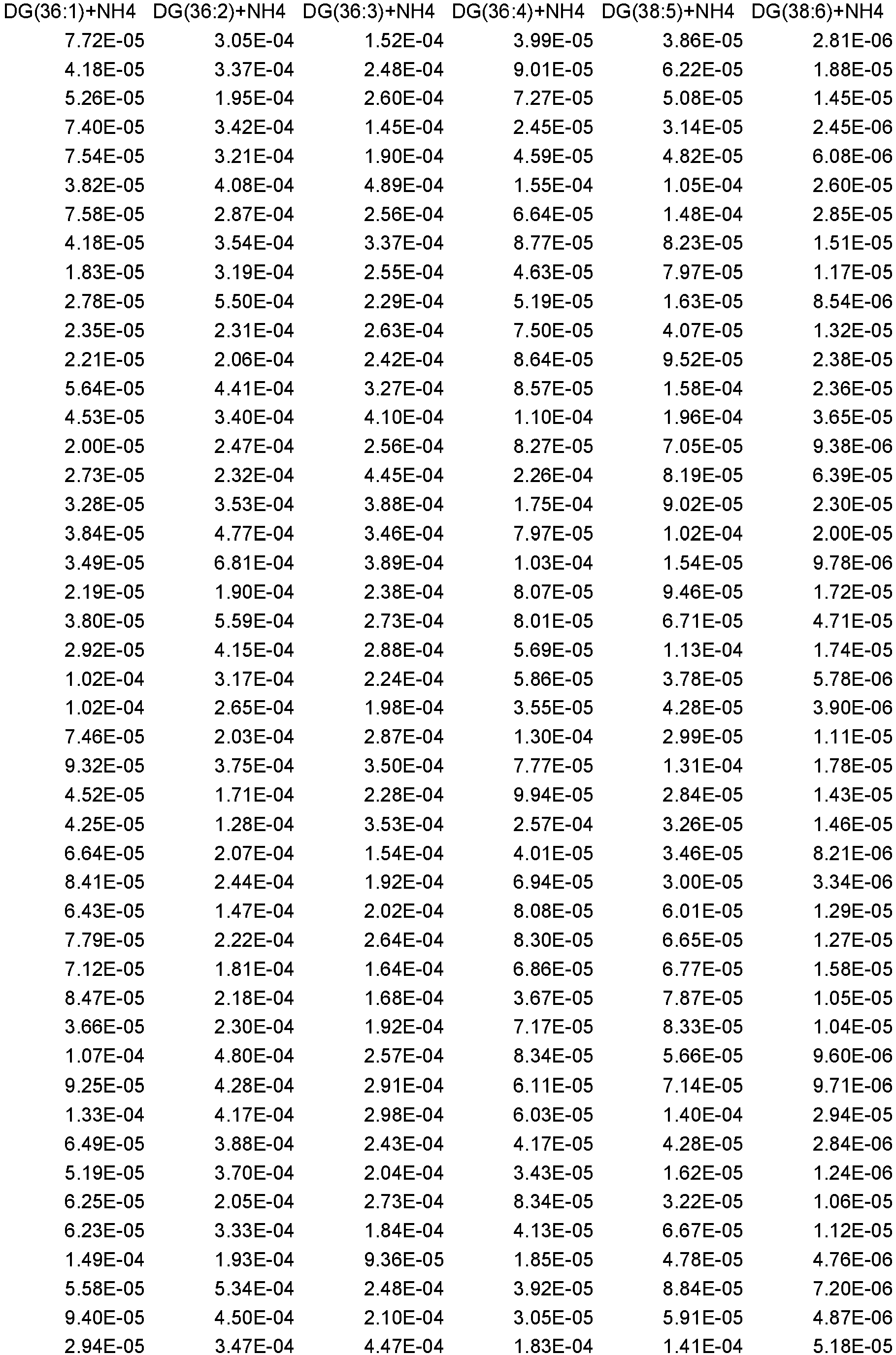

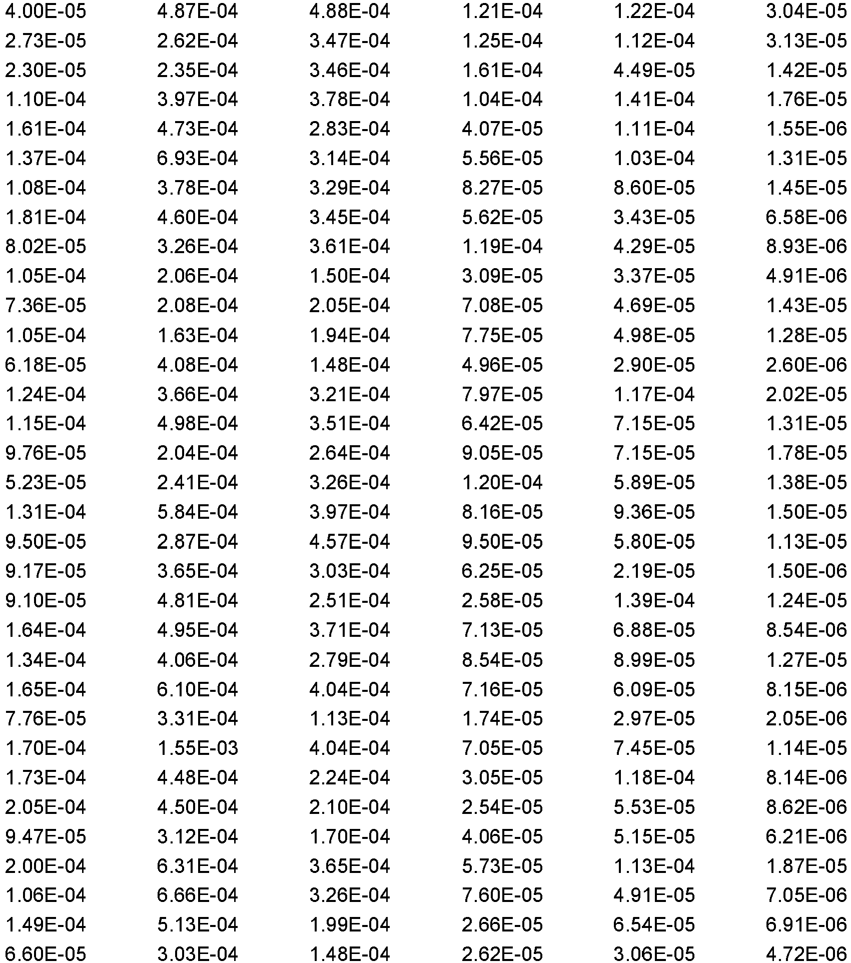

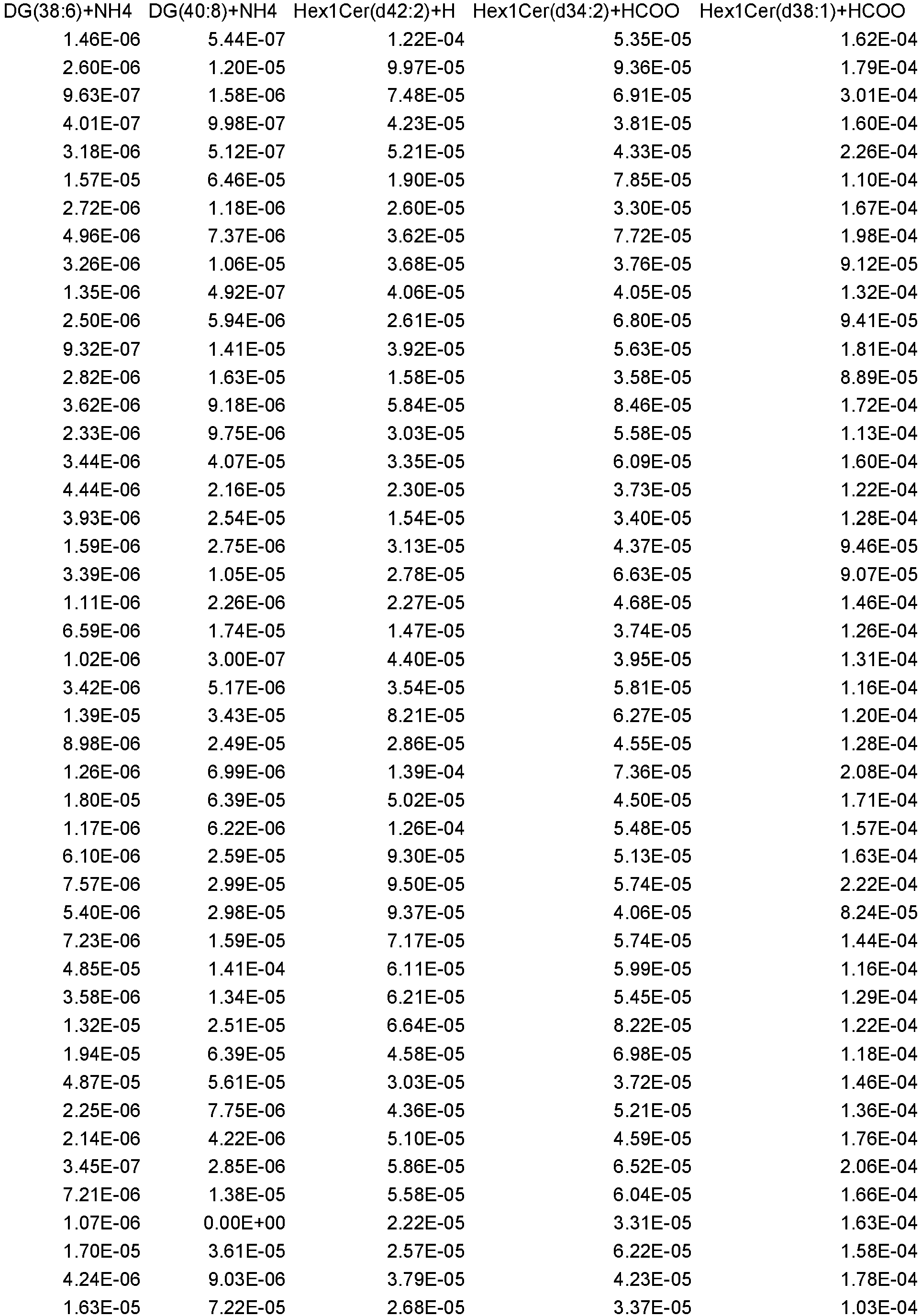

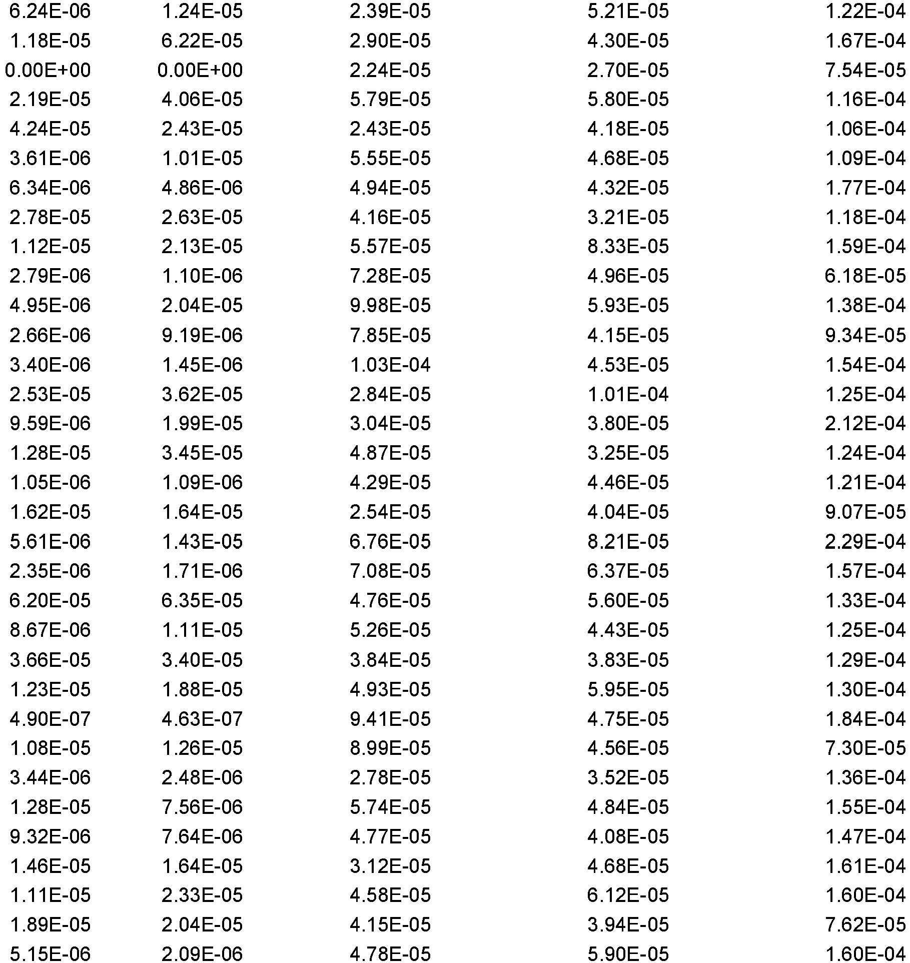

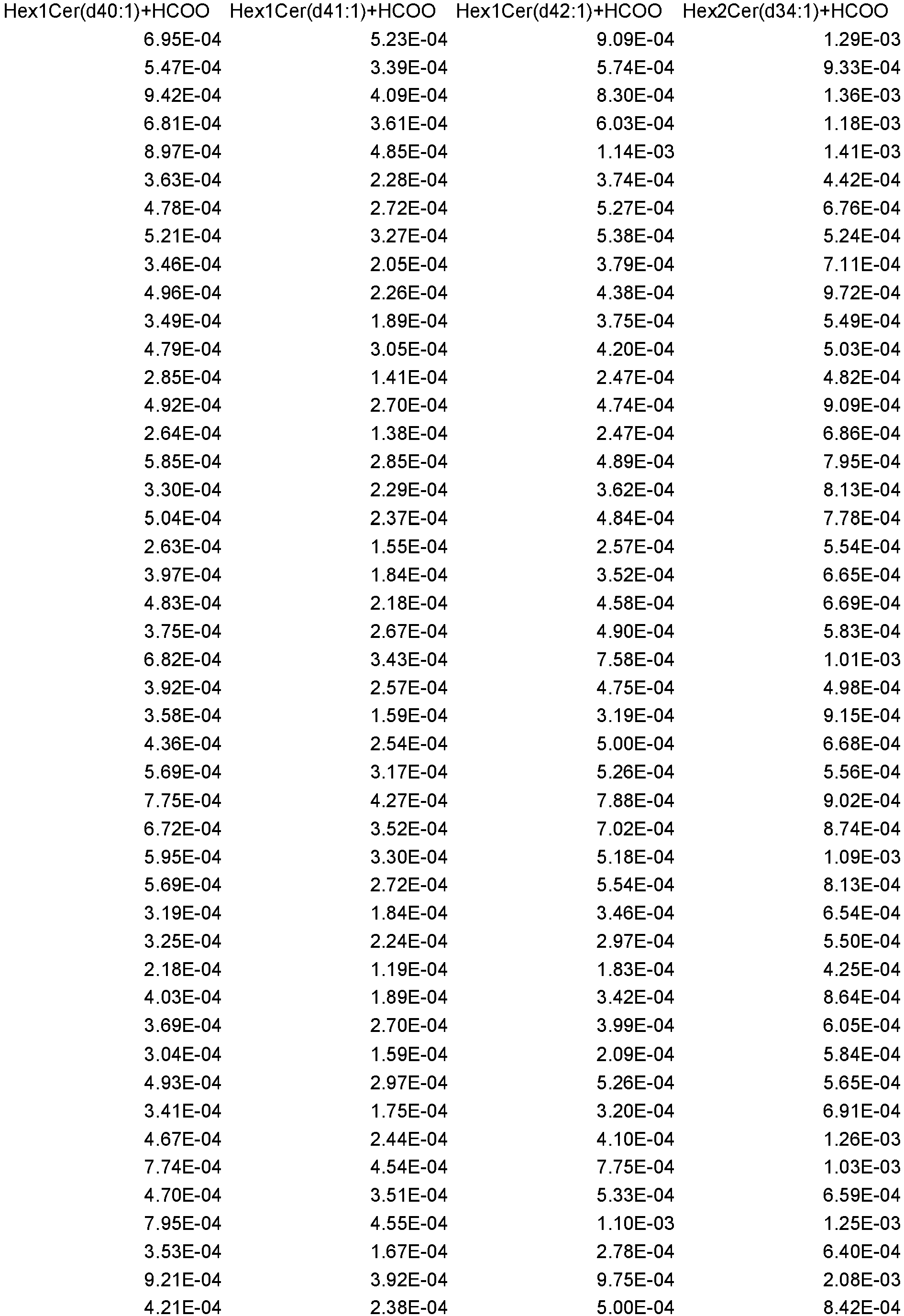

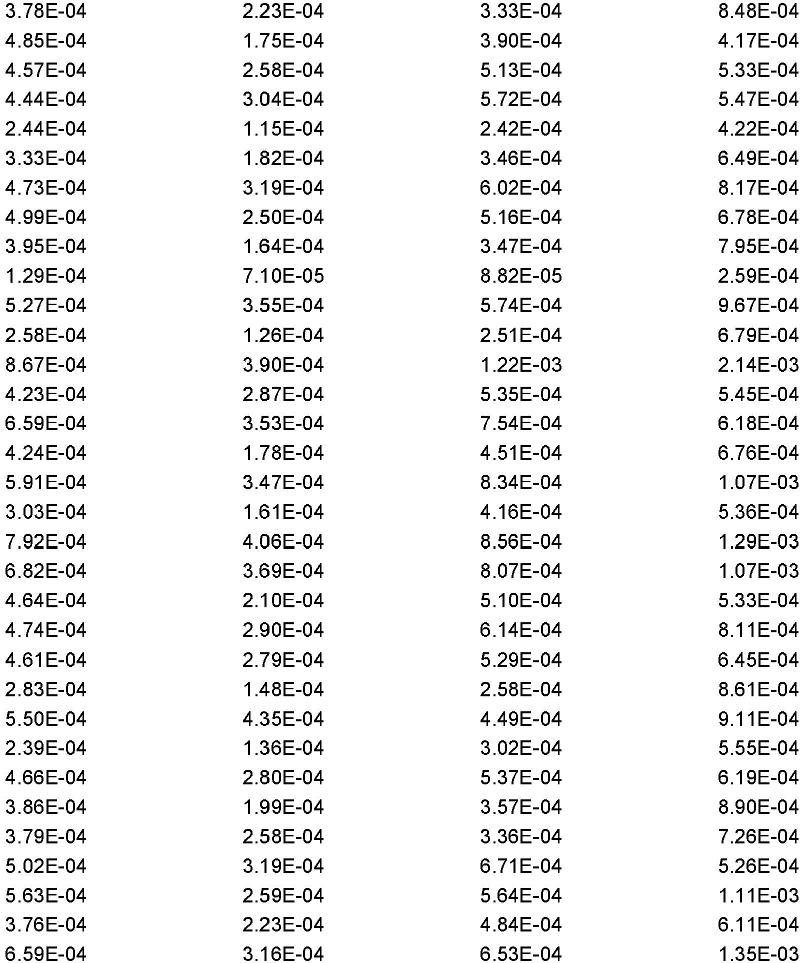

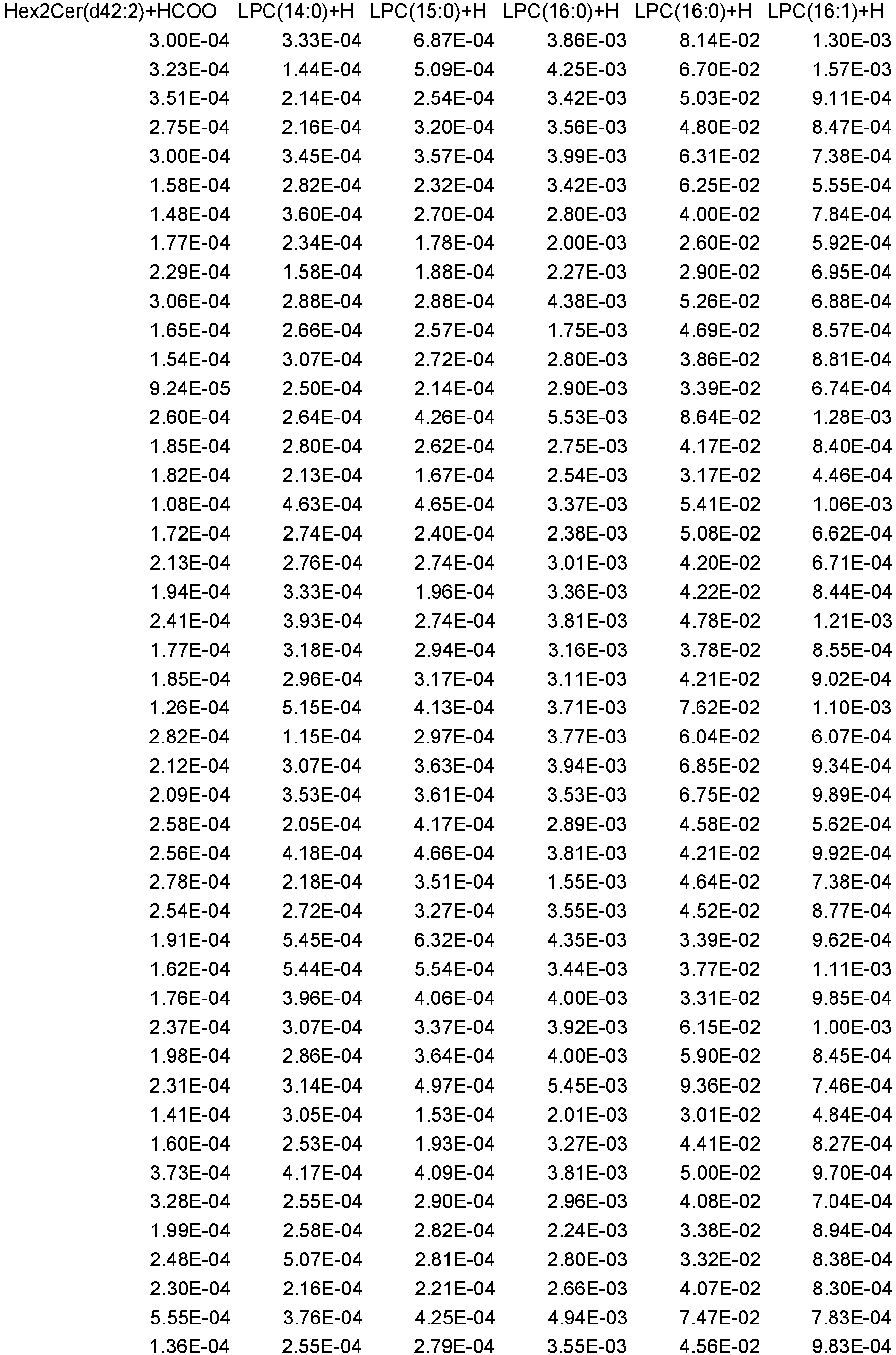

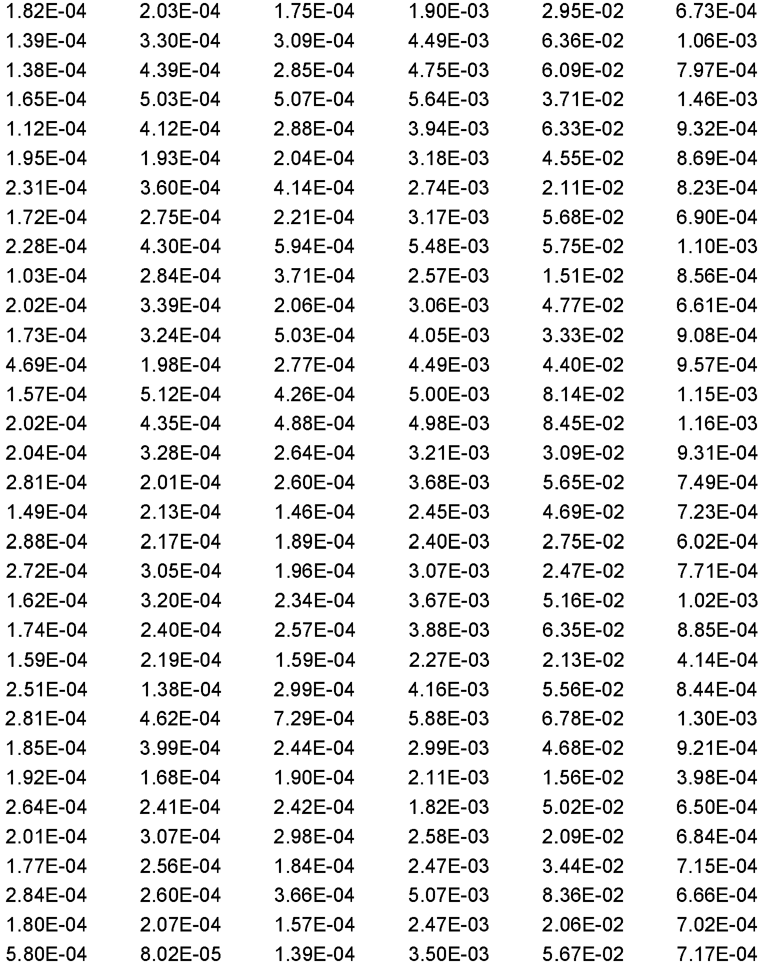

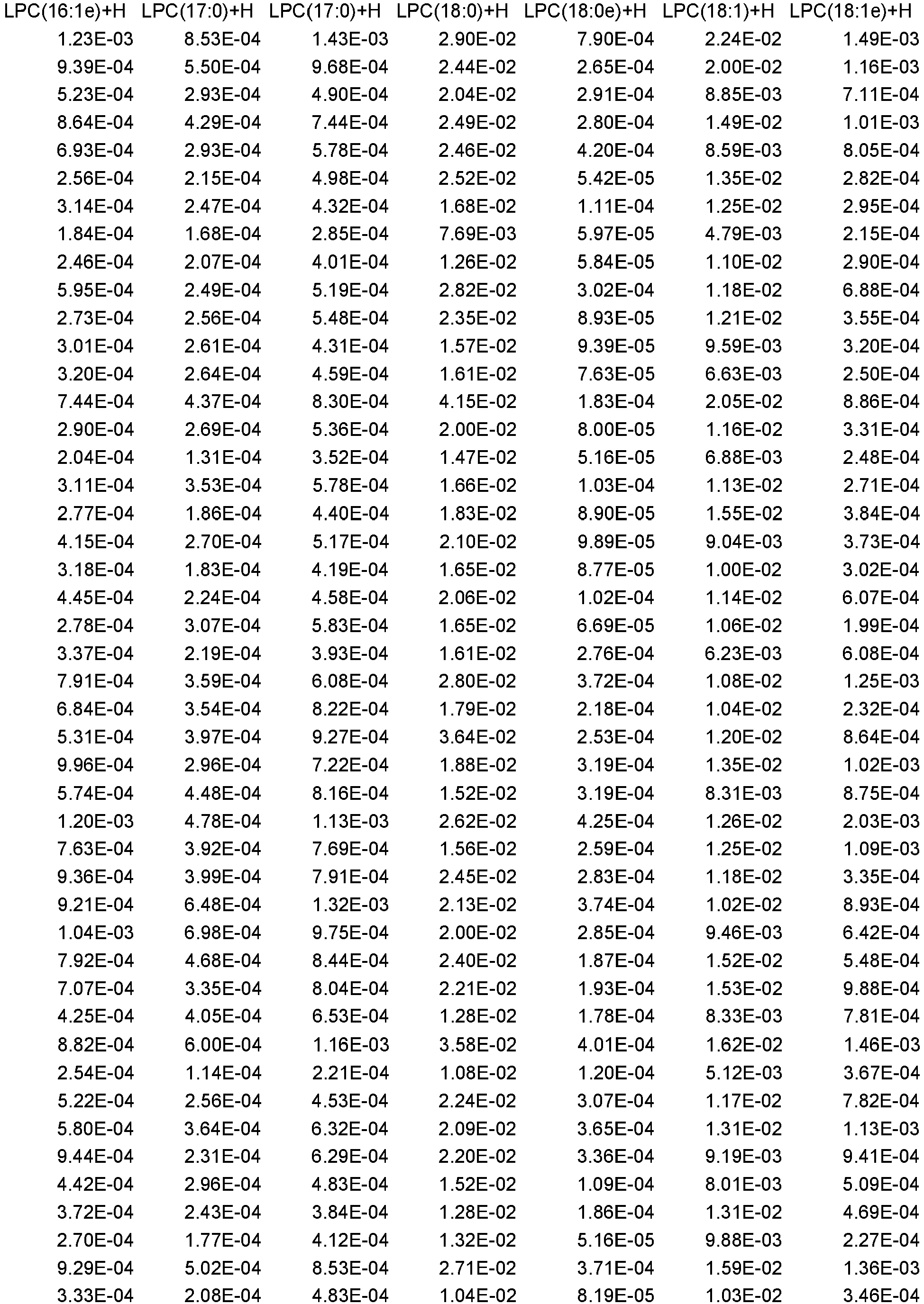

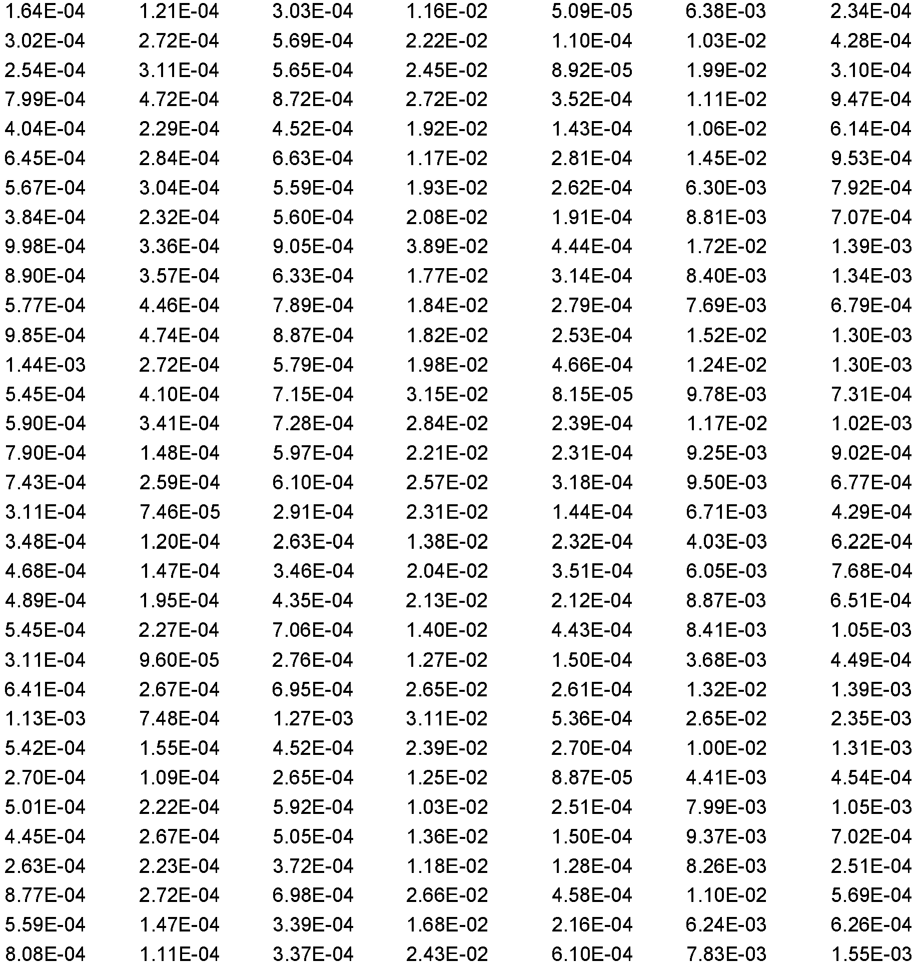

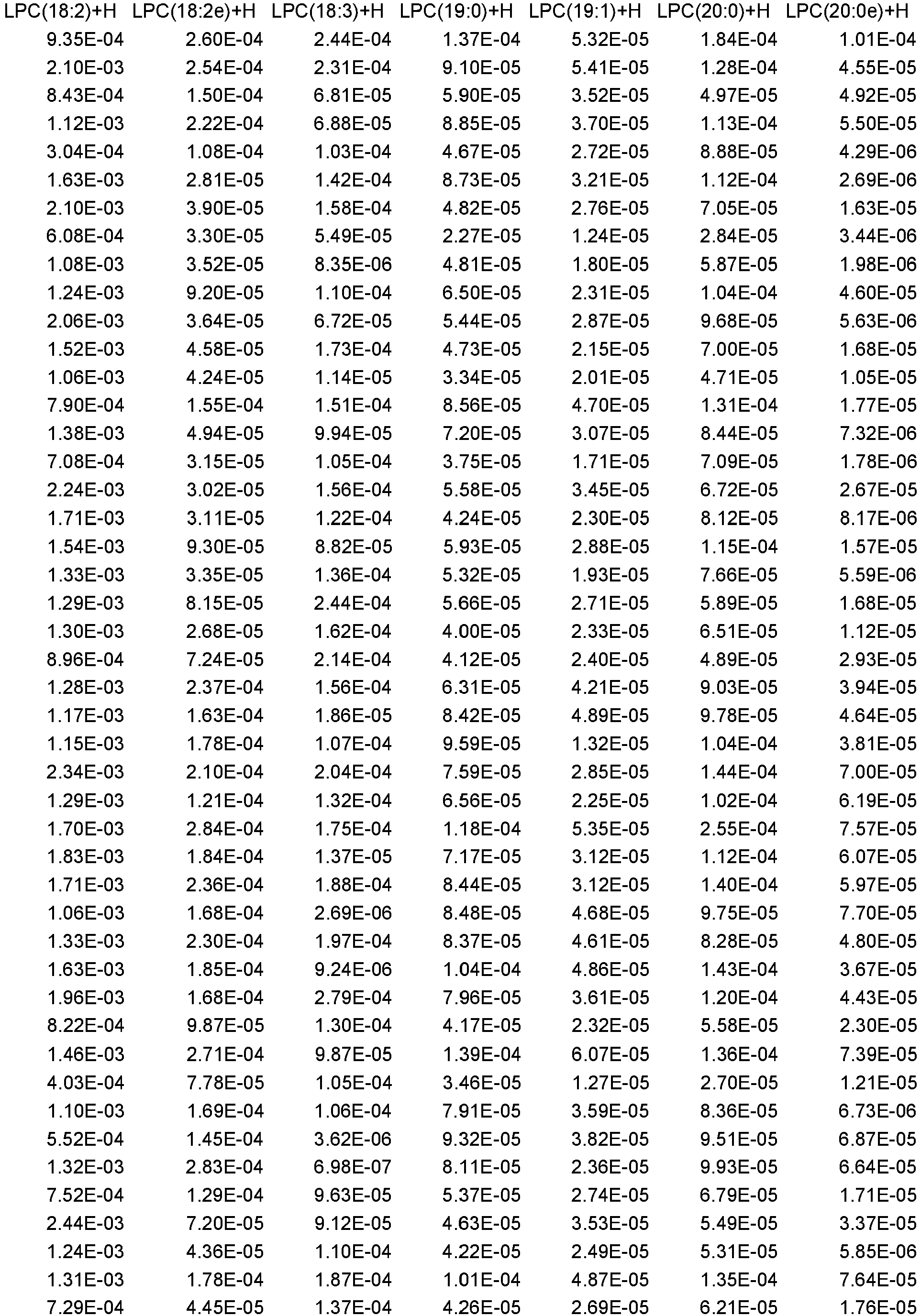

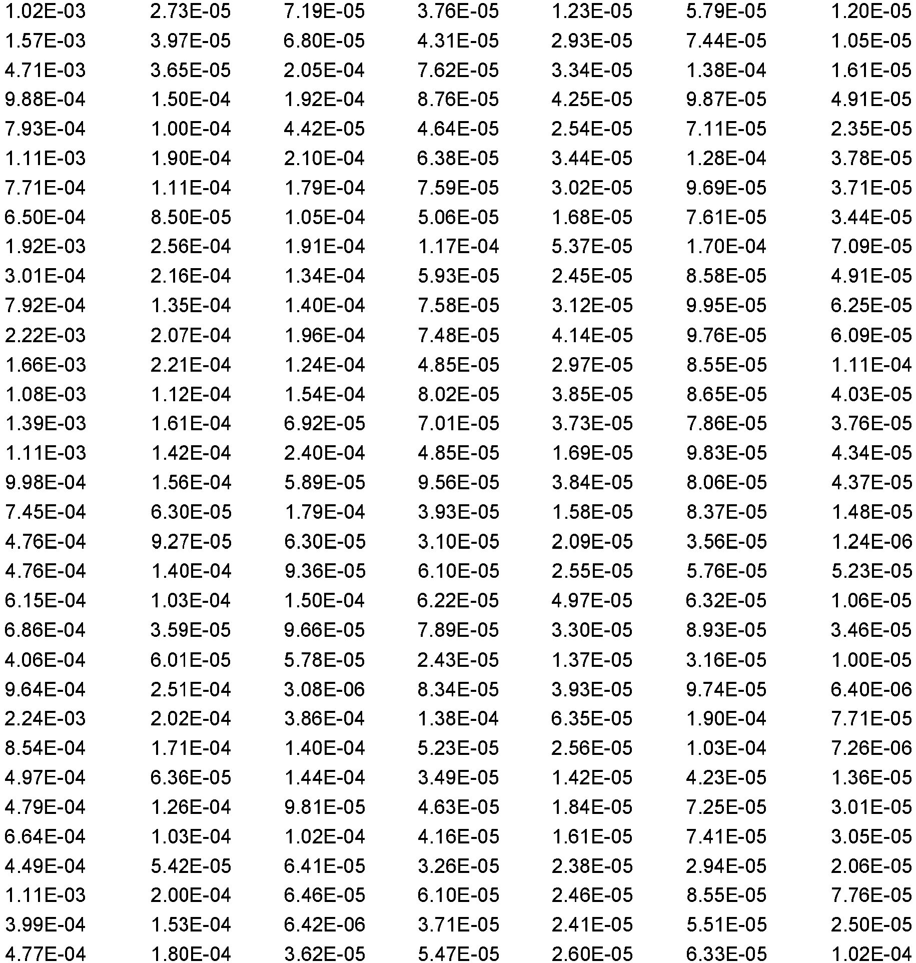

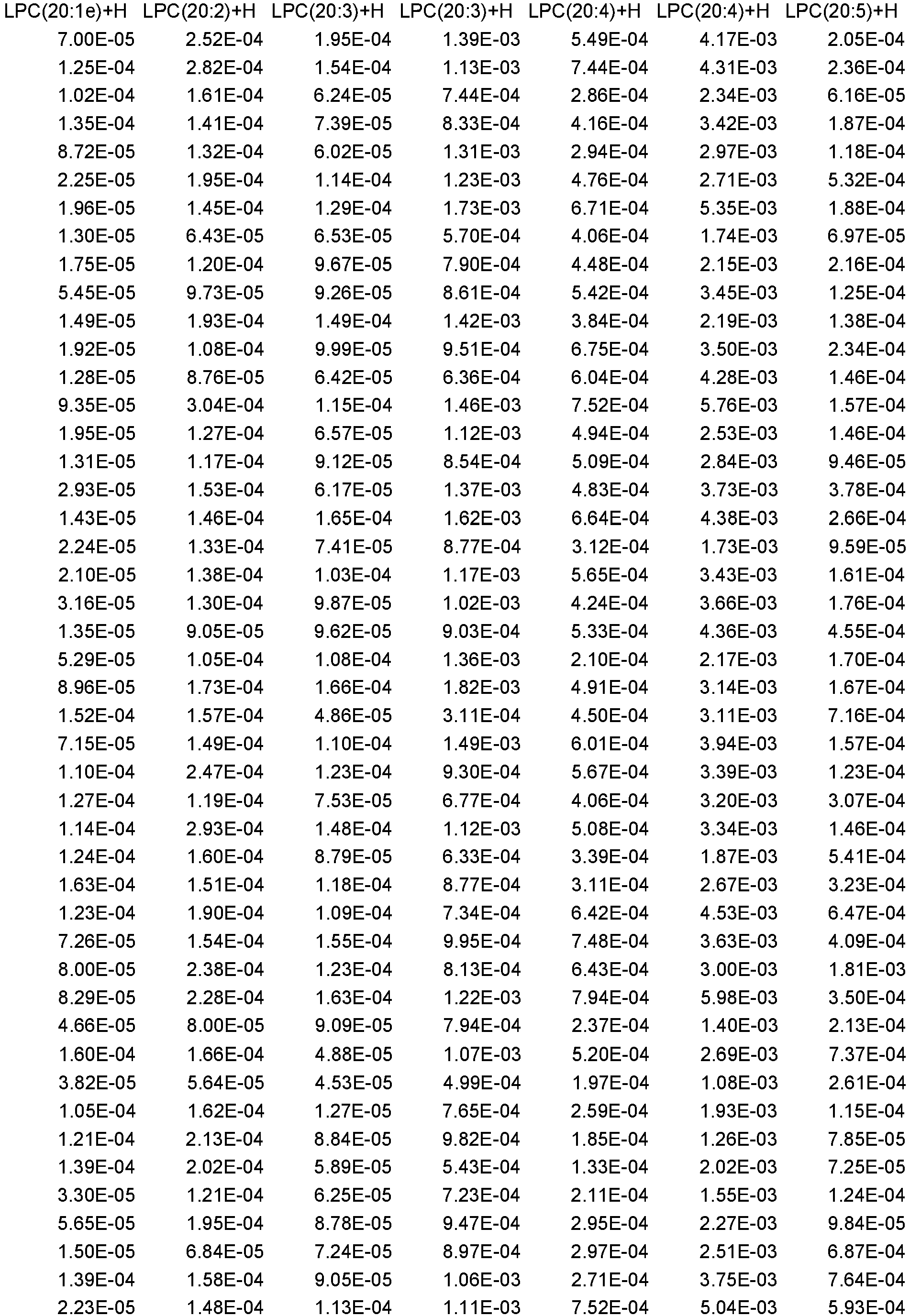

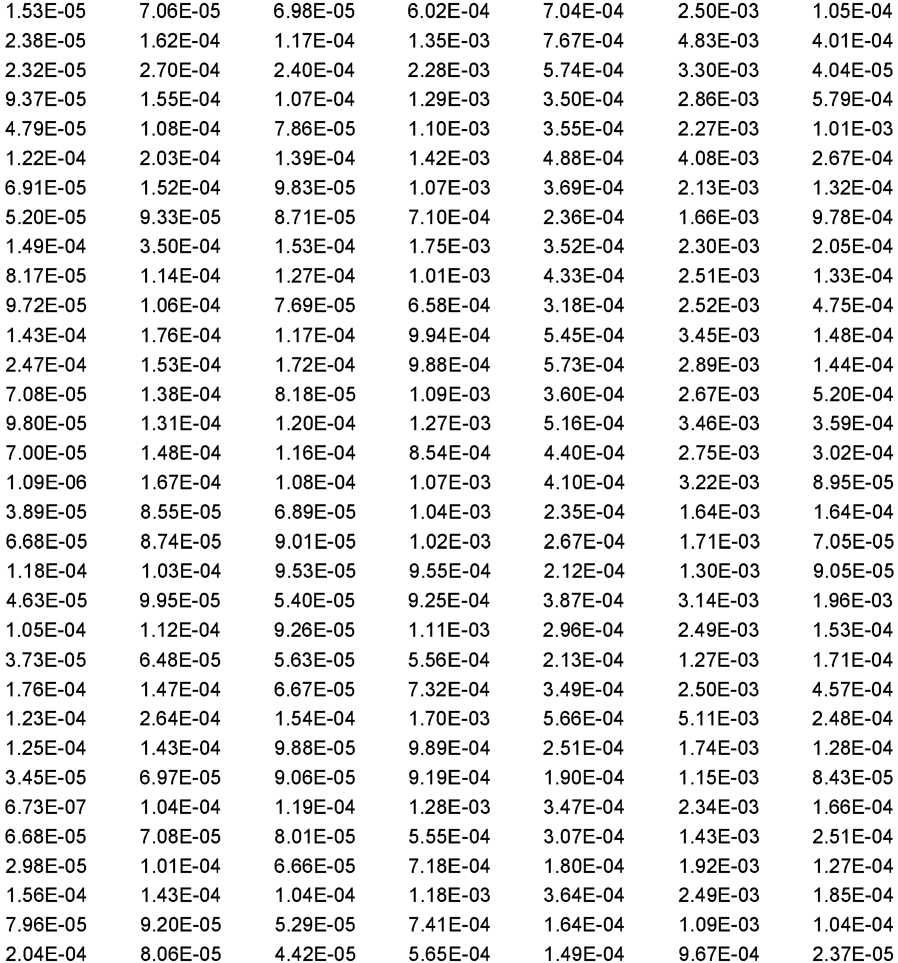

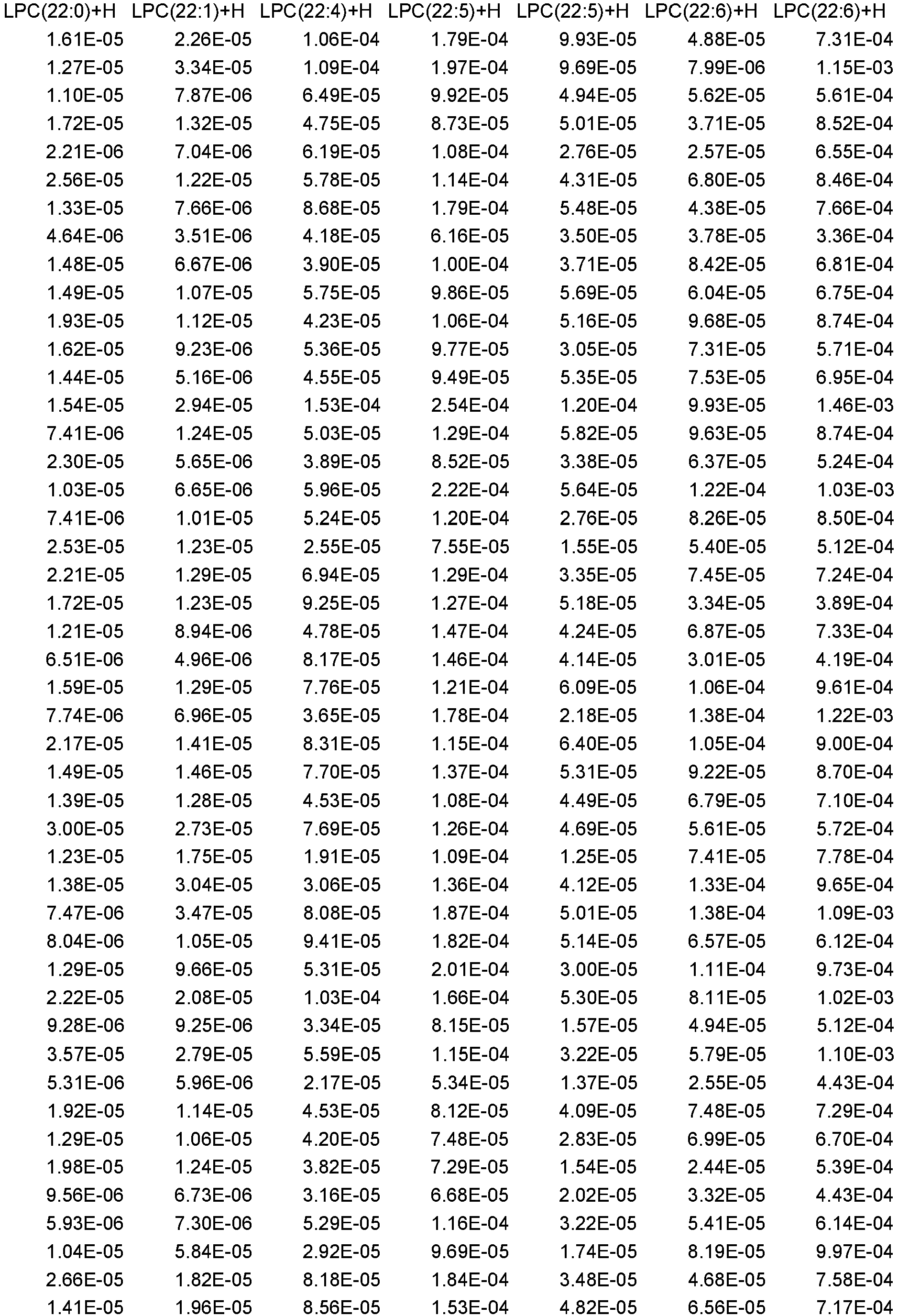

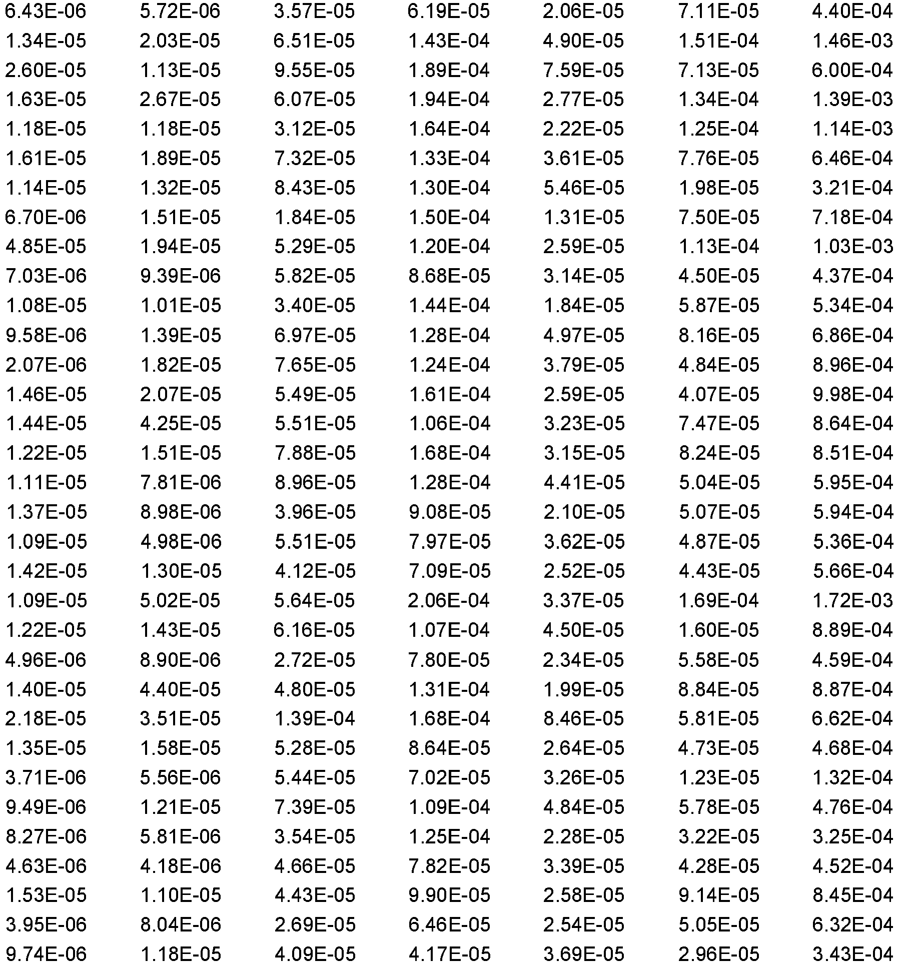

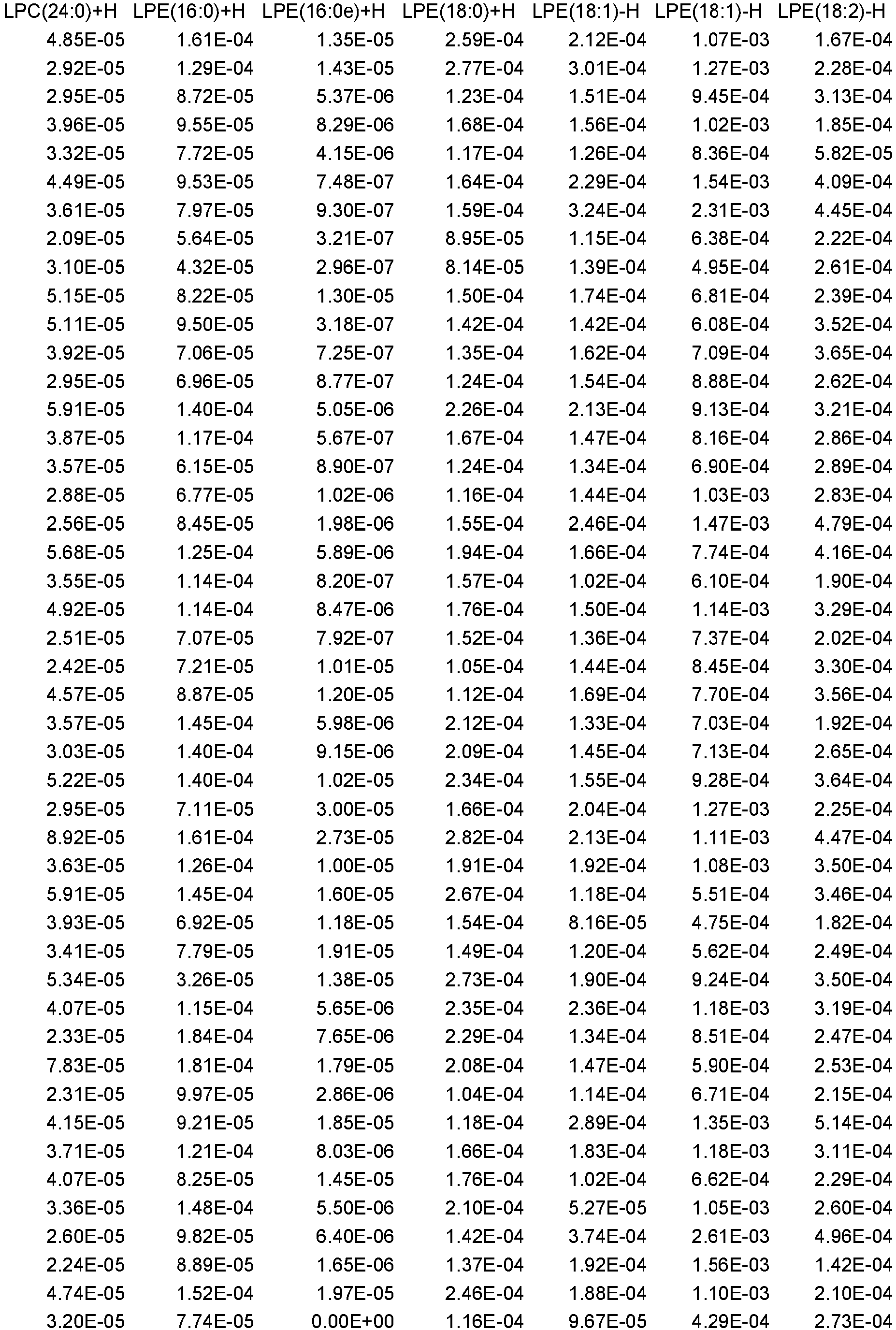

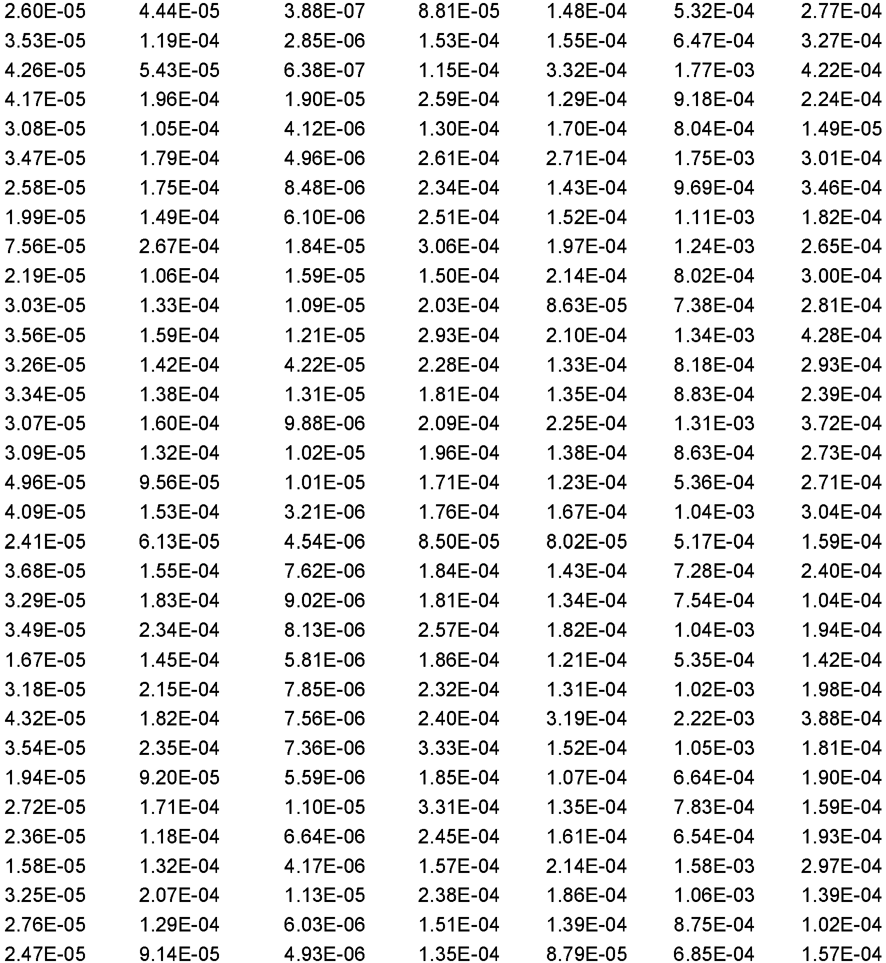

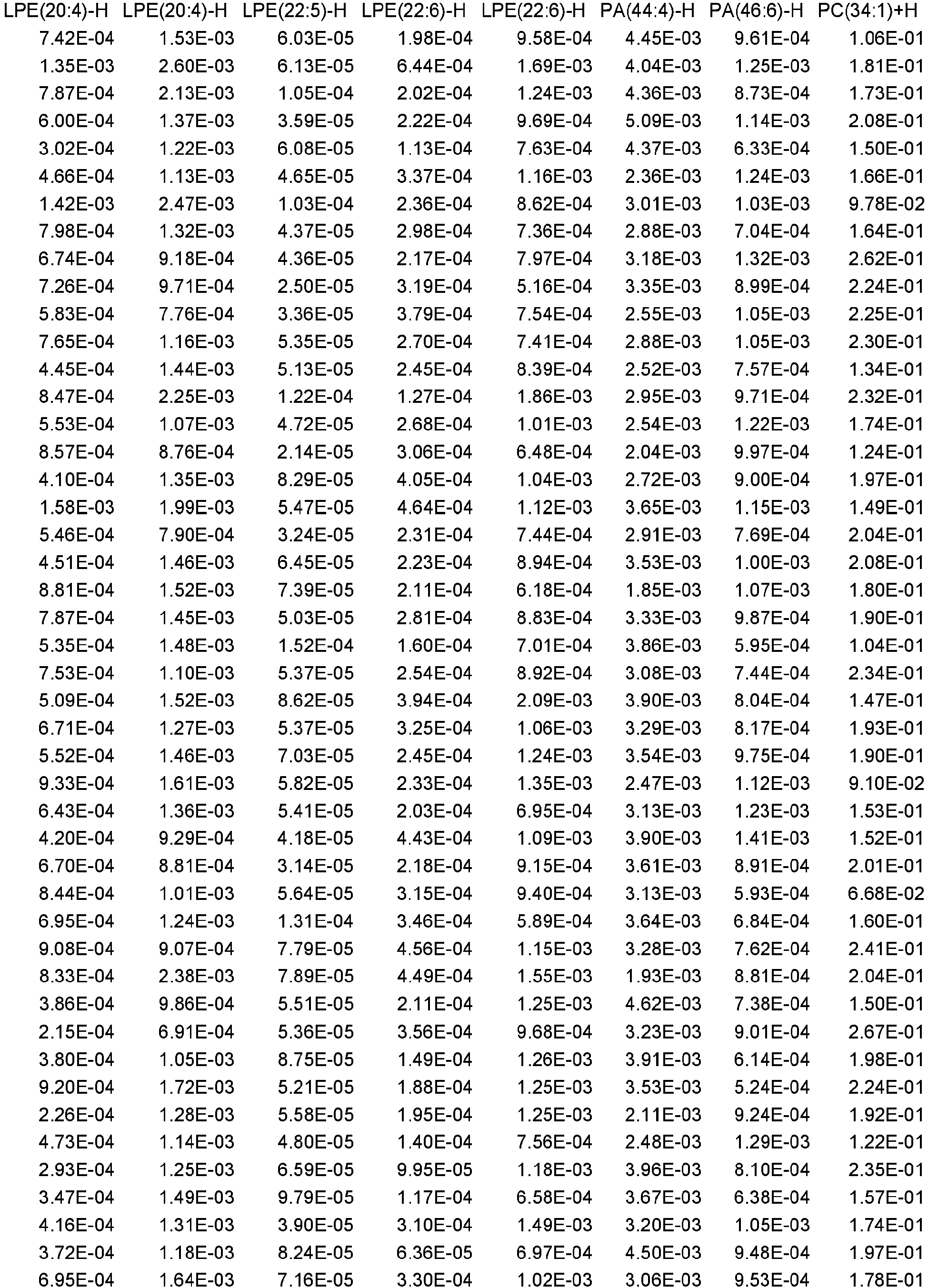

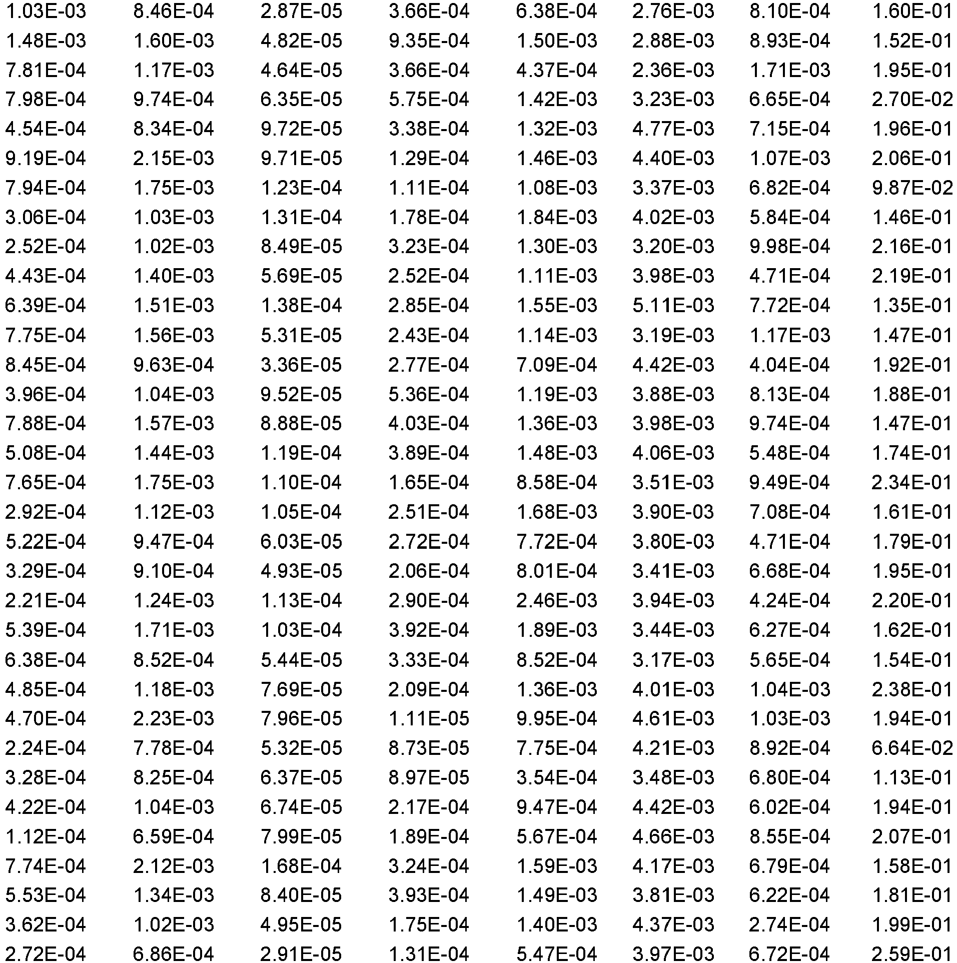

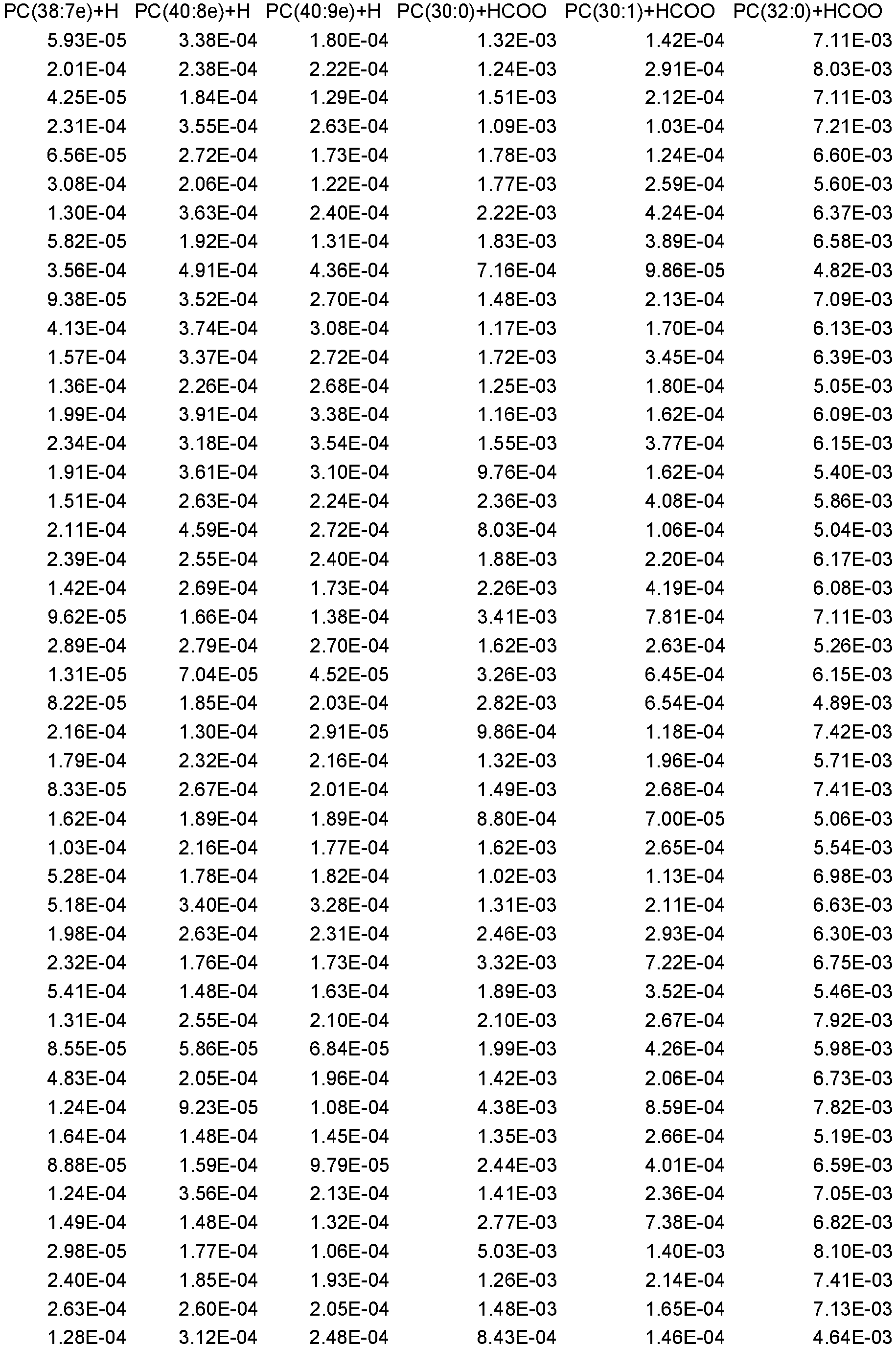

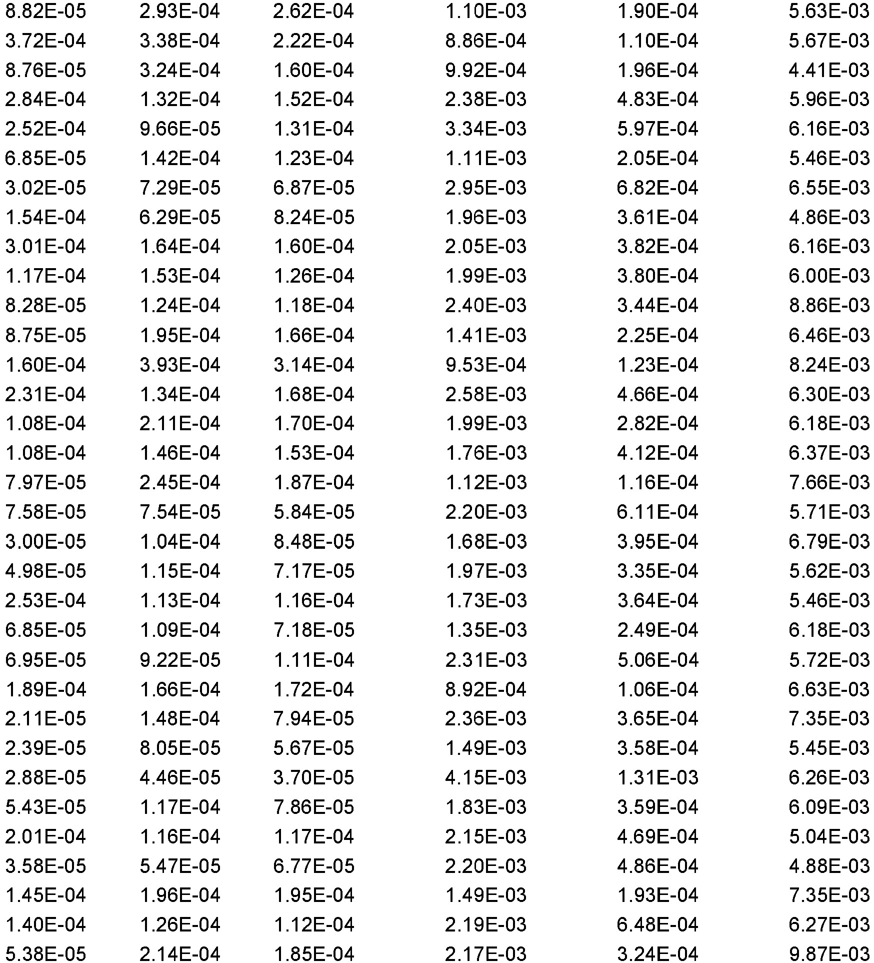

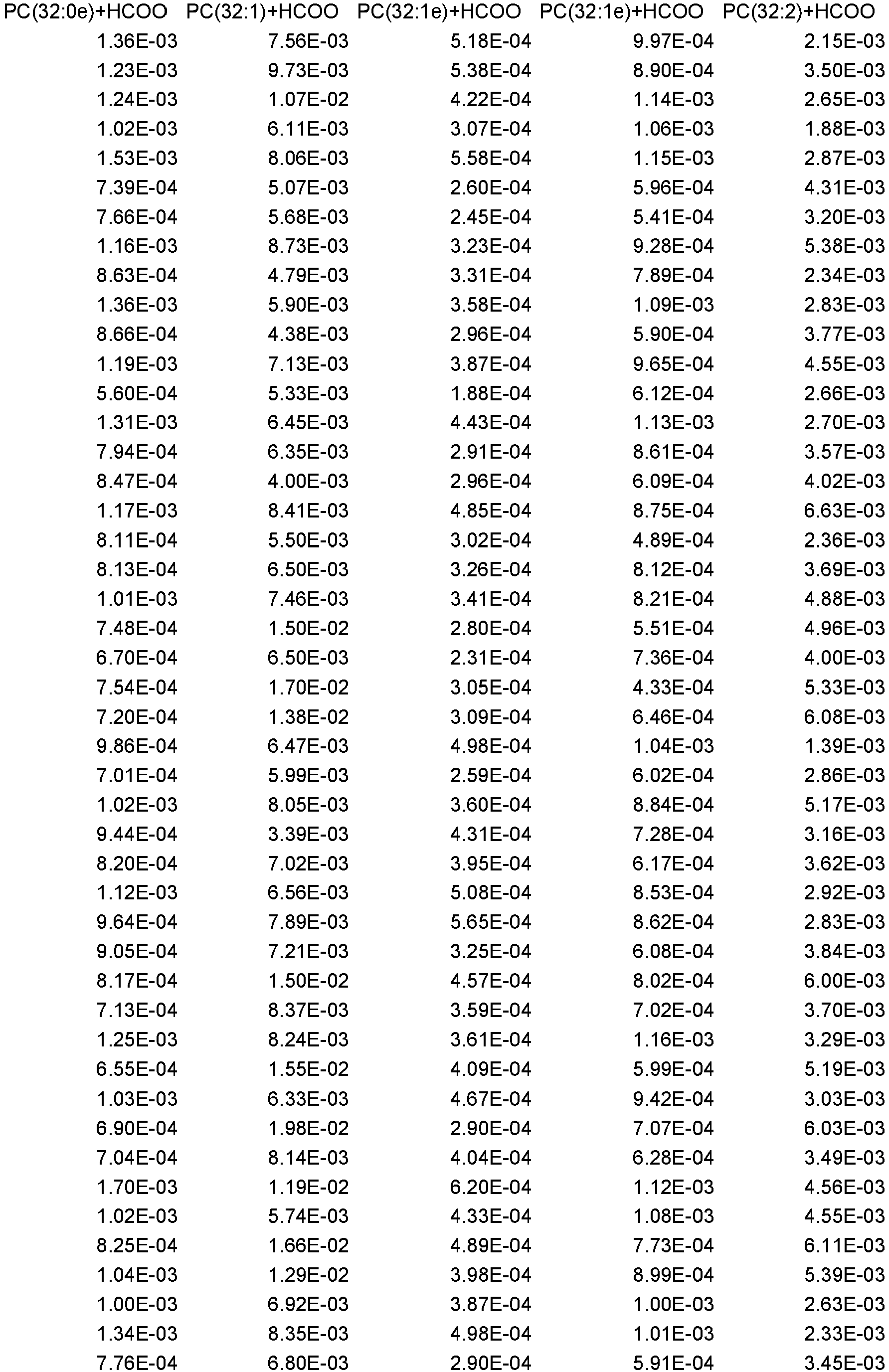

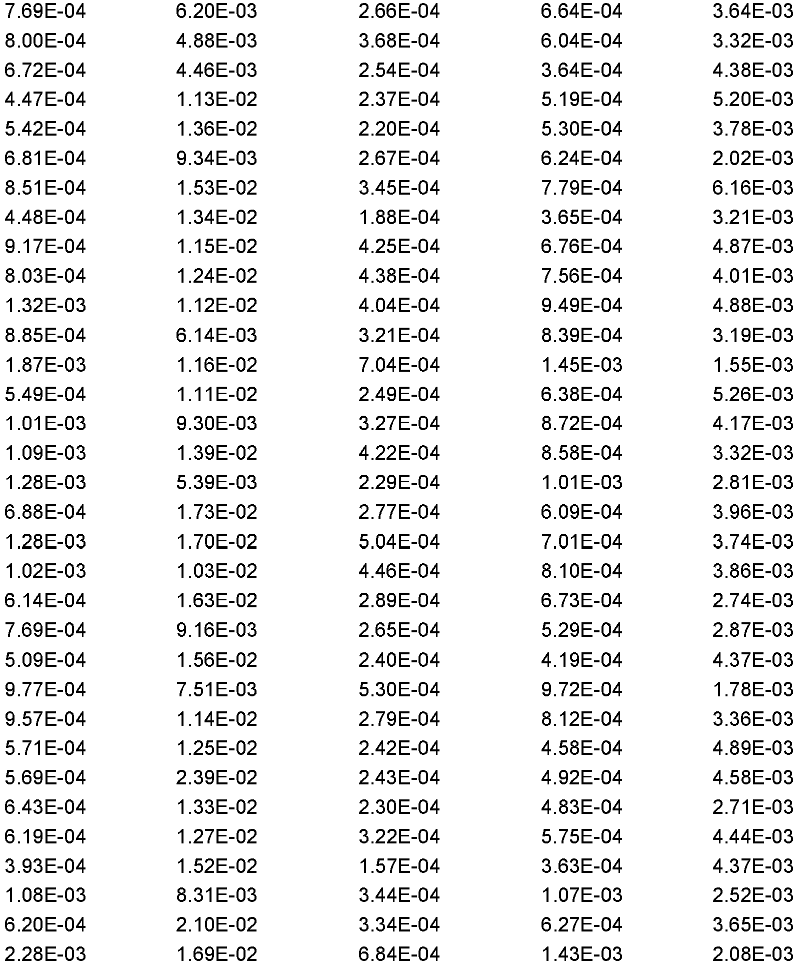

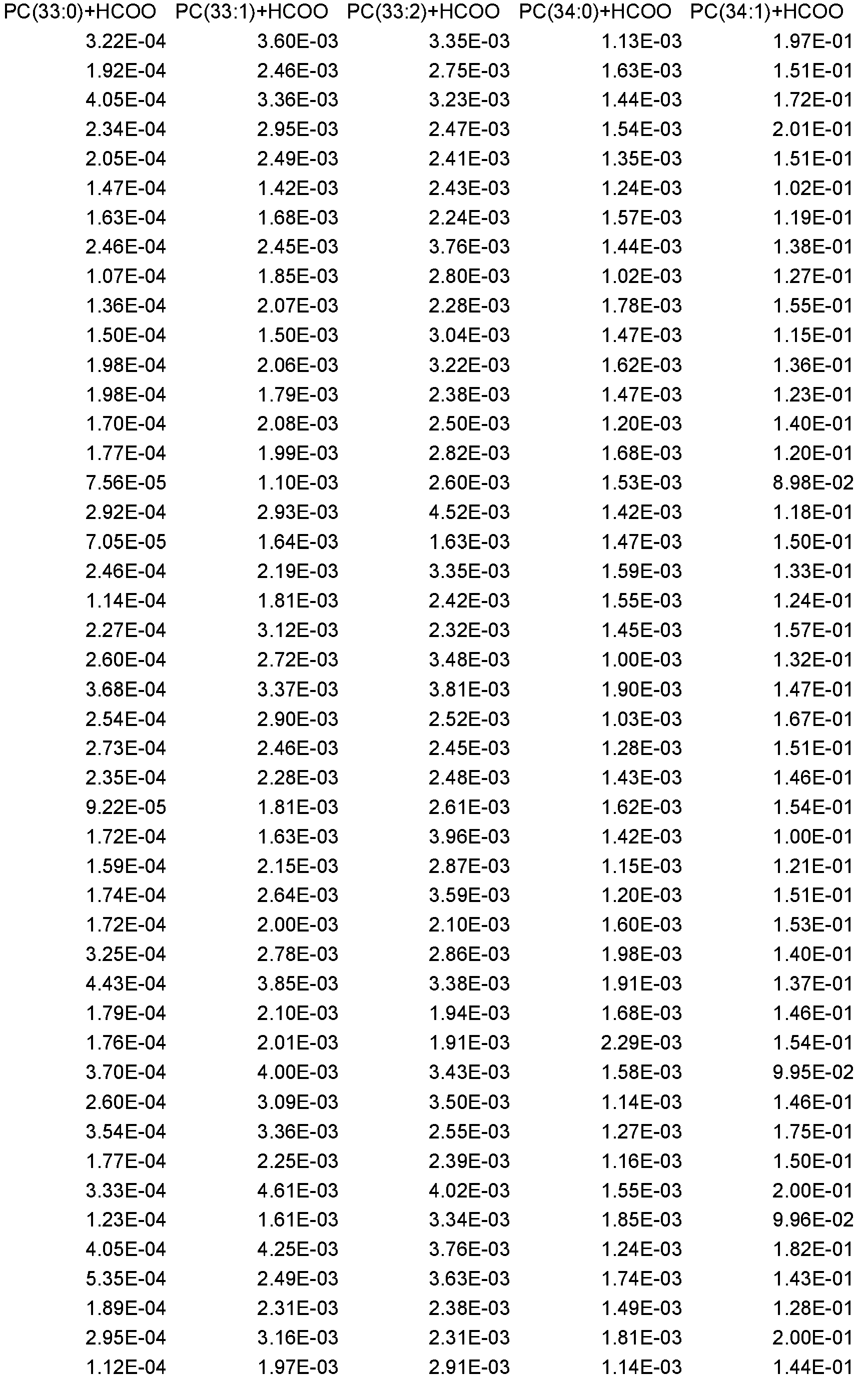

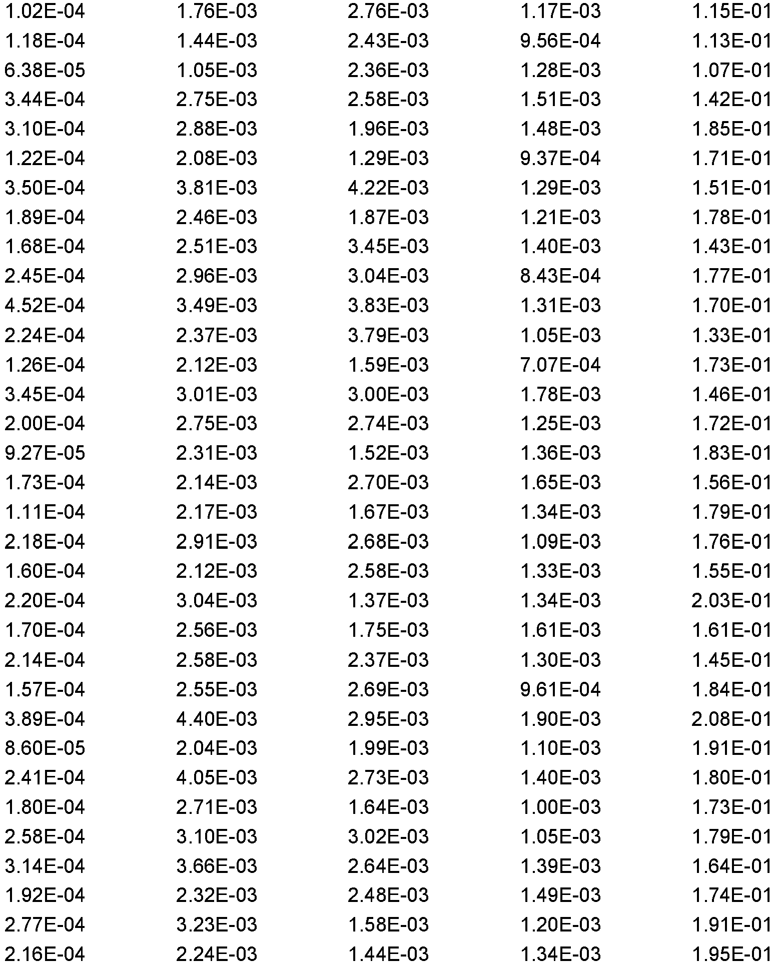

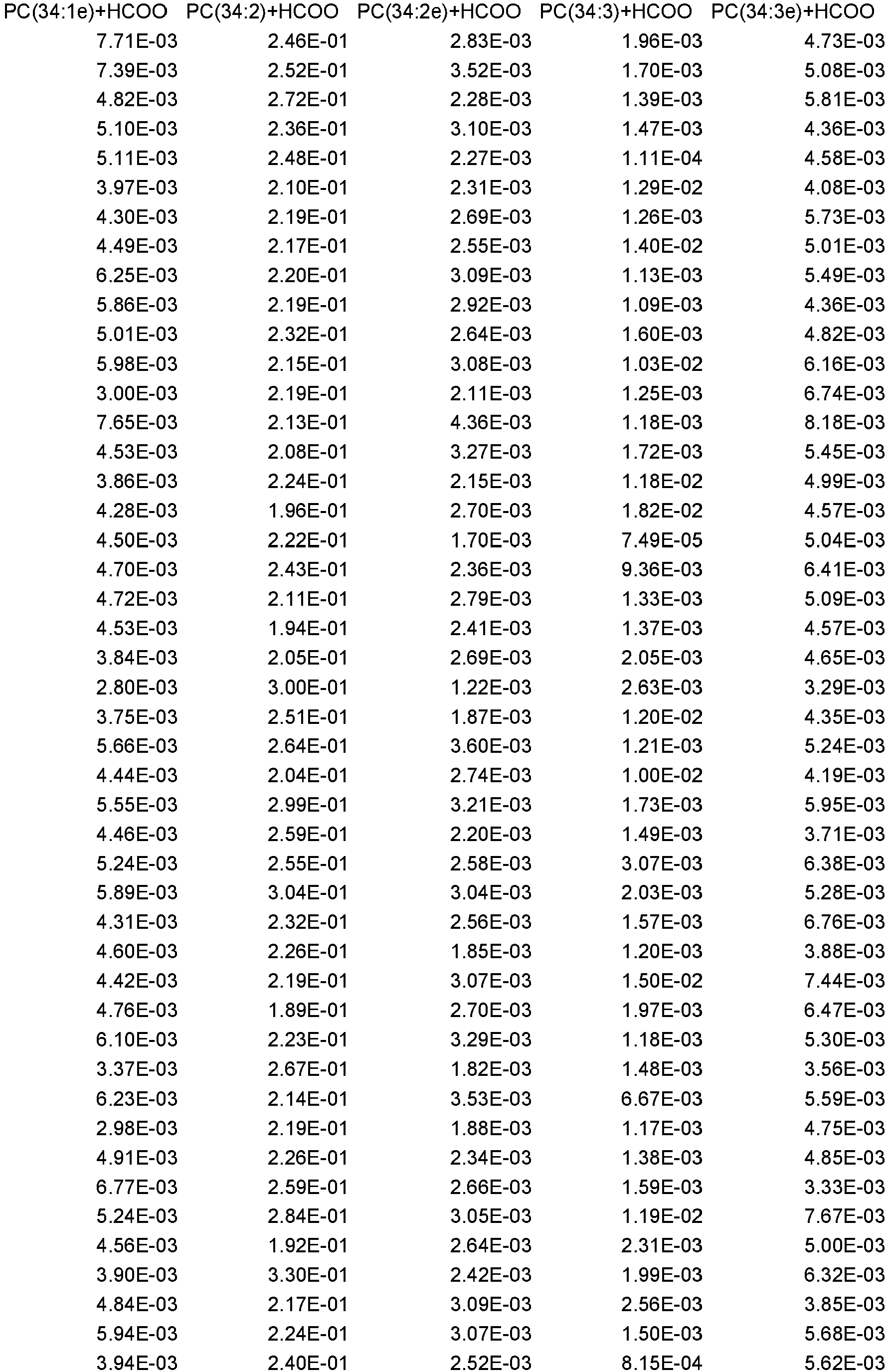

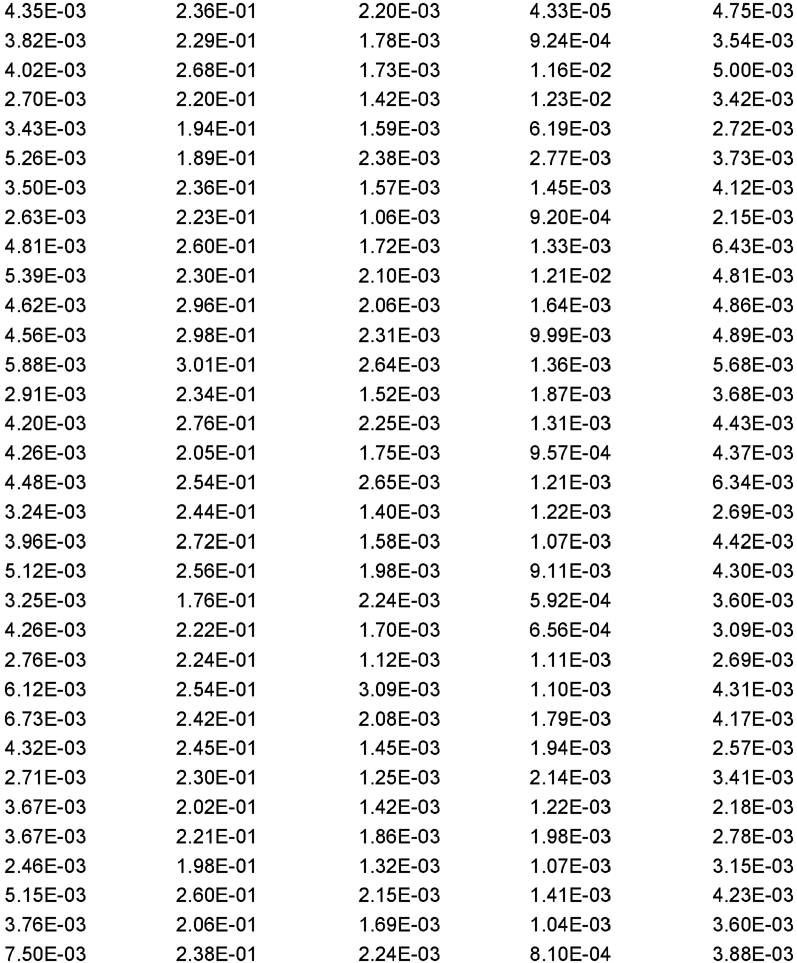

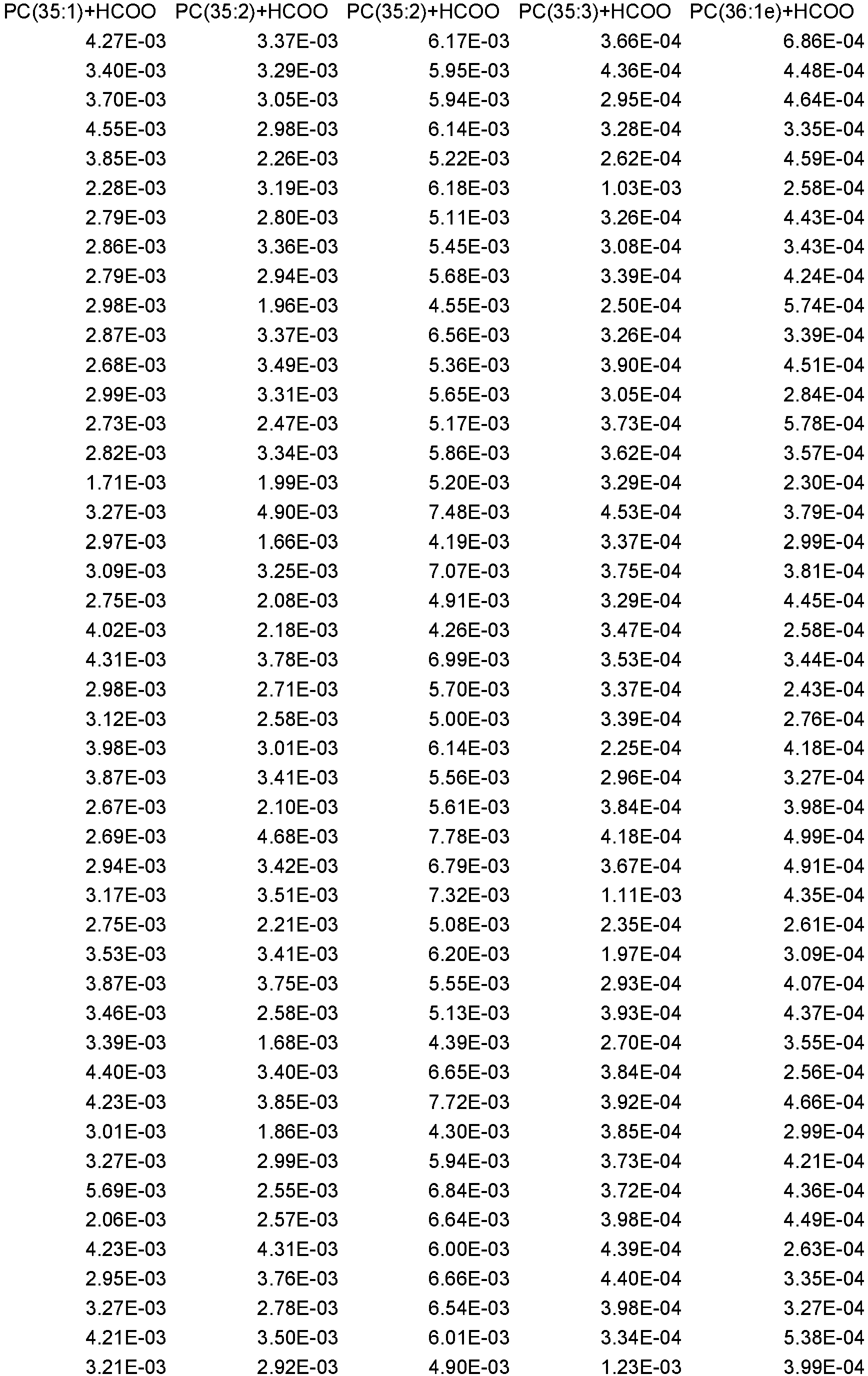

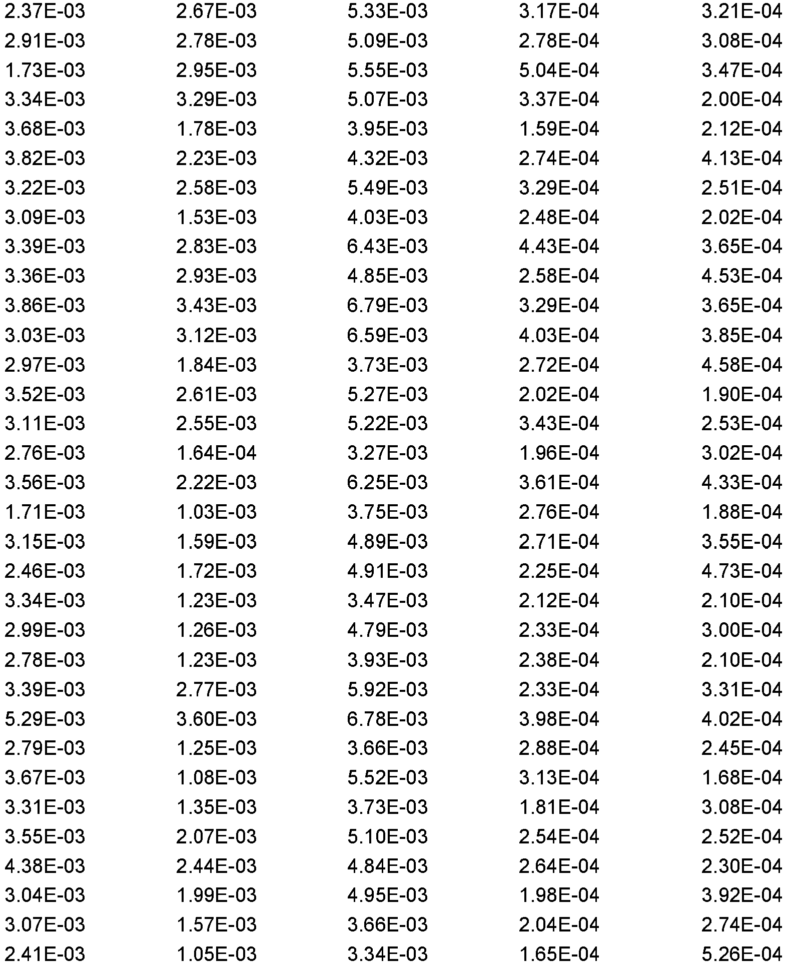

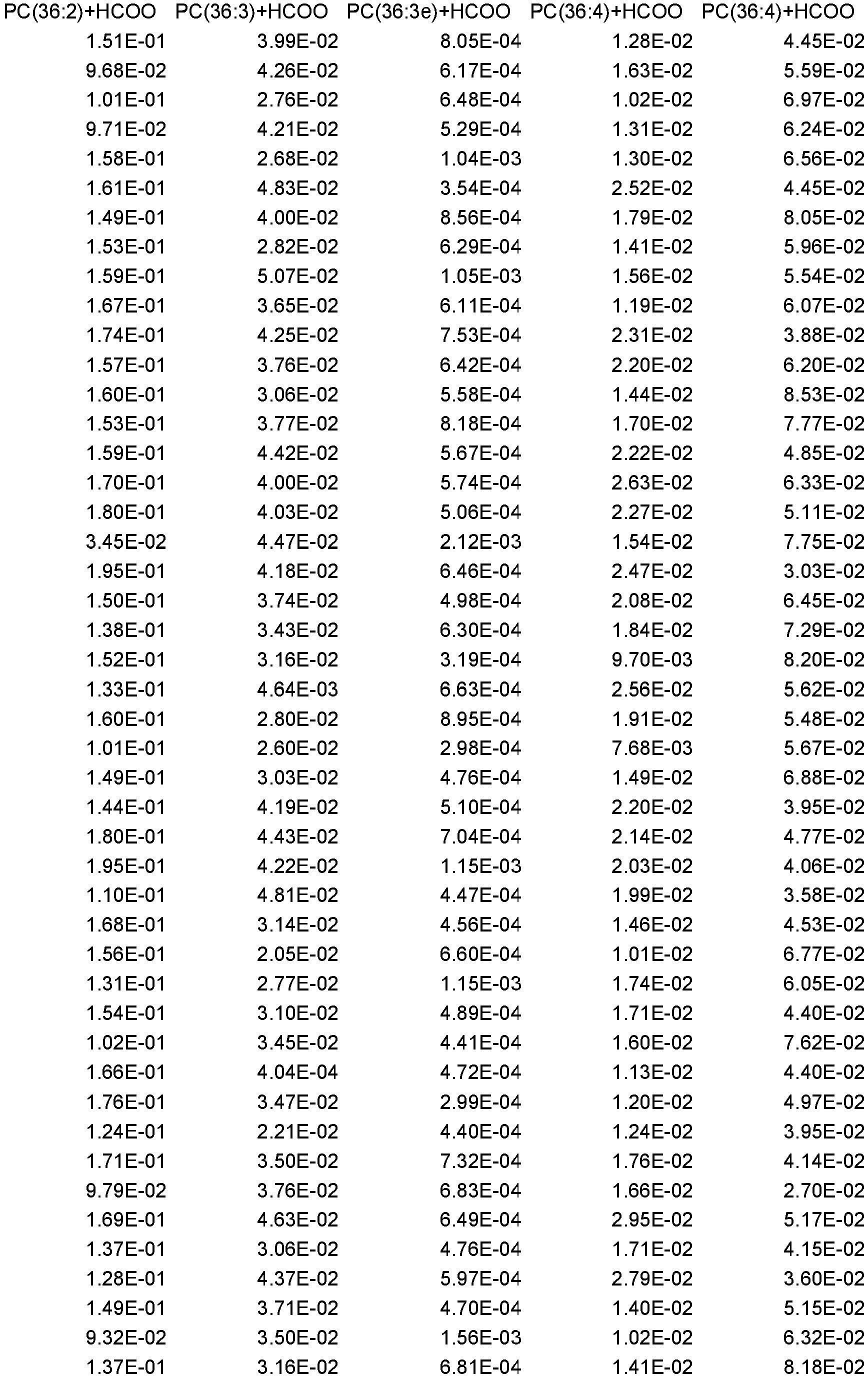

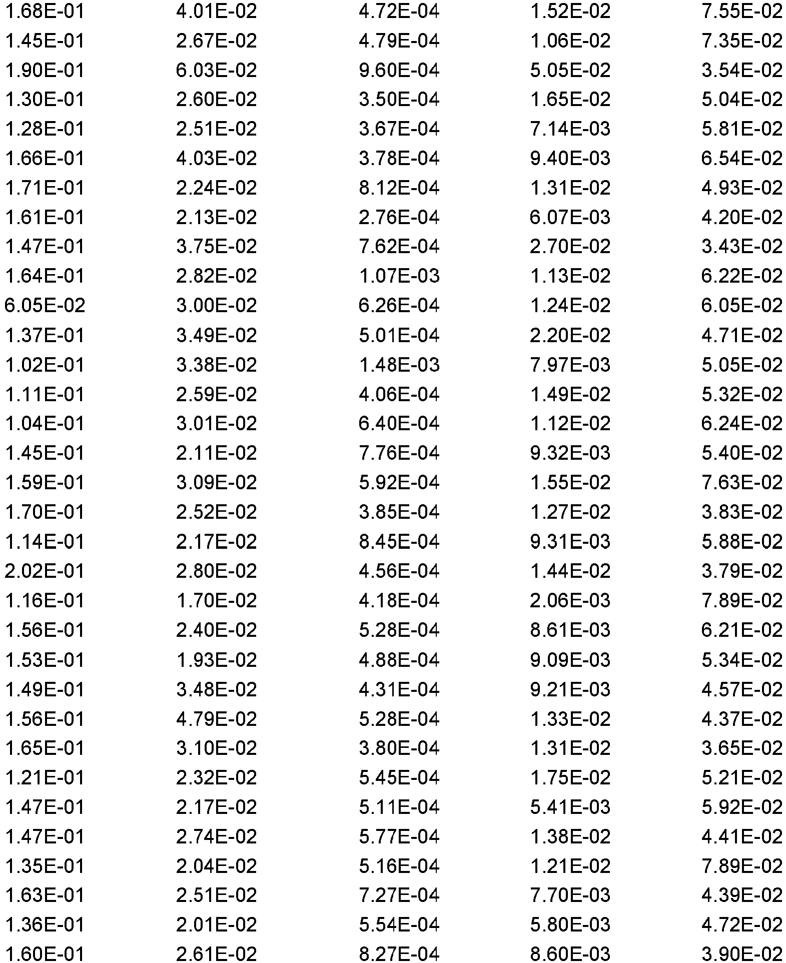

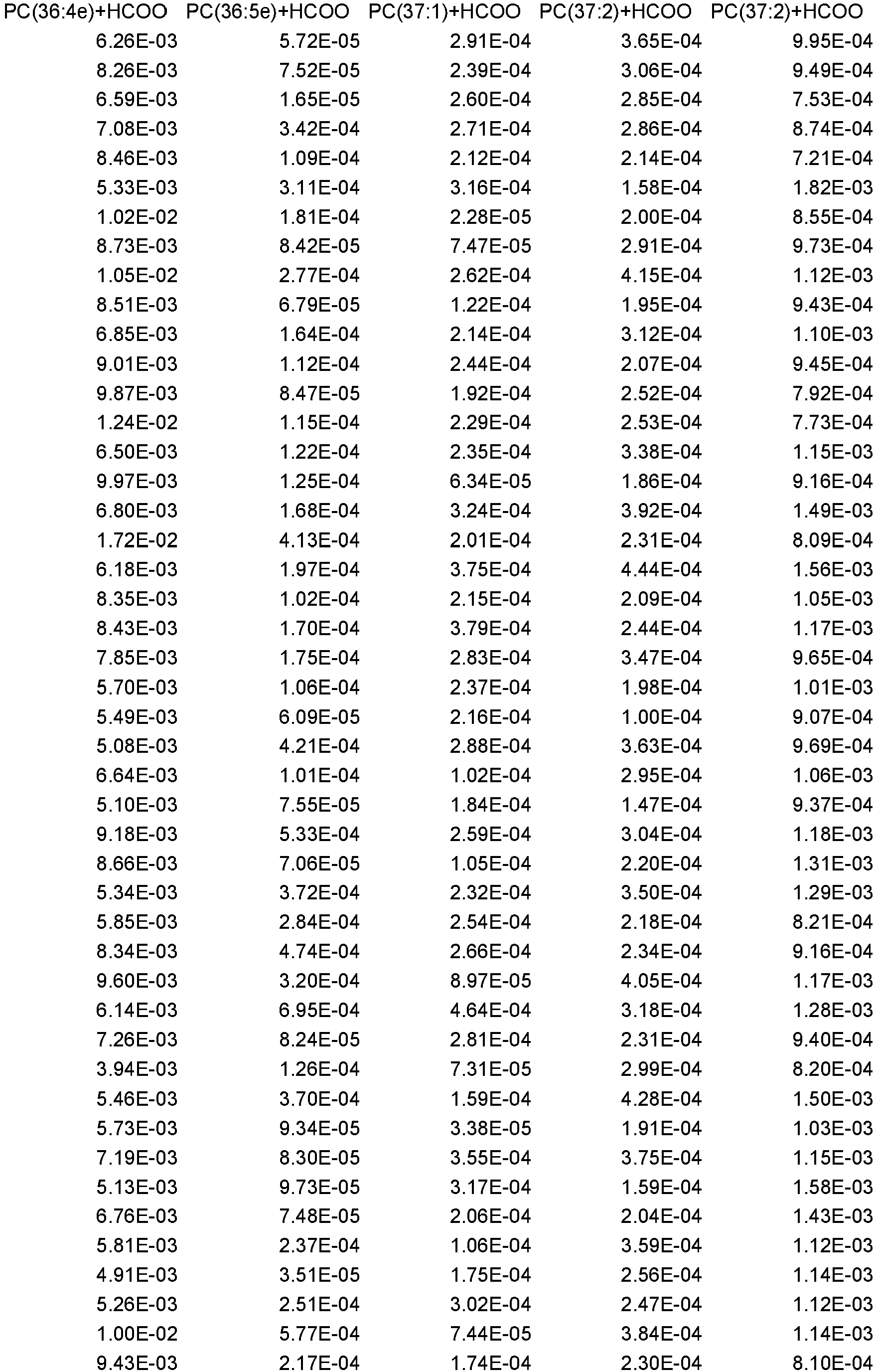

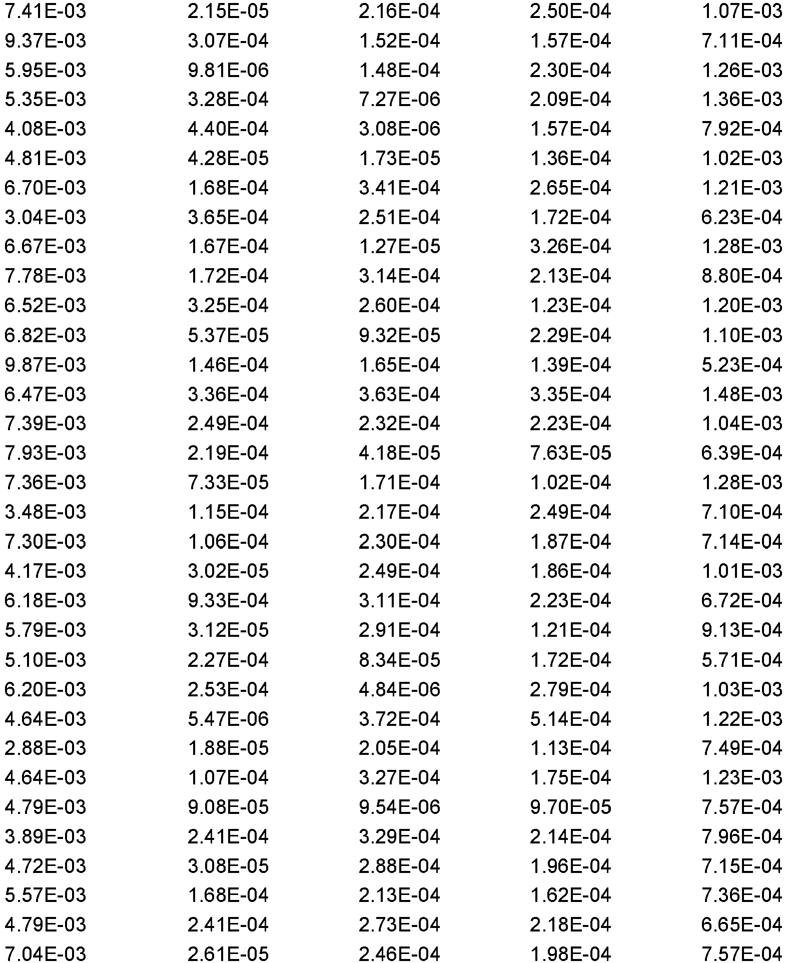

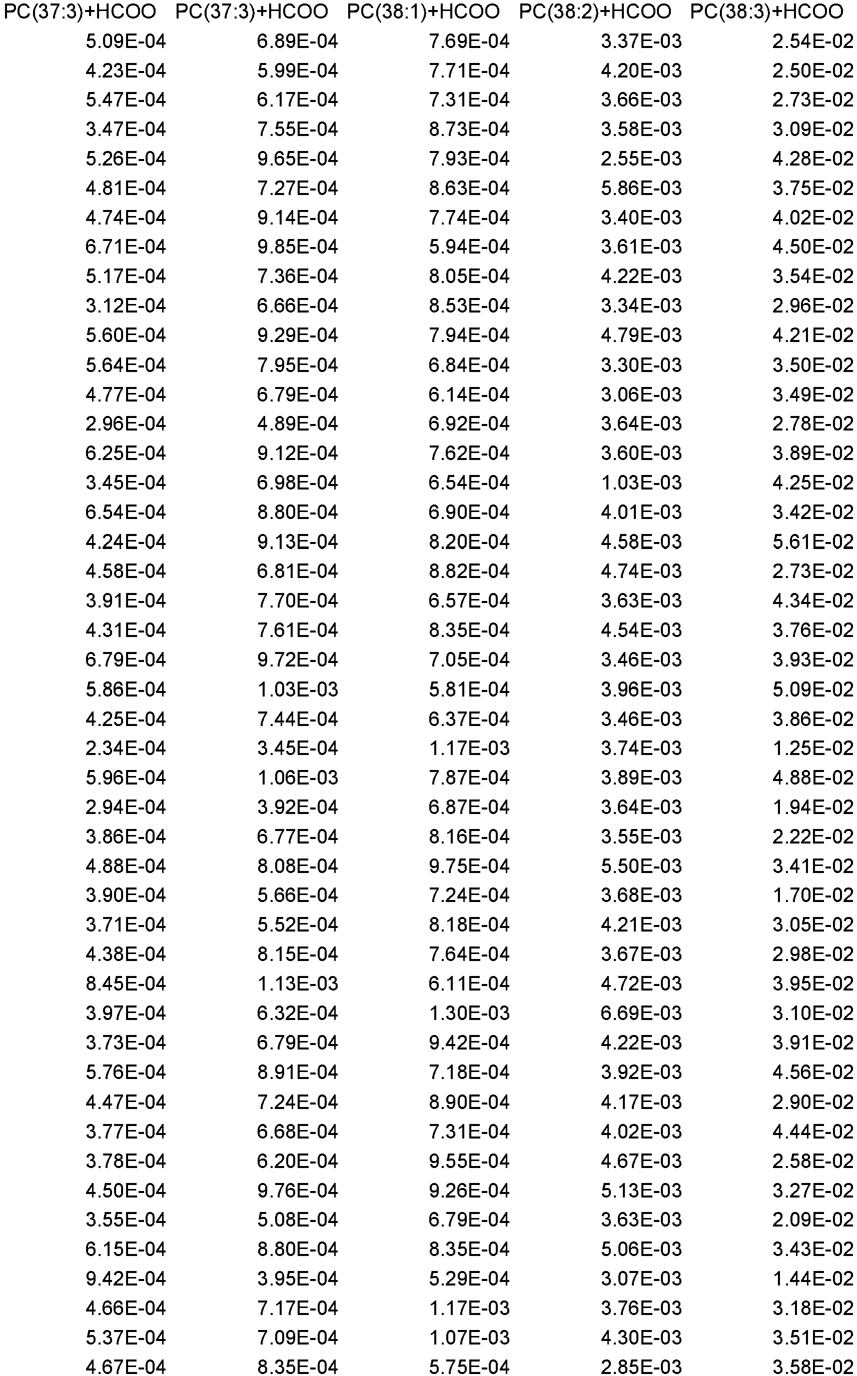

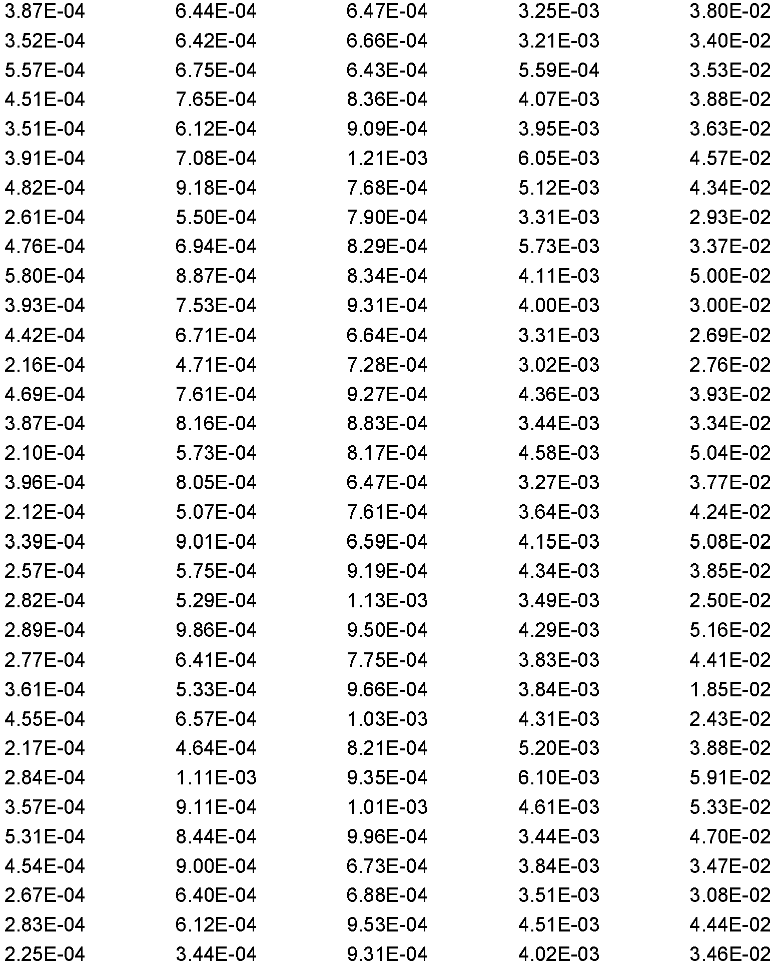

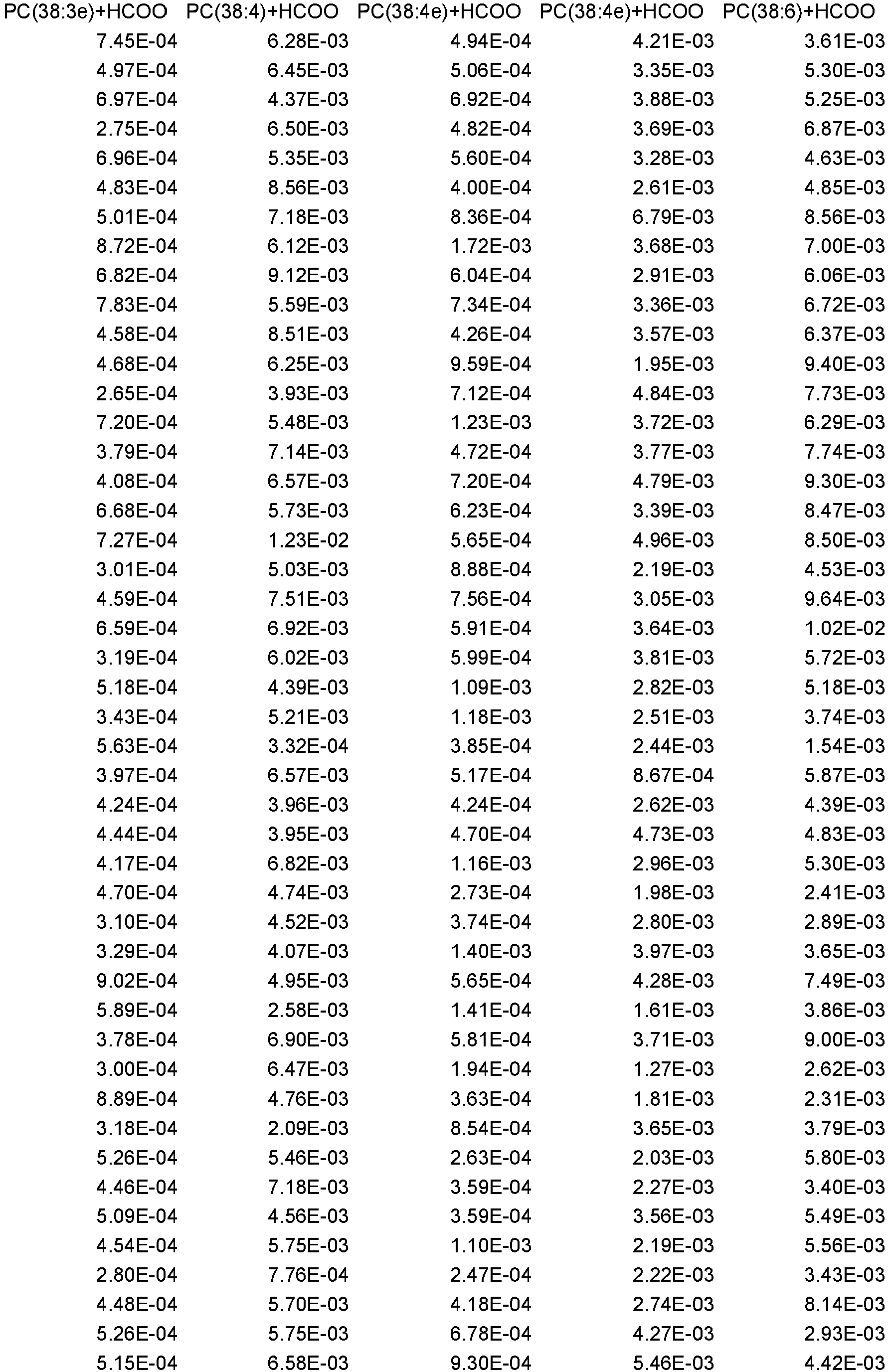

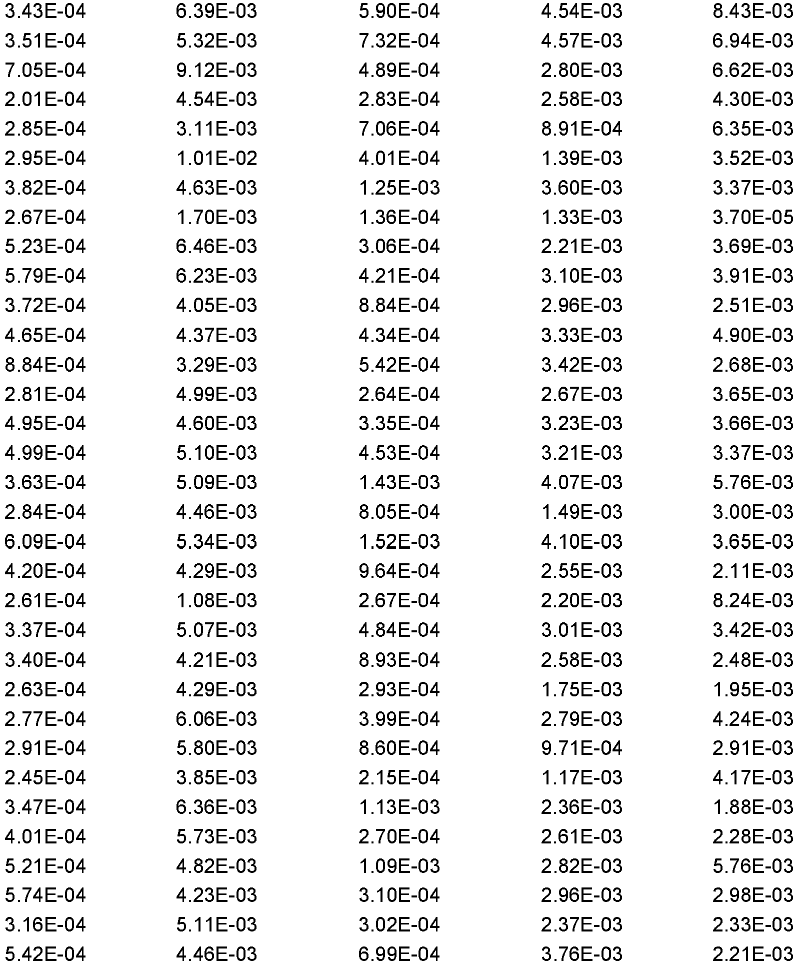

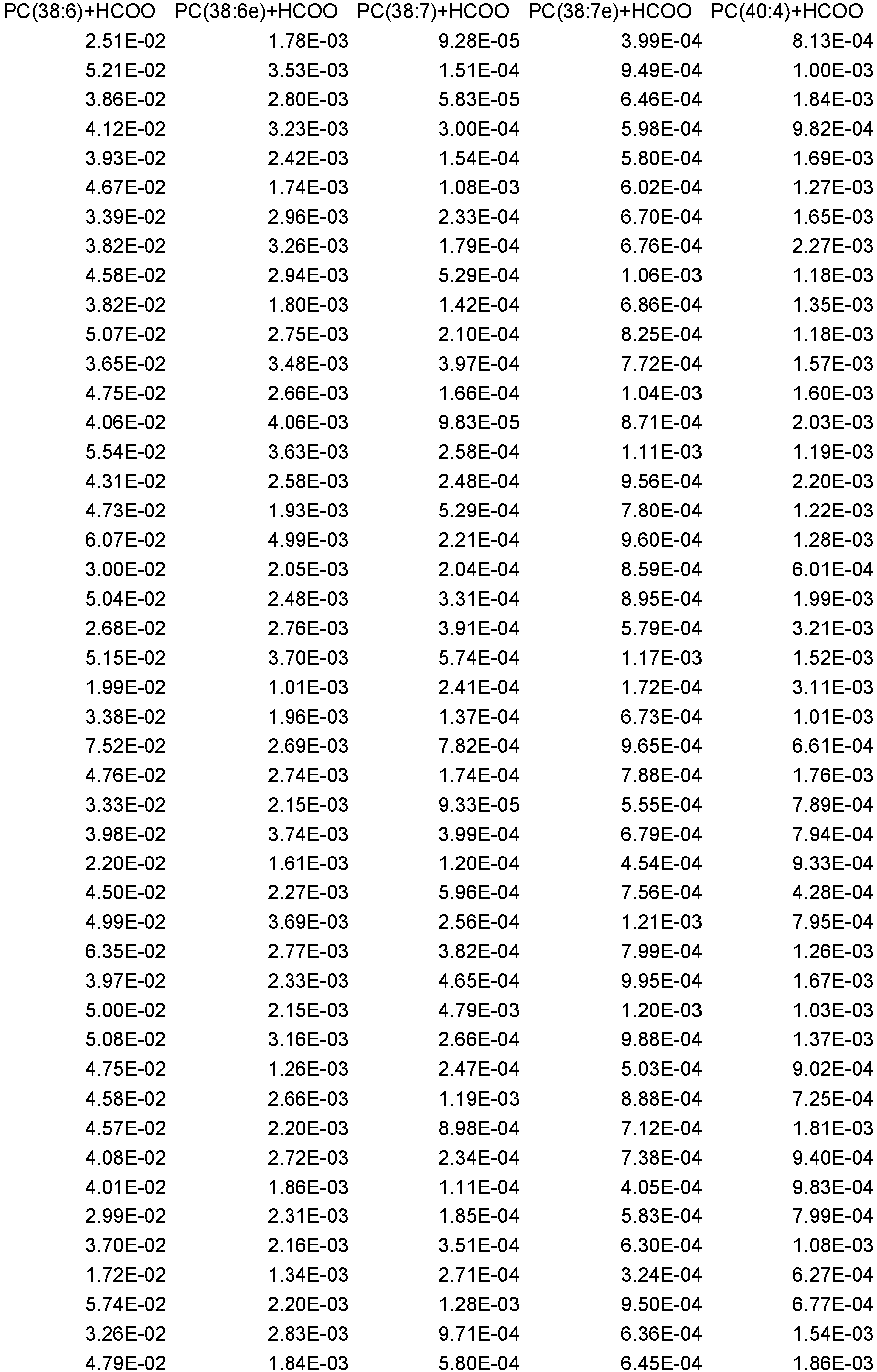

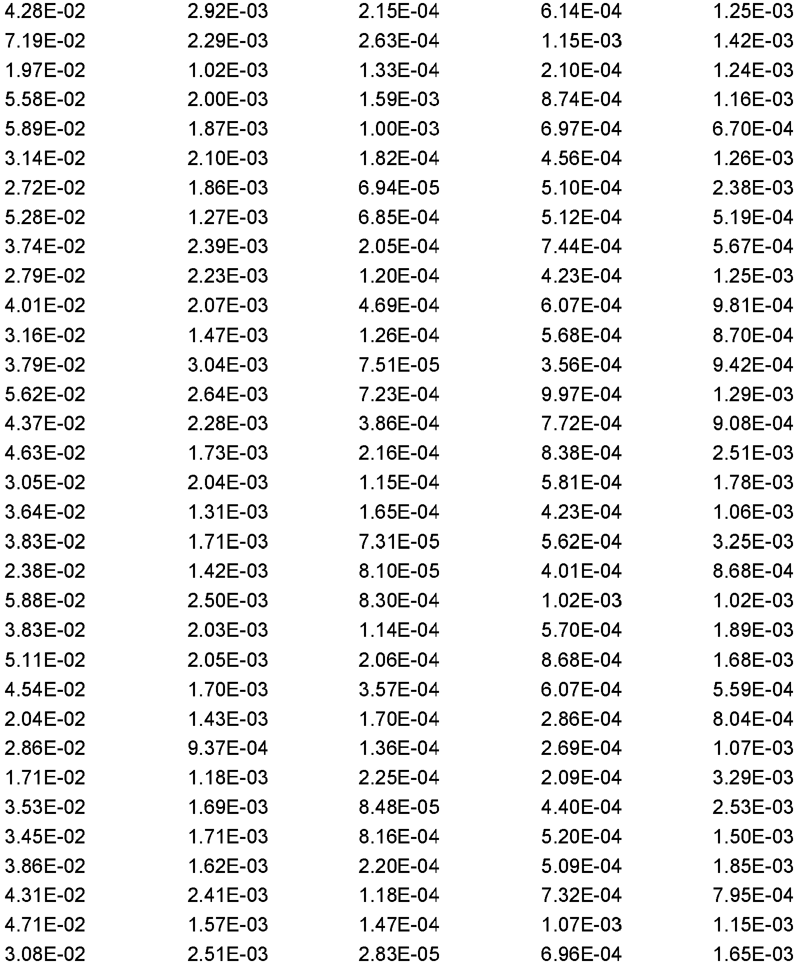

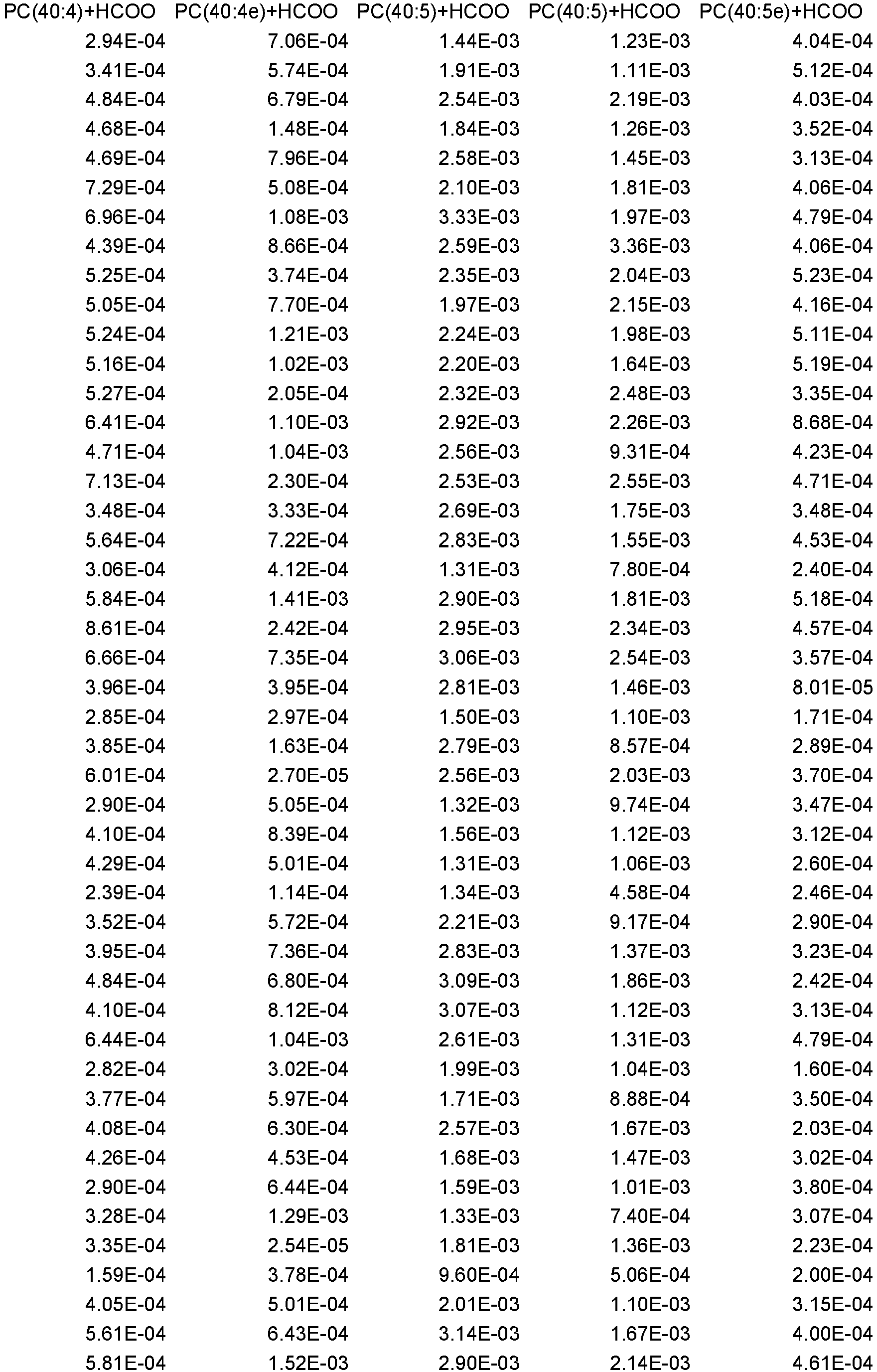

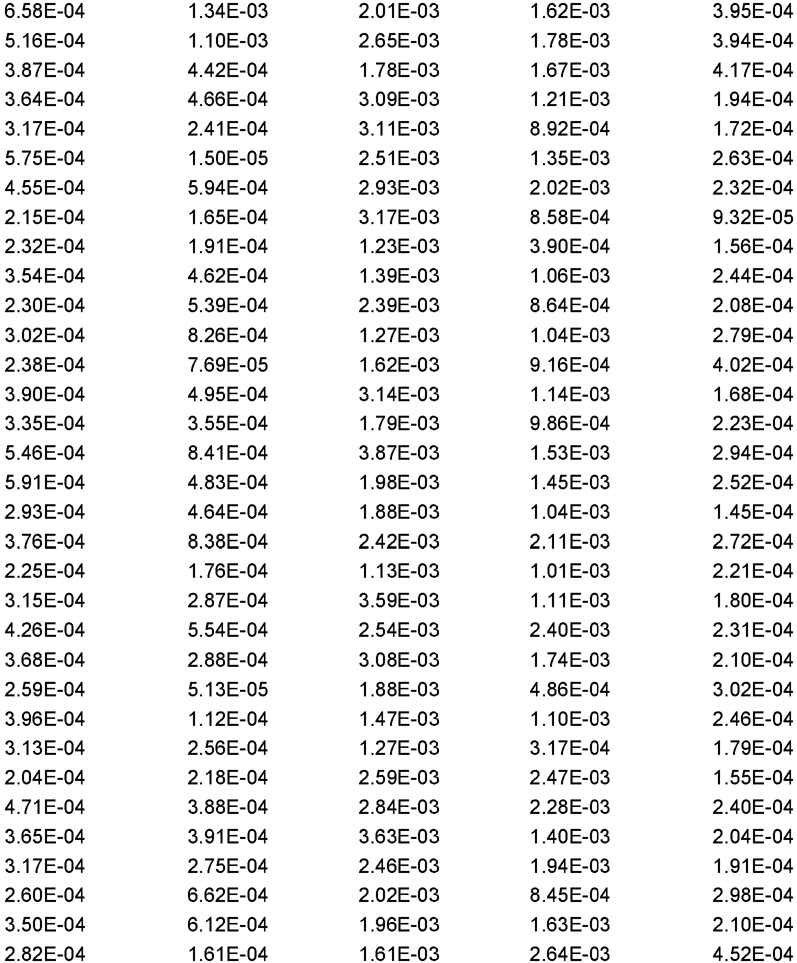

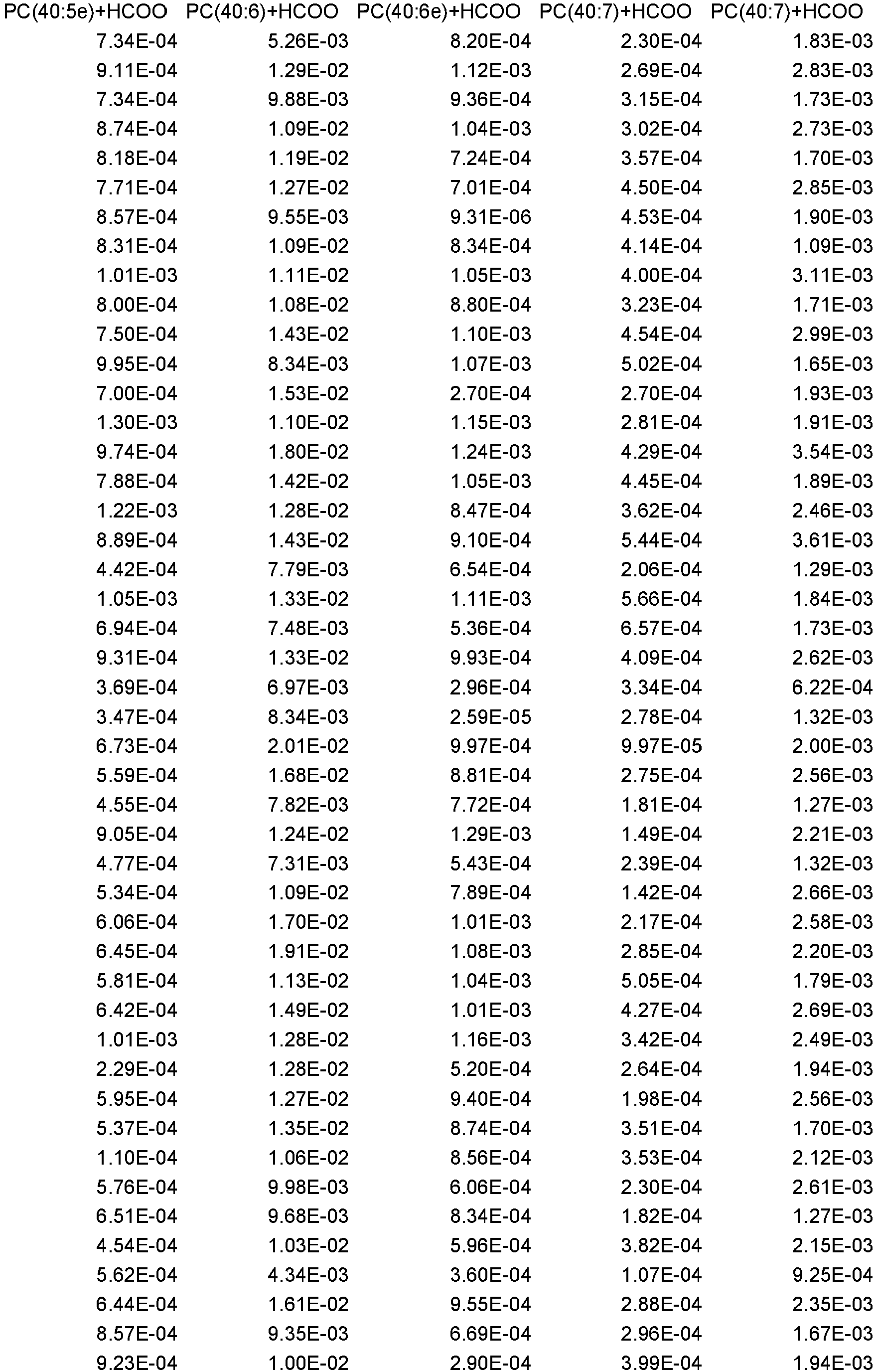

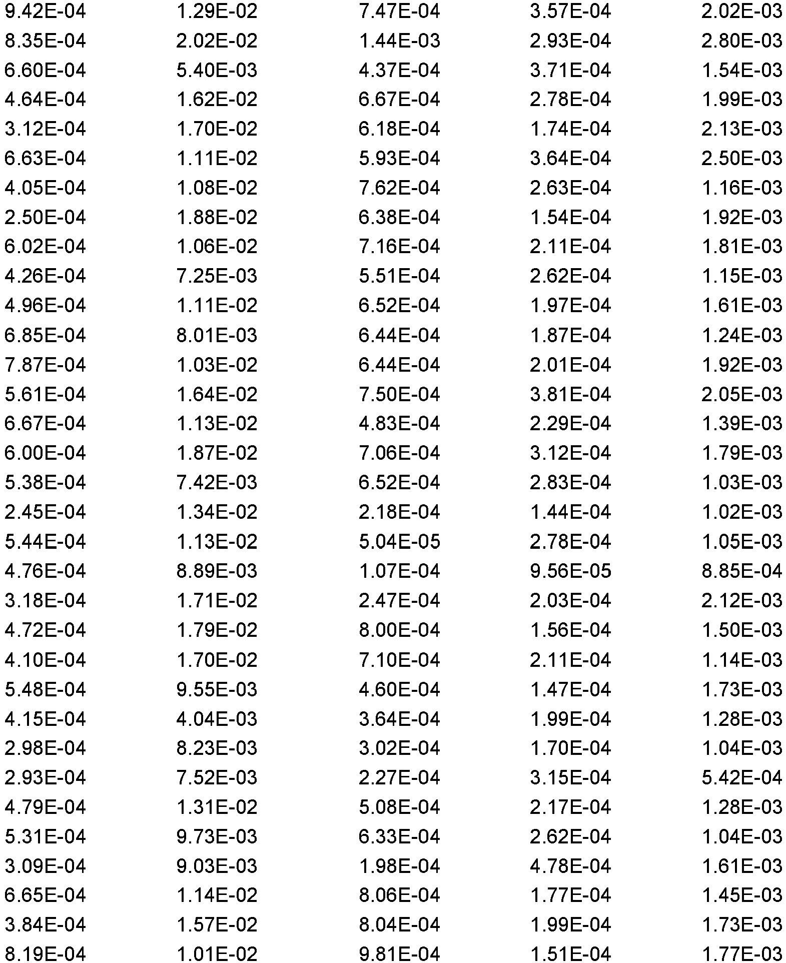

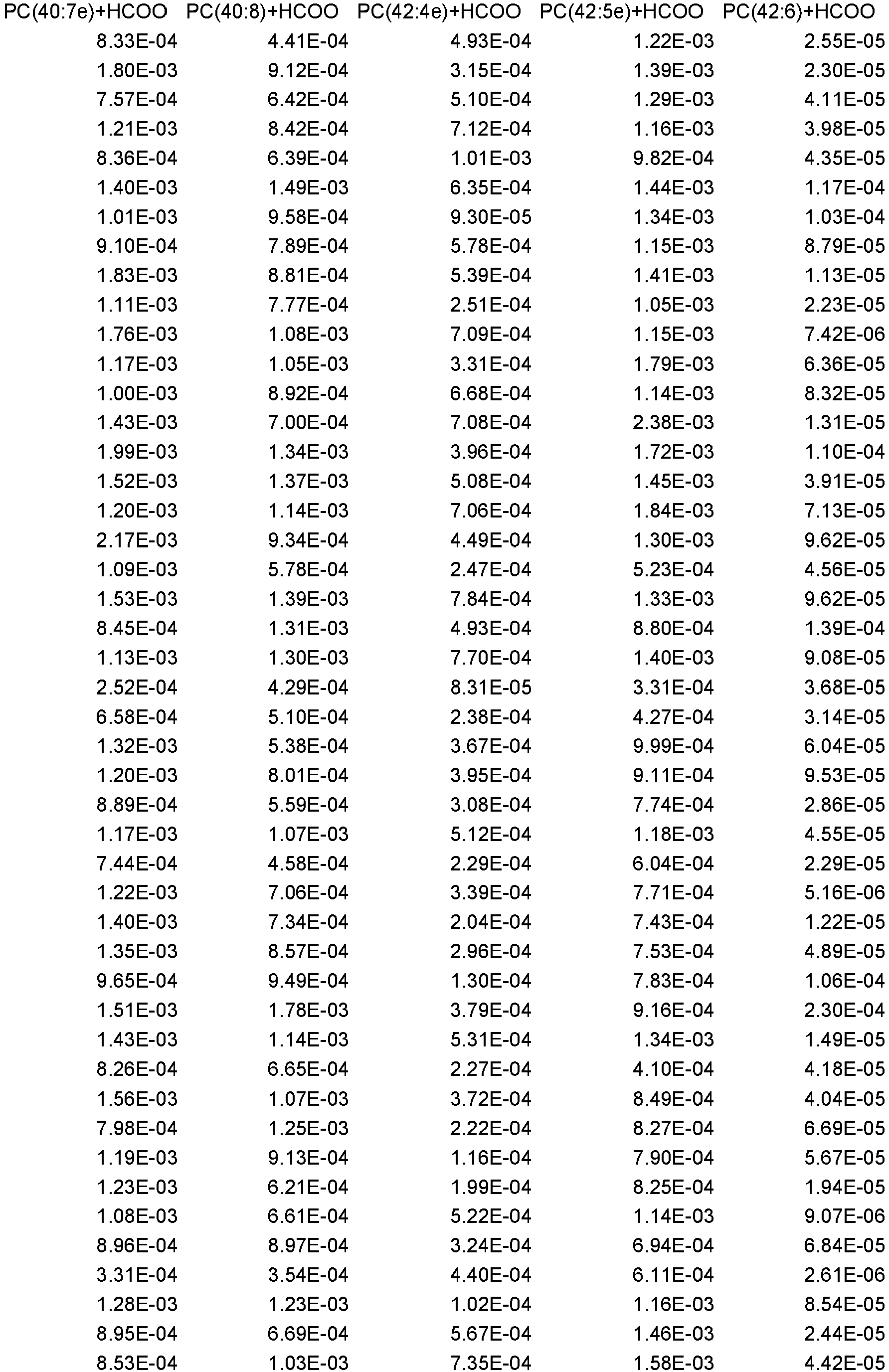

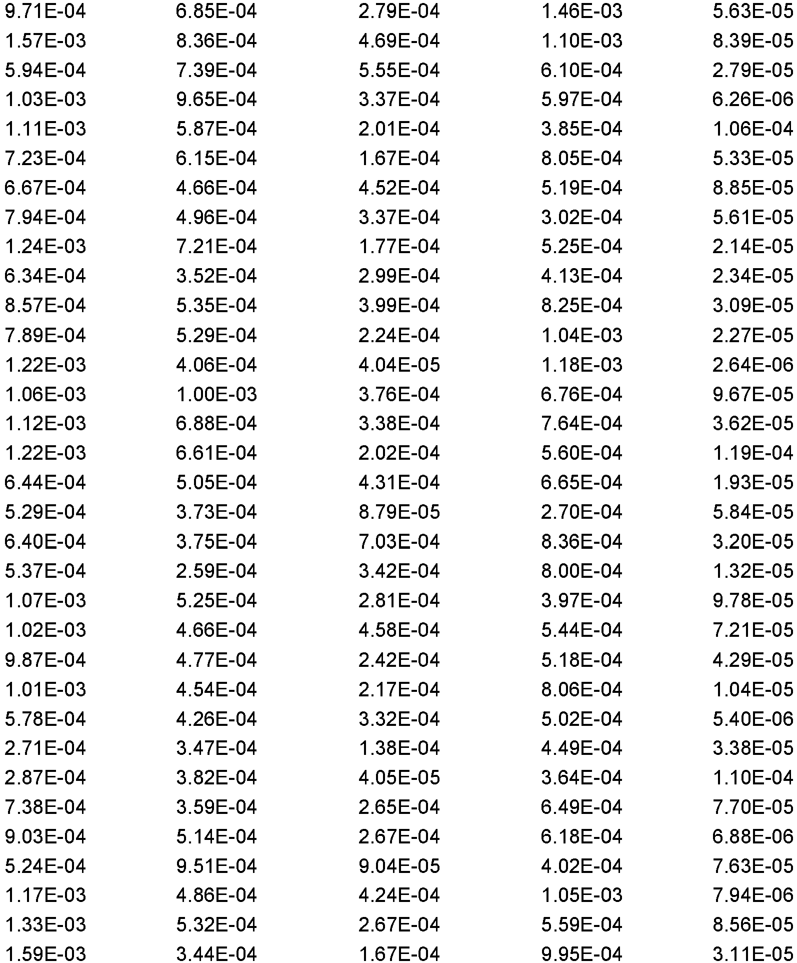

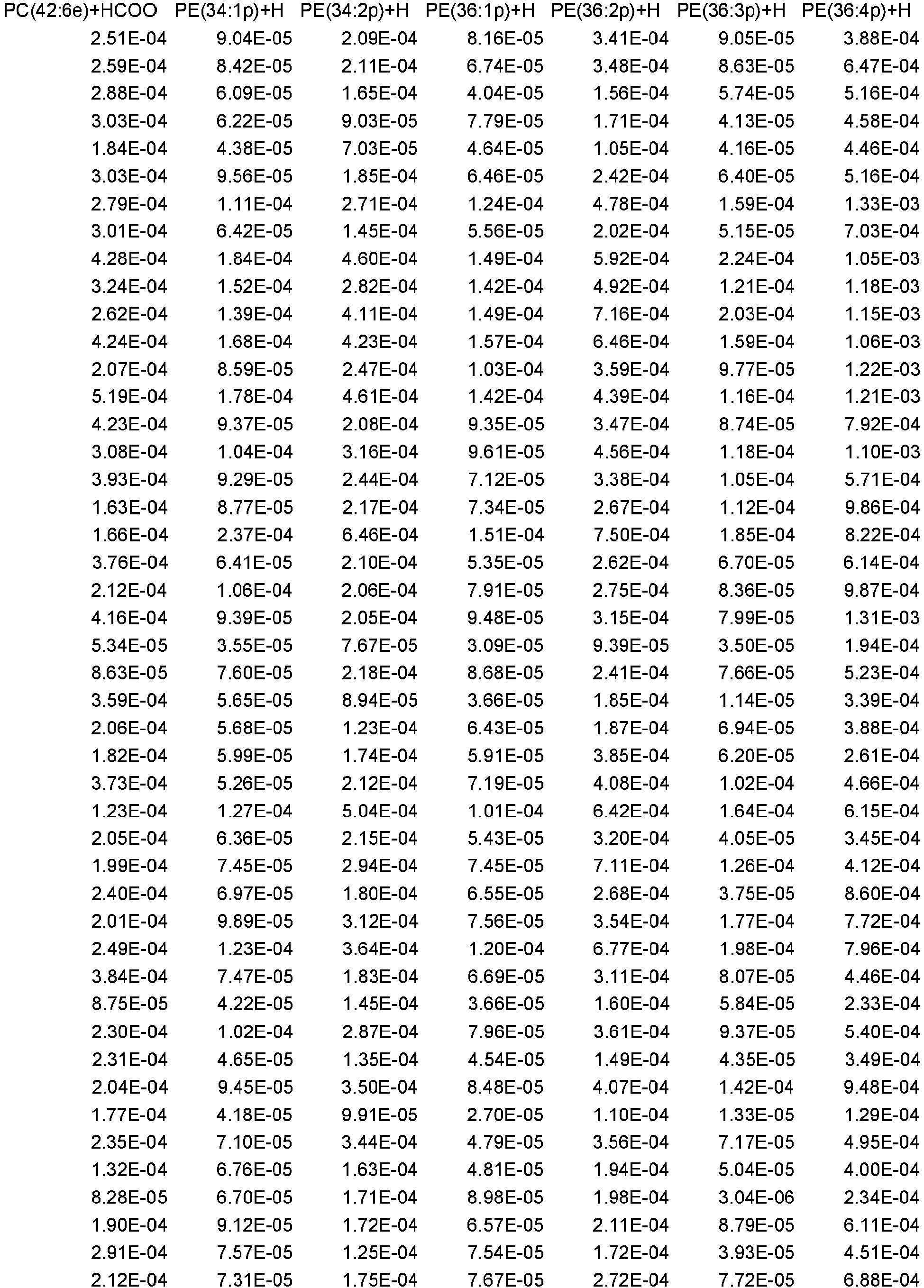

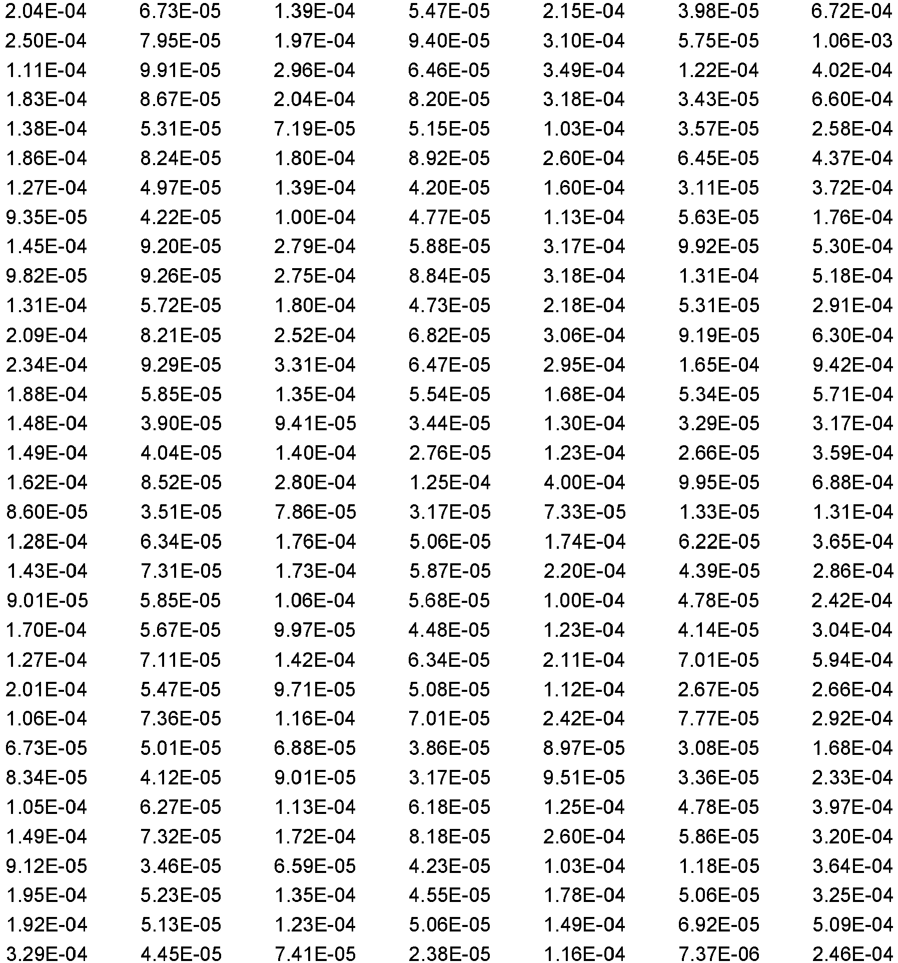

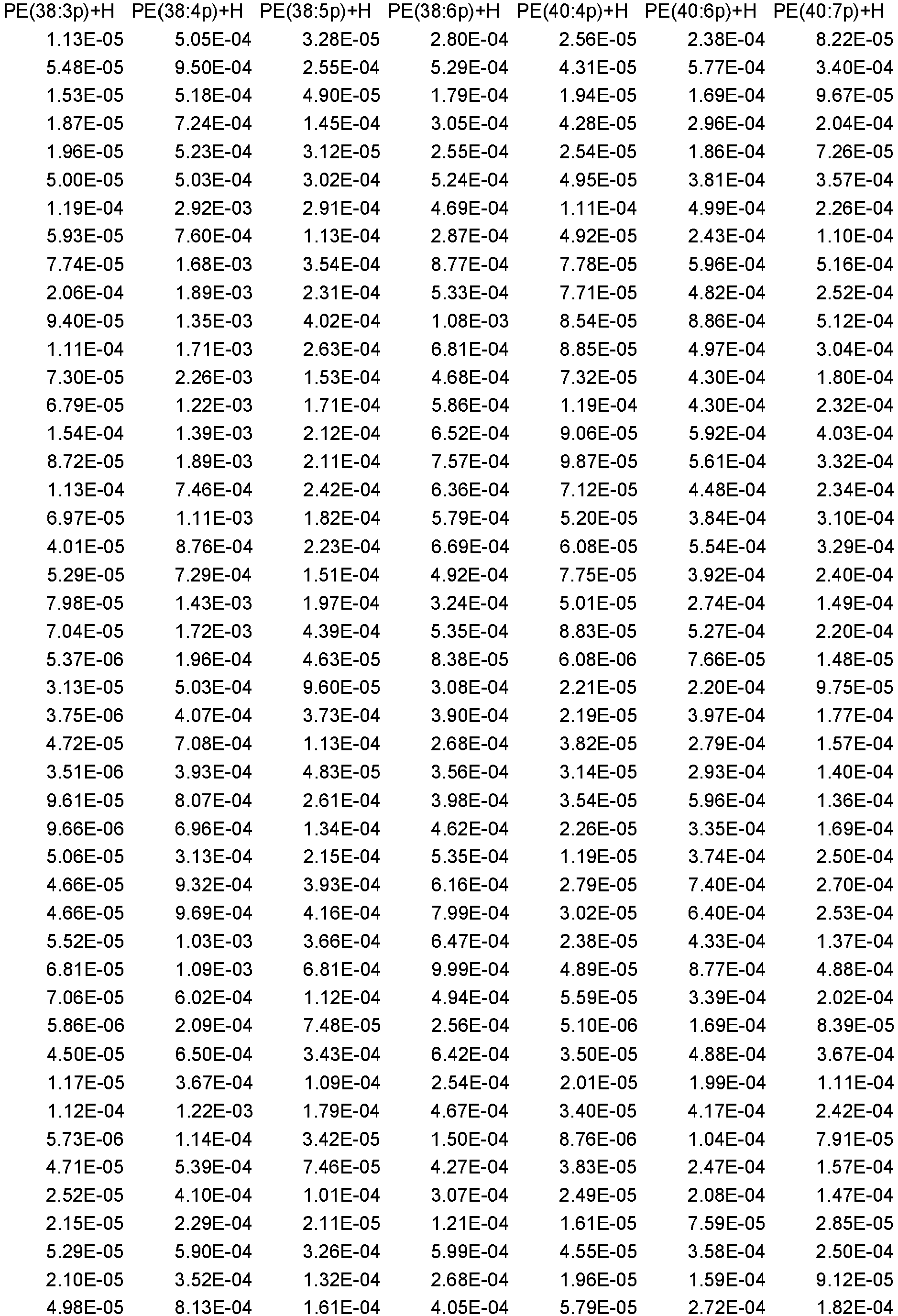

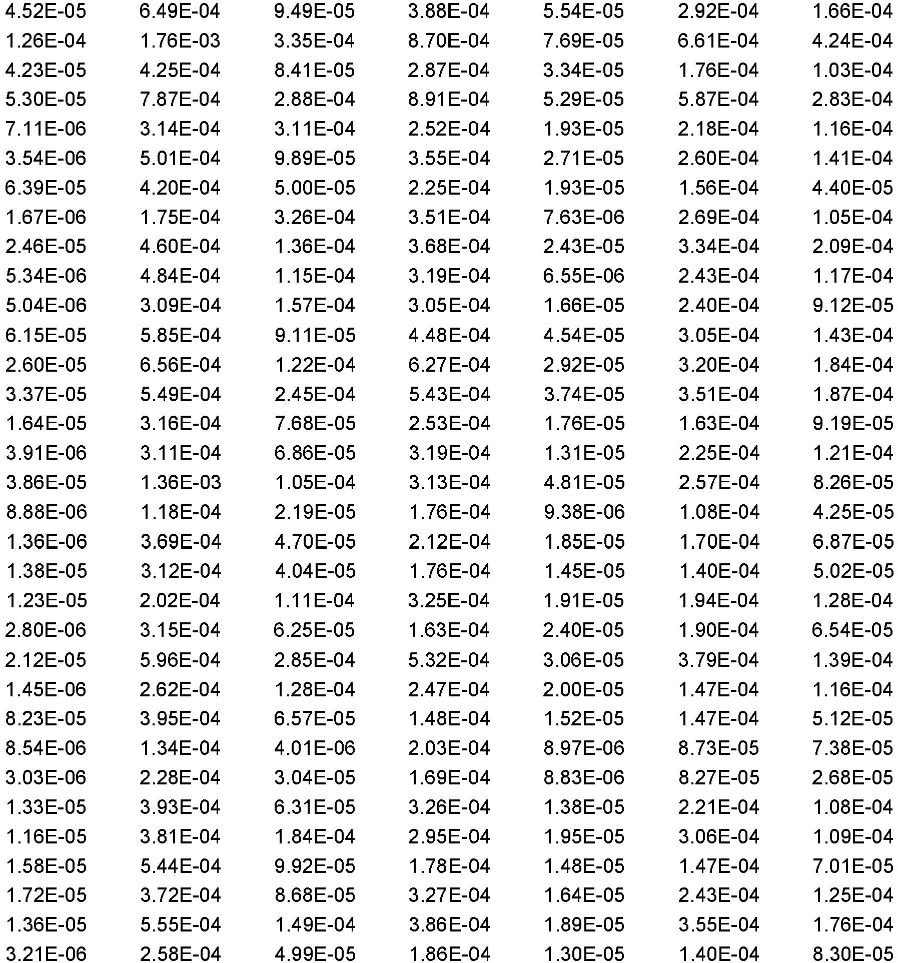

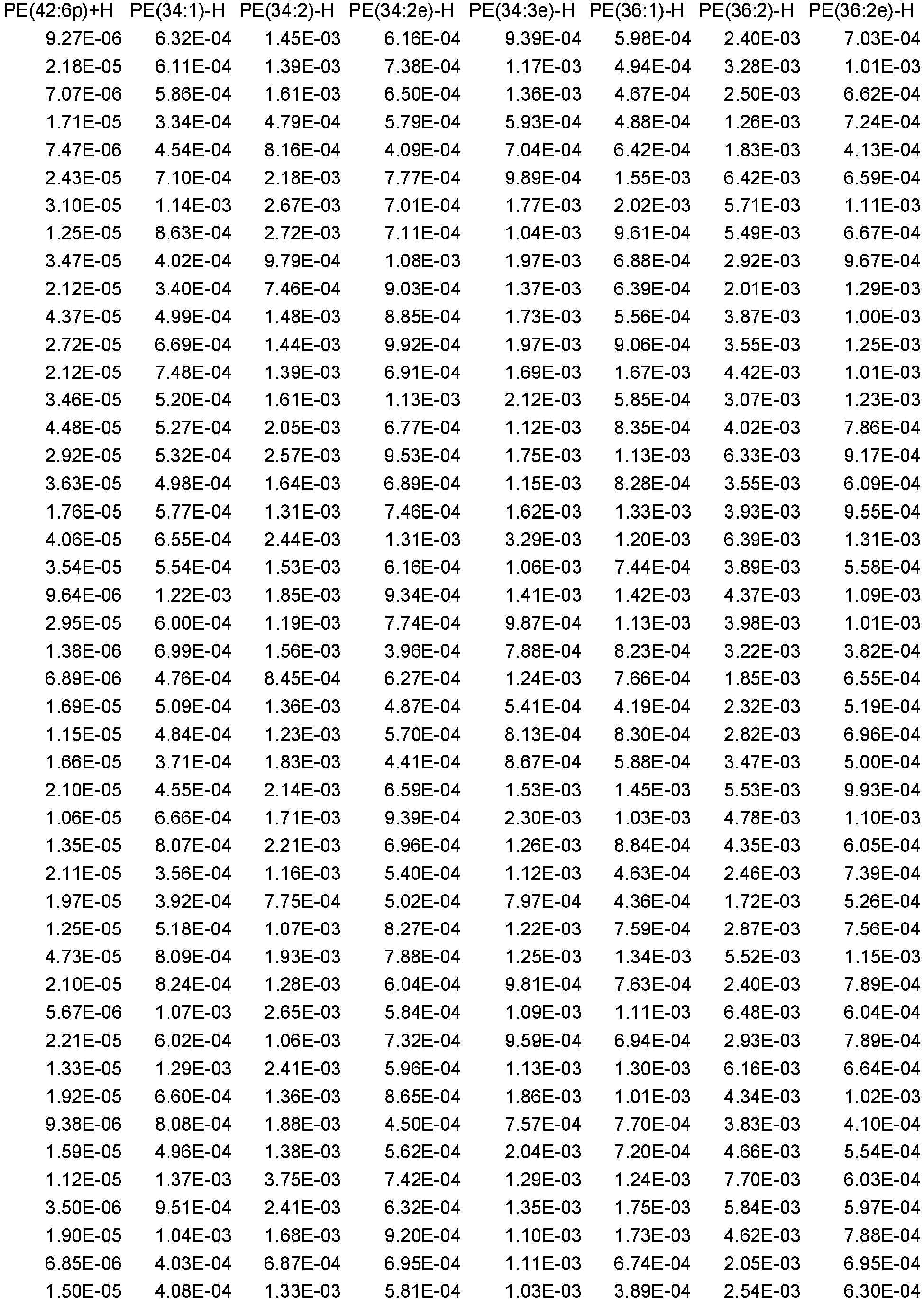

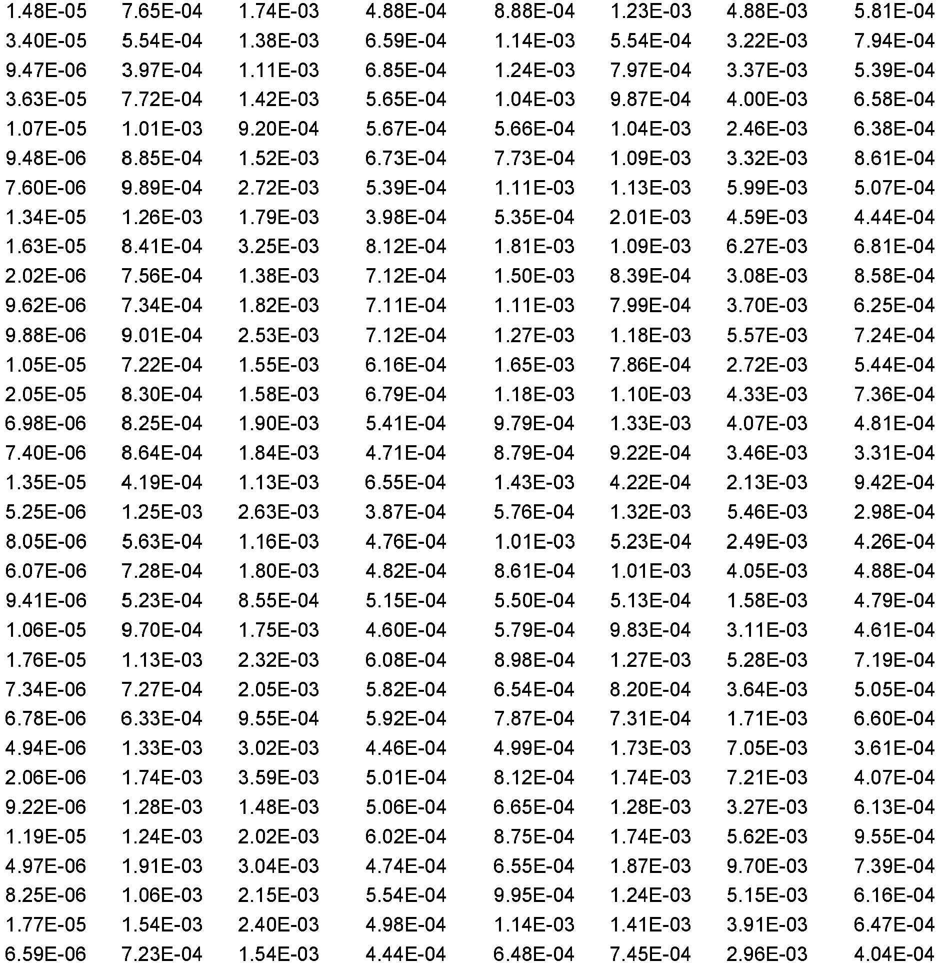

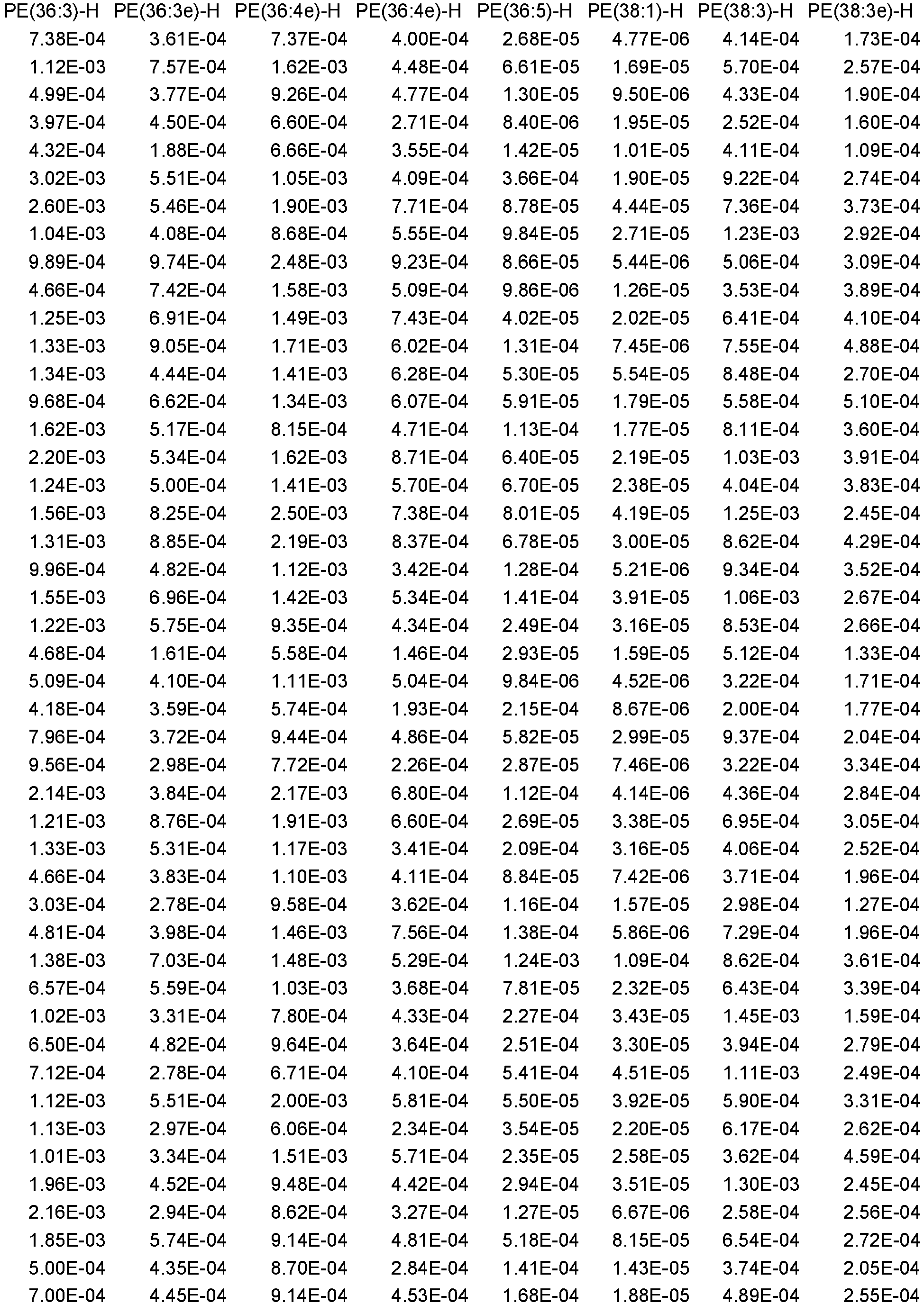

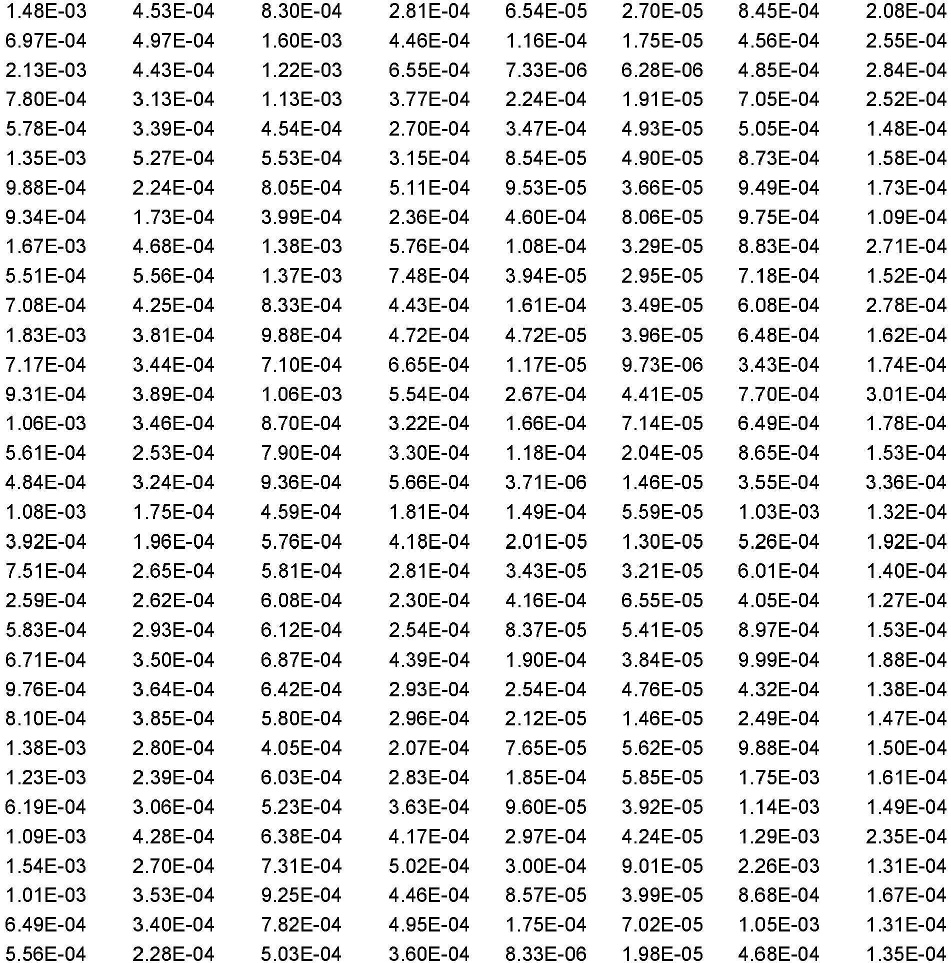

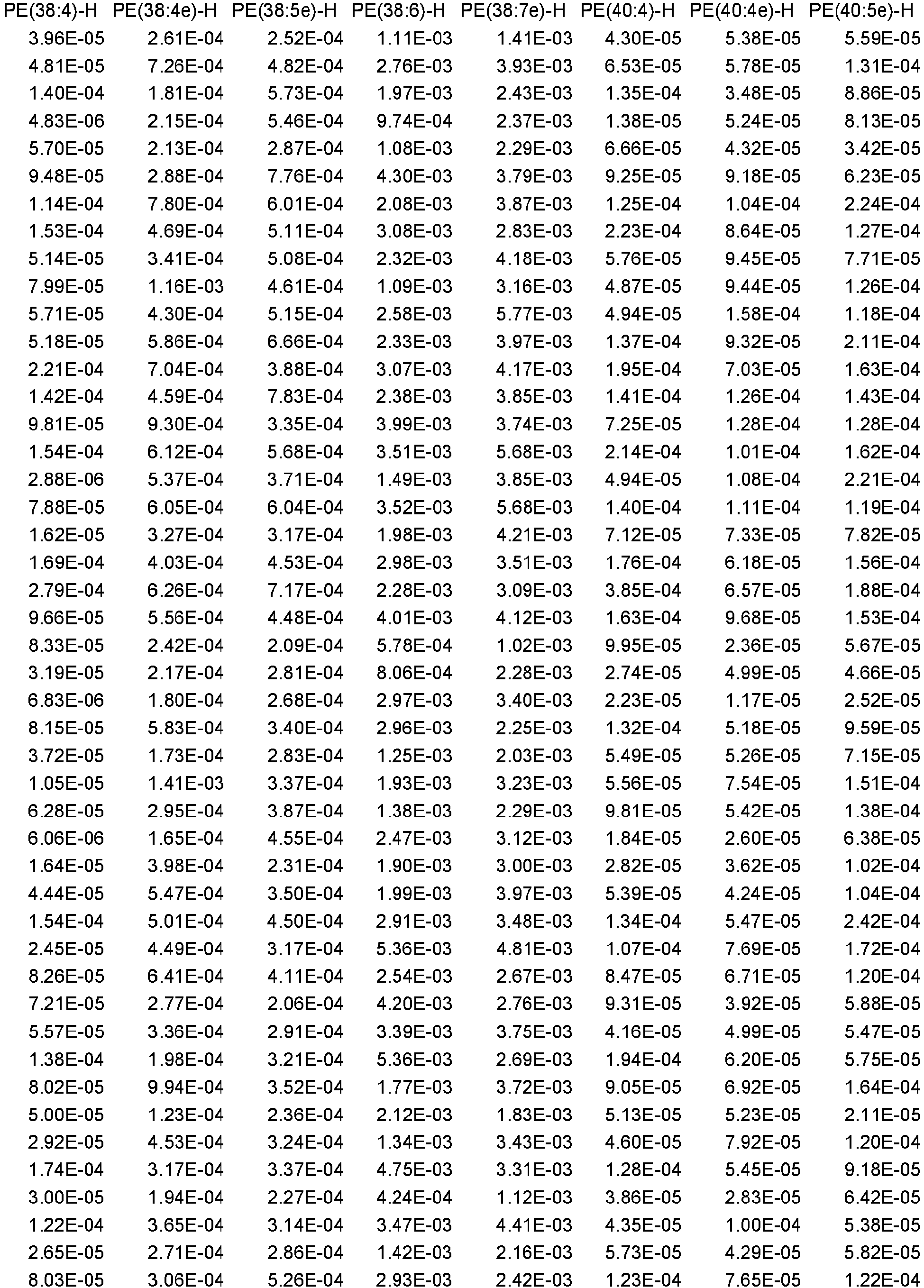

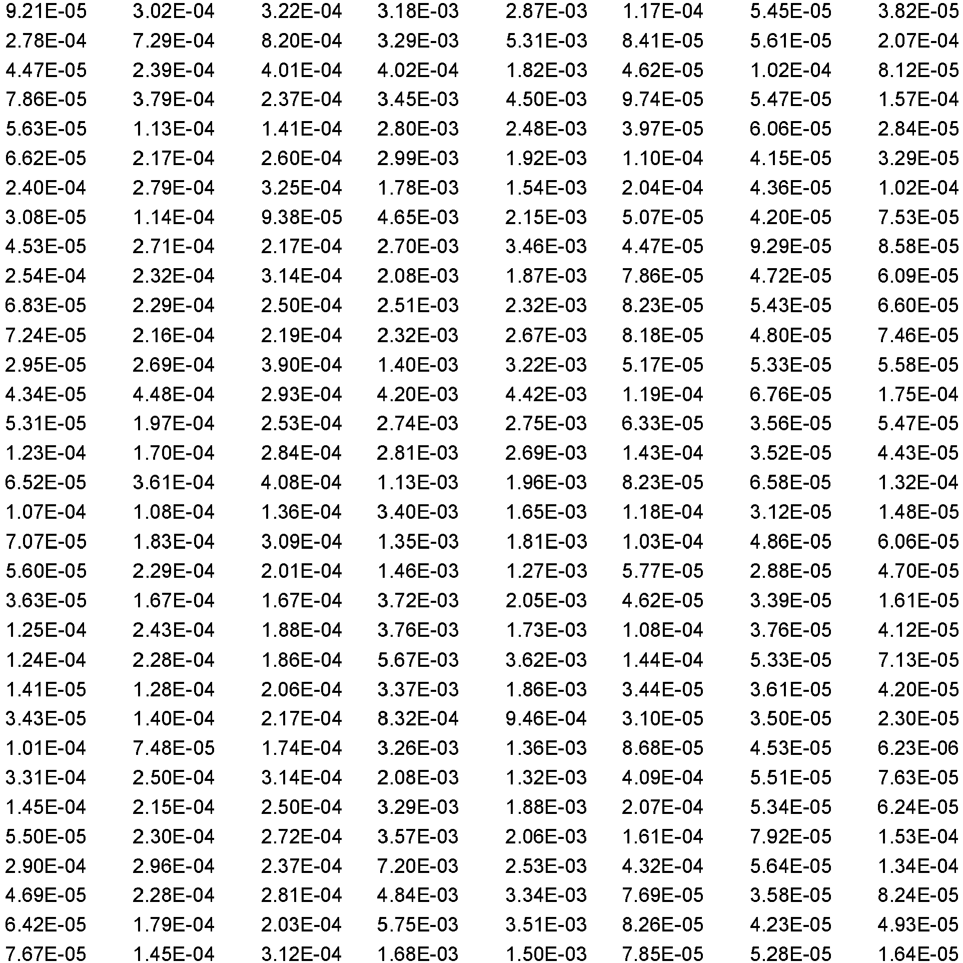

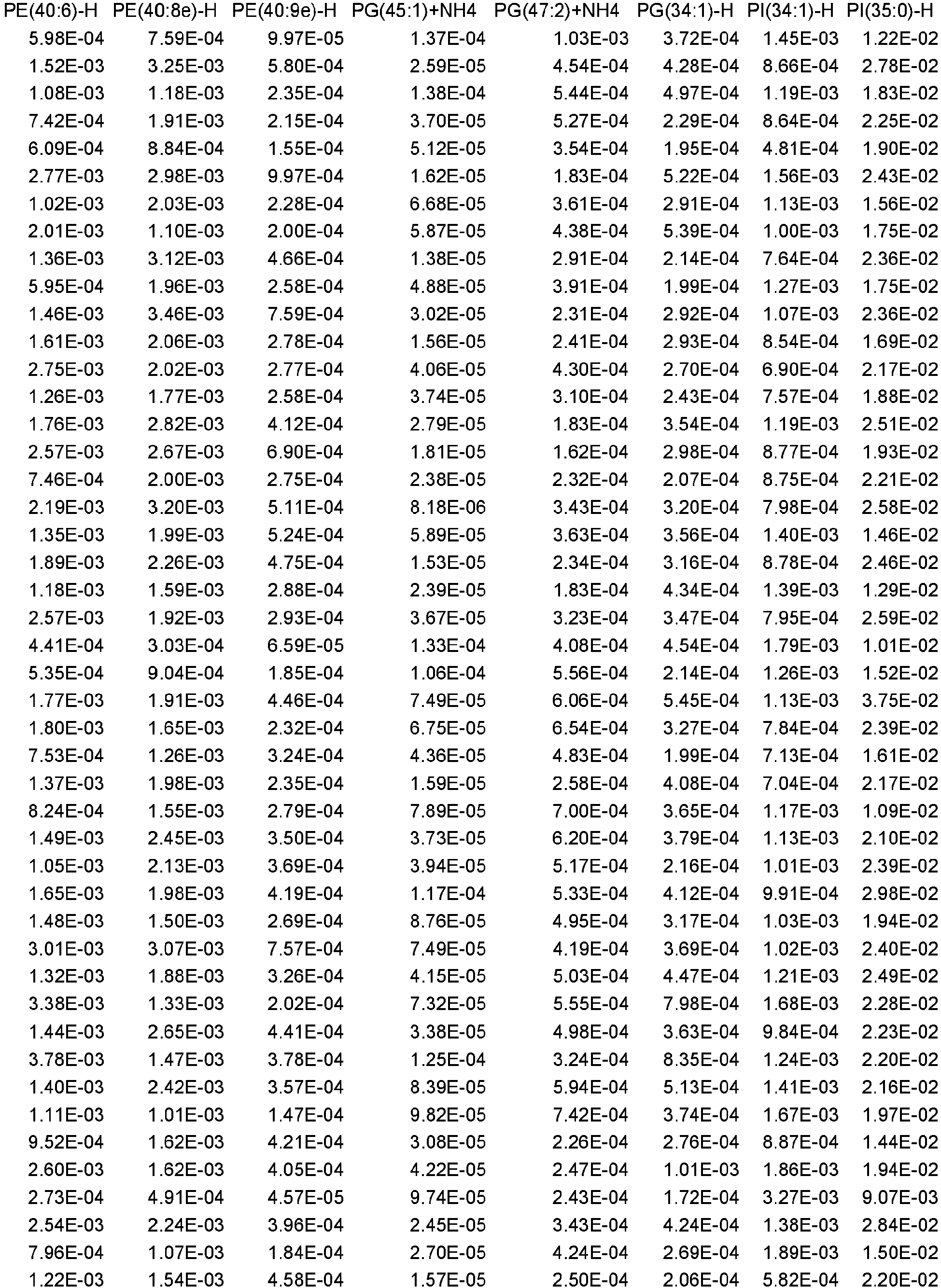

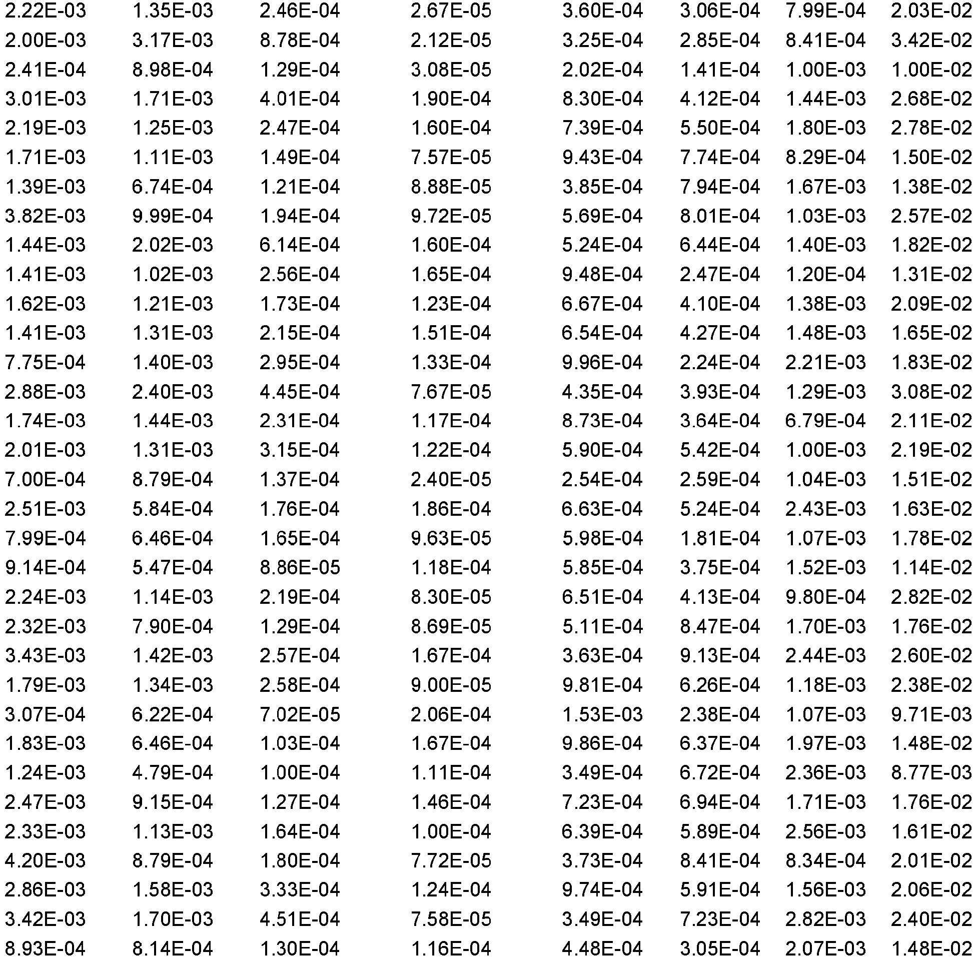

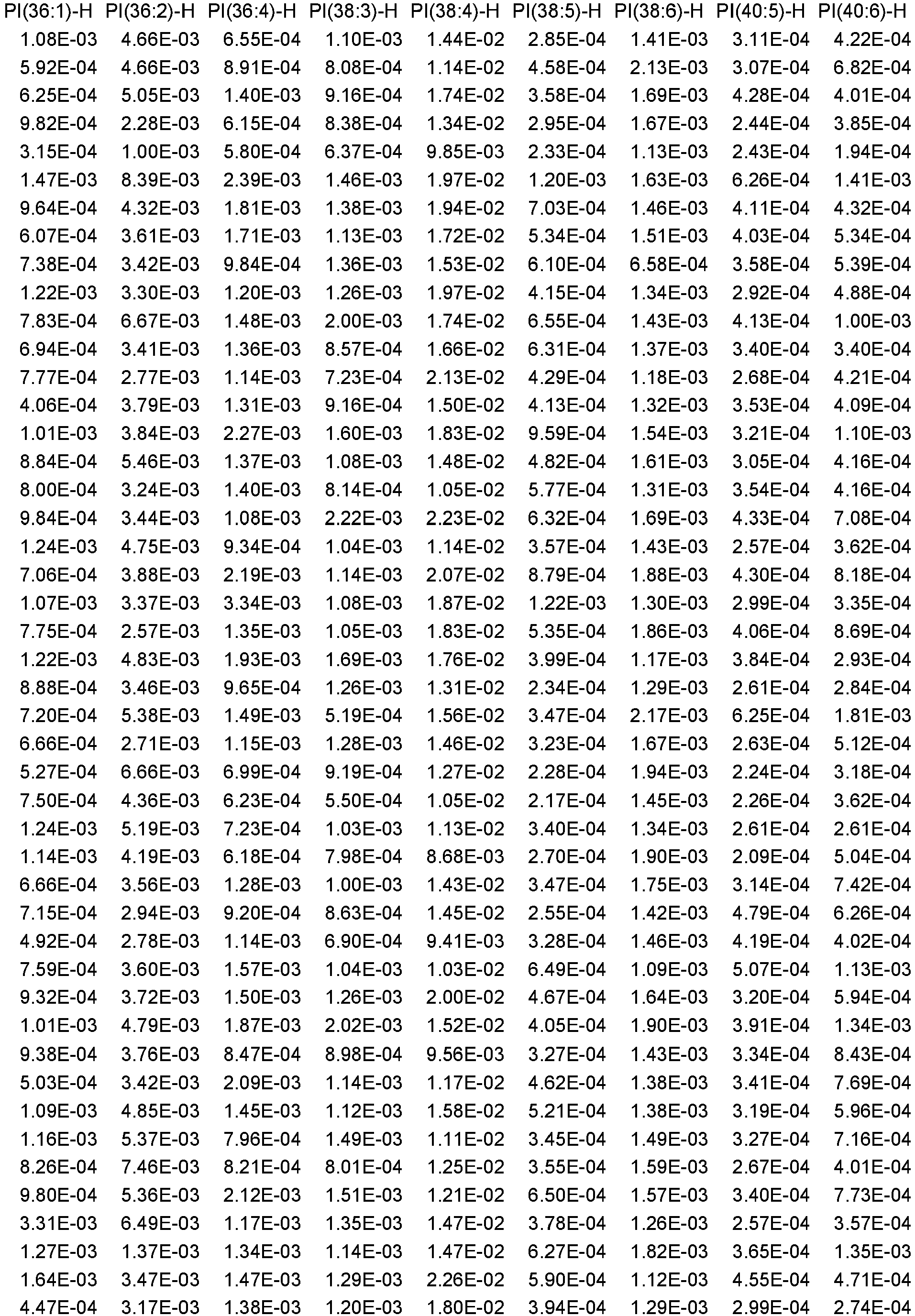

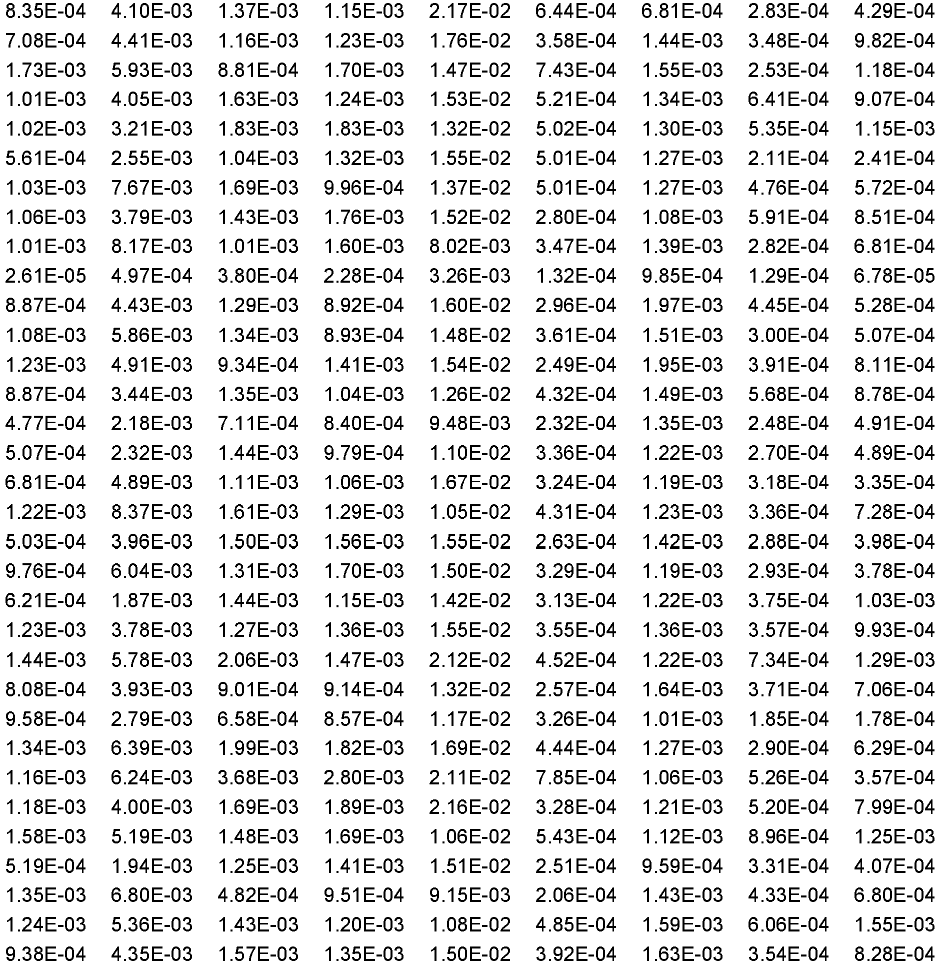

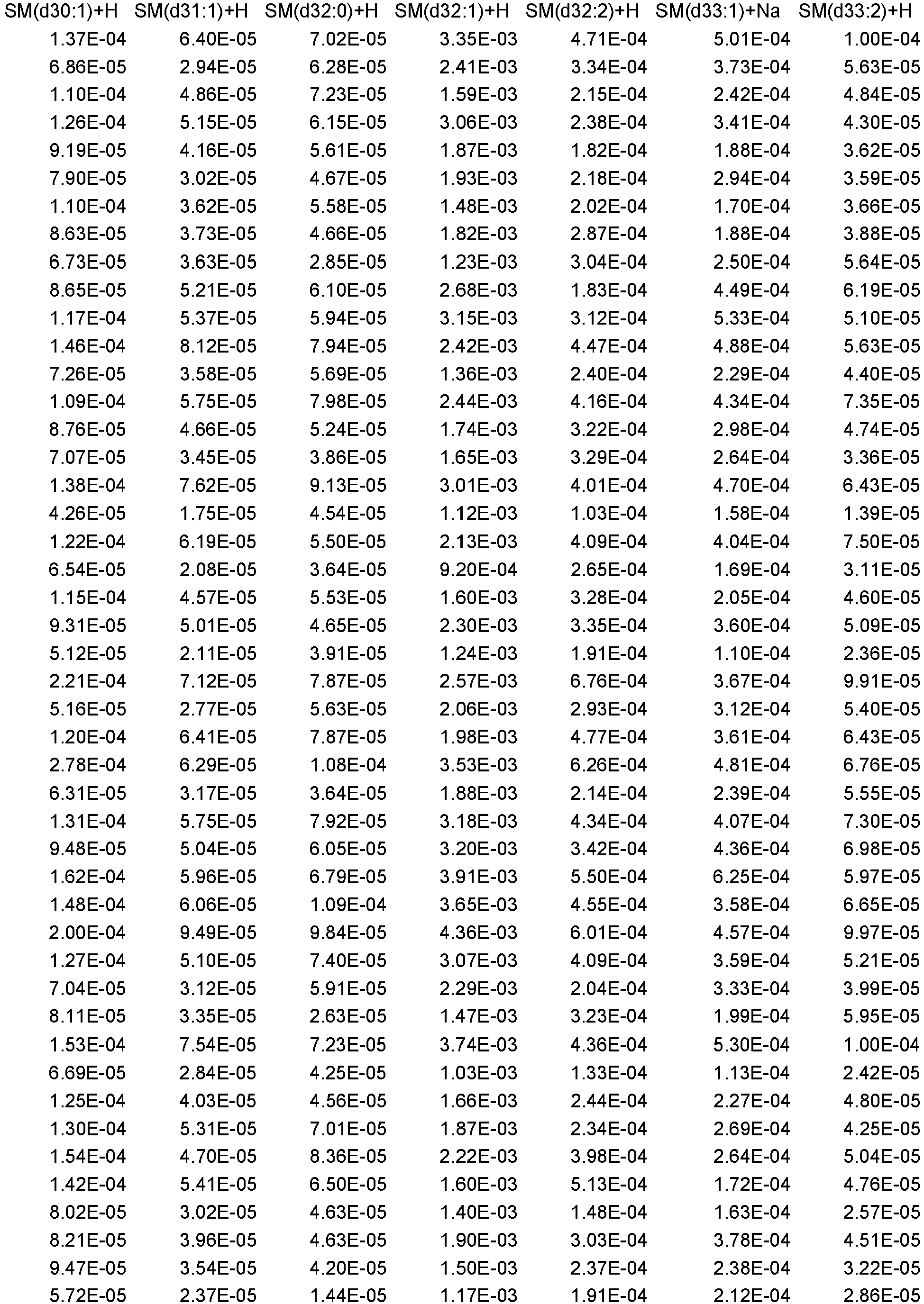

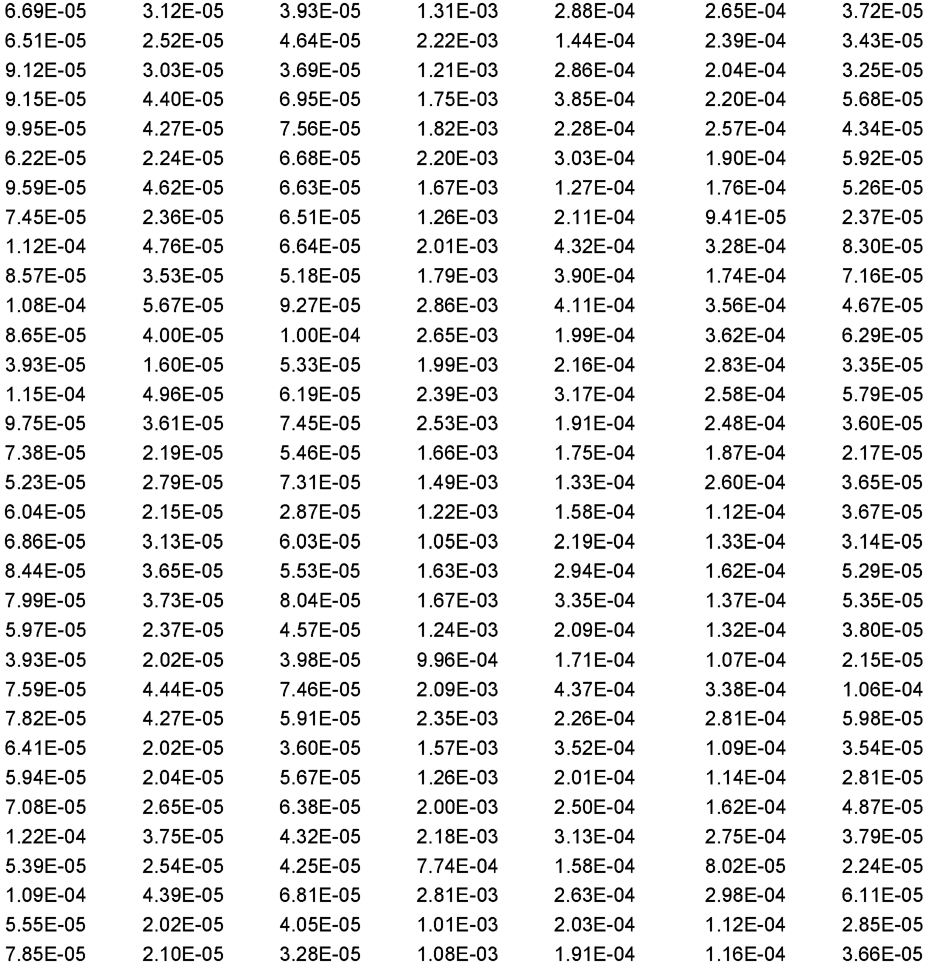

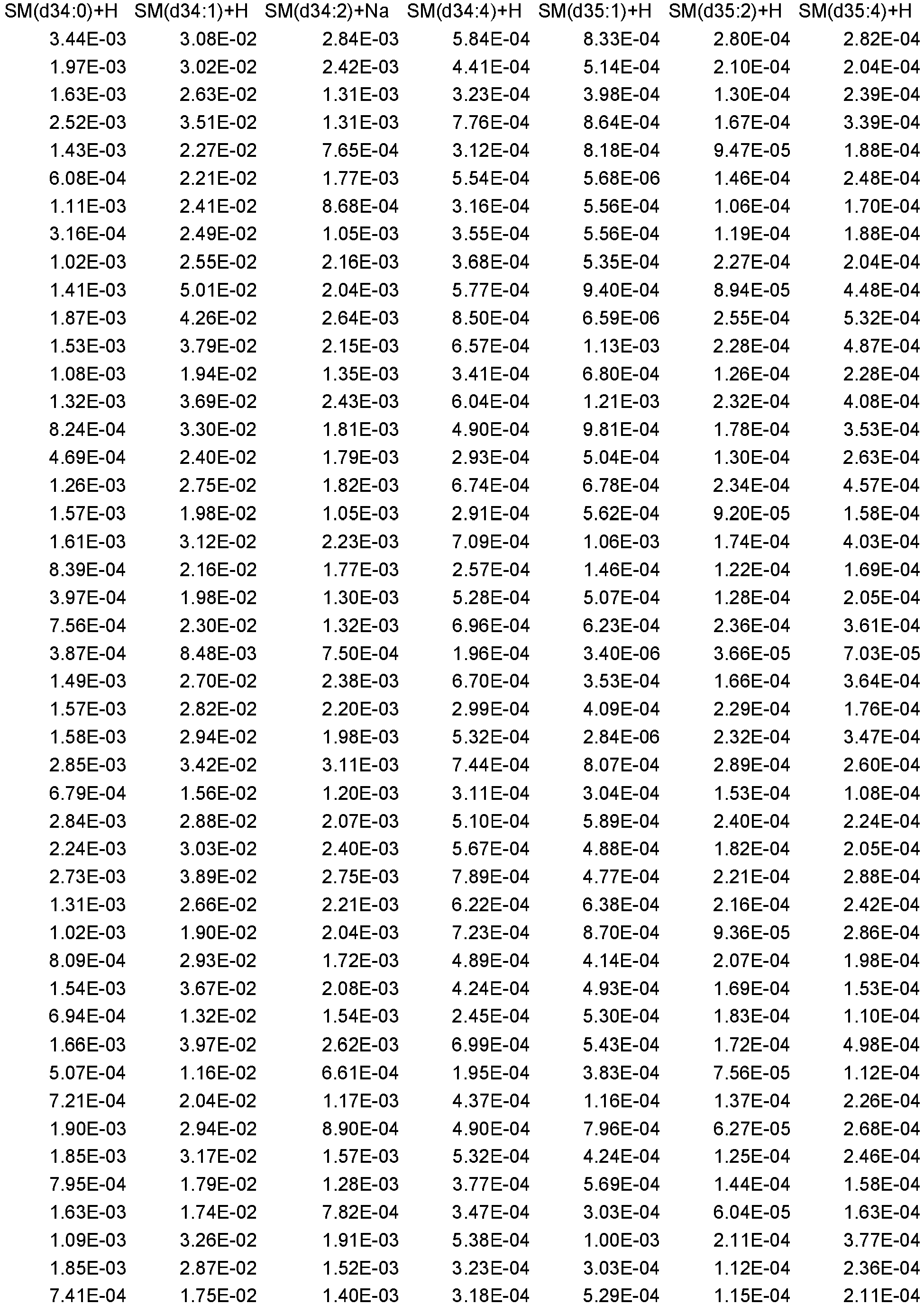

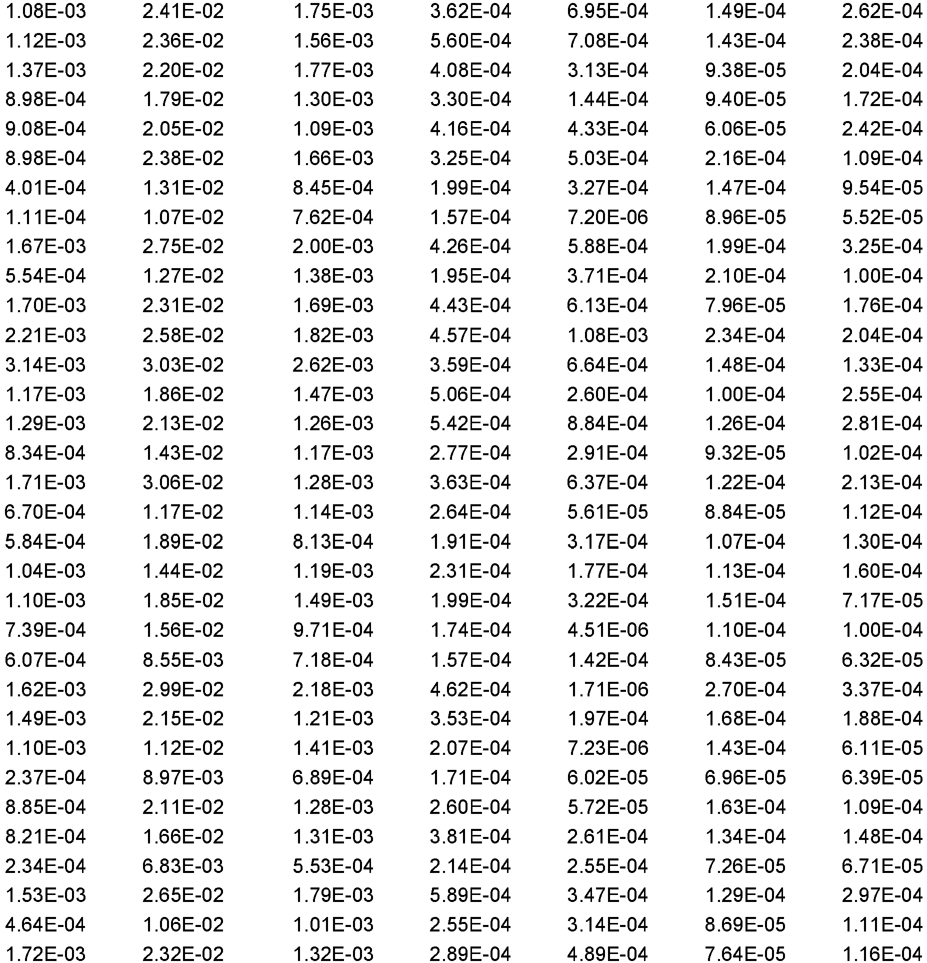

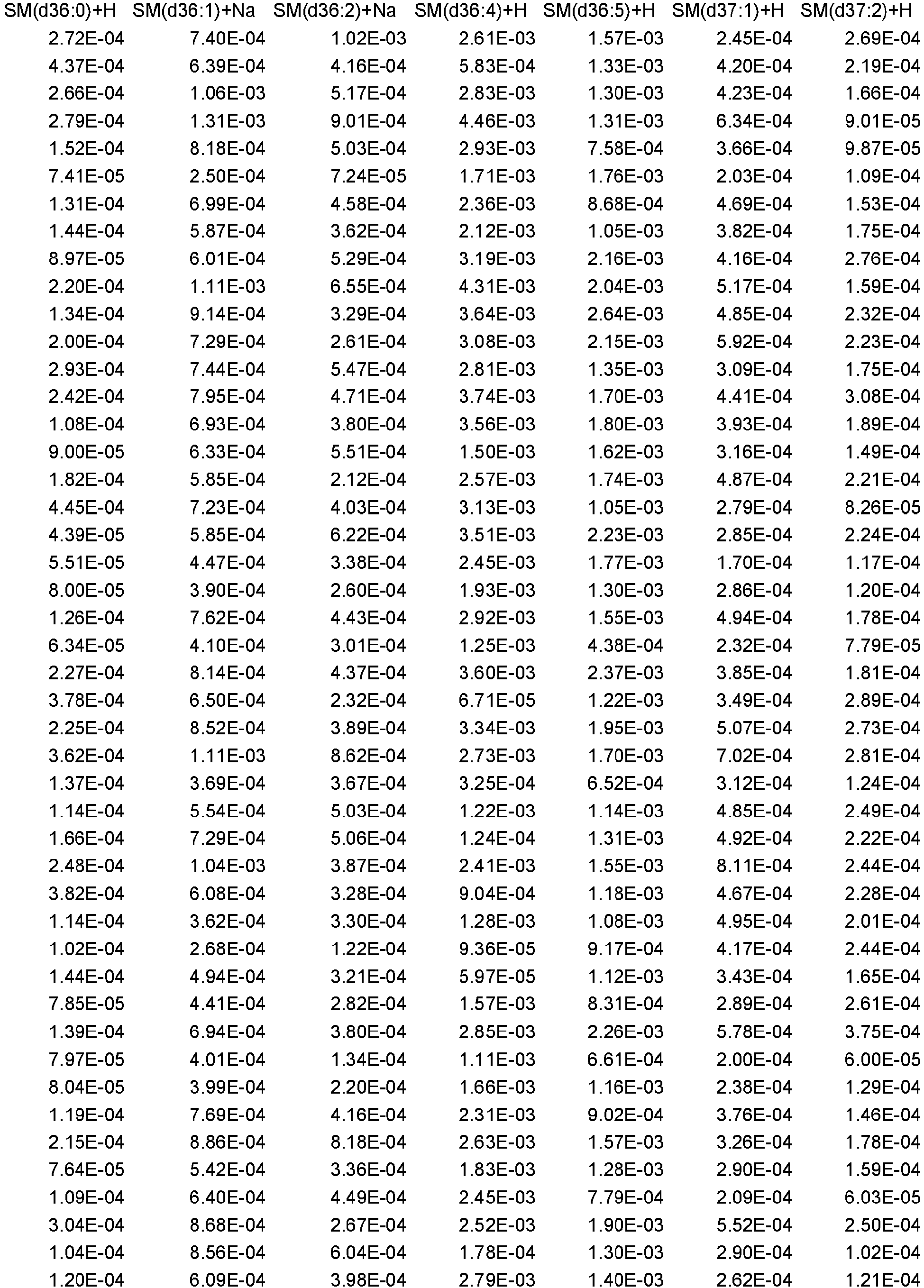

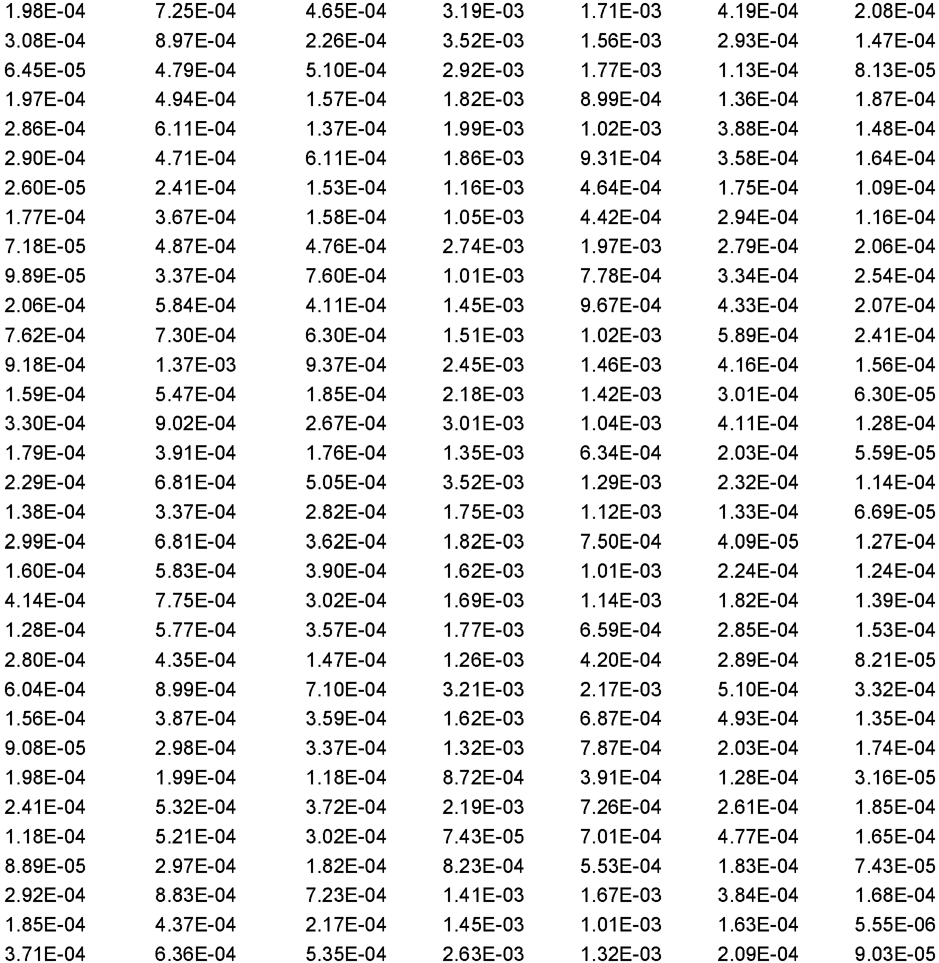

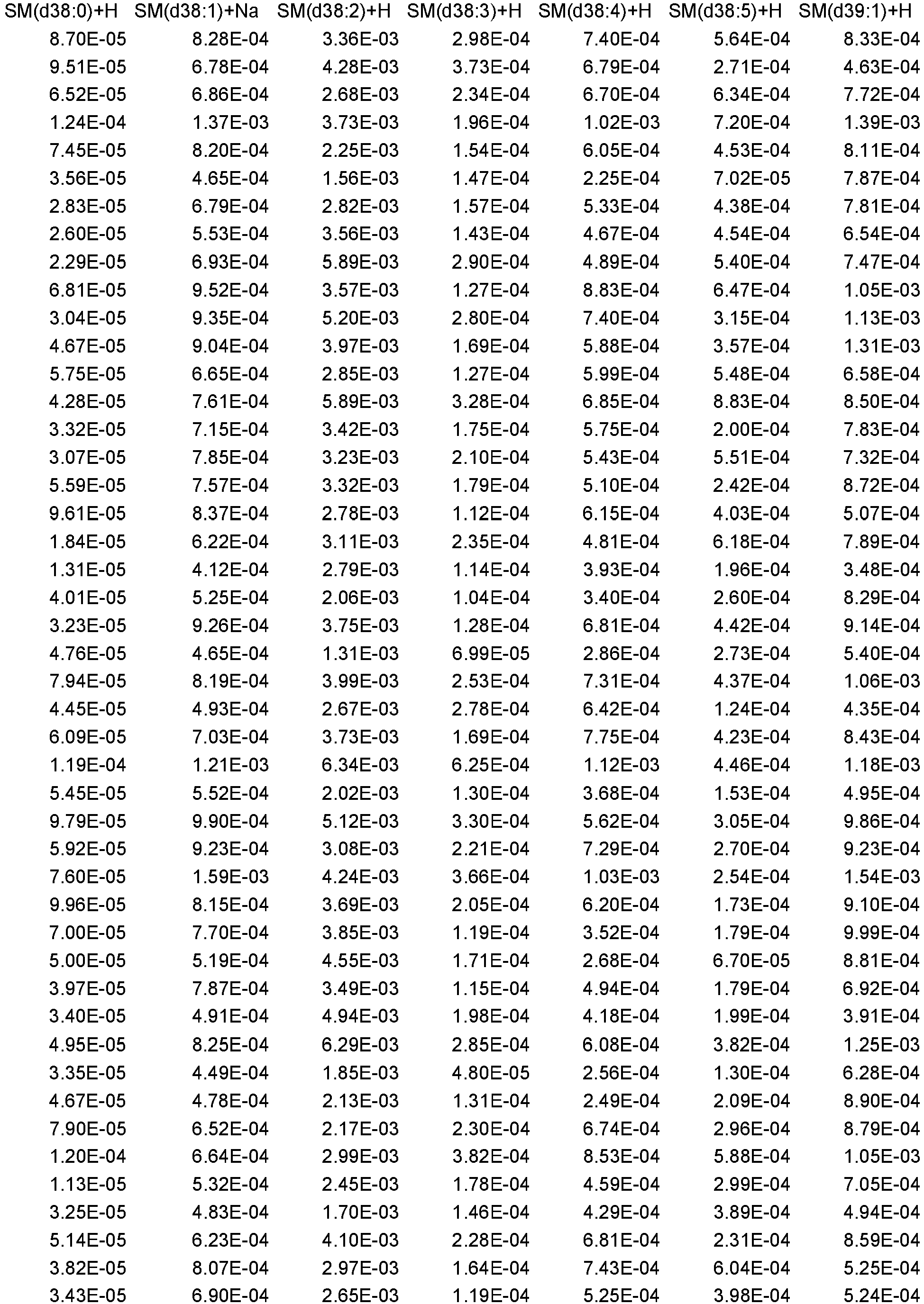

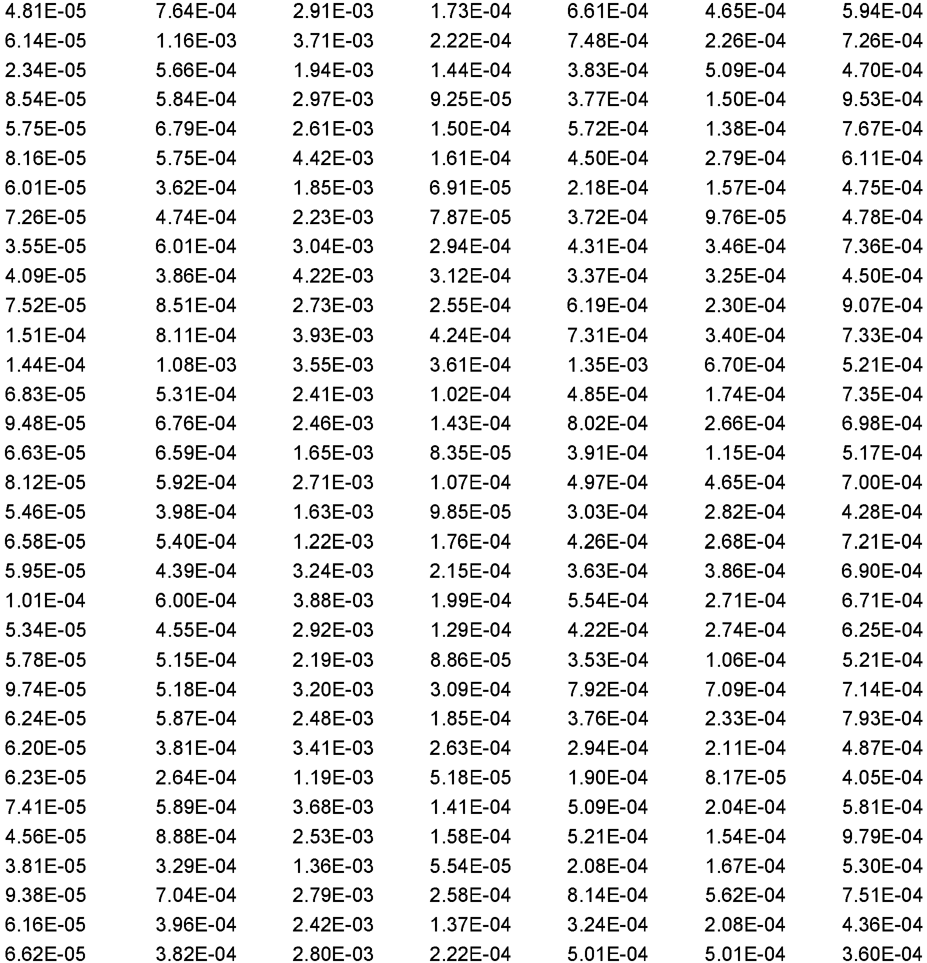

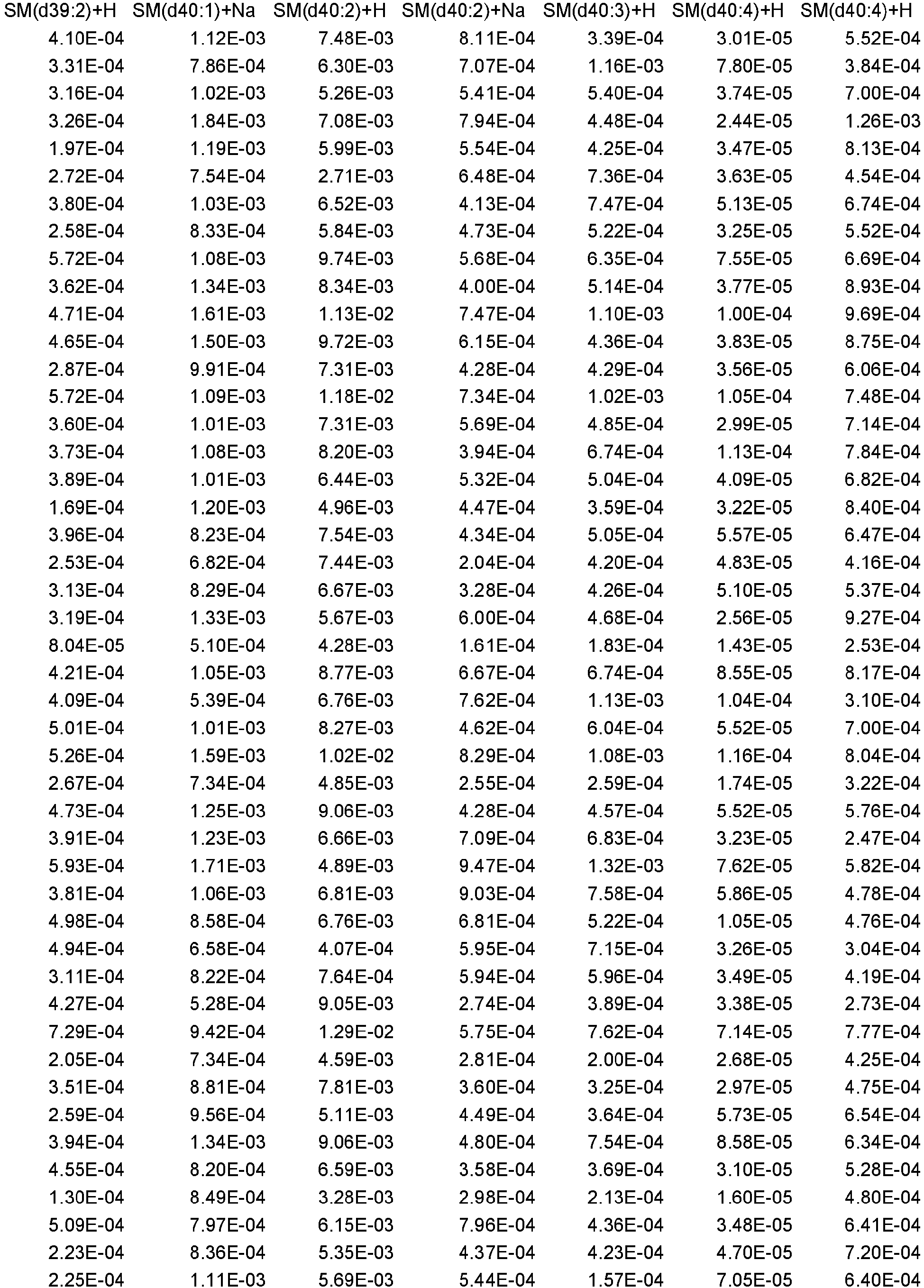

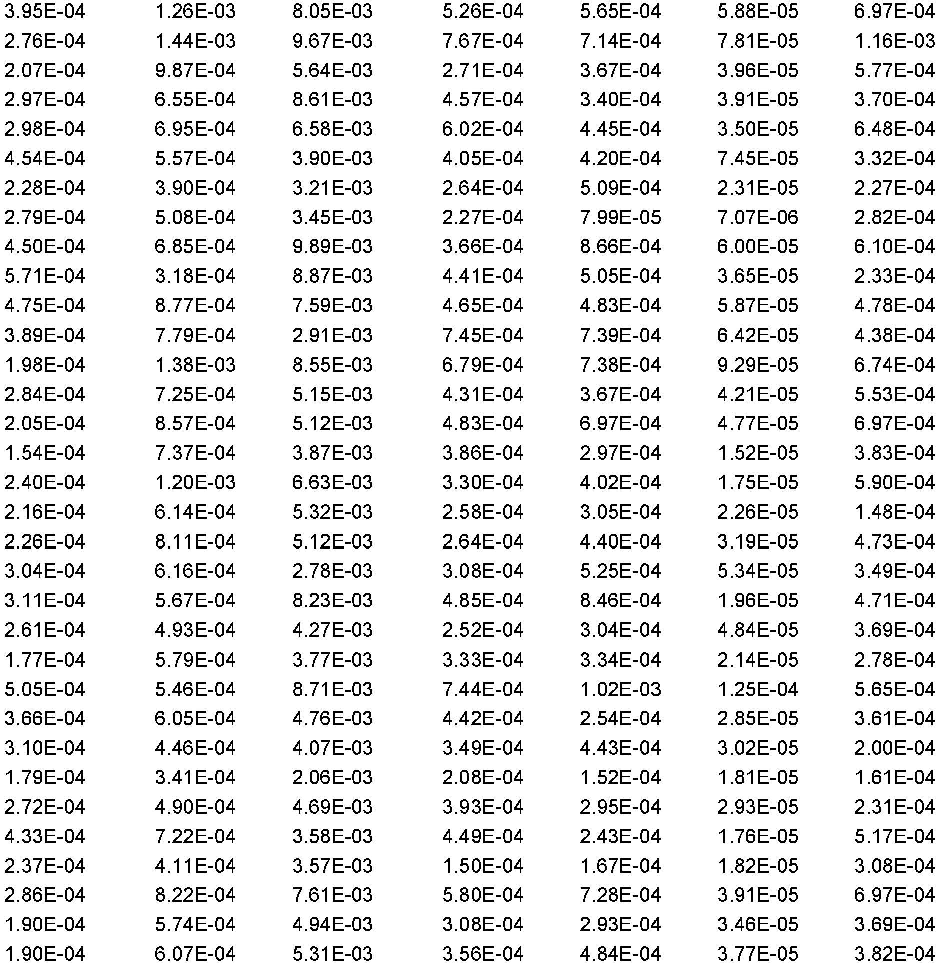

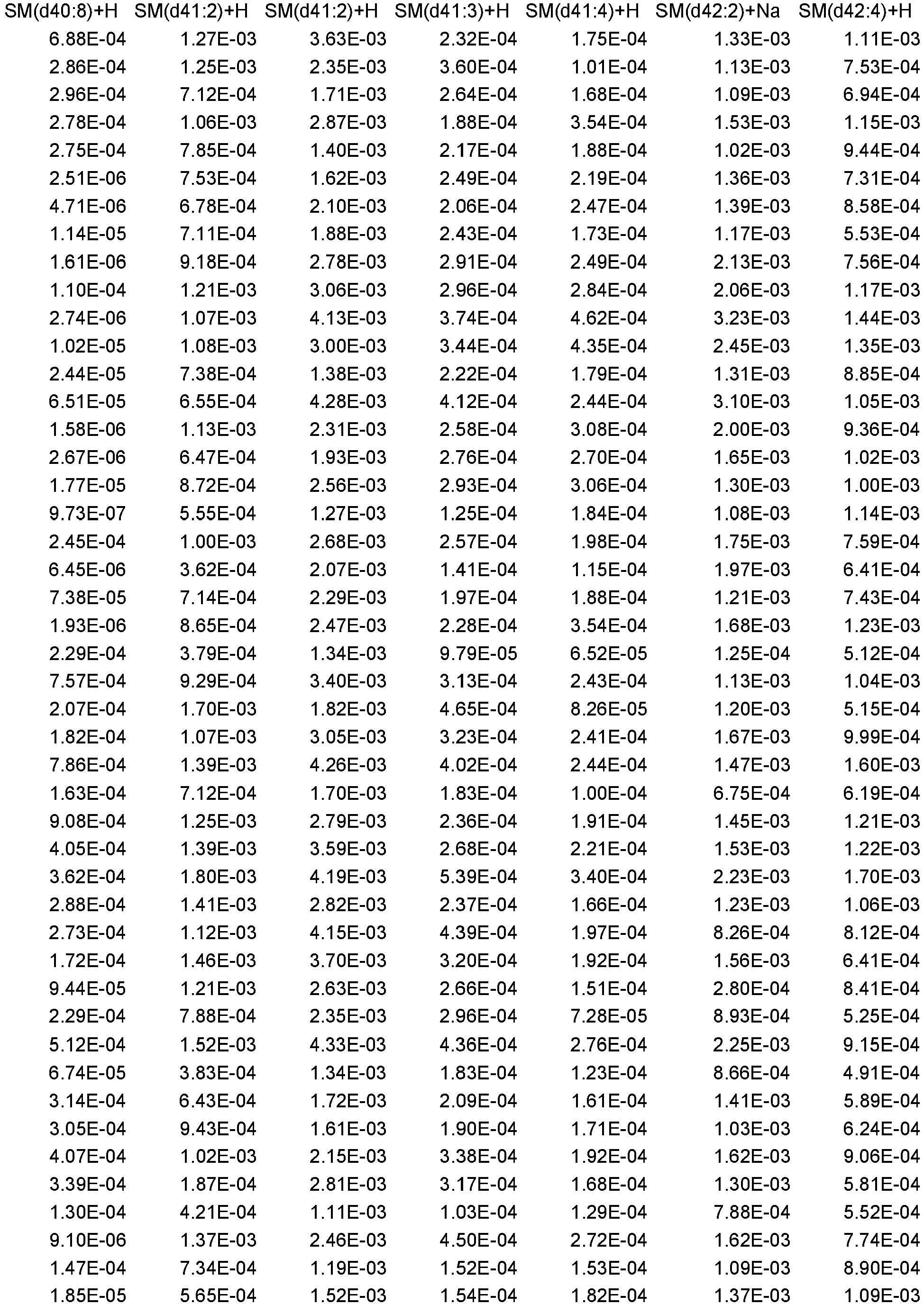

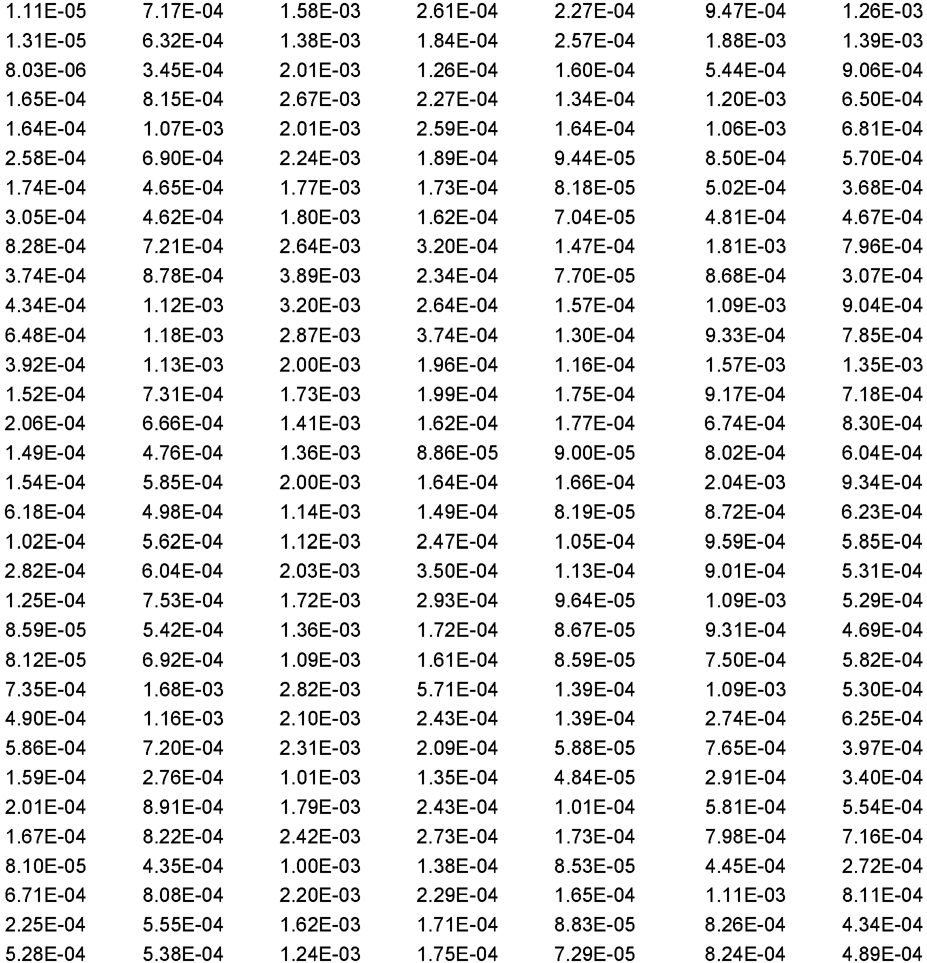

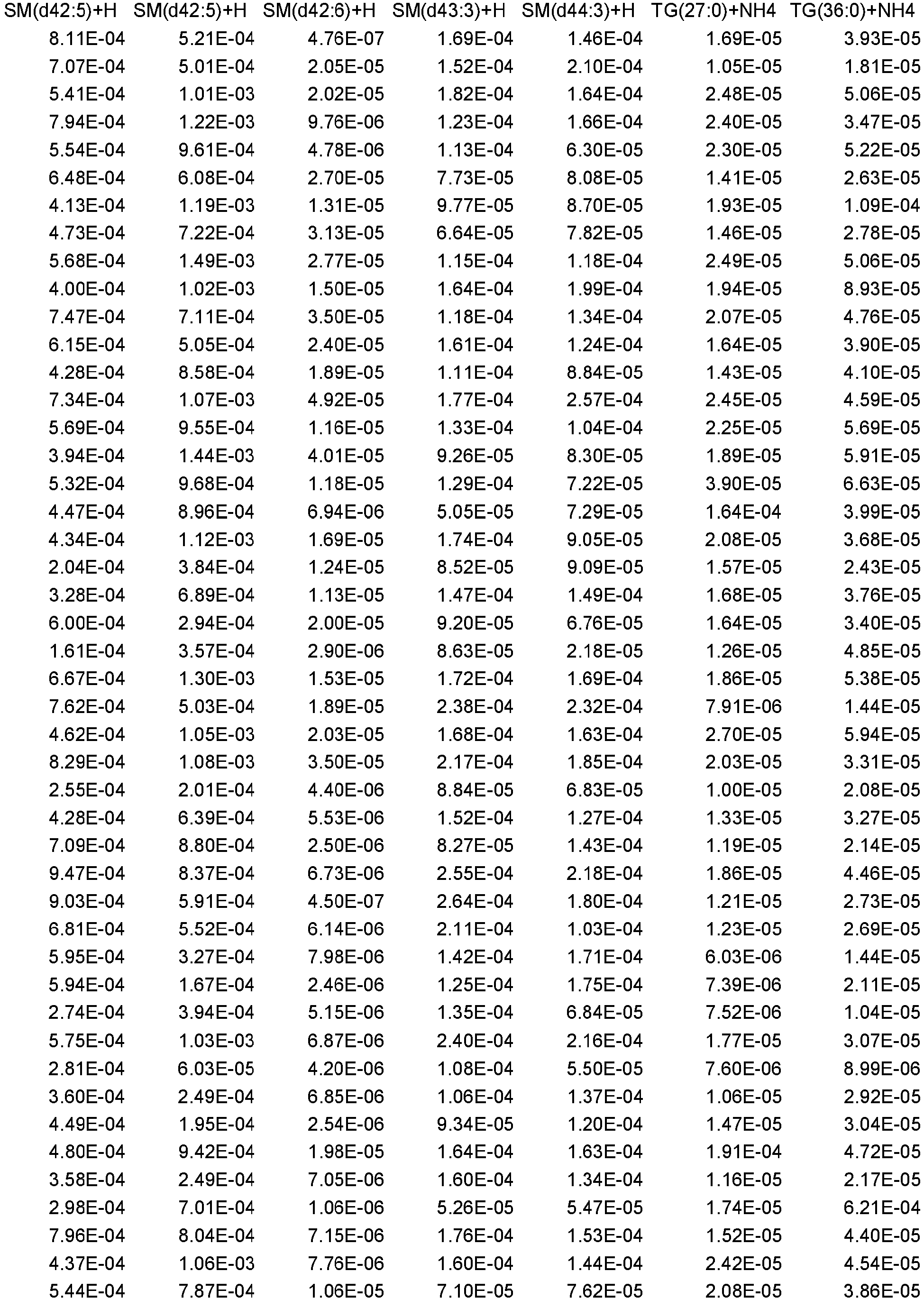

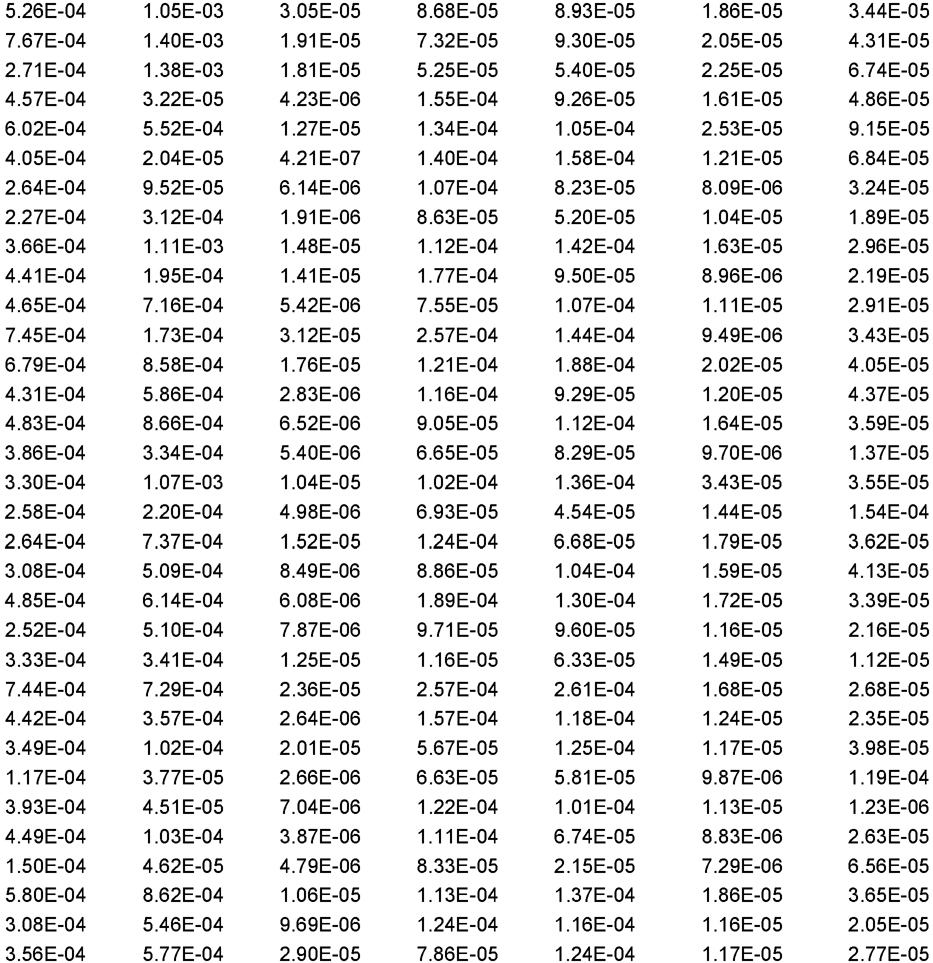

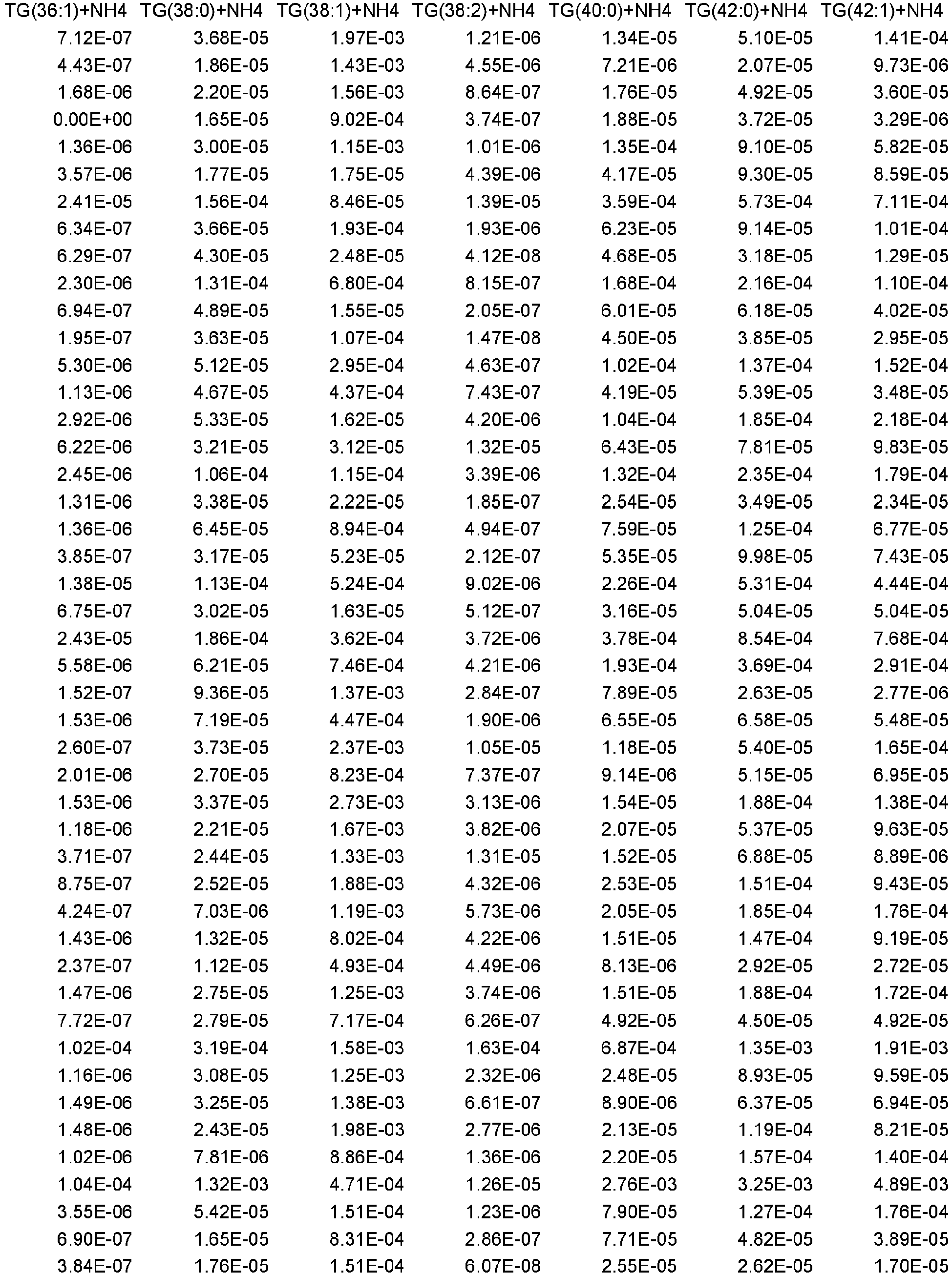

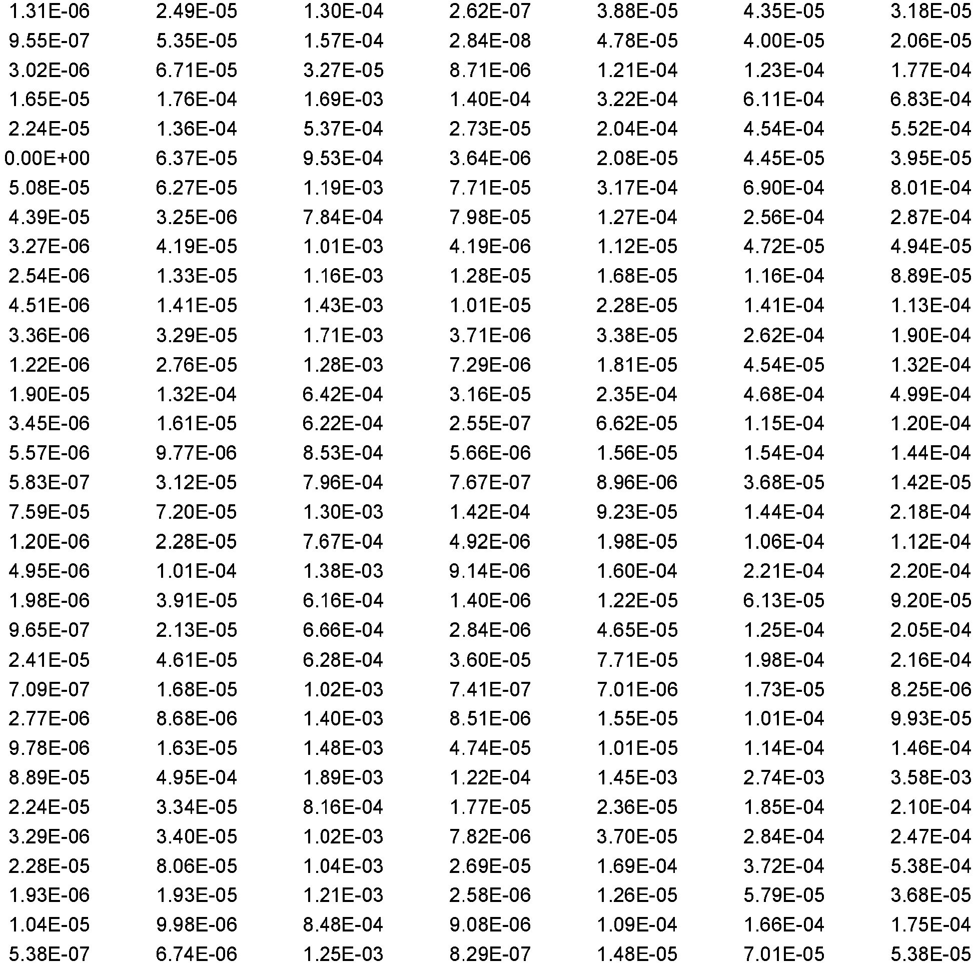

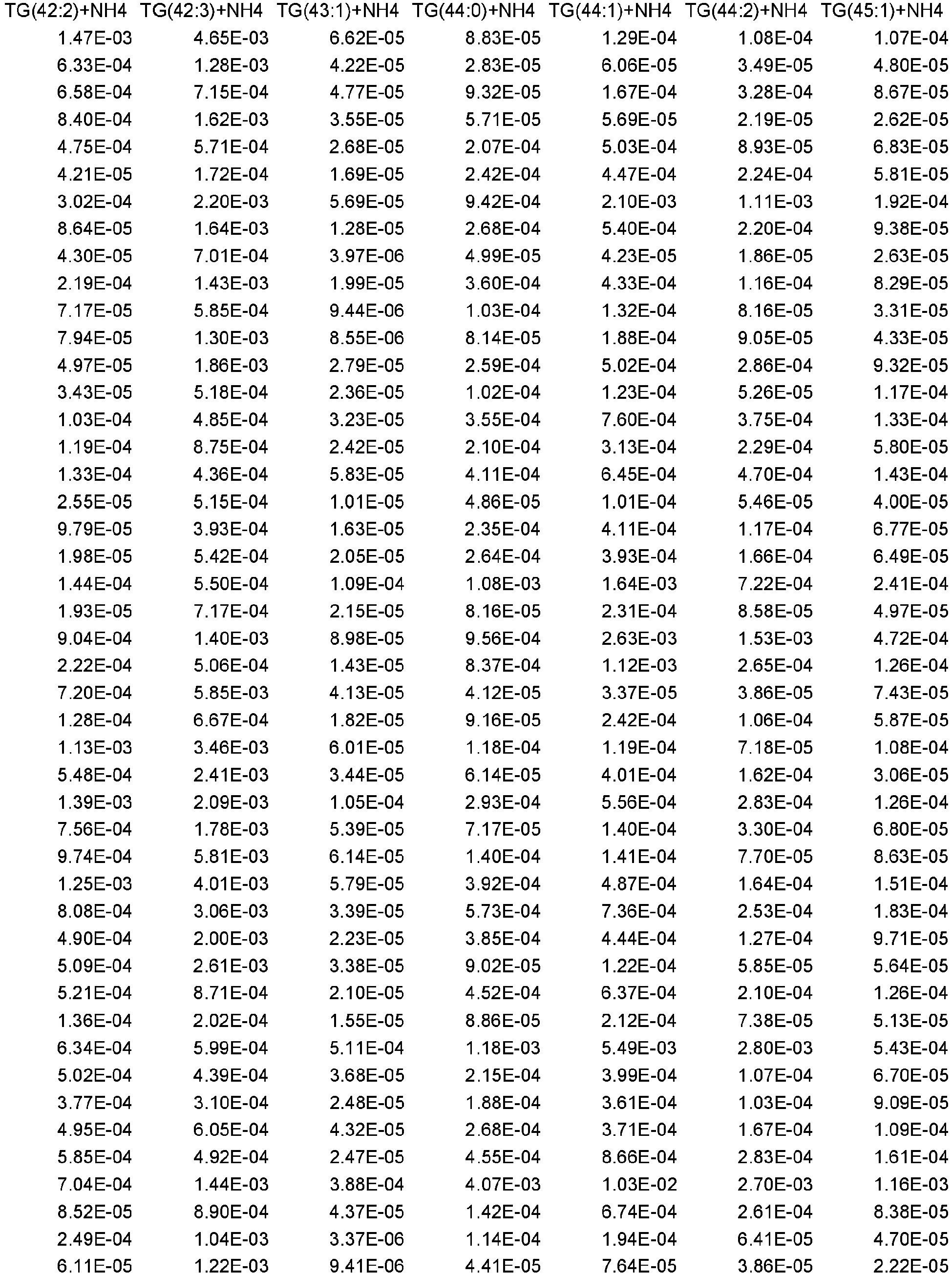

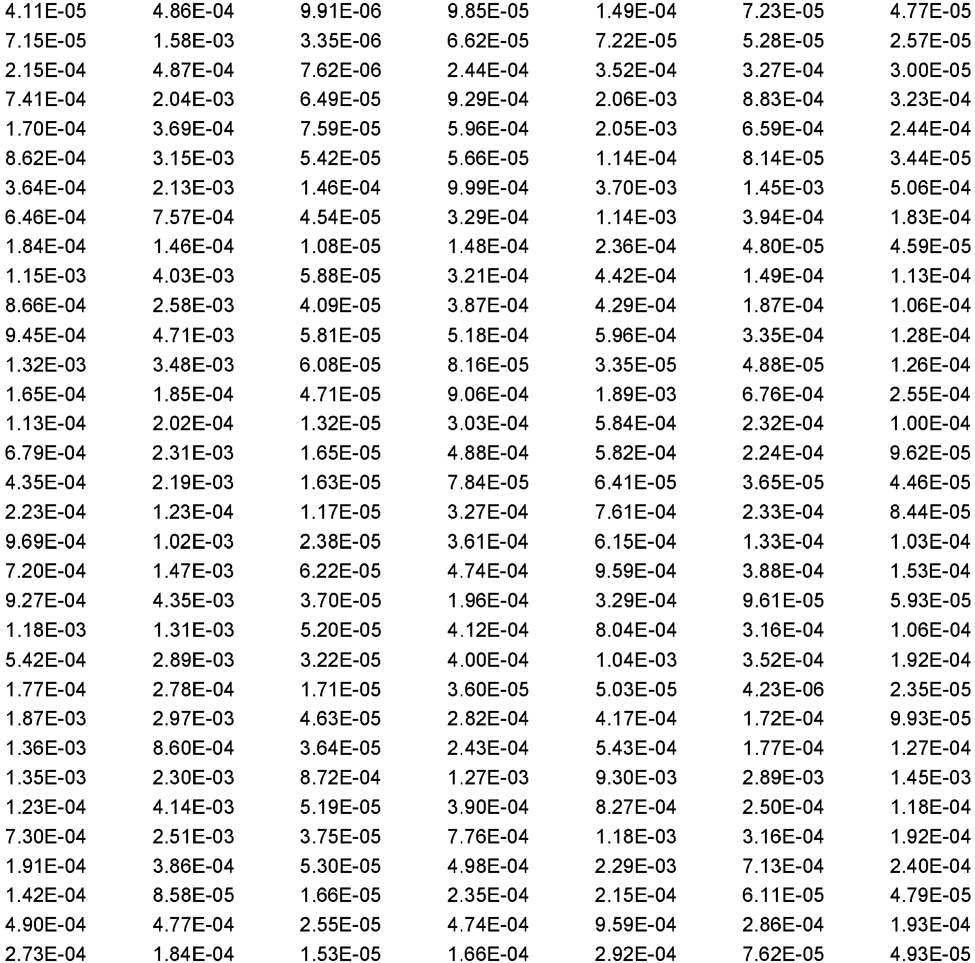

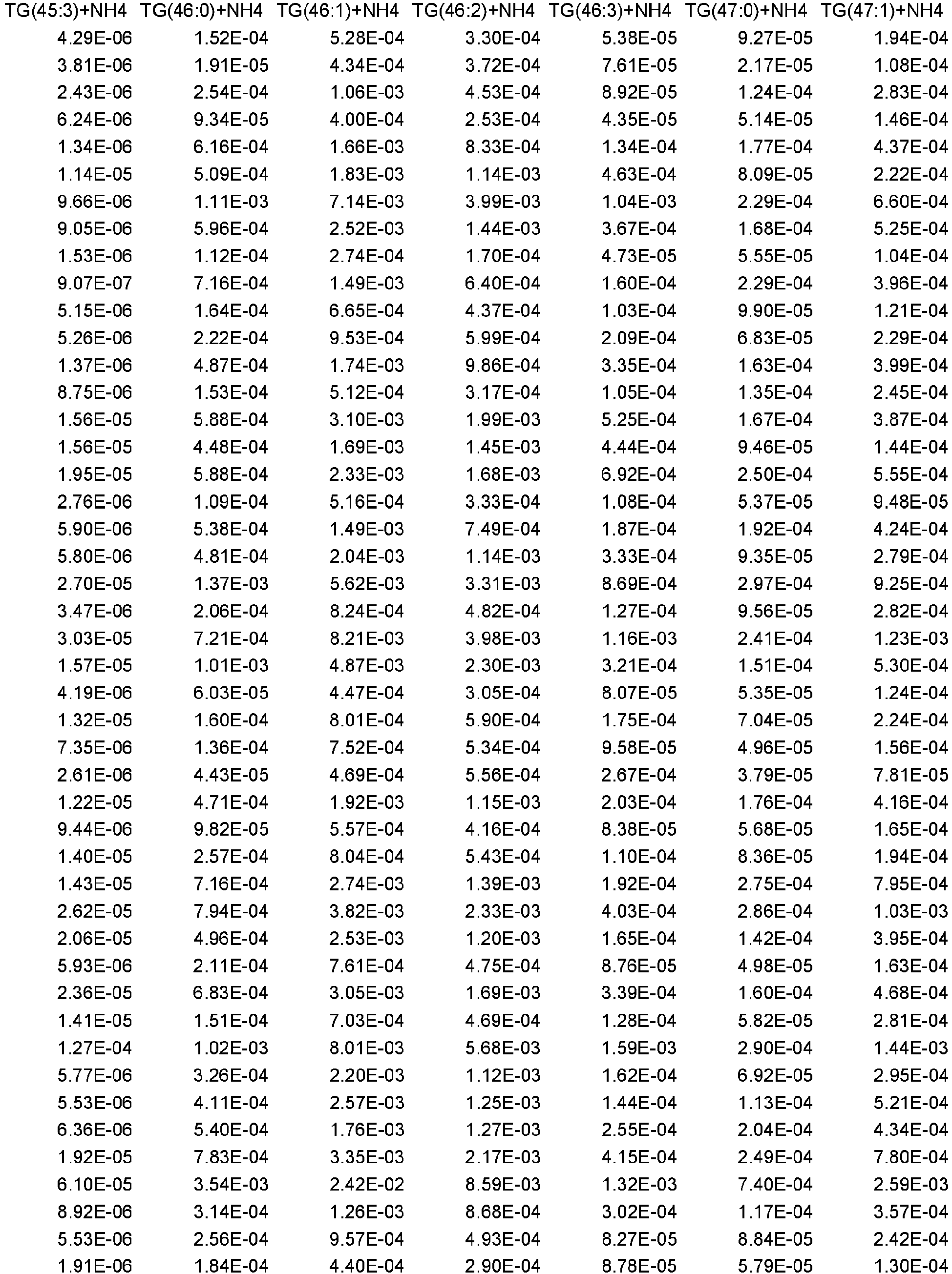

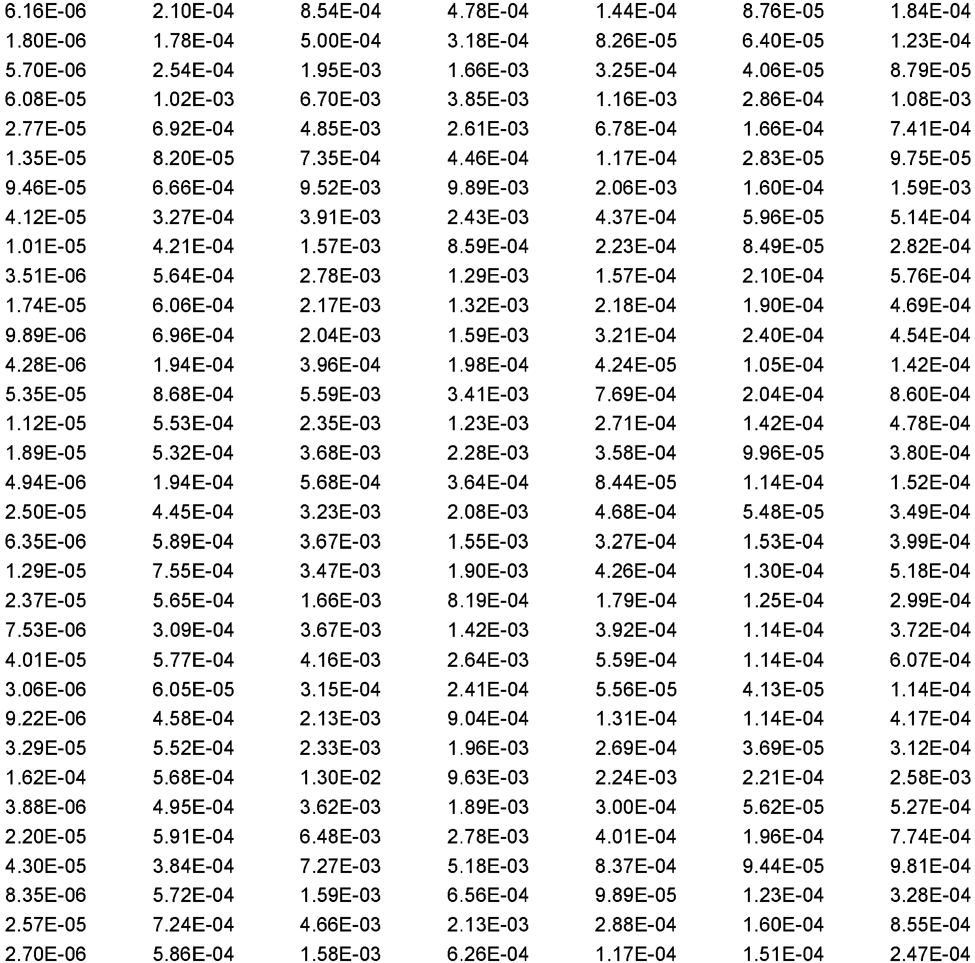

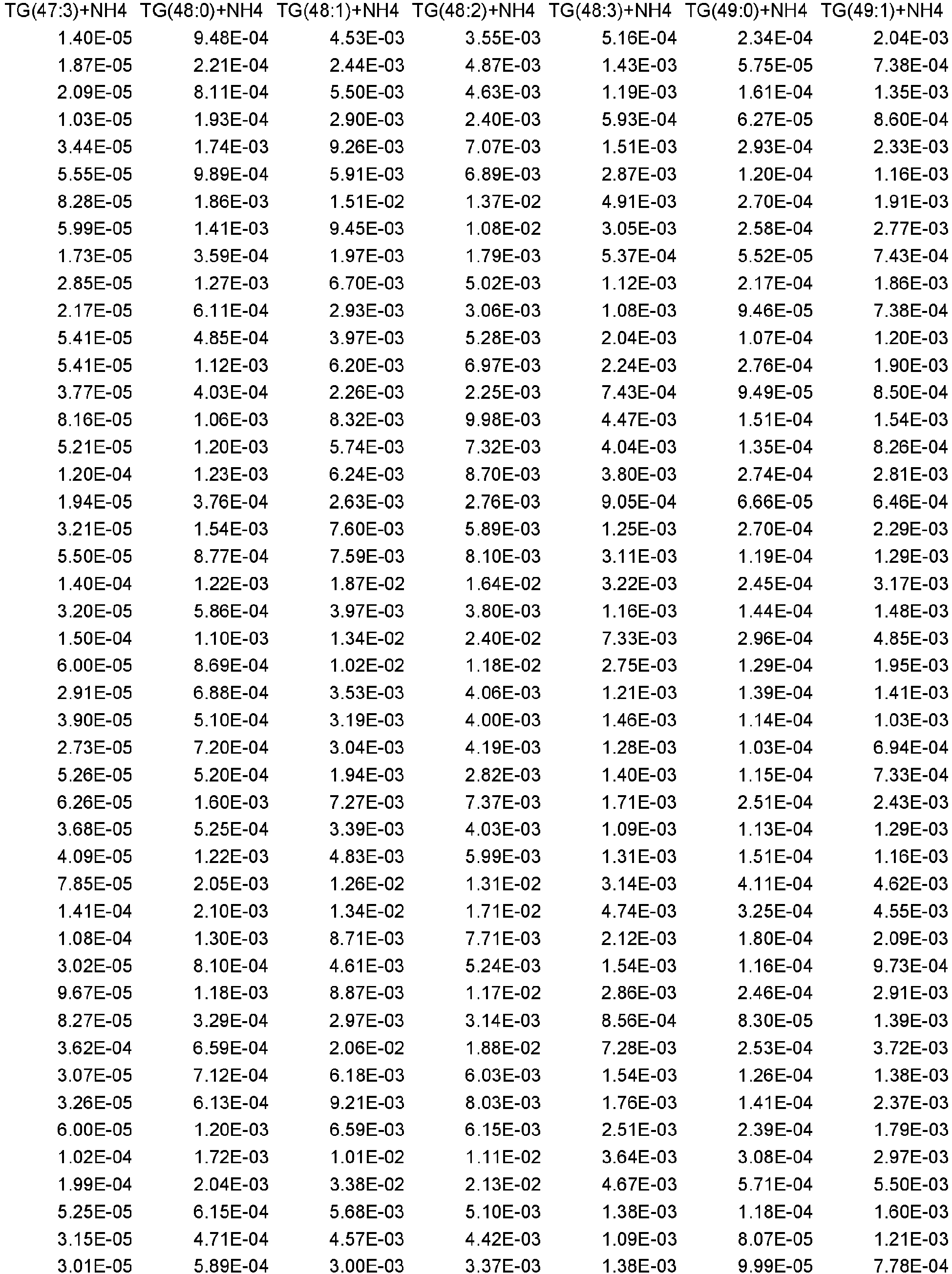

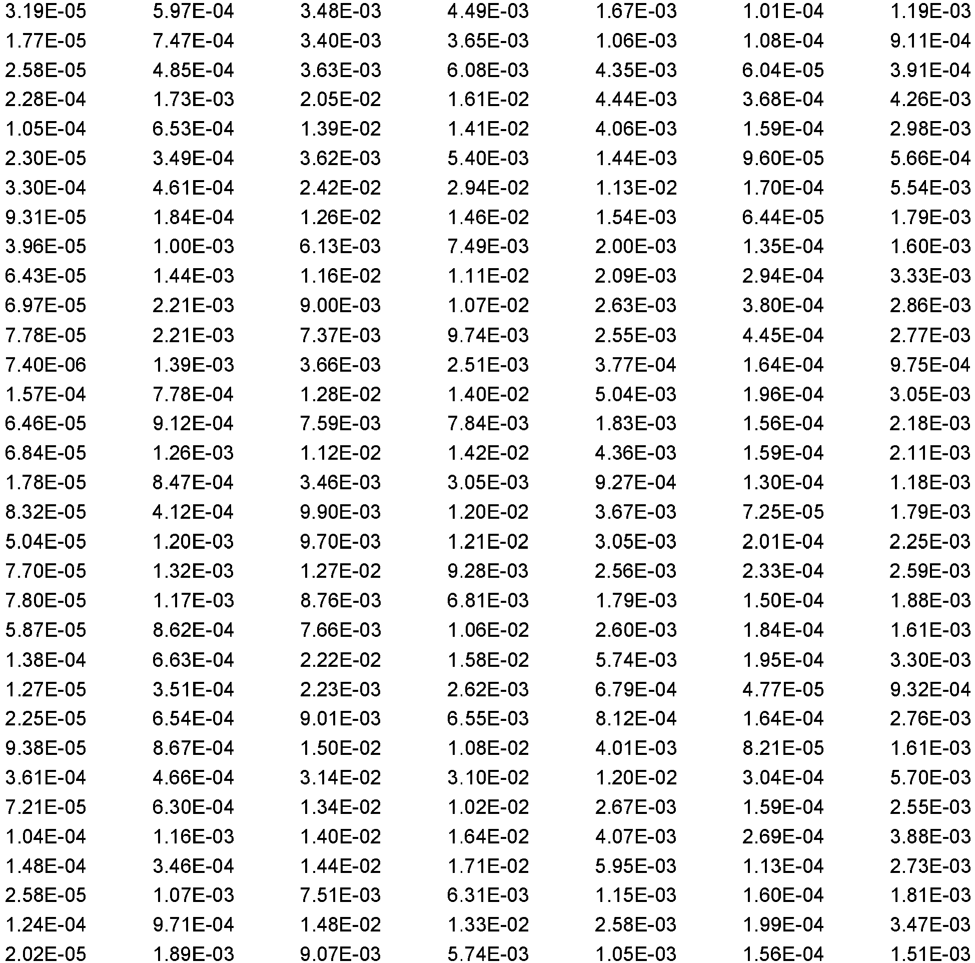

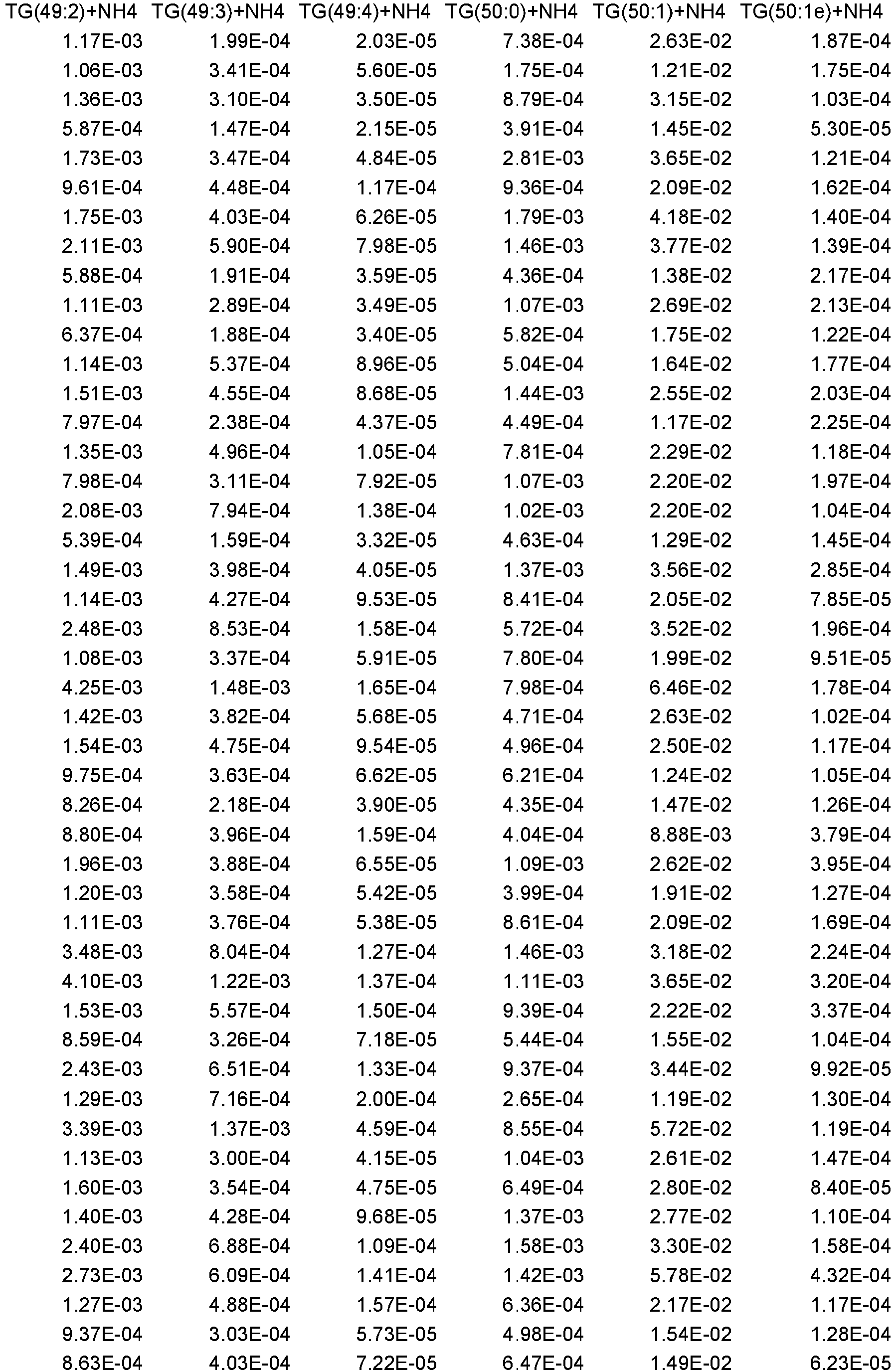

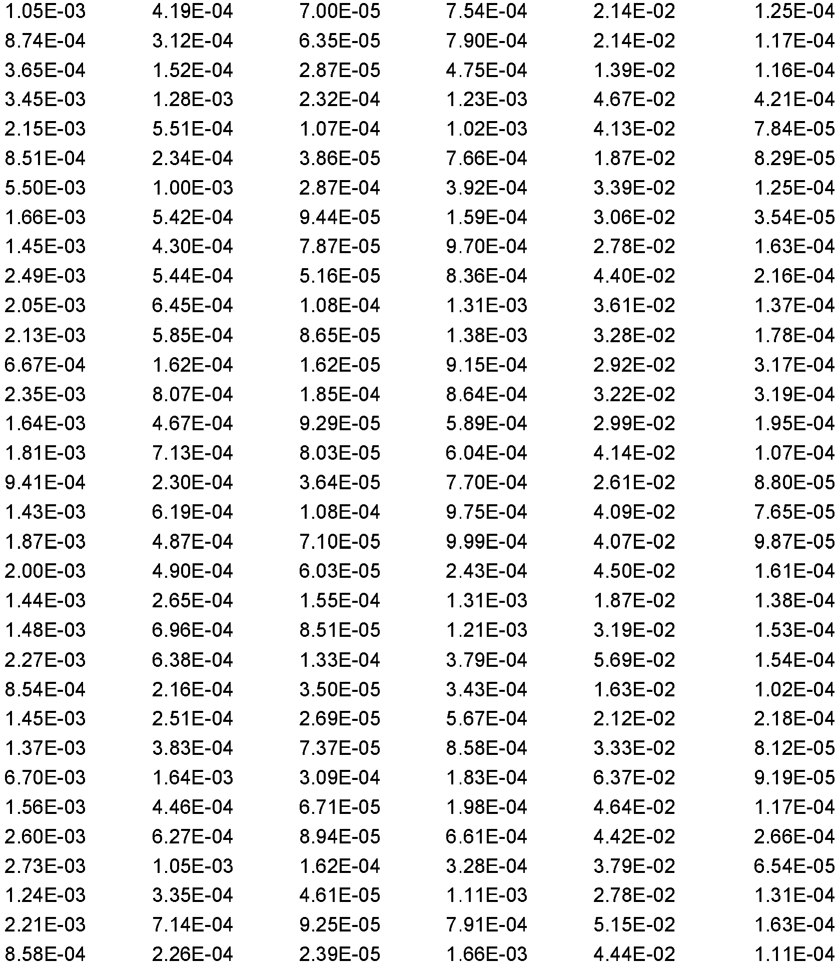

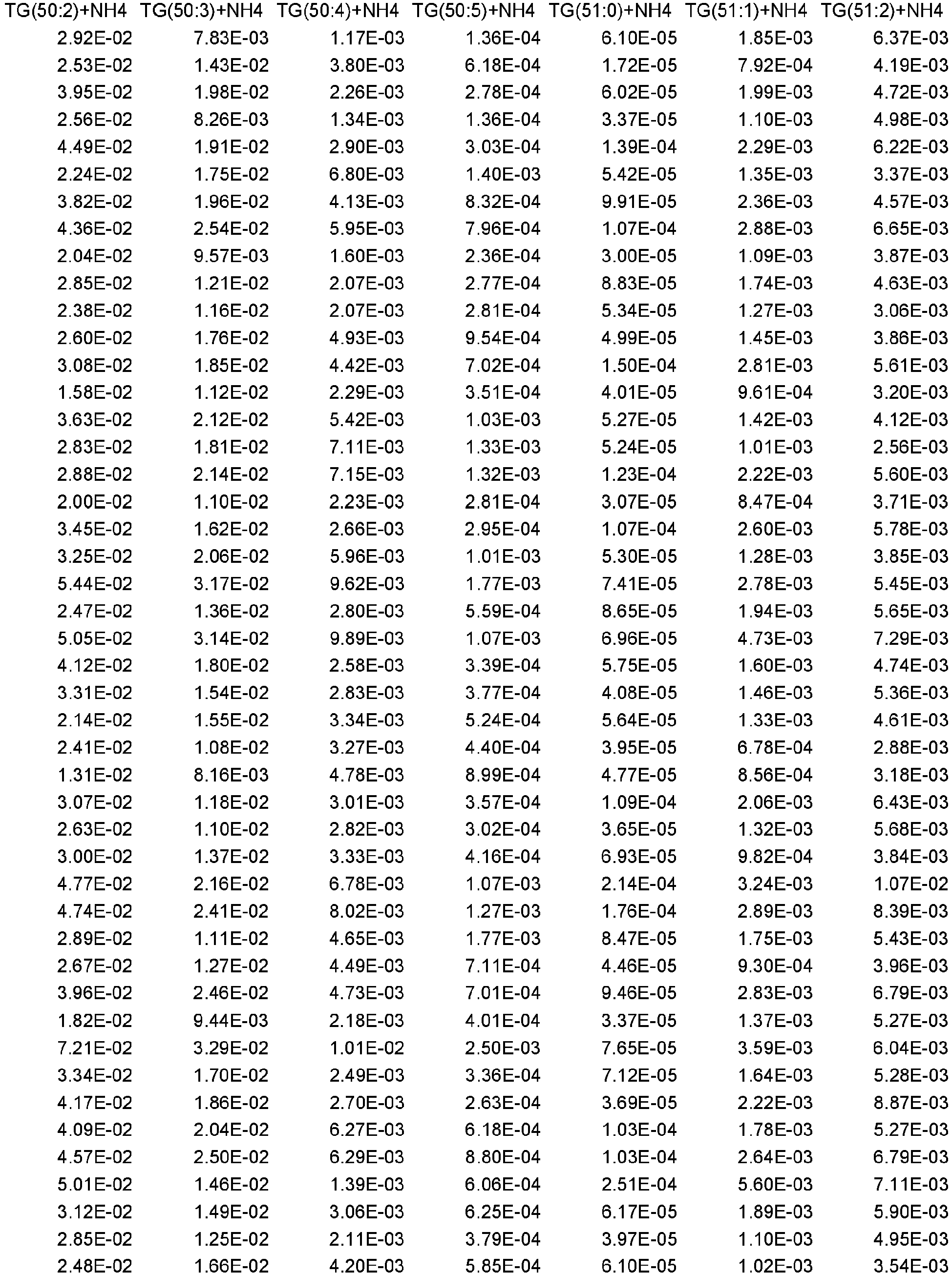

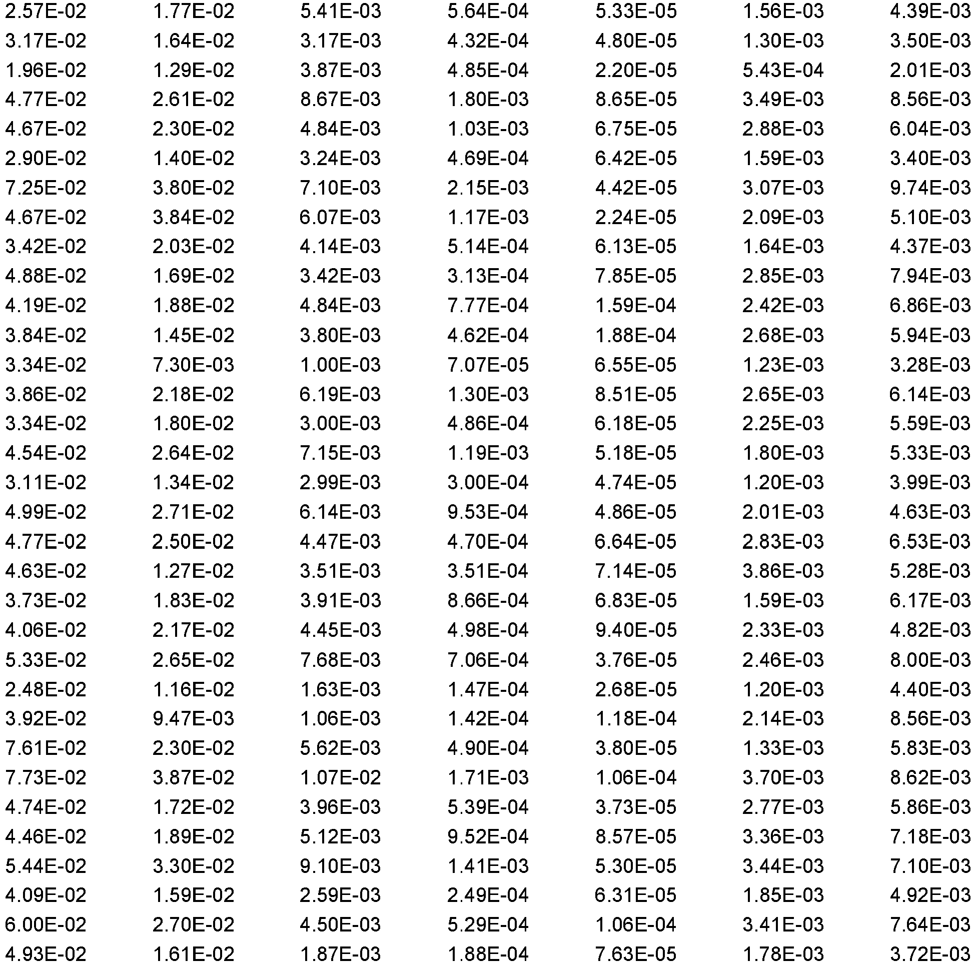

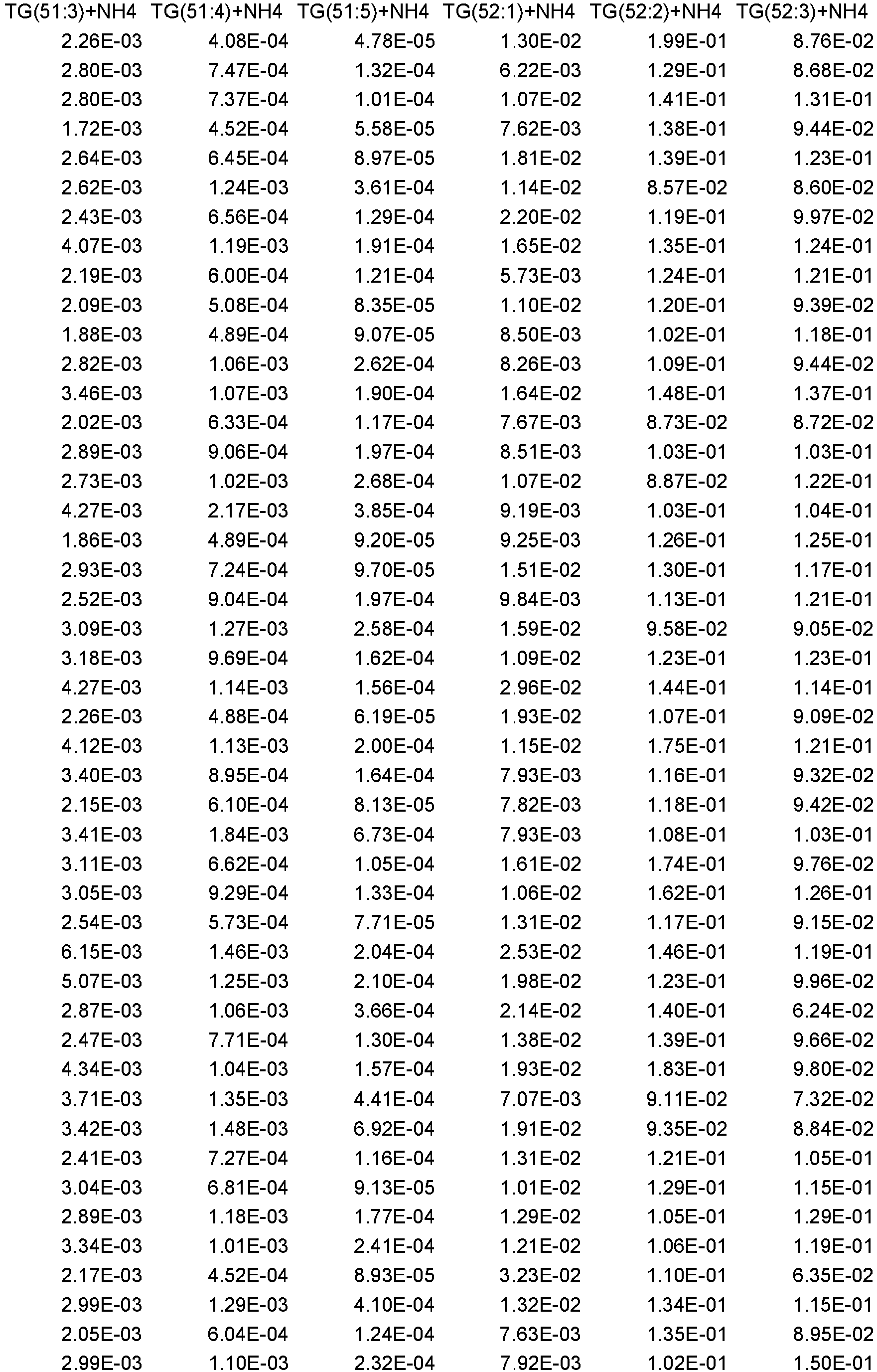

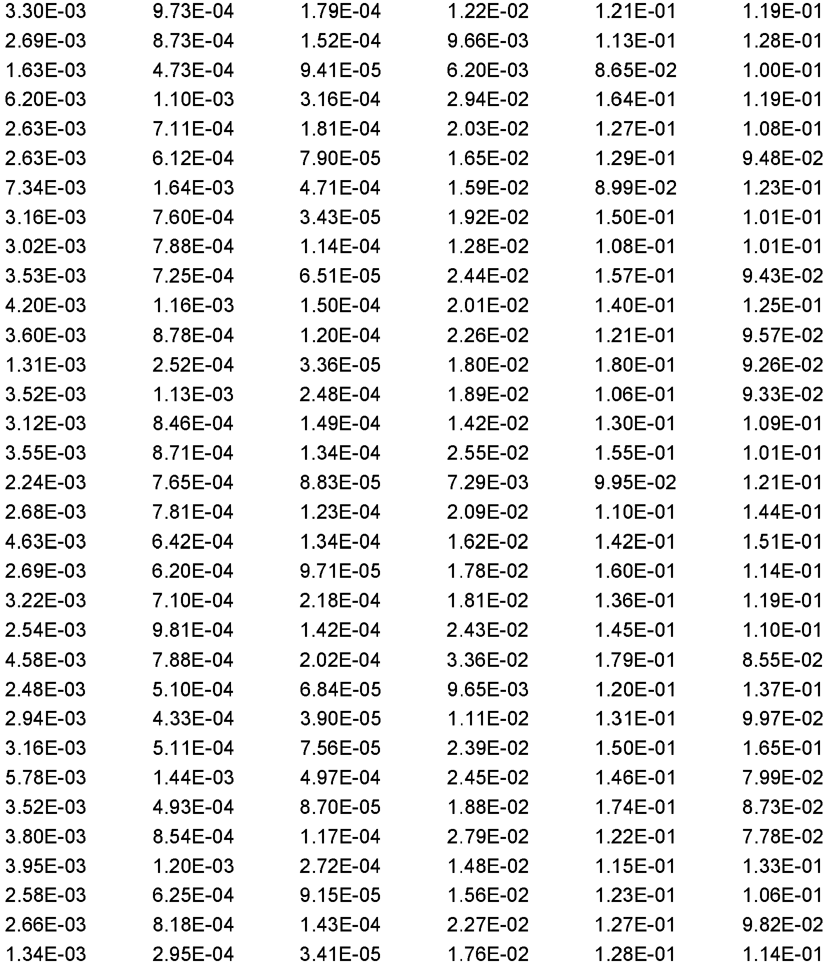

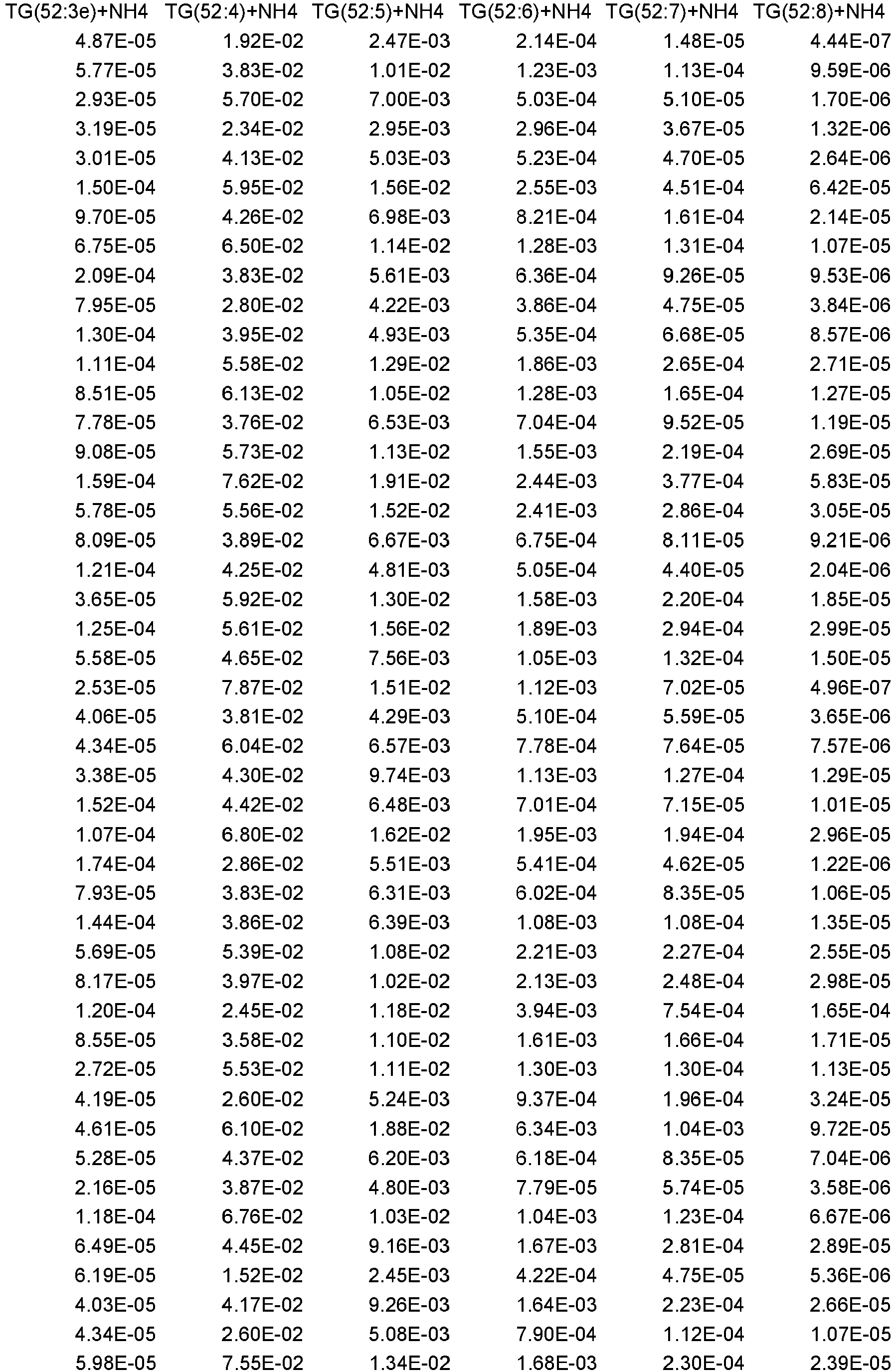

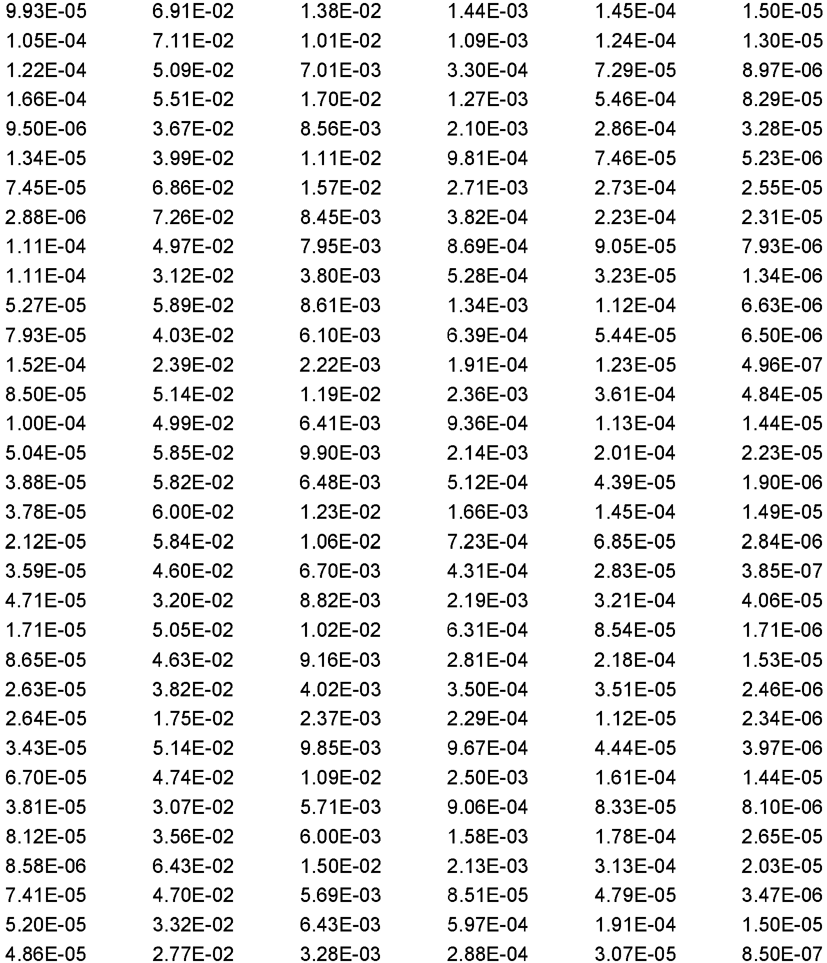

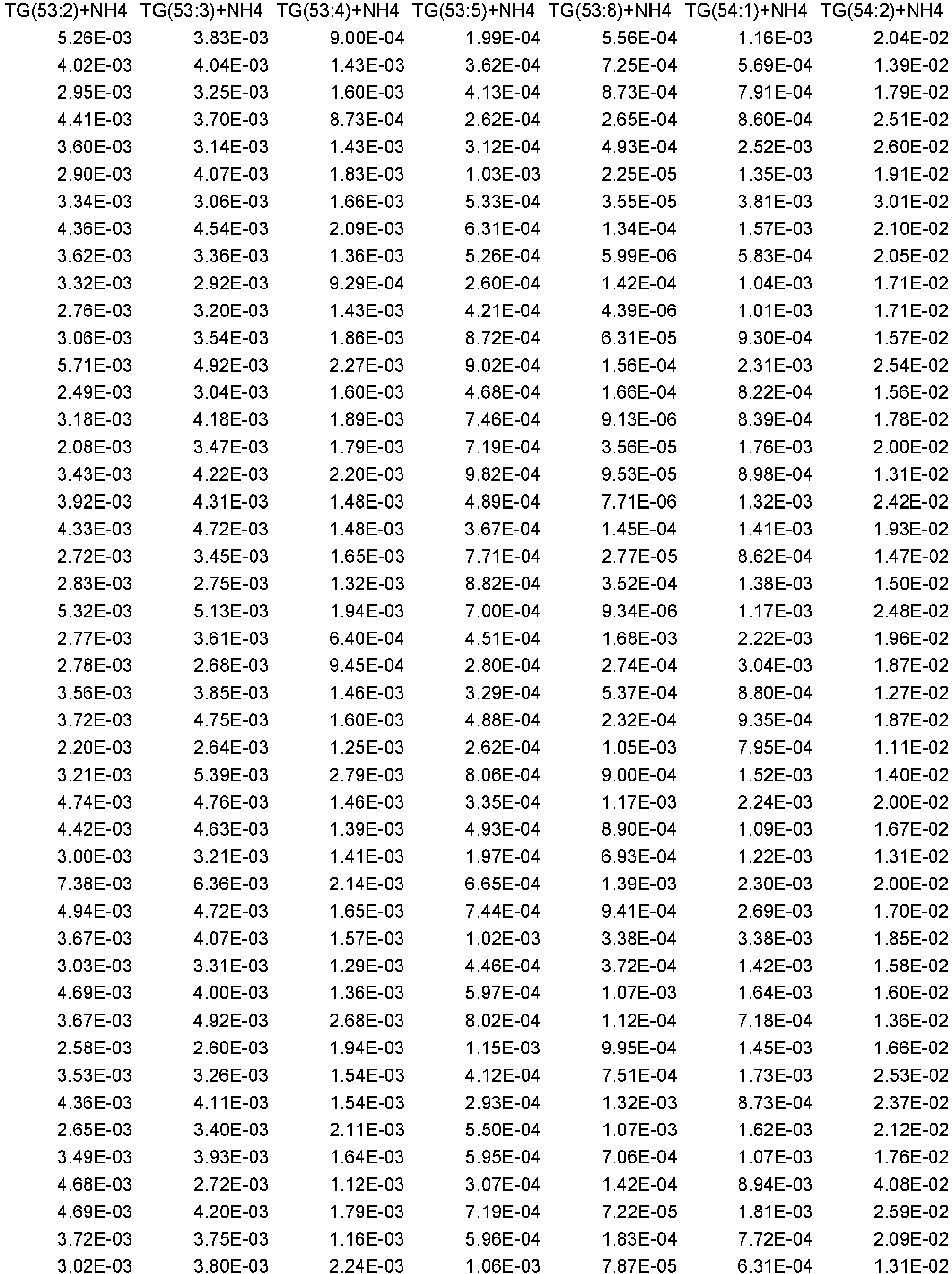

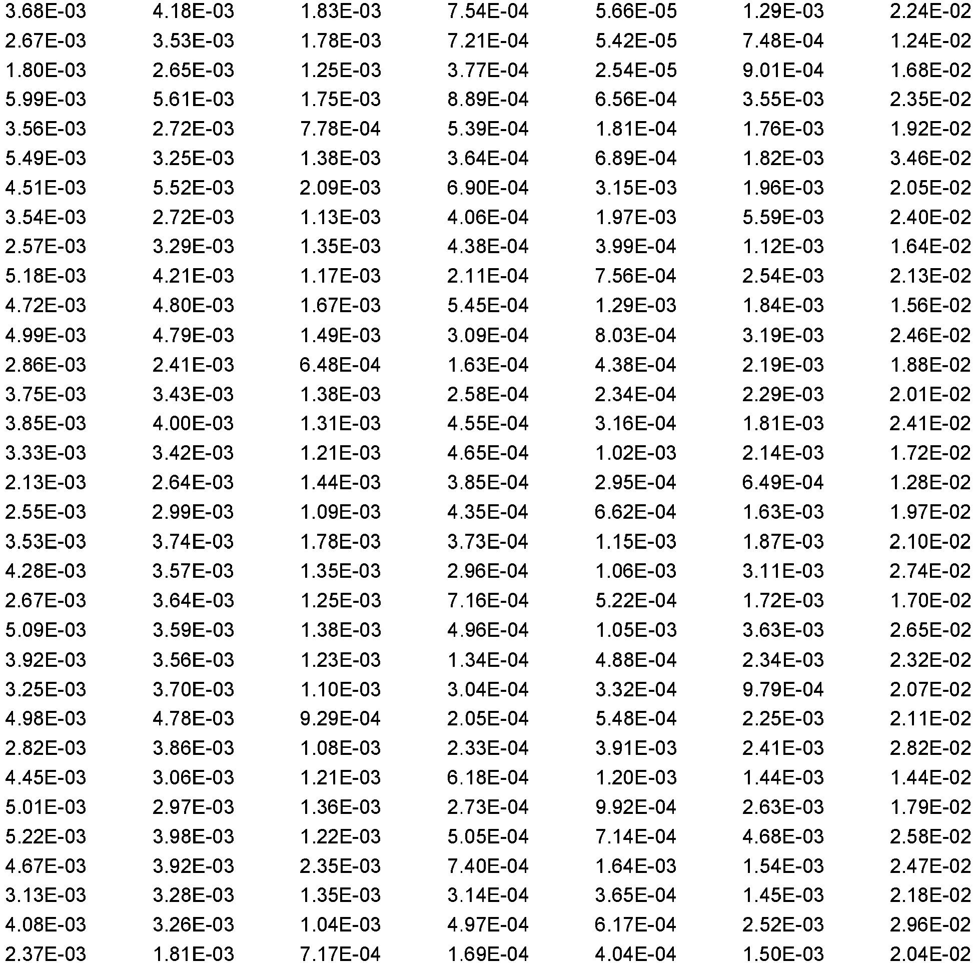

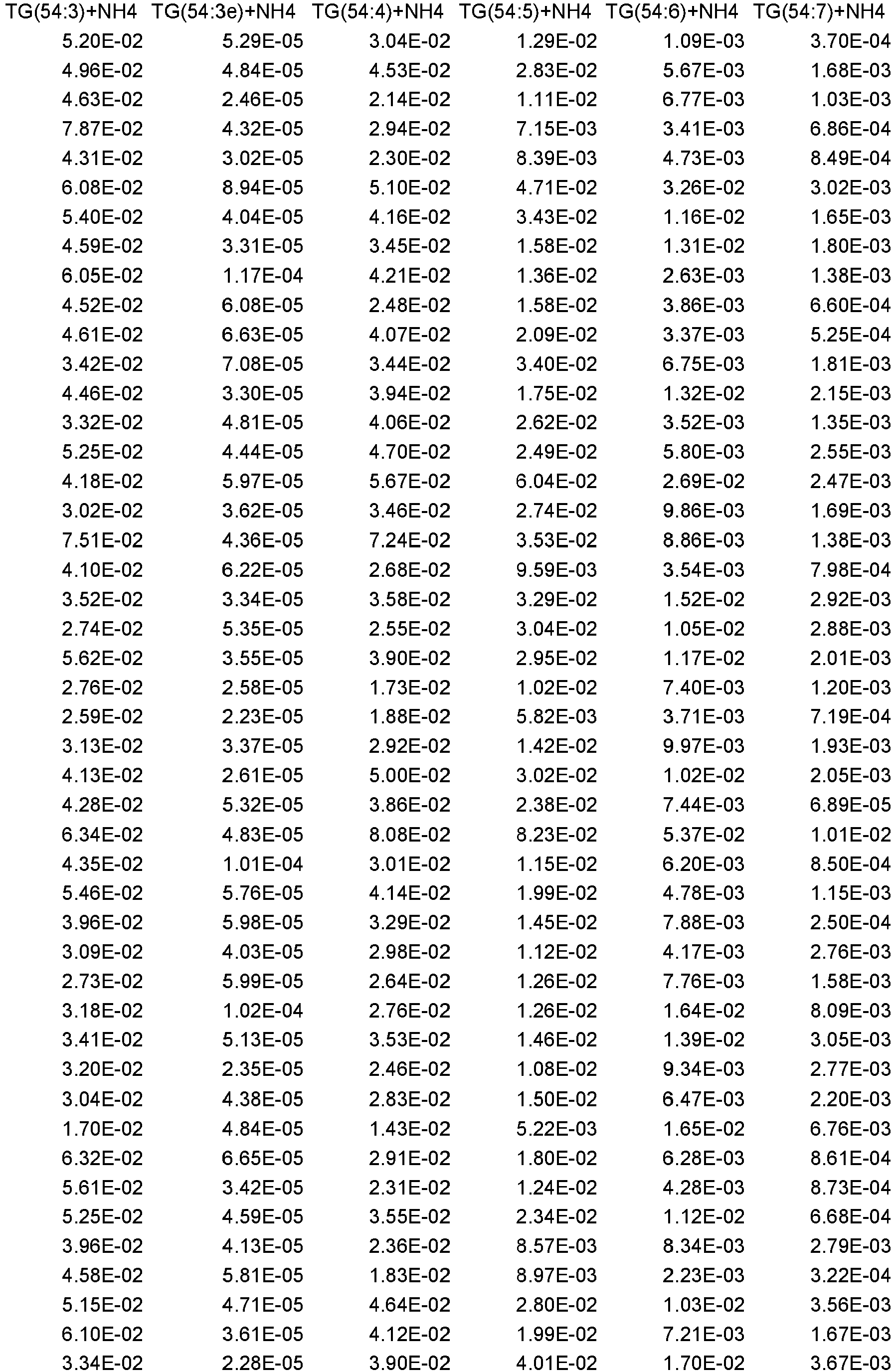

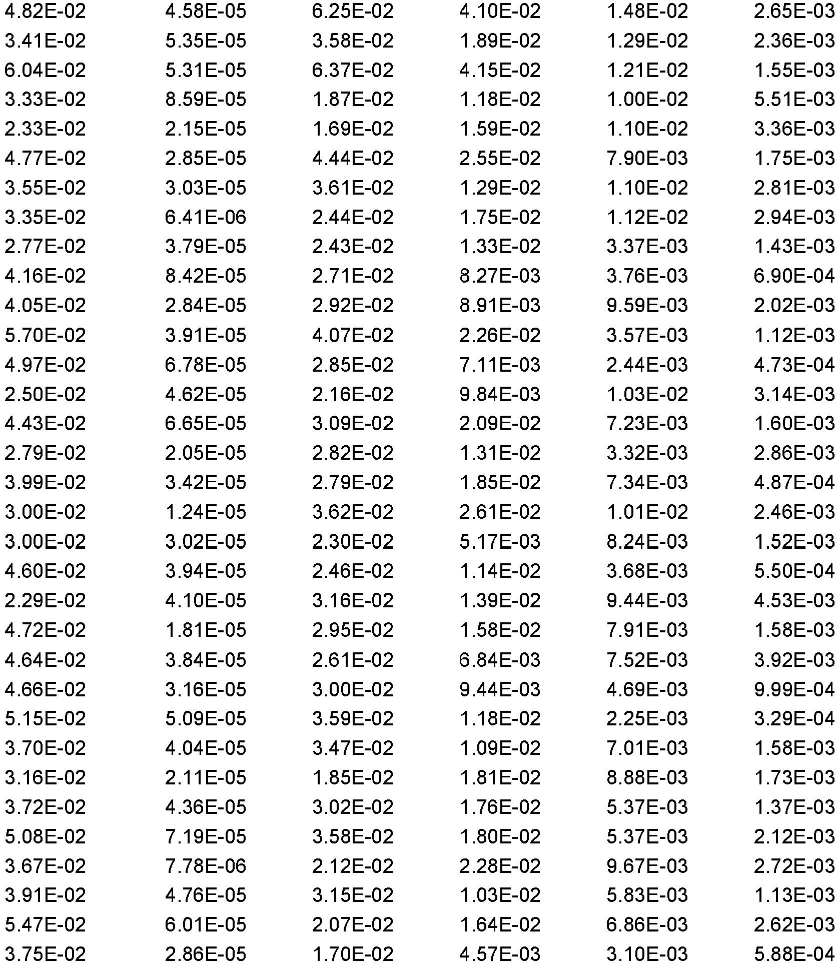

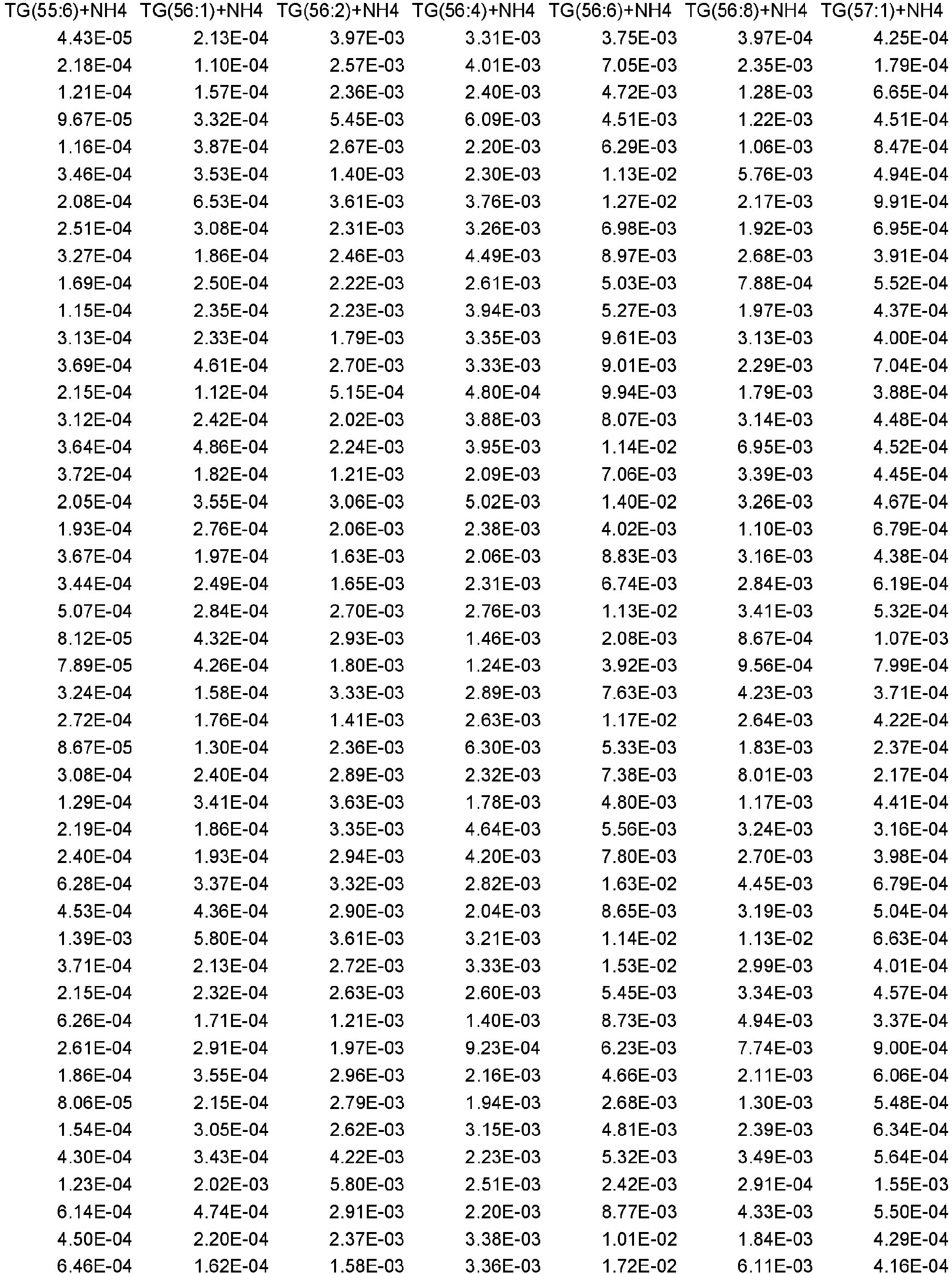

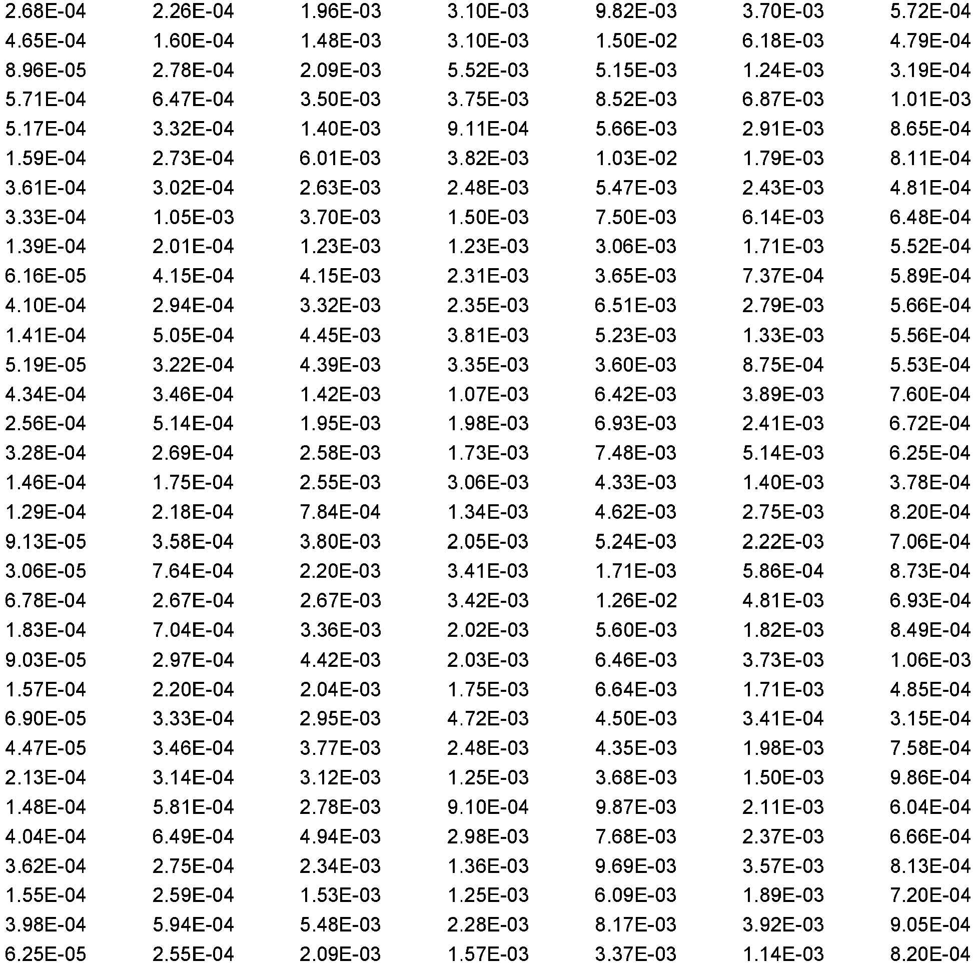

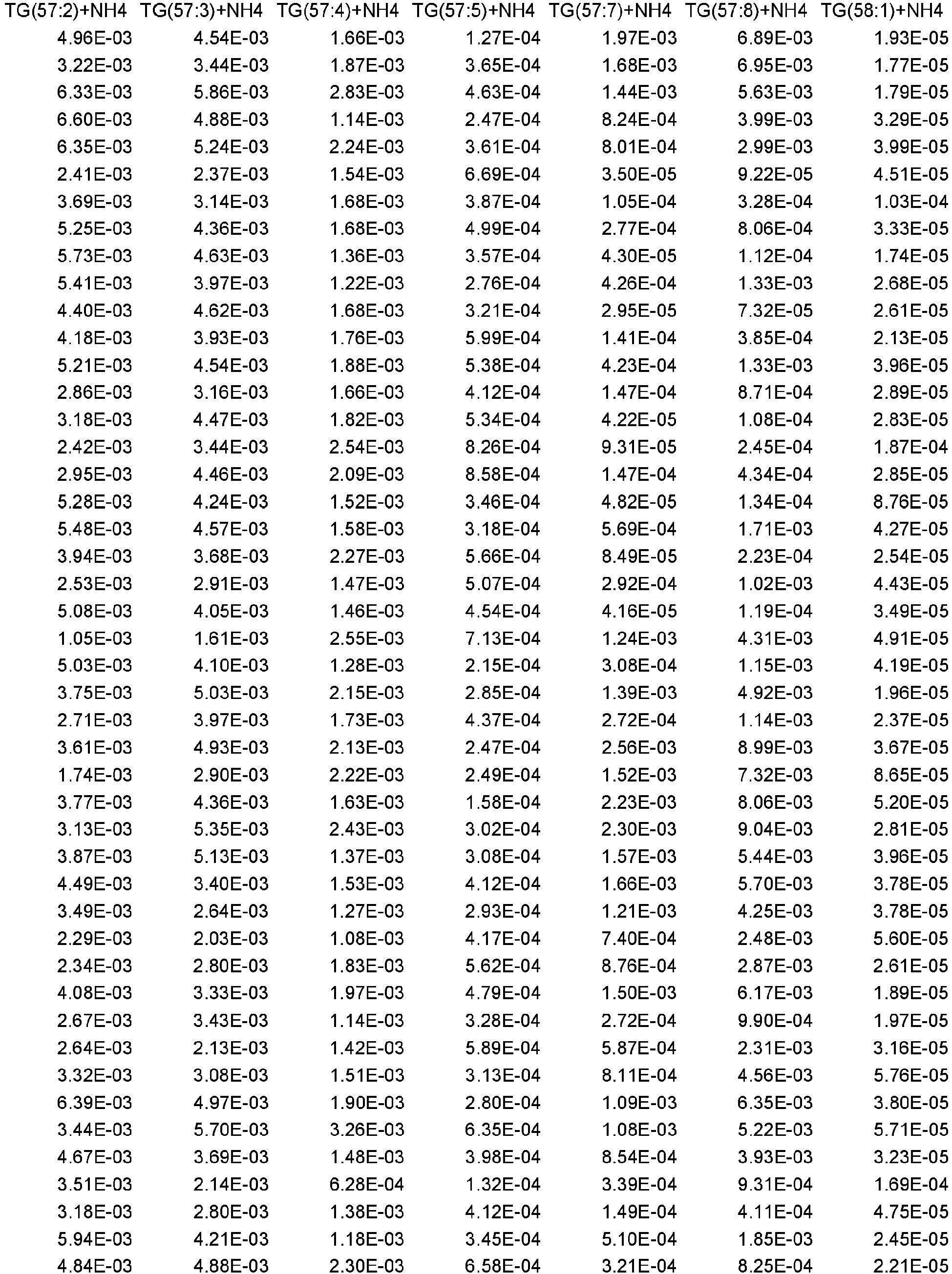

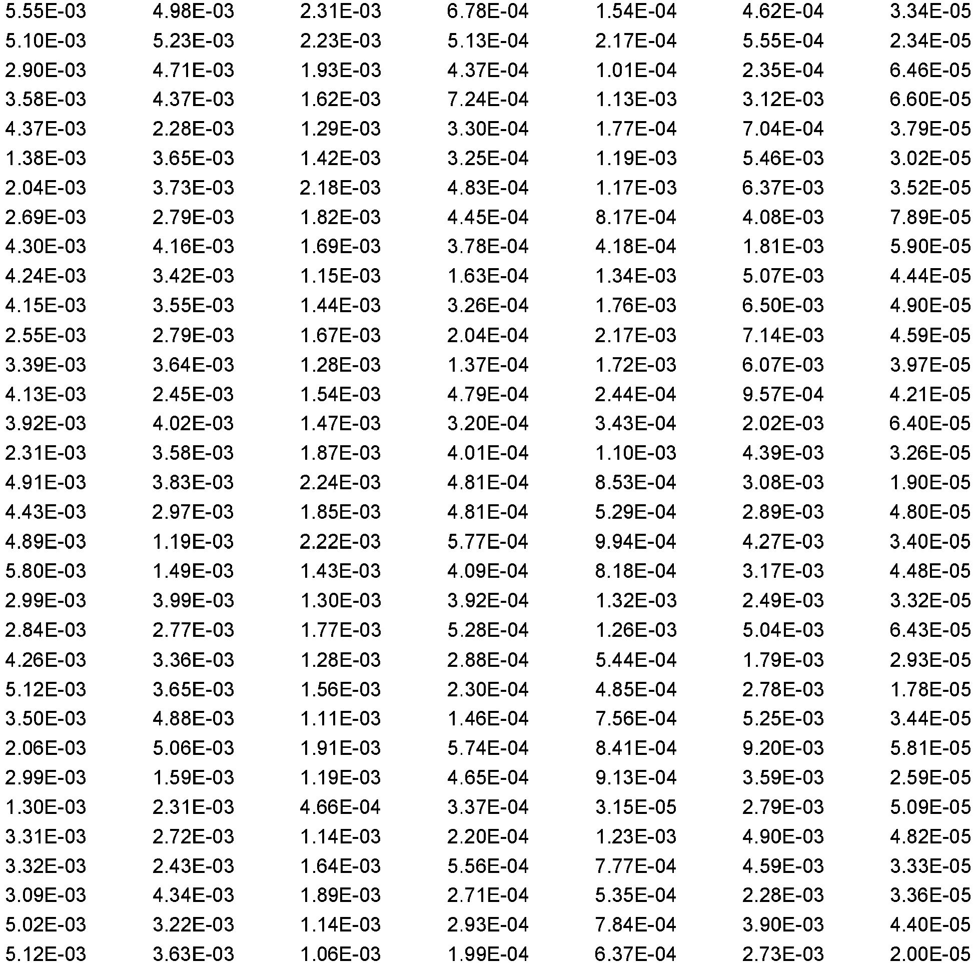

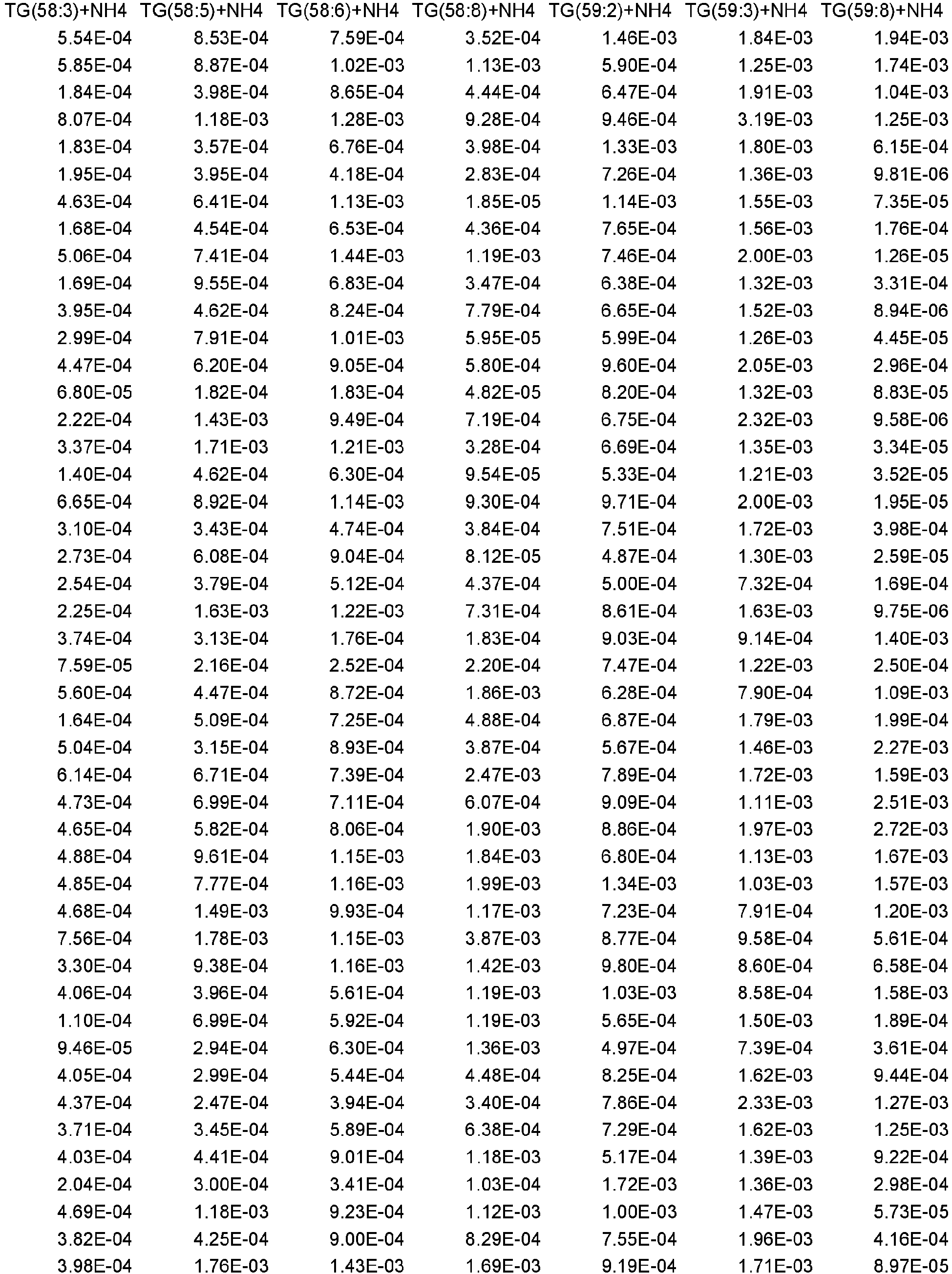

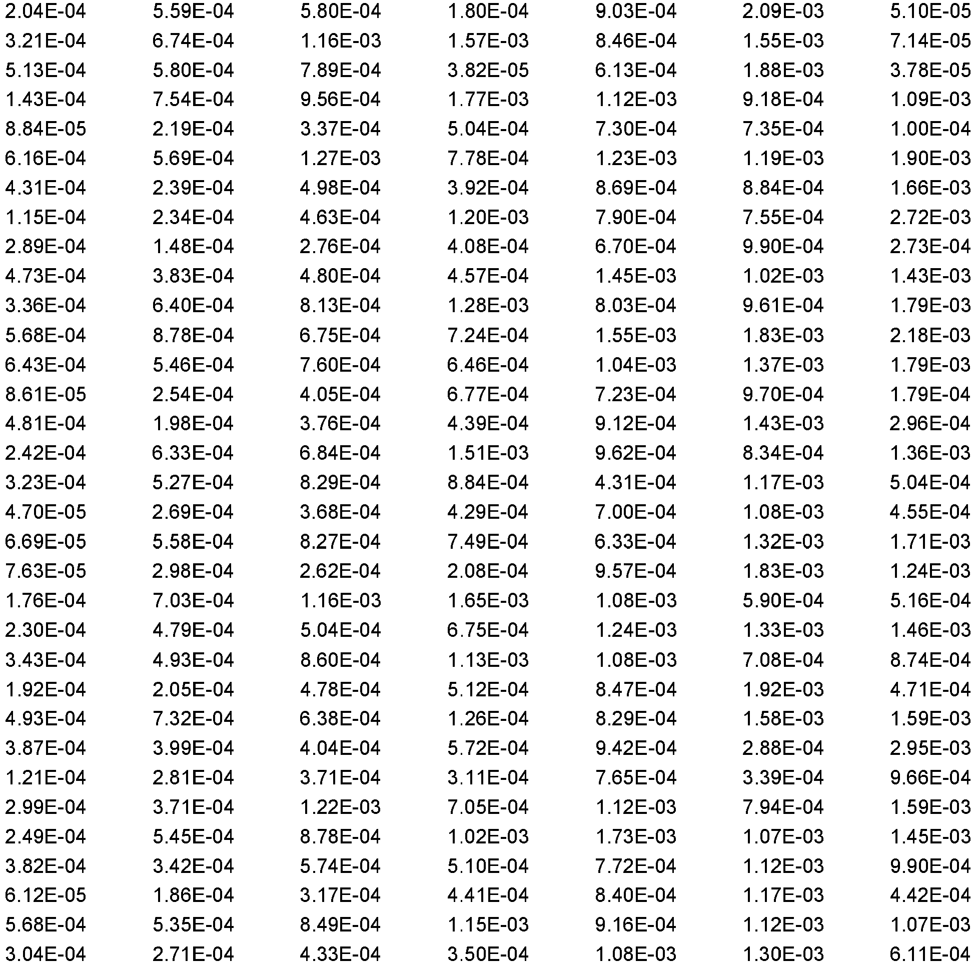

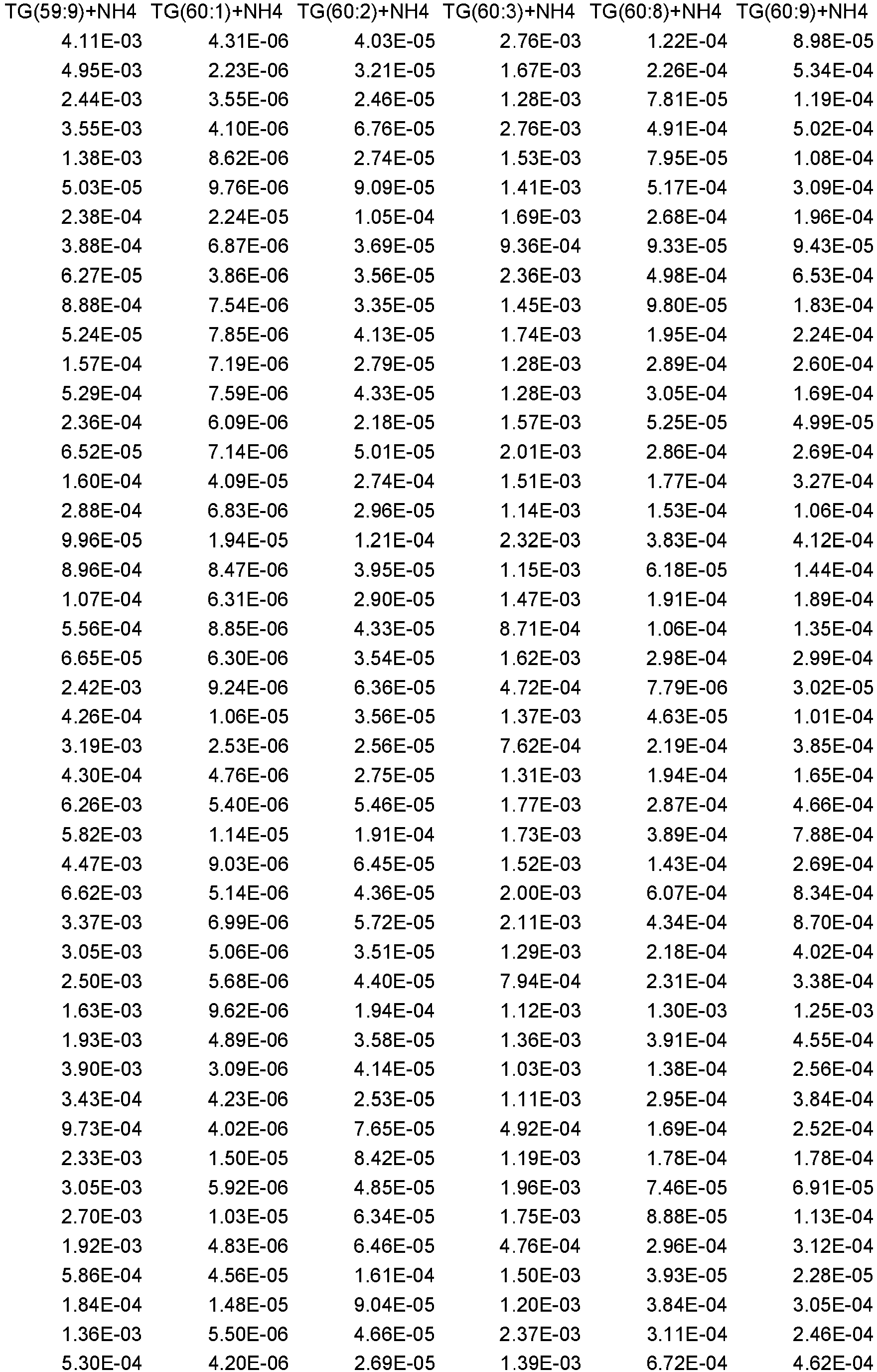

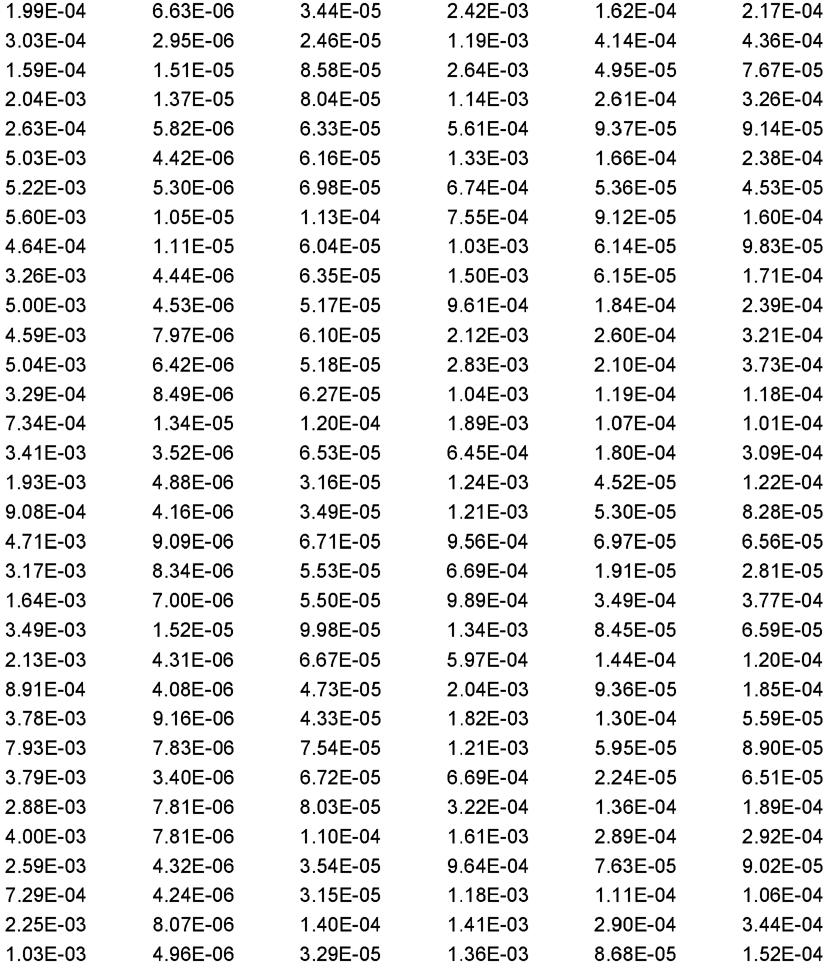

**Lengthy Table 12**

| Patient ID | C14_0 | C16_0 | C16_1n7trans | C16_1n7cis | C18_0 | C18_1n9cis |
|---|---|---|---|---|---|---|
| 13 | 0.59243415 | 0.263313483 | -0.037447313 | 0.246603087 | 0.238233325 | -0.180399747 |
| 15 | 0.210989412 | 0.079044829 | -0.235579476 | 0.1432998 | 0.13567238 | -0.132964597 |
| 33 | -0.539130049 | 0.121406143 | -0.537977533 | 0.053950335 | -0.064015514 | -0.048542098 |
| 34 | -0.171791892 | 0.074756468 | -0.26386419 | -0.320835978 | 0.342513206 | 0.121400207 |
| 36 | -0.172952658 | -0.167301879 | 0.334792147 | -0.193359282 | -0.023350703 | 0.031144405 |
| 37 | -0.159111606 | -0.046027324 | -0.110299162 | -0.203878775 | -0.086616749 | -0.163753911 |
| 38 | -0.229086796 | -0.202067948 | -0.115585518 | -0.701354818 | -0.156713686 | -0.074979991 |
| 39 | -0.093714348 | -0.103542367 | -0.027691139 | -0.306818781 | 0.096709076 | -0.103689235 |
| 40 | -0.364374732 | -0.098815688 | 0.15247059 | -0.29227307 | -0.248057607 | 0.047354014 |
| 43 | -0.226632807 | -0.071386937 | -0.24558957 | 0.042134117 | 0.065809978 | -0.187733047 |
| 44 | 0.286396269 | 0.009506372 | 0.022792767 | -0.041921801 | -0.103462186 | -0.127564889 |
| 46 | 0.401322044 | -0.017346797 | -0.000554597 | 0.450866921 | 0.051363412 | -0.141950233 |
| 50 | -0.408954364 | -0.10913716 | 0.072315264 | 0.290688495 | -0.11748261 | 0.080223727 |
| 51 | -0.159111606 | -0.064933234 | -0.249621833 | -0.068119199 | 0.20501381 | -0.112460616 |
| 52 | -0.297728729 | -0.07749422 | 0.04861045 | -0.127554395 | -0.021819219 | -0.063934061 |
| 53 | 0.114729415 | 0.055530142 | 0.237904147 | 0.274844166 | -0.108712886 | 0.173363181 |
| 54 | -0.490100622 | -0.093541182 | -0.12445869 | -0.404476035 | 0.105350012 | -0.051532155 |
| 55 | 0.743105759 | 0.150503793 | 0.147050523 | 0.565286553 | -0.049025287 | -0.074180595 |
| 56 | -0.312308179 | 0.069630728 | -0.367404869 | 0.123964135 | 0.032031133 | -0.048325774 |
| 57 | -0.289864993 | -0.065018441 | -0.03581466 | -0.187490338 | 0.105142673 | 0.072892932 |
| 59 | 0.237532735 | -0.037203859 | 0.305075675 | 0.026508799 | -0.171643387 | 0.134961458 |
| 61 | -0.162553951 | -0.128696696 | -0.221731579 | -0.351770169 | 0.108041525 | -0.291779306 |
| 62 | 0.175558975 | 0.039310483 | 0.015070721 | 0.446737528 | 0.036705079 | -0.00854949 |
| 63 | -0.50294189 | -0.113242109 | 0.028927755 | -0.493104227 | 0.042351371 | 0.151144717 |
| 66 | 0.679310456 | 0.12845314 | -0.428313622 | -0.37659689 | 0.292892703 | -0.11213779 |
| 67 | -0.006434766 | 0.021339434 | 0.044102553 | -0.057215217 | 0.038258219 | 0.027942866 |
| 72 | 0.271614034 | -0.132335876 | -0.147904309 | -0.449074673 | -0.193345808 | -0.108639513 |
| 74 | -0.291171328 | -0.230617243 | 0.073778322 | -0.24022194 | -0.247319872 | 0.133844361 |
| 75 | -0.320351118 | -0.166216999 | -0.052262399 | -0.604070304 | -0.033779809 | -0.025149469 |
| 78 | 0.041559089 | -0.12226959 | 0.112492834 | -0.225931122 | -0.05302505 | -0.051098259 |
| 79 | -0.635781161 | -0.197874456 | -0.406807417 | -0.447386909 | -0.026302473 | -0.052357077 |
| 80 | 0.164017215 | 0.019109342 | 0.287521099 | 0.01063545 | 0.195586893 | 0.208372034 |
| 85 | -0.208417367 | -0.089079623 | 0.03045564 | -0.288669462 | 0.155585424 | 0.118585209 |
| 86 | -0.361565742 | -0.09415511 | 0.008849861 | -0.120232825 | -0.217102581 | -0.183267203 |
| 88 | -0.535785564 | -0.156692447 | -0.147904309 | -0.327544911 | -0.196705274 | -0.244306556 |
| 90 | -0.404552303 | -0.202410054 | 0.255124744 | -0.415032365 | -0.226014528 | 0.111973615 |
| 93 | 0.343432396 | -0.017834384 | 0.122289973 | 0.188948985 | 0.017424132 | -0.097263034 |
| 12 | 0.458516791 | 0.023954426 | 0.129229626 | 0.088090196 | -0.277414216 | 0.065160489 |
| 18 | 0.530018352 | 0.167294311 | 0.145690903 | 0.401956557 | -0.049025287 | 0.046488758 |
| 19 | -0.392907339 | -0.100580057 | 0.33591764 | -0.010383917 | 0.1749163 | 0.285782907 |
| 24 | 0.840441598 | 0.085919477 | 0.073778322 | 0.484963051 | -0.100146702 | 0.051982199 |
| 27 | 0.434132964 | 0.12750469 | 0.115301824 | 0.355453714 | 0.074507086 | 0.19763896 |
| 29 | -0.015323714 | 0.047110476 | 0.066441532 | 0.065123635 | 0.090100082 | -0.221569436 |
| 30 | -0.025293886 | 0.067020636 | -0.040720639 | 0.083142141 | 0.261911562 | 0.068168242 |
| 31 | 0.15986266 | 0.108451764 | 0.028927755 | 0.03799118 | 0.026326101 | -0.096808839 |
| 32 | -0.20481376 | 0.060878755 | -0.276235482 | -0.404476035 | 0.051910308 | -0.159441993 |
| 41 | -0.554320214 | -0.022968451 | -0.149730794 | 0.189395115 | -0.259935995 | 0.009005665 |
| 42 | 0.471404359 | 0.006456683 | -0.01163943 | 0.267445859 | -0.037240639 | -0.011005318 |
| 48 | 0.091820924 | 0.028776149 | 0.241619321 | -0.151099473 | -0.077987921 | 0.118548598 |
| 49 | 0.118207679 | 0.023057513 | 0.188307205 | 0.234312193 | 0.06170364 | 0.016818204 |
| 65 | -0.576677121 | 0.006218654 | -0.138821594 | -0.583571783 | 0.051691585 | -0.121588868 |
| 14 | 0.401975852 | 0.153727058 | 0.025864966 | 0.411646208 | -0.005010138 | -0.005187724 |
| 16 | -0.581908171 | 0.083131201 | 0.036543938 | -0.126330405 | -0.044198441 | 0.146598447 |
| 2 | 0.311750761 | 0.119069693 | 0.306235095 | 0.666841983 | -0.206710289 | 0.201015972 |
| 21 | -0.059955868 | -0.002588464 | 0.198683433 | -0.30316247 | -0.11968711 | 0.241840664 |
| 23 | 0.400013143 | 0.244733071 | -0.282478999 | 0.519859792 | 0.477688925 | -0.323568949 |
| 26 | 0.204640185 | 0.053412939 | 0.235419673 | 0.302309544 | 0.081219593 | 0.185523375 |
| 3 | 0.062885813 | 0.218094391 | -0.117353865 | 0.270740755 | 0.226456132 | -0.167450773 |
| 4 | 0.12771099 | 0.038081415 | 0.215318494 | -0.112964471 | -0.059971639 | 0.058262573 |
| 45 | 0.302408256 | -0.020398122 | 0.112492834 | 0.242804813 | -0.048904332 | 0.149797199 |
| 58 | 0.65879435 | 0.103172243 | 0.123681762 | 0.358848022 | -0.076992276 | 0.182677447 |
| 60 | -0.043494591 | 0.023564563 | -0.082567749 | -0.034640895 | -0.119427505 | 0.06222113 |
| 64 | 0.249784037 | 0.089320903 | 0.112492834 | 0.738377275 | -0.039035435 | 0.188019743 |
| 68 | -0.287257429 | 0.040921345 | -0.135211482 | 0.387792529 | 0.023067488 | 0.104427247 |

| C18_1n9trans | C18_2n6 | C18_3n6 | C18_3n3 | C20_0 | C20_1n9 | C20_2n6 |
|---|---|---|---|---|---|---|
| -0.153010916 | -0.146584772 | 0.187059808 | -0.164781364 | 0.429556814 | -0.581168454 | 0.064010222 |
| 0.008964757 | 0.050732274 | -0.023371707 | -0.417720488 | 0.036514226 | -0.454416748 | 0.069287279 |
| 0.125977673 | 0.076450587 | -1.088345637 | -0.088241287 | -0.095255052 | -0.154900218 | -0.121490988 |
| 0.104694752 | -0.171366417 | -0.454154623 | -0.03030345 | 0.710969273 | 0.07527736 | -0.221909735 |
| 0.027838637 | 0.072656476 | 0.565875878 | -0.157234159 | -0.067475488 | -0.178710867 | -0.047838078 |
| -0.16324642 | 0.325771389 | -0.430153471 | 0.194163728 | 0.247235257 | -0.327854025 | -0.036073236 |
| -0.177754446 | 0.352040877 | -0.207800747 | 1.010371002 | 0.163853648 | -0.081212502 | -0.059742981 |
| -0.08987358 | 0.234237307 | -0.201491577 | -0.520374642 | 0.237184921 | 0.007618711 | 0.215890753 |
| 0.089895327 | 0.258579774 | -0.569915414 | 0.357343815 | -0.014129507 | -0.240842648 | -0.173790487 |
| -0.016767657 | 0.215991405 | 0.047724214 | 0.067412023 | -0.08126881 | -0.327854025 | 0.069287279 |
| 0.002593464 | 0.063199109 | 0.069543262 | 0.318392728 | -0.332583238 | -0.415501332 | -0.083986592 |
| 0.014721825 | 0.018466559 | 0.507256544 | -0.063976665 | -0.053869836 | -0.407896733 | 0.037198965 |
| 0.185302317 | 0.074717922 | 0.017871251 | -0.105940864 | -0.15324231 | 0.065887619 | 0.130530904 |
| -0.103348278 | 0.043303648 | 0.326172611 | 0.204690141 | 0.174664564 | 0.449094257 | 0.363526752 |
| 0.027838637 | 0.036570371 | 0.597487706 | -0.053755111 | -0.08126881 | -0.043674583 | 0.169370738 |
| 0.049694368 | -0.112943202 | 0.297948724 | 0.008662519 | -0.247061065 | -0.307092034 | -0.160456956 |
| 0.045806554 | 0.15180206 | -0.074665002 | -0.053755111 | -0.014129507 | -0.108915105 | -0.108832591 |
| -0.22258586 | -0.143074892 | 0.893971102 | 0.145373564 | -0.067475488 | -0.47041709 | -0.096332428 |
| 0.152835127 | 0.01777838 | -0.414466875 | -0.412854299 | -0.053869836 | -0.143204178 | 0.255985211 |
| 0.020445939 | 0.056525328 | -0.050039954 | -0.172385964 | -0.014129507 | 0.065887619 | 0.183555373 |
| 0.102593911 | 0.062175268 | -0.508221845 | 0.12573759 | 0.024091706 | 0.147380654 | 0.416974587 |
| -0.117662121 | 0.379397672 | -0.134597342 | 0.204690141 | -0.095255052 | -0.20929429 | 0.169370738 |
| 0.049139891 | -0.023845493 | 0.577487039 | -0.036947993 | -0.138427224 | -0.446511569 | 0.03174936 |
| 0.092554196 | -0.02324761 | -0.15540047 | -0.215277528 | 0.024091706 | -0.07034283 | -0.180524519 |
| -0.09114909 | 0.02979145 | -0.900947927 | -0.223277571 | 0.351595272 | -0.154900218 | 0.154982 |
| -0.052959786 | -0.027840545 | -0.023371707 | -0.493706395 | -0.053869836 | -0.012583996 | 0.115568032 |
| -0.226231507 | 0.081630641 | 0.197664362 | 0.627456719 | 0.21677605 | 1.221046674 | -0.30253947 |
| -0.040749622 | 0.129376335 | 0.050172196 | 0.503436433 | -0.214800203 | -0.260260734 | -0.333549706 |
| 0.012422973 | 0.15203617 | 0.390827081 | 0.233079144 | 0.096412367 | 0.966968933 | -0.407052168 |
| -0.102057122 | 0.056488536 | 0.36795889 | 0.222848876 | -0.123828424 | 0.693237625 | -0.29493487 |
| -0.141535933 | -0.014243007 | -0.304060744 | 0.878008516 | 0.096412367 | 2.075746493 | 1.81985919 |
| 0.088296606 | -0.268184133 | -0.273183506 | -0.046998329 | 0.237184921 | 0.168886859 | 0.150139376 |
| 0.161299206 | -0.078541986 | 0.00771888 | 0.457748249 | 0.378263519 | 1.022849391 | -0.279896993 |
| -0.012629313 | 0.165587593 | 0.471003559 | 0.188858676 | -0.297491918 | 0.683110957 | 0.069287279 |
| -0.069059205 | 0.227003667 | 0.086190495 | 0.120055756 | -0.426109296 | 0.730324694 | 0.197541615 |
| 0.088296606 | 0.059794445 | 0.311219061 | 0.036921856 | 0.267037884 | 0.254478789 | -0.450348974 |
| -0.165993674 | -0.094888115 | 1.252432043 | 0.046166914 | -0.001226102 | 0.763740691 | -0.083986592 |
| 0.004335119 | 0.112264208 | 0.030418467 | 0.285135506 | -0.247061065 | -0.253746053 | 0.026269894 |
| 0.037985285 | -0.261689285 | 0.076711751 | 0.08526964 | -0.095255052 | -0.196948454 | -0.30253947 |
| 0.138736203 | -0.239283867 | 0.120956453 | -0.187770882 | 0.360563942 | 0.084579752 | -0.036073236 |
| -0.025693631 | -0.079595349 | 0.364393824 | 0.285135506 | 0.257185588 | -0.16079994 | 0.30290213 |
| -0.022113675 | -0.297892101 | -0.383813134 | -0.12395937 | -0.001226102 | -0.280063361 | -0.173790487 |
| -0.057268811 | 0.190479736 | -0.128732223 | -0.302651158 | 0.060905679 | -0.260260734 | 0.375222792 |
| 0.019875 | -0.152371407 | -0.259760485 | -0.260091544 | 0.060905679 | -0.154900218 | -0.207923493 |
| 0.056874736 | 0.03416535 | -0.071898748 | -0.172385964 | 0.048784318 | -0.196948454 | 0.140383201 |
| -0.055419812 | 0.182597031 | -0.380046651 | -0.379438302 | 0.060905679 | -0.313964913 | -0.108832591 |
| 0.031796799 | 0.126498832 | -0.314568722 | -0.285409352 | -0.332583238 | -0.125912681 | -0.167101499 |
| -0.008508023 | 0.005503094 | 0.329876319 | 0.316053546 | -0.08126881 | -0.341938765 | -0.047838078 |
| 0.093615768 | -0.050553612 | 0.002603779 | -0.199466922 | -0.014129507 | 0.300288325 | -0.173790487 |
| -0.161190921 | -0.078036765 | 0.76628111 | 0.131387322 | -0.15324231 | -0.327854025 | 0.188239222 |
| -0.099479801 | 0.169308558 | 0.083829219 | -0.427524488 | -0.297491918 | -0.334871598 | 0.064010222 |
| -0.016175416 | -0.147306273 | -0.11421818 | -0.020418691 | -0.199051846 | -0.16079994 | 0.069287279 |
| 0.127004367 | -0.063122027 | -1.01478307 | -0.260091544 | -0.014129507 | 0.125401747 | -0.160456956 |
| -0.157092554 | -0.163083272 | 0.453189322 | 0.08526964 | 0.386997199 | -0.149035099 | -0.373715748 |
| 0.072167224 | -0.08708507 | -0.332331178 | -0.157234159 | -0.001226102 | 0.25056489 | -0.365552438 |
| -0.115044318 | -0.321612403 | 0.301758253 | 0.064404502 | 0.084716328 | -0.266818134 | 0.355652696 |
| 0.12237592 | -0.337798979 | 0.253063141 | -0.264266915 | -0.15324231 | -0.081212502 | -0.214892163 |
| -0.003818683 | -0.13279639 | -0.332331178 | -0.131258672 | 0.296025421 | -0.370717729 | 0.188239222 |
| 0.001430673 | 0.086855388 | -0.354071165 | -0.231342131 | 0.048784318 | -0.327854025 | -0.140784191 |
| -0.097551161 | -0.067943918 | 0.33356636 | 0.064404502 | 0.036514226 | -0.20310232 | -0.432804665 |
| 0.111492164 | -0.328996488 | 0.224721163 | -0.03030345 | -0.332583238 | -0.04895164 | 0.095262766 |
| 0.06291042 | 0.051878788 | -0.37255598 | 0.145373564 | -0.297491918 | -0.20310232 | -0.121490988 |
| 0.176063253 | -0.353697216 | -0.170529352 | -0.260091544 | -0.214800203 | 0.017569042 | -0.108832591 |
| 0.258095638 | -0.084031632 | -0.52112525 | -0.462615808 | -0.332583238 | -0.012583996 | 0.090121366 |

| C20_3n6 | C20_4n6 | C20_5n3 | C22_0 | C22_4n6 | C22_5n6 | C22_5n3 |
|---|---|---|---|---|---|---|
| 0.225888409 | -0.357350956 | 0.019786673 | -0.302205013 | 0.07507536 | -0.237851233 | 0.025649917 |
| 0.247519429 | -0.081368478 | 0.319492938 | -0.141862363 | -0.119566681 | 0.117603454 | -0.238651707 |
| -1.097869434 | -0.133844933 | 0.077243728 | -0.053308965 | -0.472705971 | -0.533696617 | 0.028870532 |
| -0.235512332 | -0.336423436 | 0.319492938 | 0.251180225 | -0.196858356 | 0.332163262 | -0.290387381 |
| 0.241236686 | 0.41365202 | -0.169007399 | -0.280698808 | 0.196630561 | 0.352641793 | 0.038470606 |
| -0.382917623 | -0.2368191 | 0.484399453 | 0.212959013 | -0.111336182 | -0.410463976 | -0.349630215 |
| -0.551220259 | 0.007627784 | 0.035787014 | 0.275872838 | -0.251918133 | 0.31827415 | -0.264185009 |
| 0.019929682 | -0.126862841 | 0.507772804 | -0.070403399 | -0.017517427 | 0.297071942 | -0.285972363 |
| -0.646066509 | -0.491314034 | 0.253625513 | -0.160554496 | -0.533330592 | -0.752750183 | -0.161846295 |
| -0.045745352 | 0.104309811 | -0.100014527 | -0.070403399 | -0.224009345 | 0.037560746 | 0.069821136 |
| -0.144209555 | 0.39389411 | -0.695100495 | -0.575498348 | -0.002702341 | 0.167613875 | 0.305234779 |
| 0.319985732 | 0.333073488 | -0.662665219 | 0.103260095 | 0.352500932 | 0.134553012 | 0.35518571 |
| -0.212926679 | 0.126714966 | 0.003526152 | -0.003712024 | 0.026285196 | 0.260704298 | -0.041041457 |
| 0.058494873 | 0.182299913 | -0.257200111 | 0.07384621 | 0.040469831 | -0.090693589 | 0.680994733 |
| 0.144822956 | 0.587412722 | -0.728623187 | -0.087795141 | 0.283139804 | 0.167613875 | 0.197160558 |
| 0.054071485 | -0.477591692 | -0.17552208 | -0.019972545 | -0.426720857 | -0.327082367 | 0.025649917 |
| -0.094500527 | 0.055138767 | -0.236146702 | 0.35778996 | -0.251918133 | -0.155934111 | 0.085135371 |
| 0.110103376 | -0.013568431 | -0.971853497 | -0.239026111 | 0.184509201 | 0.167613875 | 0.218666763 |
| -0.185997267 | 0.177887232 | 0.373769825 | 0.159613032 | 0.482805474 | 0.479393499 | -0.017190241 |
| -0.059840018 | -0.042915173 | -0.106093573 | 0.186641704 | -0.07907532 | 0.134553012 | -0.0479619 |
| 0.16543044 | -0.551097041 | -0.052668407 | -0.019972545 | -0.170425099 | -0.069859502 | -0.030749771 |
| -0.368318824 | -0.028242842 | 0.335303544 | 0.275872838 | -0.170425099 | -0.381890604 | -0.312759679 |
| 0.230145999 | -0.088962577 | -0.505479635 | 0.07384621 | -0.095075661 | -0.049450631 | -0.142577876 |
| -0.07629835 | 0.277321857 | -0.046904702 | 0.103260095 | -0.002702341 | -0.009841493 | -0.173588113 |
| -0.522630547 | -0.44244163 | 0.896598593 | 0.117648833 | -0.196858356 | -0.501435754 | -0.272843071 |
| 0.181727513 | 0.190019249 | 0.373769825 | -0.160554496 | 0.260792506 | 0.10901971 | -0.217860937 |
| 0.158608355 | 0.81248066 | 0.824725259 | 0.873099897 | 0.402762767 | -0.039400295 | 0.415946804 |
| 0.041965571 | 0.291948509 | 1.155342476 | 0.186641704 | 0.140888578 | 0.345862106 | 0.158374533 |
| 0.03230366 | 0.462468802 | -0.156103994 | 0.311804847 | 0.115080694 | 0.497093076 | 0.250082959 |
| -0.191649281 | 0.715762756 | -0.195324707 | -0.019972545 | 0.28864946 | 0.455295947 | -0.32185065 |
| 1.472000739 | 0.370095482 | -0.229226259 | 0.697467328 | 0.387947681 | 0.352641793 | -0.127426071 |
| 0.034243525 | -0.159788629 | 0.315500917 | -0.259645398 | 0.061376515 | -0.019597667 | -0.034168578 |
| -0.108559212 | 0.218787059 | -0.393775646 | 0.212959013 | 0.10192261 | 0.199616606 | 0.409375039 |
| 0.153461075 | 0.577023018 | 1.463538089 | 0.058808333 | 0.397848752 | 0.366064813 | 0.247502313 |
| 0.021893676 | 0.629277161 | 1.410105184 | -0.070403399 | 0.190588247 | 0.31827415 | -0.027342613 |
| 0.415295912 | 0.518872783 | 1.034315844 | 0.173218684 | 0.172239108 | -0.000179582 | 0.032080808 |
| 0.280881517 | 0.646253222 | -0.551999651 | 0.287994199 | 0.840384987 | 0.607702947 | 0.247502313 |
| -0.130405655 | -0.371345708 | -0.035476006 | -0.417274343 | -0.010082449 | 0.117603454 | -0.205590844 |
| 0.020584776 | -0.083893442 | -0.561569102 | -0.32418392 | -0.361480335 | -0.275122628 | 0.389396572 |
| -0.015419113 | 0.041850873 | -0.041174027 | -0.218823404 | -0.136233734 | -0.425062776 | -0.189461462 |
| -0.107070009 | -0.327293642 | -0.393775646 | -0.160554496 | 0.260792506 | -0.039400295 | 0.032080808 |
| -0.256889802 | -0.57147189 | -1.254716283 | -0.729649028 | -0.756474144 | -0.860380847 | 0.275529625 |
| 0.120231553 | -0.472833015 | 0.651973153 | -0.105494719 | -0.533330592 | -0.300765059 | -0.272843071 |
| 0.330128297 | -0.150410476 | -0.130786186 | 0.131833468 | -0.127865484 | 0.028258354 | -0.294821978 |
| -0.263828252 | -0.11588683 | 0.425251781 | -0.053308965 | -0.119566681 | 0.230626842 | -0.013828894 |
| -0.116038678 | -0.042474062 | 0.540243189 | -0.053308965 | -0.280628239 | -0.178662362 | -0.495986623 |
| 0.089533988 | 0.050729059 | -0.4964298 | -0.070403399 | 0.095278067 | -0.178662362 | -0.097794274 |
| -0.06839317 | 0.014941577 | -0.728623187 | 0.131833468 | -0.07907532 | -0.439877861 | 0.312525213 |
| -0.040162644 | -0.031290961 | -0.222353379 | -0.019972545 | -0.224009345 | -0.090693589 | -0.112500421 |
| 0.191710957 | 0.068647117 | -0.823933366 | 0.07384621 | 0.412518942 | 0.142921262 | 0.387151902 |
| -0.021547835 | 0.227409988 | 0.460466687 | 0.088661296 | 0.159816588 | -0.090693589 | -0.065475483 |
| 0.051535022 | -0.087346881 | -0.029810269 | -0.604485885 | 0.068249395 | -0.111970988 | 0.155545677 |
| -0.519254067 | -0.214310537 | 0.187667545 | -0.218823404 | -0.438020413 | -0.77336947 | 0.069821136 |
| -0.301066597 | -0.880844598 | 0.327429488 | 0.774428369 | -0.205827026 | -0.637237296 | -0.387912401 |
| -0.281387965 | -0.130708999 | 0.43594707 | -0.280698808 | -0.280628239 | -0.35411104 | -0.335643973 |
| 0.675008827 | 0.052936344 | -0.377646264 | -0.123513224 | 0.556913447 | 0.581176193 | 0.147010774 |
| 0.340644646 | -0.014854605 | -0.385678436 | -0.369646294 | 0.153546975 | 0.22296397 | 0.091195995 |
| 0.320956135 | -0.134086566 | -0.202013695 | 0.263602745 | 0.326389788 | 0.282683204 | 0.054268723 |
| -0.117541308 | -0.626079843 | 0.637584416 | 0.07384621 | -0.095075661 | 0.339036141 | -0.201534044 |
| 0.272771995 | -0.180330434 | -0.775143202 | 0.012288317 | -0.136233734 | -0.059603002 | 0.364423651 |
| 0.036824171 | -0.002278504 | -1.031572731 | -0.259645398 | 0.299578531 | 0.159450564 | 0.262887232 |
| -0.088639704 | -0.129986717 | 0.443014237 | 0.212959013 | -0.351009036 | -0.367904362 | -0.142577876 |
| 0.073513928 | -0.198206293 | -0.243115371 | -0.087795141 | -0.170425099 | 0.073928391 | -0.069015309 |
| 0.118451665 | -0.214310537 | -0.346147597 | -0.123513224 | 0.294128926 | 0.366064813 | -0.090521515 |

| C24_0 | | C22_6n3 | | C24_1n9 | | |
|---|---|---|---|---|---|---|
| | 0.109895213 | | -0.858593502 | | | -0.374108762 |
| | -0.410639225 | | -0.105471176 | | | -0.45415147 |
| | -0.597850767 | | 0.388159643 | | | 0.319038418 |
| | -0.866114754 | | -0.59436309 | | | 0.325428216 |
| | 0.152454827 | | 0.210143526 | | | -0.541162847 |
| | 0.364425078 | | -0.277451379 | | | 0.105464318 |
| | 0.510517696 | | 0.133758513 | | | -0.219958082 |
| | 1.07428574 | | -0.125419344 | | | 1.253118692 |
| | 0.975655136 | | 0.215436544 | | | 0.96291455 |
| | -0.153919378 | | 0.481968928 | | | 0.450965961 |
| | 0.969538909 | | 0.645238587 | | | 0.671859793 |
| | -0.212188286 | | 0.148381031 | | | -0.209029012 |
| | -0.866114754 | | 0.19255225 | | | 0.266394685 |
| | -0.098859601 | | 0.935003866 | | | 0.064972957 |
| | -0.063768281 | | 0.299993932 | | | 0.128994815 |
| | 0.12428395 | | 0.133758513 | | | 0.022772602 |
| | -1.233839534 | | 0.647199372 | | | 0.577450108 |
| | 0.4296656 | | 0.274018445 | | | -0.336368434 |
| | 0.490909225 | | -0.482245792 | | | -0.361369736 |
| | -0.658475389 | | 0.001862443 | | | -0.705465898 |
| | -0.756915462 | | -0.028471636 | | | -0.198218096 |
| | 0.500761522 | | -0.291124681 | | | -0.619943725 |
| | -1.033168838 | | -0.052827443 | | | -1.552763759 |
| | -0.27406369 | | 0.274018445 | | | 0.676354183 |
| | 0.06544345 | | -0.699723606 | | | 0.500291028 |
| | 0.460756187 | | -0.153378155 | | | -0.946627955 |
| | 0.450499687 | | -0.050856999 | | | -0.400084249 |
| | -0.295569895 | | 0.182470737 | | | -0.511309884 |
| | 0.318439965 | | -0.14469189 | | | -0.276470293 |
| | 0.646473333 | | 0.0791001 | | | 0.039867036 |
| | 0.282507956 | | -0.259102241 | | | 0.014114539 |
| | -0.340021658 | | -0.068732351 | | | -0.687766321 |
| | -0.081160024 | | -0.107552343 | | | 0.54218197 |
| | 0.460756187 | | 0.204059857 | | | 0.856431069 |
| | 0.034671792 | | 0.869363011 | | | 1.031233794 |
| | 2.070194099 | | -0.841087584 | | | -1.686295151 |
| | -0.410639225 | | -0.250653186 | | | -0.324098342 |
| | -0.363011176 | | -0.753895457 | | | -0.619943725 |
| | 0.460756187 | | 0.621906677 | | | 0.014114539 |
| | -0.098859601 | | -0.326797254 | | | -0.26490947 |
| | 0.318439965 | | -0.449455969 | | | 0.121212675 |
| | 0.095296413 | | 0.308275552 | | | 0.306135013 |
| | 0.529749058 | | 0.365052068 | | | 1.174544259 |
| | -0.658475389 | | -0.405004207 | | | -0.324098342 |
| | 0.576269074 | | 0.195633916 | | | 0.979609428 |
| | -0.295569895 | | -0.184388392 | | | 0.54218197 |
| | 0.318439965 | | 0.359190769 | | | 0.999282194 |
| | -0.098859601 | | 0.43404494 | | | 0.818258496 |
| | 0.330136004 | | -0.016988256 | | | 0.159531539 |
| | -0.988717076 | | 0.366998225 | | | -0.511309884 |
| | -0.063768281 | | -0.423405292 | | | -1.472721051 |
| | -0.56886323 | | 0.255353151 | | | -1.017245522 |
| | -0.295569895 | | 0.065174723 | | | -0.636473027 |
| | 0.12428395 | | -0.477710637 | | | -0.818794584 |
| | 0.12428395 | | -0.434899672 | | | 0.720220865 |
| | -0.063768281 | | -0.313910477 | | | 0.53704057 |
| | -0.460649646 | | 0.091981343 | | | 0.976292676 |
| | -0.62770373 | | -0.076780684 | | | -0.176940697 |
| | -0.212188286 | | -0.248252225 | | | 0.331777444 |
| | 0.750986031 | | -0.085913168 | | | -0.653280145 |
| | 0.193276822 | | -0.146856393 | | | -0.14585011 |
| | -0.317548802 | | 0.113923152 | | | -0.105844776 |
| | -0.386541673 | | 0.450276135 | | | 0.299620332 |
| | -0.386541673 | | -0.208293912 | | | 0.362932612 |
| | 0.050175978 | | | | | |

**Lengthy Table 13**

| Patient ID | 1579 | 1661 | 1784 | 1825 | 1836 | 1866 |
|---|---|---|---|---|---|---|
| 34 | -0.282967043 | 0.928115221 | -0.443575596 | 1.668914759 | -0.146939807 | -0.475260538 |
| 50 | 0.125739122 | 2.162715154 | -0.023212599 | 1.233613377 | 0.012846083 | 0.141707797 |
| 56 | 1.195101776 | 2.663293104 | 1.31500991 | 3.026081864 | 0.808389649 | 1.353836987 |
| 63 | -0.398166512 | -4.424588864 | -0.479595356 | -3.889428977 | -0.549840376 | -0.9812241 |
| 67 | -0.92875429 | 1.183006062 | -0.866182948 | 1.115555461 | -0.505068697 | -0.01710655 |
| 70 | -0.312828204 | 1.330211073 | -0.242635467 | 1.329896717 | -0.080970772 | -0.028412023 |
| 72 | 0.739357588 | -4.424588864 | 0.652462312 | -3.889428977 | 0.902593969 | 1.206472346 |
| 73 | -0.360924116 | 1.654281327 | -0.28025698 | -3.889428977 | -0.361531947 | 0.119628701 |
| 74 | 0.406080036 | 1.412342986 | 0.436171527 | 2.201638472 | 0.189236188 | 0.455518317 |
| 75 | 0.357950638 | 1.790233497 | 0.441742958 | -3.889428977 | -0.136280675 | 0.732445072 |
| 76 | 0.857903604 | 1.57381583 | 0.845728676 | 1.886452651 | 0.037060978 | 0.395766312 |
| 77 | -0.22055554 | 0.772921098 | -0.209588965 | 0.931140566 | 0.240685164 | -0.440327831 |
| 78 | 0.00731546 | - -4.424588864 | -0.071679903 | 1.335677451 | -0.139970168 | -0.550637745 |
| 79 | 0.417614448 | 1.285286124 | 0.276830352 | 1.679188118 | 0.292243883 | -0.205227115 |
| 80 | -0.073067396 | 1.338846705 | -0.514563072 | -3.889428977 | -0.083210264 | 0.082497119 |
| 81 | 1.517515063 | 1.855427912 | 1.286759955 | 2.305073592 | 0.103094686 | 0.582611207 |
| 82 | 0.871189777 | 1.374106886 | 0.905103469 | 2.341919174 | -0.450867786 | 0.240583976 |
| 90 | -0.425996789 | 1.362491089 | -0.1067312 | 1.500077536 | 0.305618974 | 0.170179966 |
| 91 | 0.411741958 | 2.090811421 | 0.252976168 | -3.889428977 | 0.584904691 | 0.467247595 |
| 92 | 0.345201664 | -4.4254588864 | 0.164720151 | -3.889428977 | -0.619535947 | -0.334596935 |
| 93 | -0.234740276 | 1.933583463 | -0.219220021 | -3.889428977 | 0.212825356 | 0.544300515 |
| 94 | -0.003507737 | 1.74656481 | -0.150814768 | 1.407339572 | 0.159870189 | 0.321137038 |
| 13 | -0.37647984 | 0.661242245 | -0.235426895 | -3.889428977 | -1.509948425 | -0.311143136 |
| 15 | -0.189382614 | 1.340340971 | -0.050535398 | 1.485298133 | 0.546699864 | -0.011722462 |
| 33 | -0.65863787 | 0.980819662 | -0.710078325 | 0.823847951 | -0.194964786 | -0.40385906 |
| 36 | 0.628348682 | 1.314821185 | 0.398185006 | 1.883864754 | 0.009362027 | 0.162726436 |
| 37 | -0.039374816 | 1.541222043 | -0.104969025 | -3.889428977 | 1.077692894 | 0.026210171 |
| 38 | 0.210090404 | 0.670402167 | 0.093619801 | 1.521047174 | -0.646340955 | -0.38882509 |
| 39 | -0.758846294 | 0.817032167 | -0.646508273 | -3.889428977 | -0.118677744 | -0.228257994 |
| 40 | -0.806972101 | 0.682726522 | -0.827895167 | -3.889428977 | -1.134830809 | -0.500996093 |
| 43 | -0.589425472 | 0.988809478 | -0.785956937 | 0.688467889 | -0.139805901 | -0.391684528 |
| 44 | 1.150825208 | 2.583701019 | 1.293340116 | 3.165966036 | 0.417392181 | 1.073778178 |
| 46 | -0.391988023 | 0.563492402 | -0.471000246 | 0.854363787 | -0.127724428 | -0.9423481 |
| 51 | -0.094339357 | 1.353873981 | -0.087711345 | 1.403157715 | -0.219376644 | -0.078057523 |
| 52 | -0.875173035 | -0.007614598 | -0.807393864 | 0.67863255 | -0.875846813 | -0.701099075 |
| 53 | -0.248112591 | 0.714983953 | -0.053282224 | 1.203371314 | -1.155176681 | -0.244629373 |
| 54 | 0.269557778 | 2.415053258 | 0.201673261 | 1.757783147 | 0.447948115 | 0.858792925 |
| 55 | -0.424437389 | -4.424588864 | -0.563698038 | -3.889428977 | 0.323117355 | -0.17837692 |
| 57 | -1.163924462 | - -4.424588864 | -1.167149372 | 2.852074926 | -1.056277328 | -6.859993095 |
| 59 | 1.011140028 | 3.111038295 | 1.024763773 | 2.871978435 | 0.963332563 | 1.933999958 |
| 61 | 0.364087648 | -4.424588864 | 0.30177503 | -3.889428977 | -0.333653401 | -0.391776904 |
| 62 | 0.05928758 | - -4.424588864 | -0.1551647 | 2.837917201 | 0.147732896 | 0.214677981 |
| 66 | 1.264053627 | 2.145927217 | 1.4807671 | 3.313249948 | 1.143354342 | 1.193250486 |
| 84 | 1.224429639 | 2.655808338 | 0.899492719 | -3.889428977 | 0.68616853 | 1.183318482 |
| 85 | -0.125594568 | -4.424588864 | -0.055901419 | -3.889428977 | -0.200747788 | -0.065913228 |
| 86 | -1.002119373 | -4.424588864 | -0.907007982 | -3.889428977 | -0.765706226 | -1.158634189 |
| 87 | 0.30794915 | -4.424588864 | 0.123166758 | -3.889428977 | 0.768498784 | 0.278864457 |
| 88 | -0.561488304 | - -4.424588864 | -0.324393793 | -3.889428977 | -0.155727681 | -0.255210804 |
| 89 | 0.663534372 | 1.905980506 | 0.762770505 | 2.293590165 | 0.403756441 | 0.37650051 |
| 12 | 0.568694788 | 1.557876982 | 0.682070571 | 2.189577561 | -0.256467396 | 0.439554885 |
| 18 | 0.587751211 | 2.695143086 | 1.032405367 | 2.648452389 | 1.137652618 | 1.587783666 |
| 19 | 1.170366486 | 2.000106493 | 1.212075437 | 2.547541416 | 1.010380577 | 0.564207701 |
| 24 | 0.859007558 | 1.263354546 | 0.991192428 | 3.15028354 | 0.649807367 | 0.057523907 |
| 25 | -0.399432132 | 1.902263148 | -0.501433111 | 1.105931519 | 0.161309521 | 0.406593136 |
| 27 | 0.116054861 | 1.247506354 | 0.213432041 | 1.53452411 | 0.138090044 | -0.139083878 |
| 29 | -1.107858735 | -4.424588864 | -1.228812623 | -3.889428977 | -0.323272777 | -0.991099814 |
| 30 | -0.801466908 | 1.200545433 | -0.852596849 | -3.889428977 | -0.282311258 | -0.259976833 |
| 31 | 0.037846224 | 1.178188675 | -0.094179728 | 0.86672361 | -0.207774799 | -0.224890539 |
| 32 | -0.599171738 | 0.376598349 | -0.486391958 | 0.712641171 | 0.163555602 | -0.569548926 |
| 41 | -1.992947702 | -4.424588864 | -1.701311106 | -3.889428977 | -0.95033469 | -0.982223967 |
| 42 | 0.525329593 | 1.84747919 | 0.695806733 | 2.61658639 | 0.559564416 | 0.743281549 |
| 48 | -0.64289789 | 0.797952473 | -0.544035308 | 0.918584867 | -0.265521818 | -0.332157017 |
| 49 | -0.512756765 | 0.898363041 | -0.273512229 | 0.904884682 | 0.323812109 | -0.344416579 |
| 65 | 0.37395387 | 0.597410189 | 0.273607286 | 1.101084592 | 0.069979343 | -0.352289034 |
| 2 | -0.803741244 | -4.424588864 | -0.459761793 | -3.889428977 | 0.05462147 | -0.231715541 |
| 3 | 0.126288259 | 2.336129263 | 0.043025716 | 1.498173965 | 1.067117646 | 0.864753361 |
| 4 | -0.59034474 | 0.708334295 | -0.371606523 | 1.171431582 | 0.585770799 | 0.046976161 |
| 6 | 0.224466749 | 2.006454622 | 0.361035398 | 1.535835655 | 0.520661379 | 0.358602592 |
| 8 | 0.882112692 | 2.372819945 | 0.883959574 | -3.889428977 | 1.165191013 | 1.060829553 |
| 14 | -0.123221844 | 0.909624096 | -0.173029688 | 1.503332394 | -0.421378201 | -0.45626123 |
| 16 | 0.23026337 | 2.672398562 | 0.455715547 | 2.43657141 | 0.684793277 | 1.518701285 |
| 21 | 1.064391669 | 1.471681568 | 0.676704792 | 2.703436155 | 0.19786675 | -0.045580884 |
| 22 | 0.670856815 | 1.41494476 | 0.626552275 | 2.260361197 | 0.041485805 | 0.013478787 |
| 23 | -0.051284374 | -4.424588864 | 0.031002254 | 1.810735851 | -0.524800641 | -0.457839015 |
| 26 | -0.456588559 | 1.236178406 | -0.493360685 | 0.940217206 | -0.824115269 | -0.575303828 |
| 45 | -1.104520919 | -4.424588864 | -1.231349519 | 1.611294592 | -1.583987883 | -0.708553028 |
| 58 | -0.331426994 | -4.424588864 | -0.385306237 | 2.069026478 | -0.12774246 | -0.584558714 |
| 60 | -0.53257282 | 0.902709236 | -0.528257905 | 0.594245935 | -0.012990104 | 0.004755265 |
| 64 | -0.322631517 | -4.424588864 | -0.059422473 | 3.207065713 | -2.553956788 | 1.968239396 |
| 68 | 0.077269491 | -4.424588864 | -0.389147355 | -3.889428977 | 0.727590598 | -0.350514536 |

| 1907 | 1988 | 2040 | 2070 | 2081 | 2111 | 2192 |
|---|---|---|---|---|---|---|
| -0.132716908 | -0.071925958 | -0.475799611 | -0.489967431 | 0.177449731 | -0.52272738 | -0.139478393 |
| 0.05881066 | 0.144248299 | -0.384215946 | -0.30612713 | 0.018562421 | -0.546713561 | -0.040514766 |
| 0.559311902 | 1.599680191 | 1.209143046 | 1.252914941 | 1.042344872 | 0.761411067 | 1.323133231 |
| -0.503571867 | -0.225174484 | -0.702745759 | -0.709145525 | -0.337029749 | -1.023159003 | -0.565088394 |
| -0.959836209 | -0.558184251 | 0.070729595 | -0.223080212 | -0.514427233 | -0.677999616 | -0.763231068 |
| 0.131458922 | 0.01170908 | -9.265786012 | -0.076383685 | 0.0090577 | 0.183866977 | -0.019755286 |
| 1.480189895 | 0.550619995 | 0.885706796 | 0.914582574 | 1.414092385 | 1.219949035 | 0.526479018 |
| -0.698776914 | -0.13485791 | -0.356262808 | -0.425807248 | -0.635289463 | -0.771039844 | -0.438749175 |
| 0.269072229 | 0.719776871 | 0.170341081 | 0.247226377 | 0.490178809 | 0.367505834 | 0.469188768 |
| -0.075926427 | 0.370584482 | 0.482480149 | 0.64589605 | -0.091095711 | 0.192157786 | 0.513534506 |
| 0.542456527 | 0.828711409 | 0.321884509 | 0.489569347 | 0.212146981 | 0.489464467 | 0.809715301 |
| -0.055942437 | -0.308424538 | 0.230819357 | -0.371035109 | -0.225986489 | -0.218822814 | -0.438344882 |
| -0.404685996 | 0.128952804 | 0.301486771 | -0.207997158 | -0.689003887 | -0.692010762 | -0.161682498 |
| 0.225140166 | 0.343869706 | 0.254765556 | -0.096653021 | -0.221817367 | -0.071297421 | 0.002794696 |
| 0.189083167 | -0.349019653 | -0.146648908 | -0.048099533 | -0.470602926 | -0.324378694 | -0.746038985 |
| -0.080347479 | 1.054869614 | 0.41716916 | 0.641593924 | -0.077856734 | 0.151327697 | 0.681279692 |
| 0.014379076 | 0.879406809 | 0.12140363 | 0.427906459 | -0.465921119 | 0.112184892 | 0.671535094 |
| 0.361945514 | 0.231617539 | 0.630328593 | 0.441859129 | 0.401508807 | 0.410044645 | 0.14648217 |
| 0.922738883 | 0.685360141 | 0.530238286 | 0.486824998 | -0.001181243 | 0.650940228 | 0.321836869 |
| -6.174642558 | 0.557932478 | -0.060130406 | 0.015322058 | -0.542420044 | -0.13204649 | 0.189572336 |
| 0.378204922 | -6.127482498 | 0.54172739 | 0.515826827 | 0.299007322 | 0.519669119 | -0.148588324 |
| 0.216496555 | 0.010424525 | 0.480667541 | 0.410795536 | 0.087511146 | 0.011241281 | -0.285520318 |
| -0.573842477 | -0.106285794 | -0.372492772 | 0.160592323 | -1.435720786 | -0.356958908 | -0.370847364 |
| 1.00906556 | 0.36816063 | 0.112440882 | 0.109458459 | 0.408552165 | 0.800460237 | 0.084701884 |
| -0.69083406 | -0.627245217 | -0.309602369 | -0.475475368 | 0.078531141 | -0.59448236 | -0.815917889 |
| 0.06683928 | 0.595863332 | -0.098602949 | 0.194208863 | 0.061723268 | -0.112179333 | 0.248679996 |
| 0.56327012 | -0.493160691 | 0.480573516 | -0.197230009 | 0.487420377 | -0.17012335 | -0.112490292 |
| -0.03493762 | -0.019849976 | -0.309540972 | 0.090501019 | -0.354620053 | -0.37189357 | -0.290737812 |
| -0.183058971 | -1.037977345 | 0.163808452 | 0.05047002 | -0.17524905 | -0.290069497 | -0.550060446 |
| -0.079408045 | -0.702742278 | -2.488112301 | -1.397830459 | -1.62993225 | -0.060609529 | -0.756402059 |
| 0.115360099 | -0.461344202 | -0.238597542 | -0.246075864 | 0.147932757 | -0.11333228 | -0.787721626 |
| 0.047413923 | 1.371090562 | 0.406410303 | 0.780987334 | 0.607626205 | 0.32594868 | 1.027362351 |
| -0.028415274 | -0.347547657 | -0.325499873 | -0.729569554 | -0.035325695 | -0.267032603 | -0.41467762 |
| -0.632259192 | -0.102968945 | -0.360937755 | -0.213269043 | -0.2508568 | -0.676261946 | -0.233142598 |
| -1.394719065 | -0.612998242 | -0.31518217 | -0.161996892 | -1.110926722 | -1.151156128 | -0.814739304 |
| -1.090653545 | 0.039559686 | -0.640413425 | 0.107790838 | -0.851375108 | -0.852703661 | -0.218192927 |
| 0.473431758 | 0.485566093 | 0.396008282 | 0.482120534 | 0.150630432 | 0.191793461 | 0.173863187 |
| 0.83622786 | -6.127482498 | 0.222726174 | 0.075193159 | 0.331236234 | 0.525558598 | -0.394551498 |
| -6.174642558 | -6.127482498 | -0.897993688 | -0.886053836 | -1.184417608 | -6.695868434 | -1.044649121 |
| 1.832633327 | 1.517744387 | 1.332473591 | 1.809099861 | 0.846282004 | 1.706979555 | 1.305338108 |
| 0.702763964 | 0.600220077 | -0.28156385 | -0.182529043 | -0.28337351 | -0.183026981 | 0.238563314 |
| 0.501060558 | 0.04130602 | 0.264992268 | 0.284693749 | -0.033247971 | 0.486347976 | -0.0307868 |
| 1.619577329 | 1.974444136 | 1.595247087 | 1.458636009 | 1.229962601 | 1.69833354 | 1.867456602 |
| 0.864339376 | 1.052832151 | 1.151840953 | 1.159590364 | 0.588572458 | 0.886281918 | 0.733773211 |
| -0.223442125 | -0.162282483 | 0.347237029 | 0.153421001 | -0.273401434 | -0.202114594 | 0.053308244 |
| -0.785136046 | -0.842708816 | -0.653317274 | -0.820607832 | -0.992274124 | -0.906964595 | -1.17319529 |
| 1.143162541 | 0.47479844 | 0.433626512 | 0.278932404 | 0.512505834 | 0.66892735 | 0.15131288 |
| -6.174642558 | -0.297871836 | 0.045195543 | -0.295065896 | -0.105939813 | -0.517282361 | -0.320200366 |
| 0.448742618 | 0.96049889 | 0.861451574 | 0.607069046 | 0.135351695 | 0.345928524 | 0.568443534 |
| -0.573842477 | 0.899895393 | 0.340953896 | 0.557854137 | -0.565548035 | -0.573245098 | 0.476785089 |
| 2.039977282 | 1.52954709 | 1.218705666 | -7.07727145 | 1.812218598 | 1.997963531 | 1.338448363 |
| 1.489312945 | 1.475138722 | 0.756536404 | 0.742116108 | 1.210944832 | 1.146503858 | 1.144048577 |
| 0.414300294 | 1.027123558 | 0.292301233 | 0.087743512 | 0.750542453 | 0.291918563 | 0.653182131 |
| 0.645178841 | -0.106719238 | 0.059724461 | 0.189896688 | 0.18345056 | 0.402940011 | -0.371981494 |
| 0.309974502 | 0.640928608 | 0.123262252 | -0.069897106 | -0.628641808 | 0.099078024 | 0.106972673 |
| 0.146232788 | -0.976809247 | -0.319567758 | -0.784953032 | -0.124412301 | -0.077558686 | -1.084531185 |
| -0.721205594 | -0.443174133 | -0.185142218 | -0.437488991 | -0.457193448 | -0.728836861 | -1.00843899 |
| -0.293876824 | -0.226313937 | -0.510740828 | -0.536636636 | -1.703476045 | -0.623290324 | -0.545213346 |
| -0.01637808 | -0.317839633 | 0.093978581 | -0.235973993 | 0.220652496 | 0.332301336 | -0.483826997 |
| -0.346782964 | -1.191468128 | -0.712148394 | -0.844870211 | -1.003930919 | -0.427516475 | -1.375961258 |
| 1.38574168 | 1.181753772 | 0.819615772 | 0.83791589 | 0.958424201 | 1.125287691 | 0.934638499 |
| -0.385738719 | -0.013379 | 0.106818971 | -0.11114619 | -0.388800367 | -0.237679438 | -0.350464827 |
| 0.583935763 | 0.071866919 | 0.406301528 | -0.18011981 | -0.035866611 | 0.286408847 | -0.166983964 |
| 0.374377834 | 0.467988965 | -0.094639497 | 0.271415666 | 0.025162379 | -0.291151717 | 0.288202165 |
| 0.417977881 | -0.030639578 | 0.159919163 | 0.020799144 | 0.366781006 | 0.294059151 | -0.089556816 |
| 0.006778019 | -1.101176975 | 0.779086905 | 0.705029522 | 0.697382442 | -0.187010592 | -0.249406087 |
| 0.784213139 | 0.132827099 | 0.466912513 | 0.388015815 | 0.821710743 | 0.782893294 | -0.118563645 |
| 0.756747864 | 0.716102446 | 0.459264572 | 0.473646383 | 0.222389959 | 0.133268717 | 0.541574481 |
| 1.892289872 | 1.277644013 | 0.607766251 | 1.088586259 | 1.648959404 | 1.563552789 | 0.998264861 |
| -0.203046712 | 0.302601737 | -0.476533943 | -0.424731053 | -0.349082656 | -0.364769139 | -0.087206772 |
| 0.677411228 | 0.875842667 | 1.086161139 | 1.392253448 | 0.722611381 | 0.757176005 | 0.677181299 |
| 0.267935521 | 1.148744535 | 0.230300631 | -0.002499633 | 0.284883429 | 0.154484704 | 0.958494145 |
| 0.303775645 | 1.10385619 | -0.22882303 | 0.042267867 | 0.242125264 | 0.155744721 | 1.100092967 |
| 0.027242082 | 0.33786579 | -0.266873859 | -0.302041267 | -0.633169503 | -0.268667486 | -0.048283754 |
| -0.194732604 | -0.233834245 | -0.925972324 | -0.700099185 | -0.785043712 | -0.248468244 | -0.739052942 |
| -0.742325159 | -0.675575834 | -1.178683669 | -1.055930305 | -1.067492469 | - 1.229014566 | -1.174249409 |
| 0.049892297 | -0.13188958 | 0.029414019 | -0.180240134 | 0.05155464 | 0.248918809 | -0.397263417 |
| 0.088392827 | -0.232194578 | 0.220095167 | 0.360229338 | -0.0354921 | -0.110945506 | -0.595484455 |
| 0.321270177 | 0.564158041 | -0.017279741 | 0.044625076 | -0.14880847 | -0.345356759 | -0.139928175 |
| 2.07423225 | 0.172297986 | 0.879808898 | 0.008420788 | 0.958270201 | 1.736943684 | -0.224537609 |

| 2244 | 2274 | 2285 | 2315 | 2396 | 2418 | 2431 |
|---|---|---|---|---|---|---|
| -0.584353295 | 0.731353935 | -0.258957006 | -0.416433819 | -1.002341872 | -0.123393935 | -0.210174319 |
| -0.752374052 | -3.693492697 | -0.3536771 | -0.625452977 | -0.45811902 | -0.155018493 | -0.173645038 |
| 1.260328713 | 2.593303163 | 1.221010003 | 1.070501639 | 1.060419165 | 0.985917269 | 0.229870121 |
| -0.997429476 | -3.693492697 | -0.395436403 | -0.562058945 | -0.526969628 | -0.756742705 | -0.347038066 |
| 0.090600961 | 1.097860628 | -0.307532275 | -0.389176426 | -0.766296247 | -0.529850842 | -0.519365353 |
| 0.046195395 | 1.004169849 | 0.19733801 | 0.219888458 | -0.180779989 | -0.031285034 | -0.189915632 |
| 0.88413382 | -3.693492697 | 1.13132473 | 0.978925963 | 0.637123242 | -0.044503643 | -0.106528333 |
| -0.592627631 | -3.693492697 | -0.618756556 | -0.629310205 | -0.834884049 | -0.862067542 | -0.514891681 |
| 0.028364594 | 1.278220252 | 0.436164248 | 0.473100083 | -0.231713521 | -0.289662949 | -9.848175297 |
| 0.720661731 | -3.693492697 | 0.158583033 | 0.186135108 | 0.770000831 | 1.0299478 | 0.25972626 |
| 0.371011367 | 1.27491419 | 0.381894965 | 0.389652373 | 0.724165408 | 0.329894411 | 0.356980439 |
| -0.231087036 | 0.988858964 | -0.329286186 | -0.344143333 | -0.412077676 | -7.314078941 | -3.441295311 |
| 0.161844777 | 1.48440423 | -0.538634694 | -0.54661377 | -0.245420834 | -0.335343877 | 0.271435508 |
| -0.104848219 | 1.553155364 | -0.326775939 | -0.053827154 | -0.074049264 | -0.430128573 | 0.356200932 |
| -0.41273507 | 1.484739879 | -0.611484802 | -0.332808115 | -0.84186142 | -0.552395949 | 0.000264428 |
| 0.44486003 | 1.974086729 | -0.149740504 | 0.40962584 | 0.386099078 | 0.16009884 | 0.608480089 |
| 0.343081776 | 1.798686523 | -0.057408412 | 0.470530099 | 0.254061196 | -0.209925066 | 0.43070427 |
| 0.59319152 | 1.874215472 | 0.511196862 | 0.540197077 | 0.323407637 | 0.38288558 | 0.497808945 |
| 0.059982621 | 2.550073006 | 0.028872748 | 0.679001724 | 0.458499724 | 0.356060805 | 0.53816233 |
| -0.164214683 | -3.693492697 | -0.510997064 | -5.928757885 | 0.107903624 | -0.110425435 | 0.39142048 |
| 0.285706377 | -3.693492697 | 0.353262406 | 0.522126203 | -0.001081131 | 0.452282467 | 0.167241684 |
| 0.277528905 | 1.926908168 | 0.049977952 | 0.251800868 | -0.127553608 | -0.164975398 | 0.50993934 |
| 0.108485831 | 1.58886465 | -0.767818722 | -0.076486989 | 0.327993784 | -0.317484591 | 0.301173881 |
| -0.341778488 | 1.371886281 | 0.030875751 | 0.645088536 | 0.323905486 | 0.643390123 | 0.483535857 |
| -0.445040508 | 0.762928858 | -0.255296866 | -0.494620081 | -0.573678365 | -2.172067352 | -0.049595982 |
| -0.205566885 | 0.947802757 | -0.116864374 | -0.169443097 | -0.043733365 | 0.491725509 | 0.330496464 |
| -0.263002603 | 1.02085215 | -0.208532164 | -0.4837667 | 0.045860782 | 0.311568395 | 0.065698017 |
| -0.085655607 | 1.000364049 | -0.20763672 | -0.179819961 | -0.418009652 | -0.314765099 | 0.363468123 |
| 0.300097532 | -3.693492697 | -0.097935729 | -0.213180169 | -0.219706982 | 0.482860117 | 0.482037456 |
| -0.74087083 | -3.693492697 | -0.750268372 | -0.132410698 | -0.361659139 | -0.016947609 | 0.286456572 |
| -0.556445681 | 1.163362219 | -0.05769988 | -0.201483673 | -0.292872994 | 0.046751776 | 0.306572978 |
| 0.311487673 | 1.873305719 | 0.730436011 | 0.658874115 | 0.335817956 | -0.137988249 | -0.029319488 |
| -0.68494995 | 1.13520192 | -0.168521892 | -0.4125967 | -0.45736609 | -0.269994104 | -0.176222805 |
| -0.483800031 | 0.782083911 | -0.605093112 | -0.502969117 | -0.245217888 | -0.603700845 | 0.054227739 |
| -0.108880517 | 1.342531831 | -0.678180424 | -0.655514156 | -0.484577385 | -0.279282032 | 0.052724983 |
| -0.442486041 | 1.366612802 | -0.708652247 | -0.365150136 | -0.110206241 | -0.099811073 | 0.105979342 |
| 0.241518434 | 1.377879257 | -0.00758769 | 0.236534912 | 0.154916406 | -0.354544771 | 0.17890852 |
| -0.297800887 | -3.693492697 | 0.246636829 | 0.271799583 | 0.072255174 | -0.019531819 | 0.089028492 |
| -0.673077452 | -3.693492697 | -0.974642557 | -5.928757885 | -1.203067052 | -0.81735782 | -0.561740662 |
| 1.482601311 | 2.573052536 | 1.180226364 | 1.60578853 | 1.41275054 | 0.655258212 | 0.643718437 |
| -0.525818906 | 2.165766109 | -0.683850386 | 0.124702892 | -0.09507381 | 0.040222455 | -0.193245263 |
| -0.054138786 | -3.693492697 | -0.019000497 | 0.271920308 | -0.028804274 | -0.073437781 | 0.165958242 |
| 1.723129503 | 3.185201513 | 1.641109173 | 1.766664399 | 1.309928207 | 1.131590117 | 0.431542247 |
| 1.209886489 | 2.376612476 | 0.821595231 | 1.148328189 | 0.61753489 | 0.958579847 | 0.580635493 |
| 0.386076826 | 1.657518768 | 0.065708255 | -0.025961931 | 0.411077016 | -0.050740981 | 0.264018276 |
| -0.764873232 | 1.156396138 | -1.127606305 | -0.716543217 | -0.955158659 | -0.420664091 | 0.041273094 |
| -0.248560162 | -3.693492697 | 0.242064869 | 0.61333662 | 0.265410202 | 0.703864227 | 0.345098808 |
| -0.156909628 | -3.693492697 | -0.179021646 | -0.249926985 | 0.014742733 | 0.010458123 | -0.087878565 |
| 0.803542821 | 2.398382636 | 0.248708103 | 0.549843188 | 0.539618402 | 0.14426511 | 0.681380866 |
| 0.553226746 | 2.021496587 | -0.398769369 | -0.147169635 | 0.48057553 | 0.289670652 | 0.273564818 |
| 1.128498072 | 2.37358167 | 1.832757151 | 1.806477729 | 1.531056383 | 1.008384602 | 1.055748038 |
| 0.538180494 | 1.756625652 | 0.946901935 | 0.96417378 | 1.05825378 | 0.665459706 | 0.610508085 |
| -0.124643714 | 1.68481243 | 0.519029006 | 0.631110255 | -0.273760283 | 1.11288732 | 0.025364699 |
| -0.143074995 | 1.089923291 | -0.111246914 | 0.160152007 | -0.139287993 | 1.032393712 | 0.35779453 |
| -0.073762104 | 1.309894168 | -0.444242912 | 0.053707962 | 0.431397059 | 0.075801149 | 0.398608097 |
| -0.738838039 | -3.693492697 | -0.132090112 | -0.199934426 | -0.999605419 | 0.474112874 | -0.305082182 |
| -0.511993749 | 0.998728547 | -0.674739783 | -0.516686753 | -0.792608088 | 0.557413929 | -0.268598376 |
| -1.101850618 | 0.59973525 | -1.013247139 | -0.793730053 | -0.593986907 | -0.661308082 | -0.460646561 |
| -0.277877481 | 1.137104146 | -0.130230138 | 0.091137922 | -0.021747005 | -0.321085682 | 0.10488386 |
| -0.762862835 | -3.693492697 | -0.889459449 | -0.45413903 | -0.968872115 | 0.186338664 | -0.147103414 |
| 0.907476671 | 2.569656101 | 1.07095006 | 1.087734346 | 0.773377246 | 0.089852321 | 0.323774647 |
| 0.063985778 | 0.790182214 | -0.193107619 | -0.294223416 | -0.114648507 | 0.362924402 | 0.210933282 |
| 0.000194274 | -3.693492697 | 0.114959483 | 0.079938294 | 0.034266331 | 0.368727131 | 0.313148886 |
| -0.192614287 | 1.14514848 | -0.270533581 | -0.288921514 | 0.431981426 | 0.167067556 | 0.434023441 |
| -0.019438302 | -3.693492697 | 0.196482499 | 0.347012506 | 0.331115598 | 0.080384898 | 0.241677708 |
| 0.420549437 | 1.376384377 | 0.234382626 | -0.089570773 | -0.200118741 | 0.532841538 | 0.452494744 |
| 0.226679141 | 1.781874418 | 0.573608402 | 0.856143488 | 0.089972201 | 0.255989916 | 0.330482903 |
| 0.292803058 | 1.291743385 | 0.150516612 | 0.159746946 | 0.586133088 | 0.309039199 | 0.415644669 |
| 0.282105425 | 2.291822026 | 1.007139946 | 1.667395219 | 0.89014801 | 0.406160901 | 0.663069795 |
| -0.803336413 | 1.279457212 | -0.611158813 | -0.142408419 | -0.380918095 | -0.554602725 | -0.425734025 |
| 1.363236055 | 2.233242834 | 0.917931173 | 0.863252459 | 0.544021349 | 0.757808809 | 0.506425408 |
| 0.088184474 | 2.125565263 | 0.415818071 | 0.486923426 | -0.194959369 | 0.417977087 | 0.262384011 |
| -0.36461883 | -3.693492697 | 0.118963594 | 0.265015626 | 0.395516793 | 0.485547515 | 0.170220262 |
| -0.227679151 | -3.693492697 | -0.356564658 | -0.322255783 | -0.277735378 | 0.695117954 | -0.022479416 |
| -1.136852296 | -3.693492697 | -0.638621367 | -0.180578859 | -0.482945965 | -0.320921714 | -0.138836829 |
| -1.136365877 | -3.693492697 | -0.943665404 | -0.923374453 | -1.04160989 | 0.194662542 | -0.257305932 |
| -0.33007785 | -3.693492697 | 0.174457227 | 0.264894064 | -0.147229002 | -0.224458558 | 0.124149003 |
| 0.228561665 | 1.796723236 | 0.031542528 | 0.069794579 | -0.011628403 | 0.060617246 | 0.294670773 |
| -0.207184103 | -3.693492697 | -0.277824617 | -0.150663377 | 0.152419843 | -0.098016471 | -0.130444481 |
| 0.844396006 | 2.128566518 | 1.175972539 | 1.201893296 | -0.449784805 | 0.133707026 | 0.163522635 |

| 2448 | 2489 | 2592 | 2605 | 2635 | 2646 | 2663 |
|---|---|---|---|---|---|---|
| -0.747927154 | 0.062104534 | 0.396612232 | -0.158136835 | -0.247432389 | 0.381633965 | 0.861693542 |
| -1.940903153 | -0.2455485 | 0.245266603 | -0.392735671 | -0.802991507 | 0.112685057 | 0.019125354 |
| 0.386525851 | 1.608962217 | -5.166538875 | 0.400961639 | 0.509333832 | 0.328700108 | 0.346227768 |
| -0.964649592 | -0.34557884 | -0.323664987 | -0.961707775 | -0.717811809 | -0.609434684 | -0.446061097 |
| -2.249004413 | -0.303024956 | 0.028004019 | -0.39753635 | -0.51254708 | -0.873604544 | -0.660206914 |
| -0.034188559 | 0.475889949 | 0.500834802 | -0.009841337 | -0.027754056 | 0.201835204 | 0.475294259 |
| -0.221938909 | 1.066390059 | 0.807028371 | 0.358480981 | 0.114968825 | 0.216858889 | 0.236960062 |
| -0.750814563 | -0.489281671 | -0.307834504 | -0.626513889 | -0.780385683 | -0.726295415 | -0.283754964 |
| 2.832378808 | -0.740790176 | 0.335884837 | -0.007510437 | -0.139464664 | 0.408033808 | 0.448298899 |
| 0.15124005 | 0.409561854 | 0.930571861 | 0.284751434 | 0.103747144 | -0.471158951 | -6.236967376 |
| 0.096609389 | 0.721507521 | 0.716053707 | 0.589309405 | 0.286205477 | 0.201446998 | -0.130395327 |
| -1.476003079 | -0.39868811 | -0.086244265 | -0.267189081 | -0.334343657 | -0.376502559 | -0.783530412 |
| -0.351598521 | -0.313106502 | 0.199047656 | 0.010820168 | -0.46997691 | -0.548024139 | -0.5326295 |
| -1.247705944 | -0.065543126 | 0.359151182 | 0.151743217 | -0.085832129 | -0.093841897 | -0.370600498 |
| -0.898472698 | -0.538734499 | -0.136334408 | -0.556289869 | -0.361017633 | -0.650904802 | -0.588706001 |
| -0.11327289 | 0.32965318 | 0.674898423 | 0.229147222 | 0.139903886 | -0.058983553 | 0.113752117 |
| -0.920409269 | 0.508645304 | 0.525428038 | 0.384303436 | 0.12829295 | -0.089677285 | -0.049534732 |
| 1.131343498 | 0.776551153 | 1.115260473 | 0.564783818 | 0.57761322 | 0.37732827 | 0.424064958 |
| -0.080822552 | 0.609989023 | 1.323425646 | 0.325465313 | 0.400936134 | 0.383415803 | 0.146257687 |
| -0.113836166 | -0.05795025 | -5.166538875 | -0.198136264 | 0.21373854 | -0.065427607 | -0.085146952 |
| 0.366264181 | 0.457955624 | 0.822512047 | 0.126419569 | 0.148006594 | 0.160935581 | 0.139192517 |
| -0.237210796 | 0.110611995 | 0.407062597 | -0.096482768 | -0.015864231 | -0.378754305 | -0.358864848 |
| -0.154829888 | -0.133695811 | 0.199325874 | 0.019010278 | 0.149205465 | -0.683999716 | -0.521193594 |
| 0.415908191 | 0.660522761 | 1.196366068 | 0.200572618 | 0.430555446 | 0.37732827 | 0.595448403 |
| -0.062992915 | -0.532452027 | -0.18947678 | -0.502567844 | -0.314924892 | -0.6502221 | -0.788384418 |
| 1.12562678 | 0.118904876 | 0.674379599 | -0.097828685 | -0.382288009 | 0.020169374 | 0.279841881 |
| 1.266131782 | -0.219214673 | 0.640959907 | -0.182165438 | -0.544726269 | 0.27181215 | 0.131260649 |
| 0.694846732 | 0.127272406 | -0.049543565 | -0.071613805 | -0.164936059 | -0.414051257 | -0.466825123 |
| 0.860381103 | 0.046017957 | 0.812618101 | 0.107018726 | -0.12962776 | -0.310719096 | 0.145050055 |
| -0.122729569 | -0.180192867 | 0.186728781 | -0.422484373 | -0.383771744 | -0.230885765 | 0.541045242 |
| 0.114769029 | 0.081232506 | 0.236460205 | -0.342049841 | -0.445791692 | 0.044879583 | 0.296250285 |
| 0.34868003 | 1.018224086 | 0.808239612 | 0.48462861 | 0.284208956 | 1.2709189 | 0.695236583 |
| -1.227157106 | 0.026355589 | -0.15836693 | -0.561057831 | -0.949559328 | 0.008935667 | -0.421629329 |
| -0.852953877 | -0.354635065 | -0.09890832 | -0.263461407 | -0.726925744 | -0.448220441 | -0.302010195 |
| -0.027225981 | -0.637732726 | 0.055038854 | -0.284912486 | 0.029961795 | -1.35163851 | -0.271309157 |
| 0.403078514 | -0.296093303 | -0.070818662 | -0.42401014 | 0.11541959 | -1.011868661 | -0.614196994 |
| -0.187041811 | 0.259625825 | 0.087291555 | 0.135883729 | -0.159878631 | 0.034839338 | -0.340369655 |
| -0.483806472 | 0.361460456 | 0.773757683 | -0.174154901 | 0.057676468 | 0.434600658 | 0.182891821 |
| -0.953777474 | -0.72068911 | -5.166538875 | -0.868402833 | -0.664806569 | -0.835103917 | -0.288870223 |
| 0.755870309 | 1.537323133 | 1.428363033 | 1.086910736 | 1.082251176 | 0.798282702 | 0.5935211 |
| 1.035578572 | -0.323440168 | 0.731203516 | -0.225389599 | -0.285896764 | -0.16827295 | -0.223310436 |
| -0.064064497 | 0.183017773 | 0.627997963 | -0.061984494 | -0.195522261 | -0.048958155 | 0.070219903 |
| 0.433616249 | 2.012160354 | 1.595488486 | 0.896136321 | 0.974506576 | 1.063621325 | 0.888430638 |
| 1.327587753 | -7.003712135 | 1.355623948 | 0.595430214 | 0.588833989 | 0.319943786 | 0.273485815 |
| 0.412680514 | 0.179860465 | 0.632156875 | 0.375423466 | -0.03257598 | -0.140835246 | -0.19207491 |
| -0.491292062 | -0.765131776 | 0.194585604 | -0.454795119 | -0.394407561 | -1.102753348 | -0.698087623 |
| 0.281332334 | 0.546517138 | 1.243283501 | -0.166765368 | 0.299039717 | 0.554782976 | 0.101626702 |
| 0.02097421 | -0.21524996 | 0.397981532 | -0.210735843 | -0.344642409 | -0.372358764 | -0.314208752 |
| -0.118272981 | 0.478674878 | 1.008833272 | 0.544838361 | 0.317435226 | 0.010699274 | -0.055423742 |
| -0.364545947 | -0.052521138 | 0.540966818 | 0.071659667 | 0.088586289 | -0.861729263 | -0.192921674 |
| 1.476452748 | 1.832357209 | 1.708471818 | 1.032873652 | 1.234086757 | 1.27722853 | 1.009544942 |
| 1.354770639 | 1.227714608 | 1.200769873 | 0.58773767 | 0.797760263 | 1.104263287 | 0.857109386 |
| -0.05211274 | 0.919398902 | 1.554729039 | 0.52903135 | 0.575055092 | 1.136786731 | 0.619607384 |
| 0.396722192 | 0.122308148 | 1.013034024 | -0.064875411 | 0.28106349 | 0.255668498 | 0.570954413 |
| -1.169778171 | 0.027987056 | 0.773287707 | 0.216804492 | 0.381094213 | -0.066061618 | 0.185409543 |
| -0.590568867 | 0.110030848 | 0.863430699 | -0.312176707 | -0.965370975 | 0.55662205 | 0.131260649 |
| -0.887071666 | -0.556217625 | 0.354159847 | -0.560425649 | -0.281530532 | -0.693931694 | 0.145654053 |
| -0.721421852 | -0.850245161 | -0.342615715 | -0.834696326 | -0.449947399 | -0.518079359 | -0.888500226 |
| -0.199240485 | -0.486902878 | 0.211766252 | -0.188789677 | 0.038636996 | -0.360540117 | -0.33011449 |
| -0.09131287 | -0.379299153 | 0.709060439 | -0.314664016 | -0.276849658 | -0.270239306 | 0.974492708 |
| 0.505810872 | 0.936566504 | 1.214471081 | 0.632473044 | 0.956595477 | 0.932182778 | 0.743540365 |
| 0.029797921 | 0.056549616 | 1.022116293 | 0.227484875 | -0.222166406 | -0.256334744 | 0.280457682 |
| 0.119496928 | 0.389616891 | 0.868144791 | 0.141091766 | -0.239046325 | 0.358591282 | 0.381103827 |
| 0.063660428 | 0.06666802 | 0.174826578 | 0.019037511 | 0.126623185 | -0.136606795 | -0.618293329 |
| 0.572453901 | 0.311522096 | -5.166538875 | 0.029312853 | 0.188192385 | 0.336108622 | 0.339701138 |
| 1.118023746 | -0.089974785 | -5.166538875 | -0.115632696 | -0.173923806 | -0.752155924 | 0.145553412 |
| 0.612254537 | 0.678901968 | -5.166538875 | 0.321588965 | 0.626637214 | 0.880527269 | 0.356549975 |
| 0.698032668 | 0.323763307 | -5.166538875 | 0.340930733 | 0.37962468 | 0.49363752 | 0.330458236 |
| 0.444405738 | 1.160147463 | 0.980070755 | 0.436912324 | 0.881507165 | 1.074464942 | 0.369344915 |
| -0.396278631 | -0.248664134 | -0.064279842 | -0.642797654 | -0.309740668 | -0.377368016 | -0.366393367 |
| 1.08868998 | 1.135175898 | 1.429501818 | 0.963101962 | 0.742324793 | 0.437057888 | 0.646434221 |
| -0.221759566 | -7.003712135 | 0.215878889 | 0.598795056 | 0.321294177 | 0.832791885 | 0.444424889 |
| 0.551612392 | 0.72972643 | 1.116372968 | 0.362386247 | 0.402036403 | 0.512448022 | 0.472540195 |
| -0.053248476 | -0.210232657 | 0.876941919 | -0.229492667 | -0.213518961 | -0.167149639 | 0.620171071 |
| -1.448721333 | -0.839420365 | 0.029323803 | -0.919060268 | -0.374166587 | -0.309909418 | -0.085273722 |
| -0.772973406 | -0.913588045 | 0.165086613 | -1.018554627 | -0.723009596 | -0.866801411 | 0.48185885 |
| -0.64711123 | 0.349560426 | -5.166538875 | -0.409190501 | -0.284383218 | -0.179433938 | -0.054194001 |
| 0.427076858 | 0.196481743 | 0.474143197 | 0.052255995 | 0.323858563 | -0.119735705 | -0.118005228 |
| 1.123550459 | -0.092403653 | 0.215331514 | -0.626085514 | -0.097891658 | -0.962795479 | -0.193204088 |
| -0.333187756 | 1.25419823 | 1.174785781 | 0.78738457 | -0.069568942 | 1.439303105 | 0.759844805 |

| 2676 | 2693 | 2738 | 2764 | 2779 | 2809 | 2850 |
|---|---|---|---|---|---|---|
| -0.026825184 | -0.090372458 | 0.113446732 | 1.746561912 | 0.60025254 | -0.168759072 | 0.419976725 |
| -0.760726008 | -0.458543145 | 0.201633454 | 1.425241019 | 0.69646586 | -0.744955429 | -0.012152061 |
| 0.156060463 | 0.814954449 | 0.177959408 | -3.858627222 | 0.218524113 | 0.154744256 | 0.839837585 |
| -0.986830685 | -4.70431359 | 0.359702595 | 0.861570885 | -0.009994976 | -0.497878973 | -0.346367272 |
| -0.317046249 | -0.111842748 | 0.047772351 | -3.858627222 | 0.088512564 | -0.374666316 | -0.619383219 |
| 0.164895532 | 0.565567558 | 1.168323178 | 1.508349627 | 0.578189713 | 0.447309918 | 0.42409237 |
| 0.499879614 | 1.061921899 | 0.944749381 | -3.858627222 | 0.099752652 | -0.307649771 | 0.381244259 |
| -0.725423288 | -0.014277183 | 0.652770101 | -3.858627222 - | -0.253473959 | -0.148710618 | -0.136478557 |
| 0.403827852 | 0.55664799 | 0.995236853 | 1.457038783 | 0.281811386 | 0.173929603 | 0.67199778 |
| 0.268359592 | 0.337112603 | 0.676597626 | -3.858627222 | 0.359075333 | 0.018038777 | -8.579266296 |
| 0.493232824 | 0.672891972 | 0.448929382 | 0.976709416 | 0.186275936 | -0.027581463 | 0.72090281 |
| -0.398941348 | -0.086250683 | -1.04324872 | -3.858627222 - | -0.204412736 | -0.927346919 | -0.197895555 |
| -0.564111445 | -4.70431359 | -1.098695293 | 1.298921744 - | -0.132863592 | -0.594342865 | -0.348045528 |
| -0.053390418 | 0.105157059 | -0.494732212 | 1.080375666 - | -0.334976676 | -1.116077326 | 0.14957414 |
| -0.899213927 | -4.70431359 | -0.465554918 | 1.274295937 - | -0.021931835 | -0.928678286 | -0.729325144 |
| 0.19310624 | 0.689259842 | -0.858340391 | 1.216472186 | -2.087193197 | -0.732075969 | 0.353812128 |
| 0.597981714 | 0.226126868 | -1.011398894 | 1.090132669 - | -0.230048044 | -0.924179255 | 0.4793704 |
| 0.744136568 | 1.263632506 | 0.664021442 | 1.656576063 | 0.448626662 | 0.723841929 | 0.654097468 |
| -0.087621206 | -4.70431359 | -6.910121389 | -3.858627222 | -0.27784007 | -0.961810112 | 0.18570545 |
| -0.15793097 | -4.70431359 | -0.682526556 | -3.858627222 - | -0.373646707 | -0.887372288 | -0.246685856 |
| 0.679348543 | 0.902188511 | 1.205488436 | 2.641410414 | 0.397235916 | 0.336256789 | 0.38514918 |
| -0.151943831 | 0.204394441 | -0.520680019 | 1.271834873 | -0.139825442 | -0.769823097 | -0.204311683 |
| 0.383629306 | 0.297873419 | 0.232037616 | 1.05008081 - | -0.084288189 | -0.353687626 | -0.103108416 |
| 0.595611064 | 0.836669804 | 0.553084553 | 1.763066803 | 0.729208791 | 0.248911128 | 0.426388586 |
| -0.27881993 | -0.06359242 | -0.111583259 | 1.565588312 | 0.367364743 | 0.504372119 | -0.332214677 |
| 0.209080561 | 0.710939893 | 1.471099337 | 1.851642104 | 0.664075681 | 0.884357179 | 0.187803253 |
| -0.691949821 | 0.37746042 | 0.932437903 | 1.82729835 | 0.679211339 | 1.289128699 | -0.268529287 |
| -0.211845911 | 0.207465562 | 0.131623511 | 1.017478449 | 0.010203217 | 0.474454621 | 0.256387553 |
| 0.222800879 | 0.424386887 | 1.070557932 | 1.824466519 | 0.816822506 | 0.628708224 | -0.17945265 |
| 0.703656888 | 0.278477304 | 0.503188503 | 1.931187314 | 0.703977013 | 0.039342419 | -0.073834861 |
| 0.039067423 | 0.309412162 | 0.157827499 | 1.218330715 | 0.298127759 | 0.140422067 | 0.025902886 |
| 0.737736731 | 1.038918509 | 0.242110176 | 1.030557605 | 0.247194592 | 0.492368797 | 1.582480955 |
| -0.091645736 | -0.495896572 | -0.421242173 | 1.432461701 | 0.48873796 | -0.660330509 | 0.244875737 |
| -0.543880939 | -0.339699584 | -0.39076342 | 0.734446284 | -0.092269127 | -0.633769293 | -0.045124687 |
| -0.512026462 | 0.092951665 | 0.580474481 | 1.462289977 | 0.399486813 | 0.444977489 | -0.430624417 |
| -0.605126126 | 0.047736006 | -0.131566758 | 1.395203208 | 0.420800096 | 0.59764666 | -0.232087845 |
| 0.256678623 | 0.329386977 | 0.138161585 | 0.621681363 | -0.180618877 | -0.190931424 | 0.184310669 |
| 0.097624371 | 0.663496523 | 0.61400287 | 1.611490275 | 0.362570842 | -0.432986224 | 0.210720105 |
| -7.326896001 | 1.89009987 | 0.31300061 | -3.858627222 | -0.123515386 | -0.301719566 | -0.788338892 |
| 1.145907331 | 1.3949371 | 0.535059713 | -3.858627222 | 0.351724987 | 0.197168163 | 1.050437393 |
| -0.789704184 | -4.70431359 | 0.569413493 | -3.858627222 | -0.323982214 | 1.239939336 | -0.303950569 |
| 0.336556396 | 0.604602176 | 0.780278937 | 1.474723992 | 0.276892345 | -0.101926698 | 0.198254288 |
| 1.038141741 | 1.464848745 | 0.561485592 | -3.858627222 | 0.405579159 | -0.023780465 | 1.333251197 |
| 0.753319301 | 1.058186928 | 0.129747611 | -3.858627222 | 0.005911279 | -0.346934591 | 0.613169503 |
| 0.249109807 | 0.700560006 | 0.073260266 | 1.220926867 | 0.020951368 | 0.237562034 | 0.217889967 |
| -0.077968663 | 0.231273119 | -0.420428084 | 0.909228546 | -0.012980373 | -0.49584938 | -0.637504977 |
| -0.023052776 | -4.70431359 | -0.058319368 | -3.858627222 - | -7.638859957 | -0.259431213 | 0.316647048 |
| -0.309603228 | 0.129129825 | 1.137140551 | 1.264415131 | 0.323659382 | 0.115485251 | -0.378108928 |
| 0.164596751 | -4.70431359 | -0.9756367 | -3.858627222 | -0.166948194 | -1.765759907 | 0.20088439 |
| -0.777875609 | 0.191569784 | -6.910121389 | -3.858627222 | -7.638859957 | -0.677242026 | -0.581791325 |
| 1.388345619 | 1.724618328 | 0.622451244 | -3.858627222 | 0.574534685 | 0.488873625 | 1.369879537 |
| 0.810235244 | 1.363863952 | 1.016369462 | -3.858627222 | 0.609282858 | 0.896109329 | 1.093889731 |
| 0.460135243 | 0.829170003 | 0.322723563 | 2.666507164 | 0.896883911 | 0.16428847 | 1.167277792 |
| 0.639100033 | 0.888093845 | 1.313080983 | 2.842791872 | 1.124698398 | 0.656652727 | -3.028292809 |
| 0.118702336 | 0.440824847 | -0.236071191 | 1.456453611 | 0.330765411 | -0.430679116 | -0.096457532 |
| 0.132785714 | 0.424386887 | 0.7617664 | 2.385516562 | 0.773604794 | 0.051414113 | 0.300640238 |
| -0.584689447 | 0.04258348 | 0.072100641 | 2.350106513 | 0.633996446 | -0.285832982 | -0.740219082 |
| -0.785316438 | -0.45598843 | -0.629770138 | 1.663262176 | 0.305860144 | -0.081425811 | -0.642023411 |
| 0.356430943 | 0.468872379 | 0.499735859 | 1.431561962 | 0.24524581 | 0.288976004 | -0.068357093 |
| 0.271885162 | 0.558808285 | 1.03163993 | 2.01479756 | 0.81798655 | 0.511000486 | -0.314881506 |
| 1.095761472 | 1.50824479 | 0.777853953 | 1.903124625 | 0.675135274 | 0.88083967 | 0.955465985 |
| 0.483529093 | 0.370413697 | 1.006456053 | 2.019975198 | 1.055839646 | 0.260554165 | 0.08869226 |
| 0.418848642 | 0.459723804 | 0.869843353 | 1.726881025 | 0.905366891 | 0.169325764 | 0.276088181 |
| -0.316079276 | 0.267811849 | -0.005358679 | 0.918794143 | 0.064599091 | -0.235756589 | 0.067040209 |
| 0.401525018 | 0.888980606 | 0.8984435 | 1.725540183 | -1.181604511 | 0.525982933 | 0.16063035 |
| -1.148803706 | 0.092460337 | -0.459989548 | 2.148165672 | -0.973267235 | 0.959644573 | -1.147811476 |
| 0.819264564 | 1.031259745 | -6.910121389 | 1.574730814 | -1.269611503 | 0.608512083 | 0.449729185 |
| 0.049887729 | 0.424034191 | -0.769262353 | 1.321616888 | -1.243936281 | 0.614421149 | 0.316850818 |
| 0.900084935 | 1.159182295 | 0.341215151 | -3.858627222 | -0.038290886 | -0.223379248 | 0.830767671 |
| -0.275411505 | 0.260360867 | -0.233442832 | 0.989862556 | -0.03659533 | 0.126872997 | -0.023713568 |
| 0.978514455 | 1.467585815 | 0.703667915 | -3.858627222 | 0.624451227 | 0.694012405 | 0.706268787 |
| -2.145313113 | 0.384824766 | -0.705467431 | 1.694655308 | 0.193669019 | -0.615733619 | 1.152690554 |
| 0.375299786 | 0.755221264 | 0.726010312 | 2.196929318 | 0.838128609 | 0.836004498 | 0.774064755 |
| 0.24978085 | 0.484600709 | 1.206715053 | 2.276834592 | 0.865667649 | 0.261848163 | -0.096408248 |
| 0.060334281 | 0.101267517 | -0.612091683 | -3.858627222 | 0.254441113 | -0.434360546 | -0.352874322 |
| -0.332755533 | 0.03584526 | 0.660990515 | 2.119172963 | 0.56221576 | -0.312950752 | -0.742473303 |
| 0.380022117 | 0.269871752 | 0.399234397 | 1.179329823 | 0.067120835 | -0.629087369 | 0.200866086 |
| 0.142337276 | 0.401920443 | -0.018096327 | 1.429760052 | 0.524796219 | 0.446481233 | -0.044691707 |
| -0.637992899 | 0.616894534 | 1.038789401 | -3.858627222 | 0.353597824 | 1.301588759 | -0.658594953 |
| 1.929605231 | 1.349007313 | 0.432919748 | -3.858627222 | 0.246724544 | -0.52690589 | 1.105375747 |

| 2867 | 2880 | 2953 | 2968 | 3054 | 3213 | 3228 |
|---|---|---|---|---|---|---|
| -0.165862731 | -0.232498547 | 0.35434198 | 0.034618612 | -0.335411983 | 0.461306444 | 0.269411514 |
| -0.90201863 | -0.821224086 | 0.232484847 | 0.001445554 | -0.573264415 | -0.338659095 | -0.325694852 |
| 0.753899381 | 0.499141741 | 0.122715754 | -0.215780688 | 0.287720526 | -0.391628848 | -0.082098981 |
| -0.637617865 | -0.513425912 | -0.317352645 | -0.769986085 | -0.762523748 | -0.870494149 | -0.610547531 |
| -1.216344934 | -1.238147336 | 0.245571724 | -0.11957073 | -0.817278179 | -0.236788784 | -0.6433146 |
| 0.346478886 | 0.23680674 | -4.886648892 | 0.335768679 | 0.206994669 | 0.472185701 | 0.567313647 |
| 0.13368119 | 0.232375195 | 0.454146241 | -0.205308208 | 0.439421266 | 0.076428665 | 0.001500812 |
| -0.495090999 | -0.588291419 | -0.166981486 | -0.377150545 | -0.690065155 | -0.470101199 | -0.472938073 |
| 0.210530895 | -0.081120967 | 0.328480946 | 0.178890368 | 0.180333635 | 0.413691782 | -0.112746285 |
| -0.380787865 | -0.147542432 | 0.309213786 | -0.009744358 | 0.034968051 | -0.122258764 | -0.346446072 |
| 0.138678563 | 0.354870247 | 0.378628621 | -3.412082696 | 0.381888574 | 0.271282121 | -0.320006231 |
| -0.957637767 | -0.464630268 | -0.009635697 | -0.064525611 | -0.409232425 | -0.437186944 | -0.957496178 |
| -0.313282823 | -0.134244124 | 0.047225341 | -0.275824618 | -0.482270933 | -0.820032789 | -0.573689694 |
| -0.551454526 | -0.272261613 | 0.170051769 | -0.147352562 | -0.201487148 | -0.626358251 | -0.855080223 |
| -0.617654743 | -0.500124112 | -0.227148361 | -0.844973456 | -0.954502726 | -0.910614313 | -0.780446568 |
| -0.091524102 | 0.130682353 | 0.117137606 | -0.290099137 | 0.036438756 | -0.7260983 | -0.53884797 |
| -0.316988711 | 0.107929033 | 0.019419223 | -0.341337427 | -0.206231058 | -0.426032391 | -0.662703487 |
| 0.995540336 | 0.272594767 | 0.632857737 | 0.223246955 | 0.35232609 | 0.244279389 | 0.278026451 |
| 0.308534327 | 0.328152637 | 1.197525053 | 0.205807557 | 0.674882304 | -0.222351999 | 0.238904301 |
| 0.071827697 | -0.038617651 | -4.886648892 | -0.705823754 | 0.100264821 | -0.581264949 | -5.666489985 |
| 0.446076919 | 0.227973769 | 0.713711223 | 0.271053198 | 0.211180049 | 0.699576959 | 0.454958002 |
| -0.566756831 | 0.00480405 | 0.237810996 | -0.256004763 | -0.186399612 | -0.554146718 | -0.550922838 |
| -0.420196686 | -0.028388972 | 0.141218034 | -0.00723243 | -0.073254591 | 0.21193277 | -0.206448647 |
| 0.681912476 | 0.792273093 | 1.314150011 | 0.541849759 | 0.813321097 | 0.410001837 | 0.77599331 |
| -0.261552268 | -0.241305402 | -0.040495236 | -0.199609896 | -0.696632091 | -0.259769512 | -0.073195789 |
| 0.563262405 | -0.025526894 | 0.262643525 | -0.251656029 | -0.333446316 | -0.124566063 | 0.324051518 |
| 0.479422354 | -0.678923403 | 0.877907587 | 0.165323339 | -0.486076258 | -0.11899632 | 0.443656665 |
| 0.316216108 | 0.26616277 | -0.276254282 | -0.24610695 | -0.325946852 | -0.343196424 | -0.370914469 |
| 0.136321805 | 0.307376361 | 1.128607746 | 0.28357373 | 0.181996504 | 0.063543498 | 0.528134041 |
| -0.331729479 | -0.12611481 | 0.667094808 | 0.190440145 | -0.308716984 | 0.680277708 | 0.465098758 |
| 0.039175051 | 0.225678347 | 0.234618712 | -0.026833816 | -0.173068514 | 0.1056081 | 0.143688184 |
| 1.569078453 | 0.263472517 | 0.117137606 | -0.094348489 | 0.290880973 | 0.495600175 | 0.375577142 |
| -0.47955389 | -0.177032886 | 0.052353996 | -0.30630426 | -0.552270806 | 0.072840918 | 0.082637573 |
| -0.703224329 | -0.321109728 | -0.309363909 | -0.397246127 | -0.623378305 | -0.507517863 | -0.670726637 |
| -0.112307548 | -0.141934867 | 0.050220252 | -0.029068572 | -0.480752849 | -0.220187274 | -0.066790183 |
| 0.290258319 | 0.415284628 | -0.045183917 | -0.068385809 | -0.359138479 | -0.826342046 | 0.022057984 |
| -0.369215804 | 0.043275856 | -0.384989082 | -0.137580736 | 0.153646338 | 0.058331095 | -0.398141028 |
| 0.031035311 | 0.109164682 | 1.038974699 | 0.548144451 | 0.336959446 | 0.540438216 | 0.141545249 |
| -5.924802059 | -0.728263657 | -4.886648892 | -0.681892658 | -0.84473025 | -0.32698282 | -0.241225353 |
| 0.79911623 | -2.518273889 | 0.866641554 | 0.65285099 | 1.355569948 | 0.754148014 | 0.48621738 |
| 0.652777784 | -0.954788009 | -4.886648892 | -0.600085039 | -0.556557139 | -0.83479341 | 0.189195748 |
| -0.018273122 | -0.199707196 | 0.640225916 | 0.185152056 | -0.10451995 | 0.241342515 | 0.075597033 |
| 0.899364551 | 0.296544878 | 0.837130714 | 0.377771995 | 1.063655441 | 0.886716356 | 0.411425266 |
| 0.425813531 | 0.337175295 | 0.577182913 | -0.267958437 | 0.237226604 | -0.210860574 | -0.050195191 |
| 0.176983893 | -0.383384266 | 0.245923057 | -0.274191598 | -0.14326803 | -0.244882921 | -0.327407768 |
| -0.079740504 | -0.138082102 | 0.358744779 | 0.242305065 | -0.274197256 | 0.234624608 | -0.123640396 |
| 0.516827776 | 0.30051615 | -4.886648892 | -0.038563097 | 0.357259581 | 0.146290075 | 0.416326703 |
| -0.406966753 | -0.582501731 | 0.427846427 | -0.122785543 | -0.620539442 | -0.464467052 | -0.354927391 |
| -0.218087379 | -0.143776468 | 0.697071166 | -0.297273466 | 0.078066232 | -0.426358696 | -0.099691568 |
| -0.45870285 | -0.367256207 | 0.054057719 | -0.587524521 | -0.525542531 | -1.435368417 | -0.279431587 |
| 1.536455119 | 1.481217253 | 1.409297778 | 0.60405845 | 1.3178724 | 0.299584624 | 0.448666035 |
| 1.431021593 | 1.11380257 | 1.030194731 | 0.402857041 | 0.997401621 | 0.331138166 | 0.525458552 |
| 0.811557473 | 0.221598476 | 1.147607138 | 0.544979042 | 0.179354184 | 0.68634883 | 0.801449026 |
| 0.398745857 | 0.272499427 | 1.270355315 | 0.538143059 | 0.375143619 | 0.96635593 | 1.128589729 |
| -0.155588604 | 0.665646651 | 0.828340393 | 0.41181074 | 0.395006592 | -0.172589751 | 0.017413669 |
| 0.118537459 | 0.07223016 | 1.257919869 | 0.812952514 | 0.146785801 | 0.994154278 | 0.730022917 |
| -0.525562583 | -0.167994092 | 0.556026464 | 0.293012232 | -0.443713646 | -0.421057788 | 0.364338351 |
| -0.817846993 | -0.68517679 | -0.203746357 | -0.347119627 | -0.852674845 | -0.528002531 | -0.716886141 |
| -0.039558185 | 0.204523848 | 0.427553541 | 0.255910843 | -0.01137515 | 0.179955105 | -0.018858029 |
| -0.166238332 | -0.468623525 | 1.312700526 | 0.755161866 | -0.236958588 | 0.698487582 | 0.569646608 |
| 1.212164043 | 0.733017842 | 0.97108016 | 0.499746478 | 0.903861996 | 0.936999707 | 0.837833831 |
| -0.925821722 | -0.56891865 | 1.143320682 | 0.56004957 | 0.065956509 | 0.588704939 | 0.386691025 |
| -0.158380186 | -0.077596591 | 1.017447072 | 0.317075449 | 0.109974865 | 0.445093993 | 0.427536523 |
| -0.150213542 | 0.406796863 | -0.334779025 | -0.420923279 | 0.139368048 | -0.383287918 | -0.388679064 |
| 0.507678581 | 0.721445792 | -4.886648892 | 0.522883234 | 0.496110303 | 0.366506221 | 0.73012216 |
| -0.369676125 | -0.272672683 | 0.515813967 | -0.085197645 | -0.508827297 | -1.341684805 | -0.051712214 |
| 0.487856422 | 0.526507435 | -4.886648892 | 0.685245853 | 0.827156575 | 0.737544204 | 0.594388098 |
| 0.417151673 | 0.692346734 | 0.301933325 | 0.128250105 | 0.182875728 | -0.099911123 | 0.438356393 |
| 0.524953446 | 0.990496876 | 0.326214326 | 0.110754041 | 0.703542619 | 0.081800289 | 0.005810013 |
| 0.036878066 | -0.381778046 | -0.027021074 | -0.412853474 | -0.646384098 | -0.628011021 | -0.27644026 |
| 0.98419055 | 0.695911756 | 0.992952562 | 0.3101427 | 0.842925119 | 0.123146313 | 0.400170378 |
| 0.321535393 | 0.178863061 | 0.73612149 | 0.139248642 | 0.349519604 | 0.578157661 | -0.008003785 |
| 0.921128474 | 0.356646746 | 1.110868727 | 0.421870094 | 0.178373772 | 0.327678546 | 0.675778137 |
| 0.068862448 | -0.187663572 | 0.852186588 | 0.149033834 | -0.154014533 | 0.586912454 | 0.836585592 |
| -0.381719441 | 0.053416478 | 0.196602332 | -0.333494773 | -0.353612956 | -0.109034719 | -0.034510448 |
| -0.401248095 | -0.024692324 | 0.739558067 | 0.038779613 | -0.338367748 | -0.226351958 | 0.465098758 |
| -0.224239046 | 0.382623469 | 0.008789306 | -0.24325532 | -0.013155503 | 0.37451277 | 0.230434919 |
| 0.180841153 | 0.241882543 | 0.523295032 | 0.373519042 | -0.064837773 | 0.265346805 | 0.46535743 |
| 0.160049825 | -0.241983009 | 0.510974988 | 0.174941188 | -0.489896118 | -0.863106986 | -0.097645788 |
| 0.211046239 | 0.101869142 | 1.211264864 | 1.024388149 | 1.22829202 | 1.742470124 | 0.583875878 |

| 3241 | 3329 | 3415 | 3503 | 3572 | 3590 | 3603 |
|---|---|---|---|---|---|---|
| 0.512197753 | -0.450375198 | -0.233824979 | -3.3543353 | 1.100184678 | 0.745669481 | 0.576942978 |
| -0.176655689 | -0.947971742 | 0.26518282 | 0.23577562 | 0.666666483 | 0.462517077 | 0.025272383 |
| -0.252810991 | 0.470486473 | -0.71729726 | 0.639706481 | 0.626903973 | -0.513458341 | -0.419663253 |
| -0.1756903 | -0.510071358 | -0.55010883 | 0.538213595 | 1.097521206 | -0.145023261 | 0.040017747 |
| -0.533985422 | -1.006859814 | 0.065764149 | -3.3543353 | 1.300214367 | -0.395873975 | -0.382173618 |
| 0.463602348 | 0.483432336 | 0.044285613 | 0.878562745 | -3.394186952 | 1.062406764 | 0.731618475 |
| -0.424748804 | 0.428349301 | -0.431611608 | 1.44978332 | -3.394186952 | 0.114713194 | 0.474627339 |
| -0.301155574 | -0.408321347 | -0.516146042 | 0.757513888 | 1.331830353 | -0.02726313 | 0.316872847 |
| -0.105208183 | -0.216760347 | 0.237498585 | 0.620886043 | 1.277640045 | 0.071460356 | 0.058841939 |
| -0.336969448 | -0.412907609 | 0.096670467 | 0.854081718 | 0.470045389 | -0.206940332 | -0.448675892 |
| -0.13233462 | 0.00802262 | -0.120950701 | 0.77203074 | 0.429265019 | -0.364578638 | -0.120212416 |
| -0.482271931 | -0.760393784 | -0.141042509 | 0.317737035 | 0.733139426 | -0.630273431 | -0.362945666 |
| -0.493770676 | -0.095911587 | -0.408988193 | -3.3543353 | 0.812458273 | -0.49281473 | -0.647024751 |
| -0.933124896 | -0.424798356 | -0.207964441 | 0.869016547 | -3.394186952 | -0.840543901 | -1.054876538 |
| -0.380725096 | -0.479426902 | -1.667751649 | -3.3543353 | -3.394186952 | -0.409208394 | -0.173389218 |
| -0.707215947 | -0.03154646 | -0.103679559 | 0.96537257 | -3.394186952 | -0.882748709 | -1.288500775 |
| -0.452562121 | -0.070412047 | -0.657739494 | -3.3543353 | -3.394186952 | -0.794113117 | -0.833296318 |
| -0.033324612 | 0.121976164 | -0.218515168 | 0.761405701 | -3.394186952 | -0.351505722 | -0.419553032 |
| -0.31366025 | 1.161486826 | 0.637443297 | -3.3543353 | -3.394186952 | 0.216833042 | -1.365782326 |
| -0.293266864 | -4.919834748 | -0.032540543 | -3.3543353 | -3.394186952 | -5.587440971 | -0.655162009 |
| 0.261223048 | 0.309744817 | 0.142159335 | 1.123948036 | 0.826545493 | 0.355045894 | 0.120780886 |
| -0.274686324 | 0.045255113 | -0.091234638 | -3.3543353 | -3.394186952 | -0.477966378 | -0.718270992 |
| 0.36410998 | -0.186983583 | 0.42596654 | 0.686834499 | 0.731217827 | 0.17412362 | 0.303851533 |
| 0.81747627 | 1.624059969 | 1.316460128 | 1.992715539 | 1.638737759 | 0.958934435 | 0.427685895 |
| 0.297798341 | -0.538701369 | -0.189137844 | 0.287003209 | 1.431170037 | 0.452927112 | 0.543911285 |
| 0.071999749 | -0.002376988 | -0.548711987 | 0.453384831 | 0.4253552 | 0.184370948 | -0.129664393 |
| -0.3247092 | -0.639702421 | -0.03611717 | 0.403259209 | 0.618327592 | 0.399070855 | -0.196160114 |
| 0.089915596 | 0.081551557 | -0.143965011 | 0.583309038 | -0.129245761 | -0.55451626 | -0.276577736 |
| 0.453284705 | 0.347900674 | 1.041917301 | 1.423086065 | 0.69298912 | 0.903065761 | 0.116027567 |
| 0.646481106 | -0.228159265 | 0.23385818 | 0.952621361 | 1.530648352 | 0.999863047 | 0.693856467 |
| 0.598907088 | 0.442407112 | 0.313733952 | 0.577490332 | 0.503205741 | 0.200733713 | 0.182716385 |
| 0.175735604 | -0.06020693 | -0.516339081 | 0.426602298 | 0.754976428 | 0.296975129 | 0.370239173 |
| 0.33567862 | 0.088337198 | -0.590034292 | 0.169571235 | 0.838711093 | 0.700240551 | 0.519174334 |
| 0.040599707 | -0.457902777 | -0.602582033 | 0.13201989 | 0.192633409 | -0.243513093 | 0.061667851 |
| 0.369807899 | -0.441551411 | -0.018008667 | 0.504889519 | 1.220344046 | 0.250705882 | 0.336395487 |
| 0.634759483 | 0.700134693 | -0.10854712 | 0.498594648 | 0.903098454 | 0.320059499 | 0.392104681 |
| 0.193395727 | 0.21973807 | -0.081314047 | 0.84519119 | 0.058819464 | -0.594097174 | -0.023647565 |
| 0.353753004 | 0.481286297 | 0.43024605 | 1.212489979 | 1.290366383 | 0.487877692 | 0.274162376 |
| -0.012552854 | 0.007591455 | -0.750655283 | -3.3543353 | 1.734866097 | 0.233736457 | 0.320169484 |
| 0.162888211 | 1.286501135 | 0.43502939 | 1.573953046 | 1.229734889 | -0.091346703 | 0.016840852 |
| -0.891288583 | -4.919834748 | -0.524869902 | -3.3543353 | -3.394186952 | -0.342361144 | -0.829896757 |
| 0.028935415 | -0.050561882 | -0.368379858 | 0.934818657 | 1.3023897 | 0.289327289 | 0.21817782 |
| -0.25243584 | -0.314783616 | 0.160674165 | 1.104156221 | 1.211459311 | -0.07607849 | -0.178498271 |
| -0.382669579 | 0.562736831 | -0.205131899 | -3.3543353 | -3.394186952 | -0.271482509 | -0.655460973 |
| -0.724572203 | -0.412907609 | -0.190391273 | 0.922496149 | -3.394186952 | -0.474736286 | -0.74317971 |
| -3.067158812 | -0.074148972 | 0.160968419 | 0.709590963 | 0.860073949 | 0.078697325 | 0.330203003 |
| -0.162805106 | 1.143230379 | 0.364767107 | -3.3543353 | -3.394186952 | 0.204737503 | -0.706601313 |
| -0.311889539 | -0.486470177 | 0.161850659 | 0.754585057 | 0.907139516 | -0.030934712 | -0.339085062 |
| -0.686868505 | -4.919834748 | -0.449147128 | -3.3543353 | -3.394186952 | -5.587440971 | -1.224799792 |
| -0.674207518 | -0.220003943 | -0.793535375 | 0.225592603 | -0.324474266 | -1.843836617 | -1.080250131 |
| 0.499978866 | 1.914056119 | 0.12186852 | 1.690548046 | 1.304560311 | 0.163959401 | 0.05539131 |
| 0.451946273 | 1.233252494 | 0.267213135 | 1.520862023 | 1.453836037 | 0.33228459 | 0.205297519 |
| 0.341977483 | -0.237483086 | -0.282129146 | -3.3543353 | 1.178804335 | 0.582344084 | 0.313724137 |
| 0.755020752 | 0.403697743 | 0.738765415 | 0.963787532 | 2.140942035 | 1.122501957 | 0.571207018 |
| 0.455032264 | 1.566326041 | 0.611408192 | 1.531708116 | 1.205491994 | 0.307519231 | 0.136424933 |
| -2.846494336 | 0.286850192 | 0.497320162 | 0.661404013 | 2.337920708 | 0.874845323 | 0.475824229 |
| 0.505091482 | 0.407463353 | -0.018947435 | 0.417056337 | 2.032649265 | 0.633788018 | 0.595965531 |
| -0.347684341 | -0.942380762 | -0.888035857 | -0.029813286 | 1.083420388 | 0.093219086 | 0.205954755 |
| 0.184793074 | 0.318947433 | -0.047407497 | 0.620886043 | 0.805339538 | -0.141935886 | -0.056153136 |
| 0.300678887 | -0.379330075 | 0.636895061 | 0.675259078 | 1.147586917 | 0.767322367 | 0.350446952 |
| 0.594342726 | 1.272600775 | 0.216382936 | 1.105533821 | 1.755450852 | 0.722888478 | 0.552953987 |
| 0.155598744 | -0.512970331 | 0.636712249 | 0.357783911 | 0.467544763 | 0.979566276 | 0.226530412 |
| 0.350291911 | 0.099414136 | 0.511372763 | 0.808816931 | 0.756852953 | 0.816586451 | 0.332988804 |
| 0.207623658 | 0.510137252 | -0.540569428 | 0.662476629 | -3.394186952 | -0.432980228 | -0.108135324 |
| 0.952750658 | 1.604775887 | 0.339329924 | 1.608259548 | -3.394186952 | -1.17695415 | 0.761623772 |
| -0.008071079 | -0.240794713 | 0.051314129 | 0.22752883 | 0.610762441 | 0.358478142 | 0.042738626 |
| 0.410593386 | 0.591527733 | 1.280107124 | 1.720764107 | 1.02422327 | 0.749679702 | 0.250162651 |
| 0.707957678 | 1.103670494 | -0.025425439 | 0.721788716 | 0.504413033 | 0.484999914 | 0.474504995 |
| 0.484183063 | 1.24420661 | 0.407743908 | 1.386343489 | -3.394186952 | -0.23699435 | -0.163053816 |
| -0.178511853 | -0.172937677 | -0.544334567 | -3.3543353 | 0.696977957 | -0.152524007 | -0.05902551 |
| 0.196060633 | 0.976599343 | -0.256344807 | 1.052529116 | 0.943570533 | 0.165471746 | 0.10942565 |
| -0.178086178 | 0.184594426 | 0.434656514 | 1.079735834 | -3.394186952 | -0.224082977 | -0.887133632 |
| 0.527827944 | 0.509093083 | 0.475219502 | 1.151277604 | 1.975010393 | 0.971752621 | 0.607267534 |
| 0.483364721 | 0.235313092 | -0.065938285 | 0.668888277 | 2.22275729 | 1.064638341 | 0.626214138 |
| 0.465555601 | 0.732612661 | 0.16263422 | 0.790121303 | 1.877529732 | 0.601580663 | 0.393201921 |
| 0.74860879 | 0.462035642 | 0.796706787 | 1.000387428 | 1.519780097 | 0.861184675 | 0.464772735 |
| 0.810230412 | 0.699703082 | -0.244947913 | 1.05687443 | 1.285958177 | 0.646129065 | 0.909539384 |
| 0.760726312 | 0.441569194 | -0.099344366 | 0.857615915 | 0.860073949 | 0.854473431 | 0.68765251 |
| -0.118132703 | -0.009809366 | -0.416996871 | -3.3543353 | 1.485543881 | 0.067717992 | 0.076321536 |
| -0.250478998 | 0.372751944 | 0.926558499 | 1.653836645 | 1.5175922 | 0.208544408 | -0.331443792 |

| 3691 | 3777 | 3865 | 3953 | 4039 | 4052 | 4226 |
|---|---|---|---|---|---|---|
| 0.348486356 | -0.183624394 | -0.503411858 | -0.416207686 | 1.150387082 | 0.910249268 | -0.076011861 |
| -0.525582215 | 0.758968377 | 0.193102765 | 1.284543984 | 0.901216278 | -0.015133909 | 0.725026163 |
| -0.133176278 | -0.678099528 | -0.282034266 | -0.265520963 | 0.572848159 | -0.241247673 | -0.378205072 |
| -0.368740623 | -0.159649543 | -0.232343419 | -0.018843888 | 0.860636428 | 0.039741007 | -0.001064462 |
| -0.844192686 | 0.442894163 | 0.071579228 | 0.523113823 | 0.652433275 | -0.590506669 | 0.444601769 |
| 0.741945676 | 0.471297675 | 0.298540857 | 0.6242668 | 1.329962861 | 1.079684783 | 0.618872508 |
| -0.088178764 | 0.105738524 | 0.353440626 | 0.596492882 | -2.76020633 | 0.384446923 | 0.400860819 |
| 0.009340181 | 0.385034404 | 0.118636669 | 0.528274934 | 1.087659034 | 0.436865312 | 0.518541064 |
| -0.095066452 | 0.746414701 | 0.512472925 | 1.171358895 | 0.791393218 | 0.065105605 | 0.830728526 |
| -0.623736602 | 0.266772722 | 0.194394922 | 0.614869497 | 0.787973242 | -0.355428282 | 0.438681088 |
| -0.299216647 | 0.031073547 | -0.062598975 | 0.567925483 | 0.484058729 | -0.164778204 | -0.102863617 |
| -0.796970942 | 0.448277483 | -0.005802401 | 0.829506508 | -2.76020633 | -0.794299072 | 0.065785489 |
| -0.497629417 | -0.292811178 | 0.09752102 | 0.409737827 | -2.76020633 | -0.814788093 | -0.105418332 |
| -0.963908017 | -0.01065304 | 0.392856191 | 0.760816748 | -2.76020633 | -1.031612168 | 0.180386126 |
| -0.560811776 | -2.673898635 | -4.329461908 | -3.865439088 | -2.76020633 | -0.376906061 | -4.351188777 |
| -0.857019178 | -0.247069523 | 0.216109915 | 0.46764112 | -2.76020633 | -1.273436735 | -0.080160212 |
| -0.381402582 | -0.992789484 | 0.005179824 | -3.865439088 | -2.76020633 | -0.676459703 | -0.221944484 |
| -0.366413309 | -0.836001613 | -0.227409036 | -0.207080208 | -2.76020633 | -0.93177362 | -0.739949166 |
| 0.210408465 | -0.026132651 | 1.624523266 | 1.821899398 | -2.76020633 | -0.152471871 | 1.233649274 |
| 0.042779713 | -0.536231961 | -4.329461908 | -3.865439088 | -2.76020633 | -0.05487581 | -4.351188777 |
| -0.08747501 | 0.040714161 | 0.100362335 | 0.498417514 | 1.09901849 | -0.085390331 | 0.026961306 |
| -0.567042679 | -0.313602827 | 0.291534757 | 0.625602092 | -2.76020633 | -0.846776098 | 0.075794152 |
| -0.238704544 | 0.434553831 | -0.013721353 | 0.547214894 | 0.718833538 | -0.012810488 | 0.219956697 |
| 1.425191175 | 1.110998939 | 2.126298043 | 1.880649993 | 1.868378067 | 0.858368615 | 1.737180998 |
| 0.122004969 | 0.364949062 | -0.144326744 | 0.19027189 | 0.796501351 | 0.680065905 | 0.383755753 |
| -0.099862795 | -0.652388048 | -0.463981594 | -3.865439088 | 0.660248868 | -0.375237447 | -0.689766169 |
| -0.387794195 | 0.35216714 | 0.065721838 | 0.834391716 | 1.005689278 | -0.113437632 | 0.414134447 |
| 0.1773566 | -0.233633532 | 0.129029613 | -0.133169512 | 0.564315684 | 0.189272741 | 0.261571578 |
| 0.453439638 | 0.929632735 | 1.287698724 | 1.660250969 | 1.432150386 | 0.292896824 | 1.331702383 |
| 0.397207197 | 0.296326223 | -0.109604124 | 0.176776408 | 1.201513458 | 0.897723783 | 0.381662388 |
| 0.585167197 | 0.06065209 | 0.257568553 | 0.214718004 | 0.664133877 | 0.334471833 | 0.21687275 |
| -5.911823241 | -0.4632839 | -0.34533975 | -0.295235926 | 1.028841613 | 0.660531744 | -0.23641931 |
| 0.644752173 | -0.469715111 | -0.44789811 | -0.734094256 | 1.10277648 | 0.850079793 | -0.350588161 |
| -0.277501434 | -0.569661434 | -0.645355733 | -0.410550496 | 0.406874821 | -0.185248351 | -0.623203085 |
| -0.035889803 | 0.164387963 | -0.337616 | 0.042767119 | -2.76020633 | 0.098358145 | -0.133082614 |
| 0.724217049 | -0.135460993 | -0.058433344 | -0.179838814 | 0.600086381 | 0.443994328 | -0.269021229 |
| 0.185287495 | -0.276929756 | -0.016906576 | -0.247787144 | 0.358059582 | 0.017663417 | -0.165148061 |
| 0.304664043 | 0.366176814 | 0.6061248 | 1.254762152 | 1.211656675 | 0.321404786 | 0.571048899 |
| 0.114560197 | -0.753080249 | 0.080301481 | -3.865439088 | 1.583753687 | 0.436865312 | 0.087814377 |
| 0.008701387 | 0.322602921 | 0.254533281 | 0.608102655 | 0.579200075 | -0.182265534 | 0.178462185 |
| -0.942588813 | -0.5885612 | -4.329461908 | -3.865439088 | -2.76020633 | -0.910667695 | 0.073657855 |
| -0.362006278 | -0.42693664 | -0.188786742 | 0.282784708 | 1.150387082 | -0.090602694 | -0.247168949 |
| -0.924147934 | 0.218345018 | -0.147957171 | 0.660997057 | -2.76020633 | -0.576476394 | -0.020455437 |
| -0.365948495 | -0.583697695 | 0.376845603 | -3.865439088 | -2.76020633 | -0.588441122 | 0.201935952 |
| -0.997419186 | -0.064608045 | 0.121418699 | 0.420443686 | -2.76020633 | -0.878441491 | 0.104541837 |
| 0.368973802 | 0.47933832 | 0.51949444 | 0.772405706 | 1.170451487 | 0.512735682 | 0.648594192 |
| 0.312004134 | 0.036708527 | 1.380807418 | -3.865439088 | -2.76020633 | 0.104740448 | 1.242769974 |
| -0.524492534 | 0.373565572 | 0.193749052 | 0.747321267 | -2.76020633 | -5.742214296 | -4.351188777 |
| -0.48106883 | -1.446349013 | -4.329461908 | -3.865439088 | -2.76020633 | -0.603339364 | -4.351188777 |
| -0.760805079 | -1.251337683 | -0.602908266 | -0.804052845 | -2.76020633 | -1.233319041 | -0.852159146 |
| 0.569881496 | -0.395374658 | 0.09752102 | 0.141677623 | 0.458193191 | -0.442469378 | -0.8081612 |
| 0.672441836 | 0.28584205 | 0.634381224 | 0.804715948 | 0.836453402 | 0.294462594 | 0.325637398 |
| 0.283771918 | -0.216716736 | -0.40114382 | -0.211693472 | 0.888904642 | 0.28373794 | -0.461070607 |
| 0.89669033 | 0.736894927 | 0.822245718 | 0.966582054 | 1.70652247 | 1.101307177 | 1.197933629 |
| 1.02275942 | 0.514111875 | 1.277258854 | 1.196175959 | 1.312882168 | 0.461477291 | 0.950337721 |
| 0.833959178 | 0.613649301 | 0.910582979 | 1.495126232 | 1.430349881 | 0.916911722 | 1.012726817 |
| 0.711799215 | 0.270214669 | 0.093957986 | 0.384585596 | 0.895079407 | 0.673272268 | 0.19816663 |
| -0.335738866 | -0.241752978 | -0.571867399 | -0.373632313 | 0.6042118 | 0.232715493 | -0.157034792 |
| 0.311532202 | -0.150502572 | 0.134528664 | 0.241523938 | 0.806639998 | 0.183885992 | 0.105923243 |
| 0.326524635 | 0.736645802 | 0.661456469 | 1.060260595 | 1.087659034 | 0.53198708 | 0.991885689 |
| 1.05075837 | -0.002793383 | 0.362759336 | -0.018843888 | 1.190121218 | 0.975604794 | 0.308311754 |
| 0.043705667 | 0.786176754 | 0.297958887 | 1.059828499 | 1.063245641 | 0.290757725 | 0.702855638 |
| 0.50910192 | 0.57764726 | 0.720394099 | 0.620250193 | 1.236026512 | 0.598690756 | 0.930866117 |
| 0.066730944 | -0.840273503 | -0.49694604 | -0.623497991 | 0.429191641 | -0.068686501 | -0.67308142 |
| 1.237467325 | 0.17015588 | 0.647600187 | 0.716121232 | -2.76020633 | 1.054196993 | 0.377462466 |
| -0.047820111 | 0.476045753 | 0.264213898 | 0.721591373 | 0.937267042 | 0.338835198 | 0.768656756 |
| 0.169770338 | 1.24921251 | 1.53504821 | 2.33499764 | 1.606676451 | 0.000445426 | 1.406999208 |
| 0.700425786 | -0.103612474 | -0.084544488 | -0.210153352 | 0.486377301 | 0.310966714 | -0.372620337 |
| 0.428242075 | -0.09886116 | 0.536147614 | 0.668056061 | 0.979162524 | -0.122244855 | 0.096909801 |
| -0.19585343 | -0.389825468 | -0.138905697 | -0.016305171 | 0.890451911 | -0.232777763 | -0.178818191 |
| 0.48578079 | -0.385339316 | -0.145233116 | -0.211693472 | 0.529438565 | 0.078080566 | -0.507138755 |
| -0.37200799 | 0.116658994 | 0.720012463 | 1.100066002 | 1.195833561 | -0.84499658 | 0.397256841 |
| 0.903764534 | 0.748288127 | 0.972064589 | 1.257248567 | 1.477000952 | 0.868193301 | 1.11314734 |
| 0.671701235 | -0.151985924 | 0.00984982 | -0.138885446 | 1.598332323 | 0.950238633 | 0.194383046 |
| 0.811665734 | 0.345502566 | 0.665495573 | 0.693313118 | 1.147999877 | 0.631410176 | 0.641751192 |
| 0.566686401 | 0.755380198 | 0.683868164 | 1.440527519 | 1.110250356 | 0.296025916 | 0.630377278 |
| 0.511616963 | -0.299565053 | -0.421255701 | -0.268779356 | 0.82990459 | 0.516273973 | -0.594984508 |
| 0.722731896 | -0.262412448 | -0.513844921 | -0.676041117 | 0.885802902 | 0.83735102 | -0.512248628 |
| -0.178944392 | -0.058298667 | -0.240289428 | 0.590291525 | 0.831545818 | 0.041024634 | -0.096082781 |
| -0.189016531 | 0.873504434 | 1.251121103 | 2.139593707 | 1.691368577 | -0.473558713 | 1.091486579 |

| | 4400 | | 4413 | | 4587 | 4761 |
|---|---|---|---|---|---|---|
| | 0.681554754 | | 1.355703687 | | 0.480299552 | -0.06141998 |
| | 0.96003915 | | 0.529913934 | | 1.325844881 | 1.690493581 |
| | 0.082001954 | | -0.002382159 | | 0.016027563 | 0.33947742 |
| | 0.568848996 | | 0.653183011 | | 0.653083661 | 0.86226522 |
| | 0.537780949 | | -0.664243864 | | 0.598270416 | 1.228318308 |
| | 1.095275749 | | 1.590496994 | | 1.350267487 | 0.883367097 |
| | 1.16926755 | | 1.102831415 | | 0.987038221 | 1.538007053 |
| | 0.852008775 | | 0.944534186 | | 1.161809116 | 1.359190886 |
| | 0.724353476 | | 0.294397218 | | 1.244114728 | 1.562367607 |
| | 0.651030742 | | 0.3107834 | | 1.155643373 | 1.564291054 |
| | 0.15401002 | | 0.338487099 | | 0.372164457 | 0.564058786 |
| | 0.183672244 | | -0.70274561 | | 0.323309539 | 0.911443057 |
| | -3.092573612 | | -1.027827852 | | -0.209261506 | -2.562074987 |
| | 0.756560305 | | -0.919167003 | | 0.273245229 | 1.35564604 |
| | -3.092573612 | | -0.088002351 | | -4.244134055 | -2.562074987 |
| | 0.562712125 | | -1.491667475 | | -0.350372009 | 0.967082743 |
| | -3.092573612 | | -0.717785163 | | -0.160962794 | -2.562074987 |
| | -3.092573612 | | -1.176915358 | | -0.837396493 | -2.562074987 |
| | -3.092573612 | | -5.381785645 | | -4.244134055 | -2.562074987 |
| | -3.092573612 | | 0.152168337 | | -4.244134055 | -2.562074987 |
| | 0.604899761 | | 0.184329914 | | 0.233473285 | 0.810543271 |
| | -3.092573612 | | -0.76078898 | | 0.116687183 | -2.562074987 |
| | 0.459025936 | | 0.372825731 | | -4.244134055 | 0.974041713 |
| | 1.356916948 | | 0.04870803 | | 1.169264287 | 1.539977885 |
| | 0.520272941 | | 1.225893613 | | 1.16154183 | 0.736403889 |
| | -0.01460324 | | -0.466508151 | | -0.667250841 | -2.562074987 |
| | 0.707186806 | | 0.284146606 | | 0.8234965 | 1.071430175 |
| | 0.234088356 | | 0.322155547 | | 0.548207022 | 0.661077395 |
| | 1.33227302 | | 0.350322016 | | 1.503855155 | 1.992303032 |
| | 0.857753936 | | 1.467369004 | | 1.09466239 | 0.4624067 |
| | 0.185917922 | | 0.308171541 | | 0.40002788 | 0.352473055 |
| | 0.494246749 | | 1.134143237 | | 0.260821085 | 0.271838392 |
| | 0.366068738 | | 1.041268041 | | -0.082835736 | -2.562074987 |
| | -0.017348232 | | 0.223403489 | | -0.193592116 | 0.141201936 |
| | 0.004403577 | | 0.424639369 | | 0.312745842 | 0.145181248 |
| | -0.118502835 | | 0.223184493 | | -0.39246785 | -0.248832599 |
| | -0.127668802 | | 8.47E-05 | | -0.004350116 | 0.199290273 |
| | 0.835744476 | | 0.418532809 | | 0.665891326 | 1.105447061 |
| | 1.117612066 | | 0.856097104 | | 0.356143228 | -2.562074987 |
| | 0.225500926 | | -0.146167223 | | 0.431583349 | 0.753841122 |
| | -3.092573612 | | -0.713305371 | | 0.145896179 | -2.562074987 |
| | 0.674698838 | | 0.541610348 | | 0.092845401 | 0.762447027 |
| | 0.393781578 | | 0.213722019 | | 0.67810362 | 1.049164625 |
| | -3.092573612 | | -0.64893448 | | 0.199776912 | -2.562074987 |
| | 0.772906702 | | -0.506134045 | | 0.524732938 | 1.397376712 |
| | 0.730878359 | | 0.606369862 | | 0.928377104 | 1.135398386 |
| | -3.092573612 | | 0.10103344 | | -4.244134055 | -2.562074987 |
| | -3.092573612 | | -5.381785645 | | -4.244134055 | -2.562074987 |
| | -3.092573612 | | -5.381785645 | | -4.244134055 | -2.562074987 |
| | -0.294276533 | | -1.500221847 | | -1.174421369 | -0.770315517 |
| | -0.338761258 | | -0.966969187 | | -1.054736084 | -2.562074987 |
| | 0.343331874 | | 0.188193291 | | 0.370986462 | 0.53758821 |
| | -0.045220717 | | 0.125238225 | | -0.457782258 | -0.341319912 |
| | 1.239724919 | | 1.217987668 | | 1.448836209 | 1.3083817 |
| | 0.929584208 | | 0.239477513 | | 0.988946133 | 1.392825043 |
| | 0.977470126 | | 0.95659808 | | 1.061832553 | 0.838725491 |
| | 0.142363021 | | 0.459305801 | | 0.184270539 | 0.062421058 |
| | 0.199287105 | | 0.782631308 | | 0.413108883 | 0.326310673 |
| | 0.401043774 | | 0.369993028 | | 0.418743336 | 0.637083921 |
| | 0.872538693 | | 0.610095482 | | 1.165277359 | 1.157287484 |
| | 0.620998455 | | 1.065425881 | | 0.554113132 | 0.349239945 |
| | 0.796326976 | | 0.517752871 | | 1.018987819 | 1.037843139 |
| | 0.862326418 | | 0.685426686 | | 1.066840959 | 1.049164625 |
| | -0.082656704 | | 0.185467751 | | -0.419382201 | 0.045014178 |
| | 0.696474331 | | 0.993766334 | | 0.273245229 | 0.289181873 |
| | 0.707186806 | | 0.337901425 | | 0.919903339 | 1.057173637 |
| | 1.654323458 | | -0.09249701 | | 1.356666026 | 2.334698146 |
| | -0.401330529 | | -0.036153124 | | -0.529111241 | -2.562074987 |
| | 0.464134069 | | -0.243609085 | | 0.005041228 | 0.747337739 |
| | 0.417193416 | | -0.286794613 | | -0.129364588 | -2.562074987 |
| | -0.194296675 | | -0.2544967 | | -0.539310951 | -0.341319912 |
| | 0.850855771 | | -1.176915358 | | 0.144418856 | 1.298404066 |
| | 1.084374479 | | 0.855399008 | | 1.345385965 | 1.380200062 |
| | 0.929584208 | | 1.323503677 | | 0.593564082 | 0.336201951 |
| | 0.733476438 | | 0.510548936 | | 0.705469788 | 0.887156415 |
| | 0.55653736 | | -0.089498327 | | 0.417618982 | 0.672861646 |
| | 0.248941164 | | 0.73389622 | | -0.421983659 | -2.562074987 |
| | 0.185917922 | | 1.118430949 | | -0.219845855 | -0.556741417 |
| | 0.427816745 | | 0.43230935 | | 0.354946196 | 0.545603214 |
| | 1.550442716 | | -0.50477245 | | 1.155912241 | 2.320817119 |

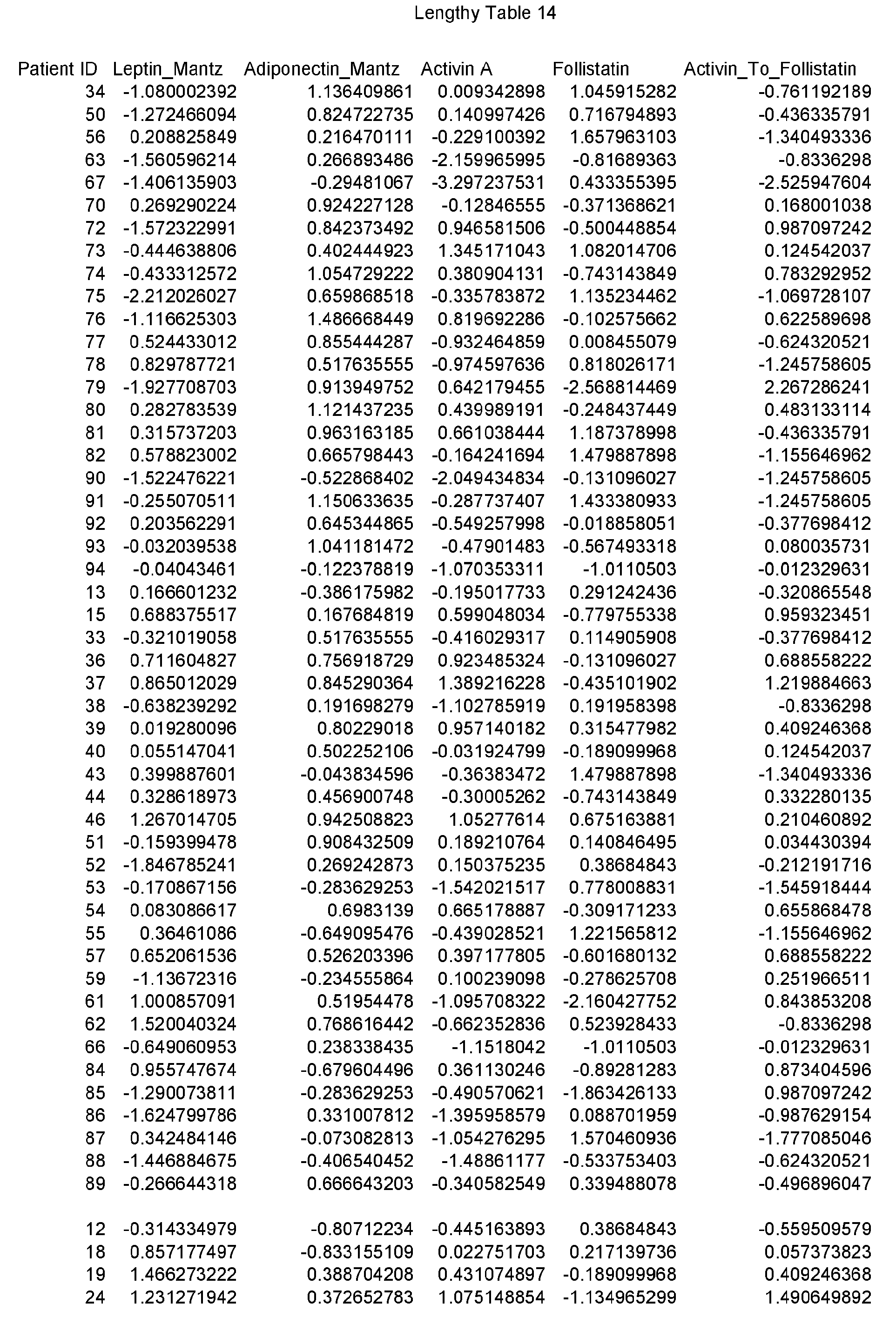

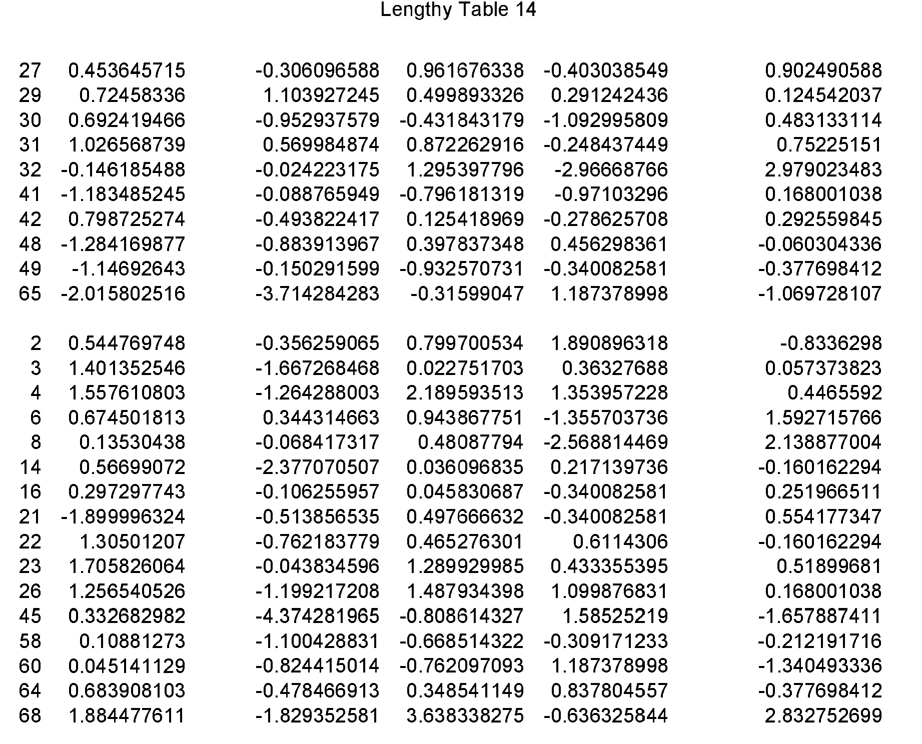

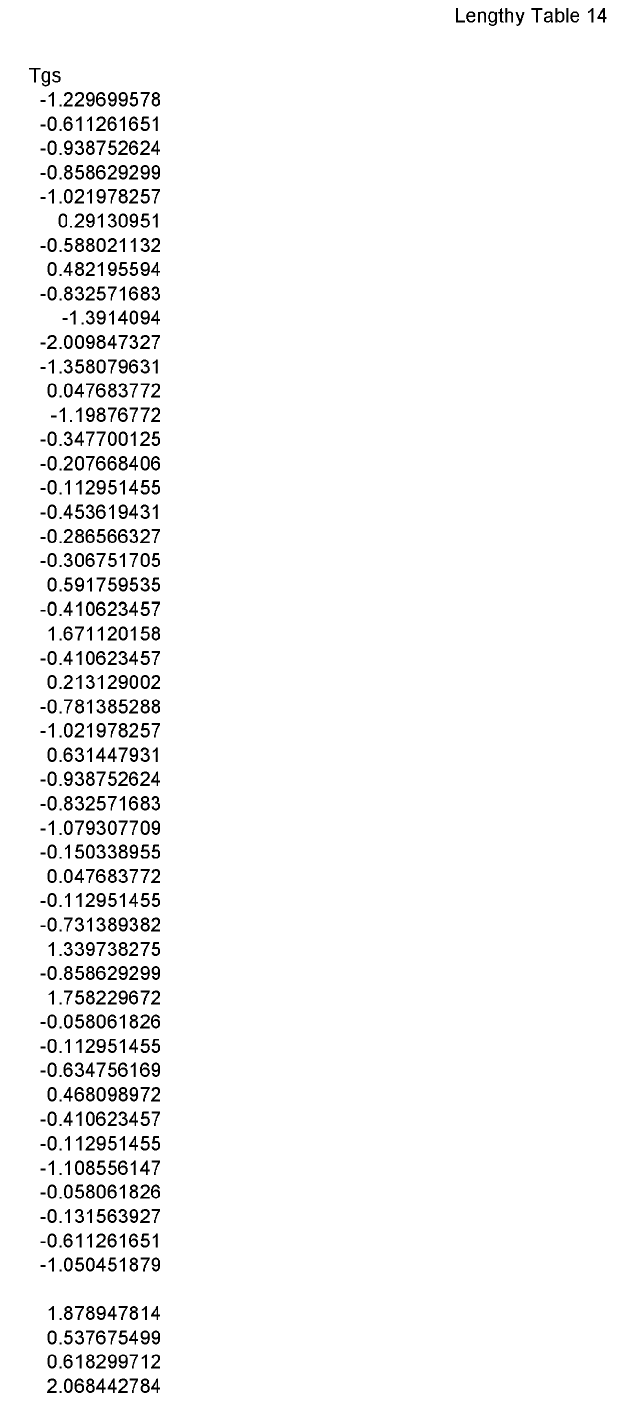

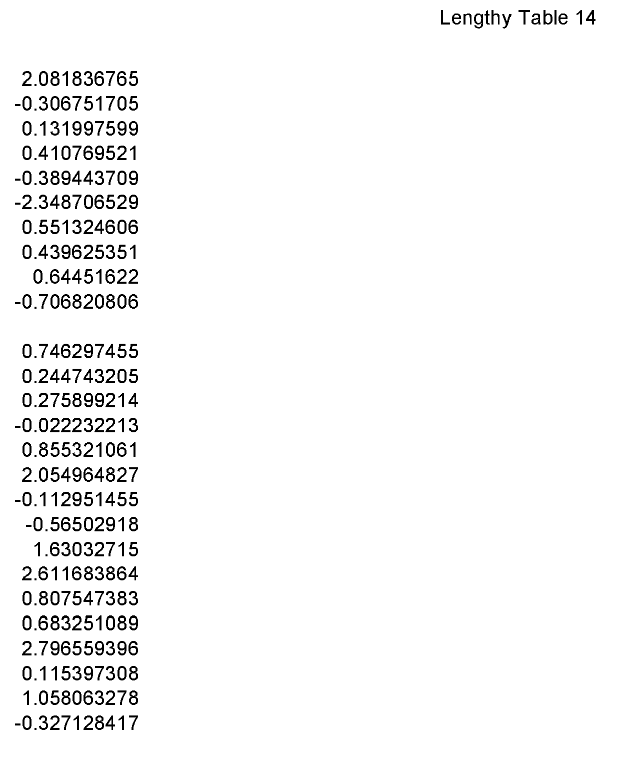

**Lengthy Table 15**

| Patient ID | AcCa | Cer | ChE | Co | DG | Hex1Cer |
|---|---|---|---|---|---|---|
| 34 | 0.929395325 | 0.052537401 | 0.304459818 | -0.14075721 | -0.346770509 | 0.356518863 |
| 50 | 0.56630836 | -0.155582348 | 0.187583407 | -0.167131312 | -0.141495251 | 0.589761523 |
| 56 | 0.181645829 | 0.167308867 | 0.50222678 | -0.147881439 | -0.314715431 | 0.649115989 |
| 63 | -0.021198712 | 0.097584608 | 0.551992713 | -0.018409753 | -0.445225021 | 0.165067271 |
| 67 | 0.677117634 | 0.190803071 | 0.516998272 | 0.090587358 | -0.243102052 | 0.4414079 |
| 70 | -0.574159288 | -0.244072609 | -0.331613608 | 0.53616824 | 0.279128515 | -0.307685661 |
| 72 | 0.084523609 | -0.32539337 | 0.225944316 | 0.2798941 | -0.060223787 | -0.009615676 |
| 73 | -0.494359124 | 0.115852566 | -0.062455156 | 0.413503177 | -0.000512645 | 0.295017447 |
| 74 | 0.77277787 | -0.229574843 | 0.374799204 | 1.417163206 | -0.339906465 | -0.244291459 |
| 75 | 0.433102954 | -0.25850723 | 0.56167617 | 0.240275545 | -0.108245507 | 0.159096109 |
| 76 | 0.170250985 | -0.255077263 | 0.437157841 | 0.435020952 | -0.423043791 | -0.331149193 |
| 77 | 0.050557668 | -0.25687484 | 0.372768105 | 0.908585623 | -0.371801666 | 0.093891659 |
| 78 | -0.119381608 | -0.332599826 | 0.147158251 | 0.182032501 | 0.114445015 | -0.510870235 |
| 79 | 1.120662445 | -0.229026201 | 0.415555852 | 1.209593599 | 0.209524179 | 0.476373301 |
| 80 | -0.239068191 | -0.697450262 | 0.199171554 | 0.241890793 | -0.354241928 | -0.202982526 |
| 81 | -0.047173731 | -0.020069006 | 0.446150577 | 1.030311472 | 0.089342128 | 0.118976026 |
| 82 | 0.332166688 | -0.300748074 | 0.231368272 | 0.504404026 | 0.109340392 | -0.175654051 |
| 90 | 0.386294272 | -0.206461968 | 0.003986869 | -0.083283339 | 0.049215183 | -0.002877921 |
| 91 | 0.228643912 | -0.040077295 | 0.289736215 | 0.75704853 | 0.226902933 | -0.370162724 |
| 92 | -0.168348999 | -0.414778126 | -0.115137797 | 0.240275545 | -0.403324486 | -0.133546835 |
| 93 | -0.809958784 | 0.184002755 | -0.166270302 | 0.550495133 | 0.186974085 | -0.123383102 |
| 94 | -0.200471883 | -0.142590299 | 0.131884152 | 0.16996547 | -0.060869995 | -0.323349177 |
| 13 | -0.974257792 | 0.404657579 | -0.478078259 | -0.823078208 | 0.03716949 | 0.054194744 |
| 15 | 0.055713657 | -0.046761225 | 0.226738644 | -0.746498708 | -0.211728019 | -0.169182267 |
| 33 | 0.531852284 | 0.11058144 | 0.235607076 | -0.41077015 | -0.020194147 | 0.274637072 |
| 36 | 0.734059402 | -0.078844076 | 0.358244763 | -0.455137376 | 0.146712693 | 0.047167906 |
| 37 | 0.634906932 | 0.198696329 | 0.514638001 | 0.543357344 | -0.431554992 | 0.372032304 |
| 38 | 0.066484771 | 0.023279187 | 0.02828292 | -0.5865416 | -0.063741857 | 0.276175546 |
| 39 | 0.021198262 | 0.16797371 | 0.185823067 | -0.074386483 | -0.471320401 | 0.206354413 |
| 40 | -0.184851934 | 0.297413159 | 0.151917413 | -0.196719269 | -0.277813416 | 0.132056515 |
| 43 | 0.171651033 | 0.054939063 | 0.672616025 | 0.432356415 | -0.364331867 | 0.218925803 |
| 44 | 0.174509866 | -0.20831565 | 0.476053103 | 0.002312082 | -0.096379701 | -0.236425655 |
| 46 | 0.049597866 | -0.052804407 | 0.242441048 | -0.085519976 | -0.366500556 | -0.204328816 |
| 51 | -0.190542502 | 0.129493365 | -0.150450399 | 0.901642738 | -0.111572624 | -0.226754378 |
| 52 | 0.156443943 | -0.0938317 | 0.063792103 | 1.006299627 | -0.362473409 | 0.183126479 |
| 53 | -0.129063273 | 0.680991938 | -0.635989458 | -0.174446633 | 0.216816505 | -0.08262383 |
| 54 | 0.633489817 | -0.060566915 | 0.362156369 | 0.542162743 | 0.16986695 | -0.068642853 |
| 55 | -1.136431312 | 0.212383471 | -0.41823057 | 0.178599611 | 0.355041845 | -0.137611879 |
| 57 | -0.124652496 | 0.119037754 | -0.021568409 | 0.228895159 | -0.181889898 | -0.276001071 |
| 59 | -0.226150949 | 0.446992362 | 0.151412998 | -0.229792749 | -0.262151332 | 0.155771931 |
| 61 | -0.196705867 | 0.068292887 | 0.520424535 | 0.014542157 | -0.309601062 | 0.388182234 |
| 62 | -0.129185176 | 0.360609464 | -0.170879988 | -0.035302047 | -0.163630446 | 0.095629219 |
| 66 | -0.311046498 | 0.020515274 | 0.170226046 | 0.240275545 | -0.487494527 | 0.230857751 |
| 84 | 0.014962642 | 0.147748368 | -0.193484236 | 0.264234525 | 0.037151457 | 0.10120959 |
| 85 | -0.265145658 | 0.237825801 | 0.401013399 | -0.242810376 | -0.072591105 | 0.508707203 |
| 86 | 0.405859515 | -0.32152532 | 0.119340805 | 0.69996101 | 0.238248362 | -0.174037389 |
| 87 | 0.22397844 | -0.30596739 | 0.263469002 | 0.625821627 | 0.266572589 | -0.2524686 |
| 88 | 0.368789148 | -0.522559025 | 0.503984595 | 0.495676879 | 0.004780095 | -0.096730729 |
| 89 | 0.567984226 | -0.420175529 | 0.067584608 | 0.366180126 | -0.211069075 | -0.316318535 |
| 12 | -0.254344984 | 0.135553548 | -0.229185058 | 0.04251042 | 0.38548737 | -0.077504639 |
| 18 | -0.114555163 | 0.108378474 | -0.238994836 | -0.597689987 | 0.448172641 | -0.371308269 |
| 19 | 0.443756725 | 0.091874189 | -0.112915155 | -0.683479119 | 0.367218617 | -0.24592766 |
| 24 | -0.469668049 | -0.001429634 | -0.759936894 | -0.57551613 | 0.290134606 | -0.003763326 |
| 27 | -0.141455307 | 0.124089688 | -1.219512254 | -0.248065249 | 0.460241603 | -0.200343264 |
| 29 | 0.420266864 | -0.161393988 | -0.042099441 | -0.09226006 | 0.160020765 | 0.01682339 |
| 30 | -0.254295388 | 0.039054763 | -0.203330744 | 0.493169355 | -0.434596958 | -0.737916833 |
| 31 | -0.369772707 | 0.377131998 | -0.220644589 | -1.192677403 | -0.236976552 | 0.150381458 |
| 32 | -0.224746709 | -0.025777429 | 0.076675462 | 0.058147956 | -0.298629876 | -0.419581275 |
| 41 | 1.037853064 | 0.05926695 | 0.309567375 | 0.111030367 | -0.272168878 | 0.62702332 |
| 42 | -0.073576322 | 0.276561735 | -0.06087483 | -0.518682655 | 0.346966713 | -0.014035363 |
| 48 | 0.224015768 | 0.304215433 | -0.001814846 | -0.58285292 | 0.291291012 | 0.264433818 |
| 49 | -0.470087016 | -0.343680617 | -0.397007117 | -0.274761976 | -0.00580531 | -0.249455525 |
| 65 | 0.596850809 | -0.444060423 | 0.482525662 | 0.107344406 | -0.112048288 | 0.258456949 |
| 2 | -0.481769852 | -0.395575988 | -0.339308218 | -0.522141669 | 0.575787992 | -0.521084844 |
| 3 | -0.205037556 | -0.192430478 | -0.410252882 | 0.187159841 | 0.182299041 | 0.478666942 |
| 4 | -0.203595283 | -0.20862928 | -0.139364352 | -1.322730532 | 0.041372828 | 0.26449351 |
| 6 | -0.153479577 | 0.254552517 | -0.275945137 | -1.199475526 | 0.368574122 | -0.083531146 |
| 8 | -0.300394048 | 0.254750342 | -0.62718674 | -1.030429513 | 0.307860311 | -0.082302658 |
| 14 | -0.846146457 | 0.166852108 | -0.548215901 | -0.374247527 | 0.356622397 | -0.048784829 |
| 16 | 0.320504234 | 0.251595736 | -0.145990238 | -0.474771514 | 0.429165964 | 0.071388727 |
| 21 | -0.115210497 | -0.049748667 | -0.168861474 | 0.075452752 | -0.406745338 | 0.123415794 |
| 22 | -0.276571253 | 0.007003755 | -0.918533827 | -0.508376827 | 0.931168939 | -0.570731902 |
| 23 | -1.493415238 | 0.061776638 | -1.1113754 | -0.639664863 | 0.398000186 | -0.14643181 |
| 26 | -0.425629903 | 0.416503379 | -0.404947999 | -0.374247527 | 0.267906886 | -0.121921793 |
| 45 | -0.48054462 | 0.267580995 | -0.26975506 | -0.083283339 | -0.188969859 | -0.300743674 |
| 58 | -0.836823257 | 0.587201261 | -0.833439802 | -0.101318093 | 0.569385926 | -0.039906155 |
| 60 | 0.391085504 | 0.082876177 | 0.265340172 | -0.263997609 | 0.324075872 | 0.258502233 |
| 64 | -0.257537296 | 0.064197259 | -0.335719666 | -0.131336509 | 0.207913383 | -0.283866062 |
| 68 | 0.811547939 | -0.377576067 | 0.008877083 | -0.413874778 | -0.411441562 | 0.391994648 |

| Hex2Cer | LPC | LPE | PA | PC | PE | PG |
|---|---|---|---|---|---|---|
| 0.367469097 | 0.534888676 | 0.107496306 | 0.224745841 | -0.0492767 | -0.423234247 | 0.486504245 |
| 0.260255117 | 0.419098783 | 0.541940735 | 0.201801427 | -0.006323782 | 0.199440955 | -0.03813558 |
| 0.533667478 | -0.051322952 | 0.194821368 | 0.191199841 | -0.005244339 | -0.220565588 | 0.222664314 |
| 0.344417355 | 0.130248701 | -0.088567639 | 0.364550881 | 0.042783432 | -0.434656961 | -0.171947085 |
| 0.477243139 | 0.169852864 | -0.269490166 | 0.145285039 | -0.001744186 | -0.553396558 | -0.450803929 |
| -0.366784639 | 0.294434137 | 0.205996318 | -0.182769646 | -0.034642464 | 0.375194041 | -0.266906249 |
| -0.2493721 | 0.068210944 | 0.605132823 | -0.068608779 | -0.087908818 | 0.436400216 | -0.270552227 |
| -0.151015645 | -0.674943556 | -0.170454526 | -0.188935796 | -0.014925405 | 0.172607163 | 0.093491492 |
| -0.02891336 | -0.334284928 | -0.309609926 | 0.039555353 | 0.080905905 | 0.244830391 | -0.594935796 |
| 0.263822334 | 0.180882996 | -0.26304671 | -0.01639072 | 0.053031799 | 0.016854245 | -0.387167879 |
| -0.259746662 | 0.099153595 | -0.179090097 | -0.18280489 | 0.036296941 | 0.368736204 | -0.531602657 |
| -0.220817057 | -0.102764376 | -0.125730755 | -0.095191818 | 0.054536323 | 0.278109757 | -0.537243241 |
| -0.463920793 | -0.240801749 | -0.127307358 | -0.278544656 | -0.042368653 | 0.32507728 | -0.240823063 |
| 0.293230621 | 0.622011856 | 0.309998092 | -0.097148091 | 0.052529767 | 0.205351534 | -0.466109355 |
| -0.043534592 | -0.024731967 | -0.069807234 | -0.138577475 | -0.006478784 | 0.283659888 | -0.510646922 |
| 0.014467038 | -0.279293937 | -0.169829184 | -0.35213672 | -0.062444367 | 0.488066653 | -0.67675146 |
| -0.029410885 | 0.175907823 | 0.099924693 | -0.176837134 | 0.031167784 | 0.047193387 | -0.707886239 |
| -0.086998168 | 0.163546342 | 0.411849663 | 0.105374309 | -0.048239606 | 0.372118535 | -0.339429666 |
| -0.125595618 | -0.012798824 | -0.138634102 | -0.162144027 | 0.029884136 | 0.365521165 | -0.192026615 |
| -0.061949459 | -0.046278242 | -0.145828748 | 0.046959963 | 0.025065561 | 0.141434732 | -0.510468804 |
| -0.061884545 | 0.050699129 | 0.066278085 | -0.391877275 | -0.001346743 | 0.228418201 | -0.38500344 |
| -0.246788009 | -0.089944524 | -0.10552483 | -0.001488469 | 0.005652508 | 0.306203369 | -0.286381928 |
| 0.11711701 | -0.153404792 | 0.002680727 | 0.03017253 | -0.058385254 | -0.676034664 | 0.052933624 |
| -0.450242511 | 0.373857755 | -0.111501692 | -0.123274027 | 0.076804784 | -0.441875705 | -0.073283175 |
| 0.322819493 | 0.084647523 | 0.108522288 | 0.0837783 | -0.04598106 | -0.138101181 | 0.26273682 |
| -0.091395619 | 0.355670721 | -0.105240059 | -0.051081384 | 0.022267711 | -0.082679703 | 0.105253526 |
| -0.064959536 | 0.251663716 | 0.171844005 | 0.043517394 | 0.022710405 | -0.32247059 | -0.259710195 |
| 0.13582981 | -0.084860142 | 0.293297168 | -0.186942692 | -0.088327108 | 0.282487254 | -0.322772488 |
| 0.126551479 | 0.168430917 | 0.085010614 | 0.007213191 | -0.012665416 | 0.070899624 | 0.193072287 |
| 0.304154199 | -0.048829404 | -0.035147836 | 0.204503596 | -0.022070583 | 0.035370826 | 0.094654299 |
| 0.22121297 | 0.09618749 | -0.203576428 | 0.039061299 | 0.027826967 | -0.066334439 | -0.199793927 |
| -0.102321152 | -0.042028927 | -0.28907513 | -0.150479936 | -0.099713346 | -0.08628327 | 0.119486652 |
| -0.166538655 | -0.053993836 | -0.196519376 | -0.000639915 | 0.00073995 | 0.038717864 | -0.04769671 |
| -0.266977618 | -0.020789593 | 0.008273882 | -0.067318025 | 0.045450229 | 0.519310414 | -0.088135879 |
| 0.198779886 | 0.305464729 | 0.366820685 | -0.431060869 | 0.021870594 | -0.054000912 | 0.050071583 |
| -0.071644963 | -0.015501443 | -0.081297578 | 0.21349424 | -0.053237324 | 0.139105699 | 0.41314838 |
| -0.053436986 | 0.577698899 | -0.346747578 | -0.046640368 | 0.092284447 | 0.051793109 | -0.051742392 |
| -0.307058569 | -0.576721141 | -0.165664268 | 0.04454376 | 0.011541855 | 0.253370066 | 0.308559742 |
| -0.070434926 | -0.004799709 | 0.318707477 | -0.06478269 | 0.034880273 | 0.214829578 | 0.233382259 |
| 0.519034969 | 0.077459838 | 0.160105323 | -0.35303521 | 0.015644198 | -0.337834265 | 0.252264749 |
| 0.332831654 | -0.023101222 | -0.149597397 | -0.136963912 | -0.043715428 | -0.035315179 | -0.569131591 |
| -0.088111229 | -0.308024031 | 0.025166294 | 0.098476366 | 0.034893172 | 0.341455694 | 0.323233992 |
| 0.314608548 | -0.05348558 | 0.505448856 | -0.004327922 | -0.010537235 | -0.266497641 | -0.608223212 |
| 0.032689829 | -0.139999045 | 0.132181921 | -0.016500461 | -0.018338794 | 0.277717446 | -0.173971628 |
| 0.869571074 | 0.391268403 | -0.203388455 | 0.230660273 | 0.020992129 | -0.414039689 | -0.26851366 |
| -0.015380605 | -0.071221246 | -0.085547926 | -0.074536123 | 0.012992585 | -0.099350402 | -0.689860368 |
| 0.128142729 | -0.455614658 | -0.309297731 | -0.192150694 | -0.012547056 | 0.065687467 | -0.307143397 |
| -0.508189555 | 0.2552951 | 0.255941557 | -0.137233277 | -0.037630125 | 0.326899579 | -0.400429151 |
| -0.374579763 | 0.432941946 | 0.301086608 | -0.060619419 | 0.039554712 | -0.30634543 | -0.921301074 |
| -0.275736737 | 0.043310867 | 0.040130719 | -0.105639377 | -0.163656052 | 0.168284911 | 0.417249486 |
| -0.515649459 | 0.129335556 | -0.211439697 | 0.237465691 | 0.020189203 | -0.230930996 | 0.428343124 |
| -0.176796548 | -0.036361879 | 0.367694646 | 0.235213444 | 0.035599113 | -0.098586729 | 0.641687862 |
| 0.061405058 | -0.424226427 | 0.234767629 | -0.063860729 | -0.071472836 | -0.059505261 | 0.295766047 |
| -0.240914884 | 0.03579164 | 0.015877053 | 0.061909418 | -0.035503028 | 0.024473164 | 0.441681939 |
| 0.260876652 | 0.448928388 | 0.211446883 | -0.029059515 | 0.037451753 | 0.211365288 | 0.341705028 |
| -0.803382822 | -0.465578529 | -0.030002844 | 0.028191241 | 0.074477688 | -0.157999538 | 0.365160684 |
| 0.15523286 | -0.061463727 | 0.082389335 | 0.30711392 | -0.047179309 | -0.127198236 | 0.240845336 |
| -0.108676184 | 0.05845306 | 0.295518297 | 0.007883322 | -0.026796762 | 0.083479677 | 0.267192537 |
| 0.849868175 | 0.071240836 | -0.145710763 | 0.109255833 | 0.017867493 | -0.160383834 | 0.360752755 |
| -0.276916481 | 0.389391462 | -0.070769308 | 0.08196931 | -0.005870919 | 0.234616026 | -0.040625877 |
| -0.198329391 | 0.44612052 | 0.257032912 | 0.135964636 | -0.023224228 | -0.088618669 | 0.36131117 |
| -0.128916309 | -0.15065045 | 0.112560827 | 0.064077909 | 0.013476721 | -0.126052249 | 0.28492377 |
| 0.245591026 | 0.137864713 | -0.068853324 | 0.029860499 | 0.073002479 | -0.27877284 | -0.560496225 |
| -0.358592957 | -0.06790004 | 0.104375788 | 0.064542207 | -0.002550341 | -0.122375643 | 0.375360976 |
| 0.409873994 | -0.51564717 | -0.369931238 | -0.012624095 | 0.021193265 | -0.496021505 | -0.074395351 |
| 0.311486426 | -0.378916142 | -0.229084966 | -0.057900611 | 0.038089673 | -0.408206099 | 0.133570972 |
| -0.259817595 | 0.036536405 | 0.009405737 | 0.008722131 | 0.044022914 | -0.365861837 | 0.195751878 |
| -0.043911927 | 0.045029229 | 0.207554376 | -0.062305183 | -0.011818452 | -0.183788611 | 0.426173201 |
| -0.211121582 | -0.725883878 | -0.336744645 | -0.147476413 | -0.044854459 | 0.238520229 | 0.424604448 |
| 0.22591951 | 0.176399702 | -0.047926606 | 0.15399358 | 0.056648576 | -0.183219367 | 0.586851846 |
| 0.156042008 | 0.528206025 | 0.404029878 | 0.266925247 | 0.045958262 | -0.691098701 | 0.740241988 |
| -0.314970756 | 0.004024827 | -0.24122758 | 0.164465897 | -0.105135008 | -0.081351333 | 0.639938545 |
| -0.275240661 | -0.828932031 | -0.447448109 | -0.038310284 | -0.06251505 | 0.016718318 | 0.183345599 |
| 0.138574502 | -0.172191111 | -0.219679596 | 0.150067557 | 0.008589738 | -0.121032204 | 0.504379811 |
| -0.080766069 | -0.524409093 | -0.521455493 | 0.243279488 | 0.027489886 | 0.108845894 | 0.342764126 |
| -0.395632695 | -0.385898893 | 0.366544951 | 0.114542421 | -0.049081496 | 0.469284883 | 0.313955928 |
| 0.260459996 | 0.387441956 | 0.121668314 | 0.025560001 | 0.001314049 | 0.134160833 | 0.582498828 |
| -0.177933805 | -0.599419928 | -0.169741105 | 0.071021349 | 0.011495203 | 0.236249227 | 0.196479139 |
| 0.618064045 | 0.029421055 | -0.553984832 | 0.07129992 | 0.094576333 | -0.458840708 | -0.081946556 |

| PI | | PS | | SM | | TG |
|---|---|---|---|---|---|---|
| | -0.190949926 | | -0.078313062 | | 0.349870309 | -0.030998228 |
| | 0.091841854 | | 0.19265086 | | 0.223118267 | -0.106943161 |
| | 0.034589604 | | 0.13478739 | | 0.098870114 | 0.010897618 |
| | -0.060666306 | | -0.91274825 | | 0.412287984 | -0.123734334 |
| | -0.325420031 | | -0.665392364 | | 0.003230222 | 0.01663426 |
| | 0.353717237 | | 0.152780172 | | -0.136666918 | 0.00323975 |
| | 0.035701263 | | 0.536005257 | | 0.044394725 | 0.112268315 |
| | -0.005278208 | | 0.122871758 | | 0.014609112 | 0.116592403 |
| | 0.040334664 | | -0.294826935 | | 0.259477544 | -0.122382784 |
| | 0.033934634 | | -0.599809623 | | 0.56728702 | -0.198877003 |
| | 0.213269517 | | -0.067687599 | | 0.587692076 | -0.181534661 |
| | -0.0551626 | | -0.23002256 | | 0.446803975 | -0.122963762 |
| | 0.099273948 | | 0.384394336 | | -0.027001102 | 0.11226535 |
| | -0.057789167 | | 0.196365103 | | 0.531519113 | -0.344225911 |
| | 0.220049178 | | 0.28734148 | | 0.278633793 | -0.007232113 |
| | 0.022744144 | | 0.607669561 | | 0.10985902 | 0.108022832 |
| | -0.074522478 | | -0.279641939 | | 0.194411127 | -0.072609197 |
| | 0.251949644 | | 0.075543139 | | -0.094013013 | 0.041774196 |
| | -0.189582767 | | 0.046738823 | | 0.285002633 | -0.066315547 |
| | 0.230752271 | | 0.032592455 | | -0.058734332 | -0.011715553 |
| | -0.008883792 | | 0.14311295 | | -0.091601843 | 0.020122912 |
| | 0.15037101 | | -0.024827349 | | 0.101546105 | -0.002803391 |
| | -0.09643351 | | -0.622076977 | | -0.729837627 | 0.202644436 |
| | -0.191719147 | | -0.911544466 | | 0.312762618 | -0.200130039 |
| | 0.369085457 | | 0.014645004 | | 0.14635349 | 0.032996759 |
| | 0.027649107 | | -0.07410477 | | 0.284263309 | -0.123275257 |
| | -0.103275236 | | 0.089670163 | | 0.547866445 | -0.138949558 |
| | -0.101627377 | | 0.332555442 | | -0.363938775 | 0.18079783 |
| | -0.298589071 | | 0.179017716 | | 0.283019619 | -0.005675357 |
| | -0.129110329 | | 0.441140445 | | 0.25564396 | 0.037576101 |
| | 0.052775941 | | -0.19933892 | | 0.509357188 | -0.131718553 |
| | 0.133515166 | | -0.505644581 | | 0.19992602 | 0.15156531 |
| | -0.212432949 | | -0.683082562 | | 0.075130252 | 0.03937612 |
| | -0.008285015 | | -0.170216277 | | 0.072837642 | -0.0888631 |
| | 0.19447936 | | -0.053430902 | | 0.149461264 | -0.10100688 |
| | 0.148137278 | | 0.255540274 | | -0.169082211 | 0.092623995 |
| | -0.08941862 | | -0.763596799 | | 0.569862262 | -0.40693696 |
| | -0.020822695 | | 0.305846546 | | -0.561134512 | 0.105343368 |
| | 0.081443865 | | -0.156791122 | | -0.099591547 | -0.045987324 |
| | -0.038795887 | | 0.099410676 | | 0.082518935 | -0.023217653 |
| | -0.113995653 | | -0.144225424 | | 0.298285922 | 0.061690399 |
| | 0.024818545 | | 0.493589795 | | -0.110377514 | -0.011382856 |
| | -0.084730507 | | 0.774293307 | | -0.312102767 | 0.081657159 |
| | 0.146295044 | | -0.041254196 | | 0.269009562 | 0.023706715 |
| | 0.07655649 | | -0.331109008 | | 0.06908324 | -0.111899156 |
| | 0.052527829 | | -0.143565564 | | -0.124934745 | 0.035823595 |
| | 0.122978653 | | 0.311571777 | | 0.114764188 | 0.085334605 |
| | 0.304069311 | | -0.014045366 | | 0.195835811 | -0.029549353 |
| | -0.136975666 | | 0.10069754 | | -0.050104275 | -0.095697073 |
| | 0.170644079 | | -0.085345963 | | -0.091085476 | 0.234188123 |
| | 0.154469585 | | -0.807260252 | | -0.056707692 | -0.02013617 |
| | -0.17176352 | | -0.013898908 | | -0.030320212 | -0.013948231 |
| | -0.035686765 | | 0.386058221 | | -0.536283784 | 0.22949008 |
| | 0.125438249 | | 0.111447371 | | -0.637739792 | 0.121587769 |
| | -0.066904077 | | 0.222762286 | | 0.246603942 | -0.172409684 |
| | -0.901720886 | | -0.331393232 | | -0.160399217 | 0.033387172 |
| | 0.062626058 | | -0.085838481 | | 0.107786013 | 0.092464108 |
| | -0.014813179 | | 0.441830969 | | 0.128787853 | 0.045075725 |
| | 0.038576573 | | 0.194081844 | | 0.335271115 | -0.068316206 |
| | 0.165607524 | | -0.010901234 | | -0.133863986 | -0.063370816 |
| | -0.207011551 | | 0.230421783 | | -0.022085832 | -0.01953895 |
| | -0.114465325 | | 0.066459665 | | -0.421912022 | 0.088874751 |
| | -0.072865041 | | -0.560898268 | | 0.180506017 | -0.140391275 |
| | 0.001055422 | | 0.458857562 | | -0.470764698 | 0.097124594 |
| | -0.037460212 | | -0.381205304 | | -0.259189016 | 0.110659825 |
| | -0.118857296 | | 0.118106757 | | -0.30806297 | 0.072123455 |
| | 0.092768004 | | -0.69099006 | | -0.054705619 | -0.041146769 |
| | -0.007319936 | | 0.121079198 | | -0.344202639 | 0.077050593 |
| | 0.328501764 | | 0.228964493 | | -0.654844667 | 0.180516124 |
| | 0.027353604 | | 0.20889693 | | 0.315775811 | -0.1459155 |
| | -0.44940523 | | -0.392837912 | | -0.108868188 | -0.108231075 |
| | 0.052752768 | | 0.375875754 | | -0.420172835 | 0.219776253 |
| | 0.07919253 | | 0.267675844 | | -0.916601138 | 0.257074655 |
| | 0.121542446 | | 0.102025279 | | -0.141675891 | 0.042377683 |
| | -0.060767091 | | 0.123787642 | | -0.264775389 | 0.067497688 |
| | -0.08118907 | | 0.71186185 | | -0.915369048 | 0.163342849 |
| | -0.054060585 | | 0.522020387 | | 0.164291026 | -0.090684826 |
| | 0.10949762 | | 0.328422272 | | -0.490545725 | 0.098753011 |
| | -0.054130535 | | -0.203601845 | | -0.054319622 | -0.113604221 |

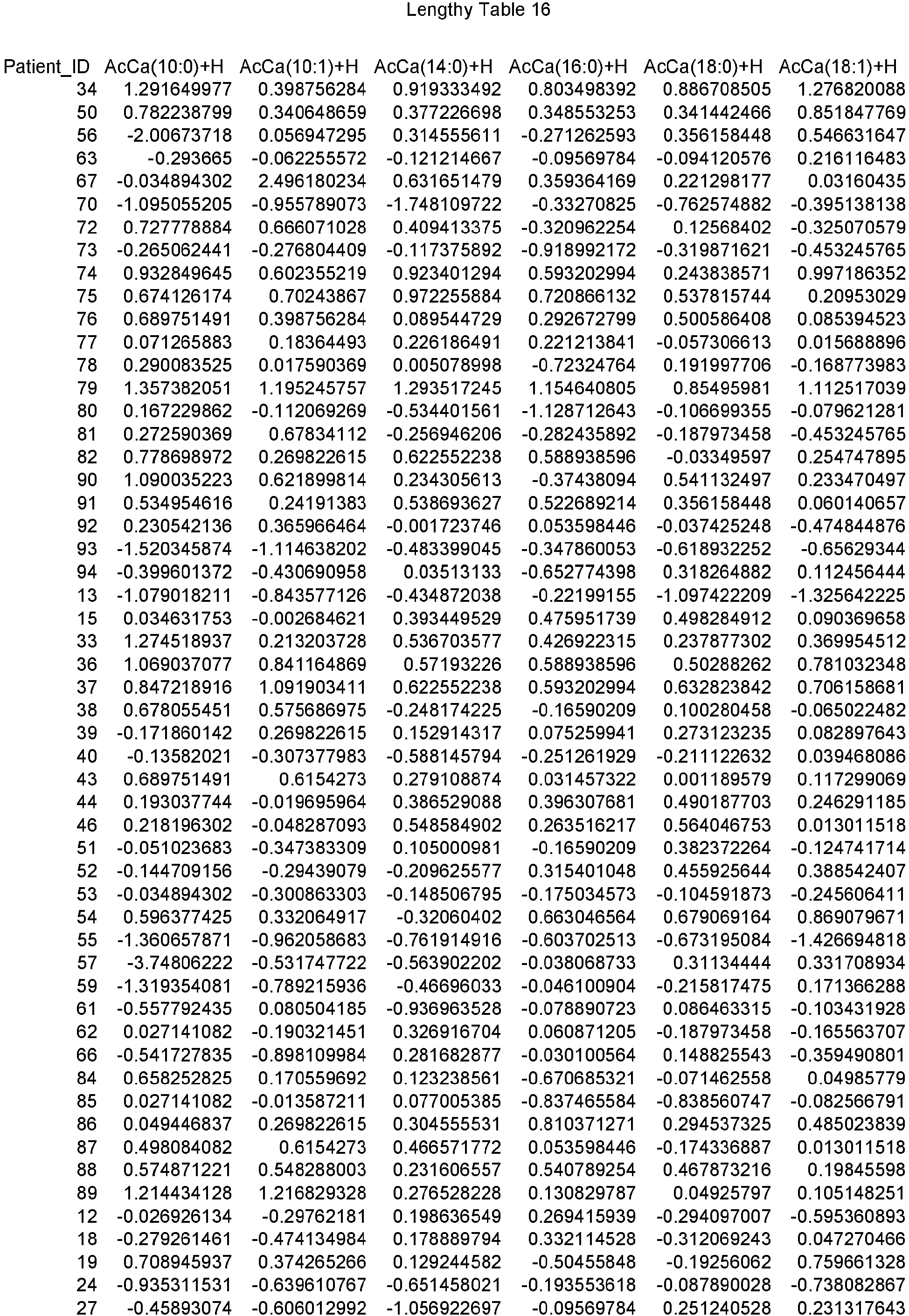

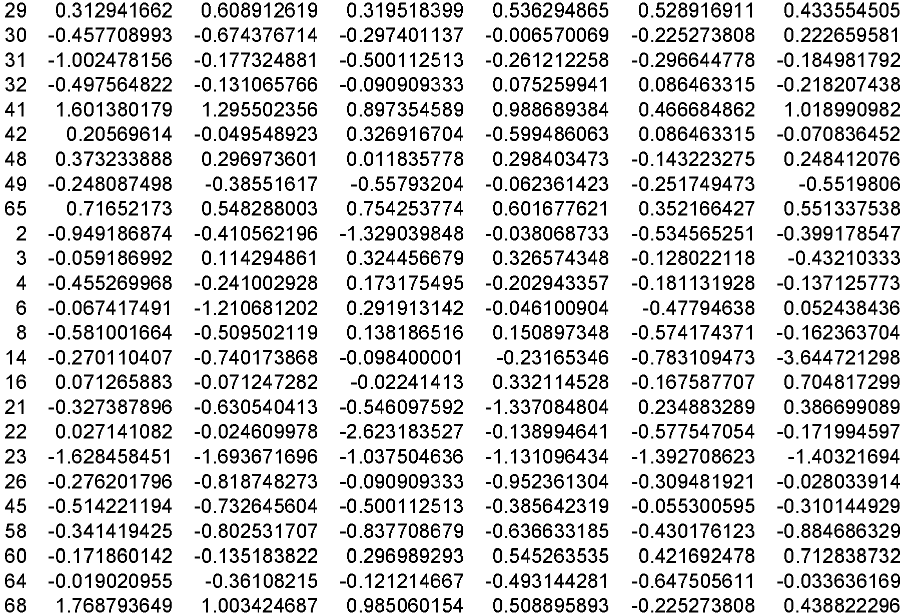

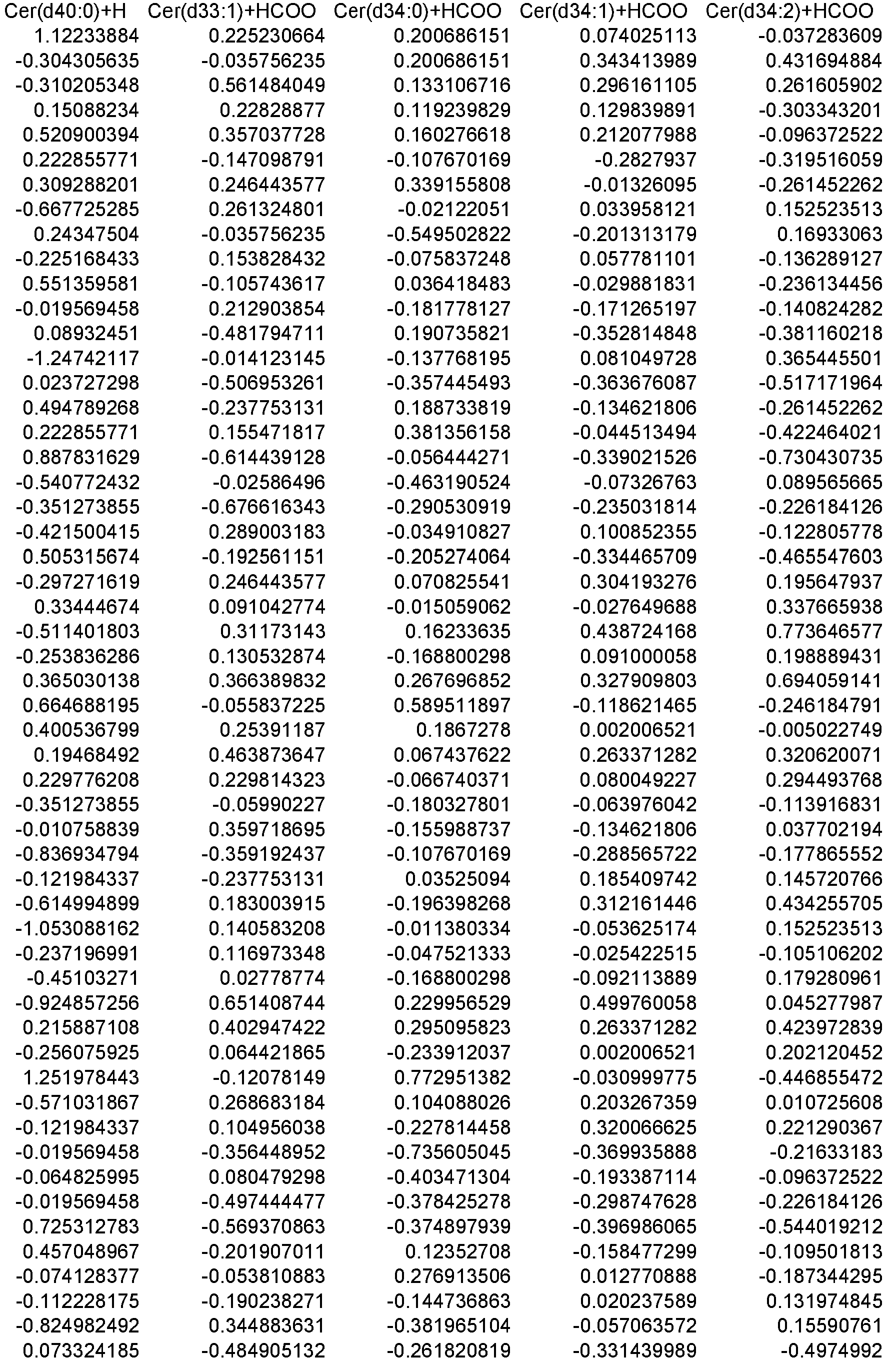

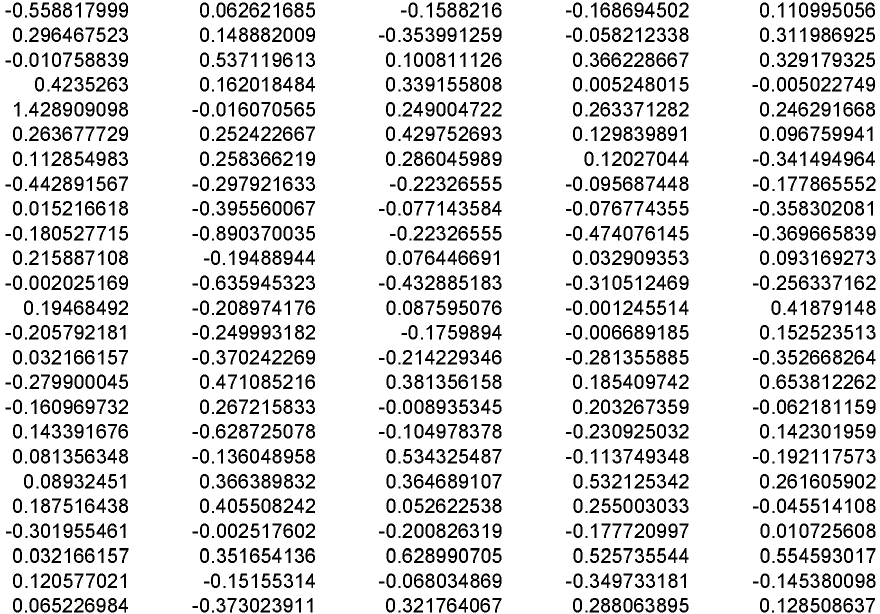

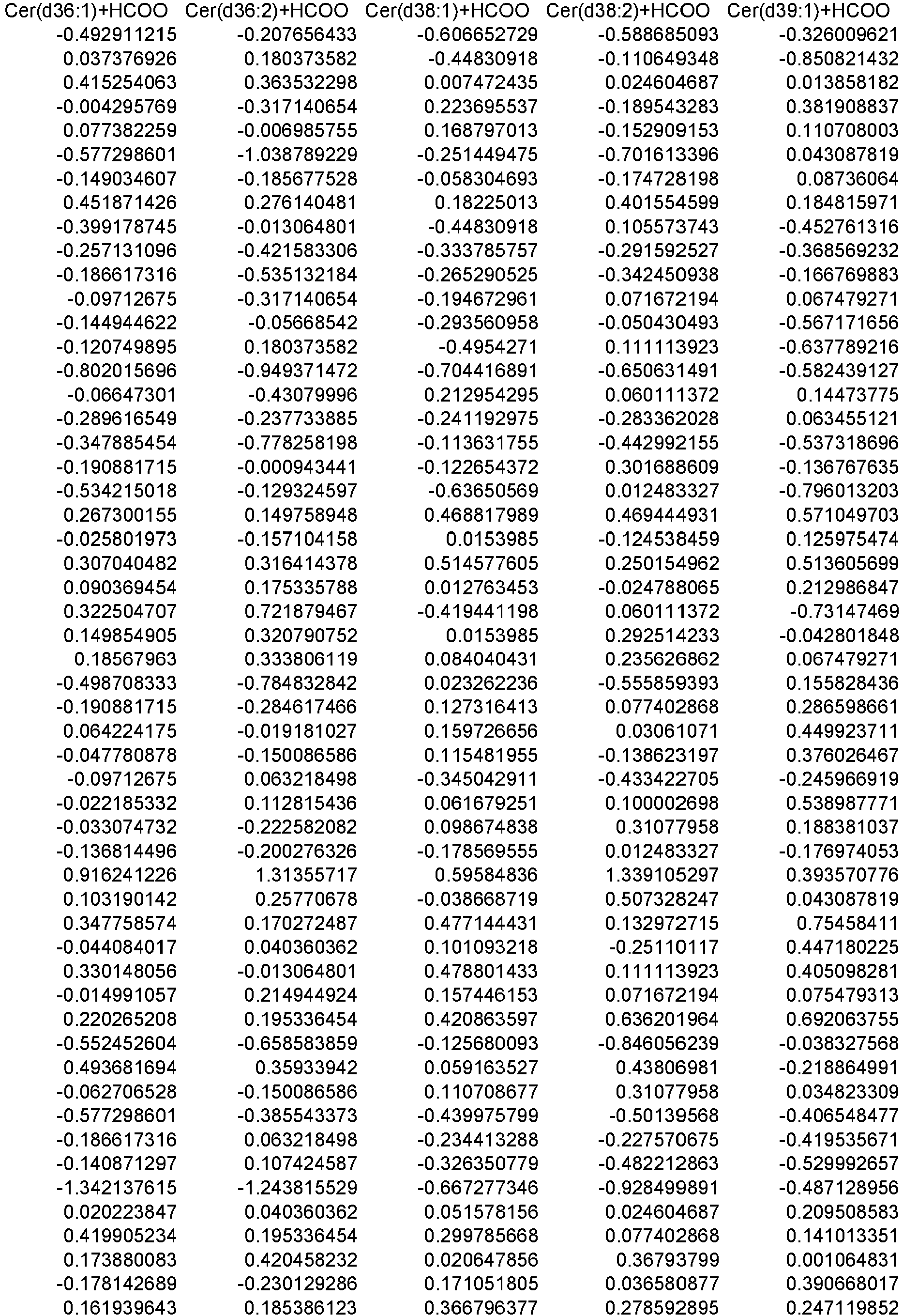

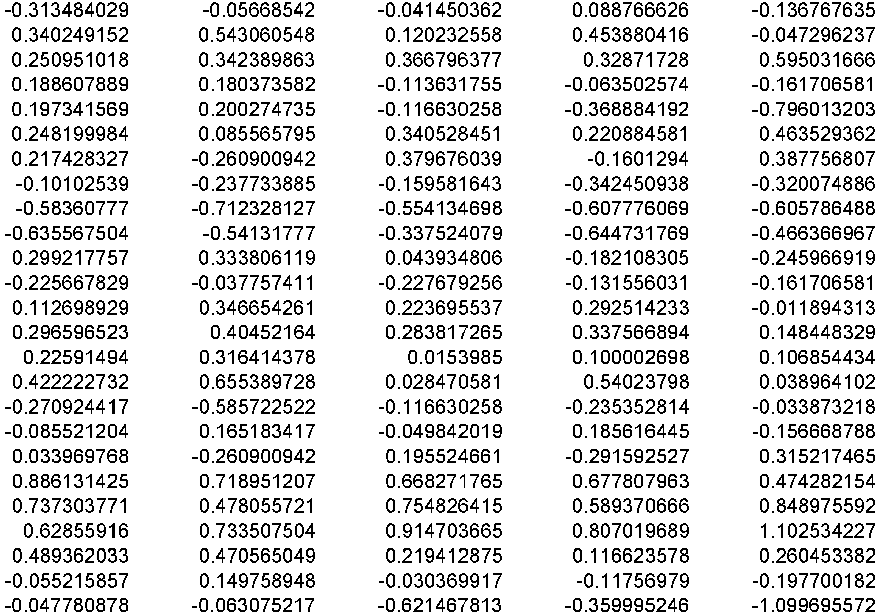

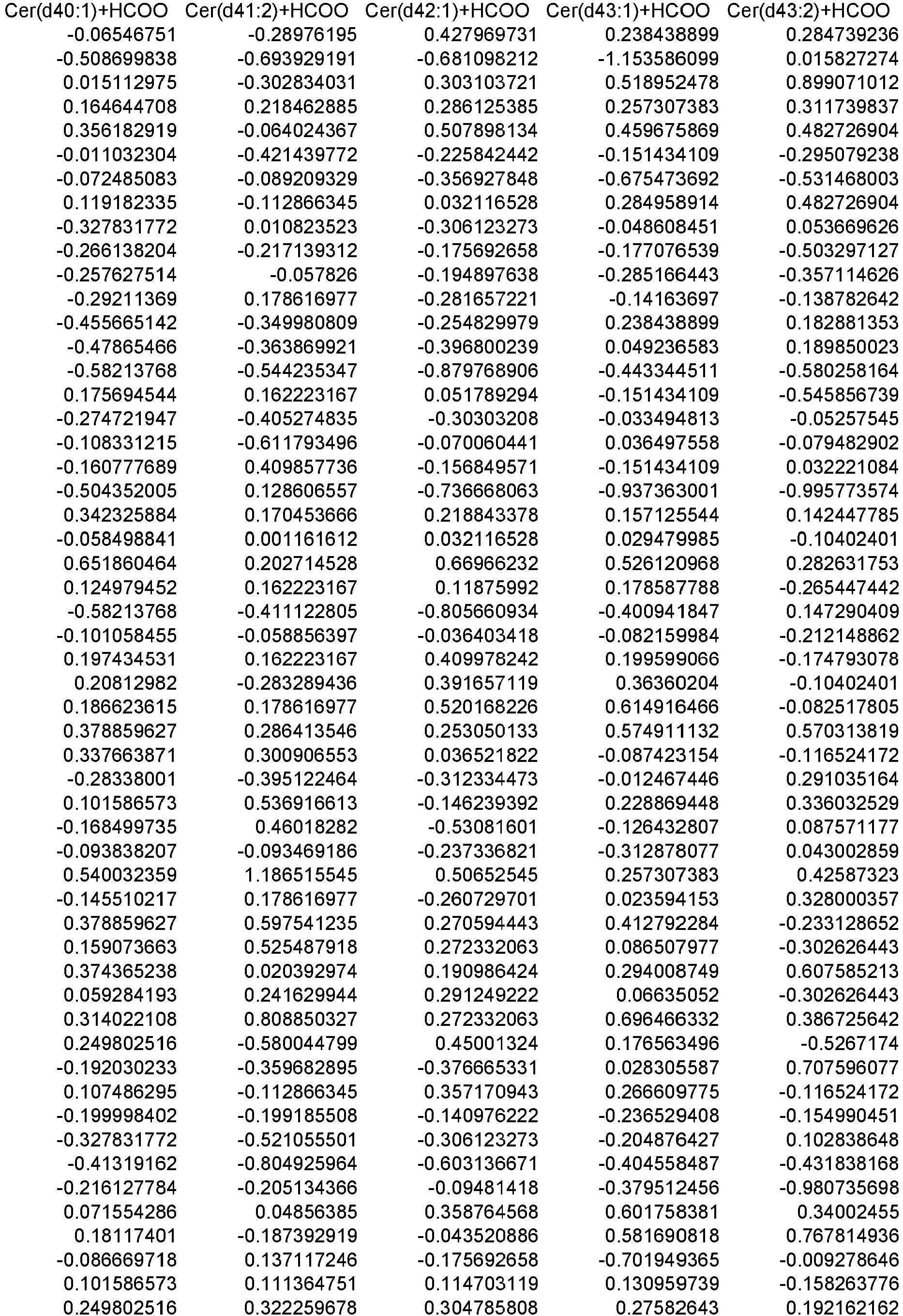

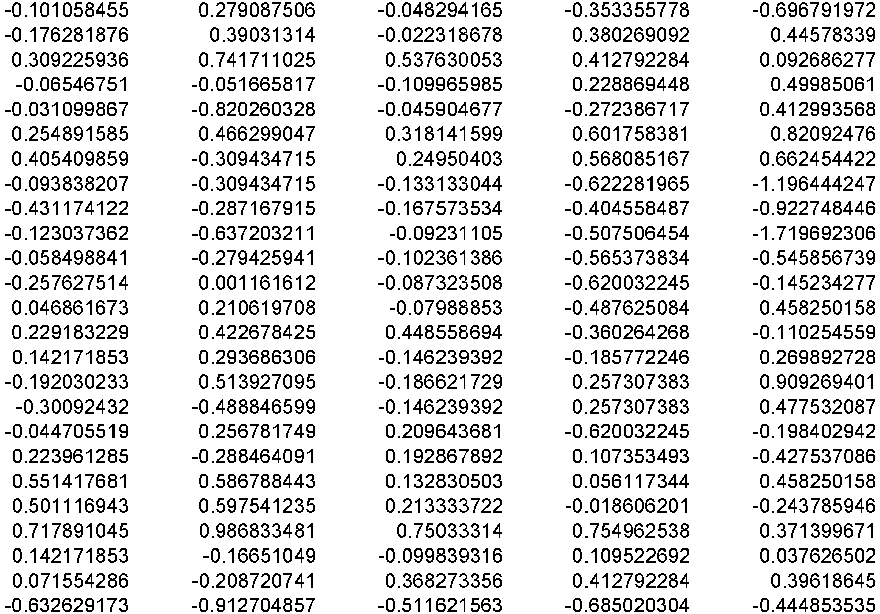

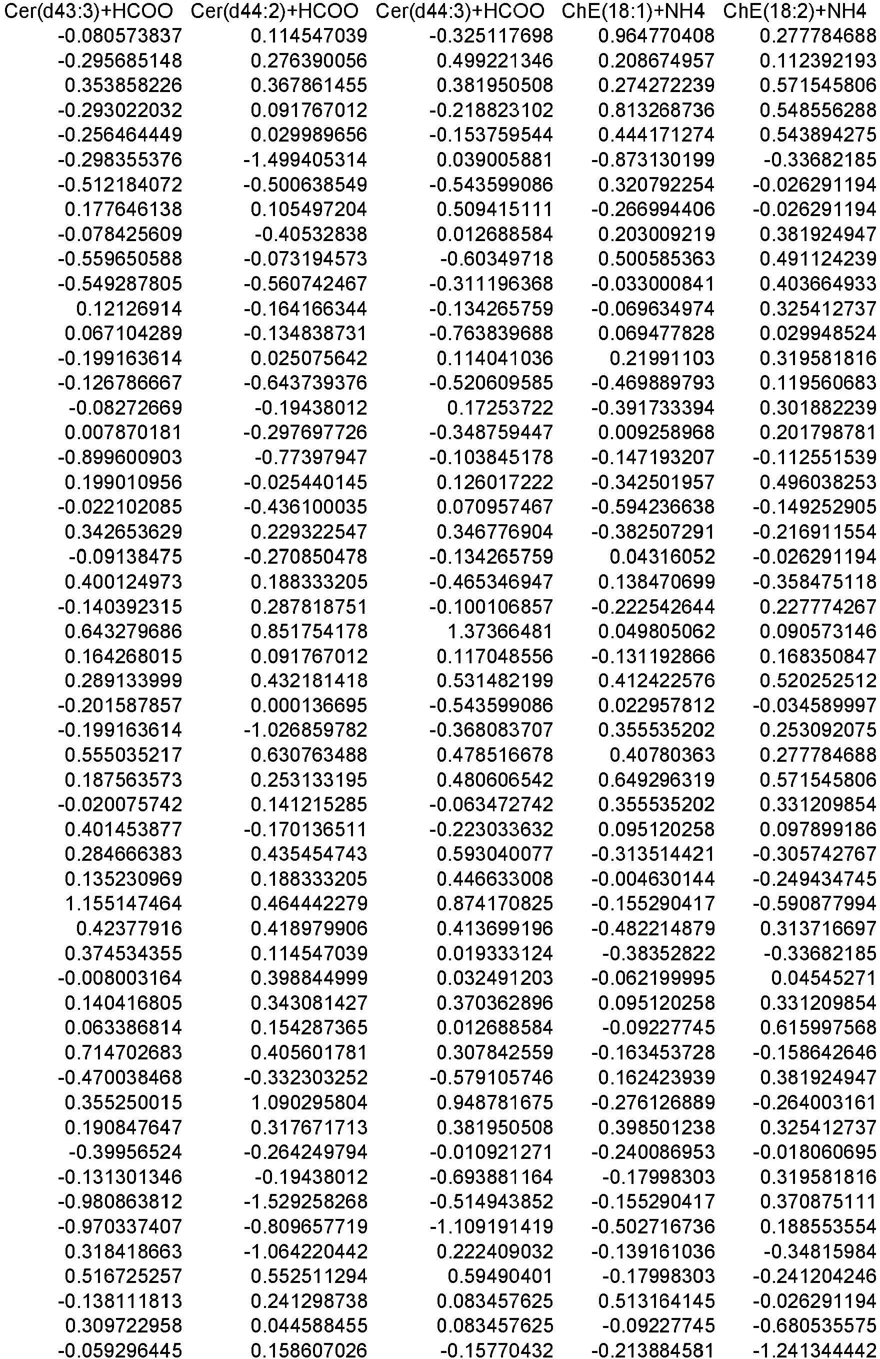

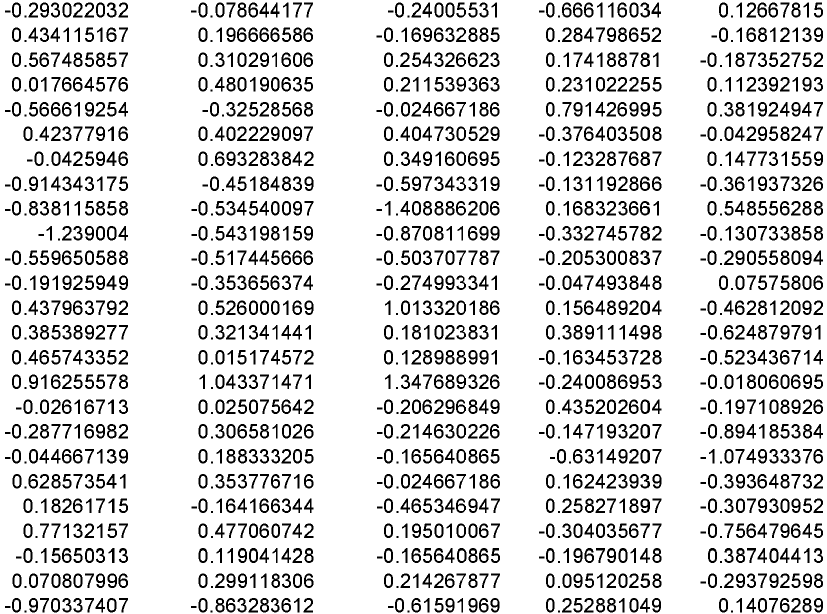

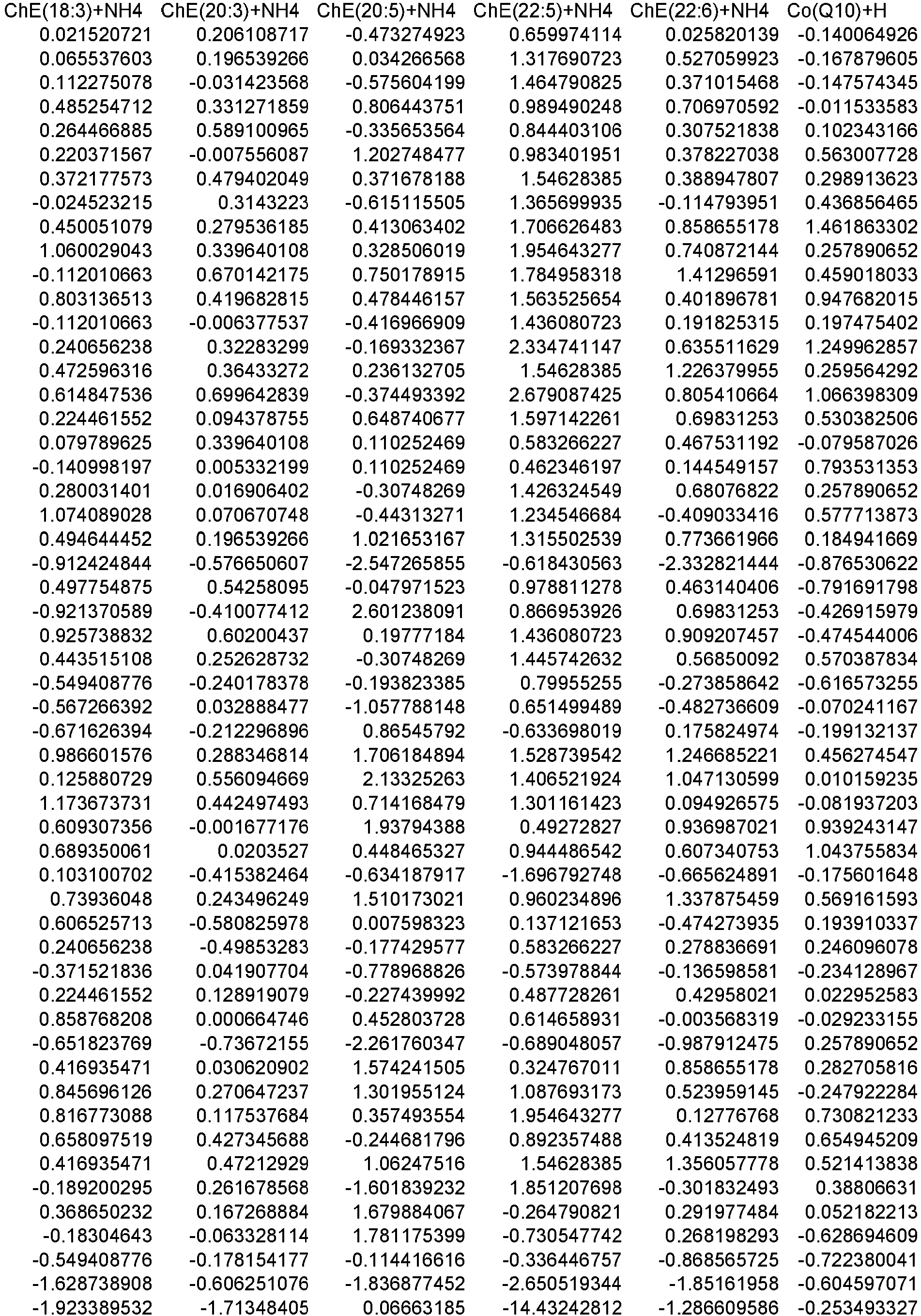

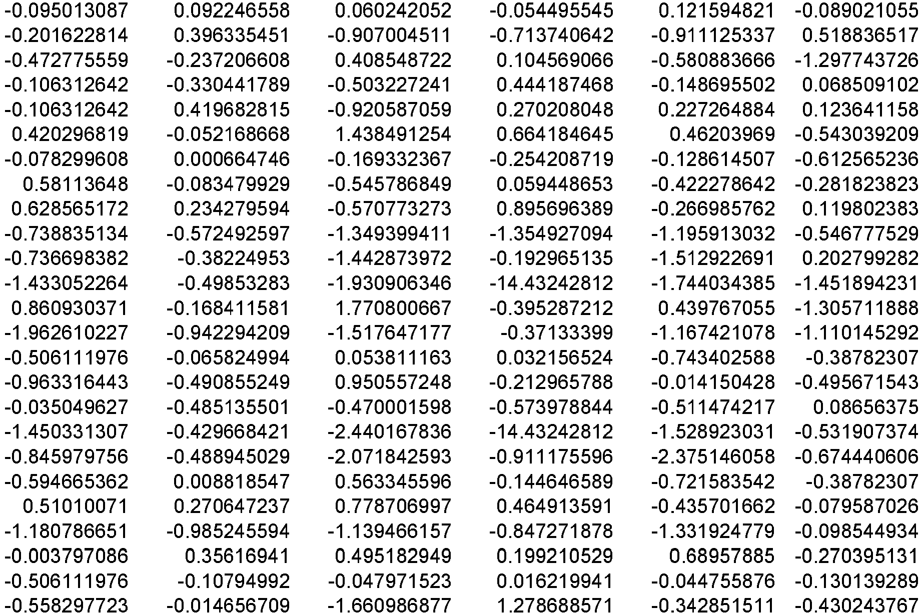

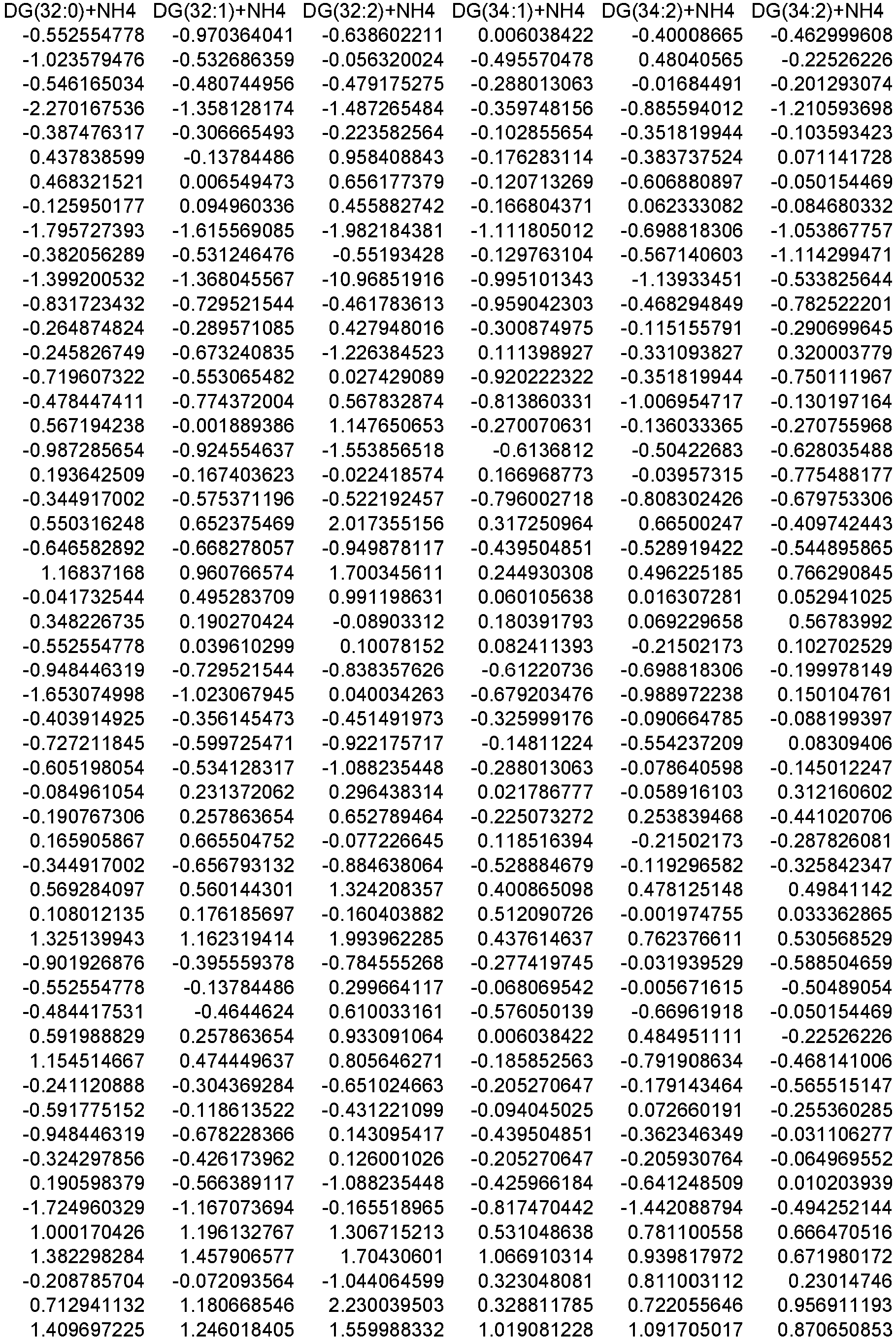

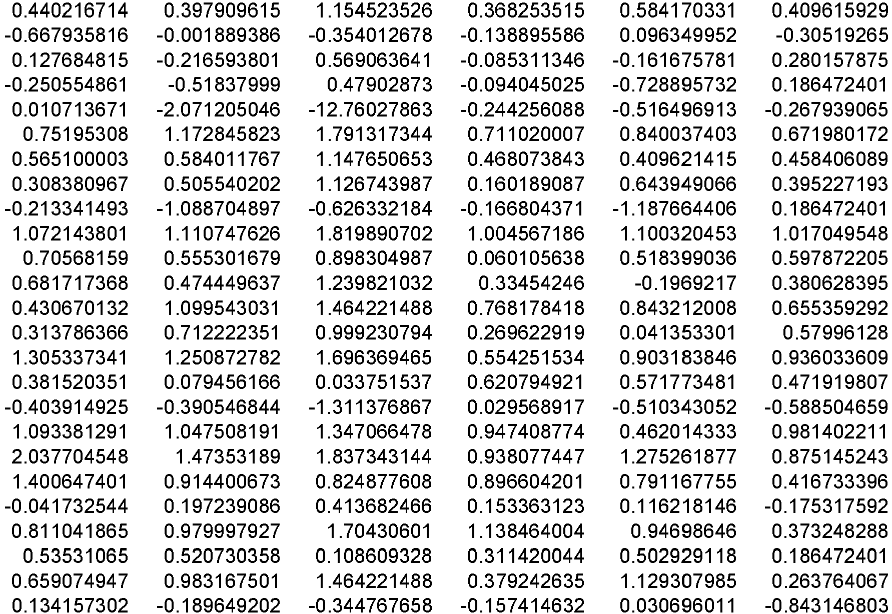

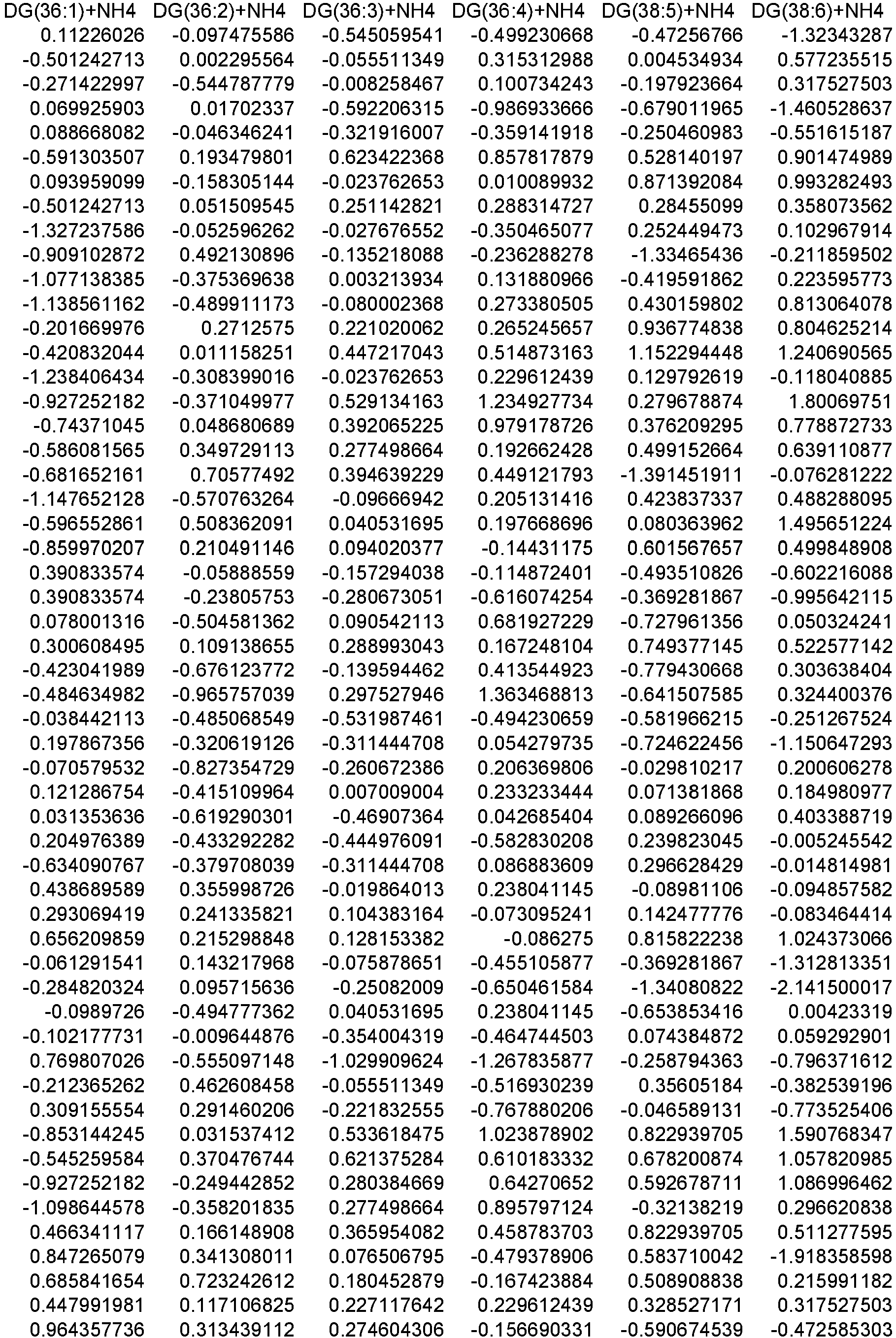

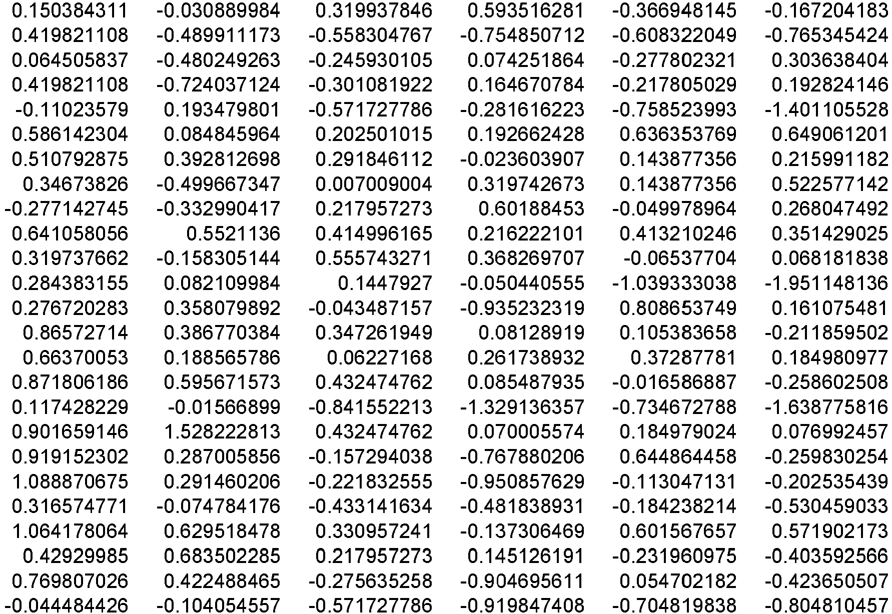

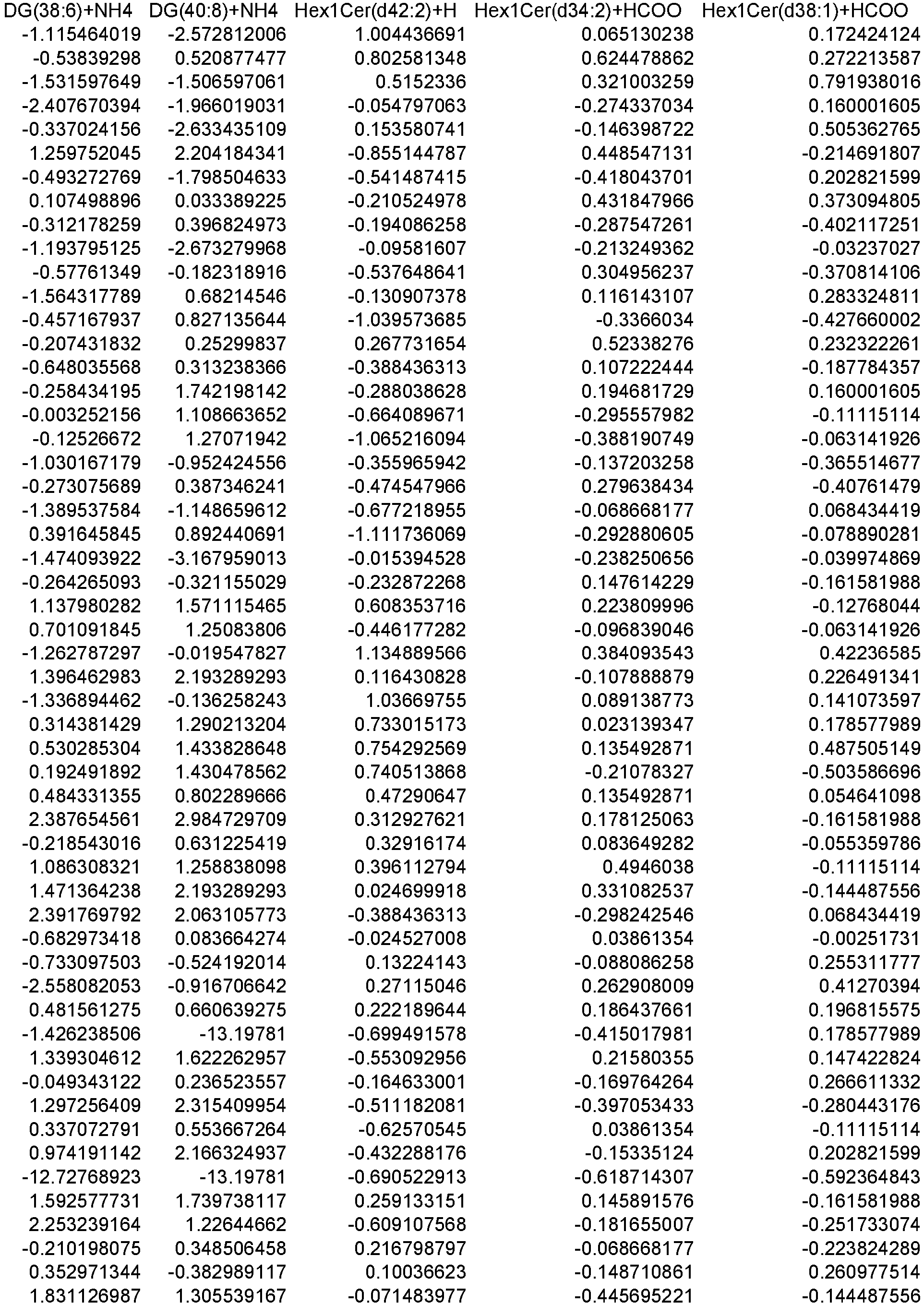

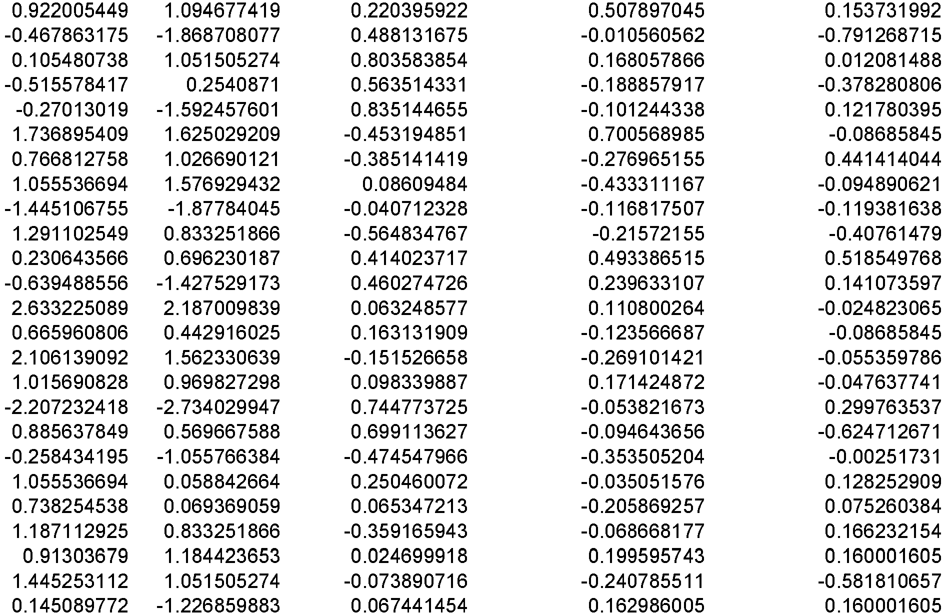

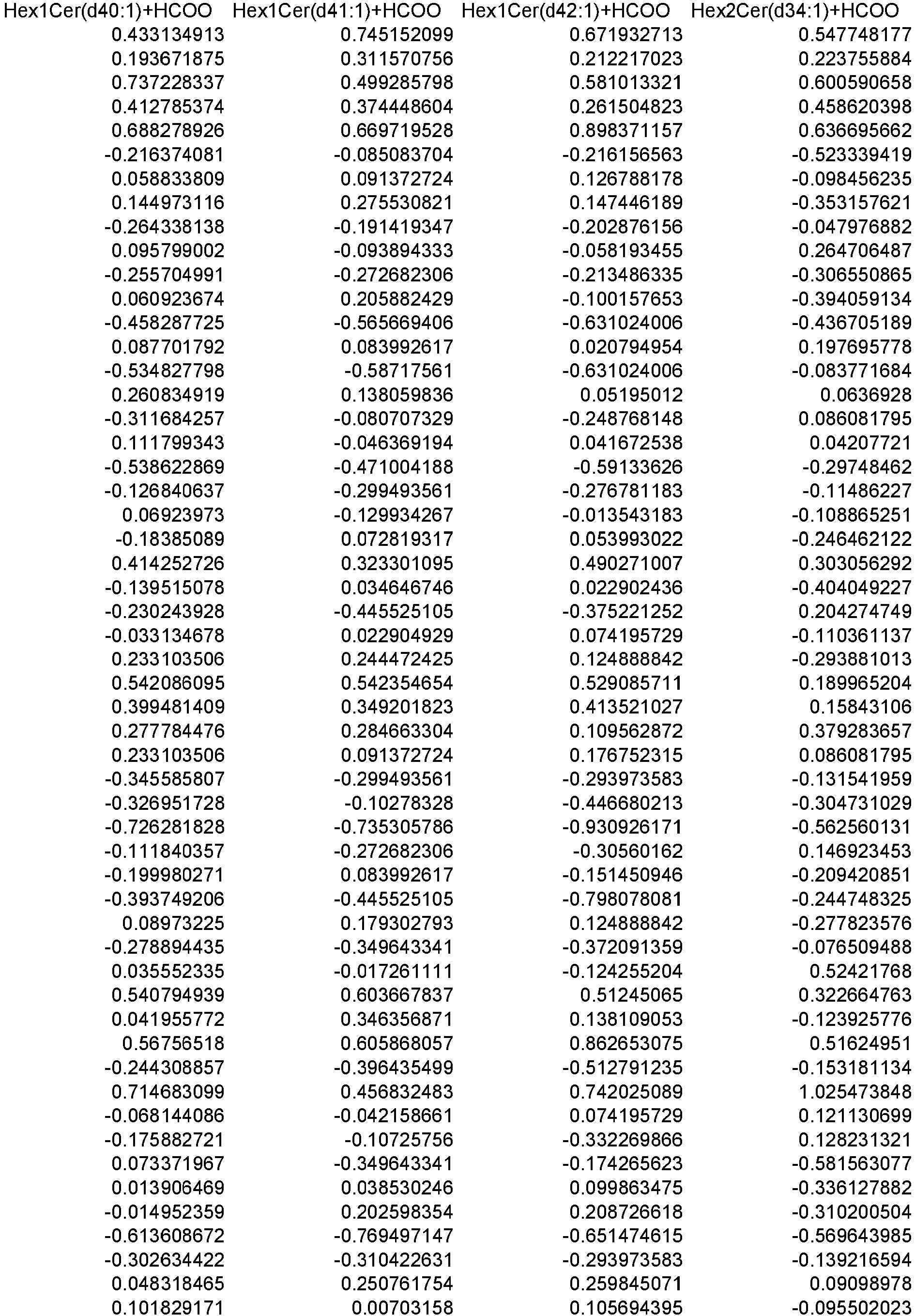

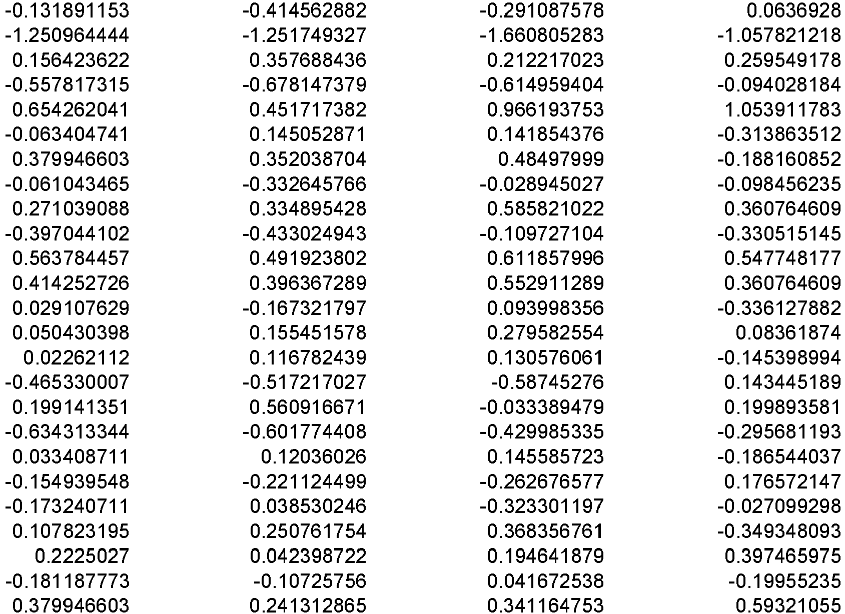

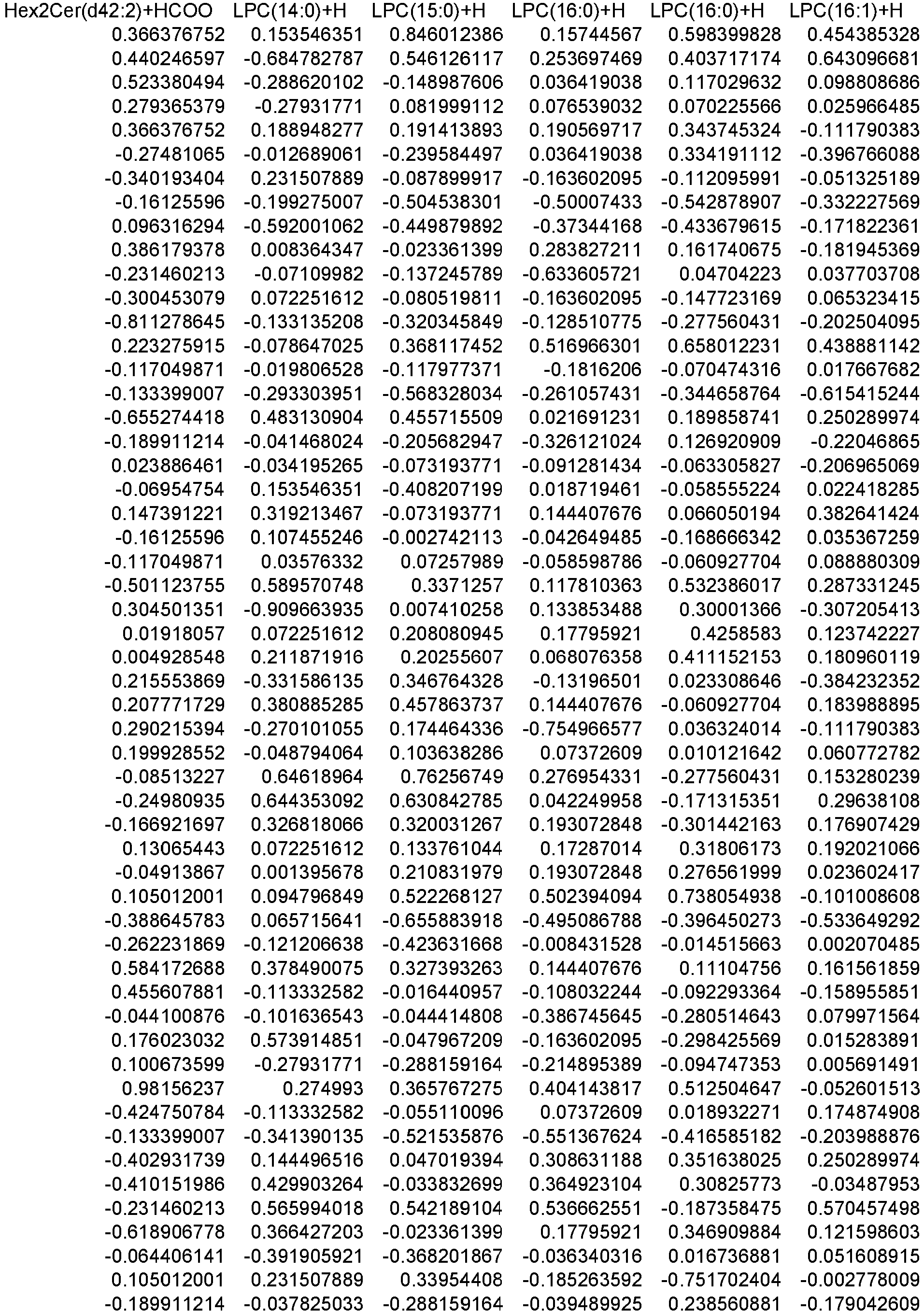

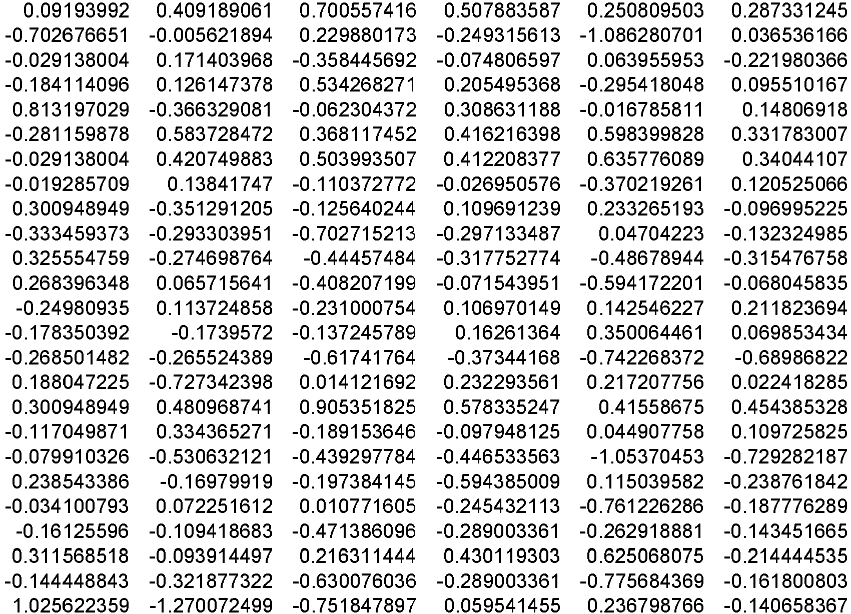

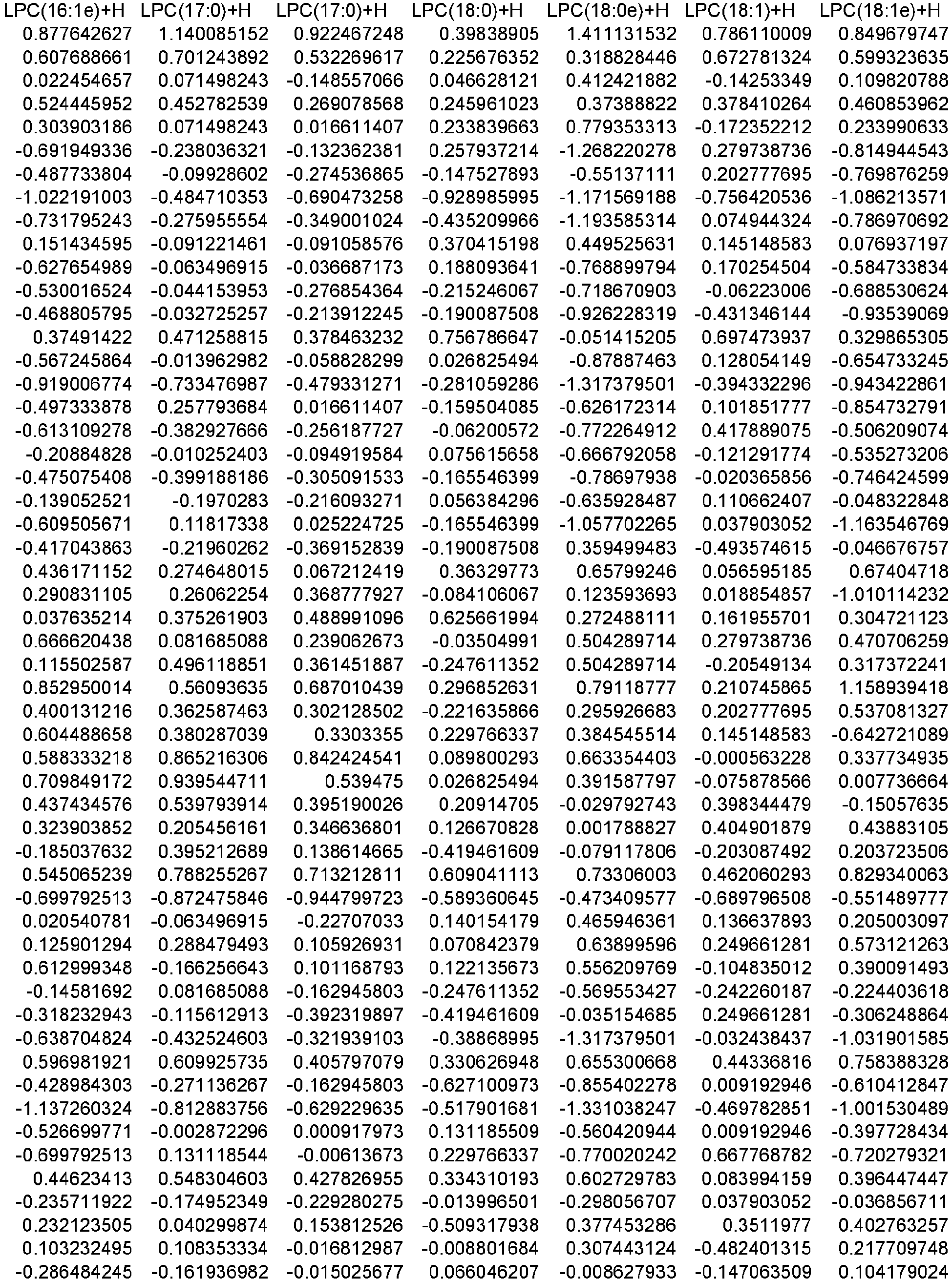

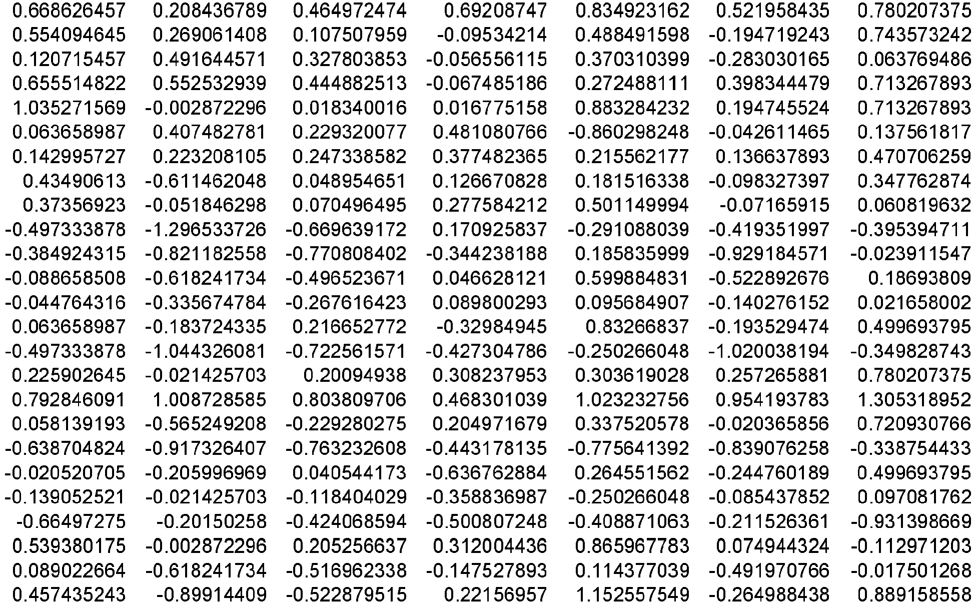

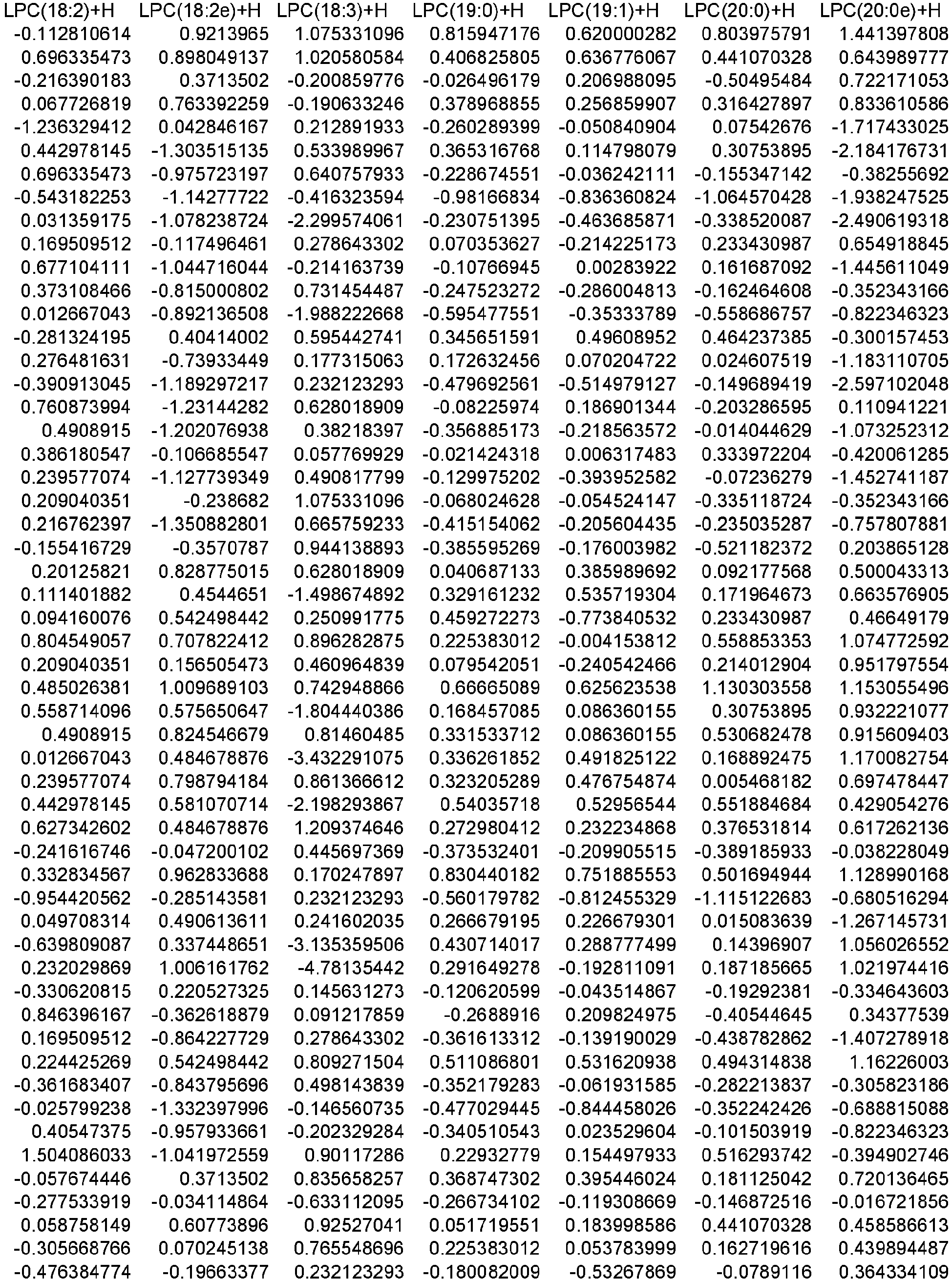

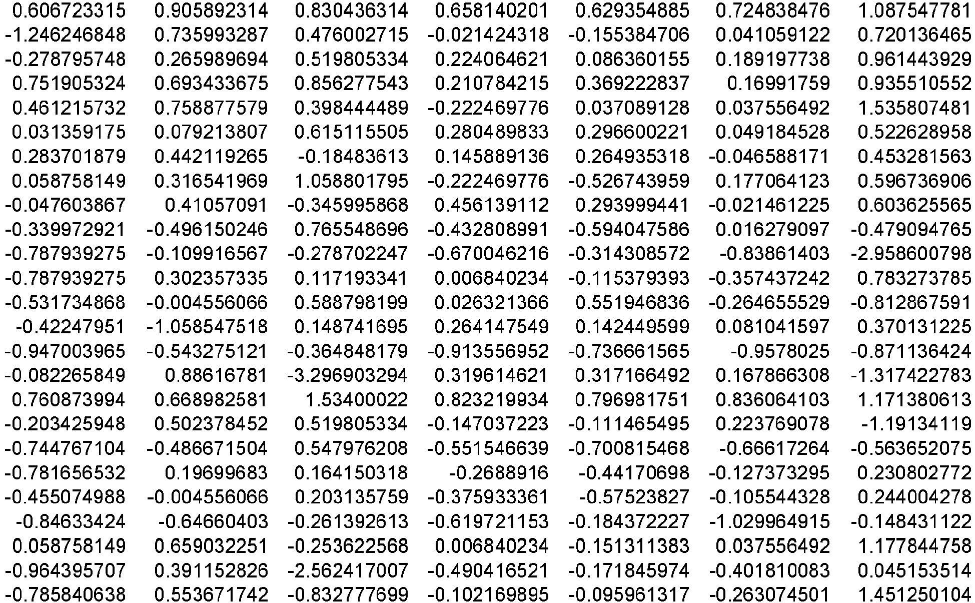

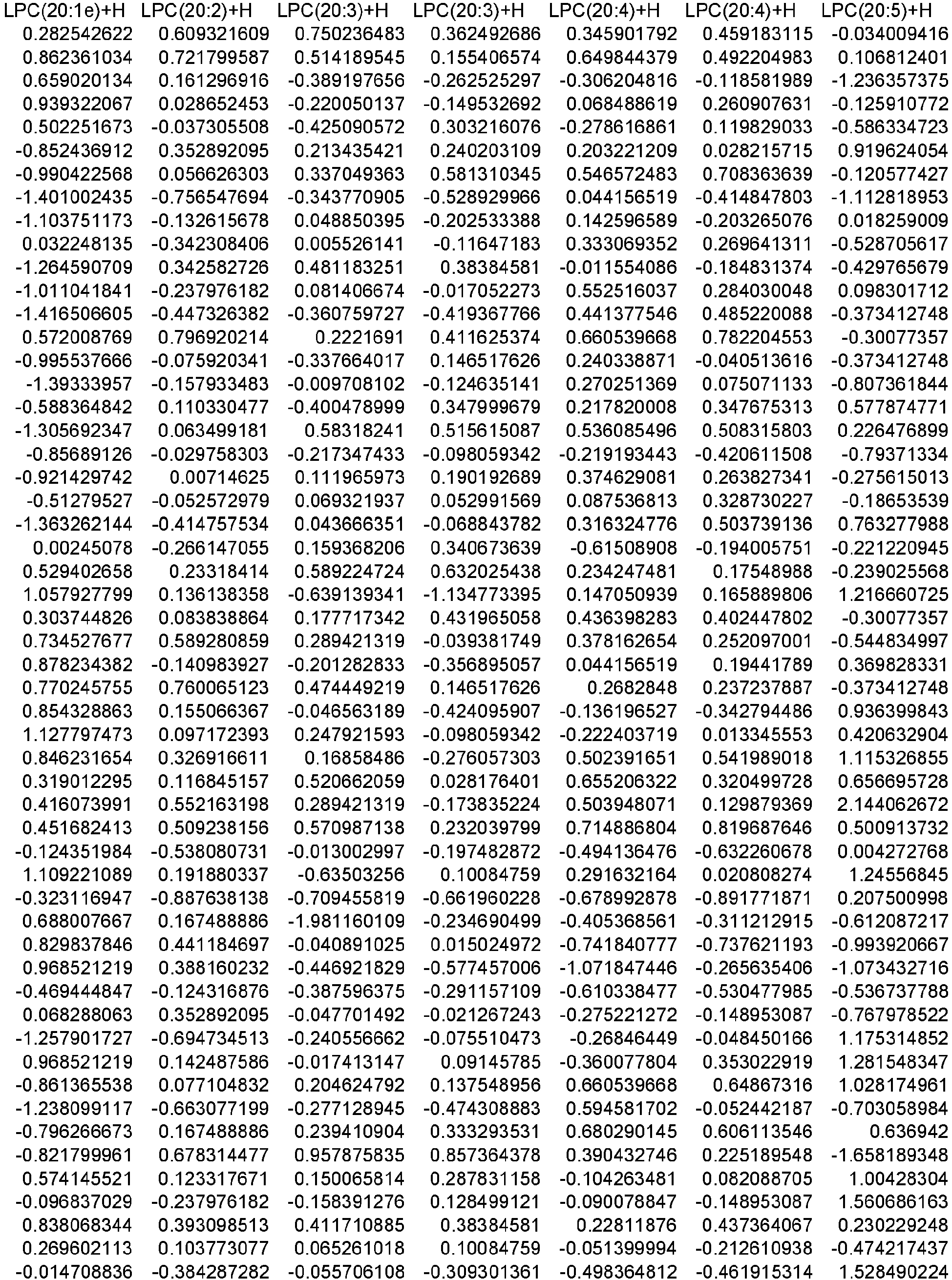

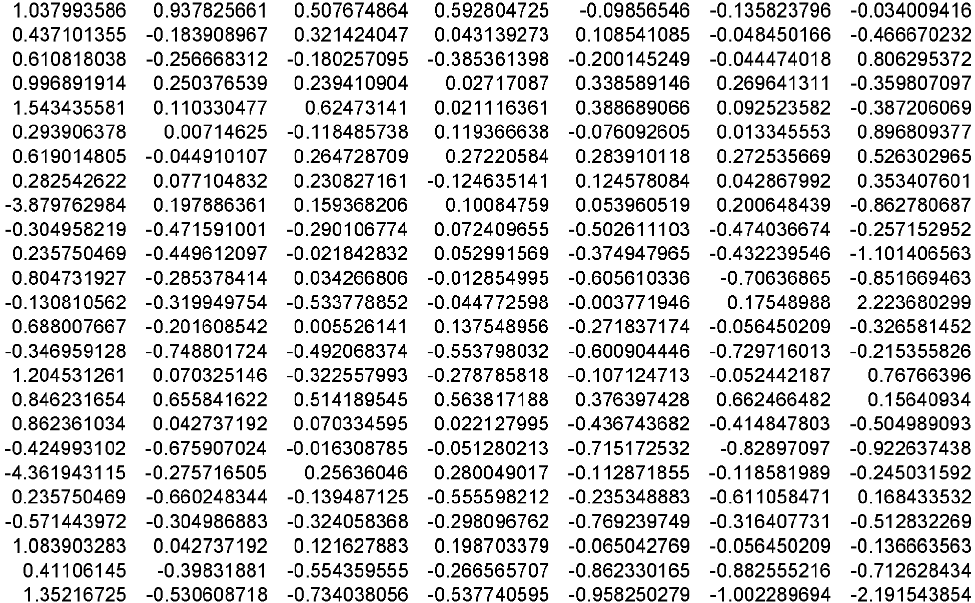

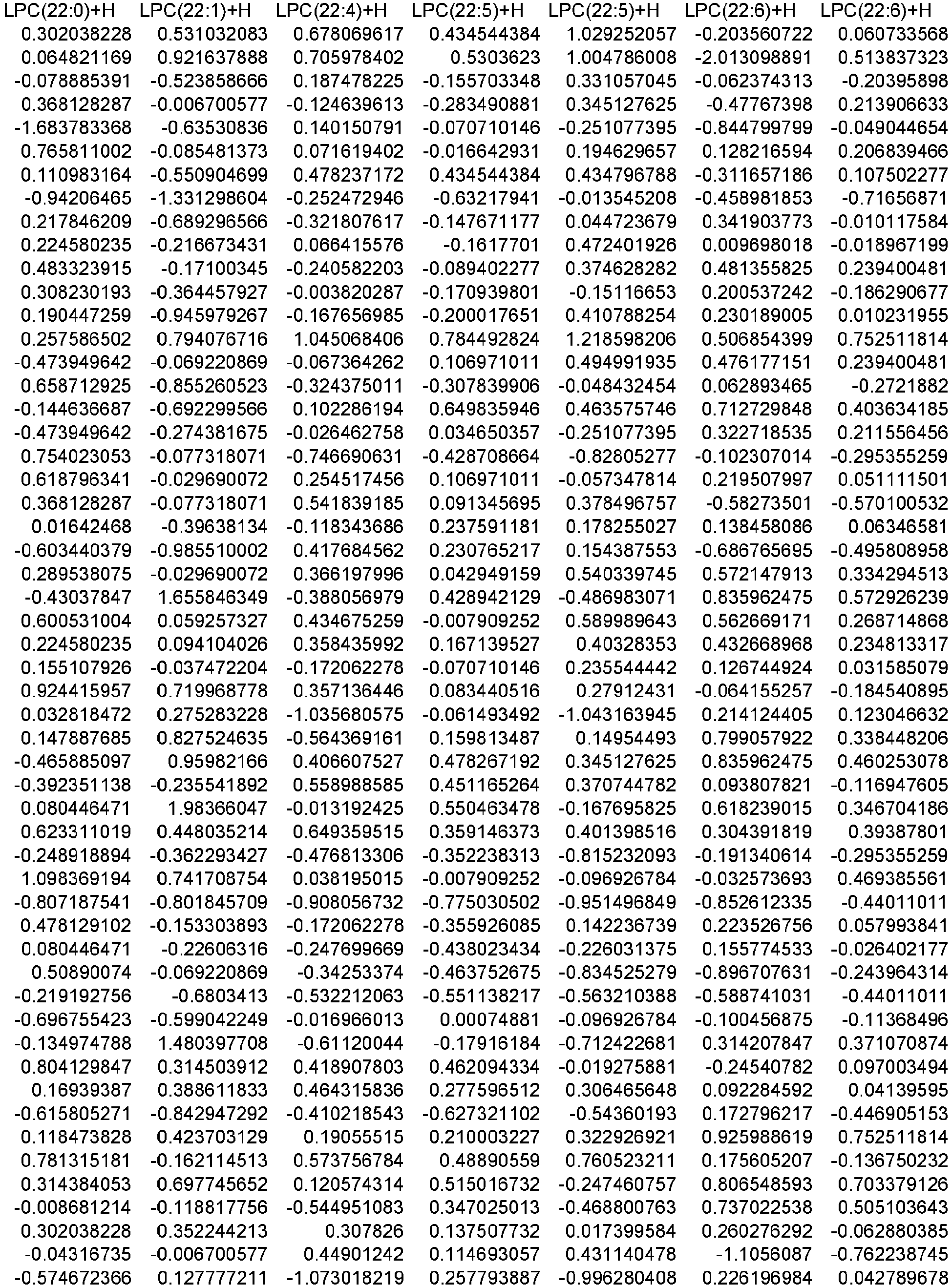

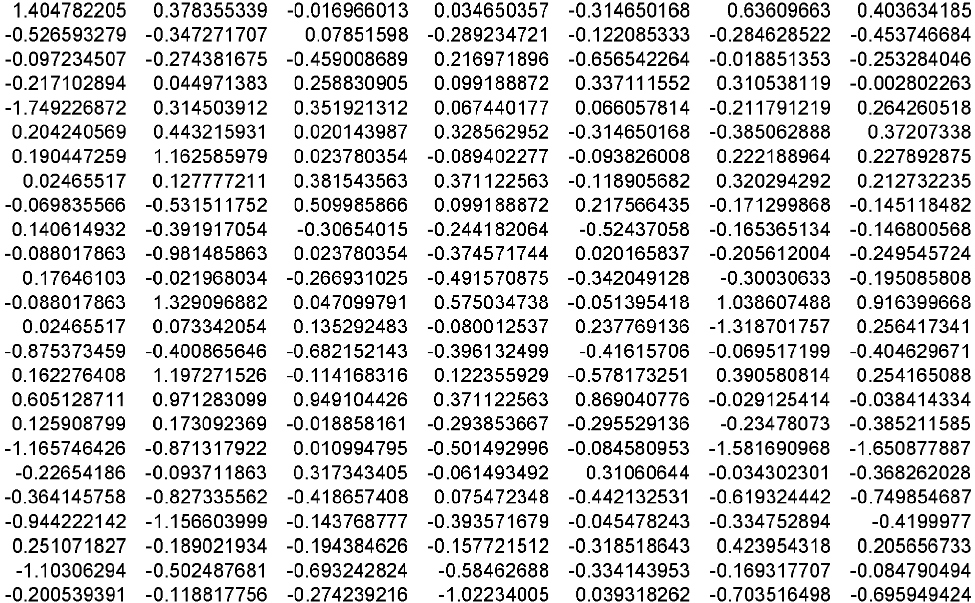

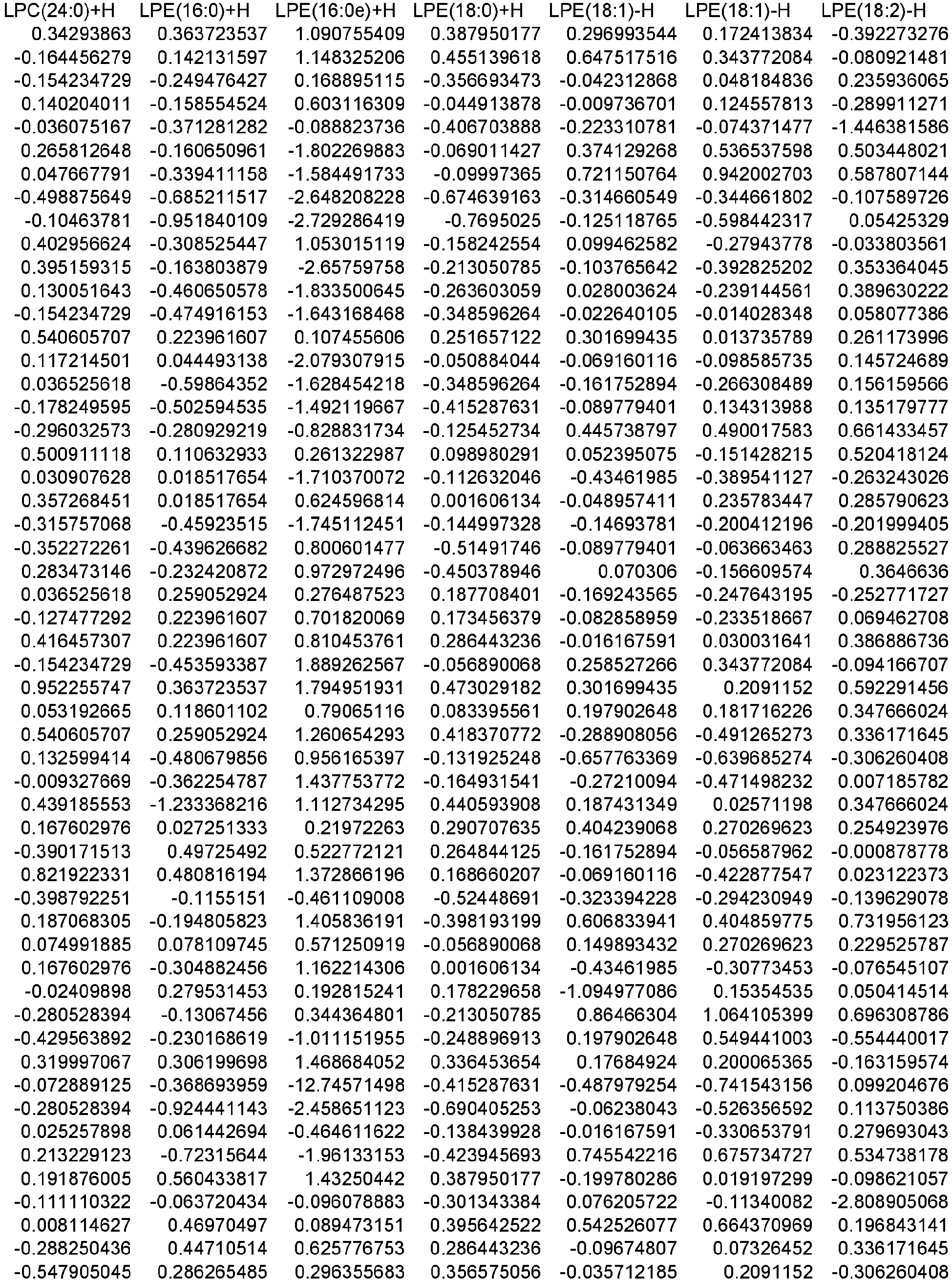

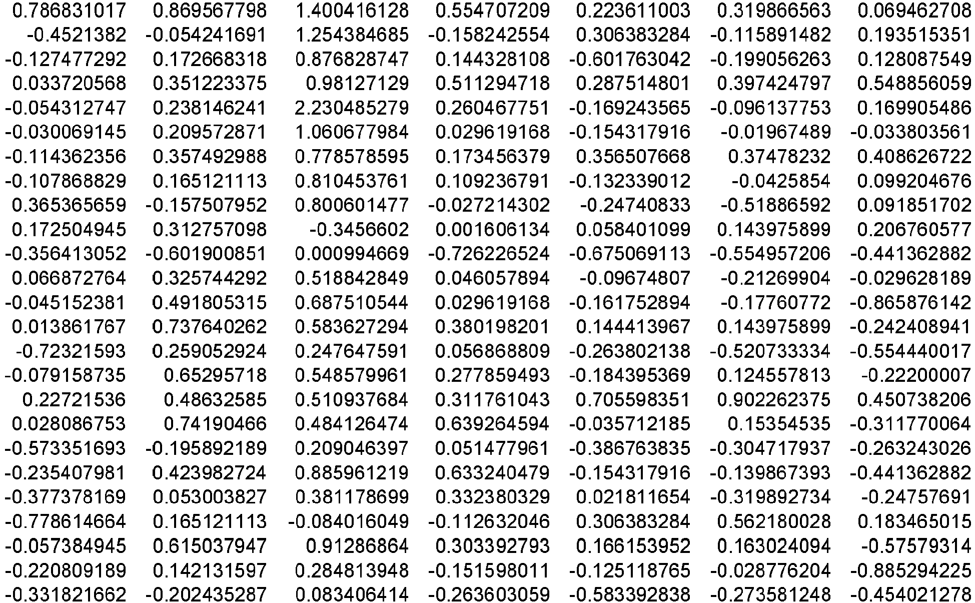

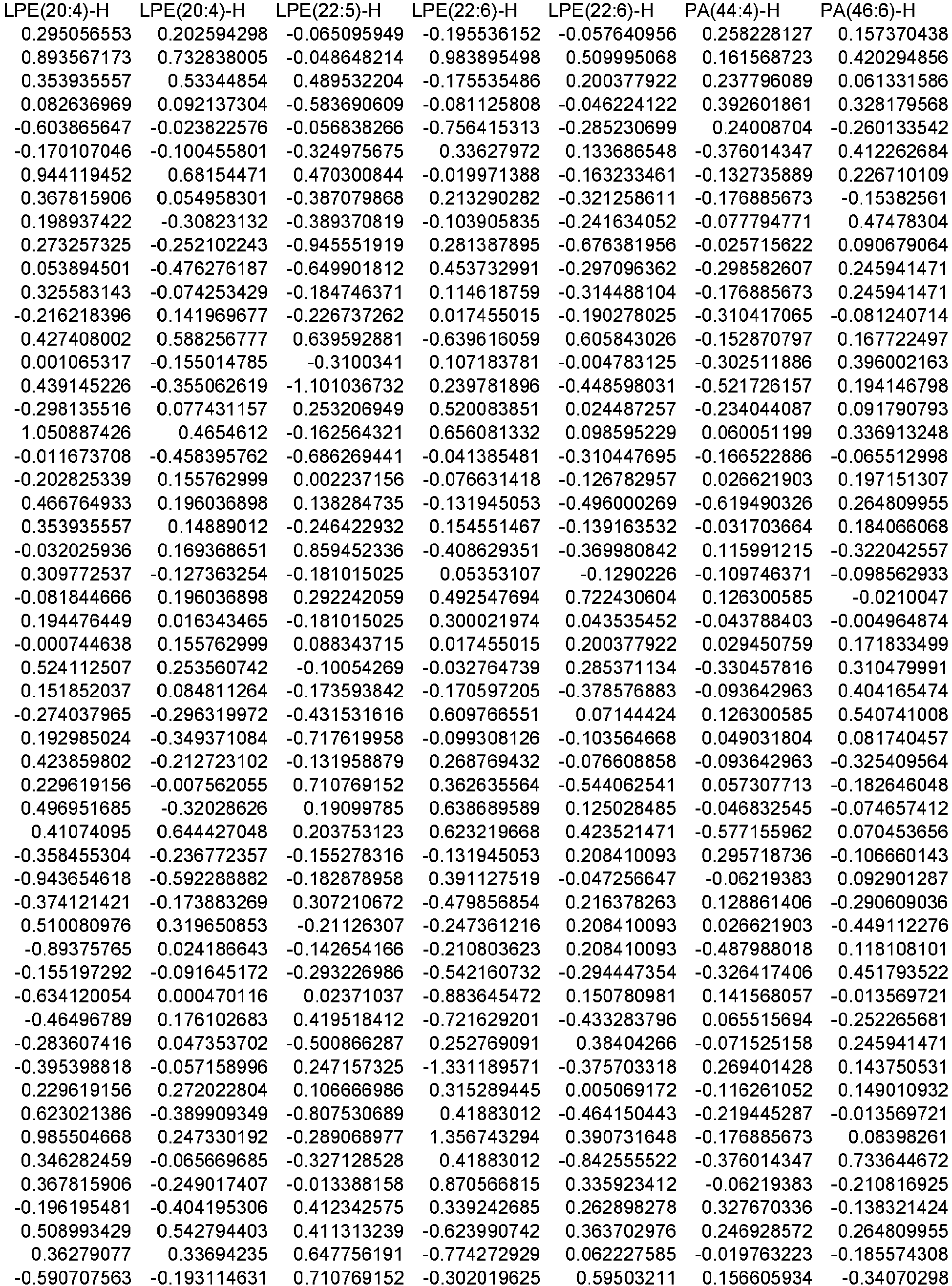

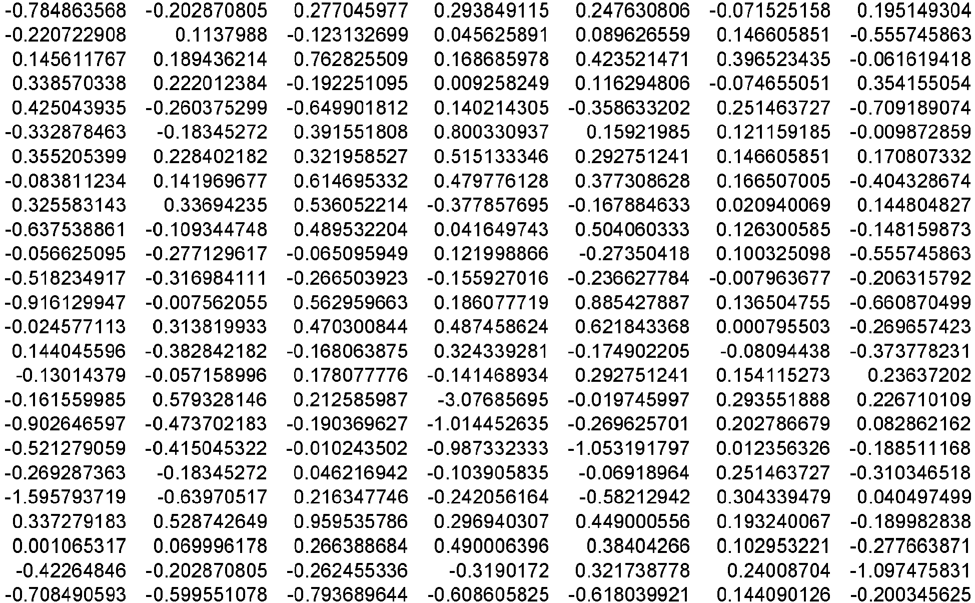

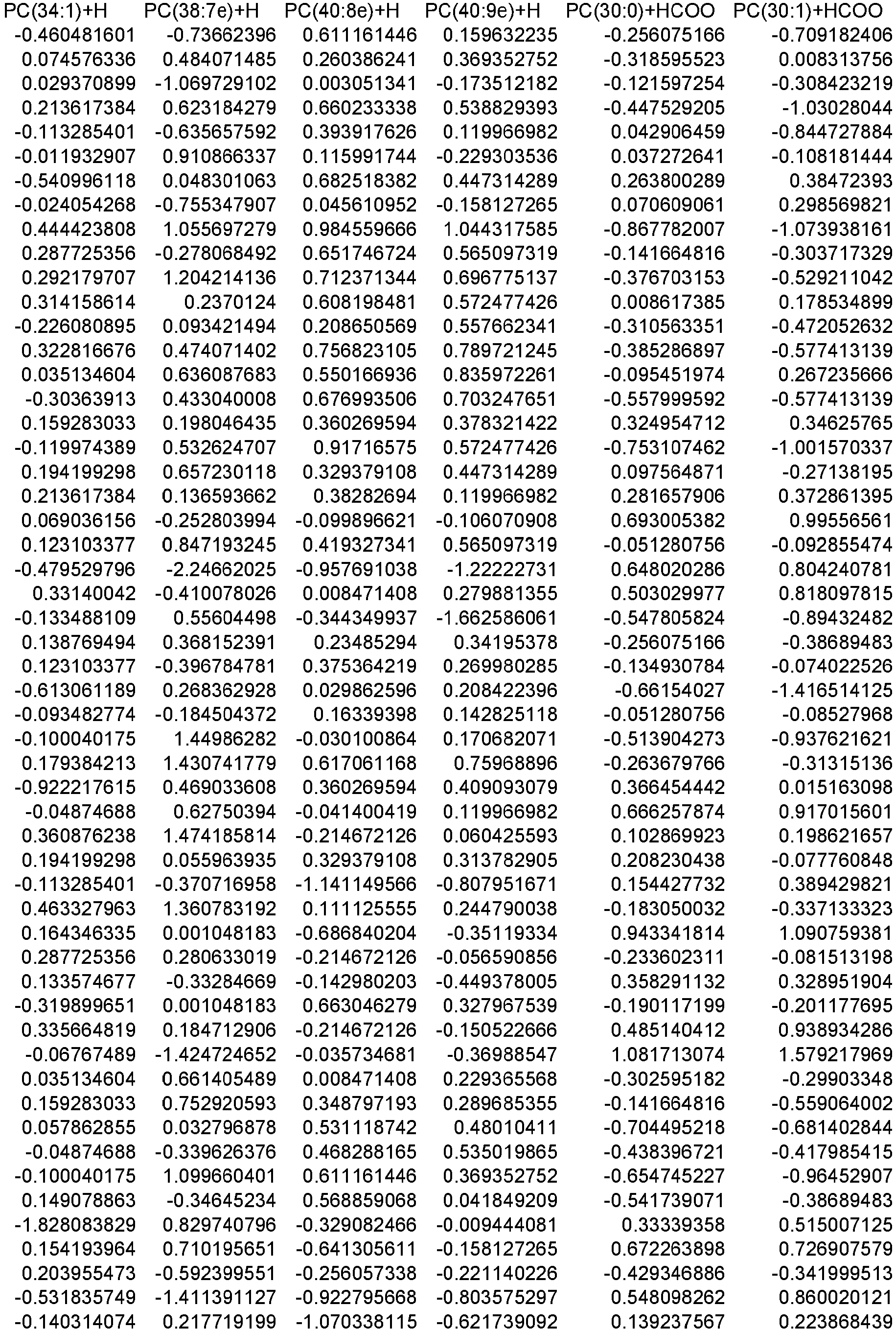

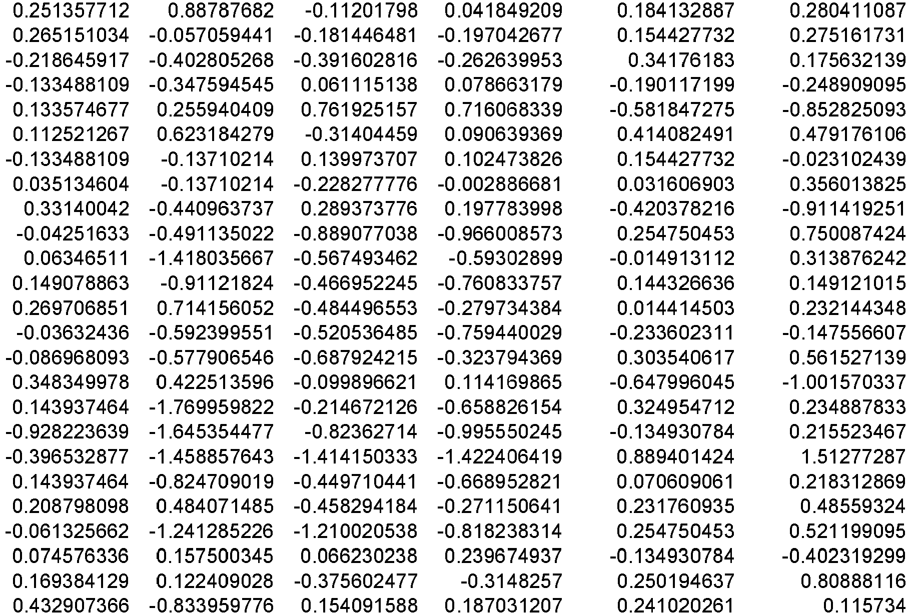

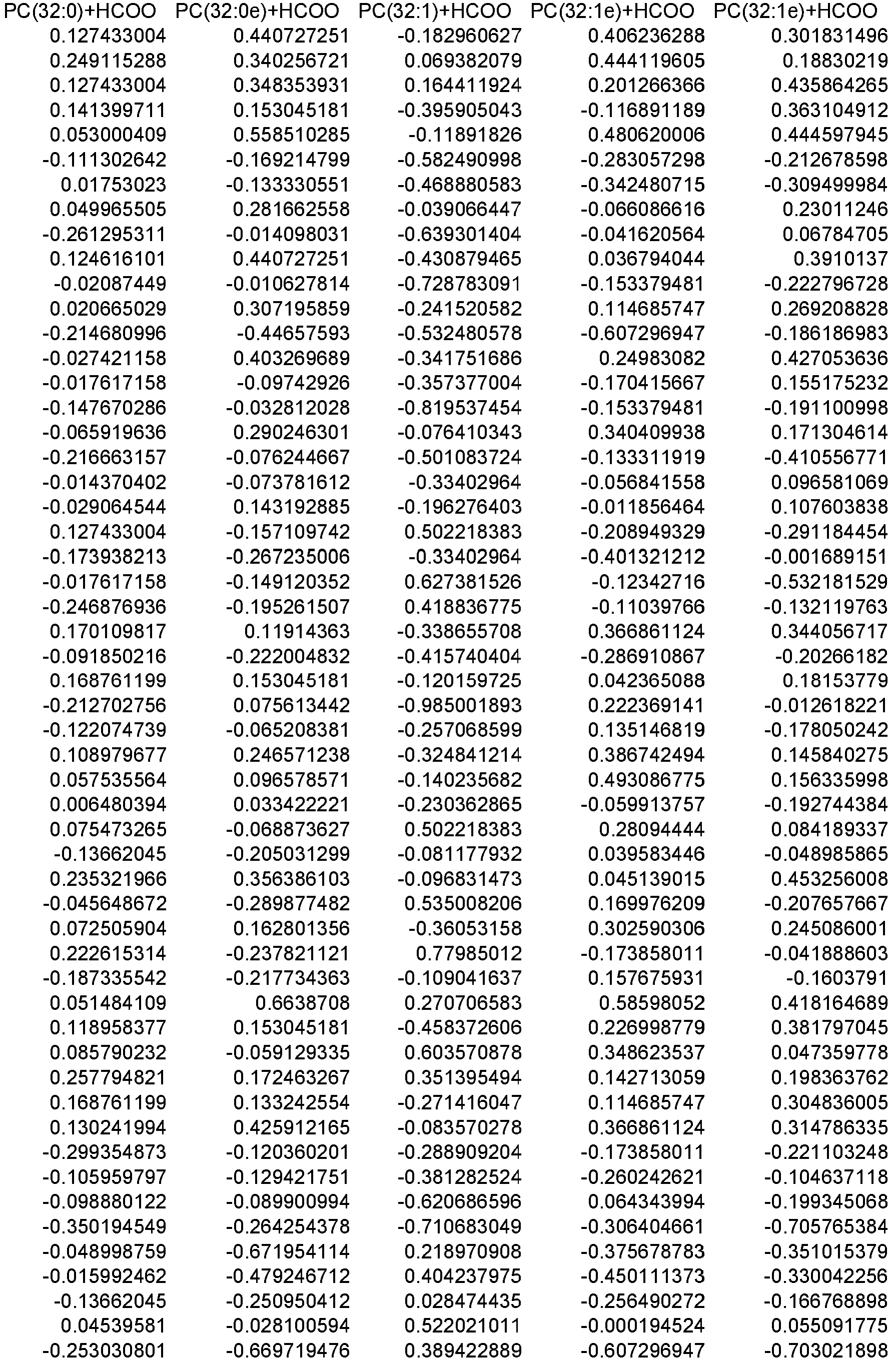

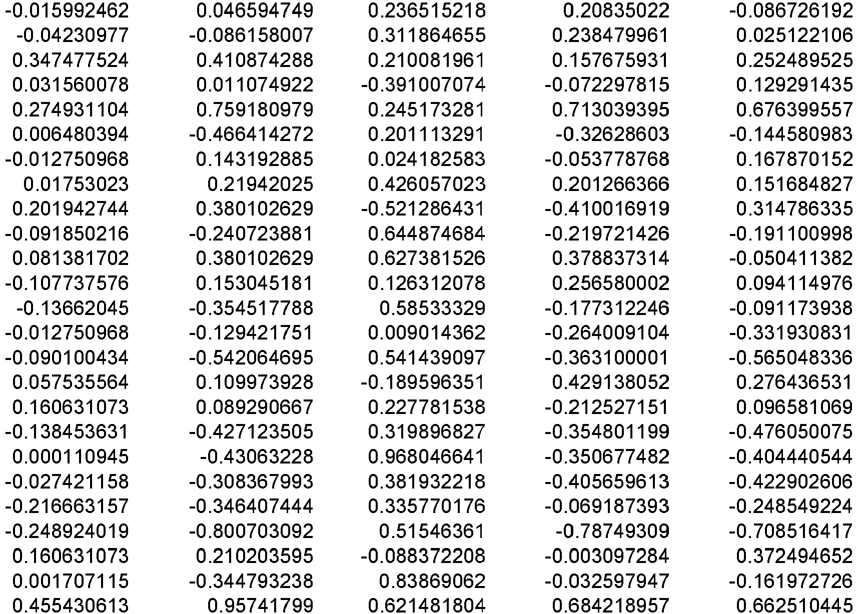

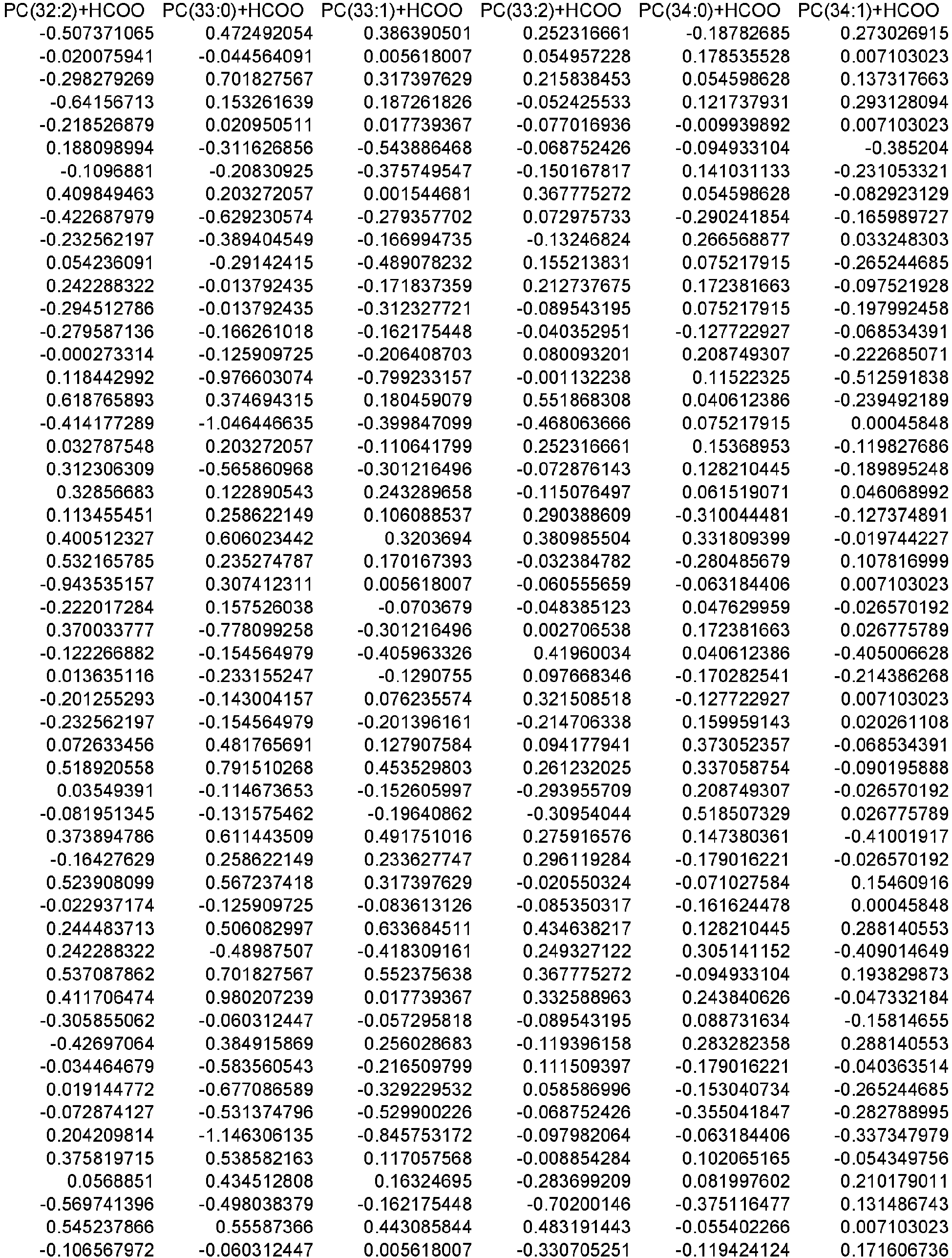

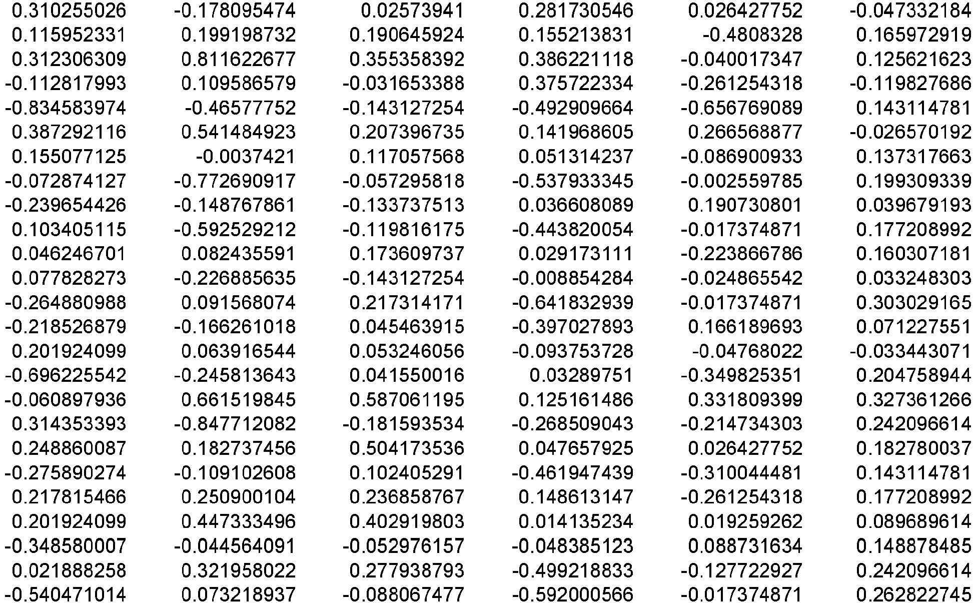

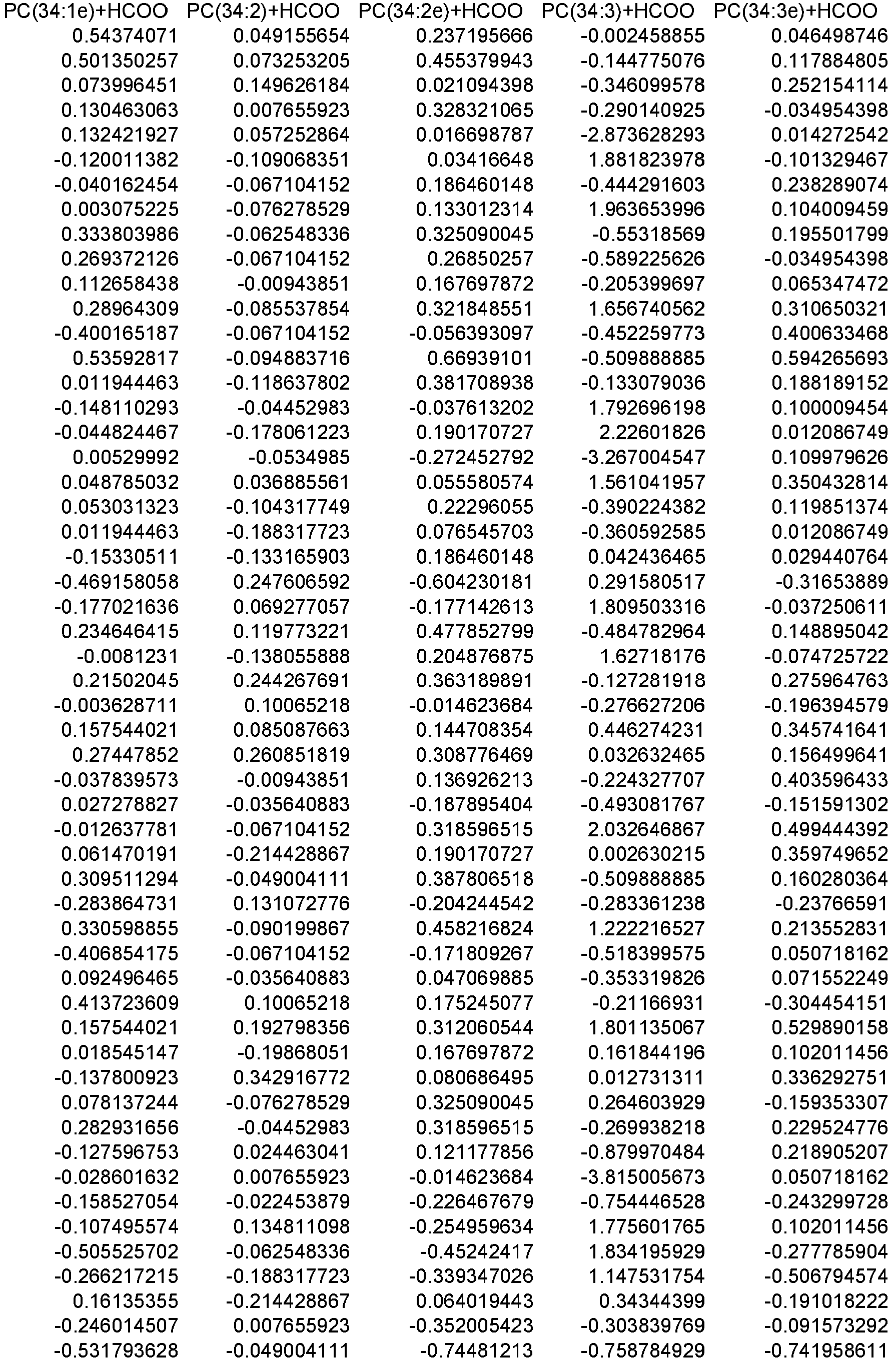

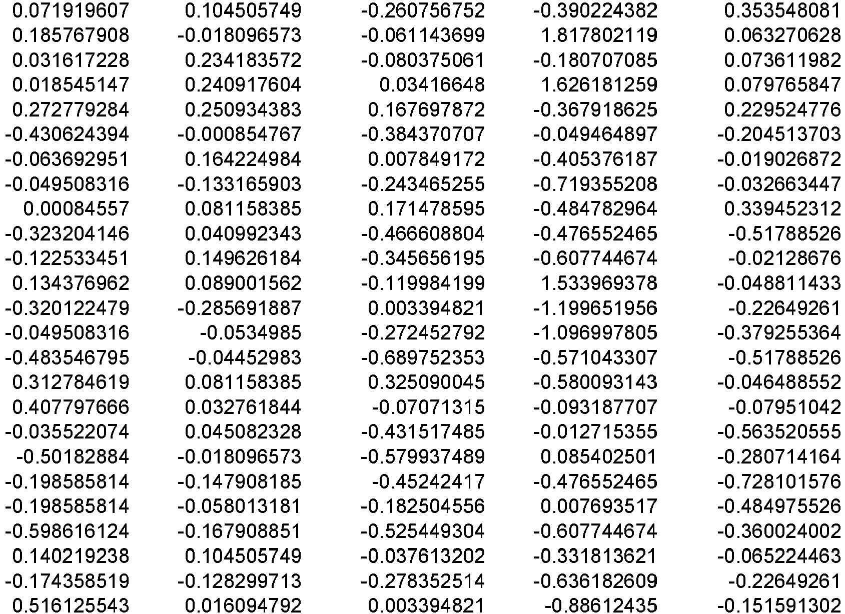

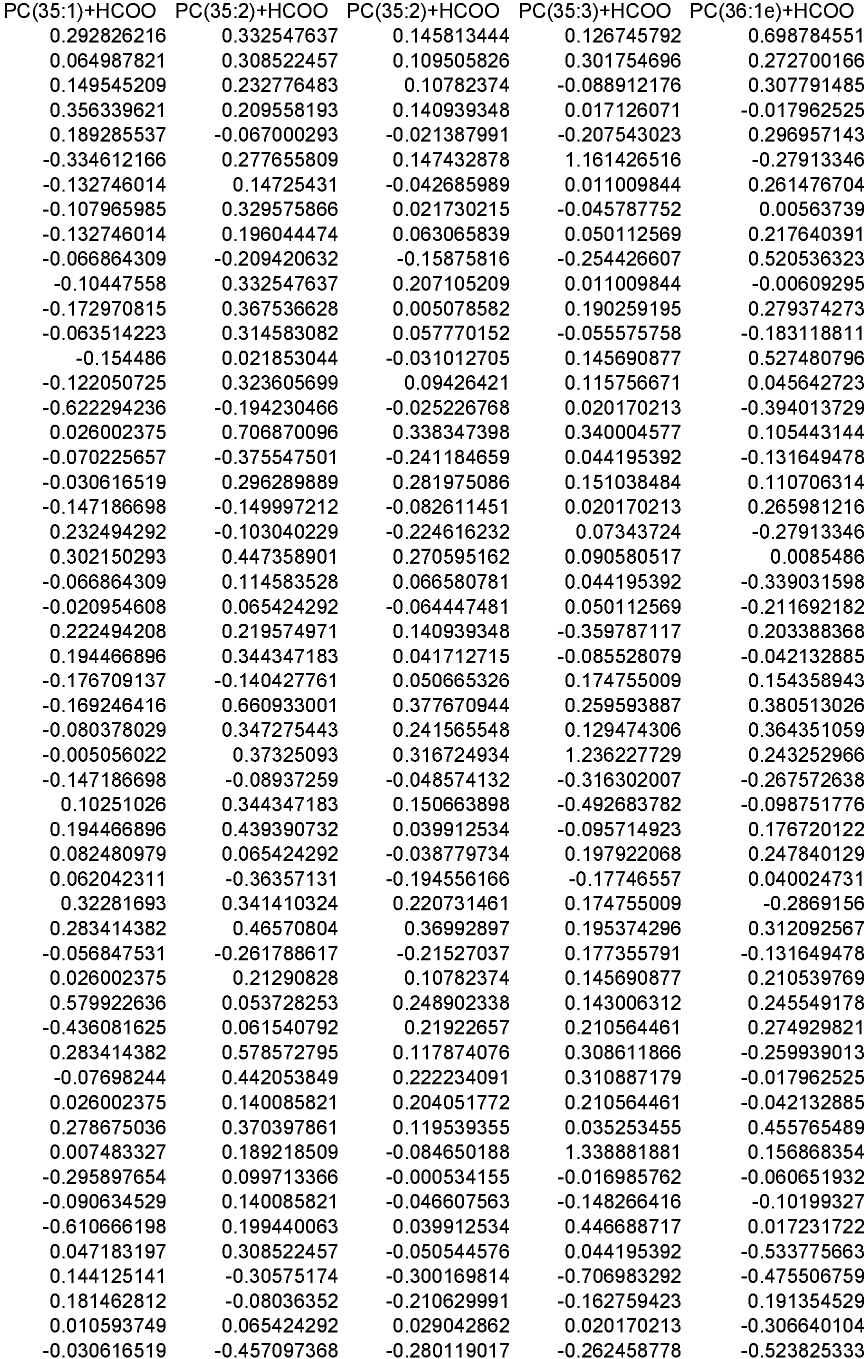

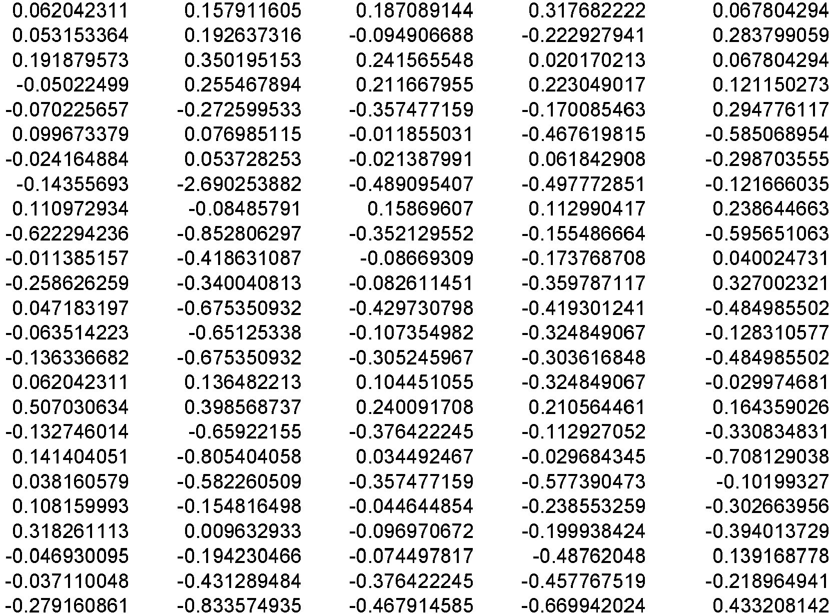

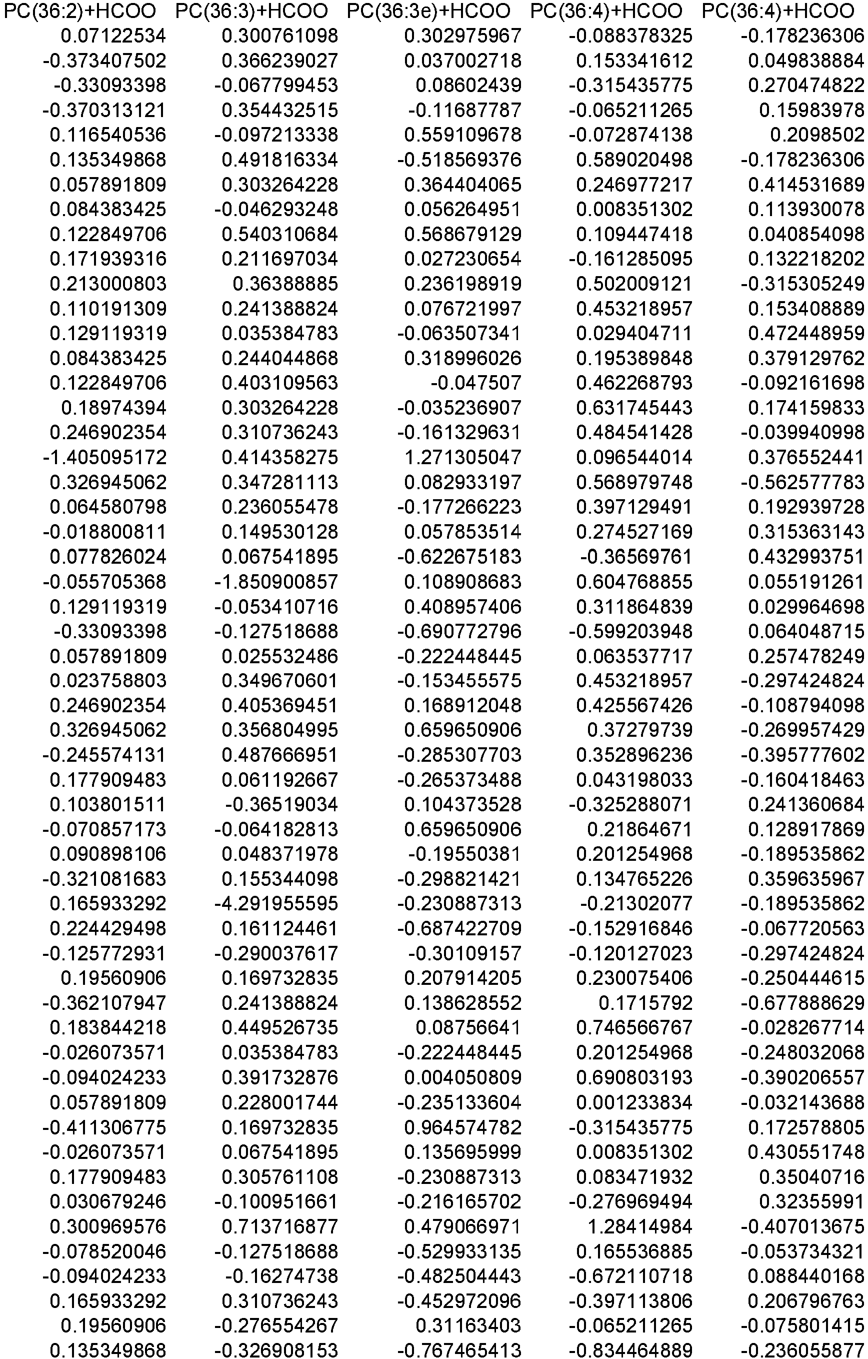

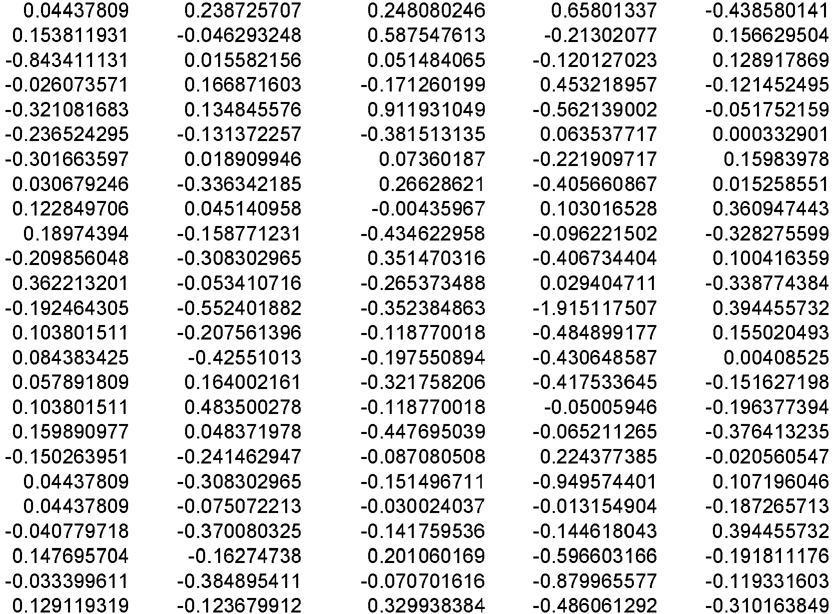

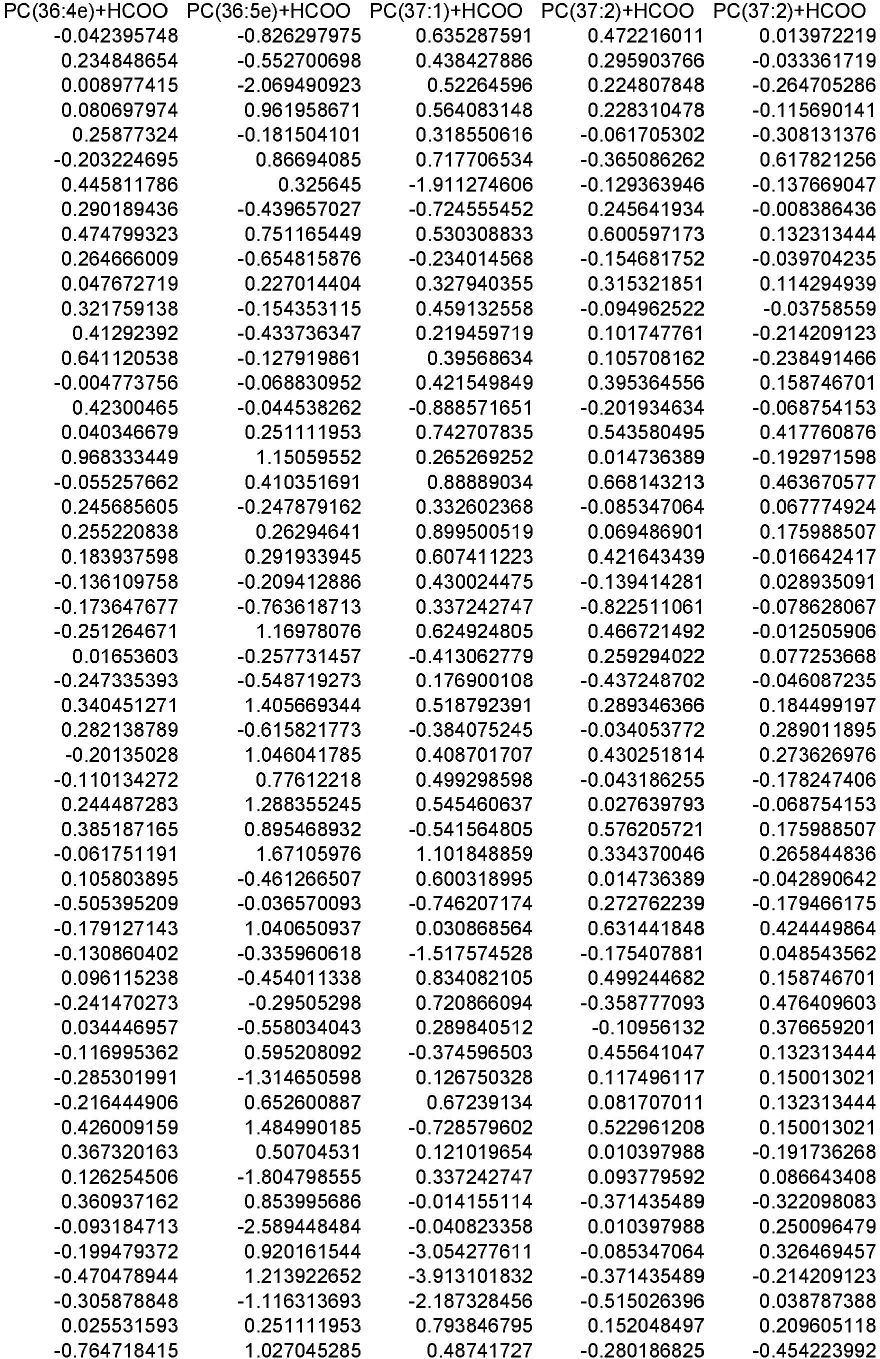

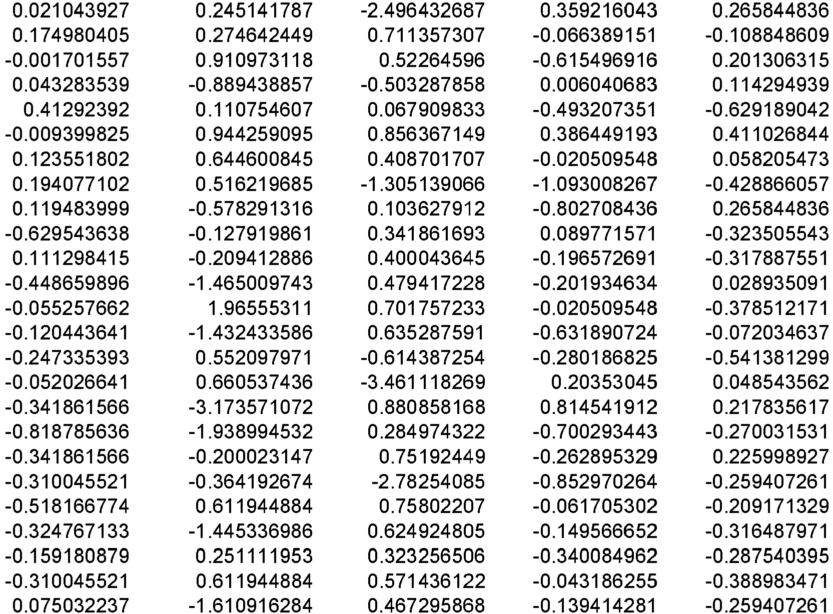

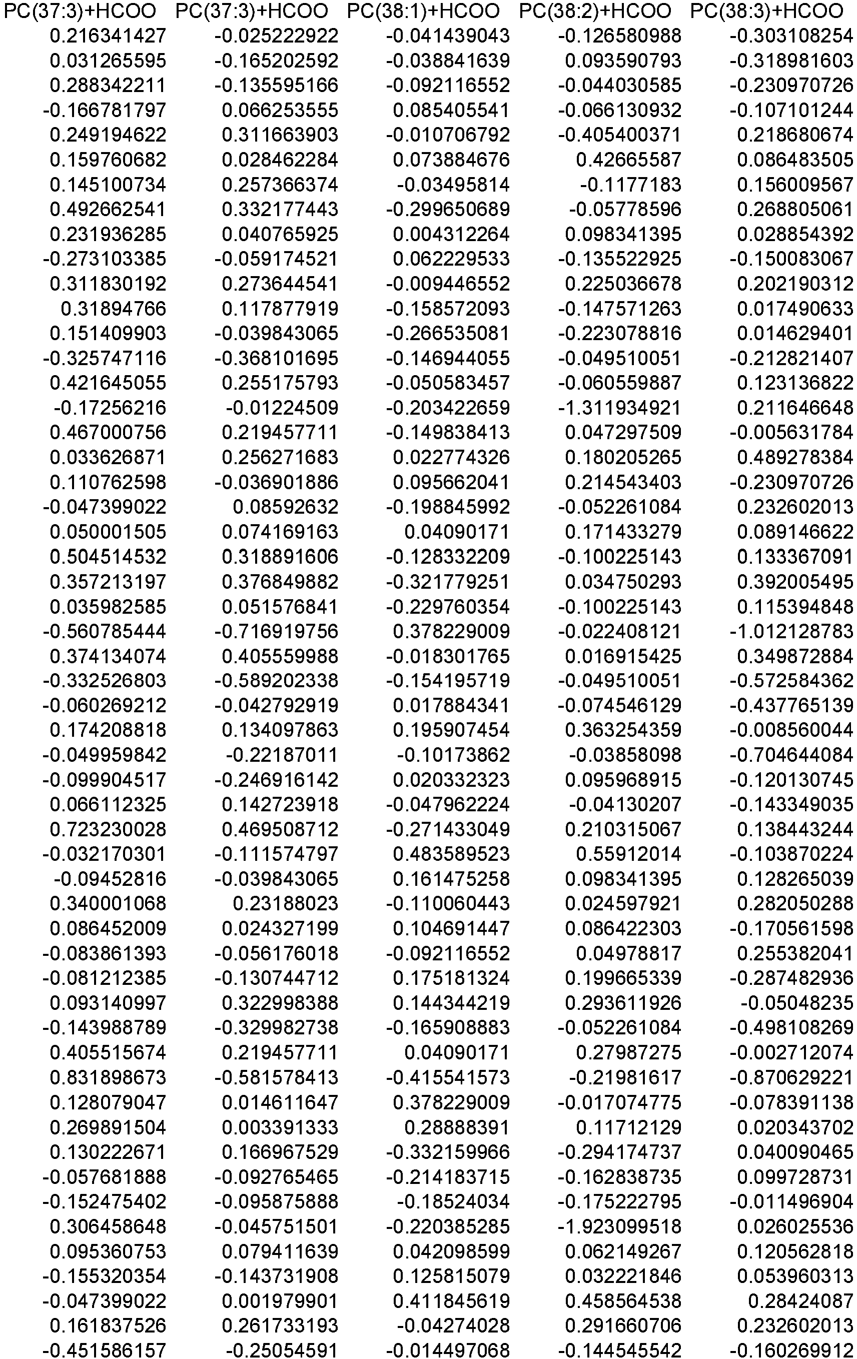

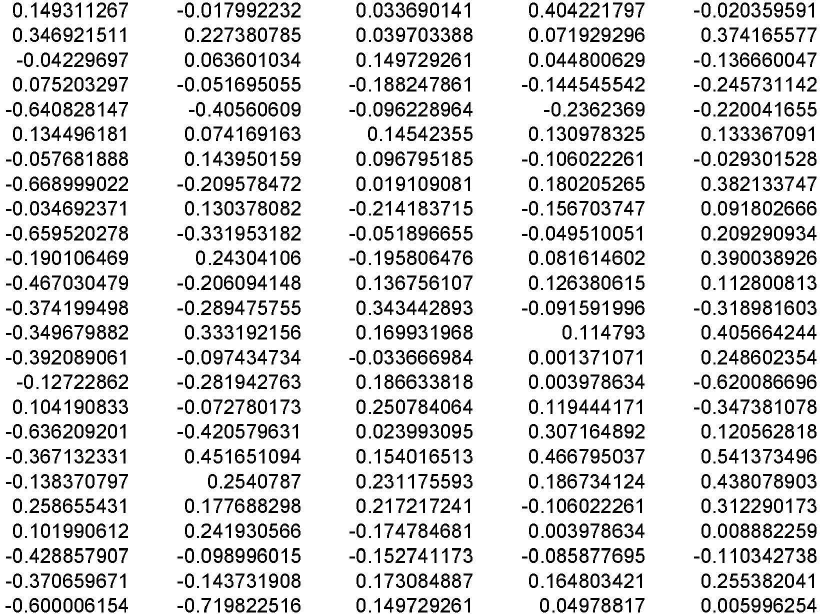

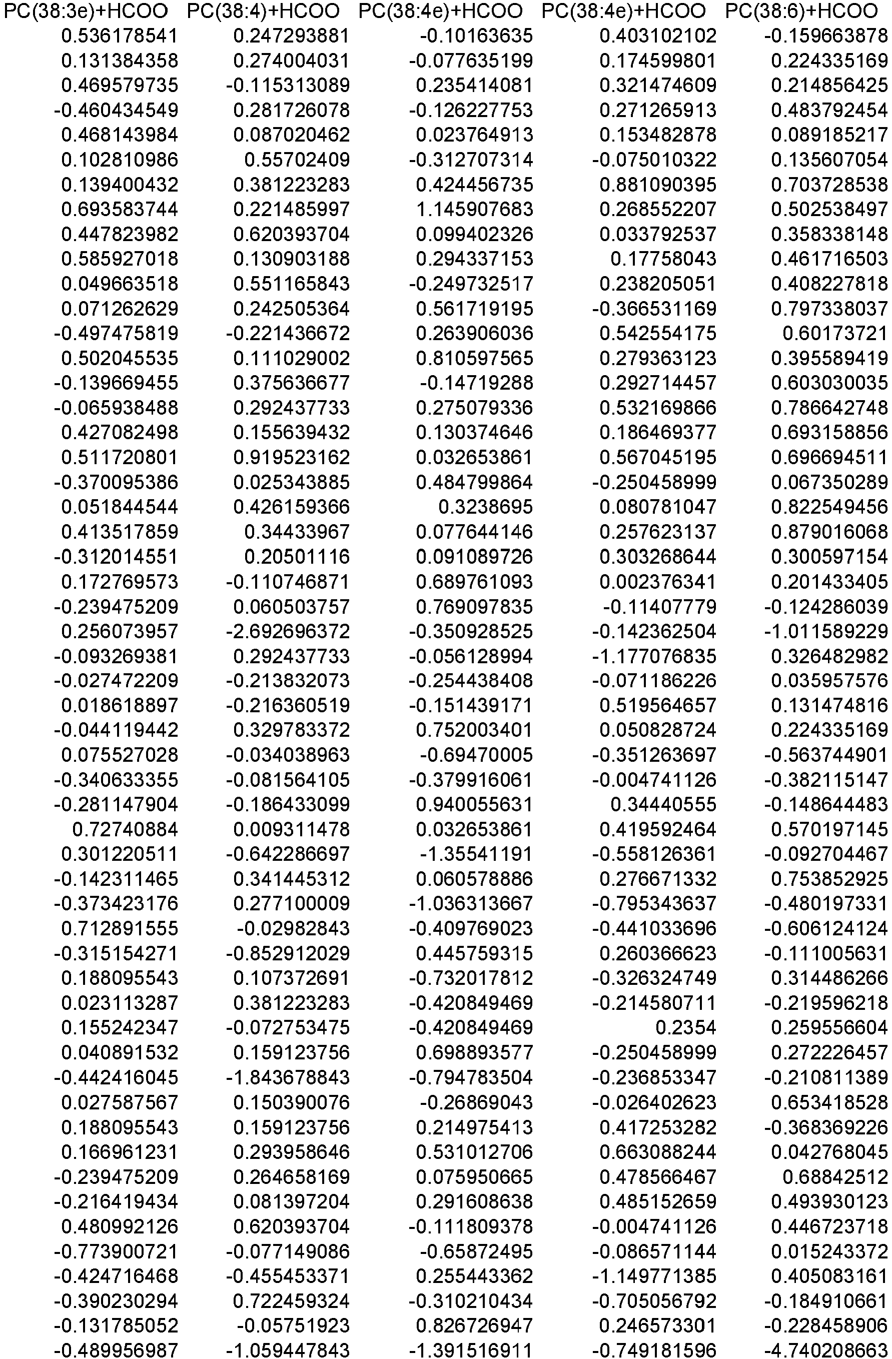

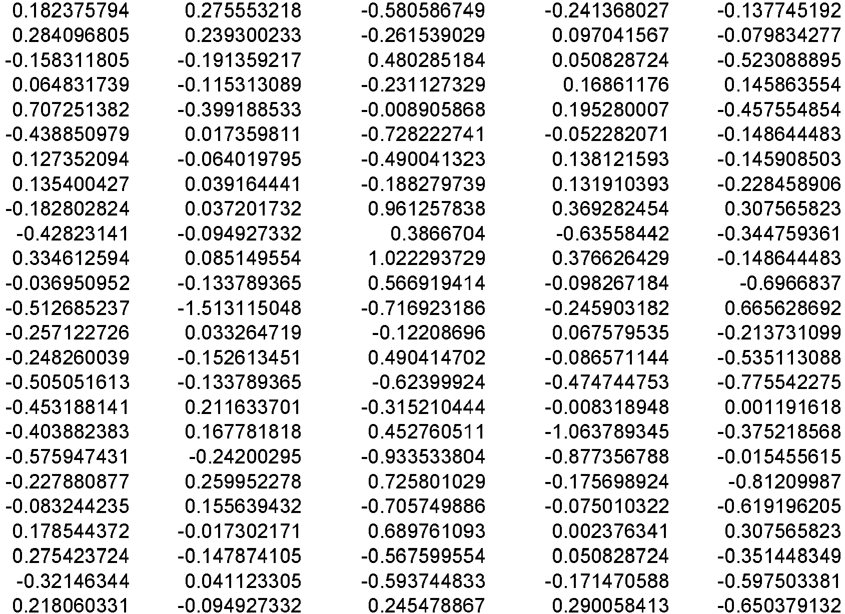

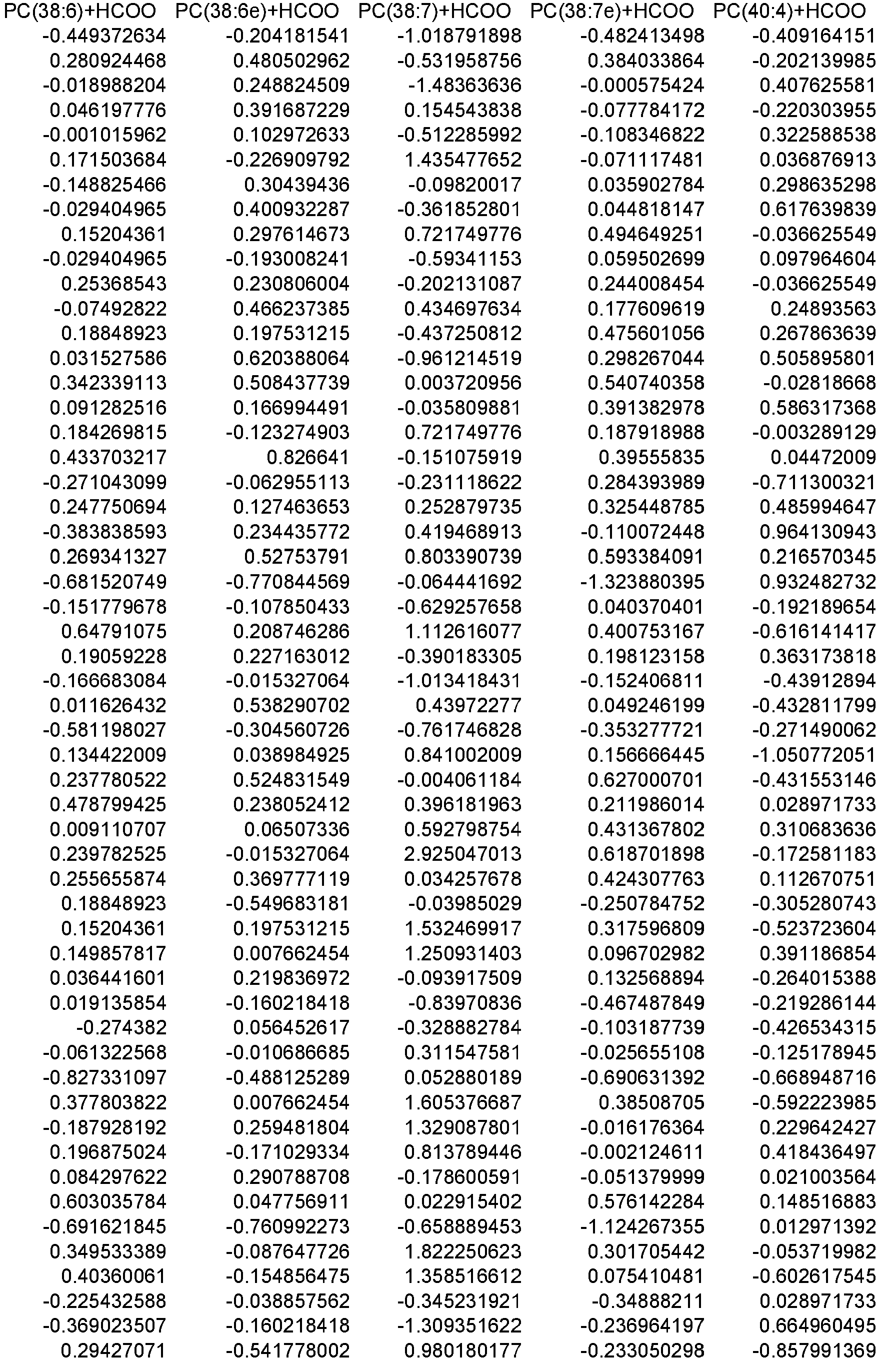

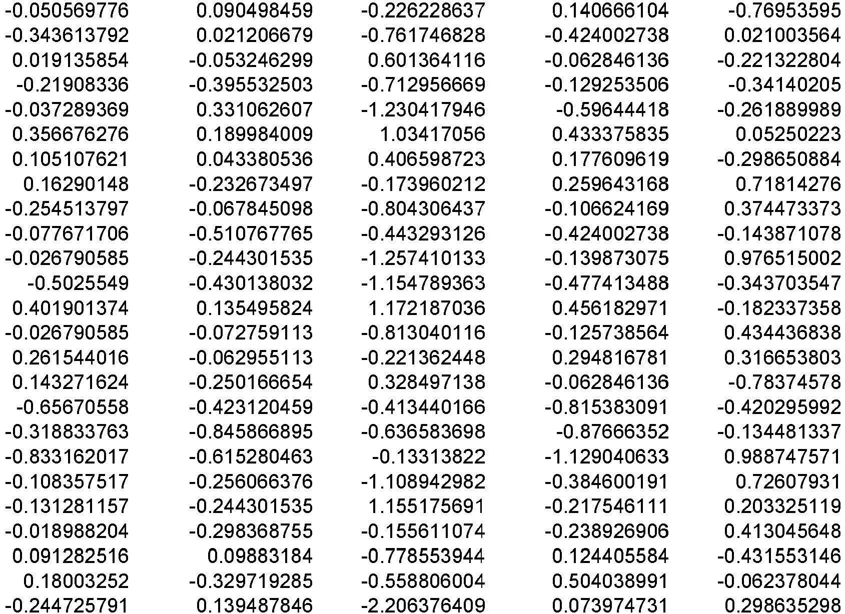

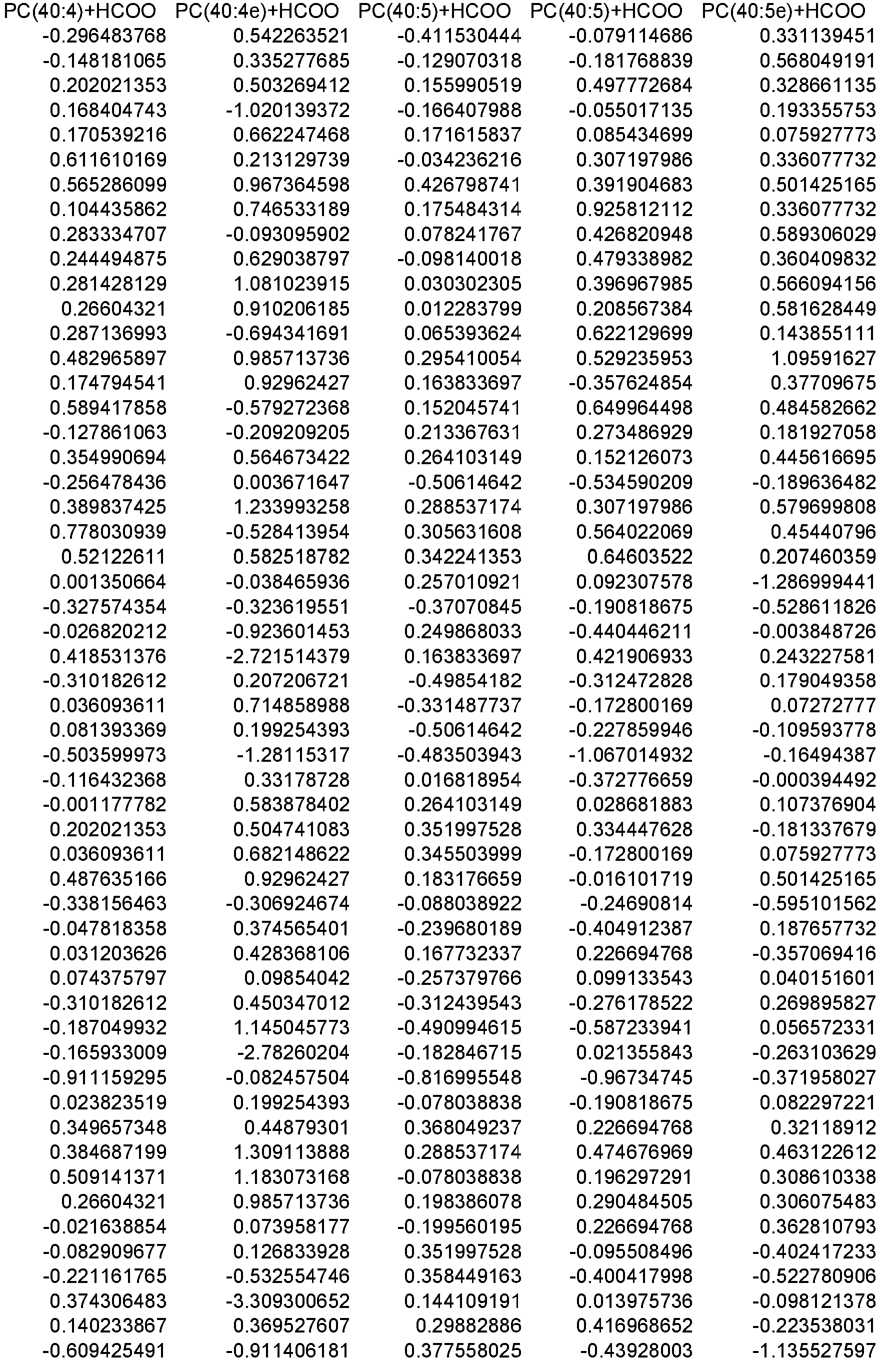

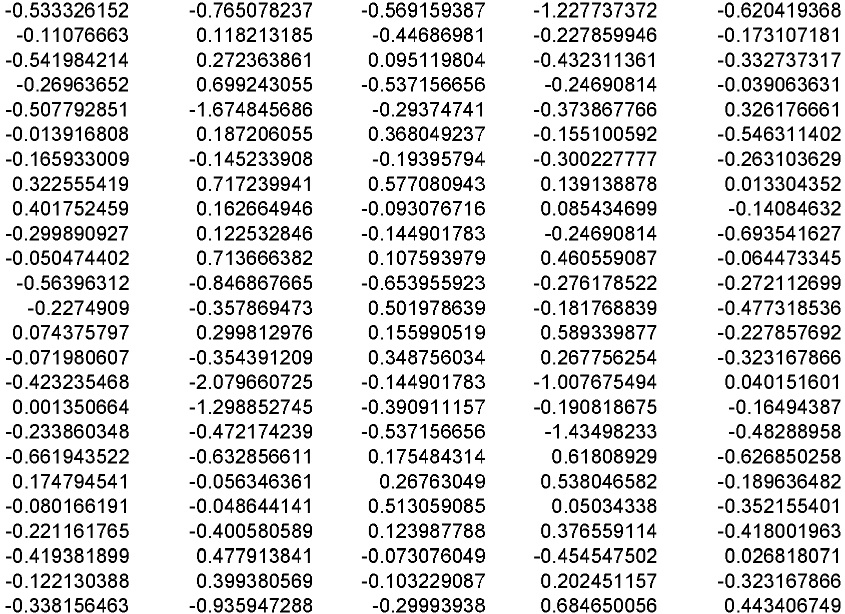

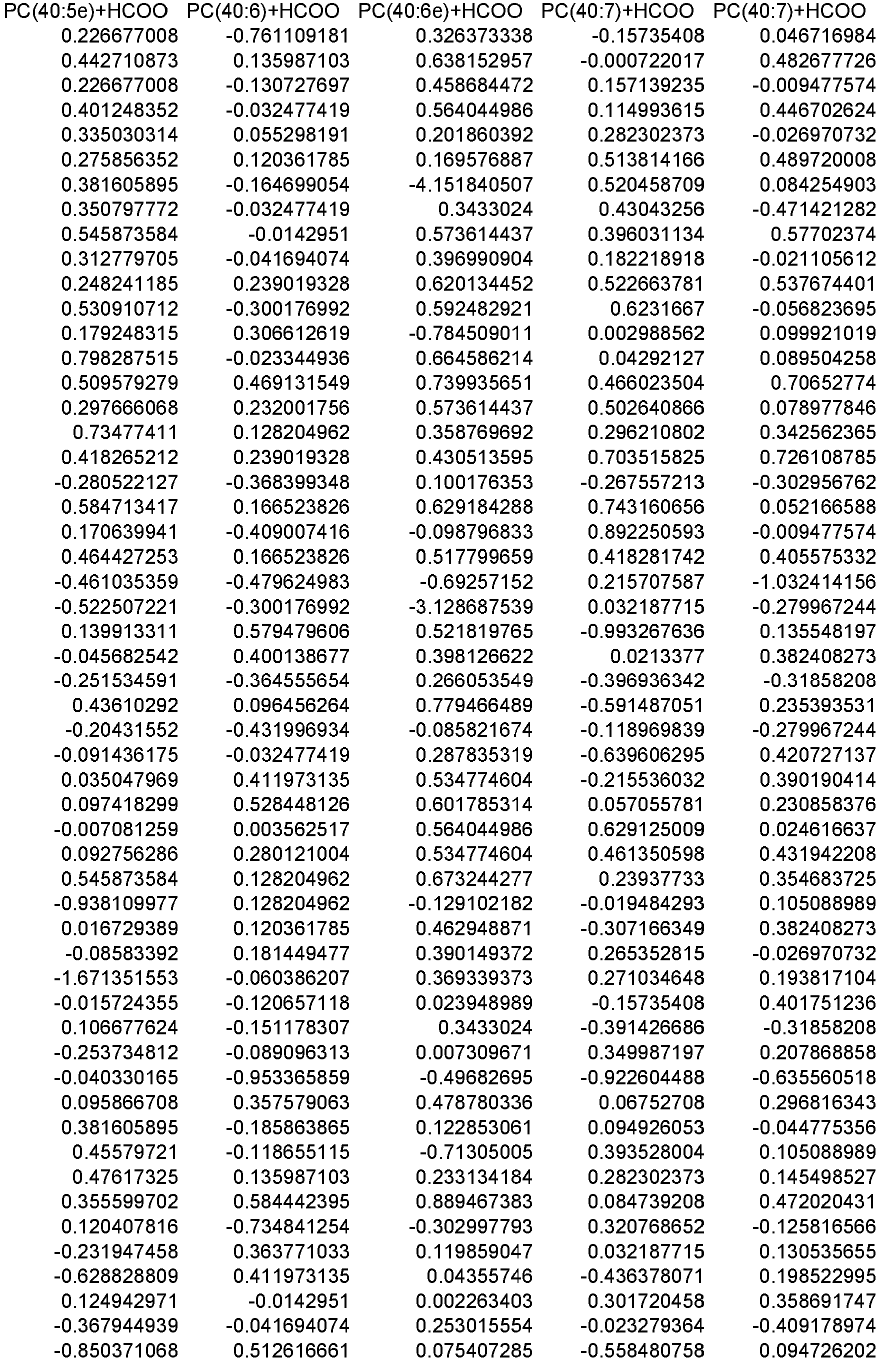

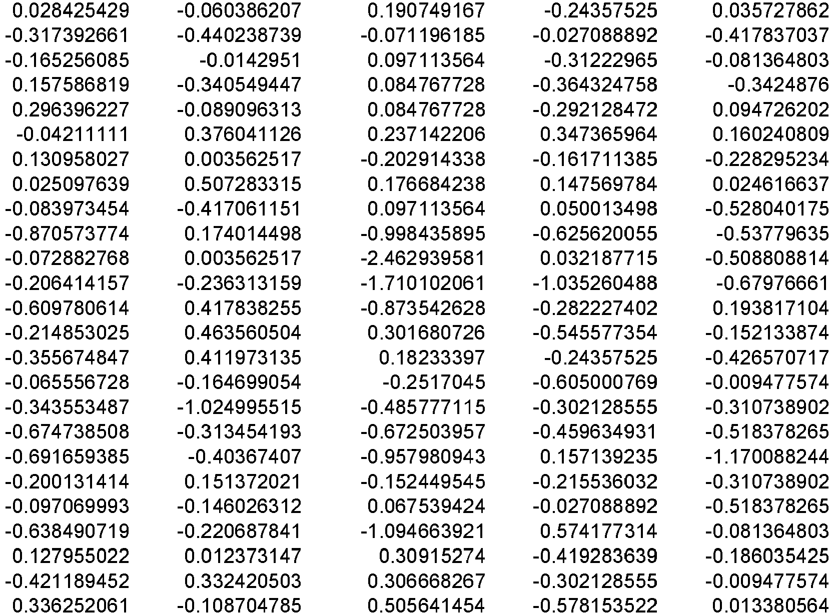

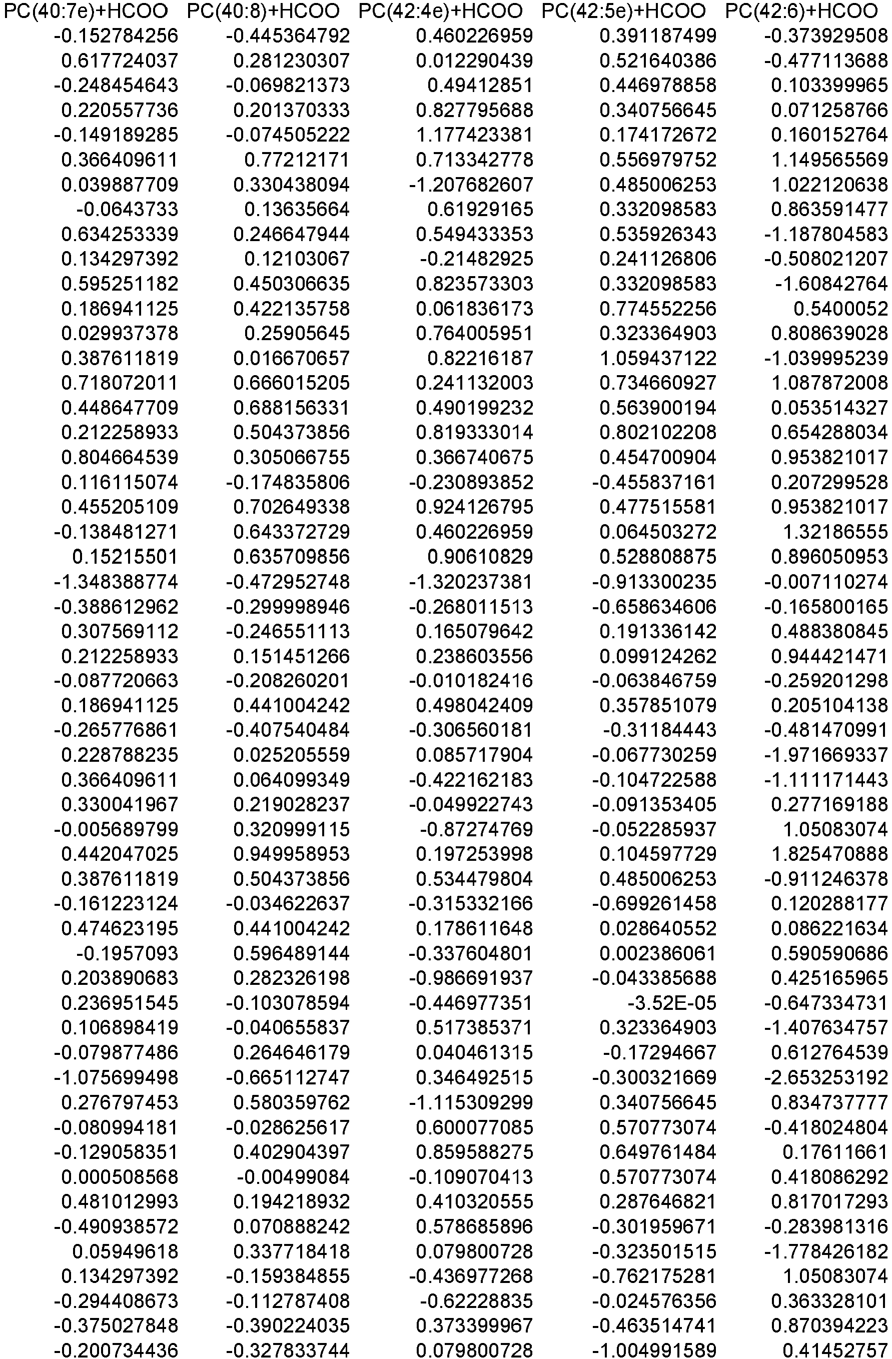

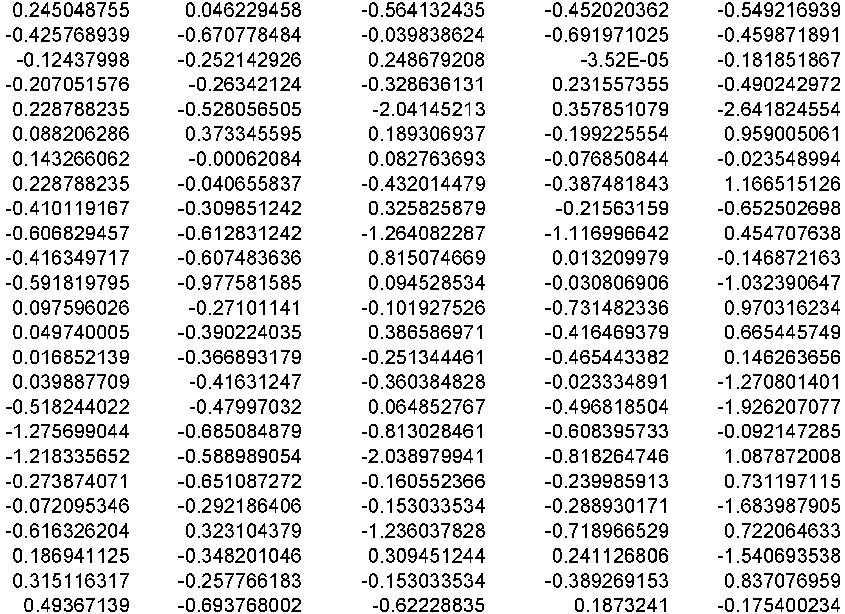

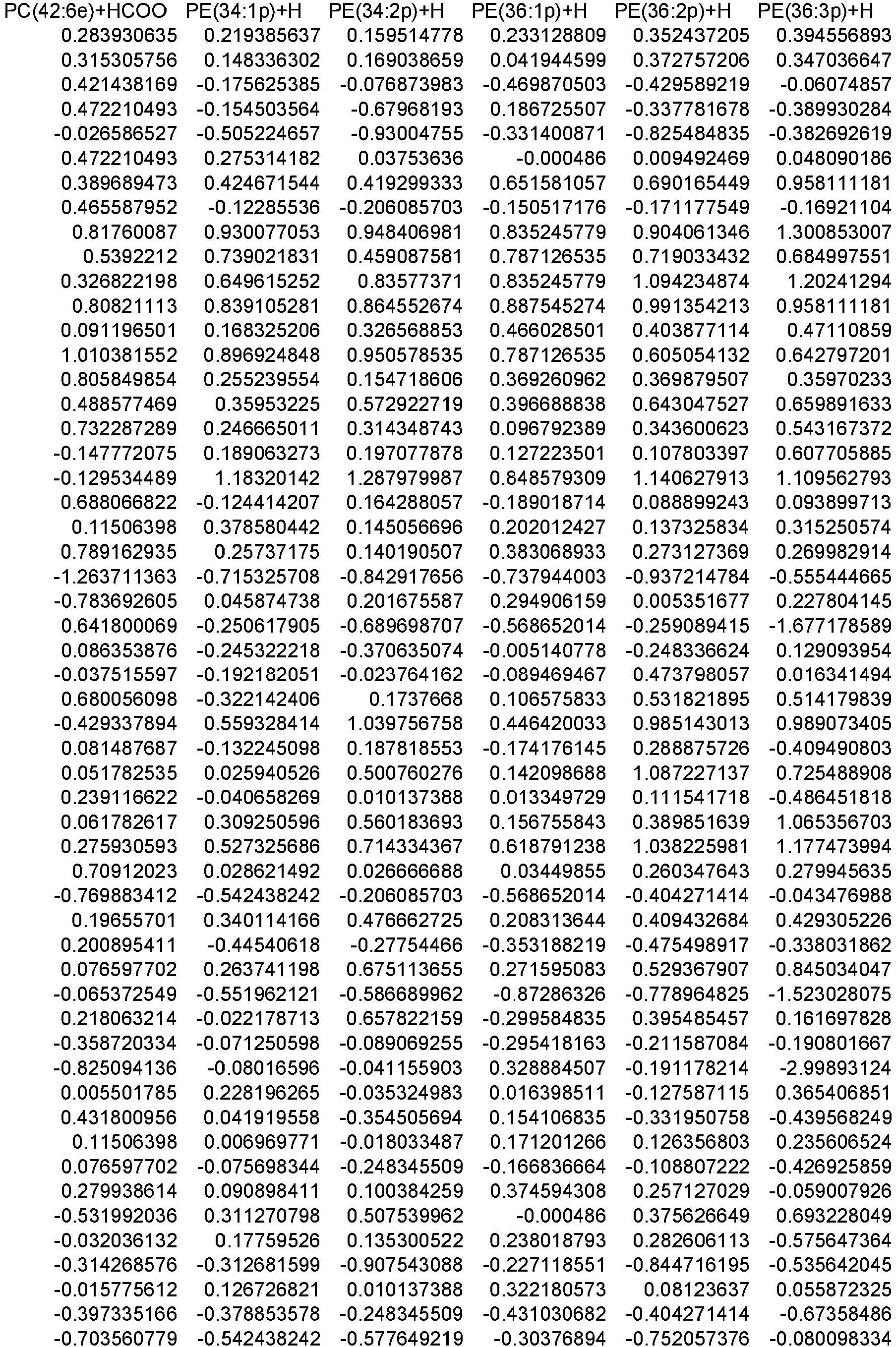

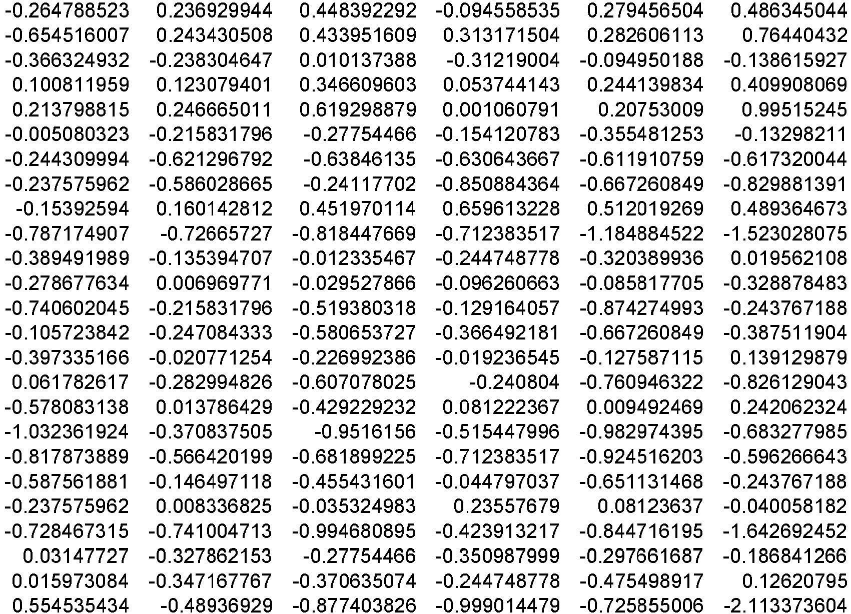

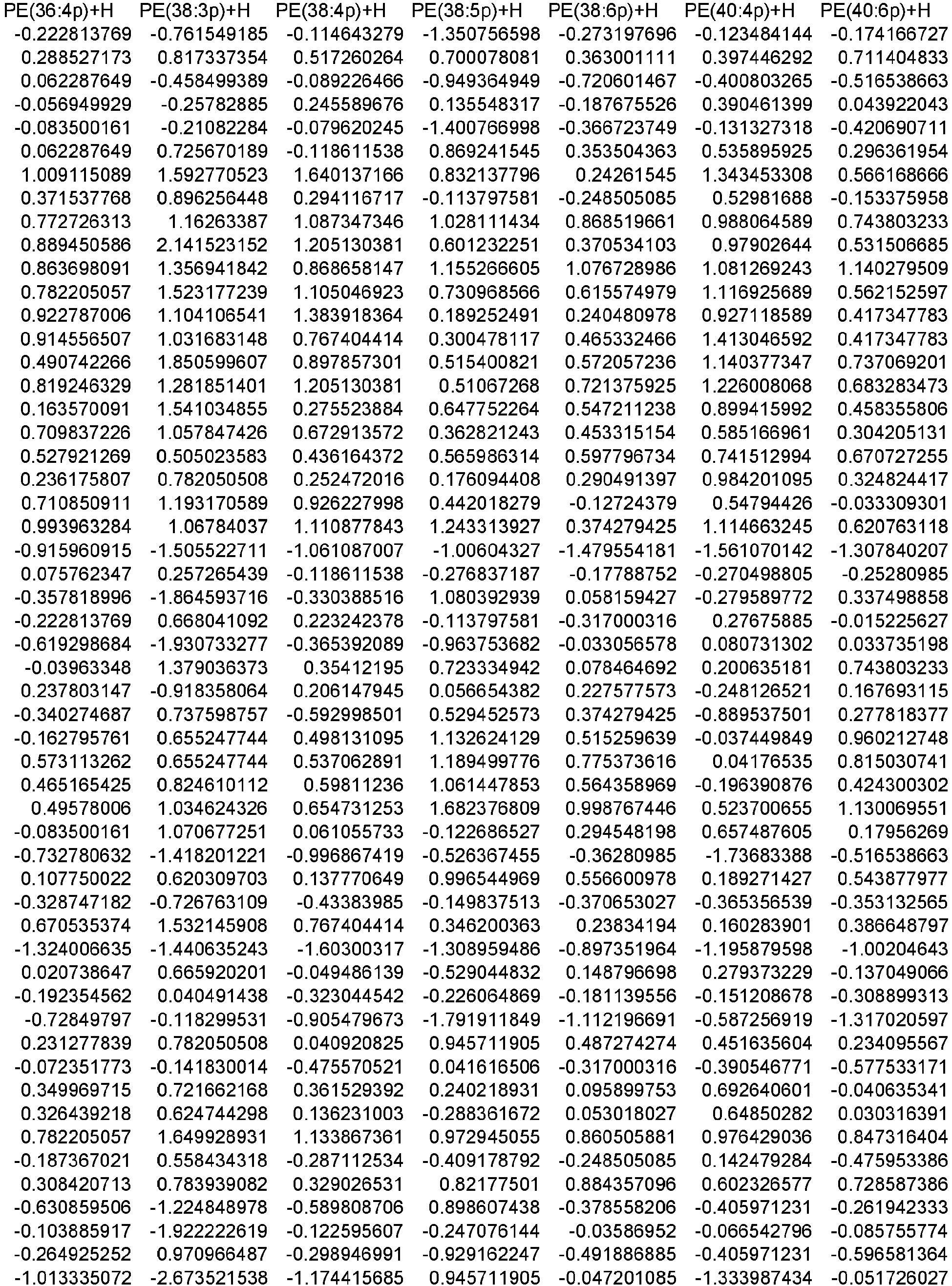

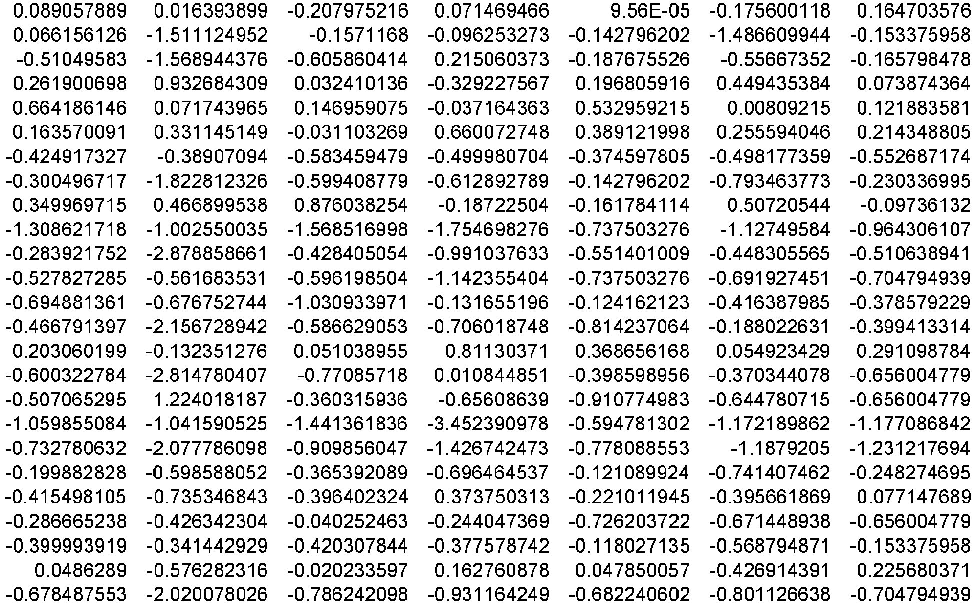

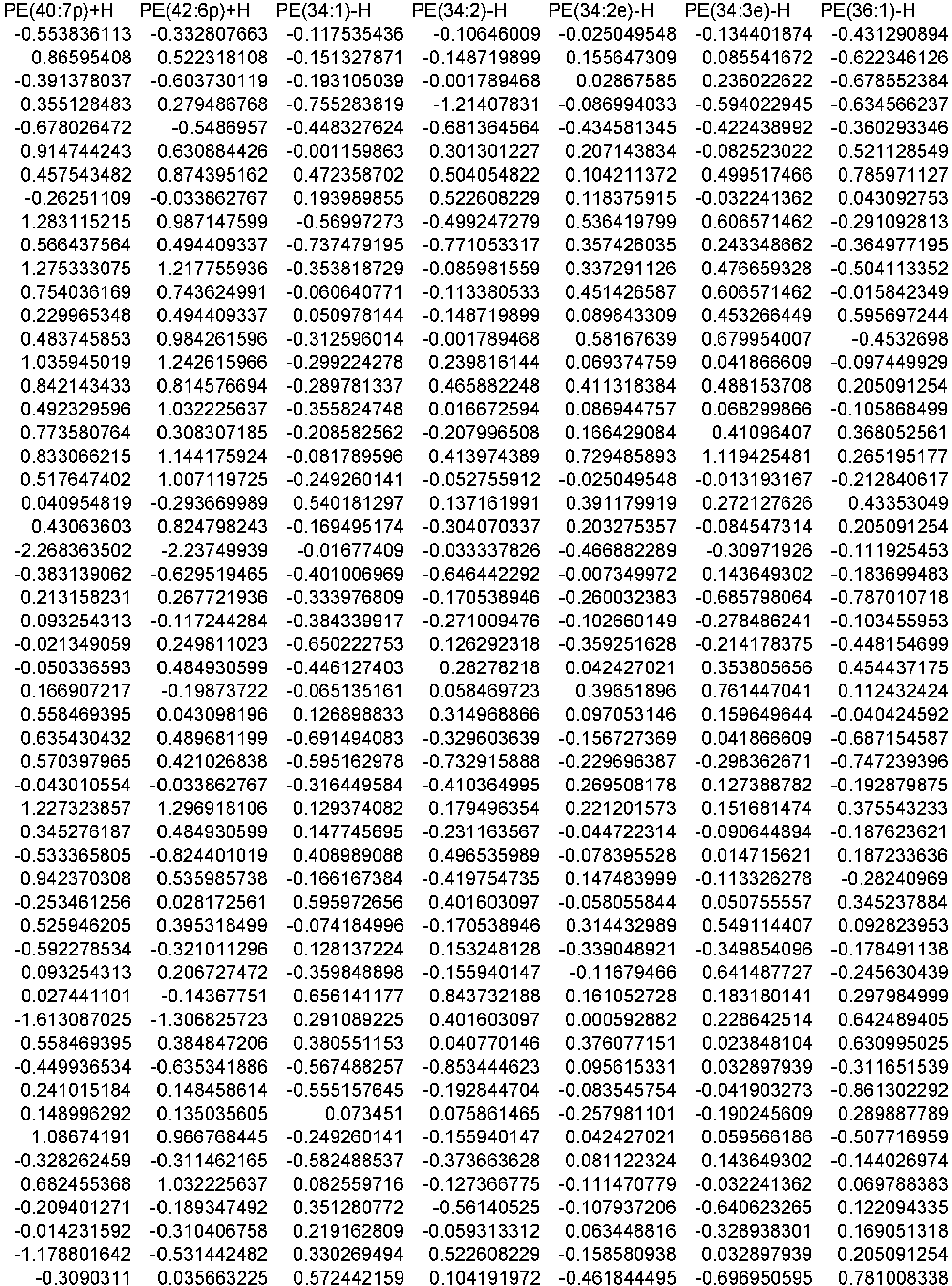

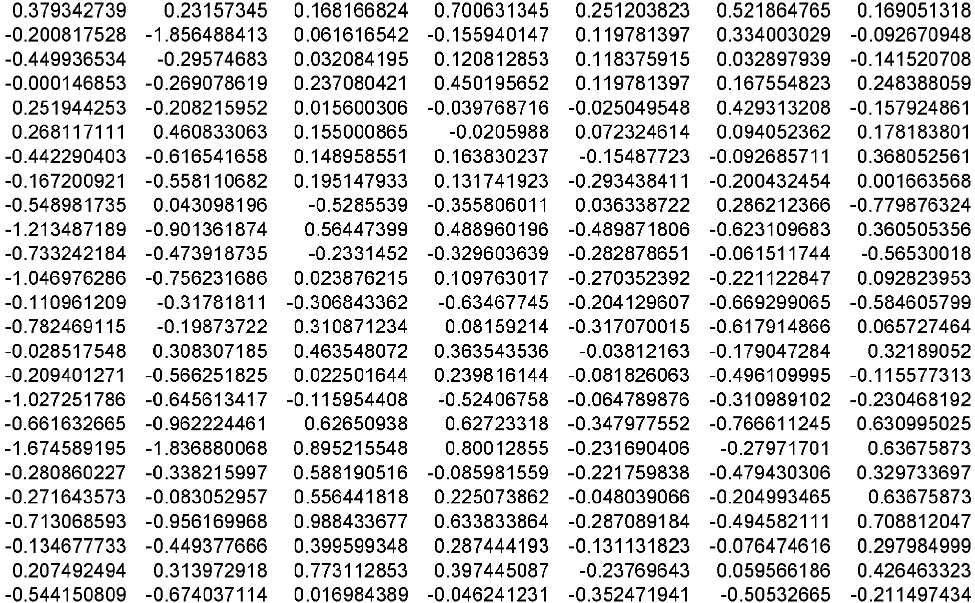

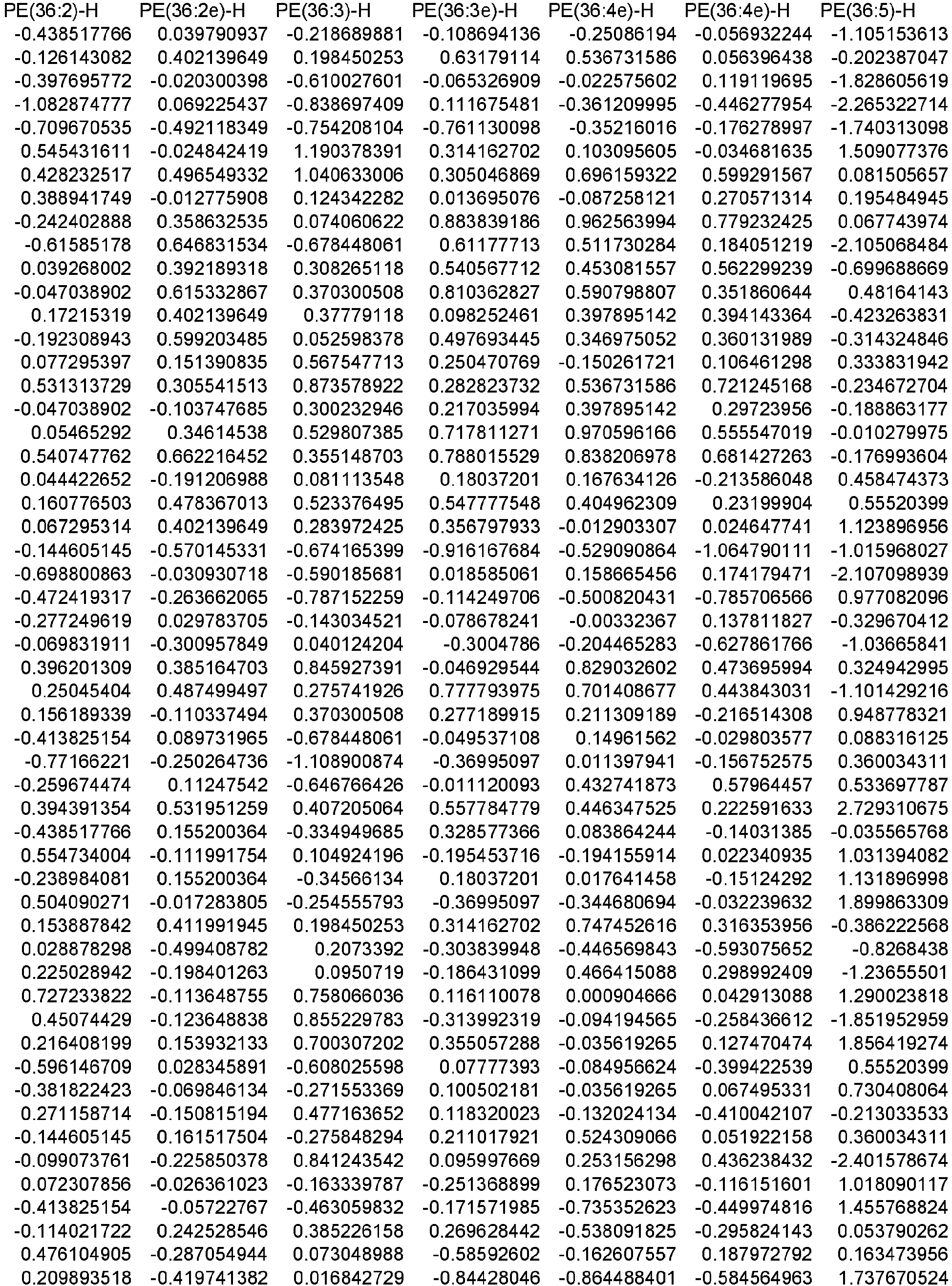

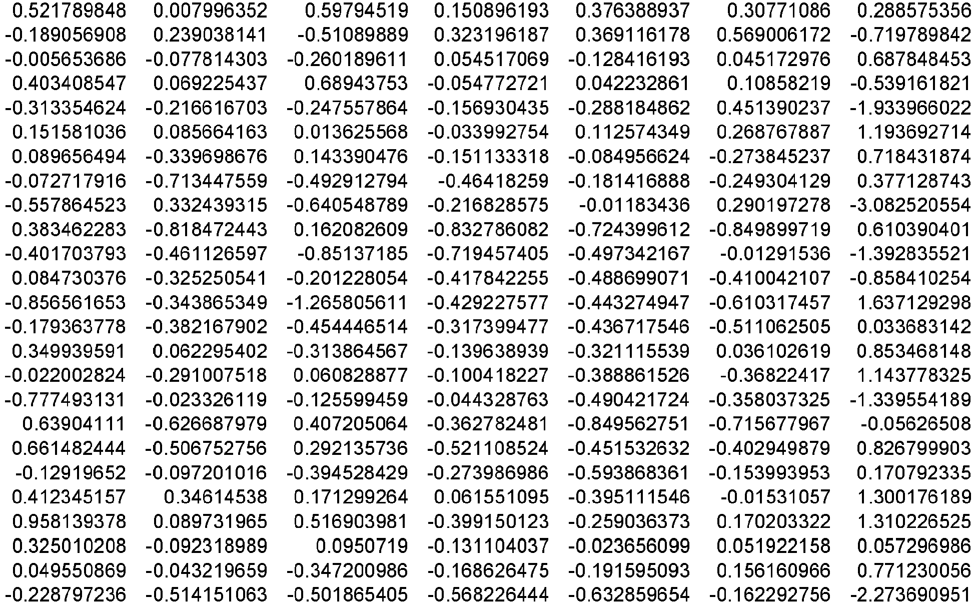

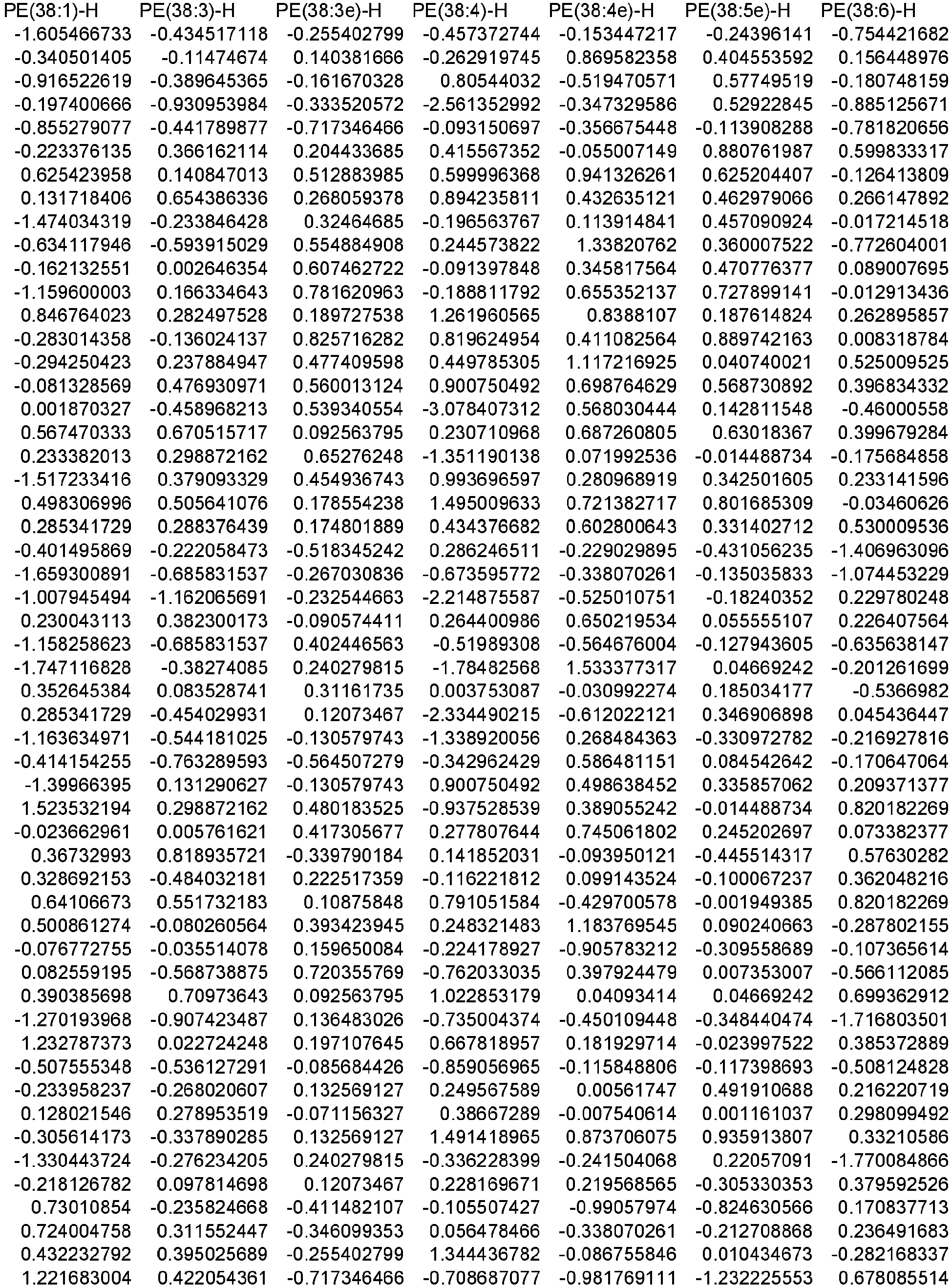

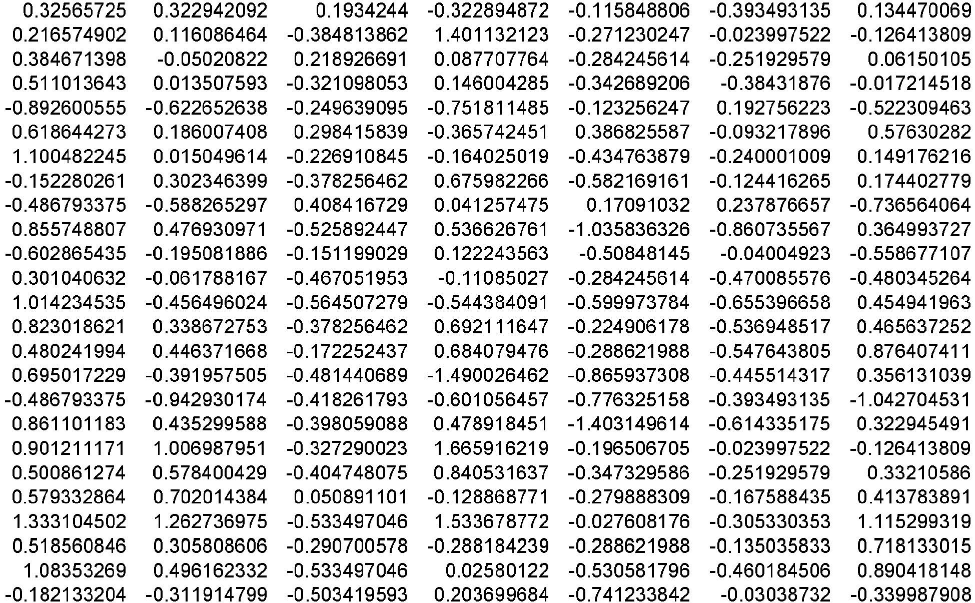

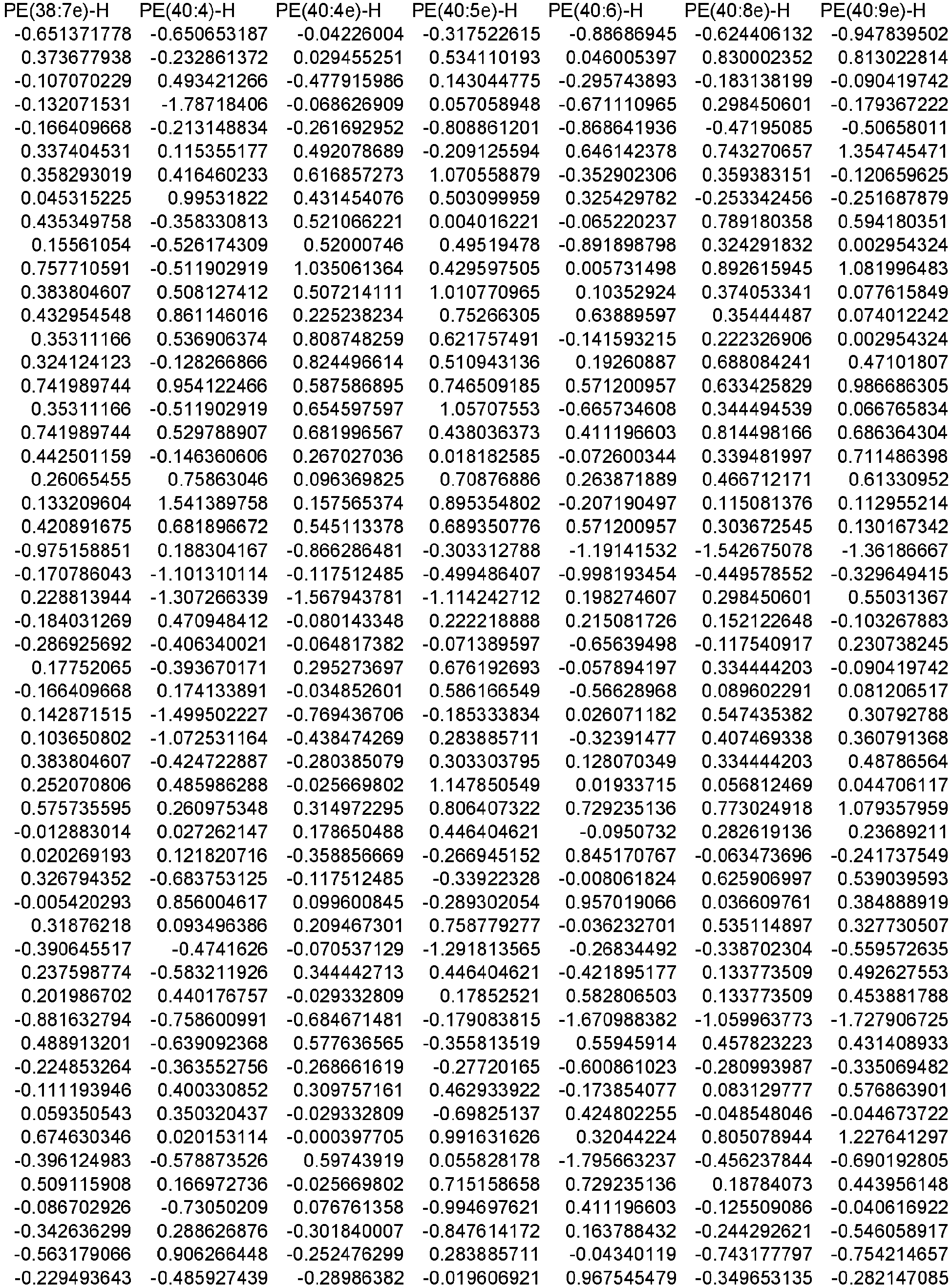

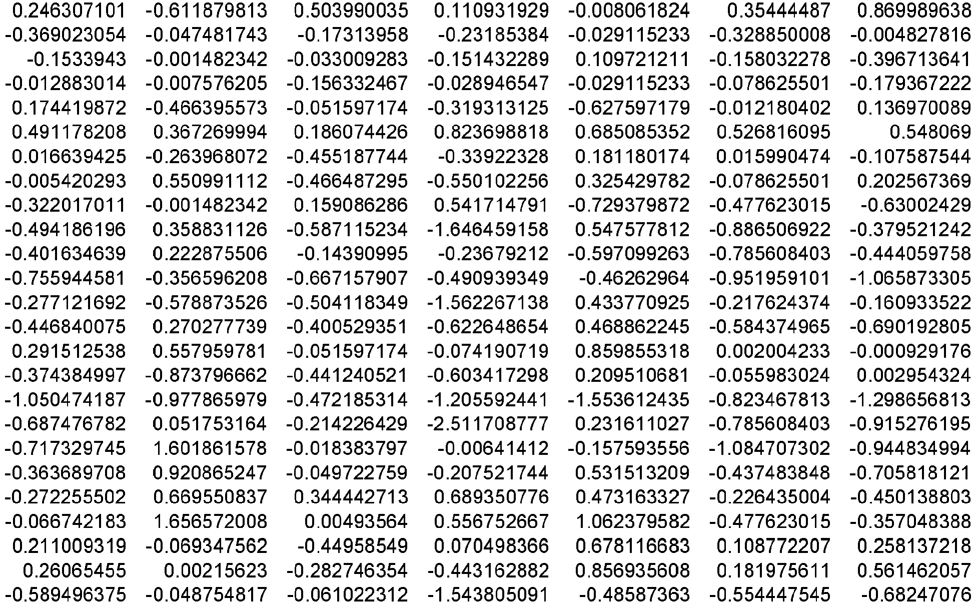

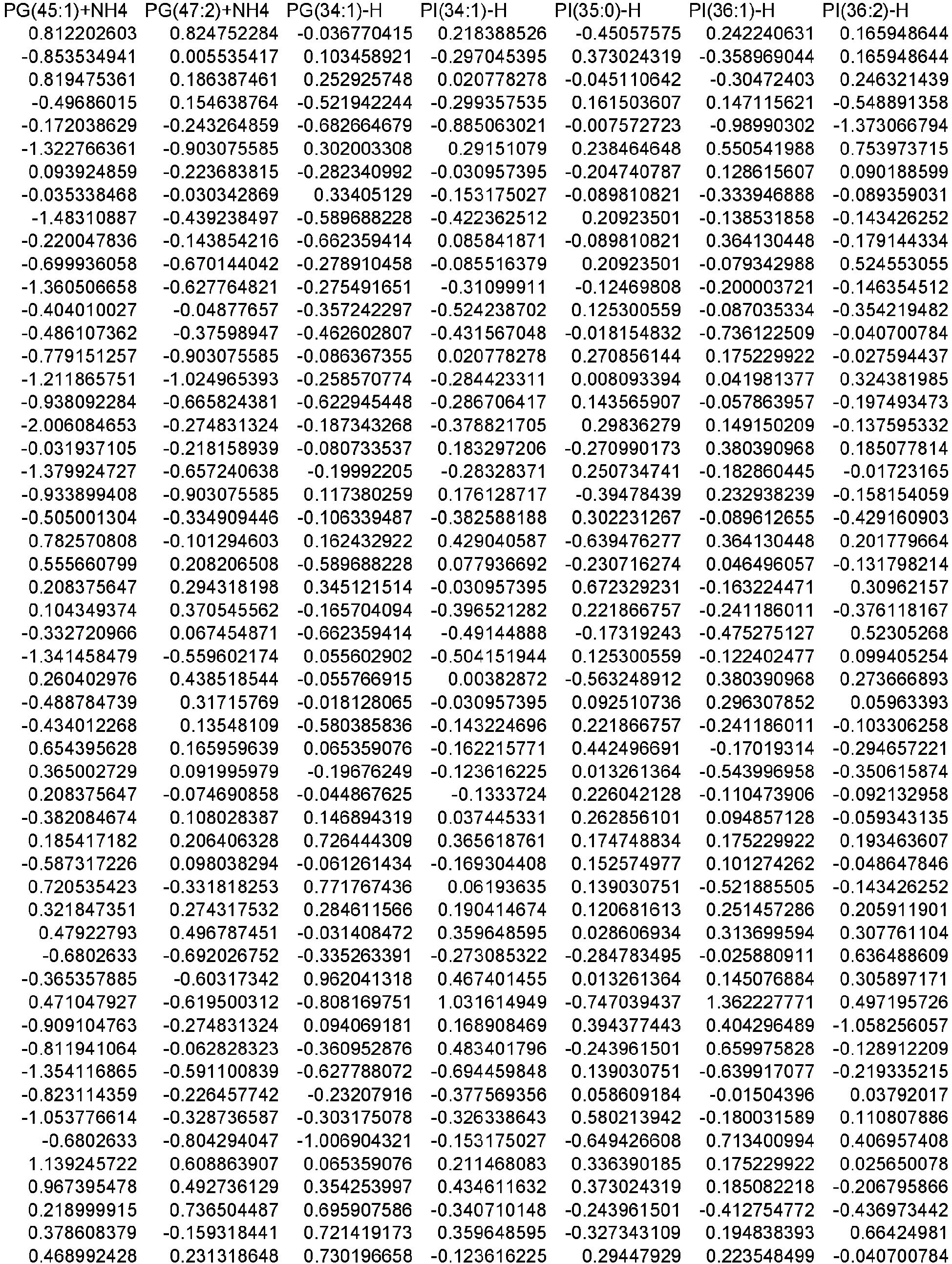

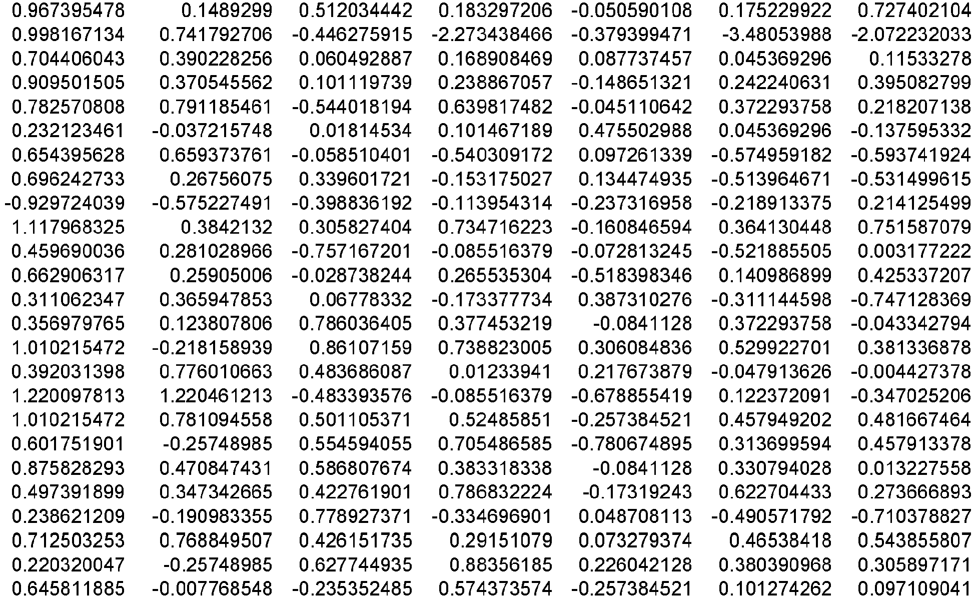

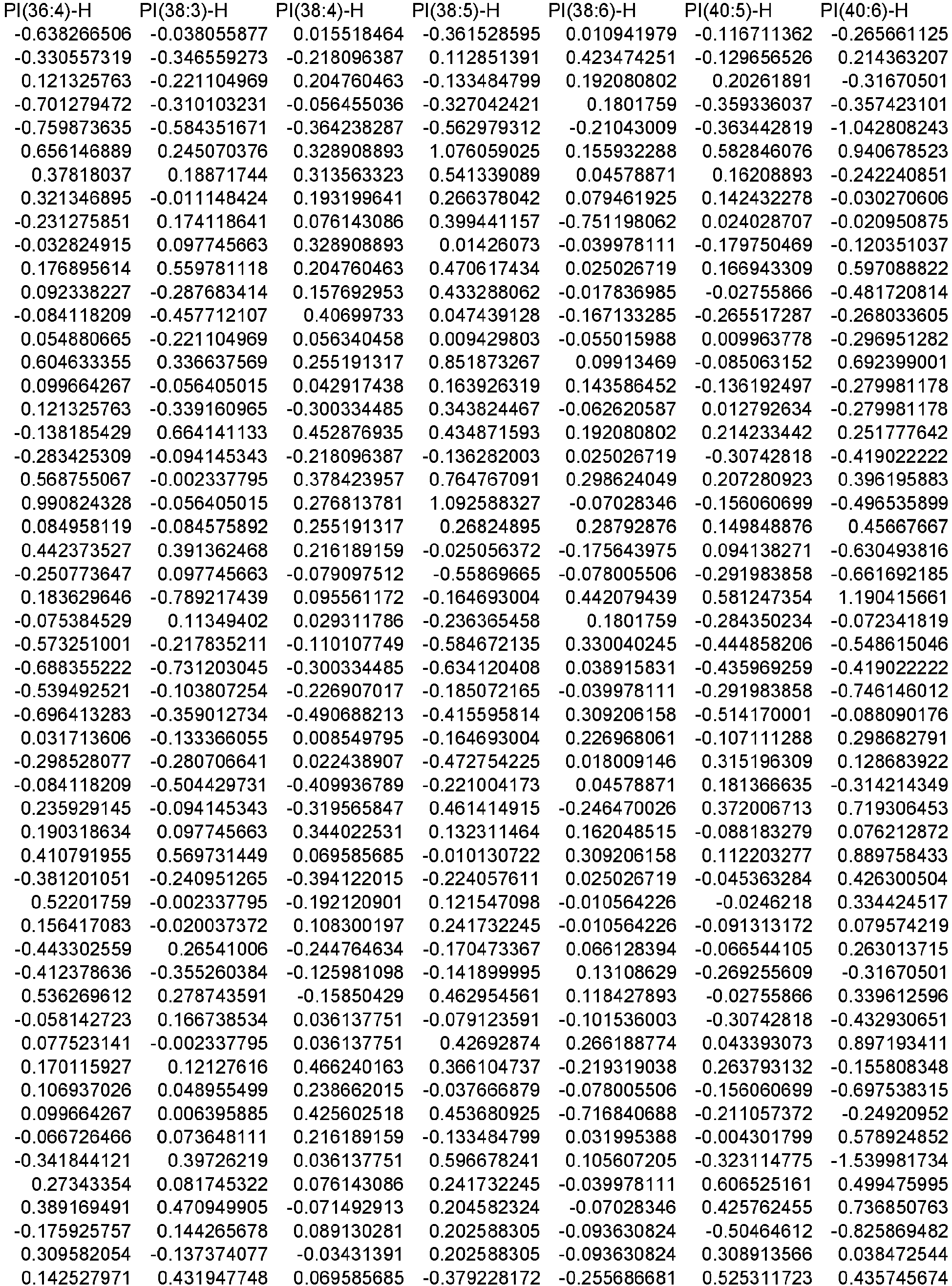

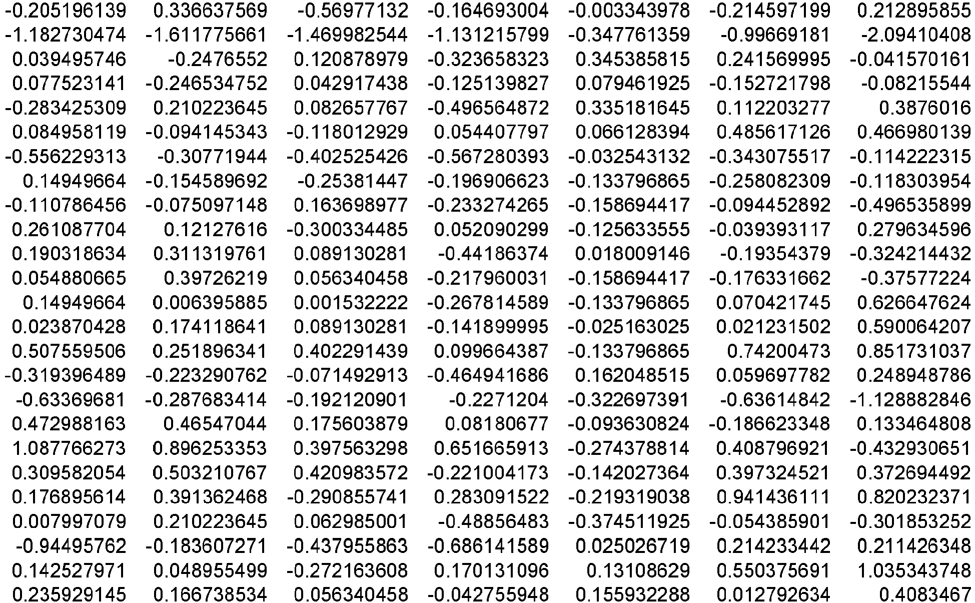

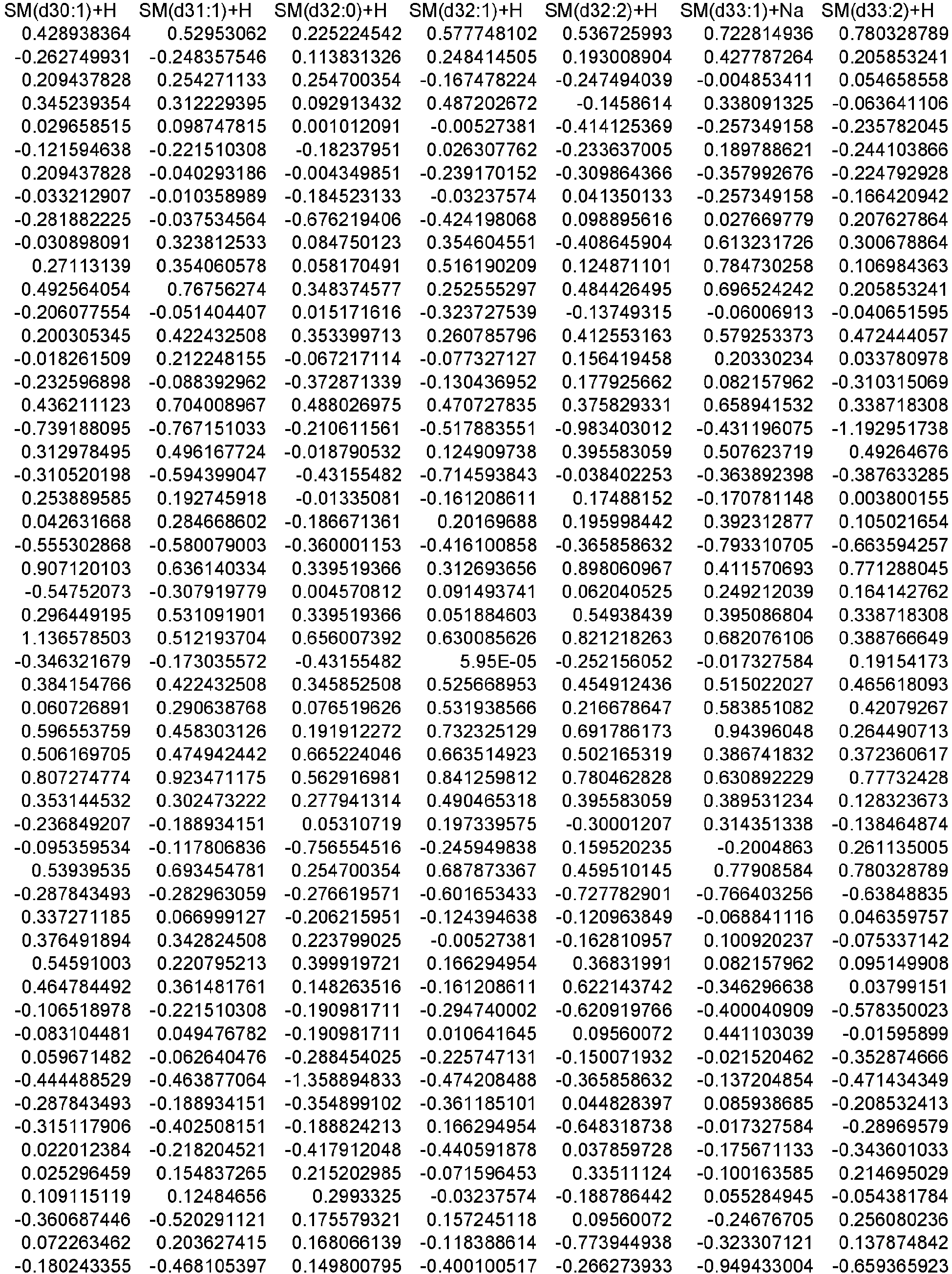

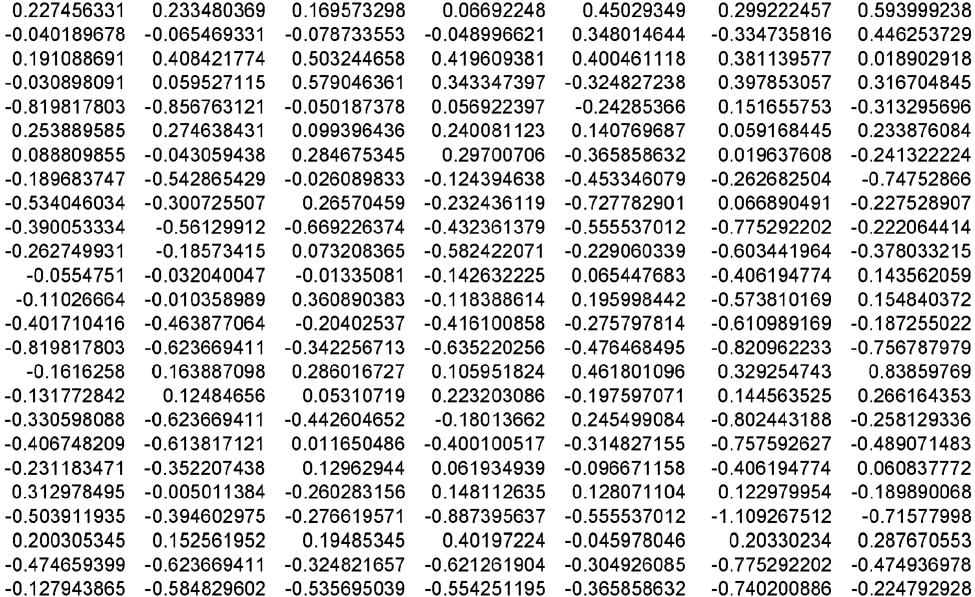

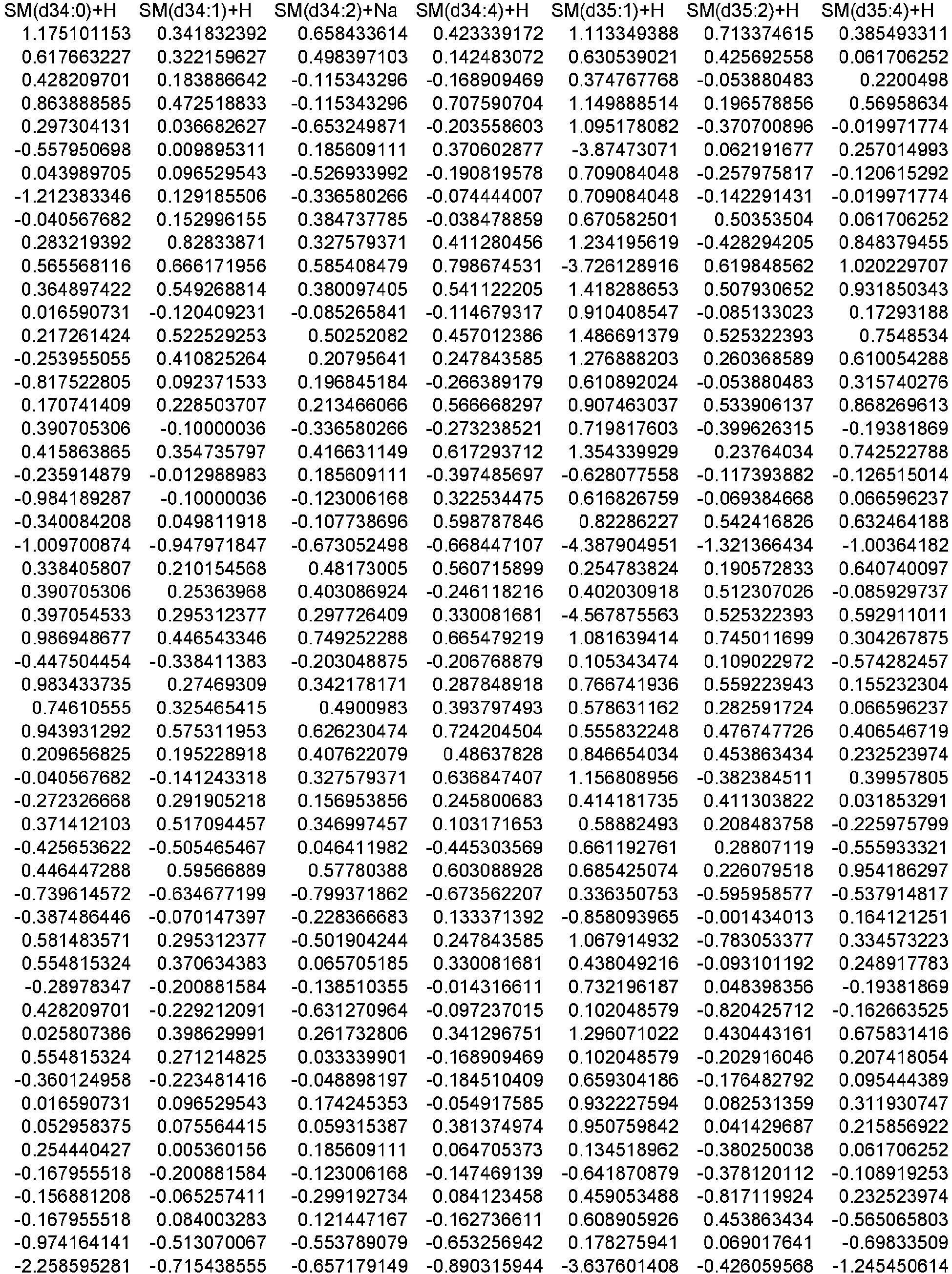

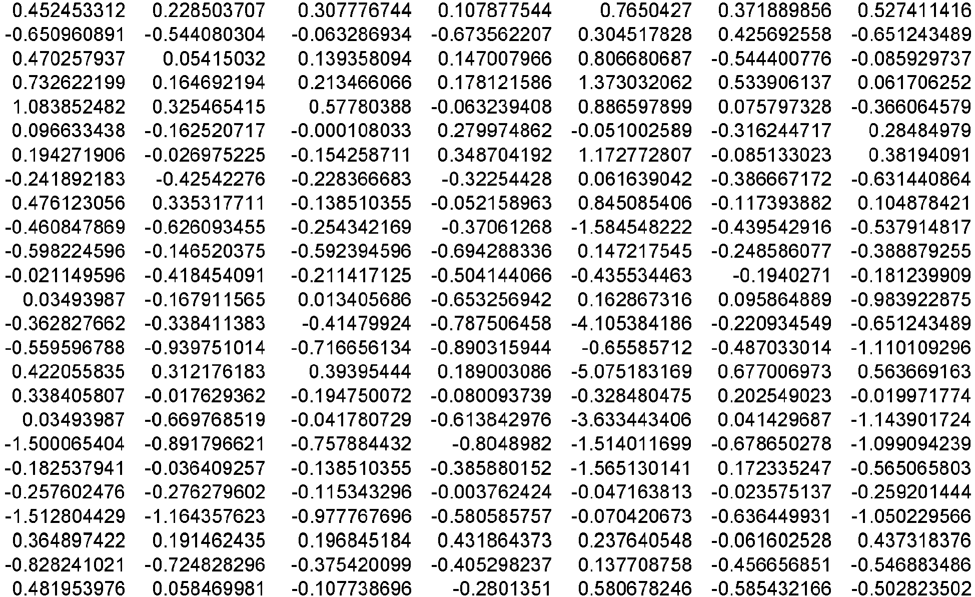

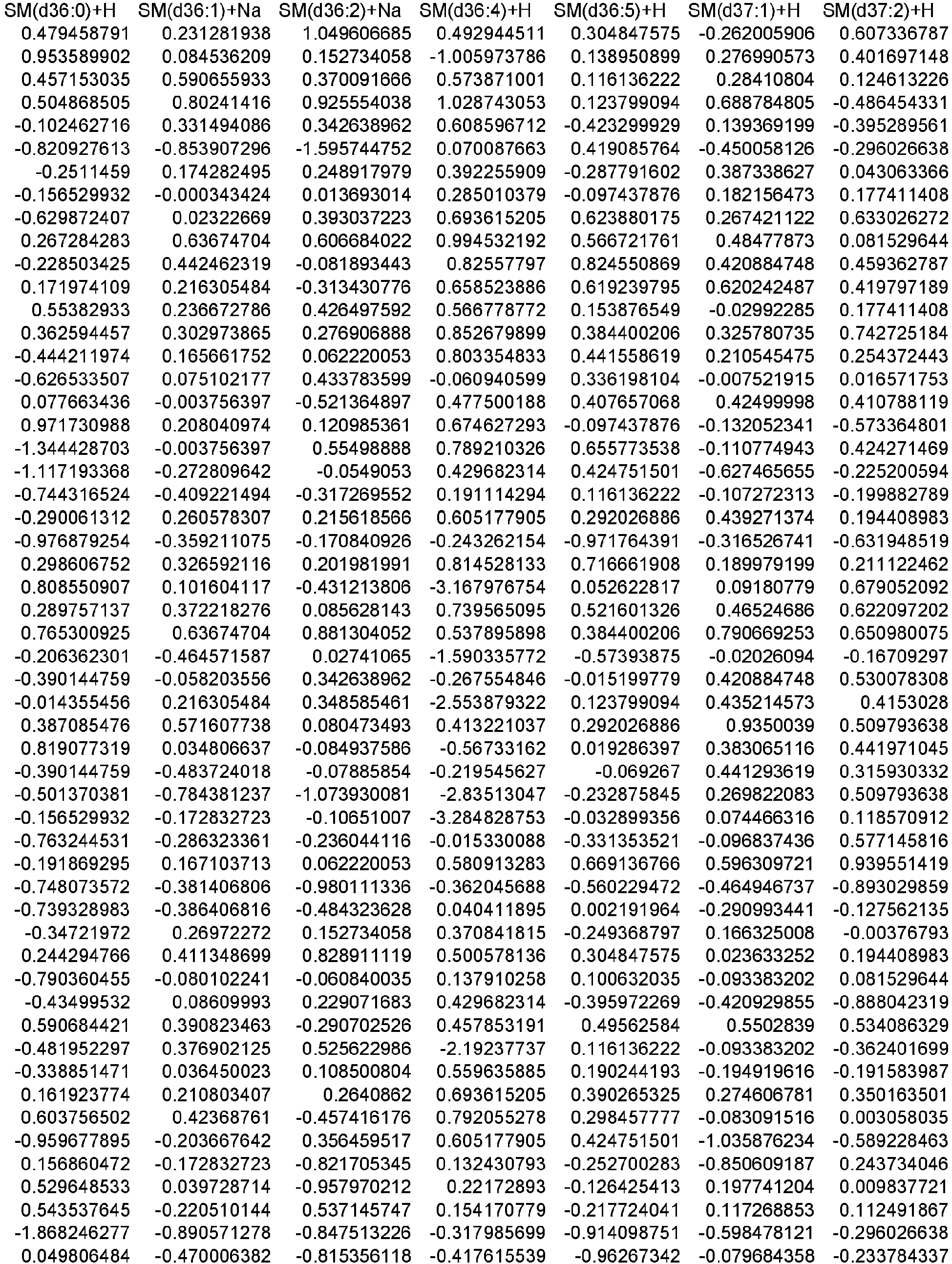

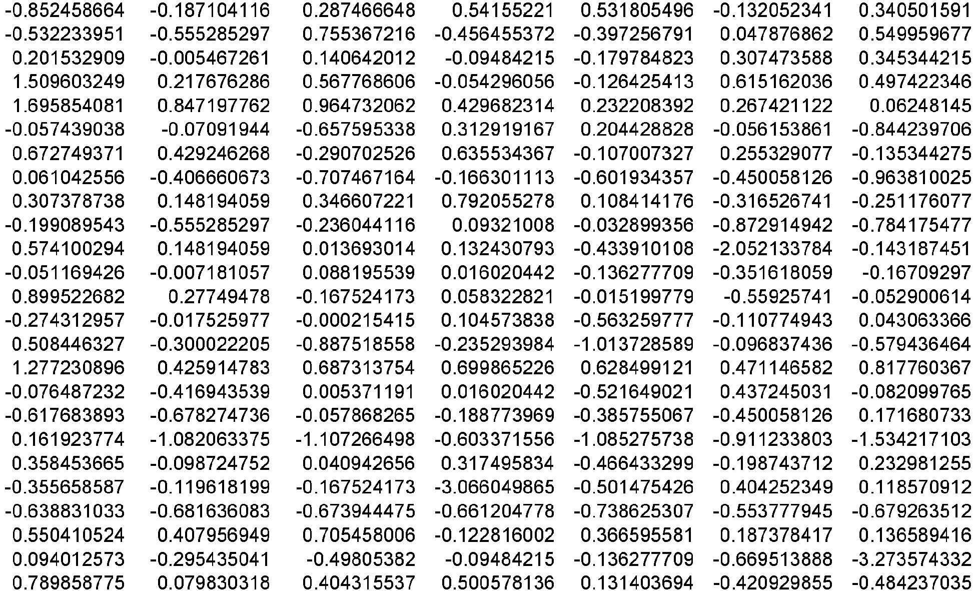

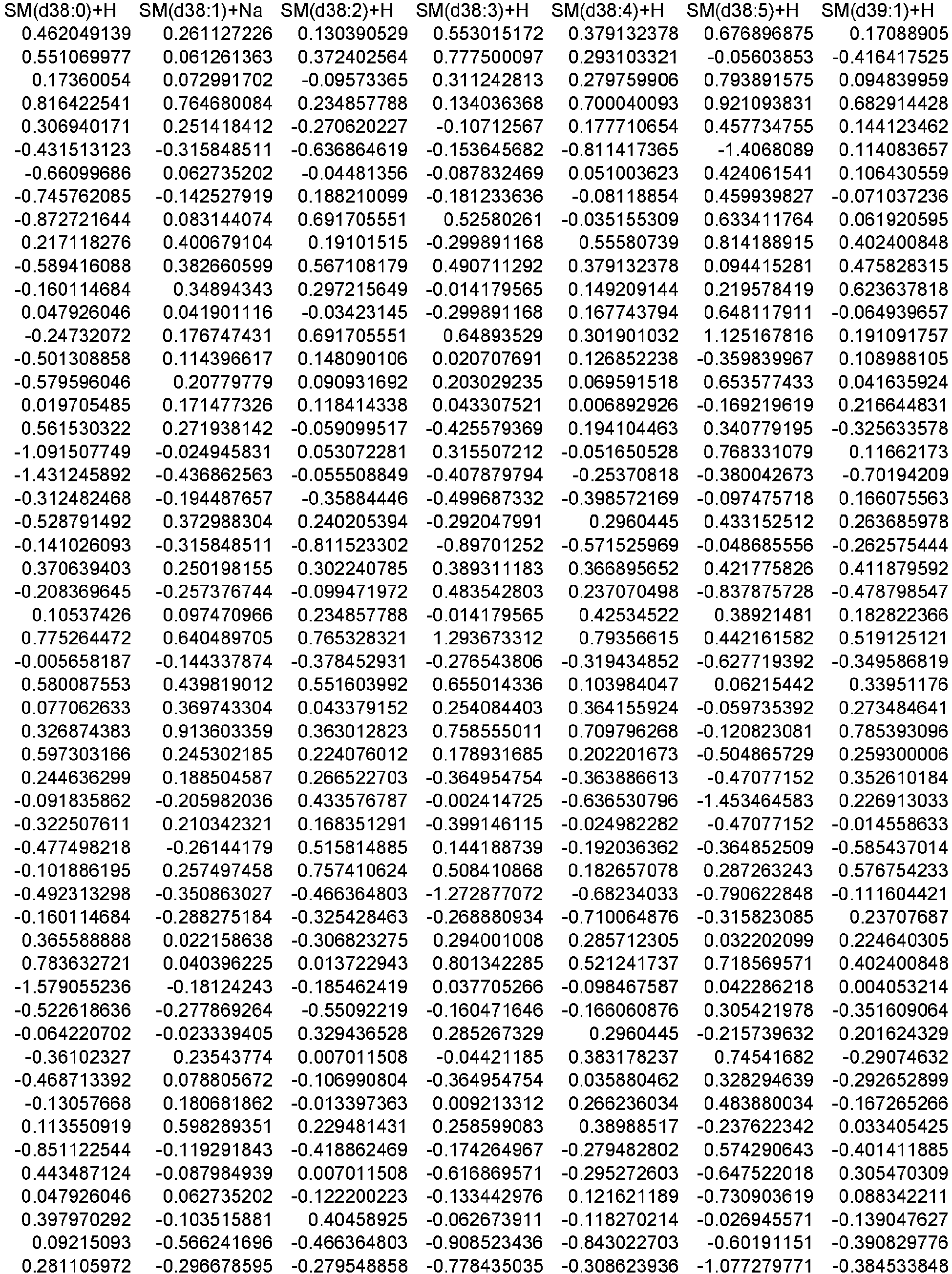

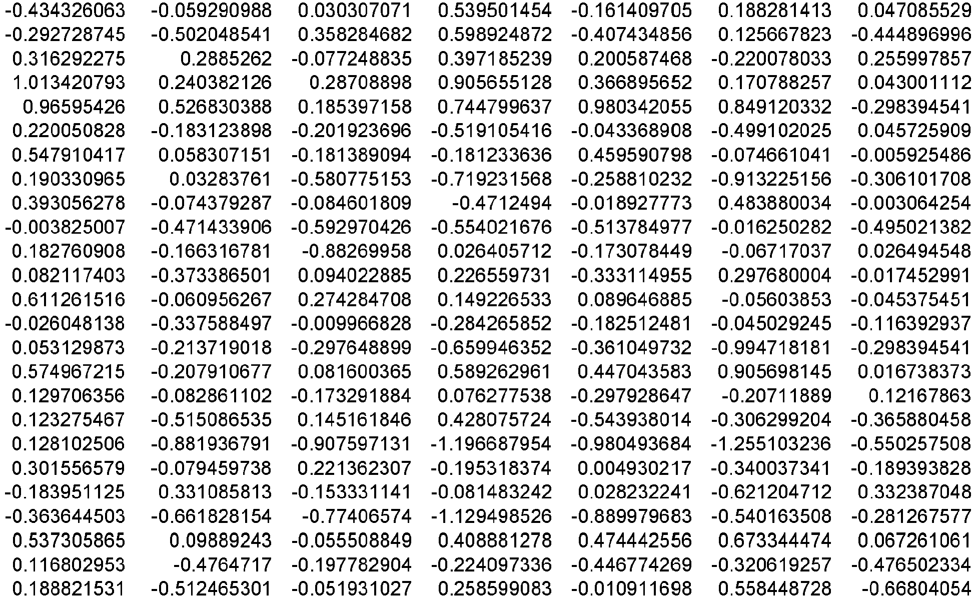

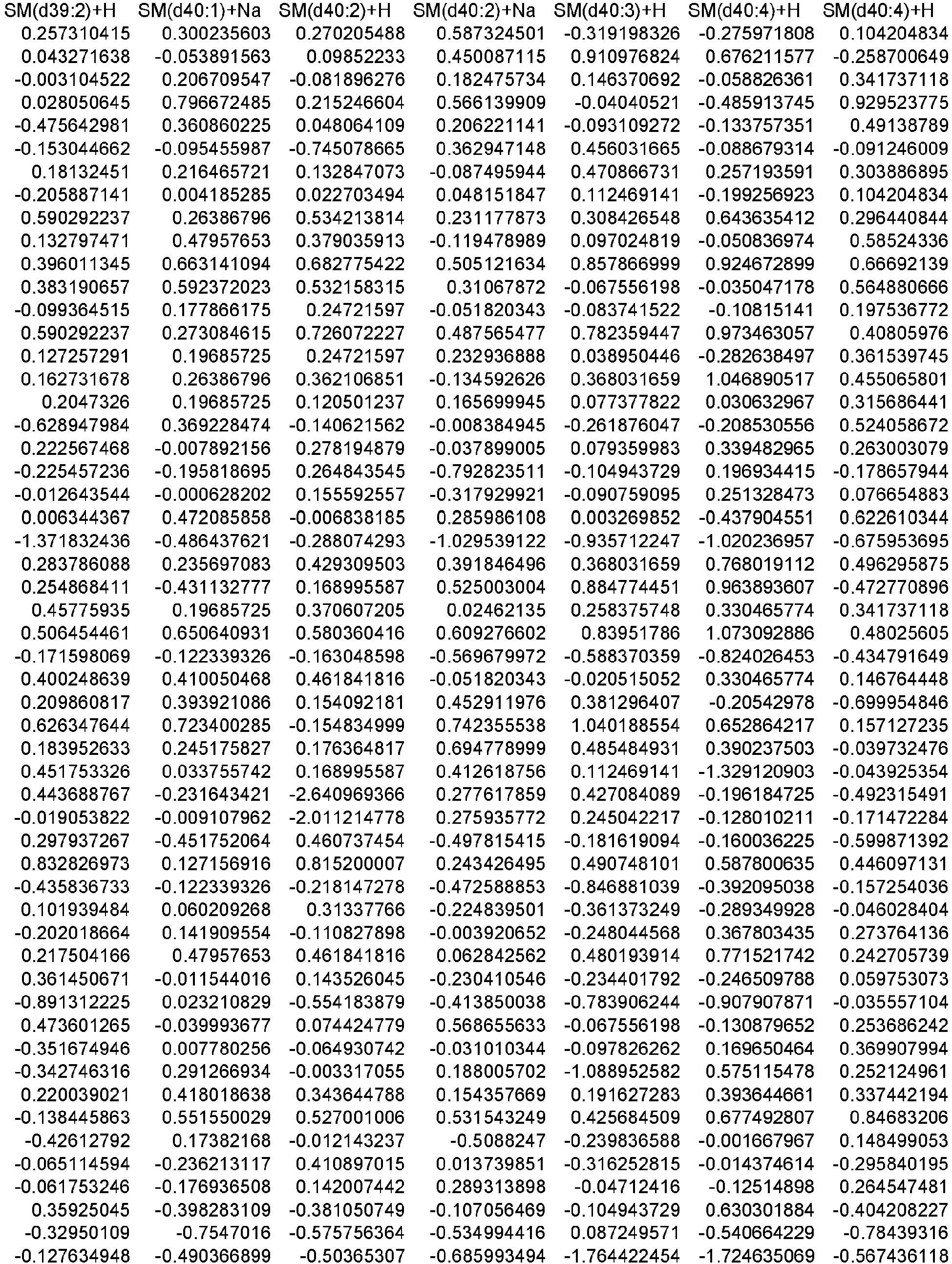

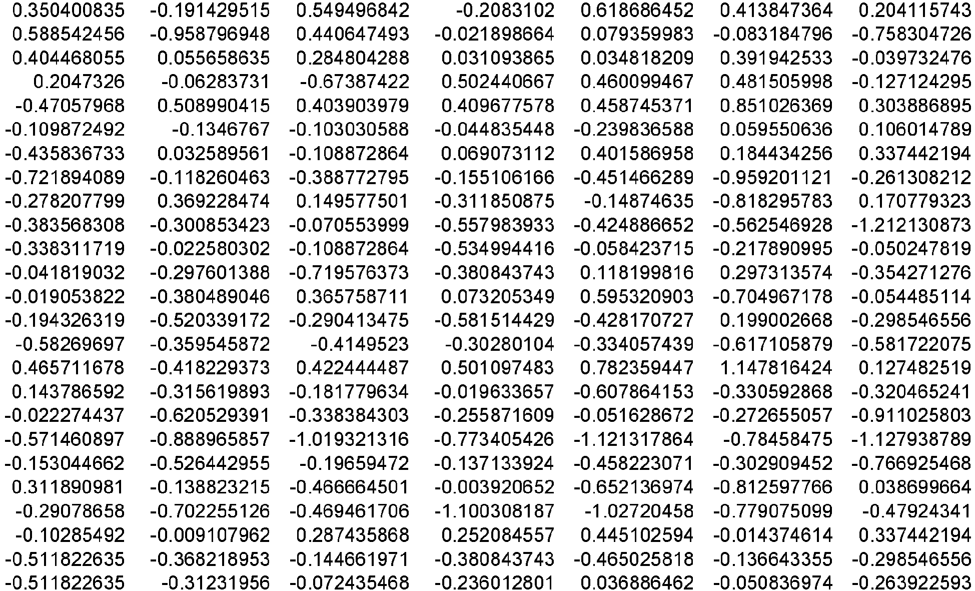

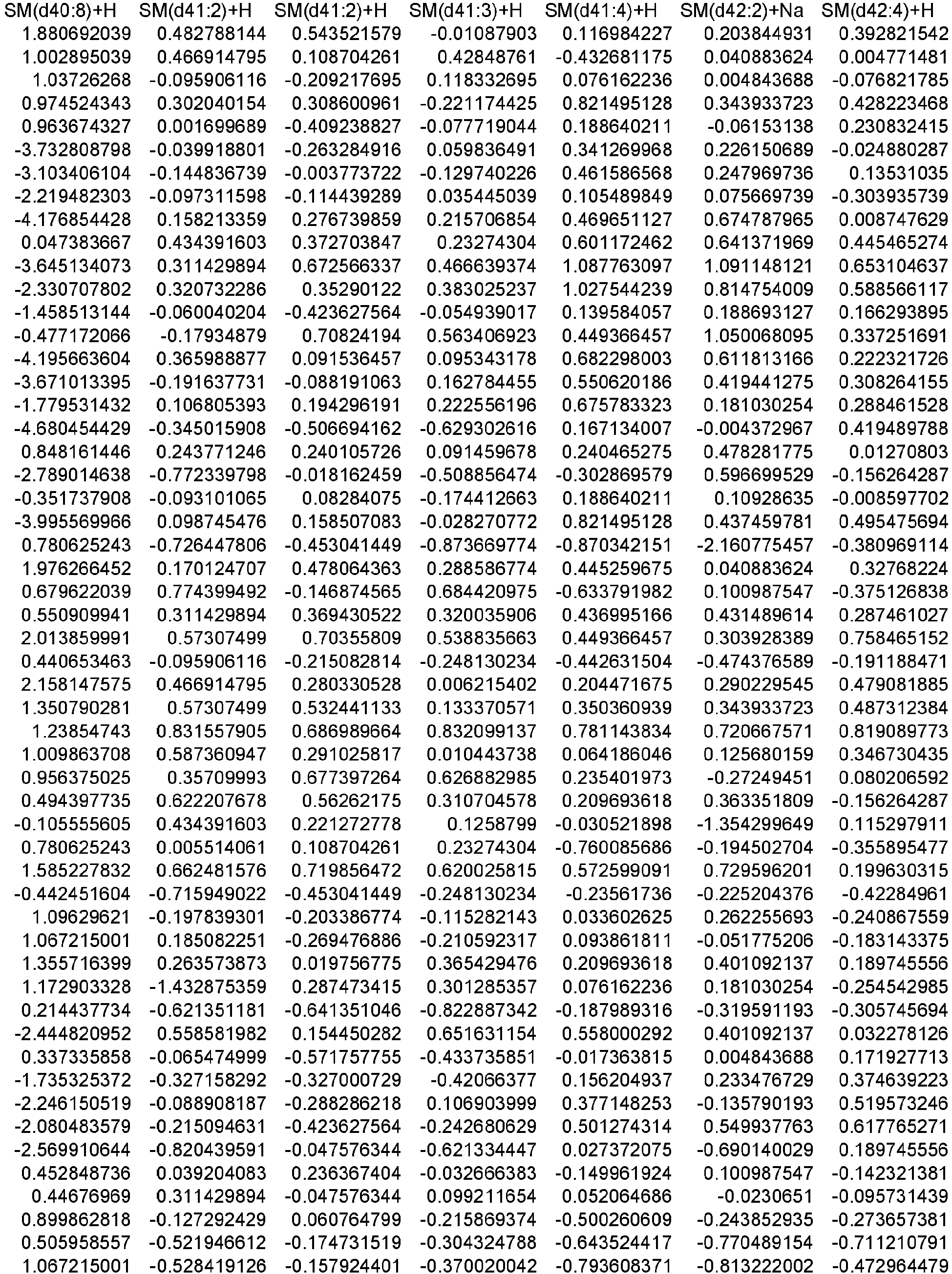

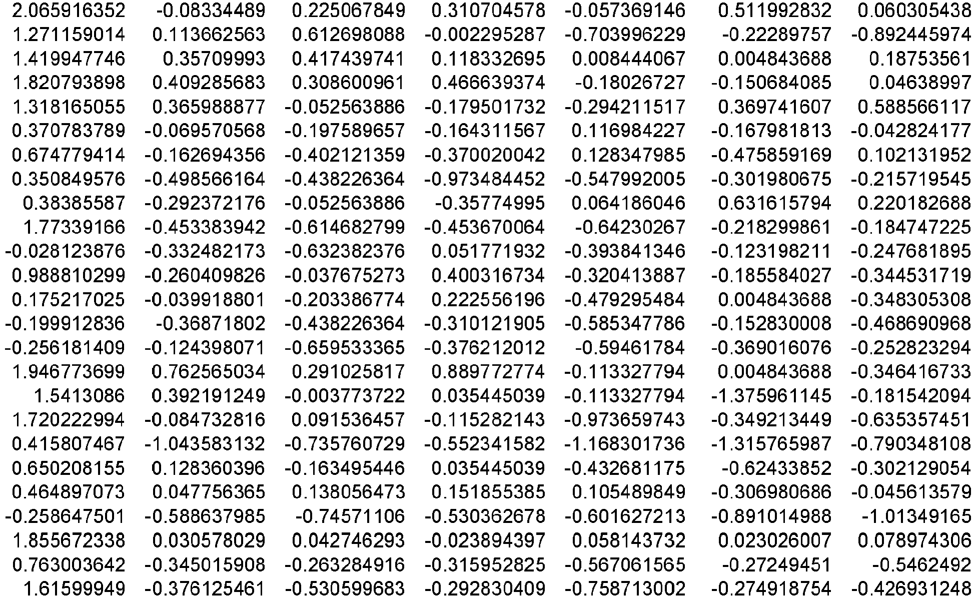

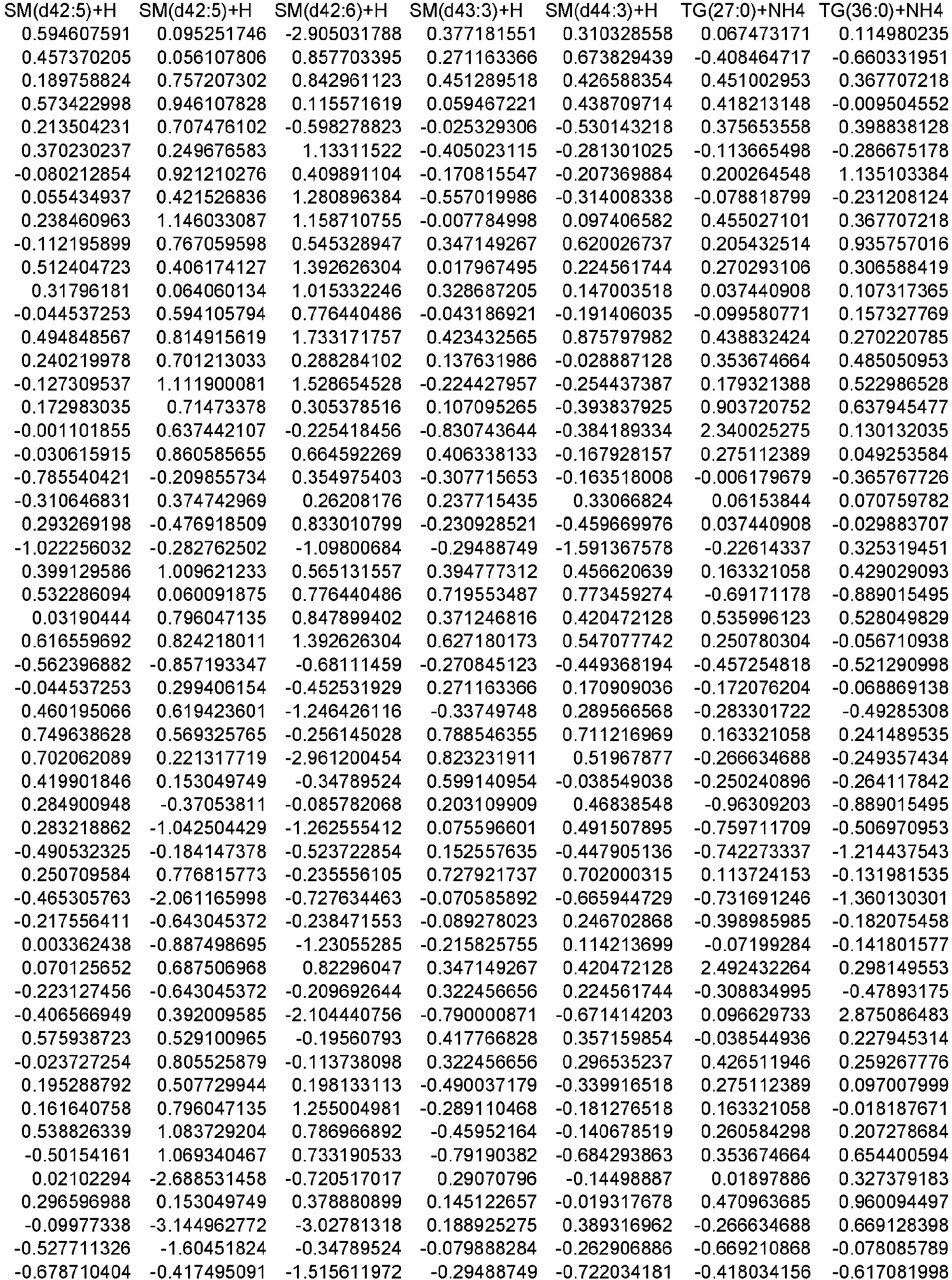

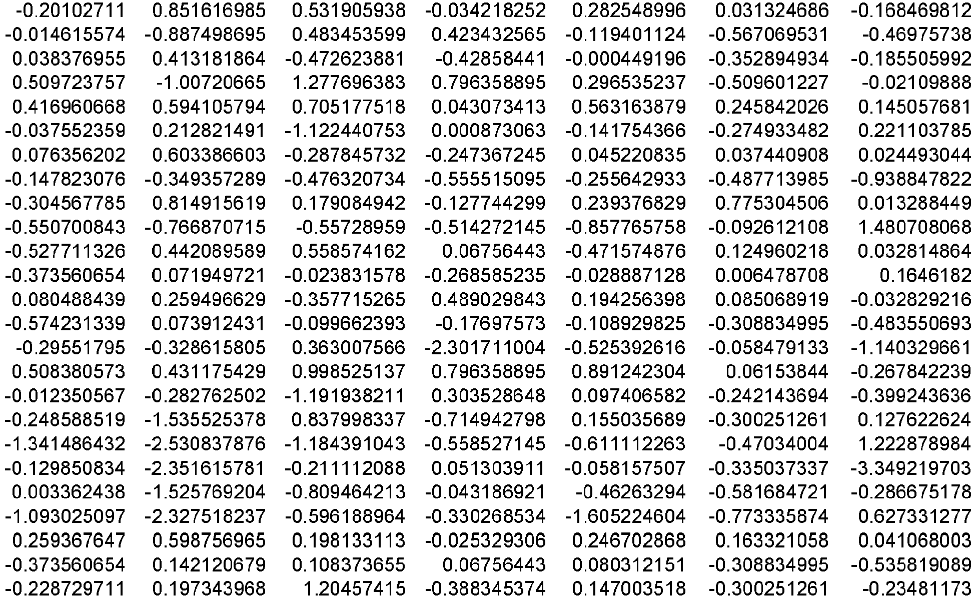

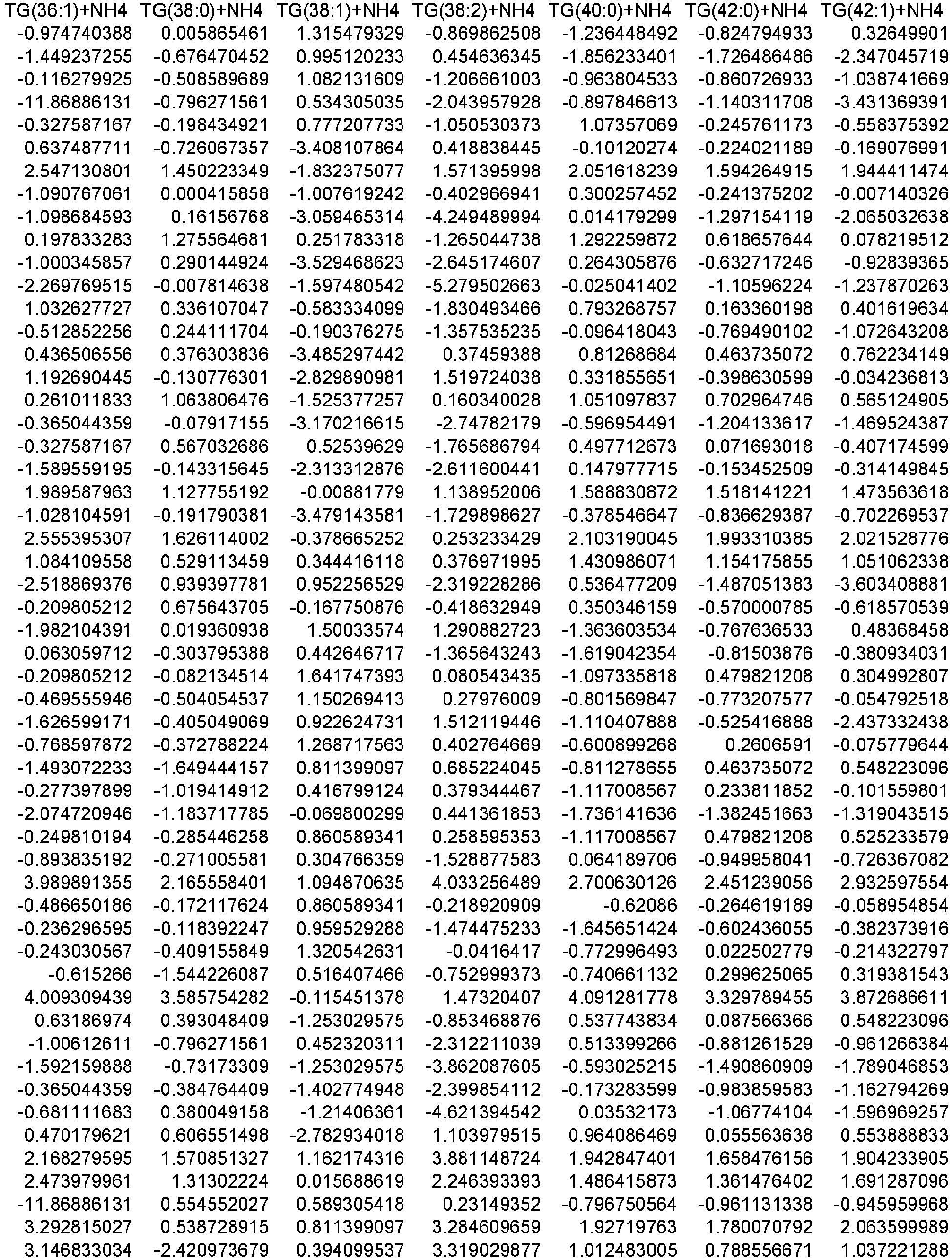

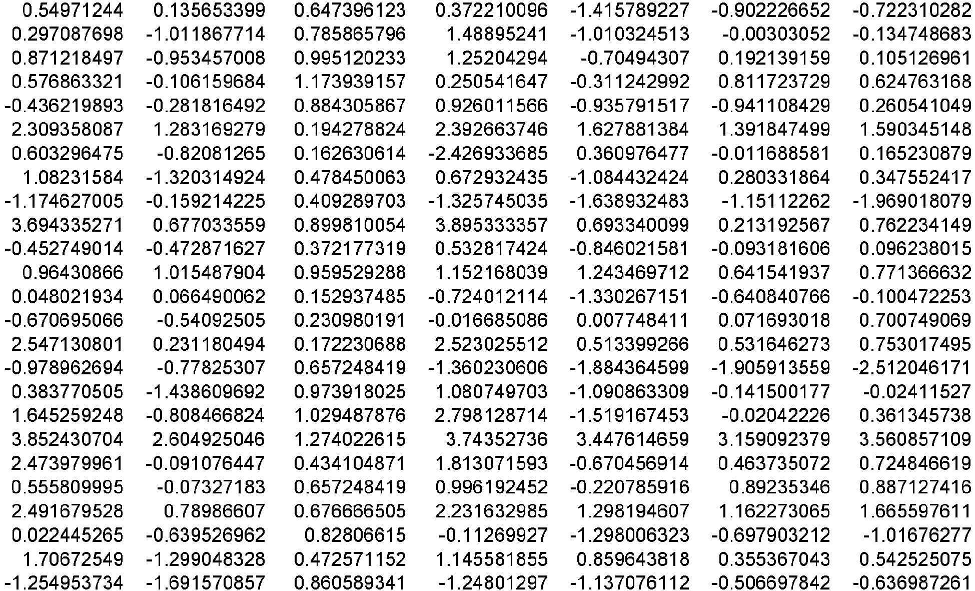

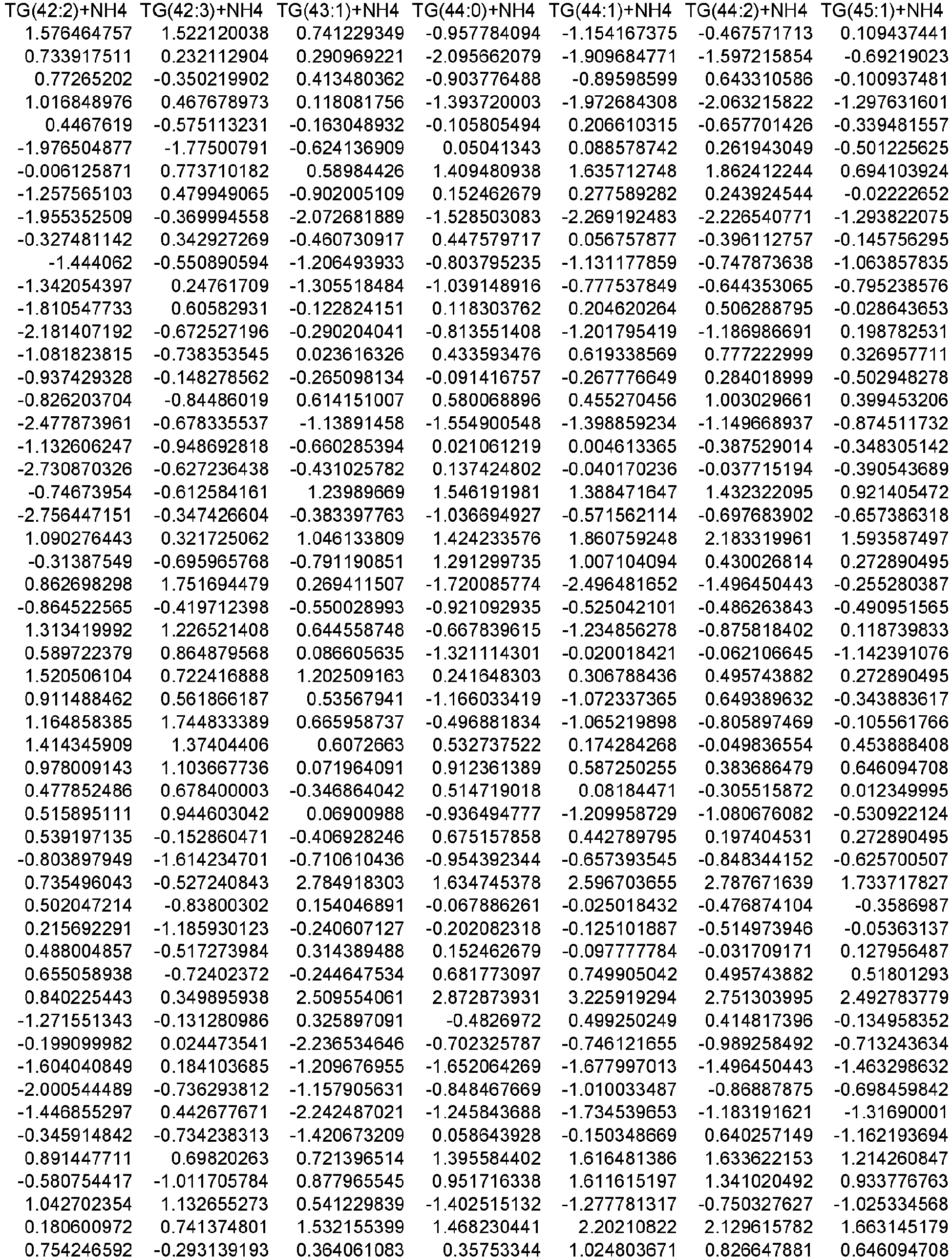

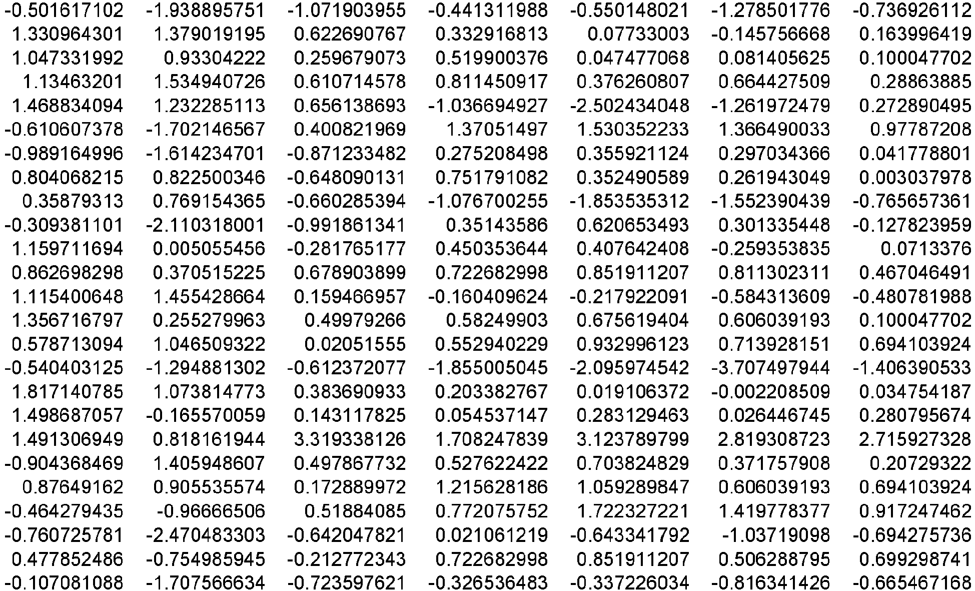

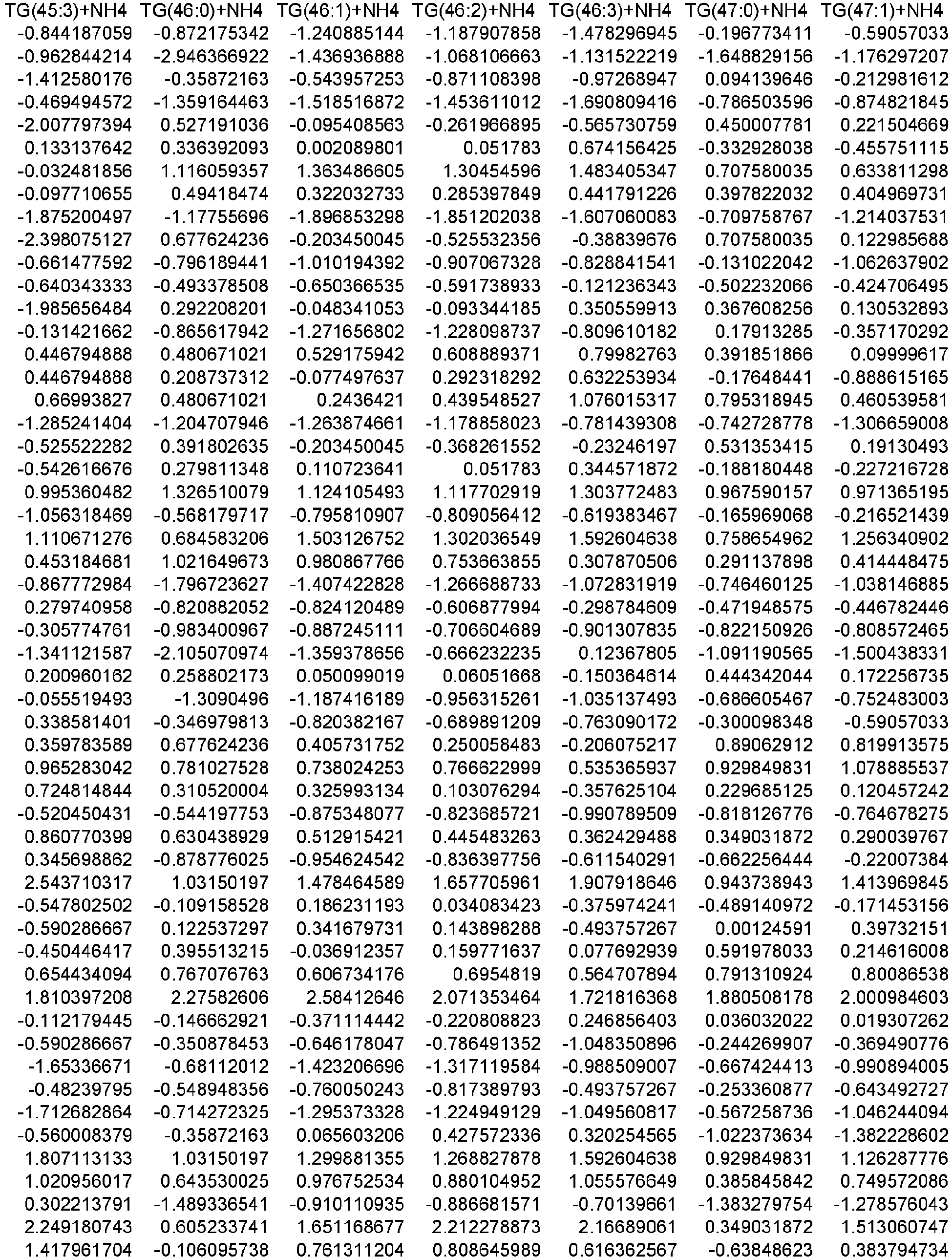

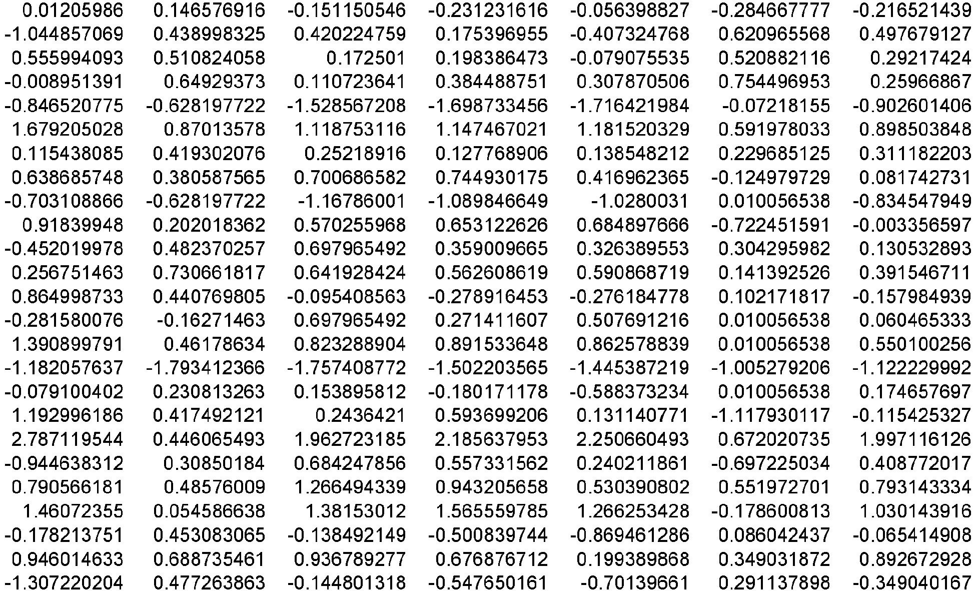

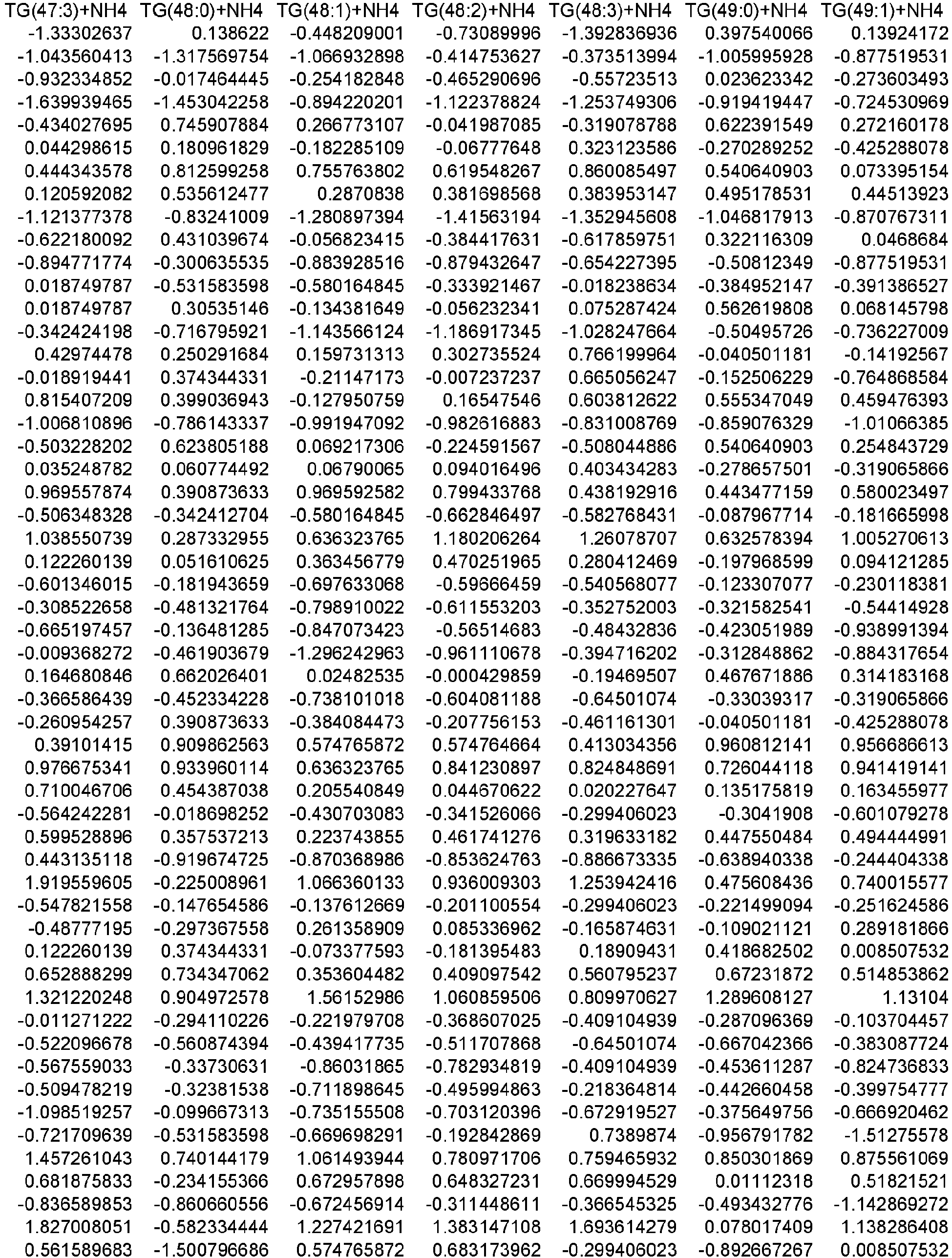

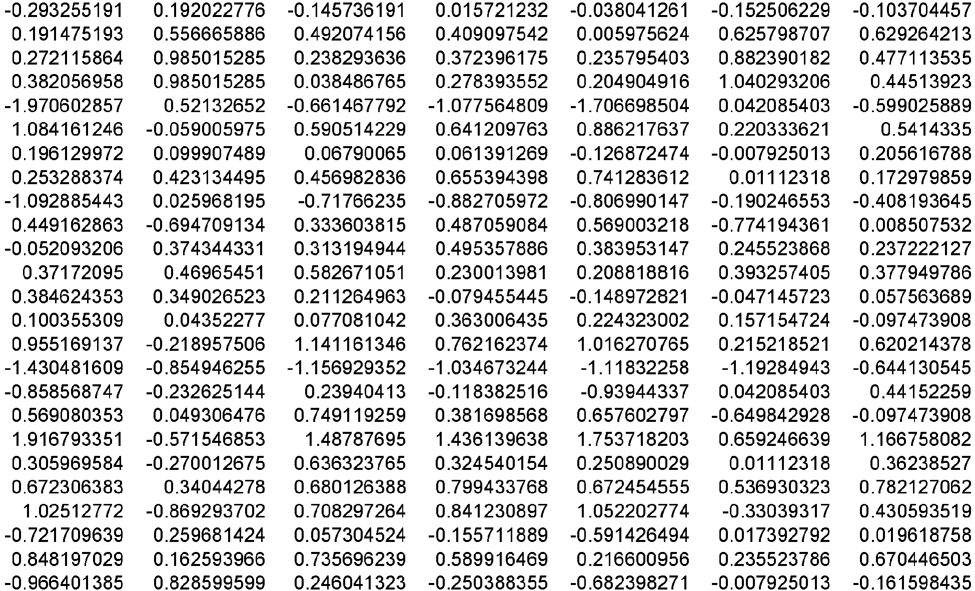

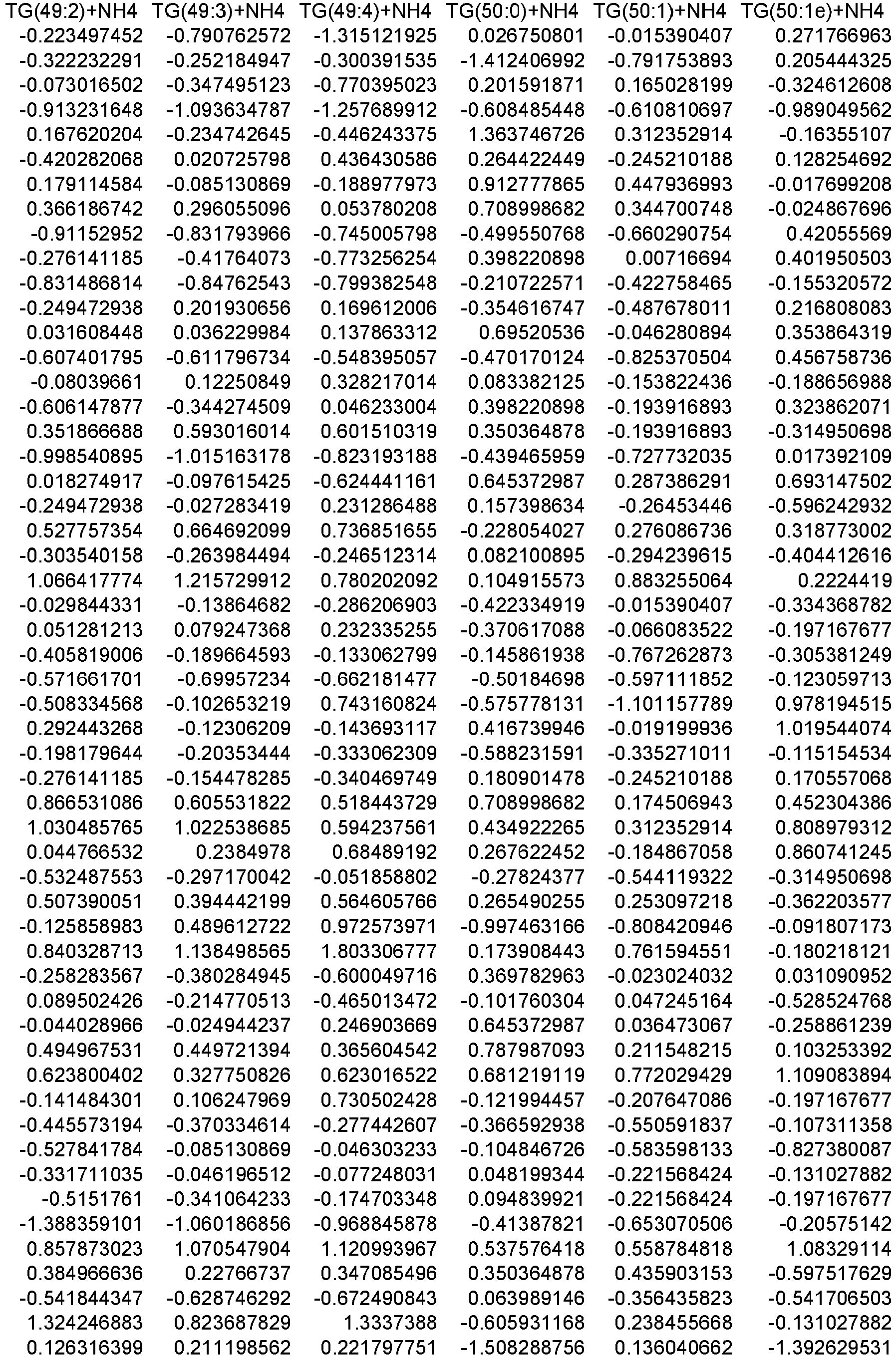

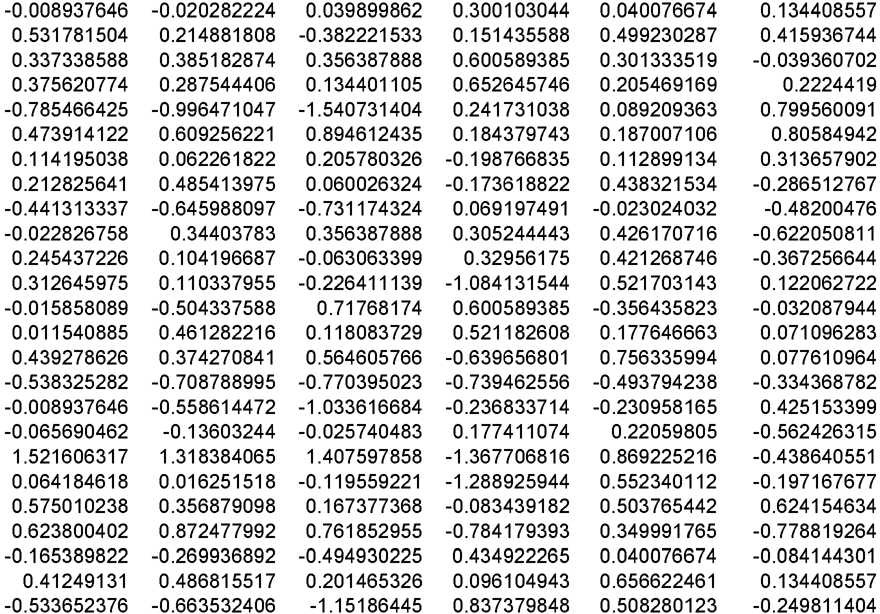

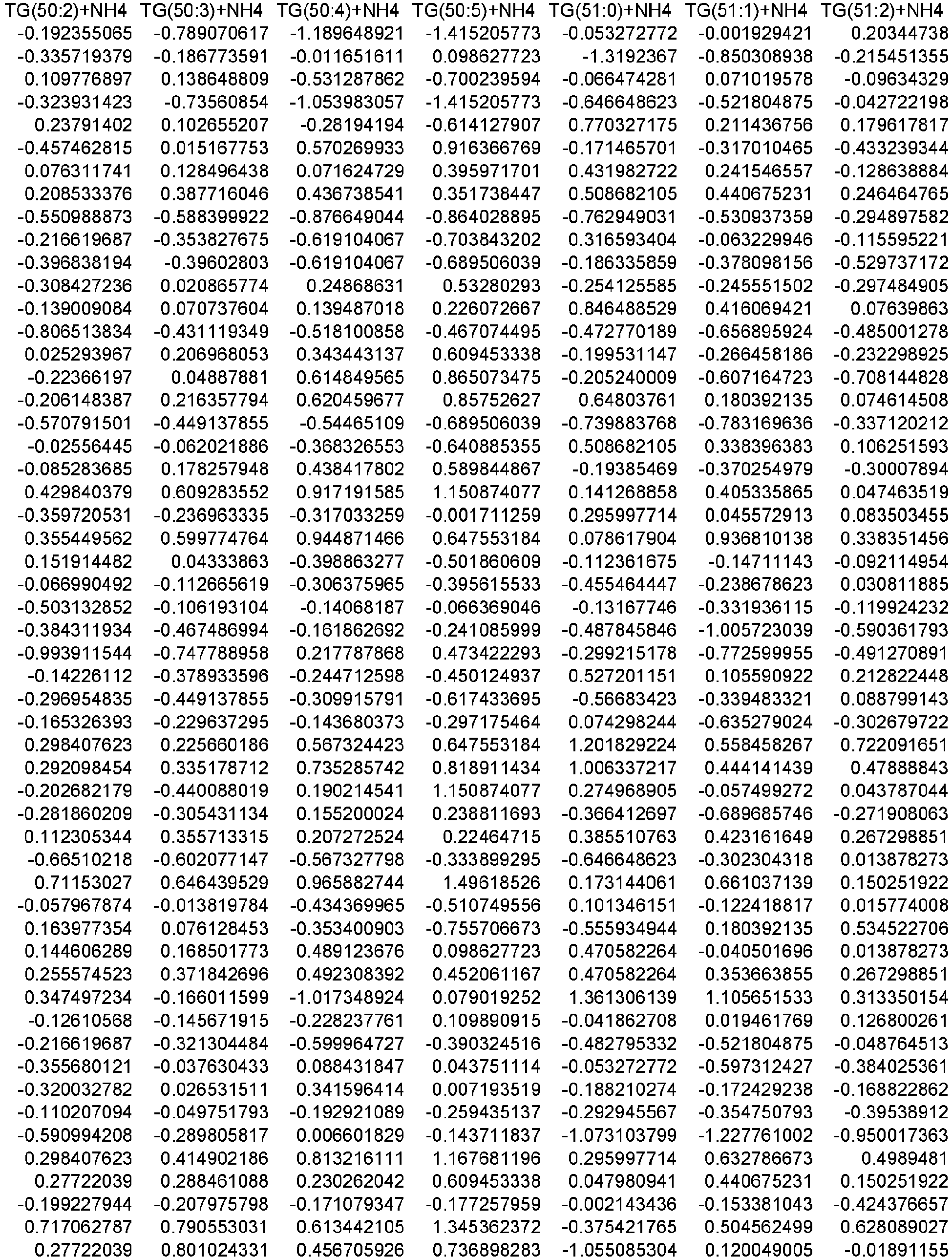

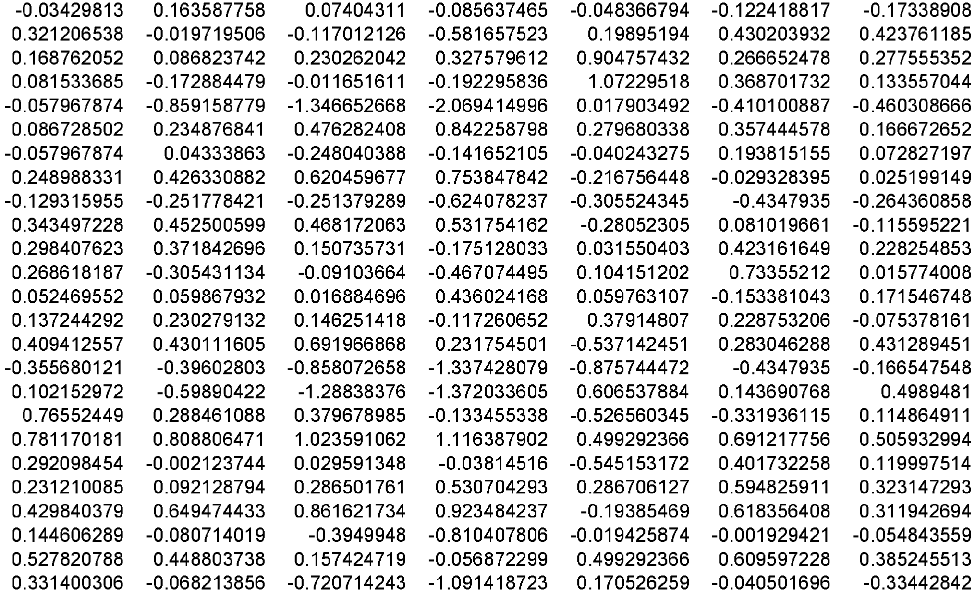

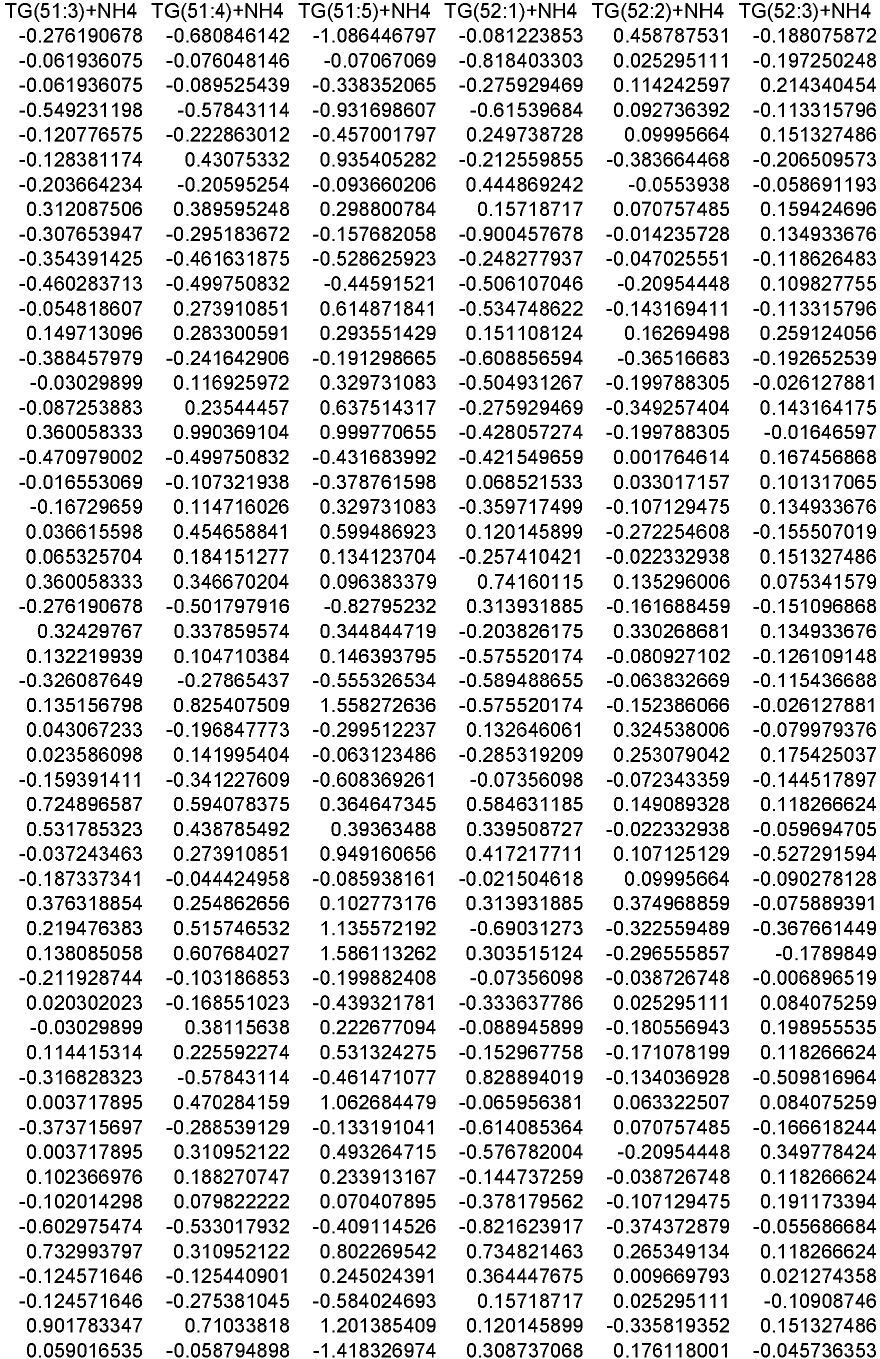

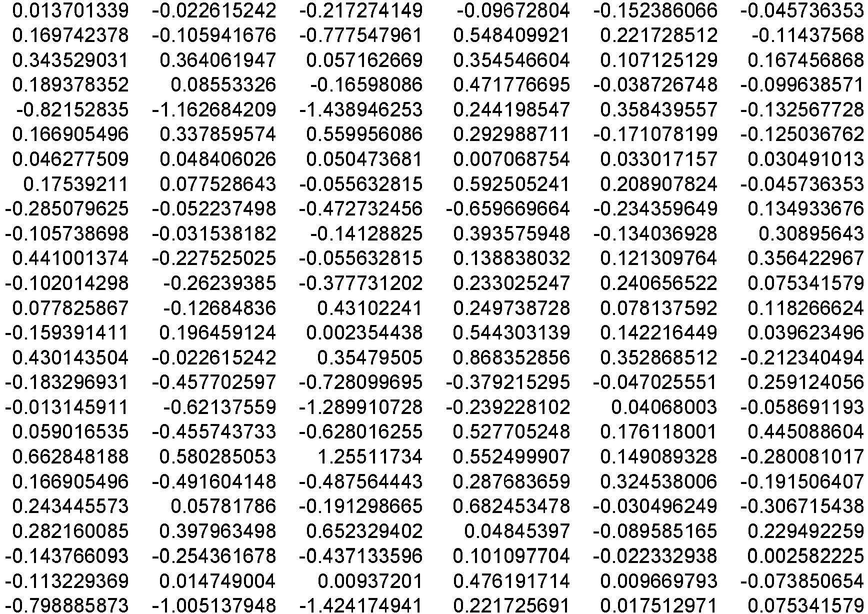

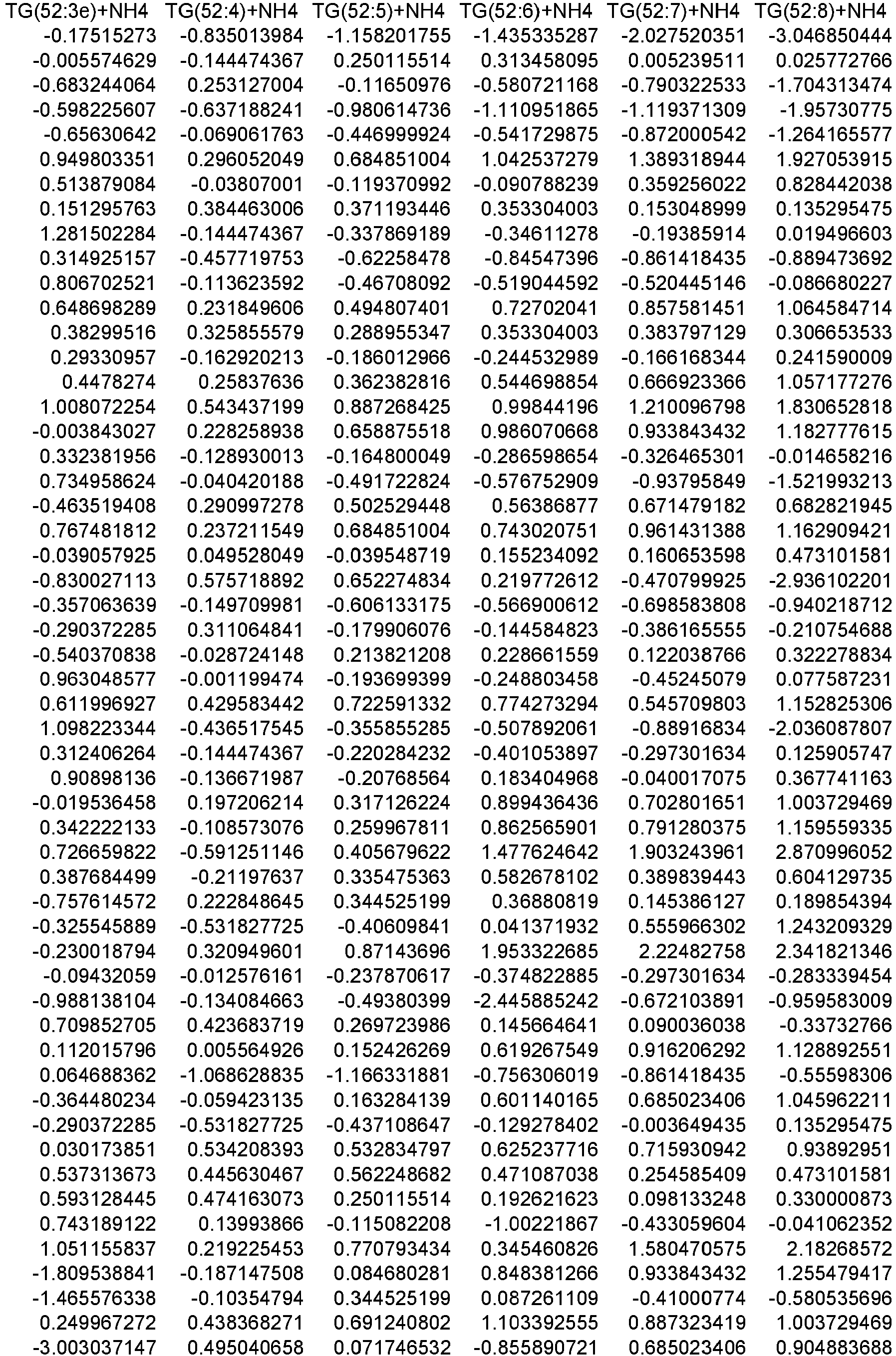

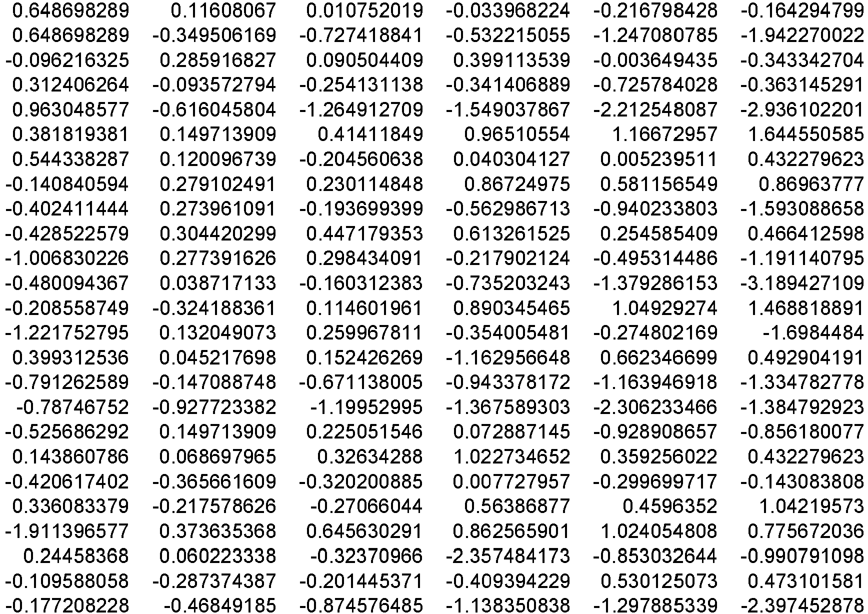

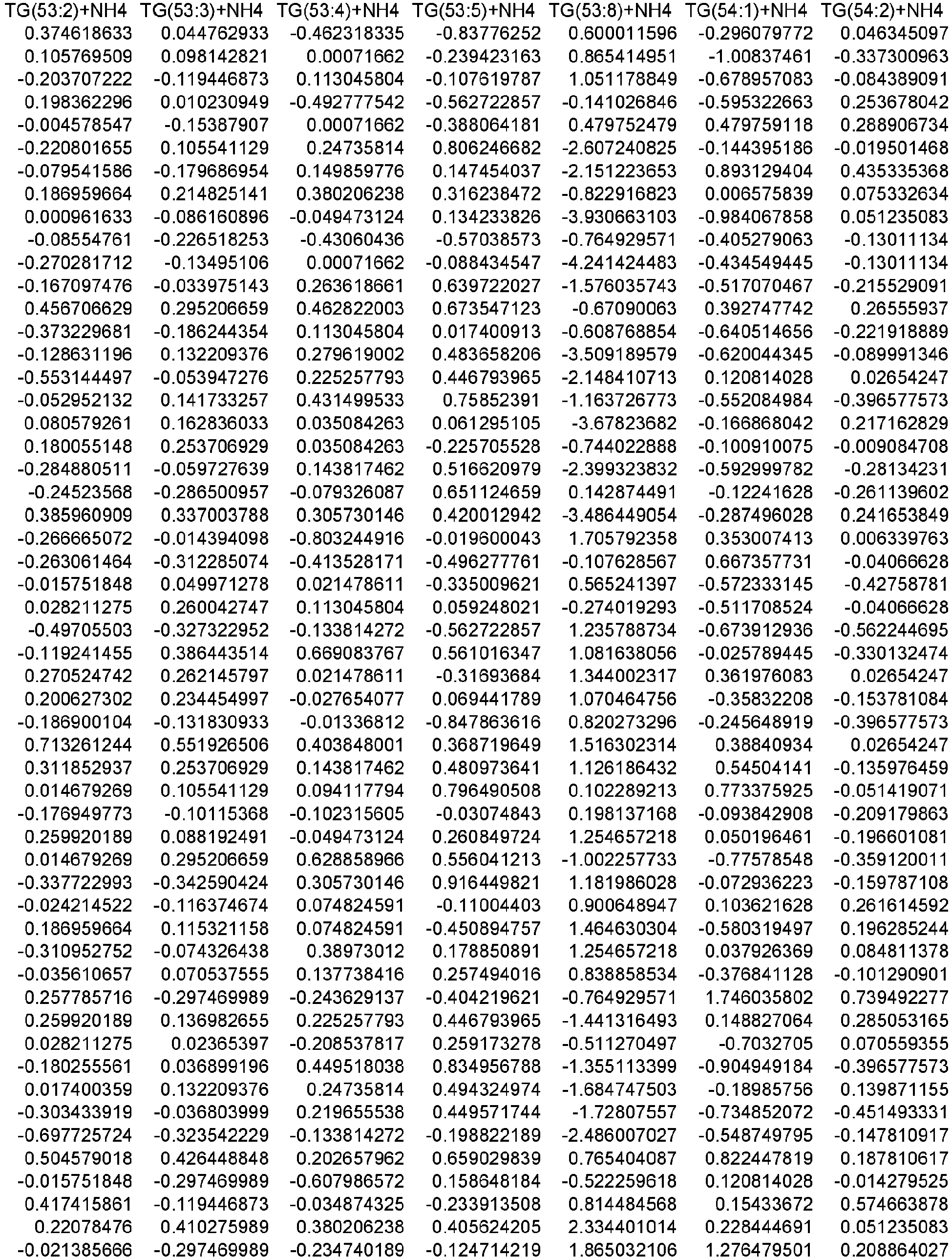

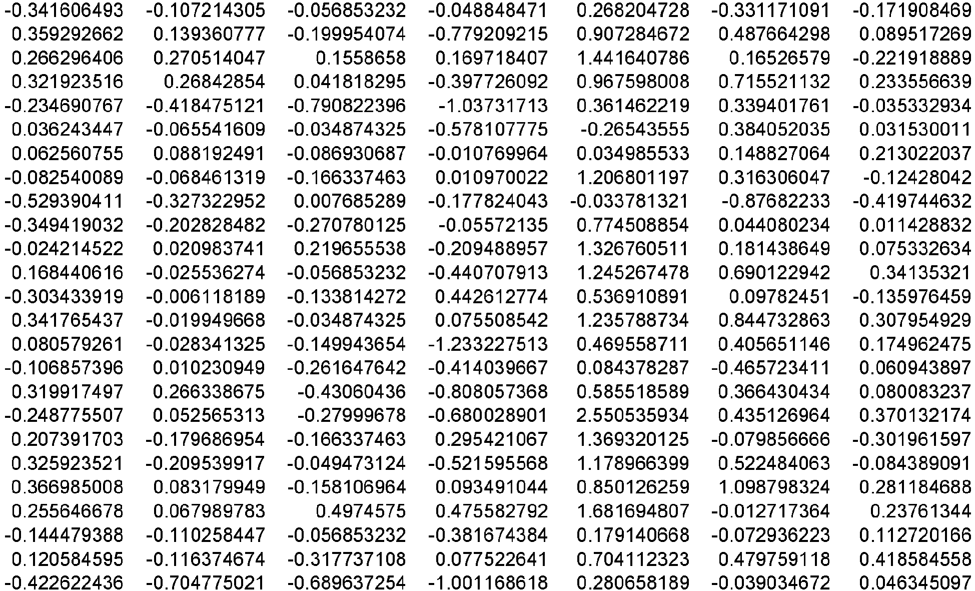

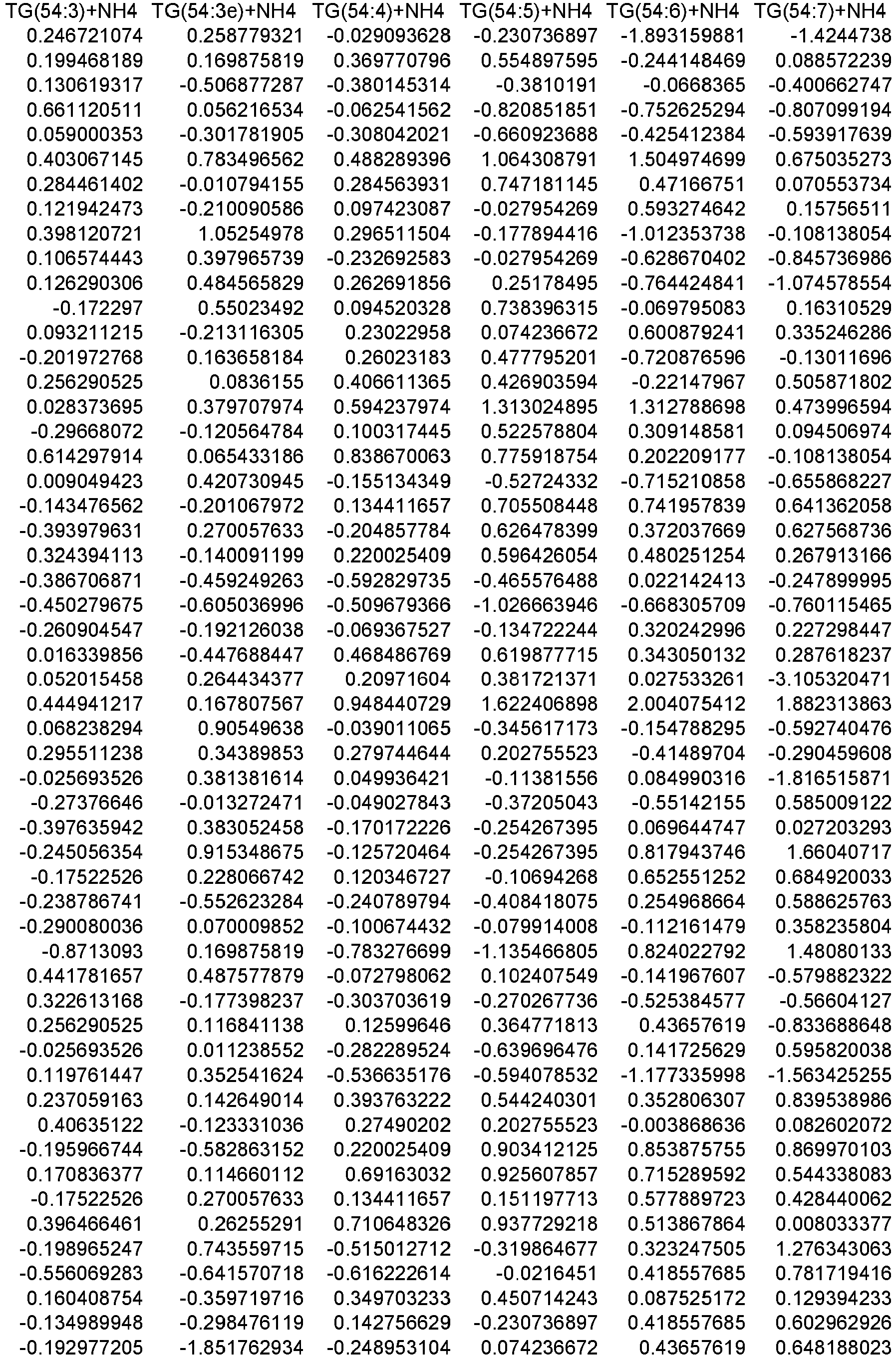

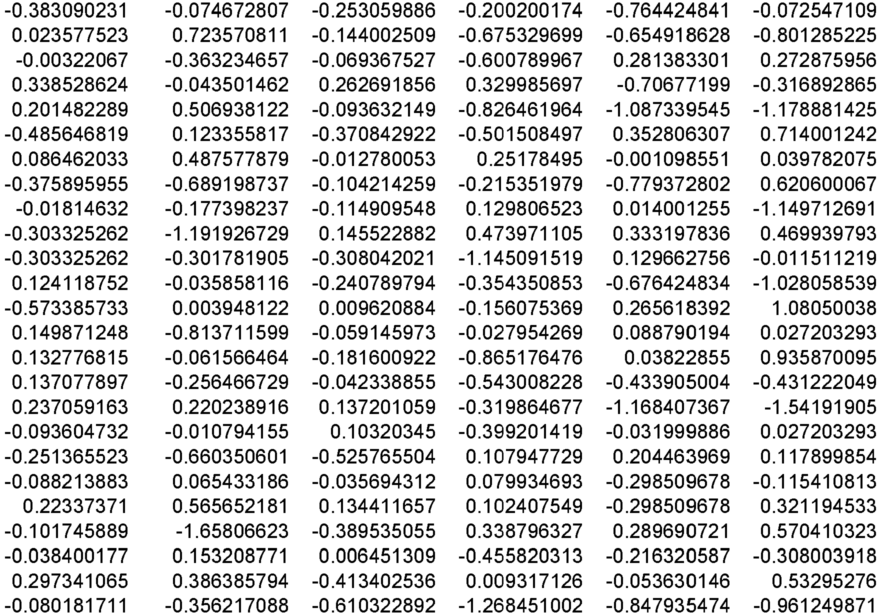

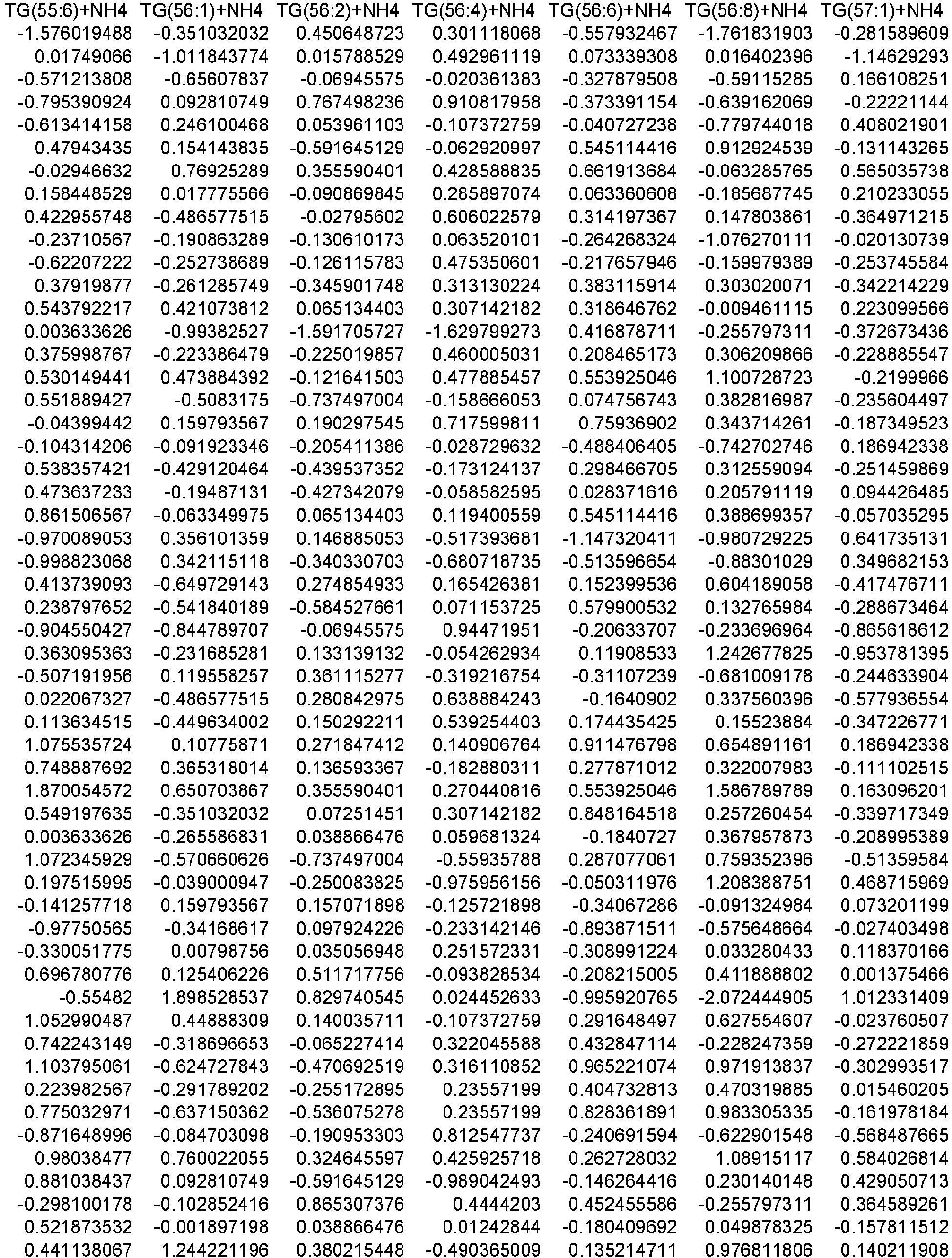

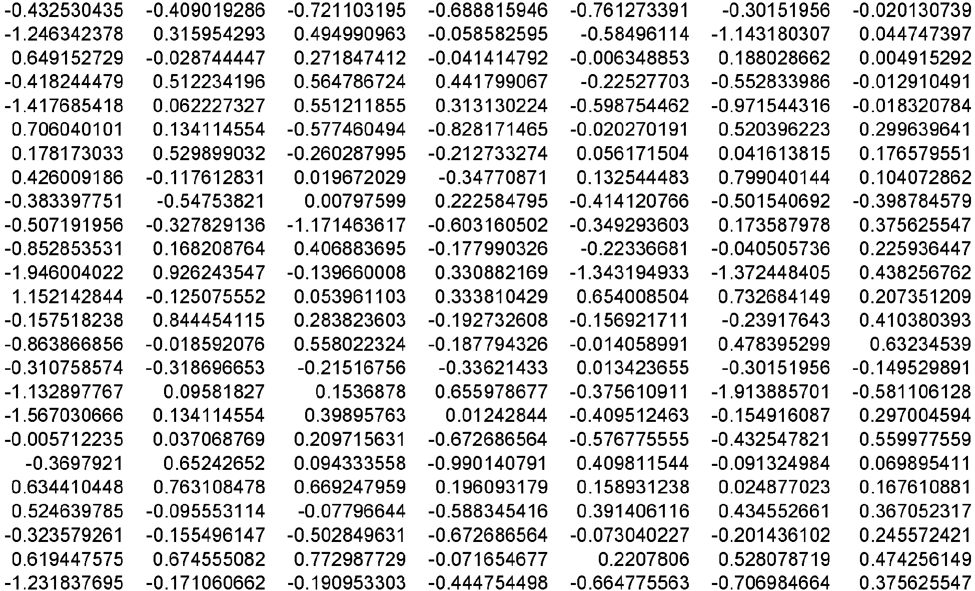

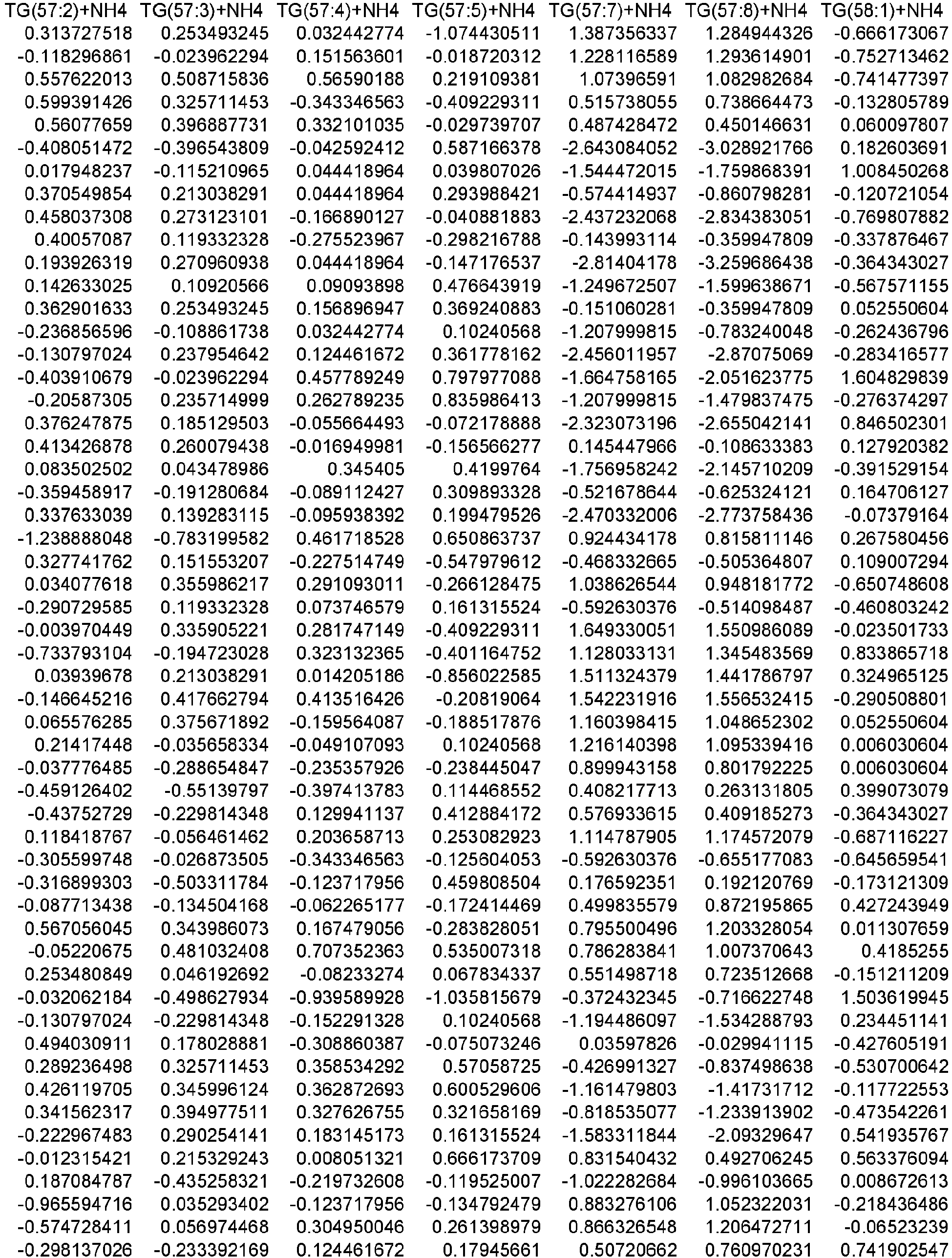

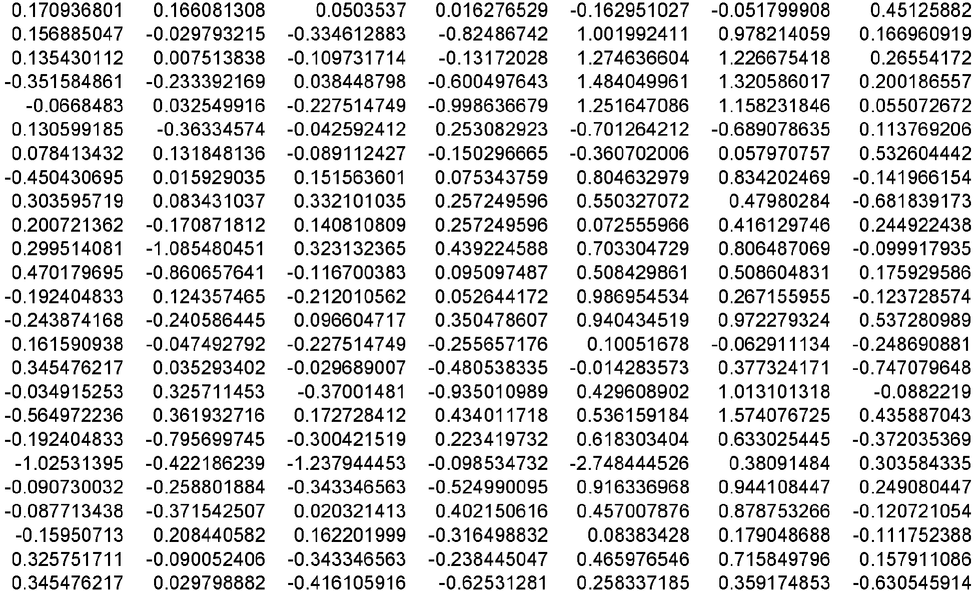

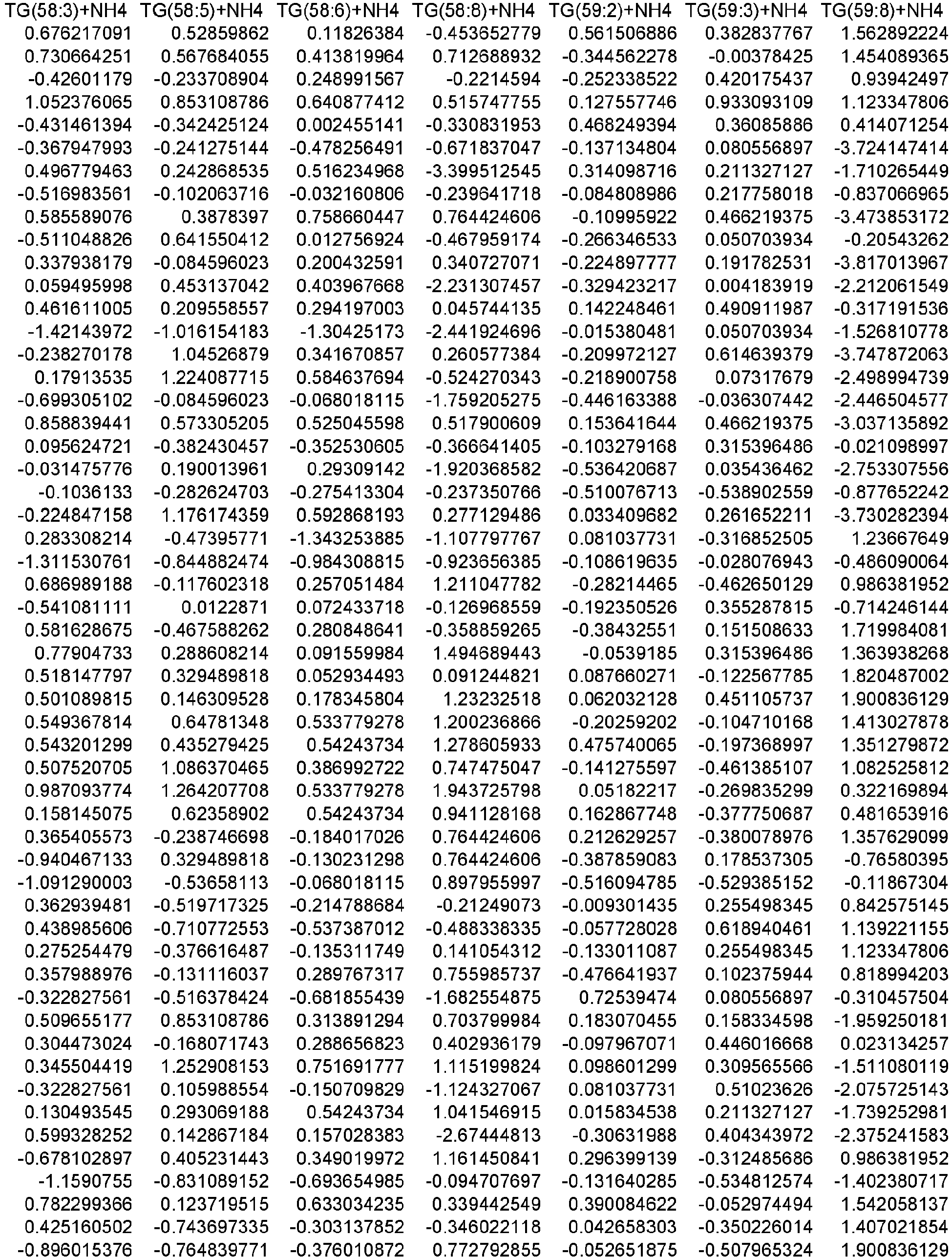

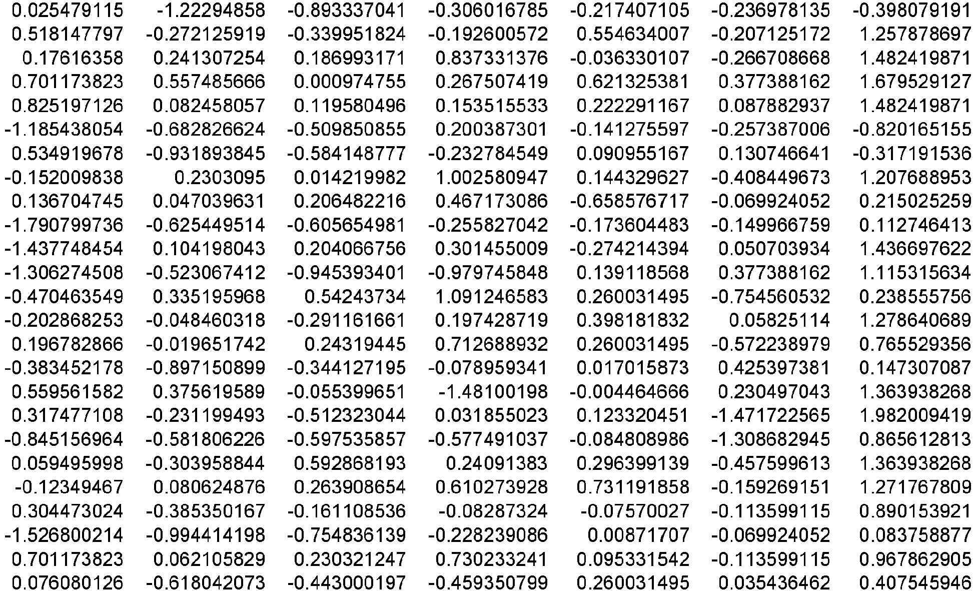

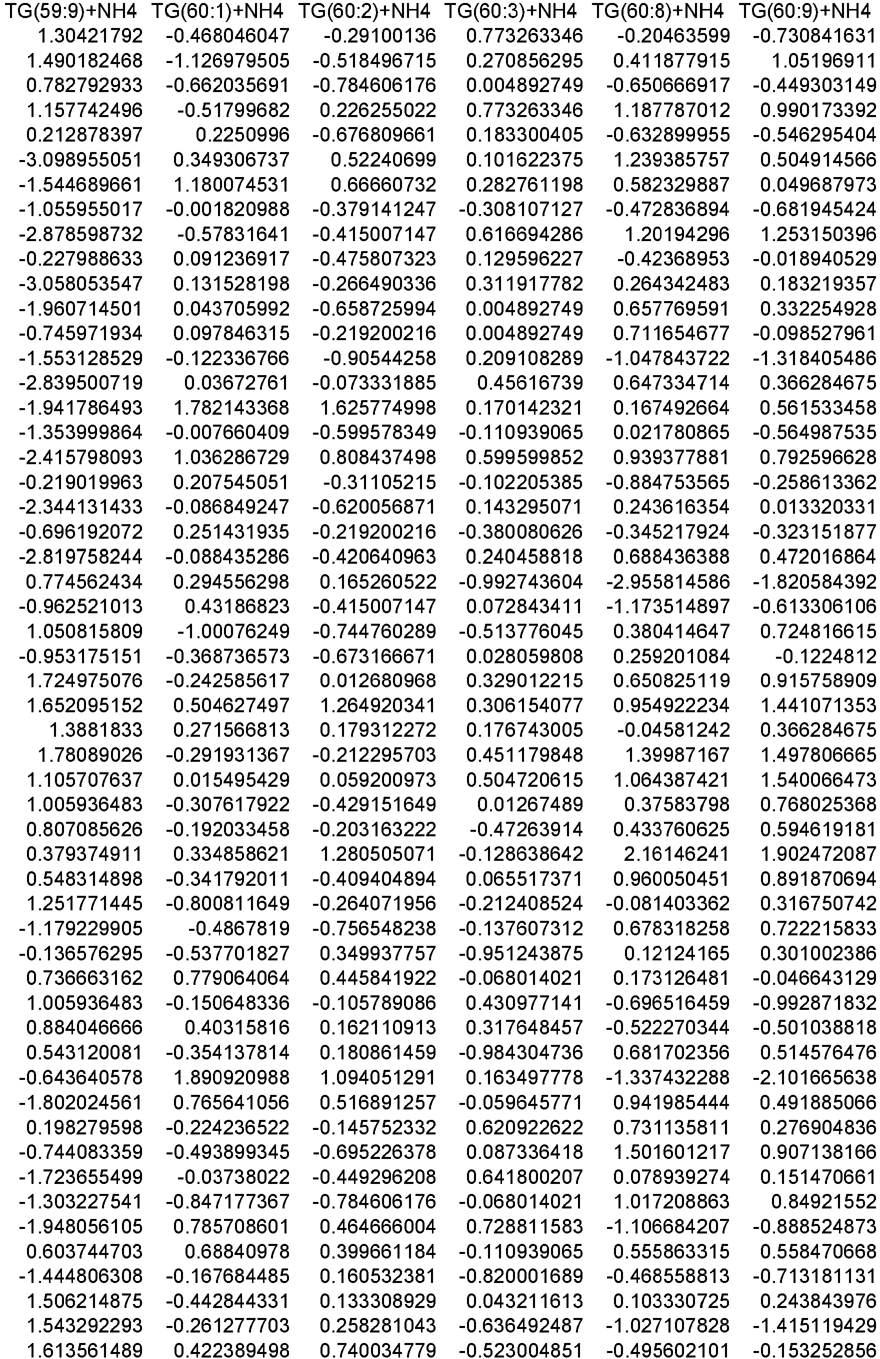

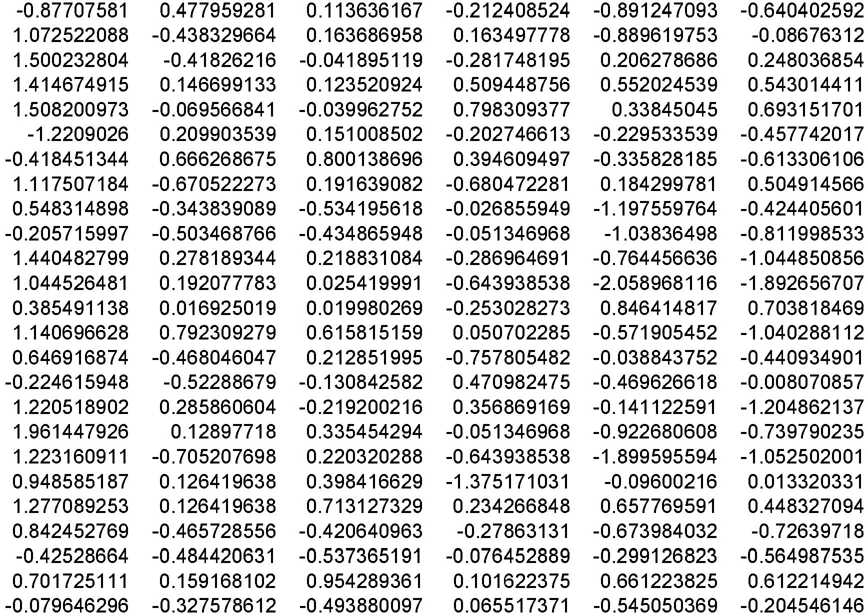

## Claims

1. A method for diagnosing nonalcoholic steatohepatitis (NASH) or nonalcoholic fatty liver (NAFL) in a human subject, the method comprising
(a) determining a level of 29 lipids in a biological sample obtained from the subject, wherein the 29 lipids comprise:
(i) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); or
(ii) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0);
wherein the biological sample is a whole blood, serum, or plasma sample.

2. A method of monitoring treatment efficacy in a human subject being treated with a NASH or NAFL therapy, the method comprising:
(a) identifying the subject's condition as unchanged or worsened by:
(i) determining that a level of 29 lipids in a biological sample obtained from the subject differs from a reference level or is unchanged relative to a previous sample obtained from the subject, wherein the 29 lipids comprise:
(a) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); or
(b) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0);
wherein the biological sample is a whole blood, serum, or plasma sample; and
(b) modifying the therapy to be administered to the subject.

3. A method of monitoring disease progression in a human subject with NAFL, the method comprising:
(a) identifying that the NAFL has progressed to NASH by
(i) determining that a level of 29 lipids in a biological sample obtained from the subject differs from a reference level, wherein the 29 lipids comprise:
(a) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), and TG(53:5); or
(b) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), and TG(40:0);
wherein the biological sample is a whole blood, serum, or plasma sample; and
(b) selecting a NASH therapy to be administered to said subject.

4. A NASH or NAFL therapeutic for use in treating in a human subject diagnosed as having NASH or NAFL by the method of claim 1.

5. The method of claim 2 or the therapeutic for use according to claim 4, wherein the NASH therapy or treatment is weight loss, a dietary change, increased physical activity and/or wherein the NASH therapy, treatment or therapeutic is an anti-inflammatory agent, an anti-apoptosis agent, a medication to treat insulin resistance or type 2 diabetes, a medication to improve cholesterol, Vitamin E, a peroxisome proliferator-activated receptor (PPAR) agonist, glutathione, usrodeoxycholic acid, pemafibrate, aramchol, GS0976, an anti-hypertensive drug, a farnesoid X receptor (FXR) agonist, MGL-3196, BI 1467335, IMM-124E, solithromycin, BMS-986036, Cenicriviroc (CVC), or JKB-121.

6. The method of claim 2 or 3, wherein the NAFL therapy or treatment is weight loss, a dietary change, increased physical activity.

7. The method of any one of claims 2, 4, 5 and 6, wherein modifying the NASH therapy or NAFL therapy comprises modifying the dosage, frequency of administration, or therapeutic agent to be administered to the subject.

## Patentansprüche

1. Verfahren zur Diagnose von nichtalkoholischer Steatohepatitis (NASH) oder nichtalkoholischer Fettleber (NAFL) bei einem menschlichen Subjekt, wobei das Verfahren umfasst:
(a) Bestimmen eines Spiegels von 29 Lipiden in einer von dem Subjekt erhaltenen biologischen Probe, wobei die 29 Lipide umfassen:
(i) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), und TG(53:5); oder
(ii) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), und TG(40:0);
wobei die biologische Probe eine Vollblut-, Serum- oder Plasmaprobe ist.

2. Verfahren zur Überwachung der Wirksamkeit einer Behandlung bei einem menschlichen Subjekt, das mit einer NASH- oder NAFL-Therapie behandelt wird, wobei das Verfahren umfasst:
(a) Identifizieren des Zustand des Subjekts als unverändert oder verschlechtert durch:
(i) Bestimmen, dass ein Spiegel von 29 Lipiden in einer von dem Subjekt erhaltenen biologischen Probe sich von einem Referenzwert unterscheidet oder im Vergleich zu einer zuvor vom Subjekt entnommenen Probe unverändert ist, wobei die 29 Lipide umfassen:
(a) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), und TG(53:5); oder
(b) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), und TG(40:0);
wobei die biologische Probe eine Vollblut-, Serum- oder Plasmaprobe ist; und
(b) Anpassen der dem Subjekt zu verabreichenden Therapie.

3. Verfahren zur Überwachung des Krankheitsverlaufs bei einem menschlichen Subjekt mit NAFL, wobei das Verfahren umfasst:
(a) Identifizieren, dass sich die NAFL zu NASH entwickelt hat, durch
(i) Bestimmen, dass ein Spiegel von 29 Lipiden in einer von dem Subjekt erhaltenen biologischen Probe sich von einem Referenzwert unterscheidet, wobei die 29 Lipide umfassen:
(a) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1), und TG(53:5); oder
(b) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1), und TG(40:0);
wobei die biologische Probe eine Vollblut-, Serum- oder Plasmaprobe ist; und
(b) Auswählen einer dem Subjekt zu verabreichenden NASH-Therapie.

4. NASH- oder NAFL-Therapeutikum zur Verwendung bei der Behandlung eines menschlichen Subjekts, das durch das Verfahren nach Anspruch 1 als an NASH oder NAFL leidend diagnostiziert wurde.

5. Verfahren nach Anspruch 2 oder Therapeutikum zur Verwendung nach Anspruch 4, wobei die NASH-Therapie oder Behandlung Gewichtsverlust, eine Ernährungsumstellung, erhöhte körperliche Aktivität und/oder wobei die NASH-Therapie, Behandlung oder das Therapeutikum ein entzündungshemmendes Mittel, ein Anti-Apoptose-Mittel, ein Medikament zur Behandlung von Insulinresistenz oder Typ-2-Diabetes, ein Medikament zur Verbesserung von Cholesterin, Vitamin E, ein Peroxisom-Proliferatoraktivierter Rezeptor (PPAR)-Agonist, Glutathion, Ursodeoxycholsäure, Pemafibrat, Aramchol, GS0976, ein Antihypertensivum, ein Farnesoid-X-Rezeptor (FXR)-Agonist, MGL-3196, BI 1467335, IMM-124E, Solithromycin, BMS-986036, Cenicriviroc (CVC) oder JKB-121 ist.

6. Verfahren nach Anspruch 2 oder 3, wobei die NAFL-Therapie oder Behandlung Gewichtsverlust, eine Ernährungsumstellung, erhöhte körperliche Aktivität ist.

7. Verfahren nach einem der Ansprüche 2, 4, 5 und 6, wobei das Anpassen der NASH- oder NAFL-Therapie das Anpassen der Dosierung, der Verabreichungshäufigkeit oder des zu verabreichenden Therapeutikums an das Subjekt umfasst.

## Revendications

1. Procédé de diagnostic de la stéatohépatite non alcoolique (NASH) ou de la stéatose hépatique non alcoolique (NAFL) chez un sujet humain, le procédé comprenant
(a) la détermination du taux de 29 lipides dans un échantillon biologique prélevé sur le sujet, les 29 lipides comprenant :
(i) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1) et TG(53:5) ; ou
(ii) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1) et TG(40:0) ;
dans lequel l'échantillon biologique est un échantillon de sang total, de sérum ou de plasma.

2. Procédé de surveillance de l'efficacité d'un traitement chez un sujet humain traité par une thérapie contre la NASH ou la NAFL, le procédé comprenant :
(a) l'identification de l'état du sujet comme inchangé ou aggravé par :
(i) déterminant qu'un niveau de 29 lipides dans un échantillon biologique obtenu à partir du sujet diffère d'un niveau de référence ou est inchangé par rapport à un échantillon précédent obtenu à partir du sujet, les 29 lipides comprenant :
(a) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1) et TG(53:5) ; ou
(b) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1) et TG(40:0) ;
dans lequel l'échantillon biologique est un échantillon de sang total, de sérum ou de plasma ; et
(b) modifier le traitement à administrer au sujet.

3. Procédé de surveillance de la progression d'une maladie chez un sujet humain atteint de NAFL, le procédé comprenant :
(a) l'identification du fait que la NAFL a évolué vers une NASH par
(i) déterminant qu'un niveau de 29 lipides dans un échantillon biologique obtenu à partir du sujet diffère d'un niveau de référence, les 29 lipides comprenant :
(a) DG(34:1), LPC(20:0e), PC(34:2e), PC(36:4), PC(36:5e), PC(40:8), PE(38:1), LPC(22:5), LPE(16:0), PC(35:3), PC(42:6), AcCa(10:0), Cer(d34:2), DG(36:4), PC(32:0), PC(32:1e), PC(34:0), PC(37:2), PC(40:6e), PC(40:7), PE(38:6), PI(36:1), SM(d32:0), SM(d32:2), SM(d40:1), TG(38:0), TG(38:2), TG(43:1) et TG(53:5) ; ou
(b) DG(34:1), LPC(20:0e), LPC(22:5), PC(40:7), PE(34:1), PE(34:3e), PC(34:1), PC(36:5e), PC(40:8), PE(38:1), PI(36:1), SM(d40:1), AcCa(10:0), Cer(d34:2), DG(36:4), Hex1Cer(d34:2), LPE(18:0), LPE(22:5), PA(44:4), PC(35:3), PC(37:2), PC(42:6), PE(40:4e), SM(d32:0), SM(d32:2), SM(d36:0), SM(d36:4), SM(d37:1) et TG(40:0) ;
dans lequel l'échantillon biologique est un échantillon de sang total, de sérum ou de plasma ; et
(b) sélectionner un traitement NASH à administrer audit sujet.

4. Thérapie contre la NASH ou la NAFL destinée à être utilisée dans le traitement d'un sujet humain diagnostiqué comme souffrant de NASH ou de NAFL selon le procédé de la revendication 1.

5. Procédé selon la revendication 2 ou traitement selon la revendication 4, dans lequel le traitement de la NASH consiste en une perte de poids, un changement de régime alimentaire, une augmentation de l'activité physique et/ou dans lequel le traitement de la NASH est un agent anti-inflammatoire, un agent anti-apoptotique, un médicament pour traiter la résistance à l'insuline ou le diabète de type 2, un médicament pour améliorer le cholestérol, de la vitamine E, un agoniste des récepteurs activés par les proliférateurs de peroxysomes (PPAR), du glutathion, de l'acide usérodésoxycholique, du pemafibrate, de l'aramchol, du GS0976, un médicament antihypertenseur, un agoniste des récepteurs farnesoïdes X (FXR), du MGL-3196, du BI 1467335, IMM-124E, solithromycine, BMS-986036, cénicriviroc (CVC) ou JKB-121.

6. Procédé selon la revendication 2 ou 3, dans lequel le traitement ou la thérapie de la NAFL consiste en une perte de poids, un changement de régime alimentaire, une augmentation de l'activité physique.

7. Procédé selon l'une quelconque des revendications 2, 4, 5 et 6, dans lequel la modification du traitement de la NASH ou de la NAFL comprend la modification de la posologie, de la fréquence d'administration ou de l'agent thérapeutique à administrer au sujet.
